# EUROPEAN PATENT APPLICATION

(11) **EP 1 398 378 A1**
(43) Date of publication of application: **17.03.2004**
(21) Application number: 02730804.8
(22) Date of filing: 30.05.2002
(51) Int. Cl.: C12N 15/12, C12Q 1/68, G01N 33/53, G01N 33/566

(54) **NUCLEIC ACIDS ISOLATED IN NEUROBLASTOMA**

(30) Priority: 31.05.2001 JP 2001163666; 24.08.2001 JP 2001255260
(71) Applicant: Chiba-Prefecture, Chiba-shi, Chiba 260-8667 (JP); HISAMITSU PHARMACEUTICAL CO., INC., Tosu-shi Saga 841-0017 (JP)
(72) Inventor: NAKAGAWARA, Akira, Chiba Cancer Center Res. Inst., Chiba-shi, Chiba 260-0801 (JP)
(74) Representative: Grünecker, Kinkeldey, Stockmair & Schwanhäusser Anwaltssozietät
(86) International application number: PCT/JP2002/005295
(87) International publication number: WO 2002/103017

(57) **Abstract**

There are disclosed a nucleic acid whose expression is enhanced in human neuroblastoma with favorable prognosis based on comparison between human neuroblastoma with favorable prognosis and human neuroblastoma with unfavorable prognosis, the nucleic acid comprising any one of base sequences set forth in SEQ ID NO:1 to NO:366 in the Sequence Listing, a nucleic acid comprising a portion of any of those base sequences, and an isolated nucleic acid capable of hybridizing to a complementary base sequence of the foregoing under stringent conditions. It discloses gene sequences relating to favorable or unfavorable prognosis of neuroblastoma and will enable the provision of their genetic information and the diagnosis of favorable or unfavorable prognosis.

SEQUENCE LISTING

<110> Hisamitsu Pharmaceutical Co., Inc.

<120> Nucleic acids isolated from neuroblastoma

<130> FP02-0111-00WO

<140>
<141>

<150> JP2001-163666
<151> 2001-05-31

<150> JP2001-255260
<151> 2001-08-24

<160> 366

<170> PatentIn Ver. 2.1

<210> 1
<211> 824
<212> DNA
<213> Homo sapiens

<220>
<221> misc_feature
<222> (1)...(824)
<223> n = a, t, c or g

<220>
<223> nbla-00015-f

<400> 1
ggatcctcga gcactgngtc gcctactggg atattgacag tgcctcagtg cccagcttag 60
aagagctgaa ccagttcagg caacatgtga caactttgag ggaaacaaag gcttctaggc 120
gtgaggagtt tgtcagtata aagagacaga tcatactgtg tatggaagaa ttagaccaca 180
ccccagacac aagctttgaa agaggtgtgg tgtgtgaaga cgaagatgcc ttttgtttgt 240
ctttggagaa tattgcaaca ctacaaaagt tgctacggca gctggaaatg cagaaatcac 300
aaaatgaagc agtgtgtgag gggctgcgta ctcaaatccg agagctctgg gacaggttgc 360
aaatacctga agaagaaaga gaagctgtgg ccaccattat gtctgggtca aaggccaagg 420

tccggaaagc gctgcaatta gaagtggatc ggttggaaga actgaaaatg caaaacatga 480
agaaagtgat tgaggcaatt cgagtggagc tggttcagta ctgggaccag tgcttttata 540
gccaggaaca gagacaagct tttgcccctt tctgtgctga ggactacaca gaaagtctgc 600
tccagctcca cgatgctgaa gattgtgcgg ttaaaaaact actatgaagt tcacaaggaa 660
ctccttgaag gtgtccaaaa gtgggaagaa acctggaggc tttcctaaag tttgaganaa 720
aagcntcaaa ttcaaatcca nttacaaacc ganggaggaa tcctccaaaa gaagaaaaca 780
acgaagccaa nctccaaaaa atgctgccca annctggaan aana 824

<210> 2
<211> 806
<212> DNA
<213> Homo sapiens

<220>
<221> misc_feature
<222> (1)...(806)
<223> n = a, t, c or g

<220>
<223> nbla-00015-rl

<400> 2
tttttttgt ntttttttt ttttgttttt tcattatggt tctcggtgta ctttattctc 60
ctgctgtcta gctaagcagc ccacacagga cagaaatggg tancactgag gaatgatcag 120
attttttttc tatccccaat atttctgccc cctccctaan ccctgaccca accctatctt 180
aataatcacg aatggttagt acatcattct gtctctcaaa gaacaactga aatggaaaca 240
aggcattaga ggaccangcc tcttcttaat agggcttang anctggtatg agctctttat 300
tcacaatgga gcgtctctcc ttcaaatacc tggatttttt tttttttgt acactggttc 360
atagatcggc acttggcttt gaacctggca ccaaaaggca caatatctga taccctgtac 420
aagagctatt agagatgctg ccatatggat gggcaaaact gagccaatcc cacttaggaa 480
tggaaggctt ggacatggaa gggaggatat aaacgaggag ttggagaaaa acgcaagccc 540
antttttgct anagtggaaa tgaaagtggg aatgagggtc ttgtttttan tcctctaagg 600
nccaaggaag caattttaaa acttccctgg gttttctgaa agcagcatat tcaaaatgcc 660
aacaaaaact cctaacaact gnaaaaccaa aagagggtta aagctcaaca aaatcccttc 720
ctaatgctga aaggntctaa aattcangat gccctgttcc cttgtaaagg gaaaatantt 780
aatctgattt natgggaatc caancn806

<210> 3
<211> 3181
<212> DNA

<213> Homo sapiens

<220>
<223> nbla-00018

<400> 3
cctactggta tgatgatcta gttgtgtagg aaagaacttg gcatgtatgg cacagatcag 60
tgactggtta acatgtccat tgggctgcct ttttcaaata ttgggtattt tatacaaggt 120
tttgatcgat attggaccac attcagaacc ctactaaatc atacaaaggg ttactttatt 180
taagatattt taatgtttgg ataaaatgag agcaatgttt ttagatactt gaactactaa 240
gcctgttggt ggaaaaataa aaggattaga cttctcttct gcacttatat actgttggta 300
ggaacgtaaa ttagttcagc cacaaattgg aaagcagttt ggagatttct caaagaactt 360
aaactaccat tcaacccagc aatcccatta ctgaatatgt attcagaaga aaataaattg 420
tcttgccaaa aaatacgtgt tcacatgttc attgaagcac tatttatgat agcaaagaca 480
cagaatcaac ctaggtgccc atcaacagtg gactggatga agaaaatgtg gtacatatat 540
actatggact accacacagc catgagaaag gaacaaagtc atgtttttgg cagcaacatg 600
gatgcagctg gaggccatta tcccaagcaa attaatgcag gaacaggaaa ccaagtacta 660
cgtattctca tttactagtg ggagctaaat gtggggtact catggacata aagatgacaa 720
caatagacac tggggactac tgttgtggca gagttgaaaa actacctatt gggtactatg 780
ctcacaacct aggtgatgag atctttcgca caccaaactt cagtgtcatg caatataccc 840
acgtaacaaa cctgcacata tacctcctga atataaaggt tgagattatt tttttaaaag 900
aattatgttc tgtccaaacc tgaggccaac atggcttagc acatgcatga gcaaaacagt 960
atccatggat gagcattatc tgggggattt cttttctaaa ttcaatccaa tttagcaata 1020
tttatagagc atctactata agctggacct gaaggttcag acttgagtta cctactctac 1080
cattagtact tgttactaat gtgtgaatac ttagtacttt attagtccct acactaccaa 1140
tttgttcagg caccaaggtg ccaaaccata taatacaatt tctcccgtaa gtccccaaag 1200
catagctgca tctaggctat aatgctgaat aggacttttc taaaaactca ggaattgatg 1260
aaaggagttg aaagcagaaa ggcaaagctg aaggataggt cctctgtaac agtgtcccca 1320
aagaccacag gctgtgcccc cttccttgga gttcgtctgg tgaccccttc tgagcccccg 1380
gagaccactg tgttgcagag tctatctcat ttcccccaag tgggaacccc tttaccagat 1440
ctctcctagc cagagtccag gcctcttaac cacctctggg ccccagtgag gaaggaaagt 1500
ctgttttctc tacacctggg ctatgttact tcagtagcca ctaacctggg gaactagctt 1560
tgatttaaat aaattttttt ttattctcaa gagcatttta agtgctcaat agctagtggc 1620
tactgtctta ttaaatggca cagatatatc gcatgtccat catcatagaa agttattttg 1680
aatgaagcca tgacaccatc catcttaggc cttccttccc ttcaatgtca taatgatctg 1740
aaaatgcaac atttccctca aggaagatgg ttagtccctg accccatagg tgagcatcac 1800
agcctgtact tccactcaca ttaggcctca aatataattt tatttatctg ctattgtcta 1860
ttttgcacac tctatttgct atctaagccc ctggaaagtt gaggtgccct agtcatcctc 1920
tgtctctagg ttttcctttg gtccccaggt gcttaccatg tgcctggctc tgtgcaagtg 1980
actttaacat gaacttaatt ctctcagtct ttcagaggat gatttgtttt tgctgcctga 2040
tagccttttt tctatttacc ttccttgaca tattaataga gtcaccaaaa tgttcagctg 2100
catttcattc acctagaagg caatgaatgt aaaaacatca acaattctgc ctgctacgaa 2160

ataagtgctc agtaaatgtc cattcccttc tttacttctg caggtgatat ttatagtaat 2220
aatgattttt taagttataa tttattatcc agcttcttta ttaggcattt tacacttatt 2280
gtcataacaa tcctatgagg gctaaagtaa aaatgtaata aaatatagct ctctcccaca 2340
tcctgttttt catctcacat gcagattttg aattccccaa gaacagacct gtgtcatctt 2400
acacattttg gaatccctgg agtatagccc agtacctggt gctgggaggg actcagtata 2460
tatttattga attcaaaaat gaatgaataa atcacttctt ggccttcctg tccgccactc 2520
ctggtcaccc agcacctata caaacctgct ttcctctccc tagcctatca aaattcctgt 2580
tacttcaact tgaatctctc ctggtctagg agctgctttc caaactaaac tcagctgcgt 2640
taagggagat ggaaaaattc acaggatact aatagaattt tagaccagtg caccaaaccc 2700
aatgtcagag gcagaggggc aagtttattt gagcagcaag gagactttgc attcagctct 2760
gtagatgacc ttggaagaca ggcctaggct aactgggcta agcctaggct aaaagtctat 2820
cgccaagctt tgttcaaaat taagagtgtt ttaagcaggg cctcaggctc tgggctgtga 2880
aggccccaag tgaccaccag aagatgctag aaaggacatc agctactgga gcatggagag 2940
gaggtgagcc tggctccagt aagagtgtgg caaactcctg atggttttac aagggtgtag 3000
ggcttttgga gactctactt tggtttgtgt agtgatgcat ttgcaaacat atgtttaaaa 3060
tactaacggc tctccacatt cttagatttc acatccatgg ttttaaccaa ccatggatgg 3120
aagatatttg gggggaaaaa aaaaaaaaaa aaaaaaaagg ccacatgtgc tcgagctgca 3180
g 3181

<210> 4
<211> 869
<212> DNA
<213> Homo sapiens

<220>
<221> misc_feature
<222> (1)...(869)
<223> n = a, t, c or g

<220>
<223> nbla-00059-f

<400> 4
cncgancact gttggcctac tggttttttt ttttgcggct ccacgtcggc accagctgcg 60
gggcaagatg gaggcgctga ttttggaacc ttccctgtat actgtcaaag ccatcctgat 120
tctggacaat gatggagatc gactttttgc caagtactat gacgacacct accccagtgt 180
caaggagcaa aaggcctttg agaagaacat tttcaacaag acccatcgga ctgacagtga 240
aattgccctc ttggaaggcc tgacagtggt atacaaaagc agtatagatc tctatttcta 300
tgtgattggc agctcctatg aaaatgagct gatgcttatg gctgttctga actgtctctt 360
cgactcattg agccagatgc tgaggaaaaa tgtagaaaag cgagcactgc tggagaacat 420
ggaggggctg ttcttggctg tggatgaaat tgtagatgga ggggtgatcc tagagagtga 480

tccccagcaa gtggtacacc gggtggcatt aaggggtgaa gatgtccccc ttacggngca 540
aancgtgtct caagtgctgc agtcagccaa agaacagtna agtggtcaca ccttcggtga 600
agactcactg ttcctggctc ctcatcctcc tcaaaaaatt gcatgtccgc tgtgaatttt 660
catccaattc cccaatcgat gctccaaggg tcatctccgg ggatnacaag gattcttaaa 720
tctcantctg tttgtggntg cccctcaacc tcccctanaa ccttccnatc ctttcaatcc 780
tcttcaattc tgggagtaaa gctccaanca tattnagtaa taggcanggg aaacaacctc 840
cttcttttan acnggnttaa gcncaaatg 869

<210> 5
<211> 906
<212> DNA
<213> Homo sapiens

<220>
<221> misc_feature
<222> (1)...(906)
<223> n = a, t, c or g

<220>
<223> nbla-00059-rl

<400> 5
gnngnnnnnn tgtggctttt tttttttttt tcagtttgga aaaaatgttt tattcctctt 60
tgcacagagc agtttatgaa ggtgggtttt ctcctgactc catgcatctt ttacacaaag 120
atgccccctt aaatatgccc agttatctgc cccacctcag tgctggagaa ctggcagtta 180
gtaagtgggg cagaatgctt aagtctcagg aaggttttta aaggcatttt tgtggggagg 240
aagttctggg tcaaggggaa agattagacc caagagtgag tattccattc tccatcttcc 300
tggggaaatc caaaccccaa aggttttatg aagaaaagca cctctctcag cgacctagag 360
acagggagag cacagaccta ctgcttgggt gtaaggctga ggcagagaga gggtaggttg 420
cagcgactgc agacccacgg cagagagtaa atgcatgtcg gggagctgag gggacagaga 480
cagcctanag gccaagtcat aagttccact ccttccccag ttctgagtag aaactttctt 540
cccaagacta naatggagtt ttaagtttan gaactggctt tgcccaagga cacagagaag 600
acaaaccaag caacgattcc acangtagta angggtggac agttaaggta gctaactaag 660
agatgggcac tcgccactgc agttttgaac tatatgncaa atnanggtac agatgcattt 720
tatatgncct ggtcatacaa tttaaattgc gttttccatg ttgtcccttc cctatgaact 780
attccaaaag cctcttccaa ggaaagggan agggnaattn gaaggaatgg ganaaaacac 840
ggaggcacnn aantggggta nggctnaant ggtaaatcct accatcccgn ccaaancgtn 900
tatnaa 906

<210> 6

<211> 906
<212> DNA
<213> Homo sapiens

<220>
<221> misc_feature
<222> (1)...(906)
<223> n = a, t, c or g

<220>
<223> nbla-00072-f

<400> 6
gngnggnnnn nnnnaatctc gancactgtt ggctactggg gtggctagcc tgtcctgaca 60
ggggagagtt aagctcccgt tctccaccgt gccggctggg ccaggtgggc tgagggtgac 120
cgagagacag gacccttgac atctccatct gcacagaggt cctggctgga ccgagcagcc 180
tcctcctcct aggatgacct caccctccag ctctccagtt ttcaggttgg agacattaga 240
tggaggccaa gaagatggct ctgaggcgga cagaggaaag ctggattttg ggagcgggct 300
gcctcccatg gagtcacagt tccagggcga ggaccggaaa ttcgcccctc agataagagt 360
caacctcaac taccgaaagg gaacaggtgc cagtcagccg gatncaaacc gatttgaccg 420
agatcggctc ttcaatgcgg tctcccgggg tgtccccgan gatctggctg gacttccaga 480
gtacctgagc aagaccaaca agtacctcac cgactcggaa tacacagagg gctccacaag 540
taagacgtgc ctgatgaang ctgtgctgaa ccttaaggac ggagtcaatg cctgcattct 600
gccactgctg caaatngaca aggactctgg aatcctcaan ccctggtaaa tgcccaantg 660
cacanatgac tattaccgan ggcacaacgc tctgcacatc gccantgaga agangagtct 720
gcaatgtgtg aagctcctgg tgganaatgg gggcaatgtt caagnccngg cctgcggcgc 780
tcctcaaaaa gggcaangga cttgcnttaa ttcggtgagt ancctcncct ttggcgcttc 840
acaagcatgg gatntggaac tactcctggg aancaaacaa ccgcaacctt gaagcactgn 900
tcccag906

<210> 7
<211> 891
<212> DNA
<213> Homo sapiens

<220>
<221> misc_feature
<222> (1)...(891)
<223> n = a, t, c or g

<220>

<223> nbla-00072-r1

<400> 7
tgtggctttt tttttntnt tttagttagt gaaaatatat atttgccacc agaattcact 60
gggaccccag agccagcaga tgtggttgga aagatcctct gctctgtcct ctggcctcct 120
gctgcatctg ggccatcagt tggactggag gagctggacg ggcacatagt tttcctcaga 180
ggcaccatcc tcatcctcct tgggagggga agccaggaca gggttctcga gagttcgagg 240
gacacctgcc cctgacgggt cctcacacag cgtaggcagc gtctgctccc atgaagccca 300
gttcacctcc tccaccctga agcaccagcg ctcatcgggg ctgccatctg gcttagtgcc 360
aacggtcagc atcacacctg cccgctgctt cttcctgcac caccaatagc cattctccat 420
ctccaggaca gagatggctt tctgcagctt ccagatgctc cagctgtcag tggcgacact 480
gttgacggtc tcgctcatga nggcgatgag catgttgagc aacangatgt aggtgagcag 540
cacgtaggcc aacaagcaac ancaccatgc cgcggaagtg caactgctcc tggaanggca 600
actcgcccat gccgatggtg aatttgaaga actccaagga gnttccagga taccctgtac 660
tgggcccgtt tgccctcntc ctcctgtccc tccatgggct gcactgactc cgttgcantg 720
gggcctgtan gancttcngg gcgccaaacc tcctggntca agctcaacaa gggctacanc 780
gaanccnaaa aggganncta anttnnttan aaggaacnca anaaggtccc caggatnacc 840
ttcggnttat gaaacgtnaa tncctgtntt ctgggaacca ctnntnnatn a 891

<210> 8
<211> 3381
<212> DNA
<213> Homo sapiens

<220>
<223> nbla-00086

<400> 8
   gcctactggt cgacgtgtgg cgtcggctct acccggaatg gagaatatcc aggagaaaag 60
   caaagaaggg atgatcgaca tcaagctggg caaaccccag gagcccccgg ccagcgaggg 120
   cggctgctcc tgctaatgca gagccgacct gtggcttccc atgacactcc ttgcttgttg 180
   tgttgcttcc tattggctag cttcctaagg ggggagggaa ccgagttatc aagatgggag 240
   gatttttctt ttctctctgt ctttaggagt agggtgggat ggggagggag gctgggcatc 300
   agggatcaca tcactcttaa cggctgttac ttaaacaact attttttggt ttggttgtaa 360
   tatattgtac tttattaaga ttgccaaaaa ctgttaaaat ttaaaaaaaa tttaaatcat 420
   gtgtatacaa ttttttgcca gataaaaatg tagtcatttt tatttgaaag atgtgctttt 480
   tgtttttgta tatttgtaaa cttatagaga accttttcca cacacctcct ccttcctgtt 540
   ctctttgaac cgttcatcac ctctgccttc ctcctatccc cagcccaata aattaaaaca 600
   attaactgag caaattaatt aggcttcagt ctggggccat ctggccccac tctctaggcc 660
   ctactccagt taaatcaaac attggttgaa cacatcagcc tctgaaaagg tagctctgac 720
   tcttgtcttt ccatggccag agtgggtcgt caacctcgtg tggagctgag accaaggtct 780
   gtgggctcta ggcagcccgg cagcagcccc cagaggaact gctcccacct ctccttctct 840
   ttccctgaaa agccttgaag ggggttgagg gtacaggcaa ccttgtgttc tctgtgccct 900
   cagggactgg cagagcaaga ggccatcaga aggacgaggt gctgttggca attgcagctg 960
   cgtggtgcgt ggtttataga ctggggtgcc atgtgtactt ctgaaaatat tttaaccagc 1020
   ccacccagca gtcagtacac atgcctgggc caagtctaag aaactcagtg tgtgatgttg 1080
   ggcagtgtgc catcctgaag ccttcctcct ggatgtcagg gaagctggac cacctggagt 1140
   tgatcctctg tgatggggag gcgggggggg gggggtgttg tctgcagaac caccaccttg 1200
   aaagatccat gttcttagag cctcagctgc taatattgtt gagtgctttc tggaaaagct 1260
   gatttgttgc tatgttttca ccccagtggc atggcgcagt tgaagtcttt gtggacatat 1320
   ctttgtggct actgtggctg cgctgtggtc acctgaggat gtggttcagg tggggtctcc 1380
   agaaggctgc ttgcccccag agagccatct cttccatttt tgattcattc ctgagaatgg 1440
   cttagtcctg agaatctgca aagtctctga agagaactct ctcttcccac taagtgatat 1500
   aacctacttg gccagcattc caaatagact cagactcact gccctgagcc tgcacagagc 1560
   caggtgcaat gggcagtgag tctggccaaa atgtaaaccc caccctccct gctgtcattg 1620
   gctctccctc caggctgctg ctgggcatgt catagatggg atgaatgacc atctggggta 1680
   tggtgggctg aactccacaa ggtcgttgga atgaattttg aaacttctca gaaattaatc 1740
   tgaaatggca ggcagtaagt gcagcagaat ctggcaggac tgatatttgt gcagtgggcc 1800
   tgccacacac cctctgccta ggaggctgta tgccagagct catctctagc aaggaattta 1860
   tcttcaggtg aagaagagag gaagcaggat cccactgagc acctcttgca gaaggcaagg 1920
   accaatgttt gaacgtgttt cagatgtcat gtgtttagtt tttatatgtg tgtacataat 1980
   tatatataga gatagacttg tgtatacacc ttccacaaac acatcctcac ggagatacat 2040
   gtgctcaatg tatattcttg tttctcctgc tctcaagttg gaacttgcat tgtgataagt 2100
   tttggaaatt caaaagtact gcaacgtgtt gaaataagaa atacttaata ttgtcaaata 2160
   acttgtgatt gtcttgccat caataaaata atgcatagtt gtgttaaact acaggcaaac 2220
   atagcccacg ggaatatctc caccatttct ctatgtactt tatatgcacg ggtgtgcatg 2280
   cctgtgtggg ccagtgtccc cccaccatgg gcacaccccc aagtggtgct tgttggaaga 2340
   gtgcttactt acctcgagag aatttacagg agtccacatg acagtgtcct gcctggttgg 2400
   cagcgggggc catcacctgt ggcctctgag accgccccat ttacaggaat ctgcactggg 2460
   tctcagtgaa tgaacgtatg gtagattttt attttcttgg cactaaagat tagctttttt 2520
   aaaaaaacat cccataaaac agtagaaact cagttccctt ctcaggggca cacacatcca 2580
   aggcagccag acctcttttt cagaagttgt tggttccctg attttgtctt tgagcaggca 2640
   ccatccctgt ccccttgctt gcaggtagaa ttggcacaga gtgaagtttc catttttcat 2700
   ccattgctgc cctgtgtgtg aacaaatgtg actccatatt tattacacag caaggactgc 2760
   tgtgtgaggg tgtgttcctc agaccatctc tactcttttt ggttaggaca ctaggttctt 2820
   cccagcatat gagtgagaat attggtggta ttttgggctc ctctactgca caagggccag 2880
   tccccaagtc tactctgatt gattgtcatg ggcacccaca cagatcagga caccctttga 2940
   atttttgttt gacatgaact atttaggtgt tccaaaacct tgggatctca atgtgcaaac 3000
   tccttggact ccagacccct gtctggttgg cactcccagc agtgtgtggt ttcatcagga 3060
   aatccttgca gcaggggttt agatggttcc ttcaggaggt gggaagcctc catagcattg 3120
   aaggccagcc tgcatctcgc ggtgtgtgtc cacttctccc ctcactttcc cctcacccat 3180
   ccctttaaag tgattggctg tgaggagcac agagggtggc aattccatct gttatgggct 3240
   cagaagacat agccgccatg ttgatttcac attaaactgt gtaaaatgtg gaaaagactc 3300
   agcatgttgg tcaaacaata aactgtccta attctcaaaa aaaaaaaaaa aaaaaaaaaa 3360
   aaaaaggcca catgtgctcg a 3381

<210> 9
<211> 866
<212> DNA
<213> Homo sapiens

<220>
<221> misc_feature
<222> (1)...(866)
<223> n = a, t, c or g

<220>
<223> nbla-00110-f

<400> 9
   tctcgagcac tgtngggcct actgggctca ttttctcttg ctgccgccat gattctgagg 60
   cctccccagc catgtggaac tgcaatactt taaagacaca aagtctcaac aaccatcttc 120
   cgcttgacga gacagatcat tctaatttga gcagaagcta ctatgtcctg ccctttgaac 180
   gcggcggccc ggacagctga caaggacaca ctgtgtattt ccattccaat tctgggagtg 240
   ctctgaggcc tctgggggag aaggacccat gaaatattca aaacataagt gaataaaata 300
   tctaggtgct agatatgggc caggaagagc cctcggccct gcaaagtgtg tgtgatggtg 360
   agaagctacc ggaagagatg gtccctgtgc ttggttcatt ccttggacat ttatcaagct 420
   gacgaatgta gcagaggtgc ttcagtcggc tgtaattcca cgggtggagt gctggctgga 480
   gagttacctg gggctgtcac actgcatggg ctccgggaca ctgtggctgc ccttatgtgg 540
   tgtccccgga gggccctgca ngtgtcacac cgctgctcca cactgccacc tgctgtcaac 600
   atctgtgcaa cgtatccaag tctctggggg ctaaantgaa aaacatgcat ctcgtaacca 660
   atgaaatccg ggcttgtcct gaanactcct gcantcatcc antccacact gctataaaga 720
   catactaana ctgggcactt catggnaaaa aaaggtttgg ttggtcacgg ntctgaaggc 780
   tttanaggaa ccgtggaact ttcanttntg gggaagcctc aaggaacttt tancaanggn 840
   ggaaggtgaa nggggganaa ggntnt 866

<210> 10
<211> 888
<212> DNA
<213> Homo sapiens

<220>
<221> misc_feature

<222> (1)...(888)
<223> n = a, t, c or g

<220>
<223> nbla-00110-r1

<400> 10
tgaaggnngt tcactgccac acaacatctg catcagagac aaaagtaacg gggctcgagg 60
cgggactggg ctggctctgc ccagtgcacc atataacagc acggacaaaa ctacacaaat 120
cccagctggg tacagtggct cacgcctgtt ttcccagcat tgttcgaggc tgcggtggga 180
ggatcacttg aggtcaggag ttcgagacct gcctggccaa catggtgaaa ccccatctcc 240
actaaaaata caaaaattag cagggcatgg tggcacgtgc ctgtgatccc agctactcgg 300
gaggttgggt gatttataaa gaaaagaggt ctatttggca cacagttctg caggctgtac 360
aagaagcacg gtgccagcat ctgcttctgg tgaggcctca ngaagtgtcc actcatggca 420
gaaggcgatg ggagcaagtg catgcagacc atatggtgag agagggaaca agagagagtg 480
ggcanaggan gtgccaagcg tttttaacaa tcagttcttg tgagaaggaa taaagcatga 540
atgcattcat gaccgcgagg acagcaccaa gctctcatgt cctaagcacc ttctgtcaag 600
tcccacctcc aacattgggg ttcaaatttc aacatgaggn tggaagtggg acctggtggg 660
aaaggattgg atcatggtgg tggtccctca caagtgggtt ancgccaact tcttggngct 720
gttcncatga taatgagtta ccgttaaatn tgggtgttca aaatgtcggg accccctcct 780
tttctccccn cnacnntccc atttaaaacn cctttccgcc ttcaacttcc aacatgacta 840
taatttcctg aggcctccca aaaattgaaa ctgcannctc cttaaanc888

<210> 11
<211> 850
<212> DNA
<213> Homo sapiens

<220>
<221> misc_feature
<222> (1)...(850)
<223> n = a, t, c or g

<220>
<223> nbla-00132-f

<400> 11
gnngnnnnnn nnnnnnnnnn ggattctcgt ngcactgtgn cgcctactgg taagatacca 60
aggaggagtg agagcgtccc tgccttcgag gagcagacag ggcaccgcga ggcgtttgcc 120
tcagcatgga gggcggggcc gcggcagcca cccccacagc actgccttac tacgtggcct 180
tctcccagct gctgggcctg accttggtgg ccatgaccgg cgcgtggctc gggctgtacc 240

gaggcggcat tgcctgggag agcgacctgc agttcaacgc gcaccccctc tgcatggtca 300
taggcctgat cttcctgcag ggaaatgccc tgctggttta ccgtgtcttc aggaacgaag 360
ctaaacgcac caccaaggtc ctgcacgggc tgctgcacat ctttgcgctc gtcatcgccc 420
tggttggtga gttcccgggc gcggccttcc ccgccacctg cctgcctctt caggcttggt 480
ggcggtgttc gactaccaca ggaagaaggg ctacgctgac ctgtacagcc tacacagctg 540
gtgcgggatt cttgtctttg tcctgtactt tgtgcaattg gctggtgggc ttcagcttcn 600
tcctgttccc cggagcttca ntctccctgc ggagccgcta ccgcccacan cacatcttcn 660
ttggtgctac atcntcctcc nttccgttgg caacgccctg ctgggcctga aggaggaatg 720
ctgttcaacc tcnggggcaa tntancncan ttnancccca nggtgtcctg gccaaagtnc 780
tgggcctgct gctggcctgc ttcngtgggg nggtgcncna aancttgacc cgggccnnct 840
ggaancggcn 850

<210> 12
<211> 860
<212> DNA
<213> Homo sapiens

<220>
<221> misc_feature
<222> (1)...(860)
<223> n = a, t, c or g

<220>
<223> nbla-00132-r1

<400> 12
ggtgnnnnnn tgtgggccgt ttttttgcct tttttttttt tcngctgtag acatttattg 60
aatcatctgt tgcaccaaca gcacattgta tttcacagaa gattggccac aggcaaaata 120
aaataaccca ccacaaatcc gcttctgggg gccttcttca gctccttagt catccagtgt 180
tcactttggc aaacagacca gtttacagac ttttgttgca aatctaaggt tccatttctc 240
tctgtagcat tccggctaga gagagacacg attgtacttt ggtagtttgt ctaaggcgga 300
gttttcccac atgtaaacat ttcactatac aagtgtgtgc cagctgattg aagaactgac 360
ggnaccccta cccctgaaga gtgtacagtc taaacaggag gcgaacactg tcatccggac 420
aggctgagag gagcagcctg ggaggaggcc cgcctgcatc tgccaagaga aagtctgtct 480
gaagtcatgg gcttctatcc cctctcccaa caaagccatc ctaaggctaa agggttaaag 540
aagangaaag tcangactac agctgggcca cagcctcatc tcaaaagctt cccaaggggc 600
aatccctccc ctctcctggg gaancttgct ttcaccanca acatacttta cacatcatat 660
cacttgctcc tctggcaaaa nggcgggncc aatttgtgcc acgtgttant caagctggag 720
ggacnccntg nttgantcaa caaccatcgt gggctttcaa gcctgtccct gagggccccc 780
aatttccctg gncttgncca agcatctgnt gncaanaagg gtggggcctc cccncngctt 840
cttaaanatt ggngnccgcg 860

<210> 13
<211> 3730
<212> DNA
<213> Homo sapiens

<220>
<223> nbla-00170

<400> 13
cggaagtgtt acttctgctc taaaagctgc ggaattcctc gagcactgtt ggcctactgg 60
gagacctcta gaaaaagagg ctgtaggcca acttaagcct acaggaaaag aggacacaca 120
gacattgcaa tccctgcaaa aggagaatca agaactaatg aaatctcttg aaggtaatct 180
agagacattt ttatttccag gaacggaaaa tcaagaatta gtaagttctc tgcaagagaa 240
cttagagtca ttgacagctc tggaaaagga gaatcaagag ccactgagat ctccagaagt 300
aggggatgag gaggcactga gacctctgac aaaggagaat caggaacccc tgaggtctct 360
tgaagatgag aacaaagagg cctttagatc tctagaaaaa gagaaccagg agccactgaa 420
gactctagaa gaagaggacc agagtattgt gagacctcta gaaacagaga atcacaaatc 480
actgaggtct ttagaagaac aggaccaaga gacattgaga actcttgaaa aagagactca 540
acagcgacgg aggtctctag gggaacagga tcagatgaca ttaagacccc cagaaaaagt 600
ggatctagaa ccactgaagt ctcttgacca ggagatagct agacctcttg aaaatgagaa 660
tcaagagttc ttaaagtcac tcaaagaaga gagcatagag gcagtaaaat ctttagaaac 720
agagatccta gaatcactga agtctgcggg acaagagaac ctggaaacac tgaaatctcc 780
agaaactcaa gcaccactgt ggactccaga agaaataaat caggggggcaa tgaatcctct 840
agaaaaggaa attcaagaac cactggagtc tgtggaagtg aaccaagaga cattcagact 900
cctggaagag gagaatcagg aatcattgag atctctggga gcatggaacc tggagaattt 960
gagatctcca gaggaggtag acaaggaaag tcaaaggaat ctggaagagg aagagaacct 1020
gggaaaggga gagtaccaag agtcactgag gtctctggag gaggagggac aggagctgcc 1080
gcagtctgca gatgtgcaga ggtgggaaga tacggtggag aaggaccagg aactggctca 1140
ggaaagcccc cctgggatgg ctggagtgga aaatgaggat gaggcagagc tgaatctgag 1200
ggagcaggat ggcttcactg ggaaggagga ggtggtagag cagggagagc tgaatgccac 1260
agaggaggtc tggatcccag gcgaggggca cccagagagc cctgagccca aagagcagag 1320
aggcctggtt gagggagcca gtgtgaaggg aggggctgag ggcctccagg accctgaagg 1380
gcaatcacaa caggtggggg ccccaggcct ccaggctccc caggggctgc cagaggcgat 1440
agagcccctg gtggaagatg atgtggcccc agggggtgac caagcctccc cagaggtcat 1500
gttggggtca gagcctgcca tgggtgagtc tgctgcggga gctgagccag gcccggggca 1560
gggggtggga gggctggggg acccaggcca tctgaccagg gaagaggtga tggaaccacc 1620
cctggaagag gagagtttgg aggcaaagag ggttcagggc ttggaagggc ctagaaagga 1680
cctagaggag gcaggtggtc tggggacaga gttctccgag ctgcctggga agagcagaga 1740
cccttgggag cctcccaggg agggtaggga ggagtcagag gctgaggccc ccaggggagc 1800
agaggaggcg ttccctgctg agaccctggg ccacactgga agtgatgccc cttcaccttg 1860

gcctctgggg tcagaggaag ctgaggagga tgtaccacca gtgctggtct cccccagccc 1920
aacgtacacc ccgatcctgg aagatgcccc tgggcctcag cctcaggctg aagggagtca 1980
ggaggctagc tgggggtgc aggggagggc tgaagccctg ggaaagtag agagcgagca 2040
ggaggagttg ggttctgggg agatccccga gggcccccag gaggaagggg aggagagcag 2100
agaagagagc gaggaggatg agctcgggga gacccttcca gactccactc ccctgggctt 2160
ctacctcagg tcccccacct cccccaggtg ggaccccact ggagagcaga ggccaccccc 2220
tcaaggggag actggaaagg agggctggga tcctgctgtc ctggcttccg agggccttga 2280
ggccccaccc tcagaaaagg aggaggggga ggagggagaa gaggagtgtg gccgtgactc 2340
tgacctgtca gaagaatttg aggacctggg gactgaggca ccttttcttc ctggggtccc 2400
tggggaggtg gcagaacctc tgggccaggt gccccagctg ctactggatc ctgcagcctg 2460
ggatcgagat ggggagtccg atgggtttgc agatgaggaa gaaagtgggg aggagggaga 2520
ggaggatcag gaggagggga gggagccagg ggctgggcgg tgggggccag ggtcttctgt 2580
tggcagcctc caggccctga gtagctccca gagaggggaa ttcctggagt ctgattctgt 2640
gagtgtcagt gtcccctggg atgacaactt gaggggtgca gtggctggtg cccccaagac 2700
tgccctggaa acggagtccc aggacagtgc tgagccttct ggctcagagg aagagtctga 2760
ccctgtttcc ttggagaggg aggacaaagt ccctggccct ctagagatcc ccagtgggat 2820
ggaggatgca ggcccagggg cagacatcat tggtgttaat ggccagggtc ccaacatgga 2880
ggggaagtca cagcatgtga atgggggagt gatgaacggg ctggagcagt ctgaggaagt 2940
ggggcaagga atgccgctag tctctgaggg agaccgaggg agcccctttc aggaggagga 3000
ggggagtgct ctgaagacct cttgggcagg ggctcctgtt cacctgggcc agggtcagtt 3060
cctgaagttc actcagaggg aaggagatag agagtcctgg tcctcagggg aggactagga 3120
aaagaccatc tgcccggcac tggggactta ggggtgcggg gaggggaagg acgcctccaa 3180
gcccgctccc tgctcaggag cagcactctt aacttacgat ctcttgacat atggtttctg 3240
gctgagaggc ctggcccgct aaggtgaaaa ggggtgtggc aaaggagcct actccaagaa 3300
tggaggctgt aggaatataa cctcccaccc tgcaaaggga atctcttgcc tgctccatct 3360
cataggctaa gtcagctgaa tcccgatagt actaggtccc cttccctccg catcccgtca 3420
gctggaaaag gcctgtggcc cagaggcttc tccaaaggga gggtgacatg ctggcttttg 3480
tgcccaagct caccagccct gcgccacctc actgcagtag tgcaccatct cactgcagta 3540
gcacgccctc ctgggccgtc tggcctgtgg ctaatggagg tgacggcact cccatgtgct 3600
gactcccccc atccctgcca cgctgtggcc ctgcctggct agtccctgcc tgaataaagt 3660
aatgcctccg cttcaaaaaa aaaaaaaaaa aaaaaaaaaa aaaaaaaggc cacatgtgct 3720
cgagctgcag 3730

<210> 14
<211> 899
<212> DNA
<213> Homo sapiens

<220>
<221> misc_feature
<222> (1)...(899)

<223> n = a, t, c or g

<220>
<223> nbla-00185-f

<400> 14
ggnngnnnnn nnngnnnnnt ttnnnngggc ccnnaatcct cgngnacgtg gccctactgg 60
gcacaaatgt acaatactgt ttgggaatat gggaagacct gggatttaga atatgccaag 120
acagatataa attgtggcac agacttgatg ttttatatag aaatggaccc accagcactg 180
cctcctaaac caccaaaacc tactactgta gccaacaacg gtatgaataa caatatgtcc 240
ttacaagatg ctgaatggta ctggggagat atctcgaggg aagaagtgaa tgaaaaactt 300
cgagatacag cagacgggac ctttttggta cgagatgcgt ctactaaaat gcatggtgat 360
tatactctta cactaaggaa agggggaaat aacaaattaa tcaaaatatt tcatcgagat 420
gggaaatatg gcttctctga cccattaacc ttcagttctg tggttgaatt aataaaccac 480
taccggaatg aatctctagc tcagtataat cccaaattgg atgtgaaatt actttatcca 540
gtatccaaat accaacagga tcaagttgtc aaagaagata atattgaagc tgtagggaaa 600
aaattacatg aatataacac tcagtttcaa gaaaaaagtc gagaatatga tagattatat 660
gaagaatata cccgcacatc ccaggaaatc caaatgaaaa ggncaactat tgaagcatta 720
atgaaaccat aaaaatattt gaagaacaat gccagaccca agagcggtac aacaaagaat 780
cattgaaaag tttaacgtga aggcaatgag aaaagaattc aaaggattat cctaattatg 840
ataantttaa ntcccnaatc cattgaaatt attgccattt naanaanatt ggganaaga 899

<210> 15
<211> 887
<212> DNA
<213> Homo sapiens

<220>
<221> misc_feature
<222> (1)...(887)
<223> n = a, t, c or g

<220>
<223> nbla-00185-r1

<400> 15
nggggttttg tnggctnttc ctcnnttgtn ctcgttttgg ttttngggcc tnggggcccn 60
tnttttgttt aaaaatgctc gntntntttt tttttgtntn ttttntngtt ttgtggtccc 120
cncttntntt gttntnttan tttttttntt gtcntnttnt ttttttttnt tttttttttt 180
ttnttgtttn gtgngtttgg ggggttttg ggggtttttt tttttnttt tntntttnta 240
ttttgtnttt ttnttttgtt tttttnttt ttcttttgtt ttttntttgt nttttttttt 300

ttngttttnt nttttttttt ctgtttttc tttntgnngn gtttgttntt ntttttntct 360
tttttntttn gttgtgtgtc tttttttttt gtttatttnt tttttttttt tgtttttttt 420
ttnttttntt tttttttttt ttgattttgt tttttnttat tttgttgttt tttttttttt 480
gttntttttt ttttctgtnn nttntttttt ttttctgntt ttctcttttt ttgtnttgtt 540
ttgttntttt ttttgttttt tntttttttt nttttttttt ntttnntntc tttttttttn 600
tntnttcttt tnttttttc tnttntnttg tttttgtttt tntttttgtt cttgttttgt 660
tttttntttt ttgttgtttt tttntnnttt ntctnntntt ttttnttctg tgttggtttn 720
tttttgttn gtnatttctt ntttgtntnt nttttnttnt ttnntttgtn tttnctttttt 780
tttttttatg tttntcggtn ttntttcttt tttcttttg tntgtttttt tnntgtcgtt 840
cttttttttt ttttgtnttt cgtttttttc tttcgattnt ctttttt 887

<210> 16
<211> 864
<212> DNA
<213> Homo sapiens

<220>
<221> misc_feature
<222> (1)...(864)
<223> n = a, t, c or g

<220>
<223> nbla-00226-f

<400> 16
   ngagttnnnn ngggcccnng ggntctcttn cacggggccc ctactgggga aagnaacttt 60
   acagggtcgc tagctagtag gagggcttta tggagcagaa aaacgacaaa gcgagaaaaa 120
   ttattttcca ctccagaaat taatgatcat gagctcgttt ttgatggact ctaactacat 180
   cgatccgaaa tttcctccat gcgaagaata ttcgcaaaat agctacatcc ctgaacacag 240
   tccggaatat tacggccgga ccagggaatc gggattccag catcaccacc aggagctgta 300
   cccaccaccg cctccgcgcc ctagctaccc tgagcgccag tatagctgca ccagtctcca 360
   gggacccggc aattcgcgag gccacgggcc ggcccaggcg ggccaccacc accccgagaa 420
   atcacagtcg ctctgcgagc cggcgcctct ctcaggcgcc tccgcctccc cgtccccagc 480
   cccgccagcc tgcagccaag ccagcccccg accatccctc caagcgccgc caagcaaagc 540
   aacccatagt ctacccatgg gatgaaaaaa attcacgtta gcacggtgaa ccccaattat 600
   aacggngggg aaaccaancg ctcgaggaca agctatancc ggcaacaagt cctggaatta 660
   gngaaagagt tcaattnaan cgctacctga cccgaaggag aanggtngan attgccaatc 720
   gctgtgcctc tctgaaaagn aagntnaaaa tccgggtcca aaancgttnc atgaatggan 780
   naagggcaac ggattcccaa aacaaannca ggtcagaaac cccggcnggg gngggcccaa 840
   nnccttttan cantgagttt cttn864

<210> 17
<211> 872
<212> DNA
<213> Homo sapiens

<220>
<221> misc_feature
<222> (1)...(872)
<223> n = a, t, c or g

<220>
<223> nbla-00226-r1

<400> 17
   gantgnnnnt gttngngttt ttttggcccn nggntttttt ttcctcaggc ncccctgaaa 60
   ttcanccaag ccagnccatc acaccttgca atatataatt tttgcagctt tttttcttaa 120
   aaaataaata accccccaaa atggcctttt aaaaaaaaaa acccagcagg taccatgcta 180
   agacaacatc acatgcttaa aagggtcctg ggcgcagcgc cggccggggg tgctgacctg 240
   actttggtgt tggggagtcg gtggtccttc ttccatttca tgcgacggtt ttggaaccag 300
   attttgatct gcctctcaga gaggcacagc gagtgggcga tctcgatcct tctccttcgg 360
   gtcaggtagc ggttgtaatg aaactctttc tctaattcca ggacttgctg ccgggtatag 420
   gctgtcctcg agcgcttggg ttcccctccg ttataattgg ggttcaccgt gctaacgtga 480
   atttttttca tccatgggta gactatgggt tgcttgctgg cggcgctgga gggatggtcg 540
   ggggctggct ggctgcaagc tggcggggct ggggacgggg aagcggangc gcctgaaaaa 600
   agcgccggct cccaaaacga ctgtgaattt cccgggtggt ggtggccgcc tgggccggnc 660
   cgttgnctcn ccnaantgcc cgggtccctg gaaaactggt gcaactatac nggngctcaa 720
   ggtaaccaan ggccccgaag gcngttgtgg gtanaactcc cngnnggnat cctggaatcc 780
   caattcctgg tncggccgta anattccgga cngtnttaaa ggaatntnnc caattttgcg 840
   aanatcctcc cannggngga aattncggtt tn 872

<210> 18
<211> 928
<212> DNA
<213> Homo sapiens
<220>
<221> misc_feature
<222> (1)...(928)
<223> n = a, t, c or g

<220>

<223> nbla-00258-f

<400> 18
ggnngnnnnt ggnggccnnt tttnnnnggg cccnnngatc ctcgntaacg gtngccctac 60
tggactcgca cgttaggagg ctcgggtcgt tgtggtgcgc tgtcttcccg cttgcgtcag 120
ggacctgccc gactcagtgg aagcggggag aggtggtgag agatgacccc tgttgaggat 180
ttcattggtg tctgccacag gccgccatgg catcagatga aggcaaactt tttgttggag 240
ggctgagttt tgacaccaat gagcagtcgc tggagcaggt cttctcaaag tacggacaga 300
tctctgaagt ggtggttgtg aaagacaggg agacccagag atctcgggga tttgggtttg 360
tcacctttga gaacattgac gacgctaagg atgccatgat ggccatgaat gggaagtctg 420
tagatggacg gcagatccga gtagaccagg caagcaagtc gtcagacaac cgatcccgtg 480
ggtaccgtgg tggctctgcc gggggccggg gcttcttccg tgggggccga ngacggggcc 540
gtgggttctc taaaaganga ngggaccgan gctatggggg gaancggttc gagtccaaga 600
atgggggcta cggaggctcc anagactact atancaaccg gagtcaaaat ggtggctaca 660
attaccggaa ctccggcggg tcctacanag acaattacga caattaccta cacacaacga 720
gttaaaancc ntcctgctca anattgtccn tccaatggnt gtgtgttaaa aattgttgga 780
acttccctga acnttaatgt ttnataaatg cacctccctt gtantcccaa ntttcnaanc 840
aattcggttc natcttgtaa aaccaacctg ancgctttta ncccggaatg gccccttaca 900
aaactttcnt ttaaggaatg cgtttttt 928

<210> 19
<211> 931
<212> DNA
<213> Homo sapiens

<220>
<221> misc_feature
<222> (1)...(931)
<223> n = a, t, c or g

<220>
<223> nbla-00258-r1

<400> 19
ggnngnnnnt ggnggcnttt tttttgggcc tcagttcacg nttactgtng gcccgagctt 60
cgtaaaataa agaaaactgc gtacaagaat tcctcagcca ctgcaaatac aacatcgatg 120
gagtatgcgt acgtcttcca gtaaaaaagg acaatataaa taaacatctt caagaacctc 180
tcagggacgt ctgcacgtct actgtggggc gcacagcaca agatggacgc aggggctggg 240
gctccagggc agttgctgcc acacccgccg gggtcctacc acactacact caagaaacgt 300
cacacggagt tggaaaaaag ccacagcttg ctcagcttcc gacaaagagc ggattcacaa 360
gtccgttcga tntcagagaa gtgagtgggg caaccacttc atggggccaa ccccactaaa 420

aaaaaaaagg caacgcaacc aaaaactaaa acaaaaacca aaaaccacac aacccccgat 480
tttttgaaa ctcatggctc gtggtcaaaa aagtaaatgc ttttcaaaat gttttgaaaa 540
ccctcaaaaa aaaacagcac ttccttaaaa agaaaatcta gtaacgaggc atcccggcgt 600
caaaagcggt caagctgggt ttgcaagatn agaaccgaaa tgactacgaa aagtgggaat 660
acaaggaggt gcattactac acattaacgt tcaacgaaac tccacaatcc ttaaacacac 720
aaccanttgg aaggacattc tnaacaagga agggttttac ncnttgtgtg tancgtaact 780
gtctaactgt ctctgtngga nccgcccaac tcccggtcac tgtnncaaca atctaatccn 840
ggtggcaaaa taatccctgg agcctccgtn acccccaatc ctggantcca accggnttcc 900
ccaaaacctc ggtccctcct cctctaaaaa a 931

<210> 20
<211> 880
<212> DNA
<213> Homo sapiens

<220>
<221> misc_feature
<222> (1)...(880)
<223> n = a, t, c or g

<220>
<223> nbla-00312-f

<400> 20
   gnnnnttnnn nnggccgnat cccgagcacg ttggcctact ggattagaaa ccacaatctg 60
   ccaaagcaac ctccacttgc cctagcaatg ggacaggcca cacaatgtct aggactgatg 120
   gctcgatcag gaattggatc ctgcgcattt gccacagtgg aagactacct acattagcta 180
   tgcatttcaa gagctcacac ttatattgtg gcatatagtc aacatggaag tagaccagct 240
   ctgctgattt gaaatttaga ttttttaaat tatgtactgg ggacaggttt ttgtcgcttt 300
   acattgcttc ctaatttaca gcatgatgca aatgattttc taacttaagt gttaggagaa 360
   attattttcc atctttaacc tcctaagtgt ctaagagtta aatattactg aatttcagac 420
   gttcaaattg atcatcacaa atcctttaaa acaattacct aaaagaaacc aaaaatcctg 480
   ccttccttgt gggggaaggg ggagagaggg gaanggaatg gaacaagttg tgtttgtgtt 540
   aacatgtggg tgatgtaaac ttcaaattgg gagatgttcc gacccccact cccatataag 600
   cattcaatca gtgtaacttg caaaatgcat aaacatcccg acagtttgaa tttatagtgt 660
   tgatggaaga aatcantttt aatgtgtact gtaaacttga aatactcang gagctgagtg 720
   atangttggt gcaacttaca atcccganct ggtaatccaa tantttgggt ttaacaatgg 780
   ccttttcaac nttggttccc ggttaactac agatgacttc cggtggnnac ncncaagttt 840
   aacnggtggn ttanacaatt ttncattcaa cggggnggtn 880

<210> 21
<211> 887
<212> DNA
<213> Homo sapiens

<220>
<221> misc_feature
<222> (1)...(887)
<223> n = a, t, c or g

<220>
<223> nbla-00312-r1

<400> 21
ggnggctttt tttttnggct tggttaatta tatcacnaca tttnattgca atatagtact 60
catttaagca cttaaaaatg gaaggtgtac aaagattaaa ttaagacacg gtaaattgac 120
taaatatttg gtttttatat aaataaaggt cataaccaca ccgttgacat gtaatactgt 180
tataatacaa cagttaaact tgtgagtcta caacagaagt catctgtagt taaacaggaa 240
acaaagttga aaaagaccat gttaaaacaa aactactggg actaacaggt cgggattgta 300
agtagcaaca aacatattca ctcagctcct gagtatttca agttttacag tacacattaa 360
aaatgatttc ttccatcaac actataaatt caaactgtcg gatgtttatg cattttgcaa 420
gttacactga ttgaatgctt atatgggagt gggggtcggg acatctccca atttgaagtt 480
tacatcaccc acatgctaac acaaacacaa cttgttccat ttccttcccc tctctccccc 540
ctcccccaca aagaaggnag gattttgggt tcctttaagt aattggttta aagggnttgt 600
gatgatcaan ttgaacgtct gaaattcagt aatatttaac tcctaagcaa ctaaagaggg 660
ttaagatngg aaataaattt ccccaacact aagttngnaa atcaattgca catgctgtaa 720
ctanggaaca atgttaagcg acaaaaacct gtcccaannc ataatttaaa aatccaaatt 780
tcaaatcaac aaaactnggc cacttccatg ttgactaaat gccacatana agtgtggctc 840
ctgaaatgca aaacctaang tnggtagnct ccaccngggg naaatgn 887

<210> 22
<211> 1740
<212> DNA
<213> Homo sapiens

<220>
<221> misc_feature
<222> (1)...(1740)
<223> n = a, t, c or g

<220>

<223> nbla-00315

<400> 22
gaattcctcg agcactgttg gcctactggt tacaagatcg agaatggtta acagaacttt 60
ttcaacaaag caaggtcaat atgcagaaaa tgctcccaga aattgatcag aataaggacc 120
gcatgttgga gattttggaa ggaaagggac tgagtttctt attcccactc ctcaaattgg 180
agaaggaact gttgaagcaa ataaagttgg atccatcccc tcaaaccata tataaatgga 240
ttaaagataa catctctccc aaacttcatg tagataaagg atttgtgaac atcttaatga 300
ctagcttctt acagtacatt tctagtgaag taaacccccc cagcgatgaa acagactcat 360
cctctgctcc ttccaaagaa cagttagagc aggaaaaaca actactacta tctttcaagc 420
cagtaatgca gaaatttctt catgatcacg ttgatctaca agtcagtgcc ctgtatgctc 480
tccaggtgca ctgctataac agcaacttcc caaaaggcat gttactcgct tttttgtgca 540
cttctatgac atggaaatta ttgaagaaga agctttcttg gcttggaaag aagatataac 600
ccaagagttt ccgggaaaag gcaaggcttt gttccaggtg aatcagtggc taacctggtt 660
agaaactgct gaagaagaag aatcagagga agaagctgac taaagaacca gccaaagcct 720
taaattgtgc aaaacatact gttgctatga tgtaactgca tttgacctaa ccactgcgaa 780
aattcattcc gctgtaatgt tttcacaata tttaaagcag aagcactttt tttcactgca 840
ttctagttgt ggtttgtcca aactcatcaa tgtatcttat catgtctgga tccccgggta 900
ccgagctcga attaattcct cttccgcttc ctcgctcact gactcgctgc gctcgtcgtt 960
cggctgcggc gagcggtatc agctcactca aaggcggtaa tacggttatc cacagaatca 1020
gggggtcagt taggatttcc ttctgcataa ggtttttttg tagtgtaatg tcttaatcat 1080
agtctaccat caaatatttt aggagtatct ttaatgttta gatagtatat tagcagcatg 1140
caataattac atcataagtt ctcaagcaga ggcagtctat tgcaaggacc ttctttgctg 1200
ccagttatca taggctgttt taagttagaa aactgaatag caacactgaa tactgtagaa 1260
atgcactttg ctcagtaata cttgagttgt tgcaatattt gattatccat ttggttgtta 1320
cagaaaaatt cttaactgta attgatggtt gttgccgtaa tagtatattg cctgtatttc 1380
tacctctagt aatgggcttt atgtgctaga ttttaatatc cttgagcctg ggcaagtgca 1440
caagtctttt taaaagaaac atggtttact tgcacaaaac tgatcagttt tgagagatcg 1500
ttaatgccct tgaagtggtt tttgtgggtg tgaaacaaat ggtgagaatt tgaattgctc 1560
cctcctatta tagtattgaa attaagtcta cttaatttat caagtcatgt tcatgccctg 1620
attttatata cttgtatcta tcaataaaca ttgtgatact tgaaaaaaaa aaaaaaaaaa 1680
aaaaaaaaaa aaaaaaaaag gccacatgtg ctcgagctgc aggtcgcggc cgctagacta 1740

<210> 23
<211> 843
<212> DNA
<213> Homo sapiens

<220>
<221> misc_feature
<222> (1)...(843)

<223> n = a, t, c or g

<220>
<223> nbla-00417-f

<400> 23
gngnntttnn nngggccngg atctcgtngc acggnggccc tactgggatt tcaagcacag 60
cacatctctc actcaggatg aaagcactct taccgaagtg aaatcctacc attgtaatga 120
ctgtggggaa gactttagtc acattacaga ctttactgac catcagagga tccatactgc 180
agagaacccc tatgattgtg agcaggcttt tagtcagcaa gctatttctc atcctggaga 240
gaaaccctat caatgtaatg tatgtgggaa agctttcaaa aggagtacaa gtttcataga 300
gcatcacaga attcatactg gagagaaacc ctatgaatgt aatgagtgtg gagaagcctt 360
tagtcgacgc tcatcgctta ctcaacatga gagaacccac actggagaga aaccctatga 420
atgtattgac tgtgggaaag cctttagtca aagttcatct ctcattcagc atgagagaac 480
tcatactgga gagaagccct atgaatgtaa tgaatgtggg agagccttcc gaaaaaaaac 540
caacctgcat gatcatcaag agaattcata ctggngaaaa accctaatcc tgtaagggat 600
gtgggaaaaa cttcaaccga agttcaactc ttactaaaca ccaagagaat tcatactcga 660
aataaactcc taggaacgtt gaaattangg antttgcana tgcnttanct aaaatgttcc 720
ngatcanggt nagagggntc ttaanacctt ggaatgtnan gaattactgg ggggttaaac 780
nttgngtnng ggnaaaantg caattnacaa attttttttt ttttacanna ngnccatccc 840
nat 843

<210> 24
<211> 836
<212> DNA
<213> Homo sapiens

<220>
<221> misc_feature
<222> (1)...(836)
<223> n = a, t, c or g

<220>
<223> nbla-00417-r1

<400> 24
gggntgnnnn nnntgtggct tttttttggg cctttaaaga gatggngttt naccatgttg 60
tccaaggctg ggtcttgaac tcctgggttc aagcagtcta tctgccttag ccacccaaag 120
tgctgggatt acaggtgtga gacaccatac ctagccaagt taattttttt aatggtgaaa 180
tcttttcttt gcacataaaa tgagccagtg catgttgctt ctctgagtac aagacaaaat 240
ttatggcaat gggcaattag acttatactt ttctgcaaga aaattaacgg gaaaattctc 300

ctcttagttt tctgttgttt tcccattgat ctgatactgt aggcttaaga aagtgctttt 360
tcatgggcat gccataaaaa gtacaataag gggacttaat agttctgtga aactggcata 420
tgttagctga aagtataatt gtaactggga aaaggggaaa aaagtcacta agtagttcaa 480
ccatctacag tttctgttaa attgtggttt gtaaagcctc caagaagttg ctttaaataa 540
tttgtgataa atttgcatac attttgctcc cacttatacc tttaagaant cncaaaagtg 600
tccaanccat aaggggccan taaatgtttg nggtanccga tacatctaaa attatttaaa 660
gcctctgatc caaaaataac ctttcacngg aaaggcaagg ggcccaaatc aagnaaccgc 720
attttaacca ncttanccta aaggcggnnc aacnggngna acanttaaac cttggnttna 780
ancctcaacg gtnaannaat ggttggcaat tntcaatcct ttaaggggnc tcccng 836

<210> 25
<211> 844
<212> DNA
<213> Homo sapiens

<220>
<221> misc_feature
<222> (1)...(844)
<223> n = a, t, c or g

<220>
<223> nbla-00428-f

<400> 25
gnnnntttnn nnnggccngg atcctcntnc acggggcccc tactgggcat tcnatttctt 60
ttggnttgaa gctaaagaga tgtgtagctc acaggggcta ccttaccagt ataaagagct 120
gataacagta ctcagaagaa tctgtgaaca aatacttgaa aatgggttca atgtagactg 180
ccattatgtg tggtcttccc attaaatgtg aacattttaa tatgtatgca ttcaccttgc 240
ctcttgcaca aatgtcaaaa aaaagatggt aatatctcaa agaaatgaac ttgtagatta 300
ccaagcagtt tgctaaaaat tcaatctttg acccaagctg tagcattttt ttttcatgtg 360
tggcatcttt ttcatgccac caacaaactt gttgtgtgtg tgcgtgtgtg tgtgtgtgtg 420
tgtgtgtgtg tgtgtgtgtt ctgtacccac taggatttgt ttaggtgccc attgcatctt 480
tttgtgctat ggagttgttt acattaagca tgaccgaacg agagacaata ctatttccca 540
caggagtcca ttgggttcag ctttgaaaga ggaatagaat cgaggctcct ttgaccatca 600
aaatgatgaa ctttactatg tggtanccaa tgccagaatg tnagagttgc aagtgatttt 660
gtgctgctat cattaaaact tgtattccaa tcctgccaac ntaaggngat naagatatta 720
anangnancc ctgatttaat ttccagtatt cagtagtaaa atttcctgtc cactggtgan 780
caaancctgn tcacncaatt aacctgggca cacnaanaag ggtttatttg atggggtccn 840
ttnc844

<210> 26

<211> 842

<212> DNA

<213> Homo sapiens

<220>

<221> misc_feature

<222> (1)...(842)

<223> n = a, t, c or g

<220>

<223> nbla-00428-r1

<400> 26
   ggntgnnnnn ntgtggcttt tttttgcctt gtttgtttgt ttgtttntgt ttcttacctt 60
   gggggtaggg aggagttaaa ggaggagaaa ttttactatt ggggggggga aacgaacaca 120
   atcaaaataa aaccttatta gtgtgtccaa ggttaaatag agtgactcag gctttgattc 180
   acagtggaca ggaaaatttt actactgaat actggaaaat aaatcaagga tacctcttaa 240
   tatcttgatc tccttaagct ggcaagactg gaatacaagt tttaatgaat agcagcacaa 300
   aatcacttgc aactcttaca ttctggcatt gggtaccaca taagtaaagt tcatcatttt 360
   gatggtcaaa ggagcctcga ttctattcct ctttcaaagc tgaacccaat ggactcctgt 420
   gggaaatagt attgtctctc gttcggtcat gcttaatgta aacaactcca tagcacaaaa 480
   agatgcaatg ggcacctaaa caaatcctan tgggtacaga acacacacac acacacacac 540
   acacacacac acacacgcac acacacaaca agtttgttng gggcatgaaa aaagatgcca 600
   cacatgaaaa aaaaatgcta caacttgggt caaagattga atttttagca aactgcttgg 660
   aaaccacaag ntcattcctt gagatattac aacctttttt gcanttgtca aaaaggaaag 720
   gtaatgnntc aatattaaat gtccaattta tgggaanncc cccttanggg atccaaattg 780
   acccantttc aataattgtc aaaaatcctc cgaatacggt tacaaaccct ttaacnggga 840
   ag 842

<210> 27

<211> 879

<212> DNA

<213> Homo sapiens

<220>

<221> misc_feature

<222> (1)...(879)

<223> n = a, t, c or g

<220>

<223> nbla-00434-f

<400> 27
gngnnnnnnn nnnngaggnn nttnnnnngg cccnggatcc tcgnncacgg tggccctact 60
ggtatatact gggccagtaa aatgggcctc ccaattgctg tttcagcagg ttttaaacct 120
tcaggaacac cagttaggaa aatagctcca gaaaatatag atatatttta tttttattaa 180
aatggcagtc tacatcataa ttggcatttc tcaagactgt ctttaccaga atctgtgtga 240
aataaggcaa tctagtctcc ttgaaaaaaa aatctcttgg atgtttagga aggaagactt 300
ggccgtgatg tggtgtcctg gctttgtggt gtagtgctgt gtgtatggag ttagtgtaaa 360
aacatggatt acaccaagtg gaagaaacgt cttcttgcca agctcattct tagaacttac 420
acatctagaa cagcttccac tttggcagtg aggtcgtagc cttttangtg gaagaagtga 480
gggtgcaacg tgtcagacac aacattcatg ttactcttac attgggatct gaaggtagtt 540
cagacttcgt gggttttgtt tttaagcaaa acaatgtgaa aacatttaag tttgaaatgt 600
tgcatttgaa gttatgatca tttaatatat tcatattacc aagactatta tactggaagt 660
ggntttgtg ttataaaggg ttaattttac ataaggcagt tacntaangt gatttttaac 720
ccntaaaaaa gtggggatgt ntacatttgt taacaatgcc atgaaagcaa tttccttccc 780
ccaanggaaa agtgaattct aacagaatat ccggctggnc ctgtaattta attaaataaa 840
attttgggga acaannnnnn nnnnnnnnnn nnnnnnnna879

<210> 28
<211> 866
<212> DNA
<213> Homo sapiens

<220>
<221> misc_feature
<222> (1)...(866)
<223> n = a, t, c or g

<220>
<223> nbla-00434-r1

<400> 28
   ggntgnnnnt gggggctttt tttgggcctn ntttttttaa ttggcctngn nntttttttt 60
   gnttctccaa aattttattt taatttaaat tacagggcca gccagatatt ctgataagaa 120
   attcactttt ccttaggaga aagaaaatgc tttcatggca ttgttaacaa atgtatacat 180
   ctccactttt ttaagggtta aaaatcacat taagtaactg ccttatgtaa aattaaacct 240
   ttataacaca aaaaccactt ccagtataat agncttggna atatgaatat attaaatgat 300
   cataacttca aatgcaacat ttcaaactta aatgttttca cattgttttg cttaaaaaca 360
   aaacccacga agtctgaact accttcagat tccaatgtaa gagtaacatg aatgttgtgt 420
   ctgacacgct gnaccctcac ttcttccacc taaaagggta cgacctcacn gncaaagtgg 480
   aactgttcna aatgggtaag ttccaaaaat gagcttggna agaanacgtt tcctccactt 540
   ggggnaatcc angtttttac actaactcca tacacacagc actacaccac aaagccaagg 600
   acaccacatc acgggcaagt cttccttccc aaacatccaa ggagattttt tttcaanggn 660
   gaccaaaatg ccctaattca cacagattcn gggaaaagac agtcctgana aatgccaaat 720
   tttgngtnaa ccggcaattt aanaaaataa atanacccaa antttccggn gccaatttcc 780
   caacnggggt tcccgaaggt taaaccngcn gnaaaancaa ttgganggcc attttcnggc 840
   aagtnnncca attgggcaan aangnc 866

<210> 29
<211> 847
<212> DNA
<213> Homo sapiens

<220>
<221> misc_feature
<222> (1)...(847)
<223> n = a, t, c or g

<220>
<223> nbla-00460-f

<400> 29
gngnntttnn nngggccngg atctcntgca cggggcncct actggttctt ccagtcacct 60
cggcccggga tcgggaagtg tcaagcgggc gctcccccat ctccgccgct attaccactg 120
aacccggacc ccctacccag gtccagggcc agccgccatg acgaacgtgt actccttgga 180
tgggattctg gtgtttggtt tgctctttgt ttgcacctgt gcctacttca agaaagtacc 240
tcgtctcaaa acctggctgc tatcagagaa gaagggtgtt tggggtgtgt tttacaaagc 300
cgctgtgatt ggaaccaggc tgcatgctgc tgtggcaatt gcttgtgttg taatggcctt 360
ttacgtcctg tttataaaat gaattccaaa gcacccaagt catcaactgc caaccaaggg 420
gacggggatg aagaacctgt tggagacctg aacccagtgt aggagagttc agctgaaatc 480
atcggtcccc aggatgacac cacagcatct gcccctgcta tatgtgggga aaactcatgg 540
tcacgaacat tatttatgct tcaggggact acagaaagcc agcttccttt gatctatgtg 600
taaatcagtc cttggcagag tgcatataat gtccggataa attcatctag tataagaact 660
tgtgatgtta gattgaagtt ttgtcatcct ataaaagaca acaaacttan tttctgttta 720
agtccgaatg ttaatggcaa nttttanttg attacttaat tttctaacca aattttaant 780
ttcctcanan tgcaattgcc cttaattgtc nctggnaatt ccaattactt taggncatga 840
gaaattn 847

<210> 30
<211> 862

<212> DNA
<213> Homo sapiens

<220>
<221> misc_feature
<222> (1)...(862)
<223> n = a, t, c or g

<220>
<223> nbla-00460-r1

<400> 30
ggntgnnnnn ntggggcctt tttttggccc tttagggaan aaaagtncat tttcancgga 60
tttgtgcaga caatggtgaa taaaaaattg tatttcaact ataaaaagga gctgacttca 120
ttccaccctg gcctacaggg tttatggtga tgccagtttg aatctgaggc aatactgcta 180
tccccattcc tccacctgta aaagattccc ccagaggaat aacattctat aacttttagt 240
caaaaatatg taagacacat caaacaaagt aatgagttaa acaagtttct gcttatctgc 300
ttagggaaag tcaagaaatg aaacaaatgt atatttgttt ctggctgtaa attaccttta 360
ccttgtgcac ttttggtac attctggtat gaaaacctct caaaatgtaa caacacaaga 420
gtttgggtca agacgaccca cccaggaggc tgtaaaaact ggtttgaact aaaactgtgg 480
aatgggacta gtttaaaata tgaagcaact ctaaacacca agcttanaga catttgccct 540
attaagaaaa caaagatcat taaagctaca aaataacaag tgcaaacatg ctgaacctgt 600
ttccaaggag tgacattccc ntctgccaac aagtcccaaa ctcacaccca caangngtaa 660
ctctccttcc tggtccacta aanttccttt ctctcanctc aaangncctc aaaaatgaca 720
atgggaaacg tatgaattgt tgaaanttan cctgtggncc aantcccgga agagataaca 780
accacaactc ctganatgat tanacatgca ntgtttactt gatgactttc cgnanttcta 840
aaaaccccaa agcaataaaa tg 862

<210> 31
<211> 859
<212> DNA
<213> Homo sapiens

<220>
<221> misc_feature
<222> (1)...(859)
<223> n = a, t, c or g

<220>
<223> nbla-00478-f

<400> 31
gngnttttnn ngggcccngg atcctcntgc acggggcncc tactgggacg ggaattttt 60
gggagctggg aaaggaattc atcttagccc caaatgacca ttttaataat ttgcctgtga 120
acatcagtct aagtgacgtc caagtaccaa cgaacatgta caacaaagac cctgcaattg 180
tcaatggggt tttttggtct gaatctctaa acaaagtttt tgtagataac tttgaccgtg 240
acccatctct catatggcag tactttggaa gtgcaaaggg ctttttttagg cagtatccgg 300
ggattaaatg ggaaccagat gagaatggag tcattgcctt cgactgcagg aaccgaaaat 360
ggtacatcca ggcagcaact tctccgaaag acgtggtcat tttagttgat gtcagtggca 420
gcatgaaagg actccgtctg actatcgcga agcaaacagt ctcatccatt ttggatacac 480
ttggggatga tgacttcttc aacataattg cttataatga ggagcttcac tatgtggaac 540
cttgcctgaa tggaactttg gtgcaagccg acaggacaaa caaagagcac ttcagggagc 600
atctggacaa acttttcgcc aaaggaattg gatgttggat atanctctga atgaggcctt 660
caacattctg agtgatttca accacacggg acaaggaagt atctgcagtc anggcatcat 720
gctcataact gatggggcgg tggncaccta tgatacaatc tttgcaaaat acaaattggg 780
cagatngaaa aggtcgcatc ttcacatacc tcantgggcg aaaagctgcc tttgcanaca 840
atccaaagtg gntggccng859

<210> 32
<211> 863
<212> DNA
<213> Homo sapiens

<220>
<221> misc_feature
<222> (1)...(863)
<223> n = a, t, c or g

<220>
<223> nbla-00478-r1

<400> 32
   ggtgnnttgt nggcgtcttt tctgggttct gctggtntta gtggcctggg cccttttttt 60
   tnaaggccnt ttttttttnt ttttttttgt tngtttttgt tttgggtcnc ntttntgttt 120
   gtntttnntn nttgctttgt tttttttttt ttttttttgn tttttntttt ttngngtttt 180
   ttgtngnttn tttngtgttt ttcttntttt ttcngttttt gttttttttn tttttttttt 240
   tntctntntt tntttttntt tttgtntttt gtttttttnt nnttgttngt nttgttgntn 300
   ntnttntnct nttnnttttt gtgttttgtt tttggttntn ttngtnttnt tttttctttt 360
   ngtcgttttn ntttctnttt tnttnntttt tttntnttnn tctgttttgg gnngtggtgn 420
   tnttctnttt tttnnnnttt tttnntttnt tgttntggtt ntttcttttn ntttttctcn 480
   tgntttnttt tnttntttct tgttntgttt ttgtttnntt tgtttttttt gtnttttttn 540
   tttttttttt tttnnctttt tttnntnntc ctgttttttt gngtttgttg tgtttctttt 600
   tnttnttncg tttgtgtnct cttcngtctt tntntttntn ttttttttnt nttttttttn 660
   ttcctttnct nntntnnggt tttgtgtgtg tntntgtttt tntttgtttt tttttttttt 720
   nnnttgtntt tttttngttt nttttnnttt ttttnttntn tttnnttttg tttcntttgt 780
   tttgtnttct tnttttttnt tngtttnntn tttctttttg ttccnttgtt ttgtttgntt 840
   gtttttnttg ttntttcttt ttg 863

<210> 33
<211> 831
<212> DNA
<213> Homo sapiens

<220>
<221> misc_feature
<222> (1)...(831)
<223> n = a, t, c or g

<220>
<223> nbla-00483-f

<400> 33
ggatctcgng cactgtggcg cctactgggt ttagtataag atgtgccatt ttgtcctctg 60
tatgtctgaa tgaagctata acatttgcct ttttattgca ggttttcctt tggaatatgg 120
ataaatacac catgatacgg aaactagaag gacatcacca tgatgtggta gcttgtgact 180
tttctcctga tggagcatta ctggctactg catcttatga tactcgagta tatatctggg 240
atccacataa tggagacatt ctgatggaat ttgggcacct gtttccccca cctactccaa 300
tatttgctgg aggagcaaat gaccggtggg tacgatctgt atcttttagc catgatggac 360
tgcatgttgc aagccttgct gatgataaaa tggtgaggtt ctggagaatt gatgaggatt 420
atccagtgca agttgcacct ttgagcaatg gtctttgctg tgccttctct actgatggca 480
gtgttttagc tgctgggaca catgacggaa gtgtgtattt ttgggccact ccacggcagg 540
tccctaacct gcaacattta tgtcgcatgt caatccgaag agtgatgccc acccaagaag 600
ttcangagct gccgattcct tccaagcttt tggagtttct cccgtatcgt anttanaaga 660
ttctgccttc cctagtaata gggactgaca gaatacactt aacacaaacc tcaaagcttt 720
actgacttca attaaccgtt tttaaagacn ttnaagatta attaatttga aaggtcctgg 780
ncngcatttt gatcaattnn cctttaaaat atanttatng ncatanantt c 831

<210> 34
<211> 874

<212> DNA
<213> Homo sapiens

<220>
<221> misc_feature
<222> (1)...(874)
<223> n = a, t, c or g


<220>
<223> nbla-00483-r1

<400> 34
gggtnnnnnn nnntggtggc cttttttttt tnttttttgt atttccagta gagacagggt 60
ttcatcatgt ttgccaggct agtctcaaac tgccgacctc aggtgatttg cccgcctcac 120
ccaattcaac tttcgtaagt cagtatttac catctaactc agtgtcccag aatttaaaat 180
ttccttgcac tttacagcaa agatacatat tgtggctcta ctgaagcaat atatacatgt 240
cagaactaaa aatcagaaaa gcaaaagggt ccattcaaca tatagcagct tatatctaaa 300
tatgtacagg tatgtatgtt ttcacagtta gatctttaaa aaaatttata tttgatatgt 360
tcaagaaata cttctattgt ctataaataa tattttaaaa gctcaactga tcaaaatgca 420
gtacaagaac atatcaaatt aaataaatct tctacgtctt taaaaacaga taattgaagt 480
cagtaaagct tgaggtttgt gttaagtgta ttctgtcagt ccctactact anggaaggca 540
gaatcttcta aatacgatac gggagaaact ccaaaagctt ggaaggatcg gcagctcctg 600
aacttcctgg gtgggcatca ctcctcggat tgacatgcga cataaatgtt gcangctaag 660
ggacctgccg tggagtgggc caaaaataca cacttccgtc atgtgtccca gcagctaaaa 720
cactgccatc aantanagaa ngnacagcaa agaccantgc tcaaaggngc aaccttgcac 780
tggntaatcc tcatcaattc tccaaaacct caccaatttn acaatcagca aggnttgcan 840
aatnagtcca caanggntaa aagntacann tngt 874


<210> 35
<211> 879
<212> DNA
<213> Homo sapiens

<220>
<221> misc_feature
<222> (1)...(879)
<223> n = a, t, c or g

<220>
<223> nbla-00491-f

<400> 35
gnnnnttnnn nnggccnngg atcncgagca cggttggcct actgggtatg aacgaacctc 60
cttcagtcca actatccaga cgttcattct ctctgccatt tggcacgggg tatacccagg 120
atattatcta acgtttctaa caggggtgtt aatgacatta gcagcaagag ctatgagaaa 180
taactttaga cattatttca ttgaaccttc ccaactgaaa ttattttatg atgttataac 240
atggatagta actcaagtag caataagtta cacagttgtg ccatttgtgc ttctttctat 300
aaaaccatca ctcacgtttt acagctcctg gtattattgc ctgcacattc ttggtatctt 360
agtattattg tgttgccagt gaaaaaaact caaagaagaa agaatacaca tgaaaacatt 420
cagctctcac aatccaaaaa gtttgatgaa ggagaaaatt ctttgggaca gaacagtttt 480
tctacaacaa acaatgtttg caatcagaat caagaaatag cctcgagaca ttcatcacta 540
aagcagtgat cgggaaggcc tganggctgt tttttttttt tgatgttaac aagaaaccta 600
tcttancanc ttttcaaggg gtttgagttt gttgggaaag caattaactg gggggaaatg 660
gncagttatn gataaggaat tcctgtcacc agattgggaa tggggtgaaa caagccctcc 720
aaggcatgtc cccgtggggc acgccntatg ttagataatt ccatatttca ntngggantc 780
ccaacctcag nacttgtccg taggggcana acncgtgccc tgntgccgaa nggnnggttg 840
cgttncccaa gggactgaan aggnggnaaa naaacccgg 879

<210> 36
<211> 892
<212> DNA
<213> Homo sapiens

<220>
<221> misc_feature
<222> (1)...(892)
<223> n = a, t, c or g

<220>
<223> nbla-00491-r1

<400> 36
ggntgnnnnn ntggggcttt tttttnggct tttgtgagac ggagtcttcc tctgtcaccc 60
gggctggagt gcagtggcac aattttagct cactgcaacc tccacctccc aggttcaagc 120
aattctcctg cctcagcctc ccaagtagct gggattacag gtgtgcgcca ccatgcccgt 180
ctaatttttg tgttttcagt agagacgggg attcaccacg ttggccaggc tggtctccaa 240
ctcctgacct caagtgatcc gcccacctcg gcctcccaaa gtgctaggat tacaggtgtg 300
agccaccatg ctcagccaac ataatttcaa tgcatgggaa aacagaggaa atttatttag 360
ttatcaatcc tttcctgggt agggttagcc ttatcagccg gaaaacactt acttttagat 420
aaataaaaat agattaggat atttctaaac ttcaaaaaag acacatgccc atttttgctg 480

agacctcaaa agctgcaagt gcatttgctt ttcctttcct ccttaaaatg aagtatatat 540
agcataagcc anatggttgc cttgtcttaa cttcatttgc aaatttatta agtgactcca 600
acccccgaaa aaaagagaga gagaacccat cacaagaaaa ttgcattcat taaaaattta 660
angnaaaaag taaaaagtaa gttccataca tangtcaaac aaaggngaan tcaaaangnt 720
ttgcccctcc cctcctanaa gggaaggagg taaatccngg ggctgaggca ctccaatggc 780
ctggatcaaa tcccatcctc ggcttcannt cctggtggca acaanagtcc tccgttggga 840
ttccaaggaa atgcttataa tccaaggnaa gattagttgt gggaaaaaan gg 892

<210> 37
<211> 885
<212> DNA
<213> Homo sapiens

<220>
<221> misc_feature
<222> (1)...(885)
<223> n = a, t, c or g

<220>
<223> nbla-00494-f

<400> 37
   gngnntnnnn ngggccngga tctcgagcac tgtggcccta ctggagagtg tggagcgtgg 60
   agcgccggga ctgtgcacgc ttgaccggaa gcccagacca gtgcggtcct agccagagag 120
   aaaggacatt tgccaacaat gagacacgaa gcgcccatgc agatggcctc tgcccaagat 180
   gccaggtacg gccagaaaga ctcctctgat cagaactttg actacatgtt caaattactc 240
   atcatcggca atagcagtgt ggggaaaaca tcttttctat tccgttatgc agatgactcc 300
   tttacatctg cattcgtcag cacagttggg atcgatttca aagtaaaaac tgtattcaaa 360
   aatgaaaaga gaatcaagct tcagatttgg gacacagcag gccaggaaag atacaggact 420
   atcaccacag cctattatcg tggagccatg ggctttattt taatgtatga cattacaaat 480
   gaagaatcct tcaatgcagt acaagattgg tcaactcaaa tcaaaacata ctcttgggac 540
   aatgcccaag ttattctggt tgggaacaag tgtgacatgg aagacgagcg ggtcatctca 600
   actgagcgan gtcaacattt aggagaacag cttgggtttg agtttttgga aacaagtgcc 660
   aaggncaaca ttaatgtcaa gcaagacatt ttgagcgccn tgtgggtatc atctgcgaca 720
   aaatgtcaga naatttggng actgattctg ccatcactgc tgcaaagcaa naacacgaag 780
   actcaaggga actcctcctc aacgnaaccc aactgtgcct gcnaatgtcc ccgtgcanan 840
   aggnaactcc agggggtccn ggttccaana aacanaattt ttaat 885

<210> 38
<211> 890

<212> DNA
<213> Homo sapiens

<220>
<221> misc_feature
<222> (1)...(890)
<223> n = a, t, c or g

<220>
<223> nbla-00494-r1

<400> 38
ggnngnnnnn ntgtgggctt tttttggcct ttttttttat agtctcntag atatttattg 60
taatcttcaa gaaataaaac aaactcaaag aacaaaaacc actttcttta tagaattcac 120
atacaaactg aatacctaat agagcacata ccatttttac acaattaatt tcaataaaga 180
taattaaaac ttcacaatgt gaatacactt catagcttta aacccacaca agagctcctg 240
tggacacatg aagagtcaca atatttgcta tgcagcagta gttttcaact aaatgtcggt 300
cactatatga aagcattaag ctgttttcaa tattttctta agaagagttc cacagtgcag 360
tcactgagaa ggggccaaat gtgcctggtt ccaaaggaag atcttcatct gtgcatttcc 420
aatgcccttt atgggaaaca tggagctaca agaaaaatct ctgggtacac atctgccaag 480
tggcattagg tcggncaatc tctcatgggt tggtgcactg gcaaatgtct aaatcaactg 540
ccgtaatatg gcattaaagc aatttgcata atggggctga gtcctgagct gctcaacaca 600
agaggaatat gataatgcaa caagcttgtc ctcctcacgg ngactaaaag gtagggagct 660
aaactggggt aanttgaatg ggttcaaagg ggcaatttgg ctgtcctaag aaaacaatga 720
tctanaactg ataacnttgn cncncctcca ntcaaaggga acataactgt ggccttccca 780
cttaatttcc tggaaaaact cctcactact aaatcnttcc gggantaagt ttggcccctc 840
aaaggggncc tctctgaatc ctcaanttct aaagggcccc aatcccggtg 890

<210> 39
<211> 862
<212> DNA
<213> Homo sapiens

<220>
<221> misc_feature
<222> (1)...(862)
<223> n = a, t, c or g

<220>
<223> nbla-00499-f

<400> 39
gnngnttnnn nnggccnngg atcctcgagc actgtggncc tactgggttt aacttgcacc 60
aagcaagaaa atataaatac agttaactgc attaagataa tcacgttaaa attgttacta 120
tgcagcacag aacttcattc ttatagtatt cttgggttca acctttgaat caattttacc 180
actgatttaa taaatgactc aaagacatct gtaagtcatg ctgctgtgtt ttgaaagtct 240
ttaactaaat taagattgca gaatgatagt gattattcaa ttagatttta agtaaggatt 300
gtgatattag aggctggaaa tccttatttt ttaaaaaatc agataggcat aaatagttaa 360
atcactttca ttctccccaa acctgtagtt acagaaaaag ttttatgcta gaggtgggat 420
gccaagtttt cactatccat gaagcagcgc tgcatgtcac taggtaacac agattcatcc 480
agatggtgtt tacatttgat ttaattggga tcttattgac atcaggtata cttgggaaga 540
catttccttt atcctcagtg tatgaattta aagctatttt tgtaaatatt ctaatcancg 600
ataattctac ctangttctc aaccaactta accagtttgt tttcanagcc tgtantctaa 660
tgggaaccaa tttatcagtg cgccttaatg atgtggattt tgcanacatc aaacattacc 720
acannttcac aagtgcacct aanttcacca ttaacttaat cccaagtcaa tttttcccgg 780
ncacncaanc anngntntnt tnnnnacttt tgaaaagccg gaanactttg ctcaaaatgg 840
gaaggttaaa tnaggtnaaa nc 862

<210> 40
<211> 888
<212> DNA
<213> Homo sapiens

<220>
<221> misc_feature
<222> (1)...(888)
<223> n = a, t, c or g

<220>
<223> nbla-00499-r1

<400> 40
ggnngnnnnn ntgtgggctt ttttttggcc tcctttttaa catgtatntc tcanaataag 60
attcaccatg aacattattt tagatatttt tacatcttct ttagctaacc aagtgaaaaa 120
tccaatatac acaaataggt actttcatca gtacataaaa cagcaatagg tagcaaggtt 180
agaaatcaga atacaagttc aattgaataa atacaatact agtacagaga cacccatttt 240
taacccacaa tgaaaaatct gagccatgaa taggttttcc attttgaatc taacttctag 300
atgatctgta ttttgacctt aaaggggcca aaatatttaa tatgggtgaa gctcatgttc 360
cattacccgt aaaggtctaa tgcaacagaa aaatggtcat tttacattgg ttagaatttt 420
taactaggtc tttcatatta ttttgtttaa gcacatgaca gacttataca catattttaa 480
aaaatcaaac atgggatttc tatatacatc aaacctttaa caatgtctgt ctctgaattg 540

ctattanagt gttcaaaatt atacaaacaa acatccagta aacagcattt tattttatga 600
cactctgtgt taatacaata aatatacaaa acttccaacc cactgaaaca tgcaacatta 660
gagggacaca tgggagggca tttctgtnga accatgacag tcaccatgat ggcagcgcaa 720
tataagttac tgacaaatat tcaaacatct ctagataaat aatccaagtc ccaaattnca 780
aatcactgag taatgacttt ccgnggtcaa acnttagttt ggaataaaat tgcaataatt 840
aatattccaa aatggtaatg gcacggtgac tgcngttgaa nccatttt 888

<210> 41
<211> 923
<212> DNA
<213> Homo sapiens

<220>
<221> misc_feature
<222> (1)...(923)
<223> n = a, t, c or g

<220>
<223> nbla-00536-f

<400> 41
   gngntgntnn ntggaggttc annggcccag gatctcttaa acgggcccta ntggatttna 60
   acccaagcat tctgattcct gggatgagcc attagagctg ttcagaagtg gaatgggctg 120
   tcttttagga cagtgctcct ctcttctttc cgggtcgctg ccagcaagta cggagagagg 180
   agctatgact ttaaggtctt agantttgtg gaatgcagtt actccccatt tggccaaaat 240
   ctcagaattt gtgtcgtgtc tctaaaccag ataaatggag agtactaaaa gcattgttgc 300
   ttctgctctg aaattgttat aggattattt ttattgcatc caagtcttaa acactgnnat 360
   ggctggggat gggttctttc tacgaatatg tangtcctga gcatngncgn gtnncanaca 420
   gccctgtgat cagnggagcn ttnncngngt gagatatgtc catgtacant gnnngggcac 480
   cagacgtnca tngagnattn tcnagtngct cctgtgnacn ncaatnnant cactcaangn 540
   tactcctgan ngnagtctnt taccttgant ttacnngagt atanggntgg ttgggaaaat 600
   ngcttncaaa nnnnngggca tngtnttnna tggaatgcag atgatgtttg naacatgtgc 660
   tnatgnaggg aangnnntna cacnctcaat gttggnagna ttgntntcta ntnggtcnnt 720
   ntcannnttg ngngagtcct ntgacngcnn gtncnatncg tcanannatg nnnngnccat 780
   cantttnnng nagncancng tgnntnnnnt aanttngagg tangnnntgc tanncnatnn 840
   ctgtanacat gnnggcgatc gnnnngntga cttcntcaca nttacnncat nnttttcnnn 900
   gcgttannnn atnngcccca gtg 923

<210> 42
<211> 877

<212> DNA
<213> Homo sapiens

<220>
<221> misc_feature
<222> (1)...(877)
<223> n = a, t, c or g

<220>
<223> nbla-00536-r1

<400> 42
gngtggnttn gtgggncttt tcnnagcgcc ttgctttgac tanacatcgc acttggccng 60
taataggaaa tcanatcatc cagaatttaa aaagtgactg tgggggcttg ttgtcagctg 120
tcgggggcag gacttatgtg cagagctagg tccgtggctc tggtcaaggc actgccttgg 180
gtcactctgt gctggcccag gcnngctcca tgcctggctg ctggataanc cagttctctt 240
ctgaccccca aagcaatcca atggtggggc aaactcagga tctctctcan cccanagatc 300
tgactgatga ggcatangtt tttanctatt tgtaanaaaa tgaaagtatc tcatactacc 360
attgacacag ccaggtggga nggggtcact ggngaaacgc naactgnnnc tgcccatggg 420
atggaacctc gncanggttt angccctttg cagtgggaan ganctggtcc ctcctcntcc 480
tggtgtanaa cctgagattc antctttcaa gtgggaagaa gcactgganc atggnacant 540
gtcccaangg agagttntgt ttccccttttt cntcttttc acccnntana accctgtctt 600
ctcaccantc acaatgtncn gtgnaccctn aattttcant ngncatggnn cannnaaccc 660
tgtcctatat ttgaanctnn aggnanattc ctgtnnacca gngnatnttg gtgattttgc 720
ccctaggaan ttttcagang gncattttnn tgggtangnc ttngcnaanc aanccggntt 780
ttntcctggg cttttcggca antaacnttt ngntccnnac ctttntgatn ttatncnatt 840
cngtngtcac anactacctn nnggtcttna tcagctt 877

<210> 43
<211> 2105
<212> DNA
<213> Homo sapiens

<220>
<221> misc_feature
<222> (1)...(2105)
<223> n = a, t, c or g

<220>
<223> nbla-00538

<400> 43
tggcctactg ctagcaaaac ttgtttagct tagcaaaaac aaacacacaa aaaaactgag 60
aactctgctg tttcagatat gccataacat acatctgaaa cacatgtgta acaatcaaaa 120
tggtgggctc tagaatggtt ttggagctcg agatcttcat gggttagact tgctggtcag 180
acccaggagc acctgtggct cacaccttct gttcccctcc tggcctgtgc agaatgtaaa 240
cagcagactc atactcaatg ggcactacag gccttatcag acgttttata caagcctgga 300
ttgcttagta ggggaataag gcattctctg agggggcttt ccacttagat tgagaatttt 360
atttgaaaag aatctggttt aaatggcatt gtggtccgag gtagctgctc tccccactga 420
gagctgagcc gaaatataag aataatatat ttgtgcttcg agttggtgtt tctttcagtg 480
taatgcatgc agtggtcaca acccagttac tcataatatt tggattgtat ttgttcgtag 540
atatgcccag aagactagag aattagtgtt atataccata tagaacttac tgtcagtcaa 600
ctataaacag gcccaattaa aaactgttcc attactacgc aaacacatat tagaggcctt 660
tgctgatgac acattagctg gatcttagcc accccagaaa gggtttgatt tgaagctgat 720
tgttgccaga tatgcatatt ggaatcccat ctacccatag ttcctctgaa ggtgattttg 780
taatttgcaa aagggtatag gaaaatatac ctaaaagcga atttgtggct gagaggataa 840
acagaagctg tttgctcatg ttctgtgccc cacacccacc aatacctaaa tctgttaagg 900
aagacagaaa atgttttctt tgtgctcatt gagtagttcc agacagaaga agaatatact 960
ctttaaaatg tatttacctg ttagctggaa gtacccagaa ttatcagaaa cgaatgcaaa 1020
aaaaaaaaaa aaaaaaaaaa gcttacacag cttcttagca attttttttt tttttgccg 1080
aaacaataaa ttgcctttag cagcagttta aaatcctatc gtgaacaacc tatattttcg 1140
ccattttaca atggagagtt gtgacaagta caggttatca agtttgcact taactatgcc 1200
aaaaaaagtt tgaagcgctc tattctcaga catgctgtat tattacttct cattcaagat 1260
tgaaaaatat aaaggtatcc aaactctgtc ttaatgtaaa tgtaactatt tttccttcaa 1320
gtgttgacta gggagtcggt ttctctctta aagacactca ctgtacaact gaaagcagct 1380
gtcatatttc tggcaaaatg tgtttacgta tctgacaagt tgtacatttg tgtatgaact 1440
gacataaaat gtgaaagcct gtaagtgtac atgtagtggt gtggtgttct gtctagagga 1500
tacaactgaa tgtttttaat ttgctgactt acagacacag gctgtttaca aaatgctagc 1560
tggaaagtct gtaatgttca tgtcataact tttagttaat tgccattgag cacctgttct 1620
gaggaggtga gatgtggact tgtgcttata aactggagag tttagtcata atccctcctg 1680
gctttgtgtg aatagcttgc tcactttgct ggcctttgaa atgtgttctc cgtgataagc 1740
tatccatgtg tttgtgataa gagtgcttgt caaccatgac catctttgag ccttcctagt 1800
cctccacctg gcacagtatt tgaaatggca aaggatgtgc ttcatcctct aacaaacagt 1860
gtacactccc agagctgata ttctggattg tgactgtgca catttcctct agttcatgtc 1920
tgtagtccct atagaatgat ctgtaataaa atagtatact ggactgtgca tcaaagggat 1980
gtaaaattac agtattccaa aggttgaagt tctgctgttt tgttataatg cctgatacac 2040
atcttgaata aagtcttaac attttcttt taaaaaaaa aaaaaaaaa ggccacatgt 2100
gctcg 2105

<210> 44
<211> 863
<212> DNA

<213> Homo sapiens

<220>
<221> misc_feature
<222> (1)...(863)
<223> n = a, t, c or g

<220>
<223> nbla-00543-f

<400> 44
gnnnnnnnnn nggnnnngga tctcgagcac tgtggcgcct actgggacct ttcggcgacc 60
tcagatagac ccagctgttg aaggatttat tcgagacaaa tatgagaaga agaaatacat 120
ggaccgaagt ctggacatca atgcctttag gaaagaaaaa gatgacaagt ggaaaagagg 180
gagcgaacca gttccagaaa aaaaattgga acctgttgtt tttgagaagg tgaaaatgcc 240
acagaaaaaa gaagacccac agctacctcg gaaaagctcc ccgaaatcca cagcgcctgt 300
catggatttg ttgggccttg atgctcctgt ggcctgctcc attgcaaata gtaagaccag 360
caatacccta gagaaggatt tagatctgtt ggcctctgtt ccatcccctt tttcttcggg 420
ttccagaaag gttgtaggtt ccatgccaac tgcagggagt gccggctctg ttcctgaaaa 480
tctgaacctg tttccggagc cagggagcaa atcagaagaa ataggcaaga aacagctctc 540
taaagactcc attctttcac tgtatggatt ccaaacgcct caaatgccta ctcaagcaat 600
gttcatggct cccgctcaga tggatatccc acaagcctac cccagcttcc ccggggttac 660
acctcctaac agcataatgg ggagcatgat gcctccacca ntaggcatgg ttgctcaacc 720
aaggaacttc tggcatgcag gcatcaatga tgggtgtgcc gaatggaatg atgaccancc 780
ancaagctgg tacatggcaa gcatggaact atgccccaaa ctgtgtatgg ggccaaccaa 840
ctcaacaact gcaatggaac ttn 863

<210> 45
<211> 874
<212> DNA
<213> Homo sapiens

<220>
<221> misc_feature
<222> (1)...(874)
<223> n = a, t, c or g

<220>
<223> nbla-00543-r1

<400> 45

gngntgnnnn nntgtgggct tttttttgg cnnnttttt tttttccct gcaatattta 60
ttaaggaatt tacacataca caggagaaag gcagccagga actgtggttc ccacacatga 120
aatcttttaa gcaaagtttt cttgtctgaa ttttcaagtg gggtgaacaa tgactgagag 180
gaaagctgtc ccggccctct gcctcgtaca cctgggaacg gtggggaaac agagcaccct 240
ggatacacag gcatgaaaga gtgatcagca gaccgggaga agggaaggga gaaagggagt 300
tatcaatgac atggcgtttt ttaaaccata agaaaaacac aacagtttta ggctgctgat 360
aaattaattc ctctctgttg taaacctaaa actaaacaaa aacaaaaata cccagagcag 420
atggggaaag ggtgggaggc ggaacacaca ctcatacaca cacacacact cacgcacaca 480
cacaagcagt acacgttaag ctctgcattc ccactatagg ggttggtcct ccagccacgg 540
agccagattc tactgaaggc aagggggaag cnggaaggga tgtgtgtcng tgtgtgggat 600
nttcanaaag tccttggggc taataaaggn tgactgaanc aacaagtcct gaaaccccaa 660
attcaaaaag caactcaagg cgaananagt antaattttg ctcatcctca tangangggg 720
cttgcctcaa cccaaggcta aggtcaaact gacccaagac ccanggagta ttaatttgat 780
ttcaatactt gcttcaatcc cataaatcat gngaacatga tgcntttcaa atttaaacac 840
tttnaacaac tttttacaca tnttgaaggg ccct 874

<210> 46
<211> 898
<212> DNA
<213> Homo sapiens

<220>
<221> misc_feature
<222> (1)...(898)
<223> n = a, t, c or g

<220>
<223> nbla-00547-f

<400> 46
gnngnngnnn nntgtggctt tttnnnnngg ccnngnnctc gatcacgtgg gcctactgtg 60
gtgagagacg gaccgagacc ggagatgttt tcaagcccgg gctccggcgg ctttacaggc 120
ggctgcagcg gcgacgaaga caacgacagc gacggctacg ccgaagcact cgttccgggg 180
gtgaagcctc ctgcgccggc cttgcctcgg atccaggatg agaagactga taaaagaaga 240
agctagctga acagctgtaa aatgcccaaa tctgggttca cagaaccaat tcagagtgaa 300
aattctgaca gtgacagcaa tatggtagag aaaccatatg gaagaaagag taaagacaag 360
attgcatcct acagcaaaac tccaaaaatt gaacgaagtg atgtgagcaa ggagatgaaa 420
gagaaatcat ccatgaaacg taaacttcct tttactatta gcccatcaag aaatgaagaa 480
cgagattcag acacagattc agattcagga catacaagtg aaaattgggg ggagagactt 540
atatcttctt acaggacata ctcagagaaa gaaggtccag aaaagaagaa gacaaaaaag 600
gaagctggaa ataagaaatc cacaccaatt agcattcctt ttggttatcc actctctgag 660

cgaaaacaga tggcacttcn tatgcagatg acagcaagag acaacaatcc agattccaca 720
ccaaatcatc catcacaaac aacgcctgnc caaaagaaaa ctcccaattc ttcatctcga 780
cagaangnta aagntaataa aagaaatgaa cgtggggaaa ctccttacac atggntgcaa 840
tccagganat gtgaacaagt taangaatta taagtttagg gcaaatttaa tgttaaaa 898

<210> 47
<211> 840
<212> DNA
<213> Homo sapiens

<220>
<221> misc_feature
<222> (1)...(840)
<223> n = a, t, c or g

<220>
<223> nbla-00547-r1

<400> 47
   gggntgnnnn ngtggctttt ttttgnntna atgtcnacct tcngtcccat ttttttcant 60
   gtgcaatctg aattttcaaa tgttggacta tgatcttcat cctttatttt ggcatttgat 120
   ttttcaccaa attcnaagtg gaaatctttc tgatgtttgc tttttcctta agcttacaag 180
   attttacact tgaactttct ggcaagaatt cagattctgt attatcatac cgaacaagat 240
   caggctgtaa tgacatttca gaacttcctg gtgataaaca tgttttaagt atgttcttgt 300
   tttagtggtg ttgactgttt ttcacttaat ccacaagaat gtttgggaga tttaccagtg 360
   ggaatatgaa ataaatgtaa tgaagtatca tcttttgggc taaaagattc ctaaagtccc 420
   ctcctctctg gcctttctga acaatctagc cagtatttac tgtcaagttt gtaatcctgg 480
   antttccttt ccttcctttt ctgcttaact cctgggtctc ttaatttaat ctgatttcaa 540
   tttcctttaa ctttgccttc cgtttgtgtc acctgaaaca catatccttt ttgggttgca 600
   aacaaaattg angttcaagg atgacggaca tgaagtanaa anctttatcc gaaanagctt 660
   ctcacaaaac ttcagtactt ganataagac ccaaatggnt ttggttttac ctttaaagga 720
   ttaagntaaa anggttcncc cgnttgcaaa aaangagttt ttccngctca aggncaattc 780
   cnttccgngg nccttcccaa aaaaagccna ncacaancaa cctaanaaat tctcaacaan 840

<210> 48
<211> 871
<212> DNA
<213> Homo sapiens

<220>

<221> misc_feature
<222> (1)...(871)
<223> n = a, t, c or g

<220>
<223> nbla-00567-f

<400> 48
ggnttnnnng gcccggtccc gntcacgtgg ccctactggt atgaangaac ctccttcagt 60
ccaactatcc agacgttcat tctctctgcc atttggcacg gggtataccc aggatattat 120
ctaacgtttc taacaggggt gttaatgaca ttagcagcaa gagctatgag aaataacttt 180
agacattatt tcattgaacc ttcccaactg aaattatttt atgatgttat aacatggata 240
gtaactcaag tagcaataag ttacacagtt gtgccatttg tgcttctttc tataaaacca 300
tcactcacgt tttacagctc ctggtattat tgcctgcaca ttcttggtat cttagtatta 360
ttgtgttgcc agtgaaaaaa actcaaagaa gaaagaatac acatgaaaac attcagctct 420
cacaatccaa aaagtttgat gaaggagaaa attctttggg acagaacagt ttttctacaa 480
caaacaatgt ttgcaatcag aatcaagaaa tagcctcgag acattcatca ctaaagcagt 540
gatcgggaag gctctgaggg ctgttttttt tttttgatgt taacagaaac ctatcttagc 600
accttttcaa ggggtttgag tttgttggaa aagcagttaa ctggggggaa atggncagtt 660
atagataagg aattcctgtn caccagattg ggaatggngt gaaacaagcc tcccatggca 720
tgtcccgtgg gccacgccnt atgtagaata ttccataatt caatgggnac tcccaaactc 780
aacacttgtc cgtnngggca caccgtgncc tgttgcngaa ggtnggttgc gtatcccaag 840
ggactggaan aggnggaaaa taanntgtcc a 871

<210> 49
<211> 901
<212> DNA
<213> Homo sapiens

<220>
<221> misc_feature
<222> (1)...(901)
<223> n = a, t, c or g

<220>
<223> nbla-00567-r1

<400> 49
gngntgnnnn tgtggggctt ttttttngcc tttgtgagac ngaatctngc nctgtcaccc 60
gggctgggag tgcagtgggc acaattttag ctcactgcaa cctccacctc ccaggttcaa 120
gcaattctcc tgcctcagcc tcccaagtag ctgggattac aggtgtgcgc caccatgccc 180

gtctaatttt tgtgttttca gtagagacgg ggattcacca cgttggccag gctggtctcc 240
aactcctgac ctcaagtgat ccgcccacct cggcctccca aagtgctagg attacaggtg 300
tgagccacca tgctcagcca acataatttc aatgcatggg aaaacagagg aaatttattt 360
agttatcaat cctttcctgg gtagggttag ccttatcagc cggaaaacac ttacttttag 420
ataaataaaa atagattagg atatttccaa acttcaaaaa agacacatgc ccatttttgc 480
tgagacctca aaagctgcaa gtgcatttgc ttttcccttc ctccttaaat gaagtattat 540
atagcataag ccaaatgggt gccttgncct taactcaatt tgcaaattta taatggctcc 600
aaccccccga aaaaaagnga gagagaaccc atcacaagaa aanttgcatt caattaaaat 660
taaggnaaaa agttaaaant agnttcaaac aataggtcaa acaaangngg aatcaaaaag 720
gnttgcccct cccctcccaa aaggnaaggn ggnanaaacc tggggntnaa ggcantccaa 780
aggcctggga ncaaaatcca atcctttggn ntttaanncc cgggggcaac aaaantccnc 840
cggngggatt tcangggaat gctnnnaatc caagggaaan ttantttngg gaaaaanggc 900
c 901

<210> 50
<211> 881
<212> DNA
<213> Homo sapiens

<220>
<221> misc_feature
<222> (1)...(881)
<223> n = a, t, c or g

<220>
<223> nbla-00569-f

<400> 50
gngnnnnnnn nggccgggat cctcgntcac gtggccctac tgggcaaccc tcccaaaact 60
gtggggcatt gatgacctga ctggggagcc tctcattcag cgtgaggatg ataaaccaga 120
gacggttatc aagagactaa aggcttatga agaccaaaca aagccagtcc tggaatatta 180
ccagaaaaaa ggggtgctgg aaacattctc cggaacagaa accaacaaga tttggcccta 240
tgtatatgct ttcctacaaa ctaaagttcc acaaagaagc cagaaagctt cagttactcc 300
atgaggagaa atgtgtgtaa ctattaatag taagatgggc aaacctccta gtccttgcat 360
ttagaagctg cttttcctaa gacttctagt atgtatgaat tctttgaaaa ttatattact 420
tttatttcta ctgattttat tttggatact aaggatgtgc caaatgattc ggatactaag 480
atgcatcgtt tgaaatcatc tantgtgttg tatgcagtta tcctcaaaaa catcagcgat 540
gtctgaacct ttaaaacatc tgttagagca aaattaaaaa gagcatttgg tagtaatcca 600
actttttggt caattaataa gtggntgata aaagtttcca naattttccg ggaaagttaa 660
aaaaaagtta catgtcantt ggngaaaata cgttatccag aanttgtgca taaantgatg 720
ccaaaaaagg canttcaaca ttgtggacaa ngggagacac tntttaaant cccgaaagaa 780

agcaatgggt tttcannttt atgggngaaa caaattaang ggggccttna acnaanaaat 840
gttatgttaa ccaanangcc gngnttnatt tggcnnnaan n 881

<210> 51
<211> 884
<212> DNA
<213> Homo sapiens

<220>
<221> misc_feature
<222> (1)...(884)
<223> n = a, t, c or g

<220>
<223> nbla-00569-r1

<400> 51
ggnngnnntg nngggctttt tttggnttgt ttaagatcat ttagnagaac agtcattcag 60
aagccattga gacatcaggc agcagaaagg aaggtgggat ggagcaggcc ctgtgaagga 120
ccaagaacaa agtaatagcc acagttatga aatttcattt tattctgata aagactaata 180
tatgcttgat aacctagtga taatccataa gtttggtatt tcacaacatt ttttagaaag 240
cacataagat taacattcaa ataaggcatt atagaaagtt ttataaagaa tgaagtgttt 300
cctataattc ttttaaaaaa ccttggttca tcttgaaaga tcgatgaatt ttttaaatat 360
cagaagaaaa gggaaataaa attttccccc caaaacacat aagaaccact tactggcact 420
tgtattttaa gtacctggga aaaaaacggg acagattttt aaaggnaata acgacttgta 480
agacggctgn ttcattgatt tggcacgaag taaagtaaga gtaaatatgc catgggagac 540
ataatcaagt ttttcctcca nctcccaaaa ttccccactt ccaacaagac acacagtnca 600
tcaacaanca ncccttaaat tccaantttt aaatngntgn tcaaacgaca ncaataaaag 660
tccttccccc ttaaataagg gggnaatnna tgctacaagn atcaaattcc ttggtnaagg 720
acaaagnant ccctcaagga aatcaanttc ccgggccctg gtaanccaac aagnnaatcc 780
ntttatngga caaccggtta acaaattaaa aatgntggca attnttncca ggggacaant 840
tccaagnaag cccaaccatt ttcnanaacn aagntggntt tctt884

<210> 52
<211> 793
<212> DNA
<213> Homo sapiens

<220>
<221> misc_feature

<222> (1)...(793)
<223> n = a, t, c or g

<220>
<223> nbla-00639-f

<400> 52
gtttaatgcc tagttatgca acttgagtgc tttttttttt ttttgcagga taattttaag 60
tgtatatatt ttaactgcac tggtacattg aacatgtcag tgtcgatgcc actggaccaa 120
cagagcattt tgtggctata gggtgcctga taataatgtc ttgatttttc tttgggattc 180
aactatatcg aaggtttttc ccccagcagt atgggggcgt acaggtgatc gatcattaat 240
gtcattgcaa ttgttatttt ttaaaataaa tttataaaaa taaccgaaaa gtaatgtgac 300
ttttgangaa tctgcaactc ctgggttcta ntttgtgttt ccagtgttga aattttgatc 360
tcccccgaca agacttgtct gcatggacgg tgtctctgta aacagtatca aacttngtna 420
tgtctgagcc tcttagcttt atctccctta atttcntccc ttttggccct tccaccctgt 480
tgaggagant tttctgtcac ctgcanggct gatnatacat ttgatgctac nactgaangt 540
gttatcngaa tgtgnnnnan tnantncatg angaaaatta acccgtantc acaaaaacag 600
gcttaantan tgnanaaatt caggtagtga nngattanan ntnctnttgg ntagaatttg 660
ttttgngaga aagncanncn anangtnctt tctnaatngg nanaatccca annnnncnca 720
accttgnaac gggttnctta agggcncnng gaatcnnanc acnannncca naagccncca 780
tantnnntag tnn 793

<210> 53
<211> 882
<212> DNA
<213> Homo sapiens

<220>
<221> misc_feature
<222> (1)...(882)
<223> n = a, t, c or g

<220>
<223> nbla-00666f

<400> 53
gnnttnnnnn ggcccnnnnn ggattcctcn ttcactgtgg cccctactgg gacaccgcca 60
aaggaagtca anaaggaagt taagaaggaa gagaagaagg aagtgaaaaa ggaagaaaag 120
gaacccaaaa aagaaattaa gaagctccct aaagacgcaa agaaatcatc tactcctctg 180
tctgaagcaa aaaaaccagc tgctttaaaa ccaaaagtac ccaagaagga agagtctgtc 240
aagaaagatt ctgttgctgc cggaaagcca aaggagaagg ggaaaataaa agtcattaag 300

aaggaaggca aggccgcaga ggctgtcgct gcagctgccg gcactggagc caccacagca 360
gctgtcatgg cggcagctgg aatagcagcc attggccctg ccaaagaact cgaagctgag 420
aggtccctta tgtcatctcc tgaggatcta accaaggact ttgaagagtt aaaggctgaa 480
gaggtcgatg taacaaagga catcaagcct cagctggagc taatcgaaga cgaagagaaa 540
ctgaaggaaa ctgagccagt cgaagcctac gtcatccaga aggagagaga agtcaccaaa 600
ggtcctgccg agtcccctga tgagggaatc actaccactg aaggggaagg cgaatgtgaa 660
caagacacct gangaactgg ancccgtcna gaaacaagga atanacgaca ttgaaaaatt 720
ttgaagatga angganccgn tttgaagaat cctcanagac tggngactat gaanagaang 780
nanaaactga ggangctgag gagccanaan angatgggga aggaacactt tgttttancg 840
cctccaaagc anaagcccac tgagggtnna ggaaatttcc aa 882

<210> 54
<211> 961
<212> DNA
<213> Homo sapiens

<220>
<221> misc_feature
<222> (1)...(961)
<223> n = a, t, c or g

<220>
<223> nbla-00666r1

<400> 54
   cnnnnannan nnnnanngna annanannnn nnnnnnnnnn ngnnnnnann ggnngnnctn 60
   agnagnanna nncnnngntg attantnagg ccnannngnn ttattttttg gccttttttt 120
   ttttnanttt tttttntnnt ttttttttaa aaantcaaat ncnggntttt tttttattta 180
   agaggaatgt aaatngtnaa nncncggtta ccaagggana nataaaanca aaancaaaaa 240
   acgggnctna nttggccngg aaacccttgt tttcnancct tttcttcaaa aggantttcg 300
   gaaccncggc naaaaccctt gnnaccaana cttaaaaaan tttcaaaagn aaacccggtt 360
   cnaangaggg gngggntgcg taaagggnaa aaaanttttt caaaaggaaa cnctgantca 420
   ggnacgggcn canccanggg gcttatggaa cccctttnan tccnaccttt ggaaacacgg 480
   anttcaaaac ccacgggaac ngcngggggt ttttcaanaa cttntgtngg aaaaanacng 540
   gatttcccgc cagtaggaaa ttgataanaa ngngngtgac caacaactgc cattcacggg 600
   cngaaaatgg gganacaact tccaaaancn cttgnccnca ggactancna aaagttcttc 660
   aaaaanttnt tgggggcccc ncangnaaaa accggggggn aattntggcc cccgaaaaaa 720
   aatttnaacc caatgggggg ncnaaaaaaa aanttcaaan ctaaggtttc accccaaggg 780
   ggggcttttt ccaaaggggg aatgggggaa annggcccca gggaaggggc tccttccaaa 840
   ggggcgnaac tttttnactt tnaaaaancn gaaaagccaa ccttgangtc ccaanagggg 900
   gngggggttt nccttnaana nnantanccc cccctnagca attnggcgaa tttnncnccc 960
   c 961

<210> 55
<211> 801
<212> DNA
<213> Homo sapiens

<220>
<221> misc_feature
<222> (1)...(801)
<223> n = a, t, c or g

<220>
<223> nbla-00724-f

<400> 55
gnnntgtngc ttttnnnngg gcatcctcta gcactggcng nctactggga aanctacaat 60
ttcanccccca ctggctgaga tgaccagctg acctttaagg ctgttgaagg atctctcatc 120
ccactctcct ggcactaggg atttctccac acccaccatc tccgaaggaa aagcagaatc 180
cactccctcc cacactcttt ctgccatttc attcatgcat gcctgaatca gaaccctcac 240
tctctctgcc attcattcat acatgcctga atcagaacct tcactctctc tgccattcat 300
tcatacatgc ctgaatcaga accctcactc tctctgtcat ttcattcata catgcctgaa 360
tcanaaccct cacactctct gtcatttcat tcatacatgc ctgaatcana accctgccca 420
gcgacacaaa cggagtgctc agttctgccg tgaagatgca gccgacaaag aagtcnccaa 480
ggtctccccg gagcctctgc ggatgacnaa acgaaaaggg gaagtaagtg gaggttaaga 540
aaccaccaga aggaaaattc caacgaaggn tactgaaaag ggtaactgtg acnggcaaag 600
gacgctcaat angnaagcgc agactactgt ancgctgctt ccgttgnaac angcactttg 660
atngtccaag ccctaagatt caaangcaaa ttnanacang gtgacggggt tccacaacaa 720
nctctgaatt ttngtgacaa cccgnaagga aggntgaaag cctacttttg tgtnccaact 780
tatcatgcaa nanactnant c 801

<210> 56
<211> 791
<212> DNA
<213> Homo sapiens

<220>
<221> misc_feature
<222> (1)...(791)
<223> n = a, t, c or g

<220>
<223> nbla-00724-r1

<400> 56
gnntgtggct tttttttggc nttttctgag acagtctcac tctgcngccn cagctgggag 60
tgcagtgggt gtgatctcag ctcactgcaa cctccgcctc ccgggttcaa gcgattctcc 120
tgcctcagcc tcctgagtag ctgggattgc aggtgcccac caccacgccc ggctaatgtt 180
tgtattttta gtagagatgg ggttttgcca tgttggccag gctgacttca aactcctgaa 240
ctcaagtgat ccgcccgcct cggcctccca aagttctggg attacaggca tgagccactg 300
cgcccggcct gagctagatg tcttgaccgt tacttaggag tcgggattgc ccctcttttc 360
tttgccactc ataggcaccc attctccttg tgcctgctgc ttcagagcca cctcccccag 420
gtgaatagtg gaggaacacc attacacatg aaacgttcat gatcctcctt accaacatca 480
acatcaacat actttttctt ttttgagaca gggtttcact ctgtcacccc gggtggagtg 540
cagttgttgg atnacagctc actgtacctc aaactactgg nctcaagtga tcctcctgcc 600
tctgcttcca agaaagctgg gaacacaggc acacactaca acgtccaact aattcctgta 660
attttgtana ntttctccat gttgcccaag ctggtctcaa actcntgggc tcaagtgatc 720
tcctgcctaa ggctccaaag tgctgggatt acaagggcna ancaatgnaa ccgggcccaa 780
aaattctttt t 791

<210> 57
<211> 796
<212> DNA
<213> Homo sapiens

<220>
<221> misc_feature
<222> (1)...(796)
<223> n = a, t, c or g

<220>
<223> nbla-00727-f

<400> 57
gnnnnnnnnt ttttnnnggc ntcttttgca ctggctcgcc tactggactn aggagtggga 60
gccccggggt cagagcgata attcccccac aggaggcatc cgctctacgg cgggagattg 120
aggacaagct gaagcaggag gaggagactc tgtccttcat ccgagacagc ctggagaaga 180
gcgaccagct cactaagaac atggtgtcta tcttatcatc ctttgagagc cgccttatga 240
agctggagaa ctccatcatc cctgtgcaca agcagacgga gaatctgcag cggctgcagg 300
agaatgttga gaagacgctg tcctgcctgg accatgtcat cagctactac catgtggcca 360
gtgacactga gaagatnatc agagagggcc ccacaggtag gctggaagag tacctgggaa 420

gcatggccaa gattcagaag gcagtggagt atttccagga caacagccca gacagcccgg 480
aactcaacaa agtgaaactg ctctttganc gcgggaagga ngccctggag tccgaatttc 540
gcancctgat gacgcggcac agtaaggtcg tctcgcccgt gctcatcttg gatntgatca 600
gtggtgacga tgatntggag ggccaagagg acntnaccct ggagcacctg ccgaaaacgt 660
gctccaagat gtcantcgca tctcccgctg gctggttgga tatggccgca accaagantt 720
catgacgtct actaacaaga tanctccaac caactggacc gctccatcaa agggctgang 780
gncatttcat tanaan 796

<210> 58
<211> 799
<212> DNA
<213> Homo sapiens

<220>
<221> misc_feature
<222> (1)...(799)
<223> n = a, t, c or g

<220>
<223> nbla-00727-r1

<400> 58
gntgtggctt ttttttggcc tttttttttt ttgccctttt tttttncaaa gctaacactg 60
ggncttatct gtccaatgaa acgccagtct ggtggaacca ggcagggcta gcagcaggct 120
caggcagagg tgtcgaaaag gcgatcgatc atgtcgccca cctgctccac cccgtacttg 180
atgtacttct ccgggttctt ggtgaagggc acgctgccaa acttctgtag aaaggccccg 240
taggtctcct tgacaatggt cttctgggcc tggcgaatcc tgtccctctg ctctgtgtct 300
ggaatanccc angccttctg gattttgcac agttcttcga ggccatcatt gaagccctta 360
aaacgctcct tgataatctg ccgctccttg tcccggagct tgactcccgg ctggaacaca 420
ggtagattct tctctgcgat gtaatcagtc acctttaacc agctgcgctg gtaggtctgg 480
atntgctgct caatgtgctc ccggtaggag cgctcaacan tcttctgtgt cactgccacc 540
anctggatna gttcaaactt ctccaaggac ttgaggatgt aattgtantt gttgtgcaag 600
aaaatggcgc tcaaanctgg gtcctcgtan ancttggnct tgctcaacaa gttcaantgc 660
angttgccaa cactttncaa atatnngtgc ttaacaaccg ctttctgaac tcaaaactgt 720
anctgggngg cccaaaaact ggtntctccg gggggtctaa aaggaatatt gtatgtgtcc 780
ccaaaaaant tgggangnc 799

<210> 59
<211> 484
<212> DNA

<213> Homo sapiens

<220>
<221> misc_feature
<222> (1)...(484)
<223> n = a, t, c or g

<220>
<223> nbla-00751-f

<400> 59
   ggggnnnntt nncttgcngc ctgtttcctc gtgcactgtt ggtaactggt cggcctcgct 60
   tcaggaaaaa aagtacctca tnctcggcca ccgaaaccac gtgagtgaga tgagccaaca 120
   gcaccggatc cacagaatgt ttcttctctg ccttaaagag ctattcacta ataacataga 180
   aatccgcaag ctgggtgtgc tttgagtgtg cagcctcaca aacatggcct tttctctctc 240
   cccttccact tttaaggatt tattttttcc cccttttctt tattttgctg gggagaggct 300
   aaagggaaag gtagtagggg cgggggtggt gacctttaag tcttctgagg ttggtaattt 360
   tccacaattg gattgtcatt atagacagca gtgtgttttt tagaaagata agagaatcac 420
   ccctatgctg ctgagatgta catttgtaat ttatctgttg catacttagt ttttagtcct 480
   gtaa484

<210> 60
<211> 485
<212> DNA
<213> Homo sapiens

<220>
<221> misc_feature
<222> (1)...(485)
<223> n = a, t, c or g

<220>
<223> nbla-00751-r1

<400> 60
gnnncntgtn tgcctttgct tttttttttt aaagccaaaa gtnttaaaga ctttattcca 60
gatgtgtatc angcattata acagaacagc agaacttcaa cctttggaat actgtaattt 120
tacatccctt tgatgcacag tccagtatac tattttatta cagatcattc tatagggact 180
acagacatga actagaggaa atgtgcacag tcacaatcca gaatatcagc tctgggagtg 240
tacactgttt gttagaggat gaagcacatc ctttgccatt tcaaatactg tgccaggtgg 300
aggactagga aggctcaaag atggtcatgg ttgacaagca ctcttatcac aaacacatgg 360

atagcttatc acggagaaca catttcaaag gccagcaaag tgagcaagct attcacacaa 420
agccaggagg gattatgact aaactctcca gtttataagc acaagtccac atctcacctc 480
ctcag 485

<210> 61
<211> 473
<212> DNA
<213> Homo sapiens

<220>
<221> misc_feature
<222> (1)...(473)
<223> n = a, t, c or g

<220>
<223> nbla-00757-f

<400> 61
tggccngcnn gtttcctcgc ccactgttgg tanactggag ttgcgcaaga tggcgccngt 60
gggggtggag aagaagctgc tgctaggtcc caacgggccc gcggtggcgg ccgccggcga 120
cctgaccagt gaggaggagg aaggccagag cctatggtcc tccattctga gcgaagtgtc 180
cacccgcgcc aggtccaagc tgccgtccgg caagaacatc ctggtcttcg gtgaagatgg 240
ttctggtaaa acaaccctca tgactaaact acaaggagct gagcatggca aaaaaggaag 300
aggcctagaa tatctctacc tcagtgtcca tgatgaggac cgagatgatc acacgcgctg 360
caacgtgtgg attctggatg gagacttgta ccacaaaggc ctgctgaaat ttgcagtttc 420
tgctgaatcc ttgccagaga ccctcgtcat ttttgttgca gacatgtcta gac 473

<210> 62
<211> 487
<212> DNA
<213> Homo sapiens

<220>
<221> misc_feature
<222> (1)...(487)
<223> n = a, t, c or g

<220>
<223> nbla-00757-r1

<400> 62
gnnngctgtt tgccttngnc tttttttttt ttaagccaca aggccgcata atgtaaccag 60
ccataatgtg cataaatagt cataaaattg tctttaacag tctaccatac aagtgtactc 120
tgcaaataaa atgaatttta ctttaaaaac taaaggctgt ttattatttg gcctttggtt 180
ccaatgcttg aaaaccccaa aataacaaaa ctcatctccc ttcctcccac ctcccaaaaa 240
gaaccctgca gctccccaaa gttggtagac tttggcaacc actgagcaca tgacactatg 300
tatcagcttc ttctgatact aggagcttcc acttctcatg agtaaataaa agtggtctcc 360
aagaaacctt ccttcacaag acatgtctta gcatttcatc agttttagta tattttatat 420
tgacacaaaa agtgcttatc agttcaaatg ataaaaaata aagacaaatt aaaattttgc 480
attaaaa 487

<210> 63
<211> 2815
<212> DNA
<213> Homo sapiens

<220>
<221> misc_feature
<222> (1)...(2815)
<223> n = a, t, c or g

<220>
<223> nbla-00761

<400> 63
gaattcctcg agcactgttg gcctactgga acagcaccac ttagatccta gctctgctgt 60
aagaaaaaca aaacgggaaa aaatggaaac aatgttaagg aatgattctc tcagttcaga 120
ccagtcagag tcagtgagac ctccaccacc aaagcctcat aaatcaaaga aaggcggtaa 180
aatgcgccag atttcgttga gcagttcaga ggaggaattg gcttccacgc ctgaatatac 240
aagttgtgat gatgttgaga ttgaaagtga gagtgtaagt gaaaaaggag acatggatta 300
caactggttg gatcatacgt cttggcatag cagtgaggca tccccaatgt ctttgcaccc 360
tgtaacctgg caaccatcta aagatggaga tcgtttaatt ggtcgcattt tattaaataa 420
gcgtctaaaa gatggaagtg tacctcgaga ttcaggagca atgcttggct tgaaggttgt 480
aggaggaaag atgactgaat caggtcggct ttgtgcattt attactaaag taaaaaaagg 540
aagtttagct gatactgtag gacatcttag accaggtgat gaagtattag aatggaatgg 600
aagactactg caaggagcca catttgagga agtgtacaac atcattctag aatccaaacc 660
tgaaccacaa gtagaacttg tagtttcaag gcctattgga gatataccgc gaatacctga 720
tagcacacat gcacaactgg agtccagttc tagctccttt gaatctcaaa aaatggatcg 780
tccttctatt tctgttacct ctcccatgag tcctggaatg ttgagggatg tcccacagtt 840
cttatcagga caactttcaa gccaaagcct tagtagaaga acaacgcctt ttgttcctag 900
ggttcagata aaactatggt ttgacaaggt tggtcaccaa ttaatagtta caattttggg 960

agcaaaagat ctcccttcca gggaagatgg gaggccaagg aatccttatg ttaaaattta 1020
ctttcttcca gacagaagtg ataaaaacaa gagaagaact aaaacagtaa agaaaacatt 1080
ggaacccaaa tggaaccaaa cattcattta ttctccagtc caccgaagag aatttcggga 1140
acgaatgcta gagattaccc tttgggatca agctcgtgtt cgagaggaag aaagtgaatt 1200
cttaggcgag attttaattg aattagaaac agcattatta gatgatgagc cacattggta 1260
caaacttcag acgcatgatg tctcttcatt gccacttccc cacccttctc catatatgcc 1320
acgaagacag ctccatggag agagcccaac acggaggttg caaaggtcaa agagaataag 1380
tgatagtgaa gtctctgact atgactgtga tgatggaatt ggtgtagtat cagattatcg 1440
acatgatggt cgagatcttc aaagctcaac attatcagtg ccagaacaag taatgtcatc 1500
aaaccactgt tcaccatcag ggtctcctca tcgagtagat gttataggaa ggactagatc 1560
atggtcaccc agtgtccctc ctccacaaag tcggaatgtg gaacaggggc ttcgagggac 1620
ccgcactatg accggacatt ataatacaat tagccgaatg gacagacatc gtgtcatgga 1680
tgaccattat tctccagata gagacaggga ttgtgaagca gcagatagac agccatatca 1740
cagatccaga tcaacagaac aacggcctct ccttgagcgg accaccaccc gctccagatc 1800
cactgaacgt cctgatacaa acctcatgag gtcgatgcct tcattaatga ctggaagatc 1860
tgcccctcct tcacctgcct tatcgaggtc tcatcctcgt actgggtctg tccagacaag 1920
cccatcaagt actccagtcg caggacgaag gggccgacag cttccacagc ttccaccaaa 1980
gggaacgttg gatagaaaag caggaggtaa aaaactaagg agcactgtcc aaagaagtac 2040
agaaacaggc ctggccgtgg aaatgaggaa ctggatgact cgacaggcaa gccgagagtc 2100
tacagatggt agcatgaaca gctacagctc agaaggaaat ctgattttcc ctggtgttcg 2160
cttggcctct gatagccagt tcagtgattt cctggatggc cttggccctg ctcagctagt 2220
gggacgccag actctggcaa cacctgcaat gggtgacatt caagtaggaa tgatggacaa 2280
aaagggacag ctggaggtag aaatcatccg ggcccgtggc cttgttgtaa aaccaggttc 2340
caagacactg ccagcaccgt atgtaaaagt gtatctatta gataacggag tctgcatagc 2400
caaaaagaaa acaaaagtgg caagaaaaac gctggaaccc ctttaccagc agctattatc 2460
tttcgaagag agtccacaag gaaaagtttt acagatcatc gtctggggag attatggccg 2520
catggatcac aaatctttta tgggagtggc ccagatactt ttagatgaac tagagctatc 2580
caatatggtg atcggatggt tcaaactttt cccaccttcc tccctagtag atccaacctt 2640
ggctcctctg acaagaagag cttcccaatc atctctggaa agttcaactg gaccttctta 2700
ctctcgttca tagcagctgt aaaaaaattg ttgtcacagc aaccagcgtt acaaaaaaaa 2760
aaaaaaaaaa aggccacatg tgctcgagct gcaggtcgcg gccgctagac tagtc 2815

<210> 64
<211> 815
<212> DNA
<213> Homo sapiens

<220>
<221> misc_feature
<222> (1)...(815)
<223> n = a, t, c or g

<220>
<223> nbla-00775-f

<400> 64
gcaattctaa ttctttttgc agatctctct gcctccaggg gnttaccnct gagctgagaa 60
agccnaaaac ggagttctgt ttatttctgc gcccctctca agtaggtatt aaaaaagatt 120
gangacatga aagaaagaat aactgaatgt tactgatgcc agaagcaact ctcgctgggc 180
gcaactctcg ctgggcgcaa ctctcgctgg gcctcagttt ctcatgagca gaactgcagg 240
gggtaggtgg tcccaagtct ctagggtctc caccaagctt ctcctcagtg cccaagaagt 300
ggtacagtgt gctctcaata gtccttcagc ttatgacagc ctcagctcat gggacttgat 360
gacggggaag agcaagctgc atgccatgaa gtcagccaag tttgcctgtg gaggctgctc 420
ccatctcctt tcaaaactgc cccctcccct atcaagnccc cacttcttcc ccanggcctt 480
gctctcagcc aactgtctgg gatggntttc ctgtatagct ttgagctccc tcagcacaag 540
gagcctgaca tggngttang aaaaaagaaa ggaaaatgat tanaggnncc aaaagnccat 600
gatnccataa ncccaaaggt taagtacctt aaggggggat tangtgccct gtnanncaga 660
atnagagaga tnaaagttgg gnnncaanca atagtaaggg gctttaggtn acctggnaac 720
tccaggagct ccaanggcca anannnatgt tttattcacg gcaactcccc ngnaattggg 780
aagtttccan catacacatg ggaanaaacc naagg815

<210> 65
<211> 799
<212> DNA
<213> Homo sapiens

<220>
<221> misc_feature
<222> (1)...(799)
<223> n = a, t, c or g

<220>
<223> nbla-00775-r1

<400> 65
aacctccacc tcttgggtcc aagtgattct cctgcctcag cctcccgagt agctaggact 60
acaggcgtgc accacgccca gctaattttt gtattttttt tttttagta gagacggggt 120
tttgccaggt tggccagggt agtctcaaac tcctgacctc aggtgatctg cctgcctcgg 180
cttcccaaag tgtttggatt acaggtgtga gccactgcac ccatccatgg cccttttctt 240
gtcctcccca cggggctttg tctgatggat ttcaagcatc agcaaaggaa atactgggtg 300
tcactgtcat ctgggaccaa cctgattctg gaggagccaa aagtctcatg gtgattgatc 360
tcgtccgcnt ggggtgtatg gttgtaatcc ttcnctctgg cattanggca cacacgaatt 420

cctaacatcc cactccaacg aaacacttgt gcacagatta caaacaccaa tatgatttaa 480
tcaaacacaa gnaaagaaca atgaccntca aaaccgggta aaaataataa anttaaaacc 540
atggtaaaaa tttanacaat cncgttaaaa gttngaagga atgattcccc tcctccccaa 600
ttgataaatc aacaccanat aaaaataant tgttccaaaa aaaanccctg gtccccaaaa 660
aatnactggg nggggcccca aggattccct tacaatccca atgncacaaa ctaaaattcc 720
cccccccaaa ttttaagnga aaatggnccc caactntagg aaaaggnggg nccagccana 780
ttnannnntt taagggann 799

<210> 66
<211> 867
<212> DNA
<213> Homo sapiens

<220>
<221> misc_feature
<222> (1)...(867)
<223> n = a, t, c or g

<220>
<223> nbla-00784-f

<400> 66
   gnngnnnnnn nnnnnggnnn nttnnnnntg gccggatcct ctttcactga ggcccctact 60
   ggctaatatt atattttcng ccccagtgtt tttaattaaa aatatgtgat agggaccana 120
   taagtaaagt acatttttct ccactaaatt tgaagtgagg gaaagaggag ccaaagtaag 180
   agattttttt ttaaggaaac ttaatctgat tgtgaaaatc atacatctgg agaaacatca 240
   gatcaggcaa tagagtcaga gggtcatgag caatagacga tgatgcgagg catttgggga 300
   gcttcctgga ggaaaattaa gtttttttcc tagcaaacta ccatgtccta caagaacttg 360
   gttatataat ggtgcgtccc tgaatcactg attaaaacca gttgcttctg attttaagtc 420
   acaggtttta caagtattca gctctccctc atgtttcatt tcntttttta agataatcta 480
   tcaacctttt ttaaatttta aaatttttag atgtanagtt tataagtaaa atatattttt 540
   agccattgtt cngttanctg aactgatgtg tttggtctaa anggcctgac ttcaaatacc 600
   cttgggatcn tgtnagggct ctacacaaac ttcantaagt ntgggaaatt gtatcctaan 660
   ggattgtata nanaatgcnt tcnntanaag tacatccact tccggangtc cccttgnaan 720
   cccaattgcn tantcagggg ttcaaanccn ttcanaaaaa taattcacta actgtccaan 780
   gggattttaa aacgtttacc ggtnnanaac ncnntnnggg tccttcaaaa ntcccnaana 840
   nacggttana aaacctcccg gnaaagn 867

<210> 67
<211> 879

<212> DNA
<213> Homo sapiens

<220>
<221> misc_feature
<222> (1)...(879)
<223> n = a, t, c or g

<220>
<223> nbla-00784-r1

<400> 67
gggnngtnnt gtggcttttt tnggcctttt acttatnang ctttnggcca ctnacgaata 60
taaaactgac agatattttc aaacattttt gcaaaaaaag taaaaccctc ccctgtcacc 120
cctccccaca agatagagaa taaggacttg gacagagaaa caatacaaat ctaaacatga 180
aactgtcgct catcggttgg tcccaagagg ctccaacata ttatattcta agtaaaggtc 240
cattaaatta agaagttaac aagccacttg acacttttat agtttaactt gccttctact 300
ctgattatga tgctcattca aaatgctgag agacaagaac aggtaacatt aacactgcac 360
gtgacaagat attcaaagtg cataaatcta tgaaaccaca aacattcata agaatactan 420
gcatgggatt aaacagtgac ttaaaaatta atccaaggga atctatgtac attttaacct 480
ttggatctgg tcaacatgtt tcctccttat gtagacatta atttggtaaa gtctantaac 540
caagattaaa tttatctgga cacatttctc atctgacaca aattgtccac aagccaantt 600
cacatgaggn aatttgggct gtttaatggc antctantgg aaattctcaa tgntacatng 660
gaattnttca tttaacatgc caatgggact gggtcaaaan ccttctaggg aacaacaaga 720
agtnntttca anagcatcct tcccaacaan aatggctcca tattcacctn ttaacangcn 780
ccatnnncaa ggggtccgtg tgggtaaaat ttnactttct nagggggttc aaccatccca 840
agggggggtt cggtttntaa ggggnaaggg agggaatan 879

<210> 68
<211> 2120
<212> DNA
<213> Homo sapiens

<220>
<221> misc_feature
<222> (1)...(2120)
<223> n = a, t, c or g

<220>
<223> nbla-00798

<400> 68
agtgttactt ctgctctaaa agctgcggaa ttcctcgagc actgttggcc tactggttaa 60
ctttatattt tcagaaaagt gtttttaatt aaaaatatgt gatagggacc aaataagtaa 120
agtacatttt tctccactaa atttgaagtg agggaaagag gagccaaagt aagagatttt 180
tttttaagga aacttaatct gattgtgaaa atcatacatc tggagaaaca tcagatcagg 240
caatagagtc agagggtcat gagcaataga cgatgatgcg aggcatttgg ggagcttcct 300
ggaggaaaat taagtttttt tcctagcaaa ctaccatgtc ctacaagaac ttggttatat 360
aatggtgcgt ccctgaatca ctgattaaaa ccagttgctt ctgattttag tcacaggttt 420
tacaagtatt cagctctccc tcatgtttca tttctttttt taagataatc tatcaacctt 480
ttttaaattt taaaattttt agatgtagag tttataagta aaatatattt ttagccattg 540
ttctgttagc tgagctgatg tgtttggtct tagagggcct gacttcagat actctttgtg 600
atcttgtaag ggctctacac aaacttcatt atgtatggta aatttgtatt cttatggatt 660
gtatatagaa tgctttcgtt agaagtacat tctacttctg tatgtccctt tgtaatccgc 720
agttgcttac tcagggtttt catagtcatt tcataaaaaa taattcacta gctgtctaat 780
ggtattttaa gactgtttat ctgtatcaca acgtcattag gagttctttc aacaattcca 840
taaatatact gtttactaga ccttccctgt aaatgttccc aattcccatc ctgtctcaga 900
cagtcaatag tcctgtgtac agtgactatt tgcatgattt ctcattgcac tgctgcattc 960
aggcactcca gggcatgatt aaacagtcat taacagtgcc tctctggtac agttgatgca 1020
tgcattgatc tttcttctct gctgttttta tatagccttt aattaaaagg aaaaaaatac 1080
cactactctg caatgcaaaa gtcttcaaaa ttctttgttt cctgtattaa tcacttctgt 1140
tccagagtga acaaatgttt tcagctaagc tatgtgagaa tgtaagaata atatcctgct 1200
tgttctaaat agttcatata tttaaagtgt ggtcagtatt tcctccctgt accttacaaa 1260
cagaaaccac cctgggatgg ttgatacccc ttacgaagtc gatcttaccc acacagactc 1320
ctgtgtatgc gtgtctgttt ataggtgtat atggagtcag tgttgatagg aaggatgctc 1380
tagaagtact tctgttgttt cctagaaggc tatgagccag ttccatggca tgtttaatgt 1440
ataattccca tgtatcatga gaatttcact agaatgtcat taaacagccc aactacctca 1500
tgtgaaattg gctgtggaca atctgtgtca gatgagaaat gtgttcagat aaatttaatc 1560
tggttaatag acttaacaaa ttaatgtcta cataaagaag aaacatgata gaccagatgc 1620
caaaggctaa aatgtacata gatttccttg gattaatttt taagtcactg tttaattcca 1680
tgcctagtat tcttatgaat gtttgtggtt tcatagattt atgcactttg aatatctgtc 1740
acgtgcagtg ttaatgttac ctgttcttgt ctctcagcat tttgaatgag catcataatc 1800
agagtagaag gcaagttaaa ctataaaagt gtcaagtggc ttgttaactt cttaatttaa 1860
tggaccttta cttagaatat aatatgttgg agcctcttgg gaccaaccga tgagcgacag 1920
tttcatgttt agatttgtat tgtttctctg tccaagtcct tattctctat cttgtgggga 1980
ggggtgacag gggagggttt tacttttttt gcaaaaatgt ttgaaaatat ctgtcagatt 2040
ttatattcgt tagttataat aaacttattt ttaaagtaaa aaaaaaaaaa aaaaaaaggc 2100
cacatgtgct cgagctgcag 2120

<210> 69
<211> 2134
<212> DNA

<213> Homo sapiens

<220>
<221> misc_feature
<222> (1)...(2134)
<223> n = a, t, c or g

<220>
<223> nbla-00830

<400> 69
ggcctactgg aaaggtattt aacatcctaa aacacttttg aaaaagcccc aatttatata 60
gaatctgaaa aacatgaaaa tgccagaatt aaatagtagt tgtttgtgat cccttgaact 120
ctgatcattt taattagcaa aaagcaataa tattttatat tccatacttc ccagaaagtt 180
aattgattca aattcttgct atgttatgcc gtcatgcaga tcaaagagaa cttgtacaaa 240
caaaaagata ttaactgttt tttctagaaa aacctttgtt agttatttag ctatagcata 300
atacacagta gaactcattt ttccgaagat gattgccata aagctttacc attttacaaa 360
gctctagcat tttaaaatgt tttcctaggc attctctcat tcagtcgtta cctcaaagct 420
ttgaagggag tataattatc ttcatttcac aaatcatgaa gctaatgaca tttcatggaa 480
gctattttta taggaattat ggaattggag ccctcctttg agtgtctttg gatgttgtca 540
ccaccatcaa caggcagtgt tatctccaac atgtttgatg agaaaaaaac ataaaggaaa 600
gggacttggg tttcatggca agagattcag cttagatggg aatgagctgg tctctgaggc 660
agagtttcct gtctcatctt tgtataaact ccttcttaga aaccagcaga ggctcttaat 720
gtacgtccat cttctccttc ccaccacaga cacccactcc tatgtgtgtt tctgaaaatt 780
acagggtcat ttgtggatgt gctttaattt agtatagata taatctgata tcattatctt 840
tctttccccc cgctccccgc caccagcatt ccccctgcac tcaatttgtg cttatattcc 900
ttttgctgta tatatacccc tggctcattg cttctggaag ctgcattact tattctagag 960
tgtgggtctc ctacaattta tttatatagc cccctaaatc agtggttctc aactgggggt 1020
gattttatgc tccccaggtg tgatttggca gtgtctggag acatttttgg ttttcacaac 1080
tggaggtggg cactgctggt acctaggtgt atagatgcca aggaggctgc taaatatcct 1140
accatgccca ggacagcccc cattacacag aatagccagt cccagagaaa ctcagcctta 1200
gattagtgtt tctccttcct ggctgcacat taaaattatc aggggagctt ttaaaataca 1260
ctgatgctgg gatccacccc tgaccaatta aatcagaccc tctgggatgg ggcctgggct 1320
ttagagtgtg tttttaacac tccccaggtg attctaatgt gcagccaggt ttgagaatct 1380
ttactctaag gactcaatct ttgaaagtta cagcattgta gaggggaagg atgatccaga 1440
attaattaaa gaaggaagaa gagtgaacag tactgaactg gatgcaagat tatgactgtg 1500
caaatcctcc tatttcagat ttaggttctt tactggtaaa ttggataaaa agttggcagt 1560
tcttttactc actggagcat gatgaagata aatgagttac ggttgcccta gacttttacg 1620
attagtaaaa ccaatggatg gacagaataa ataagtataa gcagacgcct atagtctaat 1680
atcgcaactc cagaaaatct ttgaggaaat ctcacacatg atgggagtgg gacagggagt 1740
tgttttgttt ttgcttccct tgagtctcag caaaagaaca gtcatagaaa atatttccaa 1800
aaaatttaga tataattttg cttggcaatt agaatatacc ctctgtcagc tttgttcagt 1860

tgttggacct attaaccaat ccataccaga ctaatttatt tctaaacaaa gtggcgaatg 1920
gtgtccaatg ccttttctga gaaagagaaa aatggcctct tcaaaagacg caatttggta 1980
tagacttgtc tggccgtttt gtagtgttct ggagtgggga gcatatacta ccctcacagg 2040
taaacgaaac cttacctgta tgtattgtct cacccatatc ctgctacagg agaaaaaaaa 2100
aaaaaaaaaa aggccacatg tgctcgagct gcag 2134

<210> 70
<211> 2146
<212> DNA
<213> Homo sapiens

<220>
<221> misc_feature
<222> (1)...(2146)
<223> n = a, t, c or g

<220>
<223> nbla-00831

<400> 70
ggcctactgg ttctgcaaga tgtcaagatt gttgaacacc ccagatggaa gcccttacac 60
ttggtgggtt ggcaaagcca acgagaagca ctactgctgg ggaggctctg ggcctggaat 120
ccagaaatgt gcctgcggca tcgaacgcaa ctgcacagat cccaagtact actgtaactg 180
cgacgcggac tacaagcaat ggaggaagga tgctggtttc ttatcataca aagatcacct 240
gccagtgagc caagtggtgg ttggagatac tgaccgtcaa ggctcagaag ccaaattgag 300
cgtaggtcct ctgcgctgcc aaggagacag gaattattgg aatgccgcct ctttcccaaa 360
cccatcctcc tacctgcact tctctacttt ccaaggggaa actagcgctg acatttcttt 420
ctacttcaaa acattaaccc cctggggagt gtttcttgaa aatatgggaa aggaagattt 480
catcaagctg gagctgaagt ctgccacaga agtgtccttt tcatttgatg tgggaaatgg 540
gccagtagag attgtagtga ggtcaccaac ccctctcaac gatgaccagt ggcaccgggt 600
cactgcagag aggaatgtca agcaggccag cctacaggtg gaccggctac cgcagcagat 660
ccgcaaggcc ccaacagaag gccacacccg cctggagctc tacagccagt tatttgtggg 720
tggtgctggg ggccagcagg gcttcctggg ctgcatccgc tccttgagga tgaatggggt 780
gacacttgac ctggaggaaa gagcaaaggt cacacctggg ttcatatccg gatgctcggg 840
ccattgcacc agctatggaa caaactgtga aaatggaggc aaatgcctag agagatacca 900
cggttactcc tgcgattgct ctaatactgc atatgatgga acattttgca acaaagatgt 960
tggtgcattt tttgaagaag ggatgtggct acgatataac tttcaggcac cagcaacaaa 1020
tgccagagac tccagcagca gagtagacaa cgctcccgac cagcagaact cccacccgga 1080
cctggcacag gaggagatcc gcttcagctt cagcaccacc aaggcgccct gtattctcct 1140
ctacatcagc tccttcacca cagacttctt ggcagtcctc gtcaaaccca ctggaagctt 1200
acagattcga tacaacctgg gtggcacccg agagccatac aatattgacg tagaccacag 1260

gaacatggcc aatggacagc cccacagtgt caacatcacc cgccacgaga agaccatctt 1320
tctcaagctc gatcattatc cttctgtgag ttaccatctg ccaagttcat ccgacaccct 1380
cttcaattct cccaagtcgc tctttctggg aaaagttata gaaacaggga aaattgacca 1440
agagattcac aaatacaaca ccccaggatt cactggttgc ctctccagag tccagttcaa 1500
ccagatcgcc cctctcaagg ccgccttgag gcagacaaac gcctcggctc acgtccacat 1560
ccagggcgag ctggtggagt ccaactgcgg ggcctcgccg ctgaccctct cccccatgtc 1620
gtccgccacc gacccctggc acctggatca cctggattca gccagtgcgg attttccata 1680
taatccagga caaggccaag ctataagaaa tggagtcaac agaaactcgg ctatcattgg 1740
aggcgtcatt gctgtggtga ttttcaccat cctgtgcacc ctggtcttcc tgatccggta 1800
catgttccgc cacaagggca cctaccatac caacgaagca aagggggcgg agtcggcaga 1860
gagcgcggac gccgccatca tgaacaacga ccccaacttc acagagacca ttgatgaaag 1920
caaaaaggaa tggctcattt gaggggtggc tacttggcta tgggataggg aggagggaat 1980
tactagggag gagagaaagg gacaaaagca ccctgcttca tactcttgag cacatcctta 2040
aaatatcagc acaagttggg ggaggcaggc gatggaatat aatggaatat tcttgagact 2100
gatcacaaaa aaaaaaaaaa aaaggccaca tgtgctcgag ctgcag2146

<210> 71
<211> 1878
<212> DNA
<213> Homo sapiens

<220>
<221> misc_feature
<222> (1)...(1878)
<223> n = a, t, c or g

<220>
<223> nbla-00832

<400> 71
ggcctactgg aaagaaaaaa tgtgctgcgt tctgaaaaat aactccttag cttggtctga 60
ttgttttcag accttaaaat ataaacttgt ttcacaagct ttaatccatg tggatttttt 120
tttcttagag aaccacaaaa cataaaagga gcaagtcgga ctgaatacct gtttccatag 180
tgcccacagg gtattcctca cattttctcc atagaagatg ctttttccca aggctagaac 240
gacttccacc atgatgaatt tgctttttag gtcttaatta tttcacttct ttttagaaac 300
ttaggaagaa gtggataatc ctgaggtcac acaatctgtc ctcccagaaa tgaacaaaag 360
tcatcacctt ttctgcttgc tacacaggca acgattcccc catcagctgc ccggaccctt 420
tggcctggct tggtgtgcag gcctgtctgt ttgcttaaag tcagtgggtt ctggtgcagg 480
gagtgagaag tgggggaagt gaaagggaaa gcatccgtga gaaagcggcc acggttttcc 540
ctccttgtgt gcccatgggg caccagctca tggtcttttt cagtcatccc agtttgtaca 600
gacttagctt ctgaactcta agaatgccaa agggaccgac gagactcccc atcacagcga 660

gctctgtcct tacatgtatt tgatgtgcat cagcggagga gaacactggc ttggccctgc 720
tccgctgagt gtctgtgaaa tacctctact ttccctccca tatccagaac aaaatgatac 780
ttgacatcct tccacaaaag tcagcctaaa gaagttatgg tatcatatgt taaactaagc 840
tttcaaaaac cttagtgaaa tagcaagtga ctgctttcaa gcagcagtcg acatgtaaat 900
gaaggtgttc ttagaattcg cattttgcca gctcagcgca cctccacaac gaatgaaatg 960
ctccgtatga tttgcacaaa tgacatagac ctccccaaaa gttaactggc tctccttcct 1020
cacacagttc atcataaccc aaccccccac ccccgggtca tgaaaatcac agaacttata 1080
aacacattga accctagatc tcaggcttcc tgacctaccg ccagtggccc cttgctggcc 1140
accctatagg gtcctccttc cctggcagcc ccccatgtgg gagaaatacc tgattctccc 1200
aatctgcagt gggagagctt tgctgaattc catcccaaag tcaaacatgg gcaagaggtg 1260
aggatttcac ttttaccctc aagtccgatt tgtctgtgat tttaaactaa ctgtgtatgt 1320
attgatgttt ggaagattgt ttgaatttta aagcgataat agtacttaat gttatccagt 1380
attgttcatt aaatggtgtt atcctaaagc tgcacttggg atttttacct aacgctttac 1440
tgattctctc aagcacatgg caaagtttga tttgcactcc gttcatttct gacacgtttt 1500
gctgcctcct acctttctaa gcgtcatgca aattcgagaa tggagaagga cgctgccggt 1560
ccctgagcgg tgtggagagg gcggaaggtg gactccagcg cagcttgagg ggctgaggac 1620
ggaggctgca gcatctgtgc cgttctactg agcacgcttc tctgcctcgc tcctgactca 1680
gcactttgtt cactggctca gcagttatgt ttacacatca tttttatgtt cctgctttgt 1740
aattcatgtt tgagatgggt ggccactgta cagatattta ttacgctttc cagactttct 1800
gaatagattt ttttgaataa acatggtttt atgaaaaaaa aaaaaaaaaa aaaaaggcca 1860
catgtgctcg agctgcag 1878

<210> 72
<211> 2291
<212> DNA
<213> Homo sapiens

<220>
<221> misc_feature
<222> (1)...(2291)
<223> n = a, t, c or g

<220>
<223> nbla-00908

<400> 72
cactgttggc ctactgggaa atacattttg tgtgcatatt gtttcatagc aagaattcgt 60
tgcaaaaatg ctttattttt gaacaatgct tggaaatatt atgtgacttt tttgtttgtt 120
tgttttagga ggatggtgta tggtgggggc aataaatgag gtttttttgca ttccaaggaa 180
atggcatatg gattaactgt aagaaatgaa ataagtaatt tattgtaaga caacatcaag 240
ccatggaaac ttggcagaag attcaaagca gcttaaacag cacttttaaa ttaactccta 300

agcgttacat ggttgtgact atggaaactc cagttaagac aggatcttat cagaggtgga 360
cagcgtgaag atttcctttt ccattttcaa taaactttgg aacaaccttc tcgtatctcc 420
cctagagttt cgtgcccctc tgaactgtct gttattgcaa tgtagtttat caacagaatt 480
tgtgtgtttt cgatttaagc taaaagataa tttaagaaca tttatttccc cttttcactt 540
taaaaaatta tgattattcc tattattgtt atgaaccttc ttattttaca tttgagggat 600
aaaggcaaat gatttgtgag tcttctagtt actggaccga gttttctgct ggatctggtg 660
ggaaggcagc tcggtaaagt ttccctcctg ctccccccgc ccgactttga ctctgaatca 720
gcatttggtc ctattcagag gactcttacc acaacgtttc tgttctacac ttgggtggag 780
accagttgac catagagcat ttgcagagcc tcattgtttg atttcttgtg actattctaa 840
gaatgaatgc aatcagattt taaaagtaac taaatatact tcagcacttt tttgctttaa 900
actagatcat cttagacttg tttatacctt ccagatttga ttgttttact cccaatgact 960
gcactatatg tatgcataag accacttttg agcgctgtgt tccccttct gagtagtcct 1020
ttgacgacgt gttgtgtttt ctgatgttga cttgagttcc atttagtagc atctcttcct 1080
tccatgtctt gatgttatgc aggaagtaca gacgtacttt aaatttttgt tatgaaataa 1140
aaaaaagatg ggttttgtaa aaataaaaaa aaatattttt agcagaacag gacttacagg 1200
gtcattgtcc ccacaatgtg ccagtcgact atttgcactt accttgtcct atatatccgt 1260
acggaggtgt gcaattcctc gtgtcagtag ccttgtgaca ctgaacctgg atggattata 1320
gaggagccct cacggctgat caataatgtt gcaaagggag actacaggga tctcacgacg 1380
aatattctga tacaatactc aacctcggta tatatatatg tgtataaata tatgtatatc 1440
ccagcggcac tttatactgt tcactgtaca aaagcttaca gttttccaca aggactttaa 1500
taactagctg ggaaaagacg atgtaattat ttcggggctc tgcggaacca tctctgtaca 1560
gcgccccctt tctgttgtgc tattggttgc agctgccatg ctcagaatgc gttttgagag 1620
ctgaagcaag gtgcttgcag tcacctgagg ccgtccgtgt ggcccagggc cccagctgcc 1680
tttagggccc ccattgttca taacagcata tgcatttccc caccgcgttg tgtctgcagc 1740
ttctttgcca atatagtaat gcttttagta gagtactaga tagtatcagt tttggattct 1800
tattgttatc acctatgtac aatggaaagg gattttaagc acaaacctgc tgctcatcta 1860
acgttggtac ataatctcaa atcaaaagtt atctgtgact attatatagg gatcacaaaa 1920
gtgtcacata ttagaatgct gacctttcat atggattatt gtgagtcatc agagtttatt 1980
ataacttatt gttcatattc atttctaagt taattcaagt aatcatttat taagacagaa 2040
ttttgtataa actatttatt gtgctctctg tggaactgaa gtttgattta tttttgtact 2100
acacggcatg ggtttgttga cactttaatt ttgctataaa tgtgtggaat cacaagttgc 2160
tgtgatactt catttttaaa ttgtgaactt tgtacaaatt ttgtcatgct ggatgttaac 2220
acatcttact ctaaataaac aaggtgttgc caaaaaaaaa aaaaaaaagg ccacatgtgc 2280
tcgagctgca g2291

<210> 73
<211> 852
<212> DNA
<213> Homo sapiens

<220>

<221> misc_feature
<222> (1)...(852)
<223> n = a, t, c or g

<220>
<223> nbla-02890-f

<400> 73
gagnagnnna gntggngngg anantgnnat ttgantcctc tncctgttgg cctactggag 60
atgcttctgg gtcgcggtgt gctaagcgag gagtccgagt gtgtgagctt gagagccgcg 120
cgctagagcg acccggcgag ggatggcggc cacngggacc gcggccgccg cagccacggg 180
caggctcctg cttctgctgc tggtggggct cacggcgcct gccttggcgc tggccggcta 240
catcgaggct cttgcagcca atgccggaac aggatttgct gttgctgagc ctcaaatcgc 300
aatgttttgt gggaagttaa atatgcatgt gaacattcag actgggaaat gggaacctga 360
tccaacaggc accaagagct gctttgaaac aaaagaagaa gttcttcagt actgtcagga 420
gatgtatcca gagctacana tcacaaatgt gatggaggca aaccagcggg ttantattga 480
caactggtgc cggagggaca aaaagcaatg caagagtcct ttgttacacc tttaaagtgc 540
tcgtnggtna atttgtaagn gatgtcctgc tanttncana aaantgccna gtttttcaca 600
aanagcnnat ngangtntnt tagaatnaca nncttgnaca ccgnnntnaa aanncntntt 660
ttnntnangg antnaccttn ttnnnttccg nttctgttcc ntttggntta accnnntnnn 720
tngnntnnaa tatntnnnnn nnccntnann annaanttnt gttttnnnna nataannntn 780
atnnnntnnn tnnntnatnn nntntnnnan nnntnnnntn nnnctntntn nnnntntntn 840
tntntntnnc cn 852

<210> 74
<211> 862
<212> DNA
<213> Homo sapiens

<220>
<221> misc_feature
<222> (1)...(862)
<223> n = a, t, c or g

<220>
<223> nbla-02890-r1

<400> 74
gaggngttgn anntttgtgg cctttttntt tnttcttttt ttggtggaag ttaaagtatt 60
tattgatgtg tttaaactgt gtacattctc cacagatcat attaaggagt ttgtaggtga 120
agtttaatct gtgcataggg ggtagagaca tgaatagggt caaaggggag gaaaaaggaa 180

aaaaacaaaa caaaaacagt cacaggaaaa taaaaataca ccacaggtta ccagaacctt 240
cagatttaaa ataaaaagaa agaaaaagca gaagcagtga gcatcgacat caactgtaca 300
agcattacaa agactcctgt gacgaaaaca caattgttca aagctttcca cagtgtccac 360
agactcagtt tcagggacag cctgnactac nttcctttca cacaaacaaa cctncccgtg 420
ttccctcctg ggccagcggc cgncttcttg gtgtggtctt ctttgagtga atgtcacaan 480
ggcccgtnac atggaagggg ttggnngtng atgggcaaag tttangccca nggntnaant 540
gccttttnan aaaagnccan anangatgaa ccctgcncnt ntggacctta nttgnntggg 600
gnattgaccg nttaaaanct tccnnnnnng tnatcatctt ncntttnnat ntcaaattta 660
ancttcnngn tnatnncttn ngaatcctgn nttctnnnna nnttcntttn tgnctccctt 720
anttttnncc tttntttttn aanactntgn attnatncnt ttntcgtccn tngntnnttg 780
actttttttn nacccnctgn tttcntcctt ntaacttnnt ntttnnaann ggnccttnan 840
ntcttttnnt ttntnnntna nc 862

<210> 75
<211> 851
<212> DNA
<213> Homo sapiens

<220>
<221> misc_feature
<222> (1)...(851)
<223> n = a, t, c or g

<220>
<223> nbla-02969-f

<400> 75
nnnnttnnnt ttgaattccc gttcctgttg gcctactggc aaattagaaa atgaattgag 60
gaccacaaag agtgaaatgg cgcgatacct aaaagaatac caagacctcc tcaacgtgaa 120
gatggctttg gatattgaga ttgcagctta caggaaactc ttggaaggcg aggagacccg 180
actcagtttc accagcgtgg gaagcataac cagtggctac tcccagagct cccaggtctt 240
tggccgatct gcctacggcg gtttacagac cagctcctat ctgatgtcca cccgctcctt 300
cccgtcctac tacaccagcc atgtccaaga ggagcanatc gaagtggagg aaaccattga 360
ggctgccaag gctgaggaag ccaaggatga gcccccctct gaaggagaag ccgangagga 420
ggagaaggca aggaagaagc cgaggaagaa gaagcagctg aagaggaaga agcttgccaa 480
ggaagaatct gaagaaacaa aagaagaaga agaaggangt gaaggtgaag aaggagaagg 540
aacccaagaa gcttgaagag gaggagaaag aaangttgaa ggtgcttggg aaggaccagc 600
nactttagaa gaaanattgg acccccnntt tccttaatta tttcagggat aatcttcccg 660
aattaggtna accccattan ccaaccaacc aacccnttga gnttccnaat tttttgngga 720
attaaaaagc natttttgnt tactttgnaa tgcttaggtt ggcnattcaa ngtgggcttt 780
naattccnct ttntccaaan tttgntnctt caaaaggctt ttggcttntt ncaacctngc 840

atggncaccc t                                                    851

<210> 76
<211> 817
<212> DNA
<213> Homo sapiens

<220>
<221> misc_feature
<222> (1)...(817)
<223> n = a, t, c or g

<220>
<223> nbla-02969-r1

<400> 76
   ggnggngntt tnctnnngan gcccttttga aannccctta ggttttttac tattttatta 60
   tggcacacag gatagaggat ggtacagttt tcttacttca accaagtaat tctcaaagca 120
   tccagctatt tccatttggc taaagttact ttttgcacat agcttgcatc tgtttgagac 180
   ttaccatgta catcaaccca ggtctagtaa gcagaaatgt gaaaagtttt gtttctgagg 240
   agacgcctca tctttacaga agccaataca ctgagagcct tcatagttcc aatccattac 300
   catcatggca aggaagcact ttacctattt gcatagcaac atatatttaa ctagaaatag 360
   gtggtcaaag ggattaagta actttaaatg gagaccactt tggtttcagg ttaaattaat 420
   aacttataga gatcgctaaa aaacaaatat tgaatgaaat tagctgacaa agcaattggt 480
   tcaagaacaa aggcagaata gcagatagta atatcatcta tatttattnc acatcaatgc 540
   cagaaccgtc ttaactttac gacngaaagg atcntggggc cgggatttga tgcaatgntc 600
   aancnggcaa gctntcattg gaatccaatn tttccagtng ancatgccna caatggcaag 660
   gacntacagc ctttaccttc cccttaaggg ctataattca ttactgggct ngaccnttaa 720
   aggttaaaag gggnncntta aggggnttac tgnggaccna ttcccttana cccctaatnt 780
   ggcccaggat gggggactnt tccnggangg naanacc                        817

<210> 77
<211> 866
<212> DNA
<213> Homo sapiens

<220>
<221> misc_feature
<222> (1)...(866)
<223> n = a, t, c or g

<220>
<223> nbla-03002-f

<400> 77
   gngnnnnnnn nggngnnnnn nnnnnggcng natcccgagc acgtgggcct actggaaaac 60
   ttatgagtgt gttggatgtg ggaaatcctt taggcacagt tcagcacttc ttcgacatca 120
   gaggcttcat gctggagagt aaaatttgga atataatgag tatgggaaga tttgtatgga 180
   agcacctttt ttttctccta aattcctagg aatgtaagac tcaatctgta gctagtatga 240
   atattttgta ttttgaacaa gaaatgttgt gtcctaatgt gtcctagtct ggagtttgat 300
   gccttatctg ctgctcagtg ggtgtaacct tatcccttct gagcctcagt ttccccattc 360
   cccccaatag aaaaatgggg atatttccta gggttgtcct gagaataaaa tgagttctgt 420
   gtgacagtta actgccatta attaattagc cgttctaaaa gtttatttac aatatcaaaa 480
   tctgacaagt aaatcagcat tgttgtttgg tctcataatg tattgtttct taagaacatt 540
   ttgtaattgg gctatgattt tatgatctac agacgtctat ttaaactata atgtcctggn 600
   ttaaatagga taatacaatc taagactggt ttgtccttga gaaatttcag atttaatcag 660
   agaaaaatgt actgattggg atttgagtgg acattgtggg ggtgtattct ggatancctc 720
   aagatctcaa ttaaantttg ntacnttcca atactagatc taaattgatt ttaagctcca 780
   agttcacttt tccaattaaa cgggaagcct ccctgacata actgaataaa acctcanggt 840
   tgtgggactc cgggtgngtt taaggt 866

<210> 78
<211> 872
<212> DNA
<213> Homo sapiens

<220>
<221> misc_feature
<222> (1)...(872)
<223> n = a, t, c or g

<220>
<223> nbla-03002-r1

<400> 78
gngntgnnnn tgtggctttt ttttngnttt taagacgtag tctcgctctg tcgcccaggc 60
tggagtgcag tggtgcaatc tcagctcatt gcaaccttca tctcccaggt tcaagcaatt 120
tctcctgcct cggcctcctg agtagctggg accacaggtg ccctccatca tgctggctaa 180
ttatttttgt atttttagta gaggtggggt ttcactatgt tgaccaggct ggtctcgaac 240
tcctgacctc aaatgatcca cccacctcgg cctcccaaag tgctgggatt acaggcatga 300
gccactgtgc ccagcctgag aattacctta taaatatctt gcacatgtgc ctaaagacaa 360

ggatgctgta tatgcagttt tgcttacaac agaaaaataa aagccattaa gtgtccacca 420
atagggaact aataatggtc tattaagaca atgacacact gggcaagggt gggggaaaga 480
tcttatttac tgatacaaga acaatctgtg agatggttaa tccttaaaaa atggncaact 540
gtgtatatta tgctaccatt tcnttaaaaa aagaaaaaag aaaaaaaaag tnaagagaag 600
ggatcaagga ttgagaaaat aagccacaac ctgggangga aatatttgca aaagacctat 660
ctgataaagg nctgttatac aaaatatnca agggacactt naaattcaac aataagaaaa 720
tgagcaatcc aattnaaaat gggcaaaacc tccaacaagt acctcaccaa aagctttcag 780
atggnaaaca tntgaaaaga tgctaaattc aaatattant ggggaactgc agnttaaaac 840
aattnatncc actacattcc tatttnatan tt 872

<210> 79
<211> 853
<212> DNA
<213> Homo sapiens

<220>
<221> misc_feature
<222> (1)...(853)
<223> n = a, t, c or g

<220>
<223> nbla-03037-f

<400> 79
ggnngnnnnn nnggnnnntt tnnnngggcc nggatcctct agcactggcn gcctactggg 60
ccttcgcgcc ggcccttccg cgggtgatca gctggtctgc gctcccctga cgtgggctgg 120
ggcacgtcac cgccgaatgg cagcctccag aaagccaccg cgagtaaggg tgaatcacca 180
ggattttcaa ctgagaaatt taagaataat tgaacctaac gaggtgacac actcaggaga 240
cacaggtgtg gaaacagacg gcagaatgcc tccaaaggtg acttcagagc tgcttcggca 300
gctgagacaa gccatgagga actctgagta tgtgaccgaa ccgatccagg cctacatcat 360
cccatcggga gatgctcatc agagtgagta tattgctcca tgtgactgtc ggcgggcttt 420
tgtctctgga ttcgatggct ctgcgggcac agccatcatc acagaagagc atgcagccat 480
gtggactgac gggcgctact ttctccaggc tgccaagcaa atggacaaca actggacact 540
tatgaagatg ggtctgaagg acacaccaac tcangaagac tgggctggtg agtgtgcttc 600
ctgaaaggat tcaaggttgg tgttggaccc ccttgatcan tcctacagat tantgggaag 660
aaaatggcca aagttctgaa aaantgccgg catcaacctc antcctgtca aaggagaanc 720
tcgttgacaa aatcctggca anaccgtcct taaacgcccc ttgaancctc tcctcaaaat 780
ggggcctggn tttnaaaagg aatctnctgg aanggcaaag gtncaaaccn ttcggttnaa 840
aatggttaaa agn 853

<210> 80
<211> 836
<212> DNA
<213> Homo sapiens

<220>
<221> misc_feature
<222> (1)...(836)
<223> n = a, t, c or g

<220>
<223> nbla-03037-r1

<400> 80
ggntgnnnnn tgtggctttt tttggccttt tcacagttct gacnttttta ttcactgatc 60
ataaatatag tctcatgagc attaaggtga tcatgaatga tgtactagca cctggaacat 120
gaccatcatg gagcactcac tgattcccca tcactggcca aagaagtcgg ggcatcttcc 180
tttcaccaag tgttcaactt tggagggacc ctccttctgg tgcagcaatt ttattcttgg 240
cttgttctca acaattaaaa aatcataaaa gactgagtgt ttgcaataaa atatcaaaga 300
ggataagaag attttctgtt cttcttaaag tctaaagtaa aaaggggagg tagggaagaa 360
ggaaaggaaa ggggaaagat gtcagggatc tgccacgttt cttccttcct ccagagcatt 420
ttacaaaaac aaaaccgggg aggtatttat taatgctgtt tggagatggg ttgcgtctct 480
ctgatgagcc actcgagagc ttcctggcgg ccctgtttct gcaattcctt cccaatcaca 540
tccctgcang tcaagtggta attgttgagc caatcgcact ccttgtccgt aagagaatcc 600
acatctatca atttggtctg aattgggang aatgttanan ggtcaaaggn caagcttccc 660
cgggntatta aaaattatac ttggncctca aanggaccac aagggcaaca atctcaatgc 720
gaanttcaaa agcccaacct caaaataacc cggntcaaca antggcaaat aatgccggcc 780
tccaanggnt caacaaanaa atgttttgta actgatgccc caaggncccn catggn 836

<210> 81
<211> 1895
<212> DNA
<213> Homo sapiens

<220>
<221> misc_feature
<222> (1)...(1895)
<223> n = a, t, c or g

<220>
<223> nbla-03066

<400> 81
gaagtgttac ttctgctcta aaagctgcgg aattcctcga gcactgttgg cctactggaa 60
accaataatg attgttctgt ttttctcacc ctactatgtg aatatatatt ctgttagaga 120
tgggctgtta tctgcaatgt ggactcaata ccatgggctt gagtttggtt agttcatcat 180
tagagatttt ttttcagcag ttggtttact ttatgagaaa ggatcatgag tttaattttc 240
ccactgatat atttgtgttc tcattgctga gatataagtt tcttaatgat ttgttacctt 300
tttctatttt cctagactag tattaaaatt ttgatggcat atgggtttgg ttccacttgt 360
agagaataaa tgttcacaac ttactggtat tagcacacac acatcacatg cagaaatggt 420
gttttcttgc cacttactct ttctcataag atgcttatat tcctgtgggc atttgtcttc 480
cacactacag cacataattt gatgaaactg attttctgtg caagagcttg tgtttctcca 540
actctggttt tatggaccac ttttcattgg tatataggag gcggttaaaa cgtggtggta 600
gaatgcaggt tttaggttag ctgggtgtga agaatcccag gtctttggga tagtcttggc 660
aagttactca actcctctaa gttagtttct tgatctgtca gatgacagta aacctcagag 720
ttgtgaaaat taaatggata taatgtgtgt aaagcactta gcataatgct ttatacatcg 780
taaaactttc aaaaatagga gtagtaatta ttacaaagaa atgtgaagaa gagtcctagt 840
gggctgcatg ttaaaaatag tttttagtta atgaaaatta aatcacatct tagatgttta 900
tcatctgcat ggtacttttc tggttgctgc tttaaccttt gtatagttga aagtctgtaa 960
actttctagt attctttgat ttctggccca tttccctcct accattcacg cttatatttt 1020
ttggttcaag tcaccattcc ttgcaaaact aattaaatac attcctaaat acataatatt 1080
gccaatattc attcacatcc accacgtcta gatccaagtc ttcatcttct ttatgctggt 1140
ctaatttttg ctgagcctta gaagcaaaga cagaccccgg gggttgatat gggacctcca 1200
gaatggggat tgcacagaat aatcttattt ttcctctatt cttctagtct gttatgttct 1260
gaacccacac atgttttcgg aaggaccaga tggctgacac tggctaatgg gaaccaaaag 1320
acgagtgaaa gtaacctggt tgtcacatac ccccagctta gaggtgcttc accctgcccc 1380
aaggtcaatt tctttttctt ctagagactt taccccatta tatcaaaaac ctttactaca 1440
gcctgacttt atctgatgtt agagtatcat taagtcaagc agcagcattt attgagtgaa 1500
aaaagattgt acacaggact ggaagagaag catctgcctt taaaatacag tagaaggccg 1560
ggcatggtgg ctcatgcctg taattctagc actttgggag gccaaggcag gtggattgcc 1620
tgagctcagg agttcgagac caccctgggc aacatggtga aaccctgtct ctcctaaaaa 1680
aaaaaaaaaa aaattacaaa aaatttgccg ggcatggtgg tgcacacctg tagtcccagc 1740
cactctaaag gctgaggtac aagaattgct tgagcccagg gtcagaggtt gcagtgagtg 1800
gagatcgcgc cgctgcgctg cagcctgcgc gacagagcga gactatgttt tttttaaaaa 1860
aaaaaaaaaa aaggccacat gtgctcgagc tgcag1895

<210> 82
<211> 842
<212> DNA
<213> Homo sapiens

<220>

<221> misc_feature
<222> (1)...(842)
<223> n = a, t, c or g

<220>
<223> nbla-03096-f

<400> 82
gggnngnntg ntgncgcntt ttnnnngggc ccgantcctc gagcangggt ngcctactgg 60
tgtcatgtag agcttcacat gaggtgggtt ttgtcgatta cgcccccttg gaggtgttta 120
tcatgtgcct ctgtccccgg tatttcctat aaattgacag ttacatctag gtgcctggtc 180
agattctggt tcagctttta cttttacttt tttggcaaga gcagttcacc aagggaggtg 240
ctaatgtcca actcgccttc tgtgatgcca gcagcttcct gatcagaggc tgtggaattg 300
actgtattgt gttgatgttt tataaagagt cattcattac cttttagtct tgcgcttgct 360
ggtagaacaa gatgttgtag gctcgttgga aacatttttc cccagacccg gtcatttatc 420
caaagacctc cagatcttct cagtgggaat gacatgagga gaccacaact gggctctctg 480
tgttcagttt tttccatgga caggtctaga aaccatgtat tttacgataa aagacactgg 540
acttatacag atgcttccaa gttcaacatc aagactagaa gactattaaa aaattgcaaa 600
taactctatt tctgccaaaa gtacttttgg gacagattcc taaaagtgga attgctggtc 660
aaagggcaaa cgtgtctatg acctcgccag atactgccaa gttcacatga tgaggtcatg 720
ccanttgccg caanccatgc cacgggagaa ttcanccctc cccaacttct ccagctaaac 780
ttctgantta ccaagaggca ntatccaatg ngcctttaan tcaaanttcn ccggtaagtg 840
tg 842

<210> 83
<211> 839
<212> DNA
<213> Homo sapiens

<220>
<221> misc_feature
<222> (1)...(839)
<223> n = a, t, c or g

<220>
<223> nbla-03096-r1

<400> 83
gagntgnngn tgnngctttt anggnccttt ttttttgaa aanttaaacg tactttaaac 60
tttgcatgag aagaaaacaa gaagcaaagt gaaaagtcct gtgacagact ggaaaaaata 120
ttggcaatta tcaaaatgaa aggttgctat ttaatatatg gtttctaaag taaagaatag 180

cataatttt atccctatag aaaaaaaat ctagagatat caaccagtag ttcacagaaa 240
aagaaatcca tcacagtagc caagatatgg gatcggtcac agatgagggg ataaacagaa 300
catggtccgt atccacagca gagggctgta cagcctggtc tgtttgcaca gcggagtgcg 360
gtacggcctg ctctatatac acagcggagt gcagtatggc ctggtctata tgcacagtgg 420
agtactgtac ggcctaatct atatacacag cggagtgcac tatggcctgg tctatataca 480
cagcggagtg ctgcatggcc tggtctacgt acacagcgga gtgcantacg ggctggtcta 540
catacacaac ggagtgctgc acggnctggt ctacatacac agtggagtgc aatacggcct 600
ggtctacata tacaacggag tgcaatacgg nctggtttct atacacancg ggagtactgt 660
ncgggctggt ctatatacac agcggagtgc tgcacggnct ggtctacatg cacaagcgga 720
gtactgtacg ggctggtcta natgcacaag gggggtgctg tgccacctgg tccatatgca 780
cancaaaant gctgtgttgn ntcaaccnat atacacacaa atngnggtgn cggtnnntc839

<210> 84
<211> 846
<212> DNA
<213> Homo sapiens

<220>
<221> misc_feature
<222> (1)...(846)
<223> n = a, t, c or g

<220>
<223> nbla-03107-f

<400> 84
ggnnngnnnn gngnngnntt tnnnngggcc cgnatcctcg agcactgntg gcctactggg 60
agtagctcag ctcctattcc tgggaagcct ggaacgggga cttttgaaaa taactgcagc 120
ggcattcggg ttagggtccg tgctctccgc ctgcgccagg acagggtgaa gtggtcgggg 180
cgagcagagg gtgcgaaggt gcgggtgctg gtgcctcgca gcaggaggga gccccggctg 240
cgccgcgcga ctccctcttt ggccctcgga gcgcagcacc cggcggacaa gcggcgggac 300
gccaggacgc ggcgagcaag atctctcgtg gaagaggaag accaacacat gaaattgtcc 360
cttggaggca gcgaaatggg cctctcatcc catttgcagt cttccaaggc aggacctaca 420
cgcatcttta ccaagcaata cccacagttc tgtggtgtta cagggctttg accagcttcg 480
acttgaagga ttgctttgtg atgtgaccct gatgccaggt gacacagatg atgctttccc 540
tgtgcataga gtcatgatgg catctgctag tgattacttc aaggctatgt tcacagaatg 600
aaagaacaag atttaatgtg cattaaactt catggtgtga gcaaagtcgg tctaaggaaa 660
attattgatt tcatttatac tgcaaagctt tctcctaata tggacaacct tcaagacacc 720
tggaanctgc caatttccta cagattctgc cagttttgga cntctgtaaa gtgttcccaa 780
aaccggggtc actttaacaa ctgtgttnaa tttggccggn ttgcaaanac tacaaatcta 840
accgnn 846

<210> 85
<211> 836
<212> DNA
<213> Homo sapiens

<220>
<221> misc_feature
<222> (1)...(836)
<223> n = a, t, c or g

<220>
<223> nbla-03107-r1

<400> 85
   ggntgnnnnt gggctttttt tggnctttga cattaaaagt ttttattggn cacaaaaaga 60
   taaaacatgg aagttgaatt tactgagcaa aagcagctct ccaggtgaag ctgctatact 120
   ttgtgctaaa taaccttatg aactgagtat acagaataca tataatatgc aagttacctc 180
   aacagcaaag gagaaggagt agaatacagt ttttgaagat aaaatctggt caagtgacaa 240
   attttgttgc tcaaaatttc tagcccttat ccacctaaat tctgtatggt tctacatata 300
   tgcattcagt atgtgcatac tgaattccca ttttaatgga agctgctttt tggaagaatt 360
   ctttttaatt tcacatttct ttgatgtgcc actcaatttt taaaaaaatt atatttgaca 420
   tatgtgcatg tgtgtatgtg tatgtatgta tacacacttt aaaaacacca aacccttgtt 480
   tataagtaga gggttcatgc tgctttttaa attaatatta gtgaatttaa gctacttctc 540
   ctgtgtgtct aggaaacttt gtgttctcaa tgcacccaca cagtcaagtg ggttgacaga 600
   tatgtcaaaa atacnttatg aaaagaggga ggtagctcat gcgagttggc aaccttttgt 660
   gtaatggttc ctgttcaagc angctgcctc cctttgacat cctacagtca aagatgaaan 720
   gggaaacttt tacntgaagc ctantggagc acaagttgta canttacaat aatccacctt 780
   caacttggct tatggggntt acnaangtaa ggatgncaaa taccttacac caatan 836

<210> 86
<211> 2896
<212> DNA
<213> Homo sapiens

<220>
<221> misc_feature
<222> (1)...(2896)
<223> n = a, t, c or g

<220>

<223> nbla-03113

<400> 86
ttctaggcct gtacggaagt gttacttctg ctctaaaagc tgcggaattc ctcgagcact 60
gttggcctac tggggaacag cattatgata gatttttcag tgaatatgaa aagttacttc 120
attcagaaaa ttatgtgaca aaaagacagt cactgaagct tctcggtgaa ctactactag 180
atagacacaa cttcacaatt atgacaaaat acatcagtaa acctgagaac ctcaaattaa 240
tgatgaacct gctgcgagac aaaagtcgca acatccagtt tgaggccttt cacgttttta 300
aggtgtttgt agccaatcct aacaagacgc agcccatcct agacatcctc ctcaagaacc 360
aggccaaact catagagttc ctcagcaagt ttcagaacga caggacggag gatgagcagt 420
ttaacgacga gaagacctat ttagttaaac agatcaggga tttgaagaga ccagctcagc 480
aagaagctta atctccaata aacatctatg ttaaatccaa attcagcatt tgctgttagc 540
tattcagcat caggcactca ttgattcatg aggaacatta ctgctaatct gctgttaagt 600
gaacggtttt tcattttacc cttttgtttt tcagtccagg ttggagatcg tagctgctgc 660
tgcttgcaca ctagggcaca tgtgggcttt ctcttgatct ttgtgtcatt tcagaattca 720
aagactgtgc tacgggagtt ctgaacatgg ctgggttcat gaaggcaaat gtatggatga 780
gagtgtggtt taggaaagag ggcactgata tcagattaga cctatgtgtt tgcacccatc 840
tttgttggcg atctgagtgc agtgtggcaa gtgcacacct ggcatccctg cgtcagatcg 900
cgcaccttca ggtcgcgcac cttcgctgaa ggaagatgac gcagagcttt atctgaaatc 960
agaggggagc tatccaaaat gggagtttgg gggcagctaa agttgacatg cgaataaatt 1020
gatactgaaa cttagcaact tcttaaaagt gtaaagaagc ctcataagat cataaggaaa 1080
atgtatatat gcttttcaca gctttctaga atttttgac atttgatttt cttgagactt 1140
gtaaacctgg atatgttgaa gggtatttgt taattttact tttcaaagat actttaaaac 1200
agtagagcta gcaatgacac cttgcatttc atttcaacac tgcttcaagg tttcttttgt 1260
atataattct tagaatgctc atttctttta aatggtttaa tttgtacagc agagaaatgt 1320
tattgtagta gtatgtaact attacctaat actgagtttt tgcaaaaaac aatgaatgct 1380
catatgtaat tgaaatactt cagatcacat gaaaatgctg atttaacatt taagtatcac 1440
agcattaaaa gaaaaagaaa gtaaaccagt cctttgtatt cagttaccta atggggtgcc 1500
atcaataagc tgcgatacag ccctggagct cagtcagcca caccttcctg catcctattg 1560
gccttattca ttttaaatga gttaatgaat ctgccagatc tgtgaatgat agagattatg 1620
ctaaattaat gctgattctt tgtgtgtgtg ggaaatctct gtagagcacc ttttctttct 1680
tagactaagt aacccagtac aatagttgtg aactgaataa ttaaaacttt ggcttctctt 1740
aggaaaagac gacttcctag tcataggtgt cctatgggga aatttatttt ttttaatgtc 1800
ctgttcctta atgctgcaaa ttatcagtat ttataaagta actgattttg caccactttt 1860
ttgttactgt gaccacggca gaacaatgtc ttctagacta tatctatgta aagttattag 1920
aatggtatct gttcatttta gtgatatgaa gatcacaact aacaactgac aaatcagagt 1980
ttgccagttc aaattcagca tggctgcagc tgattaagaa attgatatga ttattctttg 2040
ctagcctctc ttactaatgg aattatatac tggccagtaa aatgggcctc ccaattgctg 2100
tttcagcagg ttttaaacct tcaggaacac cagttaggaa aatagctcca gaaaatatag 2160
atatatttta tttttattaa aatggcagtc tacatcataa ttggcatttc tcaagactgt 2220
ctttaccaga atctgtgtga aataaggcaa tctagtctcc ttgaaaaaaa aatctcttgg 2280

atgtttagga aggaagactt ggccgtgatg tggtgtcctg gctttgtggt gtagtgctgt 2340
gtgtatggag ttagtgtaaa aacatggatt acaccaagtg gaagaaacgt cttcttgcca 2400
agctcattct tagaacttac acatctagaa cagcttccac tttggcagtg aggtcgtagc 2460
cttttaggtg gaagaagtga gggtgcagcg tgtcagacac aacattcatg ttactcttac 2520
attggaatct gaaggtagtt cagacttcgt gggttttgtt tttaagcaaa acaatgtgaa 2580
aacatttaag tttgaaatgt tgcatttgaa gttatgatca tttaatatat tcatattacc 2640
aagactatta tactggaagt ggtttttgtg ttataaaggt ttaattttac ataaggcagt 2700
tacttaatgt gatttttaac ccttaaaaaa gtggagatgt atacatttgt taacaatgcc 2760
atgaaagcat tttctttctc ctaaggaaaa gtgaatttct tatcagaata tctggctggc 2820
cctgtaattt aaattaaaat aaaattttgg agaaacaaaa aaaaaaaaaa aaaggccaca 2880
tgtgctcgag ctgcag 2896

<210> 87
<211> 779
<212> DNA
<213> Homo sapiens

<220>
<221> misc_feature
<222> (1)...(779)
<223> n = a, t, c or g

<220>
<223> nbla-03119-f

<400> 87
   gagggggnnn nntttgantc ctcggcactg ttggcctact ggtttatctc aattttcaac 60
   agatttgctg caatagaaaa gttatctttt tttagattta agagctccaa atagcactgt 120
   agcctacatt aagcatcaag tgccccgttc ccttcacaaa agttttctgg ttcattcgag 180
   tcactgaatt ccaaataatg cacattgggt taccaacatt agtcgccact gtcatattca 240
   gaaattaatc ctaaatggtt tacagtcctt ctcatttttt ctctttcaat tatgtgacat 300
   agtccacact tttcattaaa gttattaata atataaggat cttaggtggc aattttctgc 360
   acagctgggg aaaagttgca tattcagtgt angtatgcag atgcttactg aatcccagag 420
   acataaatgg tttattccca ggaatagtcc taccttgcta ttggttaata aaggnctaaa 480
   ttagattggc atcagtncaa caaagcattc atttttattt atgcatctcg aatcactttt 540
   atacatatgt naaatgaaca cantttgaga gnttaataag gtttataaat tatacttcta 600
   gataaatgca ctttttgaga aagacaagaa aaataattat tttctgatgg atttccatta 660
   gccaaanggg cctanaacat aaccagggca aattattgga atgcccatna gggaggactt 720
   nccanattgc tttcctttgg nacaggaact ntttgaacgn nanggntttg aaaatnacc 779

<210> 88
<211> 734
<212> DNA
<213> Homo sapiens

<220>
<221> misc_feature
<222> (1)...(734)
<223> n = a, t, c or g

<220>
<223> nbla-03119-r1

<400> 88
gagttttgtc gcaatttcta gtagtgacaa gtagaagtaa tgctagaaga cttgtgccat 60
tagtgcattc anatcactga atttttcatg atcctgcttc cattcacaag tcctttattt 120
tacactggtt ccaactttga ttttccattg gtattttgtc agaccaagcc atgtgactct 180
ttcttccttg gatgcaaaac caagtagttg gaaaaatctt tagggcatct gtaatctatg 240
ggaatcaaat aattttgcat ccaatccgta aatttatgta tgtgtctgga gaattcaaac 300
accccagaat gtttagaaaa ctagctgaca gctcggtaaa ggaagtatgc tttcacttgg 360
tcacaaaggt ttgttcatct ctcaaatagt gtattaatag atggcaacag agtttaaatg 420
cttttggaag atccttagcc aggtatgagg aaagcctcaa gagagtgcaa gcttattatg 480
gngttataag gnttatcggg ataatacact ttngagagca atgttcatga tttatcttca 540
tctgatgaag aaaagacatt ttctaacaca cattatcgag ttaaaattgg ggnaaaaaag 600
aattttntgn ctcctatttt atagcaaatt tgggatcctt ttggctgaaa anttanttgg 660
ttcttngatt aaagctggnt nttnattant tcnttaaatt ccagccnatn nnaaaagaat 720
tantgntctt ttgg 734

<210> 89
<211> 722
<212> DNA
<213> Homo sapiens

<220>
<221> misc_feature
<222> (1)...(722)
<223> n = a, t, c or g

<220>
<223> nbla-03169-f

<400> 89
gggaatagaa aaaatcacac aggttngcat ggcctcattg nttgaactag atcaaagact 60
ctttgaaaat tncntcgagt tgaaagcana tcccatcgtt ggctccttag aacctggaat 120
ttatgcagga tattttgatt ggaaggactg cctgcctcca acaggtgtca gaaactattt 180
aaaagaagca ctggtgaata taattgccgt gcatgcagag gtgttcacca tttccaaaga 240
actggtccct cgggtactat ccaaggtgat agaagcagnt tctgaagagc tcagtcgact 300
gatgcagtgt gtttcatcct tcagcaaaaa tggagcttta caggcgagac ttgaaatctg 360
tgctttgagg gacactgtgg ctgtttacct gacacccgaa agcaagtcaa gttttaagca 420
ggctttggaa gccctgccca actttcagtg gagcagataa aaagttactg gaagagctcc 480
tgaacaagtt caagagtagc atgcacttgc agctnacctg tttccaagca gcttcttcaa 540
ccatgatgaa aacataaata tctggcacat aaaagaagtc caggaaaata acacgtaata 600
agactggtca ctctctaagt ccctaaangn attttggngn attaaacatt ggggnttgnc 660
atttttctct tttttctttc tctgactttg aaaaatggtg ggatacattt naanccaaat 720
gn 722

<210> 90
<211> 714
<212> DNA
<213> Homo sapiens

<220>
<221> misc_feature
<222> (1)...(714)
<223> n = a, t, c or g

<220>
<223> nbla-03169-r1

<400> 90
gctgaatgtg ctgtagtaga tattttnaat atcacaaacn ttcatgattg caggggggtaa 60
acttagctnn nagttgagtt agatacaaca gttaacatac ttaggaaaga caattcttaa 120
cagctttatc aagcacattt gtgaaagatt aaactttcat ttccagttac catgatgtcc 180
ccccatacac caaatgaacc aacacattta tttattttaa ataatggaaa ctgtcatggc 240
tacactgtgc tctaaaatta cttgcattaa tgaggacatt aattttagca tttaaaaaaa 300
gagatttaaa aataaccata gaaaaacttg aaaaccctga caagccgact acaaagagaa 360
ttcctctctg aaaattttca gatgcgacgg tatgagggag ttgggtgggc tgctgactct 420
gagagaagtc ccggtgacct gaacctattc cagtgaagcc agtcagcagt gaatgtgggg 480
caagtgcatg cggntggcta cagttccatt gcctggtgac caaggagcat cctgaagttt 540
cagagaaaaa actgtgtgac agcagcattg gtncaactac tgagcaagat ccttancagg 600
taaaaaagtg gcanaaacag tcacctgctg aaggaacggg canggagttg gccgtcacgc 660
ggagcctggg cntttccgta ngggaccctg aaatttgnga acggcccgcc aaac 714

<210> 91
<211> 1803
<212> DNA
<213> Homo sapiens

<220>
<221> misc_feature
<222> (1)...(1803)
<223> n = a, t, c or g

<220>
<223> nbla-03199

<400> 91
cactgttggc ctactggcag atgccaaaaa agatgccaac agacactaca ttaccctaat 60
ggctgctacc cagaaccttt ttataggttg ttcttaattt ttttgttgtt gttgttcaag 120
cttttccttt cttttttttc ttggtgtttg ggccacgatt ttaaaatgac ttttattatg 180
ggtatgtgtt gccaaagctg gcttttgtc aaataaaatg aatacgaact taaaaaataa 240
aagctggtat cttaaaatgt aagagagtaa gactgtaaag cctaaaatga ctggctgaga 300
atgaaccaga aatgccattt gccaaacagt tgtaactaga aatttgattc tcacggtcca 360
ttcttttctt tgtccttaag atgacattgt tagtgttcac gtcccatgtt cagtgtccaa 420
accggcaatg taaaaagtat cctgtgtggt ttaacaggaa atctgtttat gtctctttat 480
ttgaaaccag ttttactctc agtggttctt taagttcaat gaagtctgcc aggaacattg 540
gttggtagta ttattccgac acctttaatt tccaaaatct gaagttcctg ctagtttacc 600
accttcatga tcttcttgaa ctggtaactg attaggttga acttatggaa gatttgtgga 660
cttaactcaa aagtaacctc tcagtgttct atagaacatg tatttgtgta actgaaccta 720
ccaggagaaa tgtttggaat tctatatgtg caatttttca acaaatgcaa aaaaaataca 780
gcacatgtat tgacaagctt ctgtcaagca gcttgagttg aaatttgatt taagaaaata 840
aatcatgatt gttcaaagct gctgggacgt tagaattagg ccatgatact ggtctcattt 900
taactacagt ggtatttggc actagtgtaa acttccatat aaatcactct tttggaacaa 960
caaaggggga gggagaaaaa tcacggcctg ttaaatgagt accaaagccg cccaacagta 1020
atgagatgtt ctcatccttg attctcccag cctcaaacaa cacagcttac tttttttttc 1080
ccttgctcag aaagtacctg taatttaaca aacagactgc ctgtaggtat agtgcaatta 1140
caaatgctct aatcattgta catacatctc tcttgatatt gcagcatcca tactggcttt 1200
gtaatcatta atttttggc agattgaatg tgctgtattg atatgtatct atgtaattgt 1260
attgtatgtc tatagctaat tcacgttttg aataatgtta ttttatttac tttttaaga 1320
gaggagaatg taaatttgtc agtttatttc tgactaggga tattttcttt ccatttagaa 1380
aagaagaaaa aaaaaaaacc ttactgtcat acagagcggt actagcgtcg tgctgtataa 1440
aatcatttgc acattcctga gtagaggtat actgattata agacccaaag gtaatttcat 1500
agcaaaatac ataaaatcag tcggagcttt tatacaaaca tggaaaccaa ctttgtagaa 1560

cttttgccat ttgatctagg attggaatat gagcttttat acaattcata ttcttatttg 1620
gcaaatgcac agtttagtat tacctctctg atggccttta ctagaaaggc agttttagaa 1680
gctattgtga tccactaagg aaatgtttta acagctagag accactgctt gcctgaaagg 1740
gcgttcttaa atttggtgca gcaaaaaaaa aaaaaaaaaa ggccacatgt gctcgagctg 1800
cag 1803

<210> 92
<211> 2295
<212> DNA
<213> Homo sapiens

<220>
<221> misc_feature
<222> (1)...(2295)
<223> n = a, t, c or g

<220>
<223> nbla-03212

<400> 92
   gcctactggg ctgtgattta taagatgtat atgctttcta tgtgaatata agcttgtgca 60
   caatgtttaa aagaaaaaca atgaattaga agagatcccc cgtcccccag tctgacatat 120
   ttcatacaga atgtttaaaa gaaaaactct gctagtcttg gcaaacattt ggccatgtga 180
   ggcatgtggt cagtttccat tttctattac ctggaaaggg ccataaggaa gcaggcaggg 240
   cttaggtagc ttggttacct aagttagaaa gtaagagaat catggttcgg atatgaattg 300
   aagtggttgg tacatggata cctgttcttt aaaaggaatg tgggagatga gtcaagggtg 360
   aggccgattg ttctgcccat gccctaccat accaatttca ataacaatct tgcctgtcat 420
   tgcactcaga atcttaagat catgtttcaa atatgtcgat tcaaacttgc ttctaagcct 480
   gagtccaggg atagcaatgg tactgaccag ctatggactg ggagaacatt gagtcctgtt 540
   ttccattggc ttcagggcgc atccatgtgg atataggaaa agcctctttc cacagactta 600
   ggaagccccc cgaccacgcg gacgctcgct gggctgcact cagcaggctg cttgagtggg 660
   agctgactcc aacatgccct gccctggctg ggactaggca tgcttccagc ccttcccgtg 720
   ctctccctgc tgtcaacact tgcgtacaaa cctgcctagc tgttgtaagt tttcatcagt 780
   agcttaagta ctagtagcaa aaacttaaat tcattcagaa agttgccact tctgaatgag 840
   tagagcagga aaagtaaact ttactgaaac ataatttgct gcaacagtca acattgttat 900
   gctgaatgtg ctctcaactg ccctttgctc atcgccagat ttattatcca gtgtattcct 960
   aacagtcttt tgttgtcgtt gtttttaaaa caaaggtacg gaccatgtgc tatatataaa 1020
   tcgaatggct taaactaatt tgctatgatc ctctaacacc gaaatttccc ataaacttaa 1080
   gtgcctacac ttaggacttt tgaactttca gtcatctttt tcgtggtgtc tcactgaacg 1140
   atgtgttaga acatgtgttc catttggagt tttgggcctg tgcacggtca accccaatta 1200
   ccaatttagt tgaggctgga ttcgattgct cagttcacct gccacgttct ctccaaggag 1260
   tggggtctac gtgaccttta gtcctctacc tcagagcccc gagtcatttc tgaccacccc 1320
   gttctgtgtc ctcactcttg tccctgaatg ggtccctgtg tggatctcag tgtgtgtgtg 1380
   gtttctccac tcctccccgc tcatgtccca cacctgccat attgaaccgt ttctgcacta 1440
   atcttctcca cgggcacgga gtggagggaa cgtcttggga aaggggagag cttgacctcc 1500
   atctaggttt cttttatctg gagaaaaaga acacttttga actatgtaat gcttcgccct 1560
   gaaaggcaag ctaacgctaa cttcccaggt gacagtagca ggaacaagga agggtaatgt 1620
   ttccatgaca gacacttgct tcccttggga caagtcccag aagaactacc tgaagcacca 1680
   aagctcccca ccccagcctg gtggcagagg aagcaggtgg caccaactca ccaaagggac 1740
   ttgggagcca ccttctcact ctgtggaagt actttagtga gagctgactg gcaagataaa 1800
   aaccctgaaa ttcccagaag ttacagatgc cttaaagact ttctttggaa gatcattgcc 1860
   tgtactattc cttttttgtg tggcataaat aatatattaa cctgttacaa ctgacaacat 1920
   tggttttctg cagctaggga gtcttgagtc gagactgctc gccttgatga cttgctcttt 1980
   tttggcagat gtgtggagtt ggctgttacg acatccctgt gtgatcccag aggaggggca 2040
   gtagctgggc agaaactgca ggcacacatt tgggtggatc acctggcttc cagccccttc 2100
   ggggtggtgc tcagtgtggg gtgatggtat catagaggac cacggcgctt atcagcattg 2160
   tttctttgga gtctgtgtaa tggaatgtat ttttcaaata ctgatatttg cattttctgt 2220
   aacaattcct tataataaaa tgaagtaaaa ttgtgaaaaa aaaaaaaaaa aaggccacat 2280
   gtgctcgagc tgcag 2295

<210> 93
<211> 750
<212> DNA
<213> Homo sapiens

<220>
<221> misc_feature
<222> (1)...(750)
<223> n = a, t, c or g

<220>
<223> nbla-03213-f

<400> 93
gttggaagca agacnataca gaaagctgct ctaaaatctc actntttgat ttcaccttcg 60
nttactatga aatgtngnat cagacctgac atgnctgaga taaccccgtg attcaggatt 120
tgatcaaaag aagtctagta agaattaatt acacagaagc ctcctttcat ttctatgggc 180
caaacaaagg ccatggataa ccctacccgc tttatgtcat tacccattgg gaaacacaat 240
ggctacttct gttagggtac attgaccttg gtcaagcatc ttaaagaagg caaccctaat 300
tgagagctgt cttggctaat actctgcacc acaattgtga tgtcctagtc ctaccactag 360
agggcatggt acagcctggc aaaagttaaa aggggtgtgg cagctcccat caggtctgga 420
ggtggtctat aagcacagtt gacagttgtg cattgggatg ggtggagaaa gacgacaaga 480

gagcagagaa tctgctgatg tggctgcgct tacttttagt gactttatgt acttatatta 540
acagctggaa ataggntgnt gggttttgag cangctggta tagtgaggaa tgntcatttt 600
taaatgncct aacagatttt gctttgaaaa atgcttggtc atgaataatt tgnggccagg 660
gatgctttnt gaagcagnnt agggaacatg aatnttcaag angaaatcca aaaatntgtt 720
aagggcatan ngntaannnc nncnaggaan 750

<210> 94
<211> 743
<212> DNA
<213> Homo sapiens

<220>
<221> misc_feature
<222> (1)...(743)
<223> n = a, t, c or g

<220>
<223> nbla-03213-r1

<400> 94
   tgcttgatgc atttagagac aattccaagg gcaagcactg gattttgata ttatctgctt 60
   tgtagatatt tgtacgnaag actgttaaga ctacatggcc agtccccact gcaggaaaca 120
   gaatgatttt cttttttttt tattattatt ccctgccaat agcattttcc atctaacaca 180
   tttaaattct cacagcataa ttgcaaacat gtacatattg gttcccacga gaaatctgca 240
   ggtgacattt cgggatgttt ggagtcaatc agataaagca cattatgaca gaaaccataa 300
   gaggggagct gggacctggg actgtgcaga atgaaaagcc acggctacgg gaagcataag 360
   cttgtgctgc aacggcatgt gcacatgacc tgctgctcct ggcctctacg aagcatgccc 420
   agcaagggaa cctcctncgc cctnctcacg tgcttccttc ctaaggctga gagctcaact 480
   gaagaccttc ccaataaatg acagatggct cttcagtcan gaatgcagnt agtgggttca 540
   tgacagacac actggnccca cccggcttcc tgntgaccac cantctgaat aacacctgnc 600
   caatgnggga tgacatcagc taggaacaaa gnaattggcn gggtgatgat atttttatcc 660
   naangatnnc ccatntnaag gaaccccccn nttanggcta acaggnnnta ccnggnctgg 720
   nagntannan aaananccnt cnn 743

<210> 95
<211> 3049
<212> DNA
<213> Homo sapiens

<220>

<221> misc_feature
<222> (1)...(3049)
<223> n = a, t, c or g

<220>
<223> nbla-03219

<400> 95
gaattcctcg agcactgttg gcctactgga aatttaaaaa tagctacgaa acggtgtttt 60
taaagttgag gtctggaaga cctggctcgg ggtctgggaa ggtgggtctt ttgtgatgtg 120
gtccccgggc ggtgcacttg ggagccatgg cggggccagg acctctggca gcgcagggat 180
ggagcccgca ggtgatgagc ttgggaggtg agttgtggag gctgcgctca catcaatgcc 240
cagtgccctc cccgaggggc ctggttctct ctccacaggg gcgggggaag cacacagggg 300
acagggaggg gtgctgggtt ctctctcctc gggacaggga gcgcagccag gttctctctc 360
ctcgggacag ggtggtgccg ttgcgtgcat tccccagctg cagccacgag aaacaatttg 420
gagcggaacc cgggctctga cctcccctca tcctcagcct tcccccaggg atgggccgtg 480
agatgaatgt ggtcaccggc ccaatccagg ggtctatggc caaaccgcag acccggagga 540
agcaggccag gccatctggg gagccggctc ccctcctctc ctcccctggc tcccacaaag 600
ctgtctcatc cagaagccag gcccgcctgt gagcaagggg aggctgcagg tgttccttca 660
gcctgaagcg tgtgaaagcc aacagccccc accctggtct ccagccgcag cccccctcccc 720
agactcaggg ggccaaaccc acttttcaca gccatctgta acccaaacgt ctgcccacag 780
ctttgttcga gctcagtcca cagtgggccg cgtggcctgg gagcgtccag tggcagctgg 840
ggcctgccag gactgctgcc cccagatcgc tgtttgctgt gtgtgtggct ctgtggggcc 900
ccggctccac gataccctca agcatctgcc tcccgctctt cccagattgc agggcatgcc 960
tggcaagact gcgcagtgcc gctctgacgg gcctgatctg aacctgggct cgggggggaca 1020
agggagggga ctgagcacgt gggatggaca gagatgcaca cccctcccag gcaggagagg 1080
cagagctgct tctgacacac aggcagttat caggggggaag agaaggtggg cagaggagcc 1140
accccatctc aaggcagcgt gcagggcaga gcctaagaga gggtcctcct gcaagggcca 1200
gacaaggagg agggcatggg caggaaccgc gtccaaatca acagcaaaga caaaggtcac 1260
ctagaaaggg ggactccacc agactcttac ccagcagcac gtccctcctg cctgggcctg 1320
gtgagcgagg ggctcaaggg gaaactgagg caggtgctgg cacttctgtg tgggtgagga 1380
ggacaccaat ctgaggcccc gcccgctccc tgcttcggct cctctgtgtt cccgggctgc 1440
cgactctcag gaggctttgc cacctgaaga acagggaggt ccagcaaggt aagaagagct 1500
ttccagctcc tgttccgacc tgagagccga gaggcaccat cctgaagaat ggccttggtc 1560
ccaggcctcc atgtgcgctg tggcagacaa tgccagtcga tcacctctct ttcctgaccc 1620
agacacagcc tcatgctctt cagccaagtg ccccagggaa gcccgccacc gatcagtcaa 1680
gcctggagta aaagatgaag cctatttgct tcgctgtttt cacgtggggc ttatcaagta 1740
tttactaagc accttctaga agcttggaaa gggctacaag ccatggtccc tgagcccaag 1800
acacttacag gtttgctttc tgggaggatt ggggtggatt agagactgcc ttccagtcag 1860
tgaccacagg atgggccatc ttggtctctg tccgtatctg gtttgggggt tttcgcagga 1920
ctaggcctag gctaaggaca gggagggcac aagttctggc tgggtccagg gtcctgggac 1980
ccagggccat ttgggacctg tgagaatgga tggacagcat gggatggggc atgtggtctg 2040

agccctcagt agattccctt caccctcttg ttgttggggt cgaagggcga cttcaggtga 2100
gcctgggcac cataggtcac ccccattctc tccagggcat agtccccgct cttcacaaag 2160
tccagcgaga cctaggagca gaggtgggga tgaggatcac tctctgttgg taggtacagg 2220
gctcacgtct gcctgcttct ctggaggagg gggtcttggt cttgctacct gccctgtggg 2280
caaactcatc cctgtgccag ggactccttt ctgaacccag gcattgcccc agagcaggtg 2340
cagttcgtgt ccatgtgatc ttgagttgga catgggcatc tggggtccgg caaagccaag 2400
gtctggcagc caatatgact tccaccggac aacacacctc ccaccccagc aattccagat 2460
gcctgggaaa cagcagcaac acggtggctt ccccggaccc gggcaagcac agcggccaga 2520
gcagcgccga ggctgggggc caatccggct cgaatccctc ggggcagggg cggctggaac 2580
agccttctag acagcttggc ctctgcatgc tccccatccc acaccctggc tcgggctccc 2640
cagcaagccc tcgggagcca gtcagcccca ggtgcaggtg ctcttaggca ggaggagggt 2700
gtcacctcca gcctggcaca gctggtgacg cctggcactt ttgctgcagc tgctatgagg 2760
agggcaggct aggccactca gggaagctgt tcttccttta cagcaattaa acccaaggtc 2820
agggccacag gagggagtct cgggctccgc tctgggagtc agagggctcc ttcttgttcc 2880
tagcagctca ggactggatg aaaaaaataa aaaacaaacc aagctttttc aaaaaaaaaa 2940
aaaaaaaaaa aaaaaaaaaa aaaaaaaaaa aaaaaaaaaa aaaaaaaaaa aaaaaaaaaa 3000
aaaaaaaggc cacatgtgct cgagctgcag gtgcgcggcc gctagacta 3049

<210> 96
<211> 754
<212> DNA
<213> Homo sapiens

<220>
<221> misc_feature
<222> (1)...(754)
<223> n = a, t, c or g

<220>
<223> nbla-03223-f

<400> 96
cttctttgat atggataatg gaccgagcct ctgtttgcaa attatttnag tatgcttgcc 60
ataagaagtg ctgtctgnna accacagcca agtgctctaa aaagtatgat ccagagctgt 120
catctcgaca atttggggtt gaactgtccc gtttgaccag tgaagaccga actgttcctt 180
tagtagtgga aaagctcata aactacattg aaatgcatgg actgtataca gaaggtattt 240
atcgaaagtc tggttcgact aataaaatca aggagcttcg gcagggtcta gatacagatg 300
ctgagagtgt aaatctagat gactataaca tacacgtcat tgcaagtgta ttcaaacaat 360
ggcttcgaga tttgcccaat cctctcatga cctttgaact ctatgaggaa tttcttcgag 420
ctatgggcct tcaggagagg aaggagacaa tccgtggtgt atactctgtg attgatcaac 480
tctnccgaac tcatctcaat acactggaac gcctcatctt tcatctagtc aggattgctc 540

tgcaggaaga cactaatcga atgtctgcta atgctttggc cattgtgttt gcgccctgca 600
ttctccgctg cctgcaccac tgcccactac aaagtgtcag gacatcagta agactaccac 660
ttgtgtggaa ctgattgtgt ggacaaatga ataatncang ntcgctcaaa gattcagtac 720
cttggaattg ctgagaataa gcaaaancnc gttg 754

<210> 97
<211> 761
<212> DNA
<213> Homo sapiens

<220>
<221> misc_feature
<222> (1)...(761)
<223> n = a, t, c or g

<220>
<223> nbla-03223-r1

<400> 97
ntaacatgga gtgtttttc agaaatttt ttaatagagg gattantttt ttttgtttgt 60
anggnagttt ttggaaaann attatnttct accctagctc ctaactatcc caaaataaac 120
ccaaaggctt ttgctttcac ggntaanaaa gatttatacn ttttcttcaa atgtcagaaa 180
tganagggtc cctnaggaca gcaatatccc ccctagttca acacccacct ttgggaaggg 240
aaaagagggt gggggagagg caactacaac tgacccaaat ccccaggccc taggtggctt 300
tgtatagtaa aaatctcaat tcaaatncaa cagccaaggc acagctggca ccatncccag 360
caggctttct gcttntgcag gaggcccagg aattcagcac atacagtctt agccatatgc 420
ttagaaaaga ggcaggacca caattaggat tgctattgtg gaccaagtga tgaaacccan 480
ccccaaaggn gagatttggt taccactntg taccaccgag ggaacacatt tgccggntcg 540
gccataaatt cattattttc aaaaaggcta nctgntggtt ggaagngcaa tctnggtnca 600
caagtttttt tgnggccaac atgactgtcc ctntngggnc tctttgggtt tctganggga 660
gtttttacaa tctttnaatt ttgggttgnc cttttccggg ttaaaggtcc tttgntgatn 720
aactgangct tgnntttcaa gaatttggct gggccaaagn c 761

<210> 98
<211> 763
<212> DNA
<213> Homo sapiens

<220>
<221> misc_feature

<222> (1)...(763)
<223> n = a, t, c or g

<220>
<223> nbla-03224-f

<400> 98
ctcctccatc attgaaaagt ggtcgatggt tcgattgcag gcacctttta aacccgattc 60
agggntgctg gatggatngg ngacgactga tgctgctcta atgtatgatg ctgtgcatgt 120
ggtgtctgtg gccgttcaac agtttcccca gatgacagtc agttccttgc agtgtaatcg 180
acataaaccc tggcgcttcg ggacccgctt tatgagtcta attaaagagg cacattggga 240
aggcctcaca ggcagaataa ctttcaacaa aaccaatggc ttgagaacag attttgattt 300
ggatgtgatc agtctgaagg aagaaggtct agaaaagatt ggaacgtggg atccagccag 360
tggcctgaat atgacagaaa gtcaaaaggg aaagccagcg aacatcacag attccttatc 420
caatcgttct ttgattgtta ccaccatttt ggaagagcct tatgtccttt ttaagaaatc 480
tgacaaacct ctctatggta atgatcgatt tgaaggctat tgcattgatc tcctcanaga 540
gttatctaca atccttggct ttacatatga aattagactt gtggaagatg ggaaatatgg 600
agcccangat gatgccaatg gacaatggaa tggaatggnt cgtgaactaa ttgatcataa 660
actgccttgc aattgcttca ctggctatta cctatgttcg anagaaggca tcgacttttc 720
caagncctta tgacacttgg nataagnatt ttgacccgaa gcc 763

<210> 99
<211> 767
<212> DNA
<213> Homo sapiens

<220>
<221> misc_feature
<222> (1)...(767)
<223> n = a, t, c or g

<220>
<223> nbla-03224-r1

<400> 99
   caaacatgag tacataacaa gtactggttt tttataaaag aggaanttgt atttgccttg 60
   ggggntaaga cttagctnng aatcaaccat taaattgnta atattttccc agtttgctca 120
   gttttgatgg atttattcat aagatatcct atcaatcata atctcaggac acagctcaga 180
   gcaagtgagg aggggatggg cctgtcaaat attcgtccct cttcctttat ccattgaatc 240
   acttatgcat tgacaatttc ttgagcacct atcacaggcc attgattgca ttatgtgctc 300
   aggggaaaca gatgactaga acattcaggt cctcaacata tcctgttata aaagttaana 360
   tctgaacaaa tnactaatta gaataaagtg gatttaagta atatgcttat tttcacagac 420
   ttcaaattta tgactgatgc tttggtacta acaagtaatg gcaaatatac tgngaaatca 480
   gaagtntaaa atgncaatac attattgggg cattcaaaaa caattgngtg tgataacatg 540
   caaggagaat atgttttcac tgagctatat cttacataaa ttattaccaa aataaacctt 600
   acttttttat taagactaat gngnaacant ttacagangn nttttttaac ccaaggacaa 660
   actgcaaaat gaggggagaa ccatgaccga taaagctgaa natagaccct ntgacttggt 720
   ataaaaaagg cttatnagaa gtgnnanatt tgggtttctt gnaaaaa 767

<210> 100
<211> 804
<212> DNA
<213> Homo sapiens

<220>
<221> misc_feature
<222> (1)...(804)
<223> n = a, t, c or g

<220>
<223> nbla-03298-rl

<400> 100
tntactgtat gctgtatgtt acagtctact atagtcattg ctctattttt agaatatgtc 60
agtacacact ggctgaattc cacattttct cattagtaac tttttcagtg gtctttatta 120
cactacattt tatttttgaa aagaaaaact caccttttac tagggtagga cagaggaata 180
ggttgaatat ttacagattt cataccataa acatgatctg gtctattttg gccttggaaa 240
ttaagataat tttaattaag ttttgccttt ggctagcatc agtgacatag aaatgtcctt 300
ttctgtagtt ttctttggaa atgcaacaaa ctaaaaaatg cagaagtact atactttgta 360
tgttaaggca ttaccttaag tgttggttca ctttgntact ataaaaaagg ttgatatttt 420
tggctgccct taagtgaatt taataacaac tactaataaa aatatttgca aaagtatttt 480
acatatatct aatcataaat atccacaaaa ttccggtgag aaaggnaatt catacccatt 540
ttaaggaaga taaattactt attcacgtaa catagaaaaa atgaaagtct agtttgactg 600
acttcaaaga ctatattttt nccctagaat attaactacc tttaatttct ccaactaaga 660
actggggaca ttttttaaat atntattggt atggaatcta ttncccnaag nccaaagggg 720
aactgaaccn aggaaaagat ttgaaacttn attccanata ctaactcann ggnncctgac 780
tnaaggaatn tttccggcct ctct 804

<210> 101
<211> 730
<212> DNA

<213> Homo sapiens

<220>
<221> misc_feature
<222> (1)...(730)
<223> n = a, t, c or g

<220>
<223> nbla-03401-f

<400> 101
gnagnncngg gtngttgcta ntnatcnttt gaactgccng cctactntac atttgtgttt 60
caggtgtaga gaggagagag agtgaacagg gagcggggct tttntctgtt ggtctccctg 120
gactgaagag agggngaata gaagcccaag actaagattc tcaaaatggt ttattaccca 180
gaactctttg tctgggtcag tcaagaacca tttccaaaca aggacatgga gggaaggctt 240
cctaagggaa gacttcctgt cccaaaggaa gtgaaccgca agaagaacga tgagacaaac 300
gctgcctccc tgactccact gggcagcagt gaactccgct ccccaagaat cagttacctc 360
cacttttttt aatcgtaaca cctccatttg tattacatat ggtgtatggg tattgatgag 420
gtcatggtat catatatggg atttttttct gtgtaaatca tcaagtataa gaagaaacta 480
tgggactctg agccttgctt tagagaattt acagtggaca aataggtgtc atcaaaccag 540
tttttaatca ttctgactca agtgaaaacg ctcagaattt cacactgtga atccacgttt 600
acaaccctta cangtgggcc ttnaagcctg gttcgctaca acaatggctt ncacaactna 660
aactccaccg gctnacacaa cccggccact cctggctttt nacttacaca gcttccgact 720
gnttcttgcn730

<210> 102
<211> 736
<212> DNA
<213> Homo sapiens

<220>
<221> misc_feature
<222> (1)...(736)
<223> n = a, t, c or g

<220>
<223> nbla-03421-f

<400> 102
cactgttggc ctactggata atgaagataa taatatcttt attctttatc cccttcaaa 60
gaaattacct ttgtgtcaaa tgccgctttg ngnggagccc ttaaaatncc acctcctcat 120

gtgtaaattg acacaatcac taatctggta atttaaacaa ttgagatagc aaaagtgttt 180
aacagactag gataattttt ttttcatatt tgccaaaatt tttgtaaacc ctgtcttgtc 240
aaataagtgt ataatattgt attattaatt tatttttact ttctatacca tttcaaaaca 300
cattacacta agggggaacc aagactagtt tcttcagggc agtggacgta gtagtttgta 360
aaaacgtttt ctatgacgca taagctagca tgcctatgat ttatttcctt catgaatttg 420
tcactggatc agcagctgtg gaaataaagc ttgtgagccc tctgctggcc acagtgagga 480
aagtagcaca cataggatac agttgtatgt agtcattggc aacaattgca tacaatttta 540
ctaccaagag aaggtctcnc atggaaagtc caaatgactt ccttgatgga tgttaacaag 600
ctgactggtg tgagacttga ggttcatcta gtccttcaaa actatattgg ntgcctagat 660
tctctntgga aactgacttt ncaaataaat agcanattgt anngctgngn cnnttnggac 720
antactgctt ttatnn 736

<210> 103
<211> 752
<212> DNA
<213> Homo sapiens

<220>
<221> misc_feature
<222> (1)...(752)
<223> n = a, t, c or g

<220>
<221> misc_feature
<222> (1)...(752)
<223> n = a, t, c or g

<220>
<223> nbla-03421-rl

<400> 103
   gtcccttttc ctttttnttt ttntttcctn nttttttttt tttttttttt tttttttttt 60
   tttatnccct ttttaaaatt taatttnnaa aaacnccttt ngggtaaccn ttcnnantta 120
   aannggaaan ngccnaantt tttaggnnnc cntccctgga atgntttcat ttttncaagg 180
   gnncccaaaa aagggccntt taaaancncn nttccnnntt ttnancccgg ggancnncnn 240
   tnttttccca tgnnactttt aacttnntgg aaaatccccc nanaaaaaaa gntcaaancc 300
   natnnccttn nnttaanccc ctnggttngg ctnngttttt ttnnaaaatt ccnaaanggg 360
   ngnctanttt tttcccanaa nttncncntt tcntttaggc cnccngnggg gaatttnaat 420
   ttttnaggtc cnaaanggtt ttaatttcnc ancttttcca anagngccct ttgggggatt 480
   nggccccttt ttttttnnnc aaaaattttt taatngaagg aaggttnctt tnnaaaacac 540
   cncccnnggt ttttccttaa nacttnanca tttttggatn aaactggcaa aatttntaan 600
   ntnccaaang ggcttaaggg cccccaaggn nggaaccttc ttttnggggt tttnccnctt 660
   tannaccgnt ccctgttttn aancccttac tncccttttt ttnaaggggt taanancccn 720
   cccntcccnn ggnnnncccc ccaccnccaa nc 752

<210> 104
<211> 732
<212> DNA
<213> Homo sapiens

<220>
<221> misc_feature
<222> (1)...(732)
<223> n = a, t, c or g

<220>
<223> nbla-03439-f

<400> 104
gncngcctac tgttnaacca aggtctgtgg ataacagacc acaagcacca ctggtttctg 60
cgtctgctgt tantgaagaa gtcagcnggn ttaaatctac aggnnaattt ccagaacaaa 120
aattgtttgc tgccctgttg attaaatgtg ttgtgcagct ggaactcatc cagactatcg 180
acaacattgt cttcttccca gccacaagta agaaagaaga tgcagaagac ttagctgcag 240
cacagagaga tgcggtggac tttgatgttc gcgttgatac tcaagaccaa ggaatgtacc 300
gctttttaac atcacaacaa ctttttaagc tactggactg cttattagag tcacatagat 360
ttgcaaaagc gtttaattcc aacaacgaac agaggactgc cctgtggaaa gcaggattca 420
aaggcaaatc caagcccaac cttctgaagc aggagaccag cagcctgcct gtgggctgcg 480
cattctcttc cggatgtaca tggatgagag ccgcgttagt gcctgggagg aggtccagca 540
gaagcttttg aatgtctgca gtgaancact aagttacttc ctactctaca tcaagaaagt 600
catcgagaaa cctggctaac ttactgcttt tggttctaac tnaagttctn aagataagtg 660
ataatagggt taaagctcat gcatcattct actacccttc ttatgngaaa ntatgcaatt 720
tgacttgatt cn 732

<210> 105
<211> 713
<212> DNA
<213> Homo sapiens

<220>
<221> misc_feature
<222> (1)...(713)

<223> n = a, t, c or g

<220>
<223> nbla-03439-r1

<400> 105
anctttattg acctataacc tgattagaat atgccagatg ggaatcaata ttttgcagaa 60
agttgtacag aatttttac anggnaaact ttacatngng taccatatac atttgtcca 120
tctgaaaaaa ttttctacat ccactgttaa tacggaatgc ttgacaatct tgtcttttaa 180
ccatcagagc acaattcaca gtatgaatac atttccagta aatctaacct ccgcaaacca 240
tgccagattt gttattttaa tatattcaac gttaaattct gtacatagag taaaatctac 300
atcaggcccc accacccaaa agaaaagaaa atgacagcaa tctggattca ttttgcagtg 360
attcaagttt tggtccataa aggaccattc tattttaatg gctcatcttt aaaagtccta 420
agagaaatat gtgacaggta gttactgtcn gtagggatat tttctacctt tntttcccta 480
ttggtgaaat gcngccacaa nanctttggg catgttcaan ctcaaaattn ccaccttccn 540
aaaanggngn aacttaaaan cctctttaat aaaaaanaaa gtttncccca tgtttntgcn 600
tttttcaaaa natttngggt tttcnnngnt tntaaaattt aaaaaacacg ncggngaaaa 660
ntttatggng taaaaanttn tnttnnancn cntntntnna ganttttata ana 713

<210> 106
<211> 2231
<212> DNA
<213> Homo sapiens

<220>
<221> misc_feature
<222> (1)...(2231)
<223> n = a, t, c or g

<220>
<223> nbla-03461

<400> 106
cactgttggc ctactgggta actgtcagtc aaattatccc atttcctagg tctgtctctt 60
ttttctttgc agtttaatta ttagtaaata agaaatttta agtctcaaaa ttttggtcag 120
agataaactc agacactgcc tcgatatcac gaagttctca tttataccaa ctcttatctt 180
cacgccaccg tgaattctca tcggcataag gaggaaaaga gatggcacca aaggggaaaa 240
aaatctggtg gtgtaatttg gcatcttcat taagcaagcc atgagcagct tgtgaaatgc 300
ttcatttatg gggccgccag ctgggagaga gaggcgttct cacaatgcct tgaaaatggg 360
aactttgcat cctttaaatt tttccaaact gacttagttt gtttaccttg aatttctggg 420
atggggcaaa tgtgaccttc atgctatagg gcccacgttt ccagatttgg tatggaaaga 480

aggaagaaag tctgaccctc ttgtttttaa gataggcaaa aggaagatga gatagtccat 540
ggttcaccac ccaaggtcct tcctgggcac tggctgggct gacgctgggc ctggttccca 600
gctatgccta cctttctctt gccataccac accgttgctt tatgagcatt cttttgttaa 660
ggccaagatc aagataactt ttttccttga ataataggac agtcaccttc cccagtggac 720
tttaagtggc atctgaatgt agaaaggaaa acaaccatga cctgtatttt tagagtagaa 780
ataaaattcc tcattccacg tatataatcg tggataagca caaataccta gctatagaaa 840
tgtttgtaaa agtggaaaat tggcagcaac ccaaaagccc aacagcagac aattagttaa 900
atcagttatg gtctataatc gcagtaggat ttatgttccc ctttaaaatt ctggaaagaa 960
aaatatagac ttagaaaggg catgtgatgg gggaagggga aaaacaggtc tgtaatgtaa 1020
taacttttgc aatatttttc cattttatca gaagagtatc agctattatc tttgagtaat 1080
aaggttatat tttctaaatt tgttttaaag ttttgaatga aatgtctgtt acttttatga 1140
gattaatttt tatataccga tatatgaaag aaaaatatta acctttagat ccattaaggt 1200
cctccctggg gaatggcaaa tacatagcat atgtctctcc tcaccttcct tctccgtcta 1260
tagcaaaaac tttctatcaa tctgtgatca ttccctctgt gcctggtaga agacctcaga 1320
atgctctcaa ccactatcaa tccatgttcc ttggtatata catgggaact caaatggcat 1380
tgtcctaagt ctaagtcatg gaaaagagag tgctctaggt gtgaagatgc cccaattaaa 1440
acaagttcag cgctaattca aagtaaagca tttgggcctt cctgggaagc tgtagtagca 1500
tgcagaaata ccaaggcaga gttatgatcg tgctgatttt ttcctatcag tgggagggtg 1560
cagtctgcat atattttacc cctgctctca ctgcagtgtg gggtggatcg ataggtggat 1620
gtgtaatatt aactggcaca ttgacaccct aatttttgca aatggtaaag ccattccaca 1680
tatgcatttg ttcaagaagc taatgtttat tgaaaaccta ttgtgttcta agccatgaac 1740
catccccagg taagtgacct cagctctgcc attacgagtc ctctggggct ggccagggaa 1800
ggtaagagcc caccctcctc atctgtggag ttgctatgga gatctctgca cctctagaca 1860
gatactaagg actcaatgca actggcagaa catttttttg atagcacaag atgaaaaaca 1920
atttgcaagg tatttctgaa aagttaatga aaattgtagg atagttgaaa gcaaaagtga 1980
acattatgag gcatatgata ctgttaaaat catcatacac tgctgtaaag cagtttcaat 2040
atctgtatgt ctgccccagg aataatcaac caggtattac ctagaatagt gtttttcaac 2100
ttctgcaact gtaatctaga gcaagagatg acaagttttg cattatggcc cagtacgcac 2160
atgcctatgc ctagatacag ctgaaacaaa caaaaaaaaa aaaaaaaagg ccacatgtgc 2220
tcgagctgca g2231

<210> 107
<211> 737
<212> DNA
<213> Homo sapiens

<220>
<221> misc_feature
<222> (1)...(737)
<223> n = a, t, c or g

<220>
<223> nbla-03477-f

<400> 107
ggcncgcccg cccaccgctc tccacgtgct cgctggaggg cggtgcgagg ggccgnttnn 60
acaagatgtt cttgctgcct cttccggggg nggtgcgagt agtcgtccga cgtctggccg 120
tgagacgttt cgggagccgg agtctctcca ccgcagacat gacgaagggc cttgttttag 180
gaatctattc caaagaaaaa gaagatgatg tgccacagtt cacaagtgca ggagagaatt 240
ttgataaatt gttagctgga aagctgagag agactttgaa catatctgga ccacctctga 300
aggcagggaa gactcgaacc ttttatggtc tgcatcagga cttccccagc gtggtgctag 360
ttggcctcgg caaaaaggca gctggaatcg acgaacagga aaactggcat gaaggcaaag 420
aaaacatcag agcagctgtt gcagcggggt gcaggcagat tcaagacctg gagctctcgt 480
ctgtggaggt ggatccctgt ggagacgctc aagctgctgc nganggaacg gtgcttggtc 540
tctatgaata cgatgaccta aagcaaaaaa agaagatggc ttgtgtcgca aaactctatg 600
gaagtgggga tcangaagcc tggcanaaaa ggagtcctgt ttgcttctgg gcaaaacttg 660
gcacgccaat tgatggagac ccancccatg agattacgcc aaccagattt gccnaaatta 720
ttgggaaaaa tttaaaa 737

<210> 108
<211> 750
<212> DNA
<213> Homo sapiens

<220>
<221> misc_feature
<222> (1)...(750)
<223> n = a, t, c or g

<220>
<223> nbla-03477-rl

<400> 108
   gaccccatca agttttnatt aattgtccct gacaagtgca tagtaanntt cannttgctc 60
   atgttcctag ntttncaaat ctganttaca attttgcttc ggggggaana acatatactc 120
   tgaaaagtgn cnaggaagtt ctaaaaattt taatcattta tagagtatct taaaaatcct 180
   tatcaagtaa gatattaact ttacctttat aaatctttgt gtgaaatgaa aaaaaaatca 240
   aggcatacaa atttcattgn gttctacatt tttaaatacc atcctttgtc tccgttaaaa 300
   nattttcatc catttattca aaaacctttt aagttcaact gtccaattta agacagagtg 360
   aagacatttt tgagtatctg aactaagcat tgtcttgact gaaacgaagt aagaactcaa 420
   tgagagtcct tgtgggcctc cagtcatgcc tttccgtaga tagggaactt catctttgtt 480
   ggcatcacgc ctgctatgtc taaatgtncc cacttaggat gagttccaat tctttcanga 540
   atgctgcacc tgacatgctc ctgcanatcn ggattttcca atgntgttaa catcncnang 600
   cttgtaattt acaaccngnc nttnnnaant gtccaaaaan aggcntcctt caaacaccgn 660
   nccccnnttt cnaatgctnc ccntnaaant ntnttncnta acccctgatt taatttnnaa 720
   aaaccccntn gcncctntnc ccaagttcct 750

<210> 109
<211> 2342
<212> DNA
<213> Homo sapiens

<220>
<221> misc_feature
<222> (1)...(2342)
<223> n = a, t, c or g

<220>
<223> nbla-03526

<400> 109
ttctaggcct gtacggaagt gttacttctg ctctaaaagc tgcggaattc ctcgagcact 60
gttggcctac tggcagattg ttttatattt caatgtatta tgtggagagt gacttttcat 120
taataaacta caaagaaatg ggttataagg aatattcatg aacatgctct atagaaatat 180
aataaaaatt cattgatgca aagtcctttt ttaaaaataa ttttcaatct ttttgataag 240
ctggacggaa gcatctgtct tttactatga agaaaacatt tttgaagtga actgacaaca 300
taaggcaaat actgggtaaa aatttaagca tctccaaaat cattttatta acccatttgc 360
atgcaaatac tagggaatgg ttcttcaata tgtagctagt tcttgtggtg gttaatagtt 420
cttcgtgtat ttgaaagtaa caaaactgca tttgtgttga actcttaaga ccaattaatc 480
aggtcaaatt gctcttaaga ccaataaatt aggtcagttg ctcctaacaa aggcaaattc 540
atcagattta atttctaccc ttttctaata taaattttta gaaattttgt ttctcagatg 600
tgaggcaaat aggttgatct atttgtttat tctttgacag agagactgac tagagttaga 660
cagcttttta ataccccttc cagtcaaaaa gtaacattgt tatattagta tttagtagtt 720
ttaaagtctg atgacataag aacagtaaca atttgttaat attcaaaaga ttgaaattga 780
aaaaaagggg tcctgcattc tgctctccta gaattttttt ttgtgttgaa atcttctttt 840
ccatattgtc attattgctt ctttatccca tgacattaat tttctggtga ggtgaccctg 900
aatttagtca ttttcgtcag taattttaga tttaagtaac ttatcatgct tgttttaaag 960
tctctctcag ctcacccatg aagaaacaaa ttttttttca tttttttctt caaaaatcag 1020
caagtattgc tgaatctaac aagtatcagc attcaggttt aaccgtgtta tttgattgaa 1080
ataaatcaaa ccatggaaat aatgggcact gtgtctgctc aagagtaaaa ttacccaatg 1140
aatgattatg tcaactggat gtcttttata tcaattgatg actttttaaa aaatctgtat 1200
tttaaataaa atatggagag aacagtattg gtccctacaa cattccaata gctttgtcat 1260
gacagccatg tcatatgaaa taacttactc tgaatggcta ataattcagt ggagatgaat 1320

tctaaagatt tttatttgca cttaaaatgt taaagtagta ttgttcattt gatcaatgtt 1380
tgaatactac tcagtattga aaactttaac tgcattctga aaggtcattg cgccgattgg 1440
cttacctaag atgtcattta gcttcctttt ctcatgattt acacaaatgt tgttacctta 1500
aatgcagaaa gatgtgatat tctttggagg ataaaaaacc taagtgaatg aagctagaca 1560
caaaaggcta catattatat gatttcattt atatgaaata tgcagaatag gtaaattcat 1620
agagataaat cagattcatg gttgctgggg gaatgggagt gactgcttca tgggtatggg 1680
gttttctttt ggattgatga aaatattgta gaacaagata gtggggatag ttgcacattg 1740
tgaatgtgcc aaaatgccag tgaattgtat actttaaaat ggtgtatttg atgtgtattt 1800
tattacaatt aaaaagaaaa ggatttgata tttgcaatca ttaccaaagc caaaaactag 1860
tgttctcaac gtttgtgaaa tgcccaaacc gtggtttaga attaatttat agcattagta 1920
acatgacaaa tctattcagc ttaaagttct agtaaaattt gtgaatccca tcatagaagt 1980
actcctgaca gcagcagaaa taaagctgta attcatcaaa atttccttat atttaaaact 2040
tctgtgactg agtgattgac ttctgtgtca atgtttattt tttatctttt gaaatttatt 2100
atgattcctt gtggcacatc ccaatcacgc tttttaaaga tgtgttttta aaaccatgct 2160
ttgaaaaaaa tttgtataat tttcaaggtt tttttttata gttttatact ttgtttcttt 2220
taaagaacaa atgcaacttg ttgtattaga gtaagactaa ataaattgta ctgatgtgta 2280
aaaaaaaaaa aaaaaaaaac aaaggccaca tgtgctcgag ctgcaggtcg cggccgctag 2340
ac 2342

<210> 110
<211> 739
<212> DNA
<213> Homo sapiens

<220>
<221> misc_feature
<222> (1)...(739)
<223> n = a, t, c or g

<220>
<223> nbla-03539-f

<400> 110
tnaaaccccc ctgctgttaa gacttgacat tcaatatttt ttgatcacat tcttttttat 60
agcttagcta nnggcaacat ttgtggncat ataaattgca aaagaagctt tctcgngtac 120
atacattttt aaaagcttga aattgatgtg aactttaaaa aacacgtagg atctgtatta 180
cattctacat ctcaaaacaa atttaattaa agtgaatatc attccagtat atacaatatg 240
cctaagaccc agaattggca cactgattta ctagttgaaa atataacagt attcaccaaa 300
cttcaatgta tactttttgg agagaatgaa attacagtat ttcttaattt actgnaatgt 360
catctttgta attatgaatt aacaattcaa tgagaggaga cttggttgat taaattaatg 420
ctggtcctac acattatatc taaaggatct tcgtatatga ctactatctt cttggattat 480

tttaacaggt aaaatatcaa agtggccatt aaaaacagag ttgacttttc accattgctg 540
gtttctggt gagacatgtg gaaaggaagg acaggtggac ttttcaacta actagctctc 600
tgatttttaa taagatcctc aantcttttg gnctnagnta cctatctgtc caanggtaag 660
catatgctta atcactaana cnggtanatc ctgccnttaa naaccttatn aaccaaatnc 720
tggaccntan ggtacaaaa 739

<210> 111
<211> 747
<212> DNA
<213> Homo sapiens

<220>
<221> misc_feature
<222> (1)...(747)
<223> n = a, t, c or g

<220>
<223> nbla-03539-rl

<400> 111
   aaaacctcca gnaatatttt cacactacct tctattttaa agttcacact tttnattcca 60
   gagcagggna tggtcaggcc ngggtgggct ccccnccctc tccccttggc nntggtaacc 120
   actggcccca gggactcagc ctgctttcct atccatcccc tcagtagctg tcaccatgca 180
   ggttacccct tctgtttctt ctaccactaa ctccatgtct gactgcaagt gaaaggaaca 240
   gaagcccaaa cctttgggtt ttaaggagtt tattgctaat ctgtaaaaca gaaagagaca 300
   ggagataagc atgacaaaat atagggaaga aatgactttt gcctaaactt ccaattgtgt 360
   acaattgaag cctctgcttt atagctctta gcacacctct caaataagaa ggcaagtact 420
   gggaaagctc tgaacctgtg gcanaaccac tgatagctgt ggagctattc aaggagtctg 480
   ggaatcaagg ggattatcaa nacattgnta gaataaatta atcttactgg atatatanca 540
   naaanttttc aagcatatgt aaatgctact aataccaaat aattacacct tgttttcttt 600
   aaaccggaac tcttaaanat gnctctacaa aantttttga atnggaangg ctgnatgctc 660
   naaaaacttn aaaacactac tgganaaaaa aggtctcngg aaggngatga aanccntnac 720
   attggaacnt tnatnantta aatnggg 747

<210> 112
<211> 725
<212> DNA
<213> Homo sapiens

<220>

<221> misc_feature
<222> (1)...(725)
<223> n = a, t, c or g

<220>
<223> nbla-03587-f

<400> 112
ttgacagtgc caaagctcgg tactggacac aacgagggac ctgggtctac gataacgnnc 60
ttttgctcct cctgaagtgt ctttggtcca acgttgttcc aggtttgtca ccgtttctct 120
ccggccggtc agggcaagag ccagagtgta ccatggcttc cagtagcact gtgttgatgc 180
ggttggtagc ctccgcatat tctattgctc aaaaggcagg aatgatagtc agacgtgtta 240
ttgctgaagg agacctgggt attgtggaga agacctgtgc aacagacctg cagaccaaag 300
ctgaccgatt ggcacagatg agcatatgtt cttcattggc ccggaaattc cccaaactca 360
caattatagg ggaagagctc gtggtctggg ttgatcctct ggatggaacc aaggaatata 420
ccgaaggtct tcttgacaat gtaacagttc ttattggaat tgcttatgaa ggaaaagcca 480
tagcaggagt tattaaccag ccatattaca actatgaggc aggaccagat gctgtgttgg 540
ggaggacaat ctggggagtt ttaggtttaa gcgcctttgg gtttcaactg aaagaagtcc 600
ctgctgggaa acacattatc acaactactc gatcccatac aacaagttgg gtactgactg 660
ngttgctgct atgaaccccg atgctgtgct gcnagtagga agagcaggaa ataagaatat 720
tcann 725

<210> 113
<211> 734
<212> DNA
<213> Homo sapiens

<220>
<221> misc_feature
<222> (1)...(734)
<223> n = a, t, c or g

<220>
<223> nbla-03587-r1

<400> 113
gatcaaatct ttatttaaaa aaaacttttc tcataaatct gnttcataag catntataat 60
aacatcatnt atattcttaa ttggngtaga aacgttttta aaattactgt gaaaaacaag 120
agtgagattc cagaaaaaat tgtgccctaa agaaatctgg tttaggccag gtgcggtggc 180
tcacacctgc aatcccagca ctctgggagt ccgaggcaga cagatcacga tgtcaggaga 240
tcaagaccat cctggccaac acggcgaaac accctctcta ctaaaaatac aaaaaattag 300

ccaggcgtgg tggcggtgcc tgcagtccca gctactcggg aggctgaggc aggagaatgg 360
catgaacccg ggaggcggag cctgcagtga gccgagatca cgccactaca ctccagccta 420
ggctacagag cgagactccg tctcaggaaa aaaaaagaga aatctggttt agaagatagg 480
aatgttcatt gaacattgcc tgaactgtca attggaacta aagcttttc tcatttctnc 540
acctaaatac aggaaaacaa atntnacata ttgaggttat aagttcttca nacgtcaaaa 600
ttggnctggn tgattaatca ngttaaatta tttaattcta ttttctattc ctaaatataa 660
tancttaatt taaatcctgn tnaacaatga ataaaagngg actntaattt acccattaat 720
cttaaaangn tttn 734

<210> 114
<211> 805
<212> DNA
<213> Homo sapiens

<220>
<221> misc_feature
<222> (1)...(805)
<223> n = a, t, c or g

<220>
<223> nbla-03598-f

<400> 114
ccccgnnngg ccaaaaaaac nggncaaacn ncaanttnnn anaanttttt nacanctnac 60
gancngaang aaagacgggg gggnnnttna naaaanngaa anncncnccc ccagnaagan 120
aancccaanc cacancanaa canncnncga tanaccanca naagaancca aaacacccac 180
tacactcaca acgnacanca aagcaagncc tttaaacaan gaacacacca ccccnaaaan 240
acccccnnnc taccataaac acaaaacact ataantgaac caaaaacaag naaacnaaca 300
nctaanaann aataccgaan tccccnnacc gaaaacaacn taacnccata tnnnccccnc 360
aannnncaac tcanaanaca ancnacataa nnaacccaac aaacacancc naanaaccnn 420
caannantnc cctaaaaaat nnnaanacnn nanancnaca cctccacnac caaccnacga 480
angcaanaac annaaancac naaccnacca acaacanaaa ccanncnnac anngaaaccc 540
cnaaanccaa aacnncaata cnncctaaaa ccnncnccan aaaccannaa cctngctctc 600
ncccanatca acccccntaa aacaaannac atcccannac acctaanata ccaangcacc 660
tnaaancann ccnnanaaan ncaccaaanc canncaccgc aaaannnnnt ccccancnnn 720
aanaaaccnn aaanccnann ccacananca cnacncaanc canaacntan naanacccac 780
ncaaacactc ttcntcacan ncncg 805

<210> 115
<211> 752

<212> DNA
<213> Homo sapiens

<220>
<221> misc_feature
<222> (1)...(752)
<223> n = a, t, c or g

<220>
<223> nbla-03598-r1

<400> 115
tnagtttggg tggtagcctg tgactacagt cccagctaat caggaggctg aagcagaagg 60
accntttgag cccaggagtt ccaggttaca gtgagngntg atcttgccaa cacactctag 120
gctgggtgag acagtgagac tccatctcta aaataagaat tataaaaata atagcttcat 180
atgtcaacac attcaccaat gctgaggccg atttcctcca aatcatttta gtatattaaa 240
ccgttataat acatggattt tgaggaaagt ccttcaatta ttaacagtag ctagtgaatc 300
ttttatgtga atattttcgc aaccatgagg agagaacacc aagcttttgc ttcccaccac 360
tgctccactg gaataagccc atctcattaa ggacattacc tgccctgcca taggacggtg 420
agcagcttca gcctctgcct ctgnttttcc aaattactcg gngaagatca atattccagg 480
tgtgaagatc ccaggacaag cagtaaagat gtgaagaggg ttncattcac ngggattcaa 540
cgtttaagcc ttcattagca caacaacctt taaanggccn ccacccaaat tgccaatcca 600
aattgccaat ccaaattggg gtgtgggctg gtgaatcaaa aggncangtt ccagctactt 660
taagggaata ganggggttt cttaangggg aagagnccna aaggttaact ctattgnaac 720
canggttaaa tcccnagccn tanaagnact gg 752

<210> 116
<211> 3168
<212> DNA
<213> Homo sapiens

<220>
<221> misc_feature
<222> (1)...(3168)
<223> n = a, t, c or g

<220>
<223> nbla-03614

<400> 116
ttctaggcct gtacggaagt gttacttctg ctctaaaagc tgcggaattc ctcgagcact 60

gttggcctac tgggaaaatc aagccatata catttttttc cttcgaaagt gtctaagcaa 120
caaaataaat aaatgtatta ttcattctgg gttgtgttaa tttttcctgc cagaggggaa 180
aaatgaattt acagggaatt cctggaagaa tatcttggga cctaaaaata aaaatcatgg 240
ttttatttta agcttgtcaa caggggtgac aagtgtaaga attgtagccc agatgtgcgg 300
gaagcaactc ataaattcta ttttccacta tgttttatct tgcctgccac tttccctaaa 360
atggaaatca tttttagtat catagagcga gatgggaaac gtggaagttt atttgtaaaa 420
gtggcactat ggtgagcaag cattcacagg gacactggcc tattttagca gctggcctct 480
gggagagagg gatgccctac agcttggcac tggagctctt tgggatgctg ctgagcttgt 540
cattgctgtt cctctttggg gtctccaggc tgcttttaaa ttctctagga ggtgaccatg 600
ggaccttatt agatgagctt taatttctct ttctaagttg gcttgtgcaa atacagatat 660
ctagtttttt ttcagtcaga ggggagagga aggatagagt tgaggagtgg acagtaagca 720
tccccattca gaggcagatg cagcctggtt tggggagacg gtagaggact agaacatatt 780
agtgtgtatg gagctggcct gggcgctgag atccagagtg aggaatgtgc cagagattcc 840
atctgtttga gctggcatga atcatagtgt gaatcagtag atctcttttc ttagcaaaaa 900
ccagcaaaag cccttcttgt gagggtcata tgaagcggaa ggagatgaga ttctatattg 960
atttagtact agttcatgag ctaccaaatc taaaaactag gagtcaggtc agataaaata 1020
gcatatttct agcttaaagt tttatgatca tgaatcttat agaaacatag ttaacttaga 1080
aattaaagat ttaagattaa gttagaaata gaagttgcta gaagttctca aatgtatact 1140
tctgacaact tggttaaatc cctggcatat gcagaaaagt ttctgtgatt aaaaaataac 1200
agaagtatga gagtttttcg ttgtccagcc aatttgtag tctatttgag aaggttaaaa 1260
acaactgtaa aagctaagta acaaggaaac aaaaaaatga tgagtatata accagaagga 1320
gcccaggtac tataaaagct aagtaacgag gaaacaaaaa aatgatgagt atataaccag 1380
aaggaacact caatttgatt cccatatcag gaagttcttg ggttcagtgc aagacttgaa 1440
acctgacatg gtttcattag attgtagaaa acgctttttt tttttttttc tggacaacag 1500
atggaaccat gttgtcggtt gttcaaagat ggacaggaga cagaaatcta tcaacctgaa 1560
gtcagctcca actgcagctg tctgttctcc tgaggactgt gctagtggtc cacagaggtc 1620
accttgctta tcacatgata ttttcttgac gttagttgaa gcacatcacc ttaggtgaca 1680
catgagggcc aaaggcctct ttctcaggga tgtatgtgaa agtgtggtgg gttggggacc 1740
agctaaggaa aaaaagacat gaaggagaat gcaagcatct tacagtcatc attcagtaag 1800
aaaactttat gactggacaa aggaaaacat aaacgccctt taacctagaa tgcaaatttc 1860
tagtgatttt tactttttta gtgtatcaga agtcactgag caaggtaagt accagcactt 1920
acagagacgg agtcataaag gctgctctct aaaagtagag gatgtgcaaa acctgtagta 1980
ccatttaaca gatttttgac cttgttaatt atgtcatttt gaaactggaa taaatatgta 2040
attcgaaggt tgatttttct gaacaaaagt aagtcaagtc aaagttttct cagagtctac 2100
tgtttgattc ttgagggtat tcagaattca agattgatgt gcatttcaac atttcagttt 2160
ctgcctaagt gaagcgtcac ttaaagcctt cgaaattaca gatgtaaaat gaggttgcac 2220
gtgatgtcag tagctataat tcctactctg gaggtagctc gatgtgtctg aaggctgagt 2280
gagctgcggc aggatgaatg aatctttgac ttcacgagat gctgttctag tctgtaacct 2340
tgtcgtgtgg aatcagccca tctgatgctg aacactaact tgaaatggct ggccgaatgc 2400
agagtaacac atcagagcac tagaccggtg gaaagaacct agattgcaag tccagtgaat 2460
tatctgcatc ttcattttgt taggtgtaga tttacttttg ttattggttg caattgcaat 2520
attttaaaat catgattatg cgttaactcc atctatttct aaaacattgg tatttgtgtc 2580

atttttagtg tagcttgatt tttgttttct tctttctggt acaaaaacga gtttctccct 2640
ccaacccctg ccatgtatat ctgtatcatg tatatctgta tctacattcc tgtctactat 2700
caatgcctac tatttacaag gtagttcagt tcgtttcaga tcaactagaa tttattgagt 2760
actctgtatg ctaatccctg taccaggggc ttgggatata acagtgaata agttcctggg 2820
gcttacatca gtcaatatag ggttactgtc tattgaacaa gattattttt gggtttctat 2880
ctatttgggg atatgataat gcatgtattt attcctaacc atcacacaat aatattatga 2940
aacaatgtta gtaggaggaa gatgagagcc ttcattttaa ctgggaggac ttggttctgt 3000
ctaagatgtt gcttttgtta tgggttagtg cctattagat aacattctgt ttctgtgact 3060
gattatttct ttttgacctt ctgagtttta gtttgtccac tgagttttag gtaaaaaaaa 3120
aaaaaaaaag gccacatgtg ctcgagctgc aggtcgcggc cgctagac 3168

<210> 117
<211> 758
<212> DNA
<213> Homo sapiens

<220>
<221> misc_feature
<222> (1)..(758)
<223> n = a, t, c or g

<220>
<223> nbla-03615-f

<400> 117
actggnttct ttaaaaaaat cccaacaaaa gtaagataat gcctttctta aactctcttc 60
ataaatctgt actattacat tcctcttttt aaccagtatc ataagttggt tttcatttat 120
ttaaagctta ttggaaaagt tagaattata ggagtagtag gcatatacac tcacatacaa 180
ggcaaaacct atattggtgc ctaactttat tatacatgag attgggccag aaatgggaaa 240
agacaggtat ttaccatgaa tttttattat tgntctttct acataagatt gctaactttt 300
agaacataaa ctgtataatt ataagccata acctcttcca ttttgtaaaa ttaatactct 360
gtcaatcaga attaagagac agccacattt gggccataag tgaatttaat actgcttttc 420
aacacaaaga aatggtttcc aaatatttgn attaagttcg ttgtaggtca gtatacattt 480
tatgggtggt ctgctaatgc cactttgtaa accctgnata atagtcatta ctaaacaaac 540
ttcagntatt tttaagtaaa tcatatttcg acaattattc caacaagttt aaagccttaa 600
gtaaatcatc ttttgacaat tcttcaacaa agcttgncag aatttggata aactgataaa 660
agtcaatatt tacaaatttt ttaaaatcag tggcactnaa tattttcaac tggaattttt 720
aacccattaa ggnaagtgga ataaatctat tttagtcn 758

<210> 118

<211> 778
<212> DNA
<213> Homo sapiens

<220>
<221> misc_feature
<222> (1)...(778)
<223> n = a, t, c or g

<220>
<223> nbla-03615-r1

<400> 118
   catttnacat ttnaaagcta taattttcaa ttatgtcctt ttgtttttcc tgagatgata 60
   aanntgaaag gttacttgac attcattgag attatgaaac attctaataa aatatacttt 120
   tcagtttagt tatggaagcc caaaatgtat tcagatggaa ctgaaatatt tctcaatcag 180
   taaaatgaag tatttcacaa gtttttctga tgtgaataac tgatattatt aggttactga 240
   aaaatgtgat tcataatgat tattatttgg ttgagtgtct acaatgtgct aggcactgac 300
   aaacataata taaattcatt accttcttga atattcctac caaatgaact tggctgctat 360
   cctcaattaa caaatgaaaa aacagacaaa agctagaaag caaactcatg ctctgatcca 420
   tagcccaagc tcttgagctg ntatataaag aaatctatgc tatgcagatt tcagtctgga 480
   gtctctgagc atagacagtt catgaagatc aagcctggag aatagtcttc ttataccggg 540
   gaggtccggc atactgncta ccatcaaatc actactnaag cagttcaaat catttctaac 600
   caaacacttt ggatggattc tagaaaatga tacctccaaa ccataccctg gaancctact 660
   ccnttttact atttaataat cccttttaaa aaggnggcct tttntaacag caaagangga 720
   aacancntga tgggtnaaag gtttggntta ataacagatc cgntttgncc ttctagat 778

<210> 119
<211> 757
<212> DNA
<213> Homo sapiens

<220>
<221> misc_feature
<222> (1)...(757)
<223> n = a, t, c or g

<220>
<223> nbla-03636-f

<400> 119
   gcctactgtn acagccaagc agtggnaaga gctttaaact cagcatcacc aaacaggacg 60
   atggaggggc ccctattttg gaatacattg tgaaatatgg gnagtaaaga taaggaagac 120
   caatggctag agaaaaaagt gcaaggaaat aaagaccaca tcattttgga gcatctccag 180
   tggaccatgg gtatgaagtt cagattacag ctgccaatag attgggatat tctgaaccga 240
   cagtttatga attcagcatg ccaccaaagc ccaacattat caaagataaa tgctgtgaag 300
   cgaataaagg cgaaaatgga agccagtcgt ggcagctgaa tgctgctggg ttttctttca 360
   ttatagccat aagtttgtct tgcgtgtttt aatgtcattg gtatatcata tggtatatcc 420
   tcttatgccc tgaaaattaa aaaaaaaata gtaatagcaa ggcttatcta aaattataat 480
   aaaatatttc ctaaggncca attaattatg catgnatctt cacccatgaa tgattaaacc 540
   ttttctcctt ncccaantat caancctttt aagtggatan tttgncagaa aatcaaatgg 600
   aaggaattca atcngggatt gngtactttc aagttaccag ttcactgnat accatcctta 660
   atctaaaatt tccattaatt cctaaaagtg anccttgtaa atacctggta ccttggcctt 720
   gnaacttaan cctttggtac atntaacctt cattatt 757

<210> 120
<211> 751
<212> DNA
<213> Homo sapiens

<220>
<221> misc_feature
<222> (1)...(751)
<223> n = a, t, c or g

<220>
<223> nbla-03636-r1

<400> 120
tttttttttt gagacggagt ctcggtctgt tgcccaggct ggagtgcagt ggcgcnatct 60
cggctcactg caagctccgc ctnccgggtt cacgccatgc tnntgcctna gcctcccgag 120
tagctgggac tacaggcgcc cgccaccacg cctggctaat tttttgcatt ttttagtaga 180
gacggggttt taccgtgtta gccgggatgg tctcaatctc ctgacctcgt gatccacctg 240
cctcggcctn ccaaagtgct aggattacag gcgtgagcca ccgcgcccag ccggccanaa 300
gtgtgtttta tcggttgatg ggttgcttct gcagtctaca ggtctattac tagattacaa 360
gaaaggatag canttgacag ctaggagttt ggaaaagata agttaaaaga aaatctacta 420
tgaaagaaag gcctaccctc atatgaaaag attctgnagc tatctcttta aaaagagaca 480
cgaaaaatat ttctggtcaa caaaaatgat agcttttgat gaatgaatca cagaagtcca 540
gaacccaatc attggattta nccaagtntg tatgggcact tttatgcata tatgtcatnt 600
ngggtggttc ctattggcct aaataataag tngtaaaagt nccttgttaa aatggggttc 660
aagtgaaaac ccaacctntt tacttggttt gaattcctta ggnaaagatt ncttaagnga 720
aaatgngtnt tnccttanng aaatttgcaa a 751

<210> 121
<211> 4219
<212> DNA
<213> Homo sapiens

<220>
<221> misc_feature
<222> (1)...(4219)
<223> n = a, t, c or g

<220>
<223> nbla-03646

<400> 121
   cactgttggc ctactgggct ggcggcagtg acaggaggcg cgaacccgca gcgcttaccg 60
   cgcggcgccg caccatggag cccgccgtgt cgctggccgt gtgcgcgctg ctcttcctgc 120
   tgtgggtgcg cctgaagggg ctggagttcg cttatcttcg atatctacta ctacgtgcgc 180
   gcctgggtgg tgttcaagct cagcagcgct ccgcgcctgc acgagcagcg cgtgcgggac 240
   atccagaagc aggtgcggga atggaaggag cagggtagca agaccttcat gtgcacgggg 300
   cgccctggct ggctcactgt ctcactacgt gtcgggaagt acaagaagac acacaaaaac 360
   atcatgatca acctgatgga cattctggaa gtggacacca agaaacagat tgtccgtgtg 420
   gagcccttgg tgaccatggg ccaggtgact gccctgctga cctccattgg ctggactctc 480
   cccgtgttgc ctgagcttga tgacctcaca gtggggggct tgatcatggg cacaggcatc 540
   gagtcatcat cccacaagta cggcctgttc caacacatct gcactgctta cgagctggtc 600
   ctggctgatg gcagctttgt gcgatgcact ccgtccgaaa actcagacct gttctatgcc 660
   gtaccctggt cctgtgggac gctgggtttc ctggtggccg ctgagatccg catcatccct 720
   gccaagaagt acgtcaagct gcgtttcgag ccagtgcggg gcctggaggc tatctgtgcc 780
   aagttcaccc acgagtccca gcggcaggag aaccacttcg tggaagggct gctctactcc 840
   ctggatgagg ctgtcattat gacaggggtc atgacagatg aggcagagcc cagcaagctg 900
   aatagcattg gcaattacta caagccgtgg ttctttaagc atgtggagaa ctatctgaag 960
   acaaaccgag agggcctgga gtacattccc ttgagacact actaccaccg ccacacgcgc 1020
   agcatcttct gggagctcca ggacatcatc ccctttggca acaaccccat cttccgctac 1080
   ctctttggct ggatggtgcc tcccaagatc tccctcctga agctgaccca gggtgagacc 1140
   ctgcgcaagc tgtacgagca gcaccacgtg gtgcaggaca tgctggtgcc catgaagtgc 1200
   ctgcagcagg ccctgcacac cttccaaaac gacatccacg tctaccccat ctggctgtgt 1260
   ccgttcatcc tgcccagcca gccaggccta gtgcacccca aaggaaatga ggcagagctc 1320
   tacatcgaca ttggagcata tggggagccg cgtgtgaaac actttgaagc caggtcctgc 1380
   atgaggcagc tggagaagtt tgtccgcagc gtgcatggct tccagatgct gtatgccgac 1440
   tgctacatga accgggagga gttctgggag atgtttgatg gctccttgta ccacaagctg 1500
   cgagagaagc tgggttgcca ggacgccttc cccgaggtgt acgacaagat ctgcaaggcc 1560
   gccaggcact gagctggagc ccgcctggag agacagacac gtgtgagtgg tcaggcatct 1620
   tcccttcact caagcttggc tgctttccta gatccacact ttcaaagaga aacccctcca 1680
   gaactcccac cctgacagcc caacaccacc ttcctcctgg cttccagggg gcagcccagt 1740
   ggaatggaaa gaatgtggga tttggagtca gacaagcctg agtccagttc cccgtttaga 1800
   actcattagc tgtgtgactc tgggtgagtc ccttaacccc tctgagcccg ggtctcttca 1860
   ttagttgaaa gggatagtaa tacctacttg caggttgttg tcatctgagt tgagcactgg 1920
   tcacattgaa ggtgctgggt aagtggtagc tcttgttgct tcccgttcag cgtcacatct 1980
   gcagtggagc ctgaaaaggc tccacattag gtcacctgtg cacagccatg gctggaatga 2040
   tgaaggggat acgctggagt tgccctgcca tcgcctccat cagccagacg aggtcctcac 2100
   aggagaagga cagctcttcc ccaccctggg atctcaggag ggcagccacg gagtggggag 2160
   gccccagatg cgctgtgcca aagccaggtc cgaggccaaa gttctccctg ccatccttgg 2220
   tgccgtcctg ccccttcctc cttcatgcct gggcctgcag gcccacccca gccaccactg 2280
   agtccactcg gagtgccctg tgttcctgga gaaggcattc cagggttgaa tcttgtccca 2340
   gcctcagcct gggacaccta ggtggagaga gtggtctccg ctctgaattg gatccagggg 2400
   acctgggctc attcttcttg gctcaccaac cctgcaggcc tcatctttcc caaaacccac 2460
   tttgtcttgg tgggagtggg tccgcgctgc tctgcagcag gggctgggga gtggacagca 2520
   tcaggtggga aagtggagtc caccctcatg tttctgtagg attctcaccg tggggctgga 2580
   agaaaagagc atcgacttga tttctccaac cactcatccc tctttttctt tcttccacca 2640
   ctccccaccc cagctgtagt taatttcagt gccttacaaa tcctaagctc agagaaagtt 2700
   ccatttccgt tccagaggga agggaacctc cctaggtcct tccctggctt gttataacgc 2760
   aaagcttggt tgtttatgca actctatctt aagaactgcc cagcctcagc tgaaaacccg 2820
   aatctgagaa ggaattgcgt catgtaaggg aagctggaat taagggagct gagccagtca 2880
   tggttgtggc gtgtgagtca ggagacctag gtttcagccc ctctctactg tcagcgagct 2940
   gtgcaacgtg ggcaagtcat tgtcctctga gctgcagttt cctcatctgt cacatcgcta 3000
   cagacaagac ctccctggaa cccttctgat tgtcttagac actgtggttg caaaacccac 3060
   ggaaagcctc atttgtgtgg aaagtcagag gaaaaatgat ccagtggaca cttggggatt 3120
   atctgtcatt caagatcctt ccttcaaccc caaggtcagc tcccatctca tttccagaaa 3180
   ggctcatacc tggcttgcag ggaagcatct gtcttgtcat tccaggtgcc agaatcctct 3240
   cagagtcatt gaagggtgtt cacccatccc acccaaggct tggcacactg ccagtgtctt 3300
   agcagggtct tgtgagggct gggggcatcc aggcactcag aaggcaaagg aaccacccta 3360
   cccatttggc ctctggaggg ggcagaagaa agaaagaaac ctcatcctat attttacaaa 3420
   gcatgtgaat tctggcatta gctctcatag gagacccatg tgcttccttg ctcagtgcaa 3480
   aactgatgat tctacttgct gtagatgaat ggttaacacg agctagttaa acagtgccat 3540
   tgttttgcca gtgaagcctc caaccctaag ccactgggac ggtggccaga gatgccagca 3600
   gcctctgtcg cccttagtca tataaccaaa atccagacct tatccacaac ccggggcttg 3660
   gaaaggaagg tattttggaa tcacaccctc cggttatgtt gctccagtaa aatcttgcct 3720
   ggaaagaggc agtcttctta gcatggtgag ctgagttcat ggcttttttt tgtagccagt 3780
   cctgtccctg gccatccatg tgatggtttt ggatggagtt aaacttgatg ccagtgggca 3840
   gtgcatgtgg aaagtatcag agtaaggctc tcccctccag agccctgagt ttcttggctg 3900
   catgaaggtt ttctttagaa tcagaattgt agccagtttc tttggccaga aggatgaata 3960
   cttggatatt actgaaaggg aggggtggag atgggtgtgg cagtgtatgg tgtgtgattt 4020
   ttatttcttt ctttggtcat gggggccaag gagaaaggca tgaatcttcc ctgtcaggct 4080
   cttacagcca caggcactgt gtctactgtc tggaagacat gtccccgtgg ctgtggggcc 4140
   gctgcttctg tttaaataaa agtggcctgg aaaaaaaaaa aaaaaaaaaa aaaaaaggcc 4200
   acatgtgctc gagctgcag 4219

<210> 122
<211> 726
<212> DNA
<213> Homo sapiens

<220>
<221> misc_feature
<222> (1)...(726)
<223> n = a, t, c or g

<220>
<223> nbla-03652-f

<400> 122
ngttgcccta ctggctcttg gcggcggagg ctttgtctcg tgggctggtc cccagcggct 60
ccctccccga acagctgctg ctccagggag gaagcggcgc gggtgctgct ccagcttccc 120
ggtgctgaaa accggagggc tcgtcatcca ccactaccat gtaagggcca tgagaagggc 180
tcatcctggc gcagcgcgga catggaggag gacttattcc agctaaggca gctgccggtt 240
gtgaaattcc gtcgcacagg cgagagtgca aggtcagagg acgacacggc ttcaggagag 300
catgaagtcc agattgaagg ggtccacgtg ggcctagagg ctgtggagct ggatgatggg 360
gcagctgtgc ccaaggagtt tgccaatccc accgatgata ctttcatggt ggaagatgca 420
gtggaagcca ttggctttgg aaaatttcag tggaagctgt ctgttctcac tggcttggct 480
tgggtggtct ttgtaggcat gatgtccagc tccacgctct ggggaaatat ctcagaccag 540
tacggcagga aaacagggct gaaagatcan cgtgctgtgg actctgtact atggcatcct 600
tagtgcattt gcgcccgtgt atagctggat cctggtgctt ccggggcctg gtgggctttn 660
ggaatcggag gagttcccaa tccgtgacct tnatccgagt ncttccatga aagcnnactt 720
aatgtt 726

<210> 123
<211> 749
<212> DNA
<213> Homo sapiens

<220>
<221> misc_feature
<222> (1)...(749)

<223> n = a, t, c or g

<220>
<223> nbla-03652-r1

<400> 123
tgaatccctt tttttggtcc aggtcatact cttgggtgag ttcttgatgt cggcagtgac 60
aatcagtgcc ccagttgggg cctgccagcc ccccaagctc tgnagcctca aagaccagct 120
cagtccccca tcggtcacta ttcctgagag ccagagttcg acctggtaac acctgcaccg 180
tgcattcctc ggccgaccat ctcctggccc cactcccggt ggctggactc ctgcagtcct 240
cggcctttgg tctcaatggg caaaaagcag gaggccaggg cagccaggag gcagcagcca 300
ctgtaaactg ccagagtcag gtacacagag gattccagca tcacctgggc gatgaacgga 360
gtgatgagag cacccactct tgccatgccg ctgcaggtgc ccaggccgag ggcccgcgtt 420
gccgtggggt agacctcagg tgtgtaaaca tatgccgctt gaaagcctcc agaaataaac 480
gctcttgcaa tgaagagtaa cagagtgagc acatttcttc aacacagata aacagcagga 540
ggcttgcaga aggagaagat gacaaagcac anggccatgg ncttcttgcg ccccaagcgg 600
ncaataatnc acagagtcac aagggcacct ggnaactcan aaaagggtgg gtccacaagn 660
aagtccatgt aatccttctt anttaagnac ttcgnaggnc aagcttggaa ttttggcctt 720
taaagccttt tttcgacttg gagatgccc 749

<210> 124
<211> 1990
<212> DNA
<213> Homo sapiens

<220>
<221> misc_feature
<222> (1)...(1990)
<223> n = a, t, c or g

<220>
<223> nbla-03755

<400> 124
   ttctaggcct gtacggaagt gttacttctg ctctaaaagc tgcggaattc ctcgagcact 60
   gttggcctac tggcaaaaaa aagaataatg acccggcccc cgccctctgc ccccagctgc 120
   tcctcgcagt tcggttaatt ggttaatcac ttaacctgct tttgtcactc ggctttggct 180
   cgggacttca aaatcagtga tgggagtaag agcaaatttc atctttccaa attgatgggt 240
   gggctagtaa taaaatattt aaaaaaaaac attcaaaaac atggccacat ccaacatttc 300
   ctcaggcaat tccttttgat tcttttttct tccccctcca tgtagaagag ggagaaggag 360
   aggctctgaa agctgcttct gggggatttc aagggactgg gggtgccaac cacctctggc 420
   cctgttgtgg gggtgtcaca gaggcagtgg cagcaacaaa ggatttgaaa cttggtgtgt 480
   tcgtggagcc acaggcagac gatgtcaacc ttgtgtgagt gtgacggggg ttggggtggg 540
   gcgggaggcc acgggggagg ccgaggcagg ggctgggcag aggggagagg aagcacaaga 600
   agtgggagtg ggagaggaag ccacgtgctg gagagtagac atccccctcc ttgccgctgg 660
   gagagccaag gcctatgcca cctgcagcgt ctgagcggcc gcctgtcctt ggtggccggg 720
   ggtgggggcc tgctgtgggt cagtgtgcca ccctctgcag ggcagcctgt gggagaaggg 780
   acagcgggta aaaagagaag gcaagctggc aggagggtgg cacttcgtgg atgacctcct 840
   tagaaaagac tgaccttgat gtcttgagag cgctggcctc ttcctccctc cctgcagggt 900
   agggggcctg agttgagggg cttccctctg ctccacagaa accctgtttt attgagttct 960
   gaaggttgga actgctgcca tgattttggc cactttgcag acctgggact ttagggctaa 1020
   ccagttctct ttgtaaggac ttgtgcctct tgggagacgt ccacccgttt ccaagcctgg 1080
   gccactggca tctctggagt gtgtgggggt ctgggaggca ggtcccgagc cccctgtcct 1140
   tcccacggcc actgcagtca ccccgtctgc gccgctgtgc tgttgtctgc cgtgagagcc 1200
   caatcactgc ctatacccct catcacacgt cacaatgtcc cgaattccca gcctcaccac 1260
   cccttctcag taatgaccct ggttggttgc aggaggtacc tactccatac tgagggtgaa 1320
   attaagggaa ggcaaagtcc aggcacaaga gtgggacccc agcctctcac tctcagttcc 1380
   actcatccaa ctgggaccct caccacgaat ctcatgatct gattcggttc cctgtctcct 1440
   cctcccgtca cagatgtgag ccagggcact gctcagctgt gaccctaggt gtttctgcct 1500
   tgttgacatg gagagagccc tttcccctga gaaggcctgg ccccttcctg tgctgagccc 1560
   acagcagcag gctgggcgtc ttggttgtca gtggcggcac caggatggaa gggcaaggca 1620
   cccagggcag gcccacagtc ccgctgtccc ccacttgcac cctagcttgt agctgccaac 1680
   ctcccagaca gcccagcccg ctgctcagct ccacatgcat agtatcagcc ctccacaccc 1740
   gacaaagggg aacacacccc cttggaaatg gttcttttcc cccagtccca gctggaagcc 1800
   atgctgtctg ttctgctgga gcagctgaac atatacatag atgttgccct gccctcccca 1860
   tctgcaccct gttgagttgt agttggattt gtctgtttat gcttggattc accagagtga 1920
   ctatgattga aaagaaaaaa aaaaaaaaaa aggccacatg tgctcgagct gcaggtcgcg 1980
   gccgctagac 1990

<210> 125
<211> 2180
<212> DNA
<213> Homo sapiens

<220>
<221> misc_feature
<222> (1)...(2180)
<223> n = a, t, c or g

<220>
<223> nbla-03771

<400> 125
cactgttggc ctactggcag ataaagcctt atgctgccca ccagcccact aaatgtatta 60
aatacctgtc tctatgtagc ttatgtaaaa actcaatgtt gactgtcccg tgtctgctgc 120
atttaaaagc tcattgtgat tctatcatct tgctatgcca atgccttatg ttatggtgtc 180
atgtatatag gccatggtac aaaagtgact gtcaactgct tactcaacat ctagtcagaa 240
aaggtctgag gcagtgcaat aacgctttta gtcaaactgg ctcactgttg gagtcattta 300
catctgtgta ttctttaccg taaatactga aatagtattt tttaactgtt ttttcaggct 360
tgtaataaat atctgtgtca tatctacata gtcaaaatac attgagtaat tcagtttaaa 420
agtgttgcct actaacaaac taaagagaaa catctactga ttttcccata taattgctta 480
ttttccattg ccaatgtaga cctgcctgga atggtgtctt tcaccactat catgtgtaaa 540
ataaagggag gctattgtgg tgaattttca cctgtctgac attagctctt tcactagtca 600
aaggatgtac atacctcaga ggtgacctga ctaccgaggt ccagttttca aaagtgcatc 660
ttattttaat ttttgtctcc aaaaattaaa attggatgtc tacaaaacta acatatgtag 720
actttcaaag gccagcattg gtggggaagg gaaaagaaag gatggaagga tggaaggaac 780
gaagggtgat gaggagatga agggagaggg aggggaaaga aaagaacaga aaaaagctaa 840
tagaggaagg taaatctcta aatgagttaa ataaggctta aataaagttt gcatggtata 900
ttctctaaaa gttgattggc gtggaggagg ctgtatgtgt ttgtgtgtgt atgcacatgg 960
gcatgtctat gtgtaaggtc agaaggggga gcctgaatta tctactgctt ttttggttaa 1020
ctagtcctat ttttaaaact ttgtctaact cccaaaactt attaatgcga catgtagtac 1080
tgaaattaga agattattgt taaactcagg acagctctgt ggagattcac aaagtaattt 1140
catgaaaact tatgaggaag ttgttcagta ctgtcactaa ttaacgcgca caaaaaaaaa 1200
acttgtgagt aattatgggt gatttcgctg caaaacaaat tagtatatga attattattt 1260
tttctcaaat tgtagaggtt ttgtttctat taagtctcta acatttccca gattaaaaat 1320
accaaaacta ttttttaaac ttaaggaata gagtatcaga aattaaccat taaattaata 1380
gttcttaaag ttgctatgta tatggtaagt aaaacaattt cctatttcta agttatacat 1440
ggtattagat ttctaggtac agtcgcatga agcattattg acttttatta acttcagtgt 1500
tttagagggt tttcatacta gtctatgaat gctctgaatg ctgcagggaa aaagaagcac 1560
aatcagtatt tcattattta tgaattctca attgaattat cctatcctgt ccacacacat 1620
aatagcatta gcatttcttc ttataccatg ttgaaatact tttaatacaa actctcaata 1680
gaagcttaaa cataagtggt taagtcttgt tgtctagtct cattcacctg cctcaacatg 1740
ctttctttca ttctatttgc atacaaaatg ttcttatttc agttttgtag acaggatatg 1800
agttagcata ctcgtgtttg ttcagctgtc catcctgcat cgttactaca atgccttttt 1860
ctgccattta atggtgtttg tatcaatgtt cccatatctg ctgcatttta actccataaa 1920
aaggaaatgt gatttcgtat taatagtttt gttgatcaac tcaatatttc tgcaccaatc 1980
agcataccta tatgcatgta gtagtctgta caattgttca acatcaaaat acttgtttac 2040
tttatgtcaa aatgtctata aaattgctgg cattgttctc catttcagtc tggtagaata 2100
agcgagatag aaaataaatg tgtaaatgaa aaaaaaaaaa aaaaaaaaaa aaaaaaaggc 2160
cacatgtgct cgagctgcag 2180

<210> 126
<211> 2078

<212> DNA
<213> Homo sapiens

<220>
<221> misc_feature
<222> (1)...(2078)
<223> n = a, t, c or g

<220>
<223> nbla-03777

<400> 126
cactgttggc ctactggttt aatctcattt tctcctaaag ttcttctctt caatatgatc 60
tgcaaagggt tgaaagtgct gtcctccaaa ccaggcgtaa gaaagtggtt acagataaag 120
caagtaaatc cattaaagaa gaaatgggaa gatctgttgt gtgccttatc tgacccagca 180
ttatcgctgg aggggaagtg cttataggga aggaagagaa gtttggtcaa ttgatgcagt 240
ctcagtatga tgacagctgg tgacttcctg agggttattc atcacacttc agcagggtga 300
tgttttcaaa gcctgcgtgt gaccatgtca ctcctctgtt ctccagcgct ttcaaaataa 360
aactgaaatc cgtctcagag ggccagtctc agagtatccc ctgccagccc catccttcac 420
tgctcagcag tcacctcctt ccccctcctc atccttttga ggtctcttct gatccttcag 480
gggccagctt ctctccgtcc catggttgtt tgcctatgct gatcccctac atggatctcc 540
ggtcacctcc atcactctta cctggttagt ctctccctgg cctttatccc aggtcagctt 600
cgcttctgca ggaatgcctg ccttgaaacc tcaggtcctc ctgttccccc tcttagagca 660
ctgggctatt ctcctgggag gcagtaattc cagtagtaat ttgcatacat ttgtaagcag 720
ttttgattaa tgcctgttgt ttccactaga ctctatgctc tgtaggggac aaatcttgtc 780
tgctttttgc tcatcaattt ttccccagca cagagctcaa tactggcact ggtaaaccct 840
tgagaaaatg attaaaagcc tctaagatgc atgcagttat gaagaaccaa cagaaggtac 900
aggtggcatg ggacagcagt caggaagcaa tttggcttaa ggaggaaggc cagtgactca 960
cagatggagt ctcagcactt cccttgcccg tagaagtgag aaacaataca ggtagtcaca 1020
gcatagtaaa aatttcaggc caggcacggt ggctcacggc tgtaatccca gcactttggg 1080
aggctgatgt gggtgggtca caaggtgaag agatcaggag catcgtggcc aacatggtga 1140
aaccccatct ctactcaaag tgcaaaagtt aactgggcat ggtgacacag gcctttagac 1200
ccagctgctc gggagactga ggcgggagag tcgcttgaac ccaggaggaa gaggttgcag 1260
tcagtcgaca tcacgccatt gcactccagc ctggcaacag agcgagacgt gtctttaaaa 1320
aaaaaaaaat cagatagcag tttcatatga ctagaggctg tgggctgatc agatcctgaa 1380
aaaccgggca tggaccaagc tggctaagac caagtggacc caacatggtg ctgaatttga 1440
cctaggattc acctaggacc tcgtgatgtg tttgttaaca tcctaagtca ctgtgacttg 1500
ggatgttaat gagtgactta ccatgttaat gagcatatca cgaggtccta ggtgaccagc 1560
accatgaatt ccgagaatac ctacgtttgg tgtaaaaatg gatggcatca caattctaag 1620
aaatttccac cctttcccag gaattttcat aagtattcca ccccttggct aaagaaacac 1680
ataaaggtag cagccccaaa tccccttagg catggtgctc tcttgagtcc gcctacactc 1740
cccctttctt gagtgtgtac ttttcccgtt gttataaatc tctgtacttc cactgttgcc 1800

tgactcatcc ttgaattcct tcttgagacg gtgtcaagag cctggacacc tgccgggatc 1860
agggtcccac cagcatttgg ggacctcccc aagcccacta gtatcagtag agtccctata 1920
ggtaattgtc taataagtgg aaggtgggtg tagaagtttc aagaaaccaa agaaatgtca 1980
gatgtgtcat ttgacccaaa agaaaagcgt tttaaaggaa gttaatgaaa tgcagtcaga 2040
aaaaaaaaaa aaaaaggcca catgtgctcg agctgcag 2078

<210> 127
<211> 1957
<212> DNA
<213> Homo sapiens

<220>
<221> misc_feature
<222> (1)...(1957)
<223> n = a, t, c or g

<220>
<223> nbla-03779

<400> 127
gaattcctcg agcactgttg gcctactggg atcctgtttg acatttttat ggctgtattt 60
gtaaacttaa acacaccagt gtctgttctt gatgcagttg ctatttagga tgagttaagt 120
gcctggggag tccctcaaaa ggttaaaggg attcccatca ttggaatctt atcaccagat 180
aggcaagttt atgaccaaac aagagagtac tggctttatc ctctaacctc atattttctc 240
ccacttggca agtcctttgt ggcatttatt catcagtcag ggtgtccgat tggtcctaga 300
acttccaaag gctgcttgtc atagaagcca ttgcatctat aaagcaacgg ctcctgttaa 360
atggtatctc ctttctgagg ctcctactaa aagtcatttg ttacctaaac ttatgtgctt 420
aacaggcaat gcttctcaga ccacaaagca gaaagaagaa gaaaagctcc tgactaaatc 480
agggctgggc ttagacagag ttgatctgta gaatatcttt aaaggagaga tgtcaacttt 540
ctgcactatt cccagcctct gctcctccct gtctaccctc tcccctccct ctctccctcc 600
acttcacccc acaatcttga aaaacttcct ttctcttctg tgaacatcat tggccagatc 660
cattttcagt ggtctggatt tctttttatt ttcttttcaa cttgaaagaa actggacatt 720
aggccactat gtgttgttac tgccactagt gttcaagtgc ctcttgtttt cccagagatt 780
tcctgggtct gccagaggcc cagacaggct cactcaagct ctttaactga aaagcaacaa 840
gccactccag gacaaggttc aaaatggtta caacagcctc tacctgtcgc cccaggagga 900
aaggggtagt gatacaagtc tcatagccag agatggtttt ccactccttc tagatattcc 960
caaaaagagg ctgagacagg aggttatttt caattttatt ttggaattaa atactttttt 1020
ccctttatta ctgttgtagt ccctcacttg gatatacctc tgttttcacg atagaaataa 1080
gggaggtcta gagcttttat tccttggcca ttgtcaacgg agagctggcc aagtcttcac 1140
aaacccttgc aacattgcct gaagtttatg gaataagatg tattctcact cccttgatct 1200
caagggcgta actctggaag cacagcttga ctacacgtca tttttaccaa tgattttcag 1260

gtgacctggg ctaagtcatt taaactgggt ctttataaaa gtaaaaggcc aacatttaat 1320
tattttgcaa agcaacctaa gagctaaaga tgtaattttt cttgcaattg taaatctttt 1380
gtgtctcctg aagacttccc ttaaaattag ctctgagtga aaaatcaaaa gagacaaaag 1440
acatcttcga atccatattt caagcctggt agaattggct tttctagcag aacctttcca 1500
aaagttttat attgagattc ataacaacac caagaattga ttttgtagcc aacattcatt 1560
caatactgtt atatcagagg agtaggagag aggaaacatt tgacttatct ggaaaagcaa 1620
aatgtactta agaataagaa taacatggtc cattcacctt tatgttatag atatgtcttt 1680
gtgtaaatca tttgttttga gttttcaaag aatagcccat tgttcattct tgtgctgtac 1740
aatgaccact gttattgtta ctttgacttt tcagagcaca cccttcctct ggtttttgta 1800
tatttattga tggatcaata ataatgagga aagcatgata tgtatattgc tgagttgaaa 1860
gcacttattg gaaaatatta aaaggctaac attaaaagac taaaggaaaa aaaaaaaaaa 1920
aaaaaaaaaa aaaaggccac atgtgctcga gctgcag 1957

<210> 128
<211> 775
<212> DNA
<213> Homo sapiens

<220>
<221> misc_feature
<222> (1)...(775)
<223> n = a, t, c or g

<220>
<223> nbla-03794-f

<400> 128
   gngggngngg ggttnnnntn tgaantcctt tagcactgtc ggcctactgg gttgtggttg 60
   gtgtgcgggt ttcggttgga ggactcgttg gggaggtggc ctgcgcttgt agagactgca 120
   tccccgagac gatggcggag ggagataatc gcagcaccaa cctgctggct gcagagactg 180
   caagtctgga agagcagctg caaggatggg gagaagtgat gctgatggct gataaagtcc 240
   tccgatggga aagagcctgg tttccacctg ccatcatggg tgtggnttct ttggtgtttc 300
   tgattatcta ctatctanat ccatctgttc tgtccggcgt ttcctgtttt gttatgtttt 360
   tgtgcttggc tgactacctt gttcccattc tagcgcctag aatttttggc tccaatagat 420
   ggaccactga acaacagcaa agattccatg aaatttgcag caatctagta aaaactcgac 480
   gcagagctgt gggttggtgg aaacgcctct tcacactaaa ggaagaaaaa cctaagatgt 540
   acttcatgac catgatcgtt tccttgctgc ggntgcttgg gtgggacaac aagtncacaa 600
   cctgcttctt acctacctga tagtgacttn cttactattg ctttctggac taaaccaaca 660
   tggaatcatt ttgaagtcat tggaatgggc caagaaggag ataaaccaac ttntcaaacc 720
   aaaagaaaag aaaaacgaat gatcatctgc nttaatcann gngaataatg cacac775

<210> 129
<211> 784
<212> DNA
<213> Homo sapiens

<220>
<221> misc_feature
<222> (1)...(784)
<223> n = a, t, c or g

<220>
<223> nbla-03794-r1

<400> 129
gnggnggggg nggttnnnnt ttgtgtggcc tttgtttttt tcnttttttt tttttttttt 60
tttttaagat tccagctttt tttatttttt ccccttttac acaaaacaaa gtagaagaaa 120
taatacagga ttaaaactgn aaaagtagtt aatcagtaga atatgtatgc acacaaaaaa 180
tccagatagg aagacaggct tatttacaat gaaagtaagg taaaattaac gtagttttct 240
tatgaaataa taaaaaaaaa tcaaacatat gctacaatgg gcaccactgt tcacagcaat 300
attaaagagg agaaaacaac acttatttaa tagggacaga tataccacaa ggtacaacat 360
cagtgcaata aattcacaaa actatattac agctagttaa tcagtttaag aattgttccc 420
gtcagtcaca ttttttggcc ctcagaagtt cattcctaaa gatttcaact actctaaatt 480
tctagctacc aagaagttaa gaatgattat aagaagcttt ccaaggagtt atgaaatctt 540
tgtagaccag aggccaacta tcatcacctn gagtctgntn ttaccaacag cccttgnatt 600
tttcanggag aaatctctag gaaaaagcag acaccagtgt agtactatct tccatgtcaa 660
acctanggga ctaaaatggn cagtattacc nttaaaatgg tactttgang gttaccctaa 720
aangnttatt ctgggctcaa aaantnggtt aggaatatct tttctggaaa gaatattggc 780
ttaa 784

<210> 130
<211> 772
<212> DNA
<213> Homo sapiens

<220>
<221> misc_feature
<222> (1)...(772)
<223> n = a, t, c or g

<220>

<223> nbla-03804-f

<400> 130
cactgttgcc ctactggaga tgaaaaaaa caacatccca gagccagttg tcatcggaat 60
agtctggtca agtgtaatga gcactgtgga atggaacaaa aaagaggagc ttgtagcaga 120
gcaagccatc aagcacttga agcaatacag ccctctactt gctgccttta ctactcaagg 180
tcagtctgag ctgactctgt tactgaagat tcaggagtat tgctatgaca acattcattt 240
catgaaagcc ttccagaaaa tagtggtgct tttttataaa gctgaagtcc tgagcgagga 300
gcccattttg aagtggtata aagatgcaca tgttgcaaag gggaagagtg ttttccttga 360
gcaaatgaaa aagtttgtag aatggctcaa aaatgctgaa gaagaatctg aatctgaagc 420
tgaagaangt gactgaattt tgaaactaca ccctcagtaa agcaaacagg agttgtagat 480
aaaatgtcat gtctcatgtg tcctggttct tacatcttct accttcctgt atcaagcatg 540
atataagggc tttcatggca aattttattt taactggttc tatggntgct ggaaatgttg 600
ggtttaagtt ctaaaaccat ggtttaagta actacaggag ctatagattt gaatctaatg 660
gtgcattaag cctttcaagt atcttctacc ttctggattt ctactggaat aatgnaaatt 720
aaggccttcc caatgnacag gttactttat tcctgggttt tataacagnt ta 772

<210> 131
<211> 792
<212> DNA
<213> Homo sapiens

<220>
<221> misc_feature
<222> (1)...(792)
<223> n = a, t, c or g

<220>
<223> nbla-03804-r1

<400> 131
   tgganncntt aagtgtaagt atggacttta tttttttaaa aaaaagtctt ttgagaacta 60
   caaattacaa agtaaaagca gatcactacg tagaaatcta gtgaatacat tttgcctgtg 120
   catgaaaaac tttatcaaag ttactcattt taacaaattc accccattca caacaatcag 180
   ttataatgtt ctttttaagg ttactatacc tttatttcaa attatattac atgttattcc 240
   ctcaaaatga acttttaaat gacaaacttt tggctcataa gaatggcacc ttcaaaaggg 300
   gcgatttcca ctcagtctct ggtggcatta ttccaggcta gtttcaccag ttaaaaacct 360
   tcaaaatgtt cacttttaac acaaagtctt cccataaaaa atgtggctac attttccccc 420
   caatgactac atacatagat gggtagcagc aagaaaagat gaaaatgaaa aggcagccat 480
   taaaataaat ggatcaattg natccaagaa atatttggct catggcaatt aaaaaacggn 540
   aactagggta tggtaatagc cagaagcttg agtcattact gagtatatta agagaaccga 600
   agccngtnca gtatgcccga gtaggaatat ataattgnca ntgnccttca tttggtatag 660
   caaatacttt taagnccatt gggaatctaa gctttatcta aattnccaan tngaacatgc 720
   tggcccttna caatttnaac aatttttact ggggttaaga cattaagcat tttctttact 780
   naaactccat an 792

<210> 132
<211> 1230
<212> DNA
<213> Homo sapiens

<220>
<221> misc_feature
<222> (1)...(1230)
<223> n = a, t, c or g

<220>
<223> nbla-03831

<400> 132
cactgttggc ctactggctt tttttcagcc caggggccgg cgcacgaaac ctgtttggag 60
gttatggatg ataaacccaa tcctgaagcc ctaagtgaca gttcagagcg tcttttctcc 120
tttggcgtca tcgcagatgt tcaatttgca gacttagaag atggctttaa tttccaagga 180
accaggcggc gatactacag acatagtctt cttcacttac agggtgccat tgaagactgg 240
aataatgaaa gcagcatgcc ctgttgtgtc cttcagcttg gagatatcat cgatggatat 300
aatgcacagt ataatgcatc caaaaagtcc ctagaacttg ttatggacat gttcaagagg 360
cttaaagttc cagttcatca tacatgggga aaccatgaat tctataactt cagtagagag 420
tatttaacac actctaaact taacactaag tttctagaag atcagattgt acatcatcct 480
gagaccatgc cttcagaaga ttattatgct tatcattttg taccattccc taaattccgg 540
ttcattttac ttgatgcata tgacttgagt gtcttgggcg tggatcagtc ttctccaaaa 600
tacgagcagt gtatgaagat attgagggag cacaatccaa atacggaact gaatagtcct 660
caaggacttt ctgagcccca gtttgtccag tttaatggag gattcagcca agaacagcta 720
aactggttga atgaagtgct aacattctct gacacaaacc aagaaaaggt ggtgattgtg 780
agccatcttc ccatttaccc ggacgcctct gacaatgtgt gcctggcctg gaactacaga 840
gatgccctgg cagtcatttg gtctcatgag tgtgtggtgt gtttctttgc tggtcacacc 900
catgatggtg gctactctga ggatcctttt ggtgtatacc acgtcaacct agaaggagtt 960
attgaaacag ctccagacag ccaagccttt ggcacagttc atgtctatcc tgacaaaatg 1020
atgttgaaag ggagaggcag agttccagat agaattatga attacaagaa agaaagagcc 1080
ttccattgtt agtctaattt attttaactt gatagaaaat gagctttgtg tttgtccctc 1140
ctaaacaaaa aaataaaaat cctctgtctc attgtttagt aaaaaaaaaa aaaaaaaaaa 1200
aaaaaaaggc cacatgtgct cgagctgcag 1230

<210> 133
<211> 2376
<212> DNA
<213> Homo sapiens

<220>
<221> misc_feature
<222> (1)...(2376)
<223> n = a, t, c or g

<220>
<223> nbla-03862

<400> 133
   cactgttggc ctactggctt cacaatattc tttatttctc tgtctctgtc tctctctctc 60
   aagtcagagt gtacaacagt aagcaaagtt tggcctctgt tctcgcgtga aatcaagtta 120
   acatgctcca cctgttgata tgtttgtaag agaaatctca tgtatatgca catatgcaga 180
   atttctgctc tttgcttctc aggaaatctc ttttctccaa tgtaggaaga acacattaaa 240
   atgaataagt catgttattt ttagaaaaca gaaaagcaaa taaatgtgtg aatagaatat 300
   gcactgtttc tgtgcttgaa acattgaaca ttgaatattg attgaaaggc caccatgaac 360
   tttgaaagac cactgtgttc agagaactgt gatagaaact aaaagagtat aaaaagatgt 420
   gatactttca tttttgagag gtttacagtg ggatgcagaa aaaaagaaac ctgtaaatgt 480
   gaatggcagt gtgtttggtt agtgcctact ggctatataa aattgctttt ggatgtgttt 540
   catgattcct tataaaacga agacttaata agtttacttg gcagctgatg ggcaaagttt 600
   taaaaaaaat caaatgagtt ttttgttttc ctttaagcag ttcctggcaa tgctttcttt 660
   tttttatttt caaacagatg agtttttaaa acaatgattg catttagaac cttcaagaag 720
   gcttgttaac catggatttg atgagccggg tcctgagatc tgaactggga gctagcttgg 780
   tgcacacatg gcagggccaa ggaggtcttg gatttccatg taagggatac acagacaaat 840
   aaacgtgata agggcaaacg ttagccttgc accctttgag tagcaaatca cagcgtatat 900
   tgtgtagtcc atgaagactg gtgtcatcca tcccagggcc tgggtactgt gtacagggtg 960
   agcaatgact tagatggaga aaatatttat catttgtttt gatcactttt tcattgcaac 1020
   cacatttcct aaggaaatgg gtataccagt tcaaatattt gctcttcatt tcatgctgct 1080
   tggcagtctt tgcatccttt gctctctgac ttgttcgcct ggctgtgcag tctgtttccc 1140
   aagagtgctc ttgatgagat attttgtttt tatcaggaaa ttgatttctt gcatcttttt 1200
   ctgagattac tgaatccaat attggagtag aatgactcaa ttcttggaaa aatgtctaat 1260
   ttgggactct ggtggttgat ttttattttc cagaggttca aagccgaatt tgagcatgtt 1320
   ggtgggagag gcagaggaga tgccttgcgg cccacctggt tcttccttgc atgaagctct 1380
   catcctcctc cagcagtgtg cctcatcaca ctgggctctg ggtgtatctt gcttccactg 1440
   gagagagcat cctgctccag tgtagctttc cactgtttcc ctagttctaa atcttgactg 1500
   aggtttcttg gtcttattct ttaattcctt aagcatcctg gcagttcctt actgggctgt 1560
   ctgcctttac aattgtgcgt tgttgcattt ttggtgagat tagcctaaat tcaccactct 1620
   tccttggtgt cgagaggtgg cttagtgccc taagctttgg gttaataaag gctggactct 1680
   tgagtccagg actagcacag ctgtctgtac tctgtgtctt cccataagag atgcaccaag 1740
   cagttttatt atgtgtaagg gcctttgcca tgatactttt gagaagggcg atgtgtatat 1800
   ttatatttca ccttgatgtt ttattcatga ctgatacttg tttagtctat gatggcagta 1860
   gggagtagga aagcagtctg tgtgtctgag tctggttcat ttaggtctta aactcagaac 1920
   tggtttaatg cagcacgtgt atgtagttat tataaacaat gatggcagga atgcacaatt 1980
   ttctgcaatt aaaatgccca gtatattcaa ttcactggat tttaccctgt gaccactgtg 2040
   ctgtttctct ctatgtgaag aaatatactc aattaagtgt tgcataatgt gagctttgtc 2100
   agtccaaaaa attttttcaa gttgcttgtg gtgtcattgg tatttattct gcaacgtcag 2160
   cagtgcttgc gggccaaatg cagagtgcag gctgtggttg aagcaacttt tttttgttgt 2220
   tgttcacatt ccaacattct taagaactgc ctgaaaagaa atgttggaac tgctttctaa 2280
   aagtactttt cagagccgtg cactattgga gaagttaaga accttacagt tccgaaaaaa 2340
   aaaaaaaaaa aaaggccaca tgtgctcgag ctgcag 2376

<210> 134
<211> 2145
<212> DNA
<213> Homo sapiens

<220>
<221> misc_feature
<222> (1)...(2145)
<223> n = a, t, c or g

<220>
<223> nbla-03898

<400> 134
cactgttggc ctactggaat tattcagttg cggagacctg tttgagaaaa aaaactcttt 60
gtcttcttta atcaagtgtt gtattgtctg tggcactgtt ttaaatgaaa gacaattaaa 120
ttgctttgct gttttataca ttgttgtctt taatcactag tctaaactct atgtttttat 180
gaaagcatct ttaaattttt tttcttagct gttctttctt gtttgtggta taacctttct 240
gtaccatctt ttggttctgt ggaaatgccc ttaataacac ataggattag gactaaattt 300
tggagatggg taagtttgag caaagagtca gtcaacacag gggaggattt ttgaaatttt 360
atctctaaaa acagttttcc aattcagagt ttttaaaacc cttttaaaaa tatagttagt 420
tttcagtggt ttcttttact tttagtgttt ttacacttgg aagtcagata tctaaaaata 480
gggaatgttc ttttgctatt tttagatctc tactaaaatg taatctgtag tgttttcttg 540
tttcagagca tatcttaaaa gattcagaca agtggcattt ggggacctct tccccatcca 600
ctggctttca ctcaaaggaa aataagactt cttggttctg gcagatactg tctctggcag 660
aattggtctc actgttttcc ttggggagca ttttaggtag tatgttgaaa gacagatata 720
catcagttga agacaggatc agatgctatc tggttaataa agcttatgat cagggaaggg 780

gcaaagaaga cagataccac taccattttg ttctttctgg ttttactaat atgaccataa 840
tgagtcattt tttatgcata taggctatgt gtttcaggtt gcctttcctt ttcctcctac 900
agatctattg agctttgtgt tctaaacaag atagtgtgct tatctgaatg tttcccatct 960
gtctttgatg aaaaagctcc cagttaaact aatttggatt tatttatttt tcctgtctat 1020
tccagttctc tgctatgtgt gggcaagtgc ctgttttatc ttgaggggta gattttagca 1080
tttgaactct ctcccttttt aaaatcacct tgttacttac agatcatctc agtccagtaa 1140
cttttcttta taaaggttaa aagattgttt gctttcttct caggtagtct cagtgttctc 1200
agccttgaga gggaaaggga catacttaat attttcttgt cttgcttgct aagagctgtt 1260
tttccttcgt catgtgttgg gcagggctag ccacccatct gttggaccag ctacttcata 1320
aaactttcaa aggatgatag taggtgaaat gaaattgaca agagtgttgg atgcaggtag 1380
aatgaagggt ctgctgtagc gtgtatgtgg acttctttct tttgtttatg ttcgtaaaag 1440
tggagagact ctggatatag aaagggtaat agcaaactga tatctccagt acctgtctcc 1500
tatatgatca aaaacattaa caatgtgttg gttttgtaaa attgctactg ttttgttctg 1560
aagtgctgta gccattagct ggattgtaac agtaatatga cagctgtata gtaaaatact 1620
gtctctcttt atgataggaa atgaaaaagc atctgttatg aagcctcagt gaactaaaag 1680
ccattctctg aaaagtcaag acttttgggc tttatcagta gataaacatg agccataggt 1740
tttctagcaa tagaatattt taacctatat gaatatatgc tttataggtg agactgctat 1800
ttaatgagag ttttaaagta actaaacctt gttgacagaa ttcaggatgg aaagttttac 1860
cctaaataaa acttcaggat attgaatatg atagcaaagt tccagggtat gttttatatt 1920
tatgaacaat tttcatttga atatttggag cttgggggtt ttggtgagac atgttcatgt 1980
atgttatata caaactttcg ggcctggcat gtggctcaca cctgtaatcc caacacttca 2040
ggaggctgag gcgggaggat cgcttaagcc caagagttca agacccatct ctacaaaaaa 2100
ttaaaaaaaa aaaaaaaaaa aaaaaaggcc acatgtgctc gagct 2145

<210> 135
<211> 2592
<212> DNA
<213> Homo sapiens

<220>
<221> misc_feature
<222> (1)...(2592)
<223> n = a, t, c or g

<220>
<223> nbla-03914

<400> 135
cactgttggc ctactggatc gtataaaatc ttatgtggaa gccaaacatt aaactggtaa 60
aaatcatttc aggttgaggg tgtatgttgg tgggtacgaa gtggtttcag agcttccctc 120
tcagttttcc cagtgttccc caaagactcc taggacacct cgggggagct caggggaccc 180

aatgcagcac aactagaggc cccagcctcc acactgcctg gtgggggggt ctagactgaa 240
tcgtgaaatc accctatcta tgggctgtgt gtccagttgt tggggtgagg tctggggagt 300
gggggatgca agtggtggag ggaatgaaag gagggaggga aacttccagt gcctcatcat 360
tcaccctccc catagatggc acctgggctc cccgggctg ggtcaggctc tgagtgacag 420
ccattgaaga gaagccagcc tccaggaaat ttctccagca tgactgggca tcctctctcc 480
tagccaaata tatcagagct ttgaggaaaa tgggcttctg gccaggccac actcgtcctt 540
aggaagagct gtttccatct gaggaatctt tttgtagaca ggtgctggtc cttgaagggt 600
aggtccgctg agcttgcgcc atagaatgcc tacaccactg gcatccttta gtcctgctga 660
ggggagggac taactcctgg taattttcgt tttgttgatc aataaaggtt gttgtattgg 720
caagtgccac ctgcatcact actacagatt cttaggacc taccctcaca tgtgaatctc 780
gggttacatt ttgcaaagcc ctttcacata ttttgcaaag cattttgaca catttttcac 840
atattccaca tattttgcaa agccctttca ctgtatttcg atgcacaccc acctgagatg 900
caggtgctga gagtcttact ttacagataa ggaatcaggt taaagaggtt ccctgactca 960
ggcaaggtca caaaactggt tagtgccaga gtggagaccc agcaatcaac tcttctcatt 1020
ccaagccgga cggcatctcg ccatgccaca ggggccttct ggtggtatag ctggtgactg 1080
aggacagaag agaagtaaga caatggcaga ctcttaattt ctagcaggtt ctctgcagct 1140
cccccctgaa tctcccctaa tattcccact tacctcctca cccttccctg gtgagacaga 1200
gcaggactct tccagagcag atttccattg ttatttttcg cgtctcagca ccacgtgtat 1260
tgctattttt atcctgtact agaaactatt aagtggggag aaatgttaat tggtattatt 1320
acagcaaact gtgtcctagt aacattgcaa ttcagaggta agctctccac tcccctcccc 1380
tgccttttag gcaaacatag gaattttatt cattttgctg acagccagcc ctggtttcct 1440
aggctggtgt ggggagaagt acttgggacc ctgagatgta ttggacaggc tcctgggcct 1500
ctgctctgtg gctagtttat gaaggaagga agttggggtg ggtggaatag cgatgaattt 1560
agttctgttt tatccactga tgctggactg gaaggaataa gatgtctgct caggaagccc 1620
ttgtgagggg tggcttgcag catctttgtg aaagaatcag gggcatgtgg attcatagtc 1680
cagctgaaag taagagacag tggccctcat ggtgtgtccc cgtaagccag agggaagaat 1740
gagcagatta aaaaataata gcagtgaagt ccacacaagt tctgcctaac ttccagaagg 1800
aaggggacac attattatta gggattcaac aaacgggctg gtttctaacc ctggcagctt 1860
gccttcccat ctatgtaacc ttaagcaagt ctcttatcct ctctggcatc aatttcctca 1920
tctgtcaaat gggctaaaaa tacttacatc tctgggttgt tgtgagaata aagtacattg 1980
cacaaagcac acagtaaggg ctctggaaat taaagcttaa tagtgatttt ttttcctcat 2040
gtgctctaag aatactgagg gctgagtgtg cactggtaga tggaaaatcc agtttccaac 2100
ccagcagcat ccactgactg atcaaaccta acctgtgctg gataatacct tccaaatcct 2160
ctctggccct tcctcctccc ctgggaagaa caatcagctt ggtttcatgg taccctacaa 2220
tttttatttt ttataatgtt gattgttttt aagaattatt acaggctggg cgcgatggct 2280
cacgcctgta atcctagcac tttgagaggc tgaagggggg tggatcactt gaggtcagaa 2340
gttagagacc aacctggcca acatggtgaa accccgtttc tactaaaaat atagaagtta 2400
gctgggcacg gtgttgtgtg cctgtaatct cagctactcg ggaggctgag gcaggagaat 2460
cacttgaacc tgacaggcgg aggttgcagt gagccaagat tgcaccattg tactccagcc 2520
tgggtggaaa gagcaaaact cggtccccct cacaaaaaaa aaaaaaaaag gccacatgtg 2580
ctcgagctgc ag 2592

<210> 136
<211> 754
<212> DNA
<213> Homo sapiens

<220>
<221> misc_feature
<222> (1)...(754)
<223> n = a, t, c or g

<220>
<223> nbla-03932-f

<400> 136
gcactgttgg cccggtgaaa gccttcgagg tgacattttt caaaaattta tggaaagtga 60
caagttcact agattttgtc agtggaaaaa cgttgaatta aatatccatt tgaccatgaa 120
tgagttcagt gtgcatagga ttattggacg aggaggattc ggggaagttt atggttgcag 180
gaaagcagac actggaaaaa tctcacagga agtaaaagag cacagctttt tcaaaggtgt 240
tgactggcag catgtctact tacaaaagta cccaccaccc ttgattcctc cccggggaga 300
agtcaatgct gctgatgcct ttgatattgg ctcatttgat gaagaggata ccaaagggat 360
taagctactt gattgcgacc aagaactcta caagaacttc cctttggtca tctctgaacg 420
ctggcagcaa gaagtaacgg aaacagttta tgaagcagta aatgcagaca cagataaaat 480
cgaggccagg aagagagcta aaaataagca acttggccac gaagaagatt acgctctggg 540
gaaggactgn attatgcacg ggtacatgct gaaactggga aacccatttc tgactcaatg 600
gcagcgtcgn tatttttacc tctttccaaa tagacttgaa tgganaggan aaggaaaagt 660
cccggcaaaa nttactgnca atgggaccan atctntctgg ggaagaaact caaattaagg 720
ccaaaatgcc tttgtcanaa taaaaggggg gaan 754

<210> 137
<211> 759
<212> DNA
<213> Homo sapiens

<220>
<221> misc_feature
<222> (1)...(759)
<223> n = a, t, c or g

<220>
<223> nbla-03932-r1

<400> 137
tttttnnntt ttaagactga tgacatgggg gatttcgctg gttctcataa cccagcatct 60
atttgggggt atcagtgggg cctggaggca gtgggagaaa acaaacttca cagggcagat 120
gctgctggga ccaggaaaaa ggcttcatgc cctaaggtcc tcactgtcgt acagcccagt 180
gcaaagtatc attcattcat gacagatgca tgacgacatg aaagtcaata tacacagtta 240
acccattttg acacattcat tcctcagcat tttttcaata caattcatct gaaattatac 300
taagacataa aacagacctg cttttgtaca tccattactg atatgtctcc cagggtcagg 360
cagaaggaac cagaatgact gtcacacaac tgctaattgt cgagacctga gacggactca 420
cccatgagtg ctcctgcatg ccaggtactg cttgtcgact cgtgcacact ggactcaaag 480
acttttgaaa actgntcttg ccctgaaacc taaaacttca cggcctgtcc ttcagaatca 540
ctgnaaatct ttncaatgat ggacggatga atgccttcaa tgattaagtt aatcccatac 600
acttggatag gaccgtgtcc gcanttccac ctgctgaagg ggncccggac aaactggcaa 660
aagcanggca cgcatcgtgg tgccggntcc aaagganggn ccaagtgcna naaatatctt 720
naacttttac ntcntnngaa aatancctta actttaacn 759

<210> 138
<211> 756
<212> DNA
<213> Homo sapiens

<220>
<221> misc_feature
<222> (1)...(765)
<223> n = a, t, c or g

<220>
<223> nbla-03949-f

<400> 138
cactgttggc ctactggacc ctaattgagg gagtgaggaa gagagcagct cgcttctaac 60
tggactgcac gttggtgaca gcgtcccaag ctggtgacag acccactctg taactttcag 120
ctagattcag ccaccagatc ccagaaacat gacccttgct gcctacaaag agaagatgaa 180
ggagctcccg ctggtgtcct tgttctgctc ctgcttcctg gccgatcccc tgaataagtc 240
gtcctacaaa tatgaagcag acacggtgga cctgaattgg tgcgtcattt ccgacatgga 300
agtcatcgag ctgaacaaat gcacctcggg ccaatccttt gaagtcatcc tgaagccacc 360
ctcctttgat ggggttcccg agttcaacgc ctccctgcca aggcggcgag acccatccct 420
ggaagagatc cagaagaaac tagaagcggc tgaggagcga aggaagtacc aggaagcgga 480
gctcctgaaa cacctagcag agaaacggga acatgagaga gaggtgatcc aaaaggccat 540
tgaggaaaac aacaacttca tcaagatggc taaggaaaaa ctggcccaga agatggaatc 600
caacaaggag aacagggang cccaccttgg ccggcatgtt ggaacggttg caagagaang 660

acaagcacgc cgangangtg ccggaaaaac aaggactgaa ggaagaagcc ttcaggtaaa 720
gcctagaggc caagaacttt cangtaagcc ggncan 756

<210> 139
<211> 761
<212> DNA
<213> Homo sapiens

<220>
<221> misc_feature
<222> (1)...(761)
<223> n = a, t, c or g

<220>
<223> nbla-03949-r1

<400> 139
tttttttttt tttttatacc aaagggttta tttgcaaagc tgtaaaaaac tacacagtgt 60
gcagagcaga gtgggaagag gtcctgggac ccggttaaca caggcagccc ctgcactcat 120
gtttgcaggg gaagggagga ggagagcttc ttttccagcc atggttcagt gtcctggaga 180
ccaagactgc tgggtctgga tgcctccttt gccttctact agcaaacact ttatttcctt 240
gctcgtattt tctaatctac ctagaatctc aaacccccaa agcatggagg acttctgcta 300
tggtgtgaat gttgctgtct cccccaatat acaggttgaa tcctaatccc gacactacag 360
aattaagaga tagggcctta agaggtgaga tcatgaggac tccaccttca tgaatgggat 420
tagtgcctta aagaaaaggc tcaaggaagc ccttttgcct tcccttctgc catgtgagga 480
tgcagcaaaa aggcatcatc tttgaagcag atggcaagcc ctnaccagac actgaatctg 540
ctggcacctt gatattggac ttnccagcct tcaaaactgt gagcaatcaa tttctggtat 600
caattactta atctaangna ttttggtaca gcagtctgaa cggttgtgac taagacaaat 660
tctttcttac aggcatcttg gggaccatat tanggatgtc aggtcattcg caagggaacn 720
ccgcgtttcn ttttctnatn aaatttangg cntgctatcc t 761

<210> 140
<211> 693
<212> DNA
<213> Homo sapiens

<220>
<221> misc_feature
<222> (1)...(693)
<223> n = a, t, c or g

<220>
<223> nbla-04021

<400> 140
cactgttggc ctactggatt aatttactcg cagttgctgc tgctcaggaa gagagacaag 60
gaatatttta acagaatcaa ggcatagaag aatcaccatt ttatttgagc ctctaatcag 120
agtcagacca gtagagaaat taaataagat tagaaaactc tgtactgaaa gctgctgatg 180
cttcaaaaat gaaaacaaga tctcacaact ctccctgtta gttgaaaata tatcaatttg 240
ctctgaaagg attcagctgc ctagtgttgc cattactaac ataaacatat ggctcatatt 300
tccatccaga gaaattaatg ctaaattggt gcctcgctaa catcagatac actgtattat 360
gcttaaatat attcagtaaa atgtggaaag gggtattaac aacgacaaca aaaagatgga 420
tttttttttt ctcacaatca cagttgctaa tccagtggga gatgtttgag agagttttgt 480
tcaacatcac agtgagagtg cctagggaaa tcagaaaatt acaatggatt cccctttgat 540
tgtaataagt gttgattttc tccatgagtt gtttatcctg tctagtgatt tgatggtgaa 600
cttttctaaa taaatagccc tttcccctcc ggtgtcggta aaaaaaaaaa aaaaaaaaaa 660
aaaaaaaaaa ggccacatgt gctcgagctg cag 693

<210> 141
<211> 2851
<212> DNA
<213> Homo sapiens

<220>
<221> misc_feature
<222> (1)...(2851)
<223> n = a, t, c or g

<220>
<223> nbla-04055

<400> 141
cactgttggc ctactggcag atgaactagg tcagatccct tggaaagatt gaatatagaa 60
ttttaatggc atcaaatagt tctgtccttc catattagac aattatattt caaccgaagt 120
cacattttgg agaagactct ataccagaat cttagtaaga gctttttatt ctctgtgtag 180
tagtaggata gctttttggg ggtgttttcc tggtttttcc aaattgctac aattttaaca 240
attatgatca tgaatagcaa aaagaaagaa aacatcactc agaagtgaag aaaagcgctt 300
ggtcagacac aaaagcccag tcacaaaggt taaaataacc atcattttgt gagccttttt 360
acaatgcact agacaccgtg aggtgtgcat catctccatc cctcacagca gcactgaagg 420
gtagatgata ttattcccag catcctattg ctatccagag ggaaaggagg cttagccaac 480
gggctgcaaa cattccaatt ccttttcctg agatggacgc atgaactctc ttggcccaaa 540

ggcattaaat attccgccca tgtaaccgat gccccttctt ggaattcaga gctcaccctg 600
caacctgctg gttatcattt ggcttctatc acaggctgcc aacggtgaga gtacacatgg 660
gtcacgctca tgtaaatatt ccagaccata tggcagttgt atttctcact gtaaatgaac 720
acattggctt tgttctatag aaaatcaatt tcgcaaaaac ttgagttcag ttacttttgg 780
actggaagga taaaaatttc ctaagccttc actgattgct gaaccttcta taccaagtct 840
acatgagttc cacatccctg aatgctctct ttcagttgat tcgcttgctt tactaatcca 900
aggactcaaa cattcacaca gctgtatatg gagaaaatat aaaaacaatt cacctttttg 960
gcaactgagc tacgtaacca ataaaggtgg tttcacttca ggccattacc cagattagtt 1020
ttactaaact tagctggaat gccatcctgc gcctttgagg aatgtgcaca atatgctttg 1080
gtactggcat tgctaccact gtaattctct cacacaaata gaggcactac agtcgtaata 1140
ttattccagt gaaaaactgg acctctctct ccctgggata ccacaaagct aacaatggca 1200
aagcctccca aaaccaaagt gcacagaaga agacagtgct taagctaatt aaatctgctg 1260
tggaaccagg agaattccaa gaacagcagt aacacatttt tattttataa aaatgtcccc 1320
attgaaacaa cagatctgaa tgcaaatgta aaactaagca ttcgataatg ctcactttca 1380
catctgtcaa catttagag taacttcaag aggaatacat ttgtggttct agaaattctt 1440
agtttatgca tagaatacat ttgagaggac tttgttttca caagtatcca ggtatcaaat 1500
aaaaaaatac catggaatca cacacttttg aagctggaag ggatcatgag agtcatgtat 1560
acctgcccct catttaacag gtgtgatgaa agttcagaga ggttcagtta tcgctcaaga 1620
ttactcacat gactggattt caggaaattt gaaggcaaaa aataaattag ggatagattt 1680
ccctctgtac ccttagcatt taaatggcaa atctctcagt ctgagataga gggctaaccc 1740
agaccaagat tcactggtca cctgaccttt ctgtattttt acaaacaaaa ttaaatggag 1800
aattgaaaca tttgcccaaa ttgctttgga taatttactg agctttgttt accttaaaaa 1860
aaaatgaaat agagtagaat aaaaagggtg acacatctta cagtatttga atattaataa 1920
aatcatattt gatgttaaat ttactgtttt tattcttacg tgagtatatt tcatgacact 1980
tttcatgttt cagttataac tatatgtgtg tatatatatg tatgtgtctg tatgtatata 2040
tattagatat atgctaagtt ataatatata acttttcttt accatgaatc acagtaaaaa 2100
agaccaaaat tcacttcagt ggaaatttca cttttgcccc actcaccatc tttaaaaaca 2160
aaaaaccaac aatgaaaata aaatgtaaaa tagaagcttc tcataacatt ttgggggaac 2220
cttgcattaa actatcactt tcttgtcact gcagcccaaa atgttctttc tccttcaggc 2280
ctagtgcaat gtcctatagg ttactaaaaa agaaacatta aacttaagaa actagaaaga 2340
gggcaaatgc acaaaagatg aaaaaggaaa cagatcttcc tttcgttgtt ttaagataca 2400
tgtttttttc tttcctaata tcaaagtttg gggtaaacaa caaatgtaat ttgctcagtt 2460
cctaggacag tagatgtaca ataatacgtg ttaaaggaac accaaaataa gggtaaaaca 2520
gcagacttta gactctgcta ctaacctgta caaaacttgg tggagtttat aatttctctg 2580
agcttcaact ttctcaaccg taaaaggtga tgactgggca agagctgcat ttctttcagg 2640
gatctttgca gaactctttc catgggctct taataaatga cccatgaaaa tcatttctaa 2700
atgataggtt aatagtgggc ccttcaccga tttctacctt cttatgtatt catgatcatc 2760
caatgagtcc taactttcca gctcagaaat ggtgacctcc aaagaaaaag aaaaaaaaaa 2820
aaaaaaaagg ccacatgtgc tcgagctgca g 2851

<210> 142

<211> 2157

<212> DNA

<213> Homo sapiens

<220>

<221> misc_feature

<222> (1)...(2157)

<223> n = a, t, c or g

<220>

<223> nbla-04072

<400> 142

gaattcctcg agcactgttg gcctactggc ttgataatcc ccctgattcc cagactcctt 60

ggaatagctc cacagctatc agtggttctg ccacaggttc agagccttcc cagtactgcc 120

ttcttatttg agaggtgtgc taagagctat aaagcagagg cttcaattgt acacagtttg 180

gaagtttagg caaaagtcat ttcttcccta tattttgtca tgcttatctc ctgtctcttt 240

ctgttttaca gattagcaat aaactcctta aaacccaaag gtttgggcct ctgttccttt 300

cacttgcagt cagacatgga gttagtggta gaagaaacag aaggggtaac ctgcatggtg 360

acagctactg aggggatgga taggaaagca ggctgagtcc ctggggccag tggttaccaa 420

agccaaggag agagcaaggg gagcccagtg ggcctggcca tggactgctc tggaattccg 480

agtgtgaact ttcagccaag aaggtagtgt gaaaatatta ctgtgaggtt ttaaaagtac 540

acaaataaca attgtttttt gtaaaaagaa aaaaaaaaag ttagaaaaag agatatgcca 600

aaaagaagaa agtaaaaaat acacgacttc tgtcttctgt taacatatta aaatttattc 660

ttttagattt ttatgtgtct atatacatgt aaaatatata tgcatcccca aattgtatta 720

tactcttttg agacctcaca tttttttcac ccaagaatat aacattaatt tctttccttt 780

agaaaagaca tgagactaaa gaatgtttat tgagttgaga gtttcccaat caaggagtat 840

gactctgtat aataataaat ctaatgtttt attagaaatt ataataaaat tagaaattta 900

atatttggca ggatgtgagt tgcctgcata tgtacaccct ttattatatg actagacact 960

cttttgtgtg gtcatggata tattggcatc ttttgaagtg tatccttttt gtttcaggag 1020

cttctgtcta gatgaatagc aaatgtgaca ctgataatag taatcgaata ttcctccaag 1080

tacaggctcc ttcctctttc tcctggacct ttgcacatgc tgcctggaaa actcacccac 1140

aacctacttt ccatcctctc cttcggctca tccctgctca gccctctttg ttgagatatt 1200

tgccaagcca gattctctga gaagcctttc ctggcatctt cgtctatata attctgctag 1260

ttgtttctaa aactatgttg ttagtgtata ttgtaatcac ctatttatgt ctgtttgact 1320

gtgagcctct tggagatggg aattctctcc tatgcattgc ggtttcccca ggccctagca 1380

cactgtctag cccagtattc cttctcagta agtaggtgtt gaatgactta caaaatgaat 1440

gctaaaagac ctggtagaca agaaggaatt tcttagccta agtgaccaga tgtggagaag 1500

atactcaaac acttcagggt ccccgatctg gtgatgtcac ttgcagtgga atttcatatg 1560

aatccagtgt gacagaagtt gcctagtcaa gttgtaagtc ctcttctctg ggtcacatgt 1620

tcaaatactt ttaggagaca gataatgcaa tatttaagat acaggaagtg atgaagactg 1680

tggcacactg gagagtttgg actccaagag taattaattc agttgaaaaa atcaacagca 1740

ctctgttgat caagcaaact ttctaggggc cctaatccac ctgggcctgc agtatgtaat 1800
tccataactc atgggatgcc atcaagagag acaatctact tgattgctaa agggcctttg 1860
ttttctttgg agttccccag gaaggagaaa tgtatgtgtg tgcaagggtg aggagggggg 1920
agagtgaaga aacaatacta tccccttaaa aaggtttgtc agttttcatt atgaaagaag 1980
accaaacgtg cccactccag aaatcttggc atatgcagtt attttgaagc cattcagcgt 2040
tcttcaagat gtcacaatga aaagagtgat ggttttttaa ataaaaaatt aacatgtttc 2100
tcttggcaaa aaaaaaaaaa aaggccacat gtgctcgagc tgcaggtcgc ggccgct 2157

<210> 143
<211> 2579
<212> DNA
<213> Homo sapiens

<220>
<221> misc_feature
<222> (1)...(2579)
<223> n = a, t, c or g

<220>
<223> nbla-04099

<400> 143
gaattcctcg agcactgttg gcctactgga agttagaaga aaacaatcac aaaactgaag 60
cctaagagag aaactgtcct agttgtccag agataaaaat catatagacc aattgaagca 120
tgaacgtgga ttgtatttaa gacataaaca aagacattga cagcaattca tggttcaagt 180
attaagcagt tcattctacc aagctgtcac aggttttcag agaattatct caagtaaaac 240
aaatgaaatt taattacaaa caataagaac aagttttggc agccatgata ataggtcata 300
tgttgtgttt ggttcaattt tttttccgta aatgtctgca ctgaggattt ctttttggtt 360
tgccttttat gtaaattttt tacgtagcta tttttataca ctgtaagctt tgttctggga 420
gttgctgtta atctgatgta taatgtaatg tttttatttc aattgtttat atggataatc 480
tgagcaggta catttctgat tctgattgct atcagcaatg ccccaaactt tctcataagc 540
acctaaaacc caaaggtggc agcttgtgaa gattggggac actcatattg ccctaattaa 600
aaactgtgat ttttatcaca agggagggga ggccgagagt cagactgata gacaccatag 660
gagccgactc tttgatatgc caccagcgaa ctctcagaaa taaatcacag atgcatatag 720
acacacatac ataatggtac tcccaaactg acaattttac ctattctgaa aaagacataa 780
aacagaattt ggtagcactt acctctacag acacctgcta ataaattatt ttctgtcaaa 840
agaaaaaaca caagcatgtg tgagagacag tttggaaaaa tcatggtcaa cattcccatt 900
ttcatagatc acaatgtaaa tcactataat tacaaattgg tgttaaatcc tttgggttat 960
ccactgcctt aaaattatac ctatttcatg tttaaaaaga tatcaatcag aattggagtt 1020
tttaacagtg gtcattatca aagctgtgtt attttccaca gaatatagaa tatatatttt 1080
tttcgtgtgt gtttttgtta actaccctac agatattgaa tgcaccttga gataatttag 1140

tgtttttaac tgatacataa tttatcaagc agtacatgaa agtgtaataa taaaatgtct 1200
atgtatcttt agttacattc aaatttgtaa ctttataaac atgttttatg cttgaggaaa 1260
tttttaaggt ggtagtataa atggaaactt tttgaagtag accagatatg ggctacttgt 1320
gactagactt ttaaactttg ctctttcaag cagaagcctg gtttctggga gaacactgca 1380
cagcgatttc tttcccagga tttacacaac tttaaaggga agataaatga acatcagatt 1440
tctaggtata gaactatgtt attgaaagga aagggaaaac tggtgtttgt ttcttagact 1500
catgaaataa aaaattatga aggcaatgaa aaataaattg aaaattaaag tcagatgaga 1560
ataggaataa tactttgcca cttctgcatt atttagaaac atacgttatt gtacatttgt 1620
aaaccattta ctgtctgggc aatagtgact ccgtttaata aaagcttccg tagtgcattg 1680
gtatggatta aatgcataaa atattcttag actcgatgct gtataaaata ttatgggaaa 1740
aaaagaaaat acgttatttt gcctctaaac ttttattgaa gttttatttg gcaggaaaaa 1800
aaattgaatc ttggtcaaca tttaaaccaa agtaaaaggg gaaaaaccaa agttatttgt 1860
tttgcatggc taagccattc tgttatctct gtaaatactg tgatttcttt tttattttct 1920
ctttagaatt ttgttaaaga aattctaaaa tttttaaaca cctgctctcc acaataaatc 1980
acaaacacta aaataaaatt acttccatat aaatattatt ttctcttttg gtgtgggaga 2040
tcaaaggttt aaagtctaac ttctaagata tatttgcaga aagaagcaac atgacaatag 2100
agagagttat gctacaatta tttcttggtt tccacttgca atggttaatt aagtccaaaa 2160
acagctgtca gaacctcgag agcagaacat gagaaactca gagctctgga ccgaaagcag 2220
aaagtttgcc gggaaaaaaa aagacaacat tattaccatc gattcagtgc ttggataaag 2280
aggaaagctt acttgtttaa tggcagccac atgcacgaag atgctaagaa gaaaaagaat 2340
tccaaatcct caacttttga ggtttcggct ctccaattta actctttggc aacaggaaac 2400
aggttttgca agttcaaggt tcactcccta tatgtgatta taggaattgt ttgtggaaat 2460
ggattaacat acccgtctat gcctaaaaga taataaaact gaaatatgtc ttcacaggaa 2520
aaaaaaaaaa aaaaaaaagg ccacatgtgc tcgagctgca ggtcgcggcc gctagacta 2579

<210> 144
<211> 728
<212> DNA
<213> Homo sapiens

<220>
<221> misc_feature
<222> (1)...(728)
<223> n = a, t, c or g

<220>
<223> nbla-04106-f

<400> 144
cactgttggc ctactggttt atgtcatgtt tagaattgtg tgtgatagag gctgtacata 60
cacgttttac aggaagcgcc tggcactgtg tgaggctggc ggttgaatgg tcaggagtgt 120

agtagcggct ctaagccagc ctcacagccc tttctcattt ctcgctccat ccccctcccc 180
gttcatttcc aggcctttcc acgaagagtc ctcagccttc tccctccagc cggaagagcc 240
ccctgagtct gagccctgca tttcaggtgc ctttgggccc agctgaacag ggctcatccc 300
cactcctgag ccctgtcctc agtgatgctg gcggagccgg gatggactgc gaggagccca 360
gacacaaggt gggaccctcg aggctgagaa gctagcagag ggttgcaggg tgggcctgtt 420
ttcactgttc cttcgggtgg tgctggtggg gaccctgggg cggggcaggg aagggctctg 480
cactcacagc cgcttaccag cctcctgagc agagaggagg tactgggccg ccttcctagc 540
aaaaccttct gagactttcc tcttatctct gggactccag gttccttcgt acaaaaacga 600
agtttcctta ttcatttagc aaacggtccc tggaacccac aggcttcatg tgtcaagctg 660
gatctttcag cctncccact cttccttcct tccttccacc ttcttcattt ctccttcctt 720
cctcctcc 728

<210> 145
<211> 746
<212> DNA
<213> Homo sapiens

<220>
<221> misc_feature
<222> (1)...(746)
<223> n = a, t, c or g

<220>
<223> nbla-04106-r1

<400> 145
nttttncttt gacccaaaga aacagtganc taaagagatt gtcattaggt ttccaaaata 60
aaataaaaag acatcttttt tggaaacagt cttgctgcca gggcccagaa ctagcnctca 120
cttgcaggcc tcgtgcagca gtacgtttaa tatcctttgc tgcctccaga ccagggggct 180
ccttccactc cagtgggatc cagggggacc ctcagggcac caggaaggag gagactgccc 240
tgcctgagcc atcctacctg ctgagagtcc cagcatccca cgggtagccc tggcatctgg 300
aaggcaggcc ttggacagaa tatcctctgg ccaattcggg ccaaagcagg agtgctgagg 360
tgtcacaatg acatacctcc ccctgtacca gcactgactc cctccctcct agaggcccca 420
tggcctcttc catncagctg ccantcaggg accccatgtn tnccttctct gnggactttc 480
anncncnata tantttacan acttccttgg acnctgagtc tttcttttta nggtccntcn 540
gcnttnccgn atnntgcagg nananttncc tgnncttngn atagtgnang ccacctttga 600
ggcncagnat tncctgnctn tggttntcct ggttnttgnc tgnangantc tgtttgnttg 660
nnncntttnt tcttggttan ttntntntta nnccttatnn nttattnttt gnntnttngn 720
aancatnncg ggncttcnnn ntnntc 746

<210> 146
<211> 2378
<212> DNA
<213> Homo sapiens

<220>
<221> misc_feature
<222> (1)...(2378)
<223> n = a, t, c or g

<220>
<223> nbla-04137

<400> 146
   gaattcctcg agcactgttg gcctactgga attaaccagc agagccagct ggctgaaaag 60
   ctgaagcaac agatgttgga tcaggatgag cttttagctt ccacaagaag agactatgag 120
   aagatacagg aggagctgac acgtctccag attgaaaatg aggcagccaa ggatgaggtg 180
   aaagaagttc tccaggccct ggaggagctg gctgtcaatt atgaccagaa atcacaggaa 240
   gtggaggata agacccgggc caatgagcag ctgacagacg agctggccca gaaaacgact 300
   acatcgacaa ccacacagag agagctgagc cagctacaag agcttagcaa ccaccagaag 360
   aaaagggcaa ctgagatcct gaatttgctg ttgaaagatc tgggggagat aggtggaatt 420
   attggcacca atgatgtgaa aactttggca gatgtgaacg gagtcattga ggaggagttt 480
   accatggccc gcctgtacat cagcaagatg aagtcagagg tcaagtccct ggtgaaccgc 540
   agcaaacagc tcgagagcgc ccagatggac tccaacagga agatgaatgc cagcgagcgg 600
   gagctggcag cctgccagct gctcatctcc cagcacgaag ccaagatcaa gtctctgaca 660
   gactacatgc agaacatgga acagaagagg aggcagctag aagagtccca ggactcgctc 720
   agcgaagagc tggcaaagct ccgagcccag gaaaaaatgc acgaagtcag cttccaggat 780
   aaggagaagg aacatctgac gcggttgcag gatgctgaag aaatgaaggt gtgtgtggct 840
   gccccttcca tcaagcccag tgtgcatttg atgcatttgg attggaaatg cttgttctag 900
   gaaaatctgt caccttcaga tccgtatatg gcaaggacag ggggaggggc tgttgttggg 960
   cattcagagt cgatctcaga gtatagcctg gcttagaaat gaacaagtcc agaaacccca 1020
   agatgtctgt agagaatggt taagttgcgt taataccttt gtcttcacca ttcctgatga 1080
   agagggaaag ggtagcaccc ccaatcactg catgggctct tttgtcggag ttaacttcta 1140
   aggatttctt cctccaacct ttacgcagaa cacaccaaat ttgagtttct atctcaaaca 1200
   tagagatttt tcttttggtc acattttaaa ctgtaaagga aaattgattg tggagtaaga 1260
   cttttaggtg aataaaatga gacaggtgaa gatgtaggat tttgattagt tgtaggcaaa 1320
   ctatgagcct cattgagtca aggtccacag ttaaagacgt ccacggcatg cccttgctcc 1380
   agccagcaga ataaaatcat gcgtcgtggg agtccctttc ctgttttcag agtgctgttg 1440
   ccaaaaggtc tcctttggac atcatcataa atctgtgcag tgggcagggc aggggctttt 1500
   atctcagttt tacatgtgag ggagtgcagc tcacacaggt tacaggctta tctggaggtt 1560
   acgtggatag ggagaggttg gtgccctctg actgacctgg ctggagaagc tcaggctggg 1620
   ctgtaaaagg tcaagggaac actttttatt tagttggtgg gctaattggc ttctagggcc 1680
   aacttggttt gataacagat cttgggtttt gttatcccaa taaagcaagg ccgggtcctg 1740
   ggggcaaaac agccagaaat gcccccagtt ttgggatctg acatctgagg gacacaggga 1800
   aggaggtagg tccagatcca gggcttgtca cagagtgggc tgagtgccaa cgagtagatg 1860
   acatgtttct cttggcctgg gatgcagaag gcgctggagc agcagatgga gagccaccgg 1920
   gaagctcacc agaagcagct gtccagactc cgagacgaaa ttgaggagaa gcagaaaatc 1980
   attgatgaga ttcgggagtg agtcggccca gggcaccagg ggtgtggggg tggtcatgcc 2040
   tcggtcctct tggggaagcc tggaaggatg tggctcttag tcgagggccc tgctcacctt 2100
   gccctgtggg cactgccctg gtgacacagc aggctgggcg ggctgcttcc aaggtctgtt 2160
   ctccgatctg gagctgagcc tcctggagcc ctggcatggc aggtggcagg cgggcccagc 2220
   tgcctctcct agtccccggg ggccagggtc acataggtga ttccgctgga tggacgcatg 2280
   ccccagtaga tgggggggag catctgtaat gaaagcacca aaaaaaaaaa aaaaaaaggc 2340
   cacatgtgct cgagctgcag gtcgcggccg ctagacta 2378

<210> 147
<211> 4684
<212> DNA
<213> Homo sapiens

<220>
<221> misc_feature
<222> (1)...(4684)
<223> n = a, t, c or g

<220>
<223> nbla-04145

<400> 147
cactgtggcc tttttttttt tttttttca caattcagaa gaactttaat gcatatacca 60
tcattgccac tataatagag atagaagata cattaagaaa attcagtttg tatcaataaa 120
acagatcaac acagaacaag gaaacaccat agatatttgt aaatgagatc ttctcttttg 180
ttactgtgta tatatattcc tttatattta tacaaactca caacacatga catttcatat 240
ttcatatgcc actgagaaga ggtgtcagta tacagaacat aggaagaaga aaaaagcatg 300
agaacatctg cttagttaga atctgatgag gagagacgtg agagctattg ttcctctctc 360
tgctcaggcc tatcgagagg caactgcagt ttttgctaat tgttcctcct gaggaattct 420
gctcatactg ctatggtaca tctcctaggt tggcatgaga ccagcctggt ccccagttga 480
ataaattaga aagagcagtt gggggaattg aatatgatat gcaaattgca taaggtgaca 540
tctctctgtg tggccacaga gatagatcat ctgctcctgg ctccttgatc tgagggaagc 600
ctctgctgtt caacctgctc cagggtttaa ttctgttggg gttgtggggc tgacaacaat 660
gtgttccttt ccttccttct ctcccacaag cagaggaagt catacgtaga caaaacagat 720
gtttaggttt tctcttcagc aggcttcctc ttgttcccct ctatcacccc cagataggtg 780
aagttcagaa ttaggactgc ccactgcggg gggaacagac cctcccctgt tctgagacta 840

accctgccat ttactgcctt ctctatgtag tcttggatag gttgcttcac tgatgcaggc 900
ctcagtcttc ctcatctgta ctggaaataa taaaacccac atcacagtgt tgtgtcaaag 960
atcatcaggg catggatggg aaagtgcttt gggaactgta aagtgcctaa cacatgatcg 1020
atgatttttg ttataatatt tgaatcacgg tgcatacaaa ctctcctgcc tgctcctcct 1080
gggccccagc cccagcccca tcacagctca ctgctctgtt catccaggcc cagcatgtag 1140
tggctgattc ttcttggctg cttttagcct ccagaagttt ctctgaagcc aaccaaacct 1200
ctaggtgtaa ggcatgctgg ccctggtatt tctagatgag gttaggttcc tgggacagga 1260
gtgtgtattg aggtggaggg aaggagctga aagaaggagg ggatccttgg aaagggctct 1320
gagaggggtg tattcgtttc attctgagac gactaccatc tatcccccta gactctcaca 1380
gtggctccac cttaaatgtc cacagcattt cactttctaa aatgtacttt tatttgactc 1440
tcataactat tttgtgaagt agggaagatt gtcagcctca ttaggtgaaa atatgttggt 1500
tcaagatcac acagttagta tgctgcaaag tcaggactca aacccaggtc tctggaatcc 1560
tggttttctg aatgtcggag ggcacctcta acaaaggctc tggagcactc taaactctca 1620
ttctgggctc attcctcact cctggaggct gatttctggg ggaaggagga agtgcatgag 1680
gtacctttct gtcacatgac tggtggcaga ccctacttta acccagccca acaaggtcat 1740
gctacagagc tgctcatggc attttcctgg actctcctcc cttggtcctg actggattct 1800
tctaccctga tccacttagt aagtccaagc tccatctcct acttacacaa tgccccttct 1860
cttgctcctt tgctcatggt gtttacactg gctttctcca gtctctggtt ctcagtcccc 1920
ttacaaagat cctggattag atttgcctgc ctagctctgc ctgattggga ttacacattg 1980
tgtagtgcca acatcacacg ttcggcatct acggtgtgtg cacggcactg cactaagggt 2040
tttacaccca ttctccattt aatcctttca acagccttgt gaagtagata gtattacttc 2100
atttacatat gaagtaactg aggctcactg aggctccggg agtcatcttc ccgaagtcac 2160
aaaactagtg atgaagaccc aagaactgaa cccaggtctg tcagactgta agccctgtag 2220
gcttaactat tctgccttat gaccttctga aacctcatcc tgtggtctga tgcctacagg 2280
ggttggaaga tagtttacta catgaaatcc tttagtttcc aggcttttag ctgcatggca 2340
gagtttgtcc tgagaaagat cttgcccagc tcagcctggc acacacgggt ccataaatta 2400
agtcctcctt gaggtggaat gcatttctgg cacataaaca tggctgcctg gtgtctcttt 2460
tcaaacaaat gggactgtgc tctattctca acaagtgtca gagattcttg tttctcttcc 2520
tcttgttact ctttaggaac ctccagccta gaatagtcag gcatggacca tccaaacaca 2580
aattgctaag atgctttctg gcagcctcag aaaccaggac catacgcctg gctaagtgat 2640
gagggggtca cacagccttg gctcttttca ctgtacccga aaagaaagga agctttagta 2700
atgaaaggat tgctgccaaa atcctgtttc cccttgcag atcttacaga aaacagccac 2760
agaatcccaa ggcatcagca ccaagagagc ctgtagaaac tatctggtct aagctcctga 2820
agcccagaga taagaatgac ttaacccaag ctgcacagcc tcttaacagt ccactgttaa 2880
ccccagaaac agatgcattt gaatctgaaa ggagacagga atcctaatcc tactgctcag 2940
ggacttgctg atttactttg ctgtctatta gcctttctgt gacatgattt tctaccaagc 3000
aaagtctttt attcaagtct cccagagatt tcctatttaa gcgtttgcta tttggccact 3060
taaaaaagac agaagagaag taaacagaag catataccac acaactcagt ccagtcagta 3120
gttgactctc aagatggcgg atgtcactga gatggctgag aaataggaac ggatgtggag 3180
aaaggagtgg cagtggagtt agctgttttg tctacctgct ggatacctgg gaagccagac 3240
tctgtgtgga cagtgatccc tagtcatagg aaactttgcc tgtgctgtca tcagtgtcca 3300
tggagaactg cttctcacct tgagaaatcc ctcttcttcg ttccttcagg tacaagaaga 3360

gaggatggag acgagggtag gcttcttttc agattataca ttctggatca ggaagtctat 3420
atcagtggga ttttctgaat gacccaggtt tttttgtttt gtttttgttt gtttgtttgt 3480
ttgttttaga atttctgtgc tttggagcta gaaggatata taaatgagaa aactgagaac 3540
aaaagaagag acaatatatt tttggttgca agggataaga acctaactcc aacaagcaaa 3600
gcaaaggggg gttttattag aaagatacat tgagctttct atcagaatca aaggaagagg 3660
tgatcagcca agcctccaaa ccagaatcag ggtctagaaa accagagacc acactctttc 3720
tcaccttatg tctctgctgc cctctgccta atggccttgt tttcgcttac tatagaccag 3780
aatttacaca gggatggaga ataagccatc acagctttag acctcatatc ttcaaatatt 3840
tgccactcaa atggaaaaag aataggtcca gcttttata aaaatcttag ggaaggattc 3900
tgatgggccc tgcttgatca catgctccgt ttattgacag tcttcactag aatcacctgg 3960
tttgggttag ggagtagtag tactactaaa gaagagatta ctatttctgg aagaaacaag 4020
gagagaggtg acagctgctt attacaggaa atgatttacc agagatccta tagcaagtta 4080
ggagcggact cagggctaga aacagaatca ataaaatagg tattaataat aatggaagtc 4140
tctgaattct cttcatttct ttccttttct tctttttttt ctttcttcct ttcttttttt 4200
tttttttttg agagggggac agggtcttgc tctgtcaccc aggctggagt ggcacagtgt 4260
acaatcatgg ctatggctca ctgtattctt gacctccagg gctcaatcga tcctcccacc 4320
tcagcctccc aagtagctgg gactataggt gcaggctact ttgcccagtt aatttttgt 4380
atttttgta gagacagggt ttcaccgtct tgccaaggtt tggattgttt ttgtgtgaag 4440
taaacctcag tgttactgag ccgttcattt atttagattt ttaagagatt gccattgagg 4500
ctggatgcgg tggctcatac gtacaatcct agtgactcag gaggccaagg caagaggatc 4560
gcttgaggcc agaatttgaa accagcttgg gcaacatagc aagaccgtct ccattaaaaa 4620
aaaaaaaaaa aaaaaaaaaa aaaggccaca tgtgctcgag ctgcaggtcg cggccgctag 4680
acta 4684

<210> 148
<211> 4125
<212> DNA
<213> Homo sapiens

<220>
<221> misc_feature
<222> (1)...(4125)
<223> n = a, t, c or g

<220>
<223> nbla-04171

<400> 148
gaattcctcg agcactgttg gcctactgga tatttctata aacttttggg aagttgcacc 60
aaacccaata aaggtgcagt atataattaa agttttggtg tattaattag acaatctgag 120
agctcacttg aagataaaag gacaccagat gtgtcaggga gaaaaagttg acatgcttta 180

gataaaattt aaaatcaaaa gctttcaaat cgtcttatgc tctattatag cagaatgatt 240
gcttagttca aagtcttgct gacaatttaa cacattcagt ttgatcattt gggcttagaa 300
tttttttttc tctttcatat ttgcccctcc cttgtctcca aataattttt gagatgatag 360
aagaatatac agtatatcaa atatctgaca gagggttccc tatcaggttc ctggctttga 420
aggtcgagtc tcatttatat ttagttctct tgtttacaag atggaaaacg ttctttcagt 480
tttatcactg tagtctactt aggtcaagct tttaatatga gcaaaggcaa cttattaata 540
tttataagtt gtaaaattta ttgactttta aagaataaaa tctcagtaaa atctcacatg 600
cctggaactt gatatttagt ggtgacattc tctcttcttt gtatttatta tgagtagaga 660
agtagtttta cttcctgctg ggttgtattg atggagaata atgaagttaa cagtcacaga 720
gagcaagtgt gcaggggcac gctcatcctt ggctgatatg agccccctaa tagagcagca 780
ccacctcctc ctcctccttc ctgccatctg taaaagctga gacgaaatga caaccattgt 840
accctcttgg tactgagttg atttaacctc acctttgaaa tctggtaggg aagaggggtg 900
tcgtatgttt tgatttttcc tcccctctct tcacatctcc tcatccctaa ttcacttcat 960
taatgaaaat tctcttttct tcttttctca gctcttccaa ctaaggcttt gggatataat 1020
tacaagacat aaatcttgca gggaaagaac ttttggtttt aaagggggaa gccatcaaac 1080
aaacatctca atgtcctcat gcctttgttt tgtcattact ggtcattgtt gtgttcagca 1140
ttgtgggttt gatttccttt tttttttcta tccagatccc cccacaacta tccctcctcc 1200
cacaacaacc accaccacca ccaccaccac caccaccacc atccttacca tcatcacagg 1260
tatggtatct tcattaccat tgaaaatgtc ctgaatgtat attgtctttc tggtatgtat 1320
aaaaagaagc cgaaaagttc cctttaaatc cttggaattt aatcgttcct ttattttgga 1380
actccaacga cattagatac tatgttctta agttgaatct taaggacctc agaaacatcc 1440
cagcatttaa cagtacgtca gatattttgc agcttatgca taaaagaatt cttttttaag 1500
catcactatt caaagcaaaa tgctagggaa aatggcagct ggctactaga gaacacgcct 1560
atagcttcta cttagacatg tttgatgaaa tggtaagtac tgtggctttc aatcttgtct 1620
ctgtcttagt ttcatctcag tccatgtaag ggaaaatata cacacctaac ttcaatgtgc 1680
ctatttgggc catgctacta aatgcatagt catccttcct gctccagaga gtacccgctg 1740
acaagtgata ctacctgttt gtgctgccta ggtagtcaca agagatcaga gggagtcact 1800
gtcttttcca tgctgagcac tcatgtgccc ctccctgccc tcggtcacat attggaagag 1860
tggaagaggc agccactgtg gttctaactc tgcggcttta tctttttgga ctgtctgaaa 1920
ctcttattca gcatgagttt tactgtctct tgtttctgtc actgagggag gtggcatgcc 1980
atccagccct tcctggcatc cagtggctct ctcatactgc ttgatgtggc ttgtctggga 2040
gccatggggt ttcttctgcc tacctaagta ggaaagcttc tggccacact atgtccactt 2100
gacatgaagg atttccctag ataaggcctt cccaacctgt gtttacaggg acttaacaca 2160
aaaacatgtg aaagtaatta ttcagtgaga aaaaaatatc tcttgaaaga gtaacttttc 2220
cgctgcattt tgttagctgg tgagactgca aggtatctga accgtgtgcc ctggtaccca 2280
cccctgtctc tggacttctt tatgaacact gagtatttga ctcccccaccc ttcatggtca 2340
gctcctttta acaccctaga tatcattgct gtccttttca ttgacatgct agagacattt 2400
ccaattttca tgggagtcag taagatagag atgatgaaaa ttcagagtgt tcaaagaaag 2460
aggaggcctc atcagttgat agaatataca acagattgtc taatctcagt tttcttcact 2520
tttctaccca ttacagtgga aaaaagaacc cttaaggaag ccagtgcttc tcaacattgg 2580
ttacaaaacc atcagtgcca gcacttaatt taaaatcttc tgaaaatcca tcagttggtt 2640
ttcatgtctc actctaaact cagctccaac caggctgctt cctagattct tgtctgtgtg 2700

tgtttctgtt ttggctcttt gttacttcct tctgtaaact taagtaaact cagtttgttg 2760
acctagtgaa ttgattcttt ccattttcta tactattcac cagcatatca aagatgcttc 2820
ttcctaactc tgatttccag agtaactgtg ctatcagctt ttcatactaa gaactaggtt 2880
tttacctttt cacatttctt gcctcctacc cagttcgtaa gccaaattag actcgtactc 2940
ttgtgaagtg ccctttagat actagtgtaa aatatactgc gtaattctcc attgccctac 3000
atatattaac cctttaagaa aatatatcac cagtctattt tatcttcgca gaacttgccc 3060
gggtatgagc cagcatttat taccactcat tctagaaggt gccagttagg tagctatcca 3120
ggtttacatt ttaaggcagt cgagacctta ttcacgtggt atataaacaa ccacatttct 3180
cttttatatg gaacaacacc tttcatttgc ggctggcctt ctcactagct tatgcttttt 3240
tttttaagac ctttcttagc actcgctgaa ctcctccccc tcacaatcaa tctcagcaac 3300
tcagcagagt cgcttcagtt cacagctctt aatccttcac tccagactaa agctaatctt 3360
atgtcctcaa acagctacaa ctcactgtta tcacagttcc gactgcagag actccattta 3420
agaggaaatt taaaaaataa acaatgctct atttctgtcc acatcaaagg tacctctaac 3480
agaaacttgt ctttgcttct gtctctctgc tactggactc tcagctcaag gtaatgtaag 3540
atgatctcat agatgcactt tagcctcttg atgttagcag ttgagtcctc cctgcgcaca 3600
aaatacattt gtggtaatga atgcccagaa tctcactggc acacccaaga gtagttaaac 3660
taaaattgct actaacacaa cattgtagaa ttcgttgcct taatggatgt gatcttctag 3720
gcttactgtg tcttaacaag cagtcttata tatatttatc ttgcccagaa agtacacttg 3780
tagaatttaa catttgctcc tttctccaaa cagattaaag gttatgcagc tgaatgattc 3840
tgtgtcccat tttaaaggtc caggctatcc cctagattta gattagttcc taaaaaatct 3900
tccagtctga atcctgacaa atggatagct aaattaattt ccactcctct tctctcttgc 3960
actgtttggt acattacagc caccattagc cttaaaagct gcagcagcca aattcggtga 4020
cagtaacata aagcctgagc ttaaaaaaaa aaaaaaaaaa aaaaaaaaaa aaaaaaaaaa 4080
aaaaggccac atgtgctcga gctgcaggtc gcggccgcta gacta 4125

<210> 149
<211> 739
<212> DNA
<213> Homo sapiens

<220>
<221> misc_feature
<222> (1)...(739)
<223> n = a, t, c or g

<220>
<223> nbla-04178-f

<400> 149
cactgttngc ctactgggtt accaccacca aatctttacc ttctgaccaa gtcatgcttg 60
tttatgatca acaagaagtt gaagctatat tcttggtgat cttggtagtt gtcataatag 120

gagaattttt cagtgcctct tctgtcacaa tcgtagacac ggtcacactc cagtatctgg 180
gaaaacacag agatcgctat gggttgcagc gcatgtgggg ctccctgggc tggggcctgg 240
cgatgctgtc tgtgggcatc ggnatcgact acacccacat cgaagtgctc atcgatggaa 300
aggggtgtaa gccccccgag tacaggaatt accanatcgt cttcatcgtc ttcggcgttc 360
tcatgaccat ggccttgatc cgttgccact cagttccggt tccgctacaa ccatttcaaa 420
aacgatgatt ctaaagggaa agaggtggan atcccgcagg tggaaaggaa caactctaca 480
gagtcctctg aggagacacc aaccaccaca agccactcgc aggccttcaa cttttgggac 540
ctaatcaagc tgctctgcan cgtgcagtat ggctcaatgc tggttgtggc ttgggtcatg 600
gnttttggat atggcttcgg gttanctttc tctactggca tttggaagaa cttaatggac 660
tcaacccttt ttggggtctg gtcaatcctg aatcatgtgt ctgactgaca gcatattntt 720
tagtccaagc ttattgaat 739

<210> 150
<211> 748
<212> DNA
<213> Homo sapiens

<220>
<221> misc_feature
<222> (1)...(748)
<223> n = a, t, c or g

<220>
<223> nbla-04178-r1

<400> 150
cnttttaaaa atggtattca tttttatttc aacatgtcaa ctgtgcattt ccaaaacagc 60
aggcttttca aaggaataaa tcagaactgt aaacacaaga tacagtacaa gtttttgact 120
tcctacagtc agtttcacaa atccacatac tgtacattca taggtgaggt taagcctgtc 180
acccatttct ttatttctat aattacacaa gcataataaa tacatctgat tttaaaggtc 240
acttaaaatg agtcataatt tacagtacag tacgtttcag ttcaagtgca aaaaataact 300
atttgctgaa ttctatttct ttcagttatt ttatttttaa gctgtgtttt attgtgaagc 360
gagacgtcca agtgtagaat ttcttatccc aaatgcagta ttgggcagaa ctatggtgtc 420
atggagaagc cctcacttta caattgatcc tgctaatcag ggtcagaggg taaggtgaag 480
ttgctcattt cgactgntct tttctttcct ctctctctgt gctcatccac agctattata 540
aattatataa ttcaccttta ttcaagctaa aacaggtttt ccctttttca cacttccccc 600
aggtttnctg gtaacctctg ctttgntttt taagtggcaa gctttacgtg cgatccancc 660
anccatggtc tggctggann tgnaacgttt gcagttgcat acattacatt gnccantggc 720
tgactgggct tcatganaac gaatttnt 748

<210> 151
<211> 747
<212> DNA
<213> Homo sapiens

<220>
<221> misc_feature
<222> (1)...(747)
<223> n = a, t, c or g

<220>
<223> nbla-04183-f

<400> 151
   gcctgttggc ctactgggat gcattcattt aaaatatatt gagcaatcat ataggttttc 60
   tgatgttcat aagccaagta ctattctgaa ggtgctgagt gactgctctg gttatttgat 120
   tgaaaaaaag atttttattc aatctttttt tgaaaaatct agtttctaaa caagttagtc 180
   taccttgtta cataatgtag gagaatgtta tgttttagag aattaggaat aattactatt 240
   gcaaaatcat tagattatac tcaataaaat ggntttaaaa cactcctaat ggtatgtgtg 300
   ccatctccta aaataaaaaa atgcagttca agaactttac tattaaaagt gtcctgaacc 360
   aattttttag ttgtcaatca atctacaatg atttcctgaa agaactcaga gaatcaagta 420
   cttgaaggat tataatatcc caaaatctta acatttattt tccagtaaca tttaagtaaa 480
   cattcctacc acaaatactg gacagagaat aatcttttgc cacctattat tctttatttt 540
   aagttgcata aattagcctt tngtctgtaa aaagatgaan gccnaaagat ctgggnnant 600
   tacnaatcta aagnttntga agngctantt cttgntattn nnttnaggat tncaatttta 660
   canntttttt gaccannnnt gntnnngctg nnnannttca ncnttgnnnn gatnntnann 720
   gggntnctan ggntnnnttn nntntgg 747

<210> 152
<211> 748
<212> DNA
<213> Homo sapiens

<220>
<221> misc_feature
<222> (1)...(748)
<223> n = a, t, c or g

<220>
<223> nbla-04183-r1

<400> 152
tttttcnttt tttgtacatt cgaacctttt taatgaaaat tacaggtcat cgcctggtta 60
agtgttaaat agcatgcatc agaccatgag aaagctgaga cactccatcc cccatccaca 120
ctcccgtcat cctcttccac tctcattaga atcaatgaga tgtaaggtga tgcactgggc 180
atcttaataa gtgaaataag aaaaaagtag gaataagcta tgtgagtctt atacggcaat 240
tttgctgact ggggaagagg ctcagtttaa ggaaggaaca tcatgtccag ttttaggcat 300
tttaaacatc aaaccttgcg cctactgtgt aatgcagaaa ttcgactatt gagttgtttc 360
aatgaaaatt tacaagactg agagcaaaac acattcacta ttaagtgcaa atgaatcaca 420
aacaggcaga aaacacttcc acatagtaat tgctattttg agttgagatt ttaacttcta 480
atctaaaact cctctgtttt gtgtcctcac ccttgctcaa ccacagaata aagacaatat 540
tttggatctt ctggactaaa gaaagaactg gcagagaaaa atctcctcat actcagtcac 600
tgnacaggaa gatggcctgt agtctagatt gggggggtgg gttctggcan tacaaccctg 660
tcttccatnc caaagatctg aatttaaatt taagcaaagt aagactgagg gaaaaattaa 720
cctaaagtaa atgnatggna atatatga 748

<210> 153
<211> 741
<212> DNA
<213> Homo sapiens

<220>
<221> misc_feature
<222> (1)...(741)
<223> n = a, t, c or g

<220>
<223> nbla-04193-f

<400> 153
gcactgttgg cctactggag agtatgatga tgttaatttg ctgactgctg acttccagct 60
tctttttaac aatgcaaagt cctattataa gccagattct cctgaatata aagccgcttg 120
caaactctgg gatttgtacc ttcgaacaag aaatgagttt gttcagaaag gagaagcaga 180
tgacgaagat gatgatgaag atgggcaaga caatcagggc acagtgactg aaggatcttc 240
tccagcttac ttgaaggaga tcctggagca gcttcttgaa gccatagttg tagctacaaa 300
tccatcagga cgtctcatta gcgaactttt tcagaaactg ccttctaaag tgcaatatcc 360
agattattat gcaataatta aggagcctat agatctcaag accattgccc agaggataca 420
gaatggaagc tacaaaagta ttcatgcaat ggccaaagat atagatctcc tcgcaaaaaa 480
tgccaaaact tataatgagc ctggctctca agtattcaag gatgcaaatt caattaaaaa 540
aatattttat atgaaaaagg ctgaaattga acatcatgaa atggctaagt caagcttcga 600
atgaggactt catccacttt ggctgcagcc agactgacag gtccttnaca cagtaaangc 660
agccttggtg aagagagaaa tccactngca gnattancgn aataaagagc cgtccaggag 720

gtcgttatna gcaataccat g                                    741

<210> 154
<211> 793
<212> DNA
<213> Homo sapiens

<220>
<221> misc_feature
<222> (1)...(793)
<223> n = a, t, c or g

<220>
<223> nbla-04193-r1

<400> 154
nnaagaaccc tttggactgt ccgcctactg ttgagtatga tgacnttaat ttgctgactg 60
ctgacttcca gcttcttttt aacaatgcat ngtcctatta taagccagat gctcctgang 120
ggnaactctc ttnncaaacn nnggcctntc nccangcnca nntaanntan gtanctntnn 180
ttganacngc gtttnaccct gttgnncang atggnnttga tcttcnnacc tcntnatcct 240
gcntcgncct cccaaagtgc tgggattaca ggtgtgagcc actgtngccc attcaactat 300
gggattttta taatctnnag nttatgcann atgaaacnna ngcttacctc gtncatnanc 360
tgnnnantnn cnacaaangt aantnttaac cacaatccnt nctncctgct naataatnaa 420
tntanggtan ancttntctn aatatatttn gnncctnnng atnaatncaa cttgnaatnc 480
cttatttctc gntaccnana accngtgcac ttnnannntn tanactaatt gtngcnannn 540
ntcannnnnn gcggacatcc anaannctat tagacanntt ggttccnntn ncnnaanaat 600
cctnctcnnt tngcgaatna anncncnnan nntntanntn ttnnttgcct tnnancttnt 660
cncnaacnnt natnatnntn naacgtnnnt atnaatcntc nctgggancc naaatnnttc 720
nntanccnnn tcnttcctaa ggnnatccct tttntctctc taccttncnn naaatttnaa 780
tngcngnann ncc793

<210> 155
<211> 2753
<212> DNA
<213> Homo sapiens

<220>
<221> misc_feature
<222> (1)...(2753)
<223> n = a, t, c or g

<220>
<223> nbla-04196

<400> 155
   gaattcctcg agcactgttg gcctactgga tcgtaatgct catcggaaga ataagccgtg 60
   tactgatttg gaaagtggag aggaaaagaa aaatattatc aatacccctg tgtcttcatt 120
   agaaatactt ttacaatctt tctgcaaact tggcctgatt atggcatatt tctatatgtg 180
   tgaccgtgca aatctgttca tgaaggaaaa caaattttat acacattcat ctttctttat 240
   tccaattatc tacattttgg ttttgggagt attttataat gaaaatacta aagagactaa 300
   agtattaaat agagaacaaa cagacgaatg gaaaggctgg atgcaacttg tgattttgat 360
   ttatcacatt tctggagcaa gtacattttt gcctgtatac atgcacattc gagttctggt 420
   tgctgcatat ttatttcaga cagggtatgg gcatttctca tacttttgga taaaaggaga 480
   ttttggaatc tatagagtat gtcaggtttt atttcgtctc aatttcctgg tagtggtgtt 540
   atgtatagta atggatcgac cttatcaatt ctattacttt gtccccttgg tcactgtatg 600
   gttcatggtc atatatgtta ctttagcact atggccacaa ataatccaaa aaaaagcaaa 660
   cggaaattgt ttctggcatt ttggcttact gttgaaacta ggctttttgc tgttattcat 720
   atgttttttg gcatattctc agggtgcatt tgagaagatc ttttctcttt ggccattgtc 780
   caagtgtttt gaactgaaag ggaatgtata tgaatggtgg ttcagatgga ggttagaccg 840
   ttatgtagtt ttccacggaa tgctgtttgc ttttatttat ctggctttgc agaagcgtca 900
   aatactttct gaaggaaagg gtgaacctct tttttcaaac aaaatttcaa attttctgtt 960
   gtttatttca gtagtttctt tcttgaccta ttccatctgg gctagcagtt gtaaaaacaa 1020
   agcagagtgc aatgaactcc atccgtctgt ttctgtggta cagattttag ccttcatcct 1080
   aataagaaac atccctggat atgcccgttc agtttacagt tcattttttg cttggtttgg 1140
   aaaaatttca ttagagctat ttatttgcca gtatcacata tggctggcag cggacacaag 1200
   gggtatcttg gtactgatac ctggaaaccc tatgctcaac atcattgtca gcactttcat 1260
   atttgtttgt gtggcacatg aaatttctca gatcactaat gatcttgcac agattattat 1320
   tcctaaagat aactcatctc tcttgaaaag gttggcatgt atagctgcat ttttttgtgg 1380
   actcctcatc ttatcatcca ttcaagataa atcaaaacat taggttccaa aaattctaaa 1440
   aaacctaaac tcttcaggct acctttgtgt gtctctagaa gagaaaagca tctatctgga 1500
   gatataaatg tgtatgtaaa tataaacgtt tgtggcaaga ggacagttct gtgacatctg 1560
   ttgaacatat gtggttgtat atattggaaa tgtacatatc caatatgaaa tactaaaaca 1620
   aacaaacaaa caaaaaacca gaatgcattg tataggattg catgtgaagt cttttctact 1680
   gaatctatat ttccatttat aagtgatttt aagttaacat atgaaggcag ggaaatgatt 1740
   acctttccag taaaaagtat agataattta attaacttag tgacaccacc aagtgttttg 1800
   atataactaa atttgtggta ataagactgt ctgcacctgt attcattgtg gaacttcctc 1860
   tttcattgga aactttctta ctcaagaatg acggcagtat tgttttctta tatgtgcaat 1920
   gaagtggaat gataaacagt atgcctttaa tttatatgtg ttcttgttct gatgttgttt 1980
   cctgaaatga tttttcttcc taactgtggt tttcgggtat gcaagcctaa atctttgtac 2040
   actttgtctc acagaatagt tctgaggctc catgacaggg ttttgtcatt gttgatgtta 2100
   ttgttgcttc gttttataaa aaagccaaaa tttttttcca atccaaacgt tcacctgttt 2160
   cctttcctca agctatacca gtgtaatacc agttaccctg tggatccatt taatatgtta 2220
   tccccactaa ttaattttcg tatattattt ccaatatttg gaaagctctt tatagccatt 2280
   tggtatttcc tattacccac ctcctatttt aaatatttat cagtctaaac ttgtgcagtg 2340
   tagtaaacat gcaagttgtt atgattgagc tgtattacca taagtagaat tttaagtaaa 2400
   ctggtgaatt tgggccatag atgtttttgc tttttgtttg attttttttt acaagctaac 2460
   tgttagaggt atacatttat ttatctgttg tacagatttg attatgattt taatgtttga 2520
   aagattgcac ttgtttgctt ttactatatg tggggtaaaa tatattttct gttcacagta 2580
   tatgaaaata tggagtaatt taaacagtaa ataaacattc tgtggatgct tatttttgta 2640
   ttggcaaagt atcaattaaa ctatatgtgt tctttttcaa aaaaaaaaaa aaaaaaaaaa 2700
   aaaaaaaaaa aaggccacat gtgctcgagc tgcaggtcgc ggccgctaga cta 2753

<210> 156
<211> 809
<212> DNA
<213> Homo sapiens

<220>
<221> misc_feature
<222> (1)...(809)
<223> n = a, t, c or g

<220>
<223> nbla-04219-f

<400> 156
   gnagatgnnn nnttgnnnnc cnttntttga annccctnga gcactgttgg cctactggtt 60
   tatcaatgtg actccacggg ggagtccatg gtgatgatga tgaggaggag gatgatgatg 120
   atgagacacc tctaaacttg gaacaagttt aagactttat gagagaagaa aaaaaatcac 180
   caacaagaat tgtttgagga aaaattataa ctatcctgtg ttcatttttt ttttataaac 240
   aataagaaaa agttgttgga ttttttttta atgatttctt ttttggggga gggaattttg 300
   ttgcagtttt atggtggaaa atgcaaaaac cagagccagg tgcataatct tgtaatctgt 360
   ggatatccct ggagcaggac tgagtaccag ttaaaatact ttttggggat acacatgtga 420
   gatactaagt acttgcagaa gatttttgtc tctcttttta aagtctcttt ccttggaata 480
   ttgtgagaat atttgtggcc atttaaaggt ttgtgtgatt tttgctaaaa tgcatcacca 540
   acagcgaatg gctgccttaa ggaccgacca aagaactnag tgatttactg gattcagtgc 600
   natgttttca cctcctgtga gcagtgggaa aaatggccac ttcttttggc aagtggacat 660
   tttactggct caaatgtaga agacnnaagt tagctcaagg tcctgggggg aatggaggac 720
   atccaagccc cntccaggaa cctntgggng atgggacttc cntttgaccc ccatgaccag 780
   cangggacct ttgggtcaca ttgacattc 809

<210> 157

<211> 769
<212> DNA
<213> Homo sapiens

<220>
<221> misc_feature
<222> (1)...(769)
<223> n = a, t, c or g

<220>
<223> nbla-04219-r1

<400> 157
gagnggntnn ntttgtggcc tttttttttt nnntttgttt ttctgntttt tgataattgg 60
gaatgctgaa acctcttgcg tctgcgattc ataactactc agacttgtct tatattacaa 120
aaatgggggt taaggagaag tgtttatgtg ggtttaagat aatacagctg ttaaggaagt 180
ggtctcttgt tttaatgaag caatgtggca acttggaccc ttttacatct gtcccatgtg 240
attcgatgcg tctcccattc cagggtgtgg gccggccaag gagagagggg gaggctccga 300
ggacaccttc tcttcctccc ttcttttcag acacgcagct ttcggattca gattcctttc 360
tcggacttgc tgctccagac tgaggatgac ggccaccgcc tggtggagga tcaggagctt 420
ggtctggggc ttgcactctt gaggtggagc tgcaccatgc ggncgagctc tttgaaaagc 480
ctcgttgatg tcacggaccc gaaacgctct cgggcattgt gggcatcttc ggtcctttta 540
cgctctgcct tctgntctgg nggcaaggnc ctcatcgcan tattgntaga tcttgaccta 600
ntaattgatt tgatatcctc ttggcgcatc taattcttgn cctccgaaaa attcgggctt 660
tgaaggtctt ataaccttgg cattggattt gactcaaact gcanangaac actttgccct 720
gtagcctggg gcatccttgt aaggncctgg ctgggttagn cagggaant 769

<210> 158
<211> 768
<212> DNA
<213> Homo sapiens

<220>
<221> misc_feature
<222> (1)...(768)
<223> n = a, t, c or g

<220>
<223> nbla-04241-f

<400> 158

gnnnnnnnnn nnnnnnnnnn nnttggattc ctcggcactg ttggcctact gggagtaatg 60
aagggtgaat ttcgagctgt gaggcagtaa acttattgca gttcctcaaa cttagaaatt 120
gggttgaatc ccaataccct aatttcctgt tccacatcac atttcaacta atacttgcct 180
tttatcacag caaccaccaa caacctagct ttgcaaagaa atgacatcat caaaaggaaa 240
ataacacaat atgttaaact gtgaattcgc ttgaaatagt cattcatgca agcatctgag 300
aaatgttgtg tattgctttg tttccctcaa agacaaaata tcctgcacac ctctgtgaat 360
ttgctgtagt gccctgaggt acactggcac ataatttggg gacctcagaa ctaaaatcat 420
gtctttttca gcaactcttg accgcatcac tgtaacatat gagattgtaa gatgccaatt 480
tggttaagac tacgagtaat gtgtaaagtg aaaattgtat ggggtcaagt attattttaa 540
aaagcctctt angaagttac caggttggag gctgatatac catctnctat taagtataag 600
acttactggc actggttcct tancattgaa acaggttgct attctttaag ancagatcct 660
ananttagct tggctttgag cattttgcta anaaccaatn ccttggcnta aacccaacat 720
cattntcagn gngngaaaag ctnacanngg aaaagnccat tccnagnn 768

<210> 159
<211> 3553
<212> DNA
<213> Homo sapiens

<220>
<221> misc_feature
<222> (1)...(3553)
<223> n = a, t, c or g

<220>
<223> nbla-04246

<400> 159
gcactgttgg cctactggtt atgccaaaga acaaacagtt cctgcagctc tcatgttgta 60
ttcaccacaa attcaggaag tggctgaaga aggggattac atgcctgcac aaatctttag 120
tgctatcgag agtgtcaagc tttgaaaccc tttgaatact tctgaagagc aagcgcccac 180
tgatgctgag gtcaacaaaa tcagagaagc tgacatttcc atttttgcc aatacttcag 240
gtgacctcat aatgaaaccc ttgctgctct acagaaaatt gtgcccaaac cctctcaggg 300
gaaataaatg agccaagttt ccagtgtact agcaagcaaa cagaaaagcc cagatgaatc 360
ttcctctcct taagggatgg tttgaacagt actttcttgt ggatgttcaa gactacttaa 420
aagaaaaaaa aaataccttg aattcaaagt cctgctgatt cttcagtcta tttggtgctt 480
caggtacatt tgccaatatg catcctcatg gtaaggttgt ctttataact agccacatgt 540
ctgagattct tgagcctttc agtcagtgtt tgatctggcc attcaggaag gcttattata 600
aactaatgta taactttgtt cacaatctcg caaagtttcc actgtctgaa aatcctagtg 660
catgagactc ctacatcgtt attaatggca tatccttaat aaaagtttgg cttttgattt 720
ttaatgggtt ttcaggagat aacttcccaa agaggcatta gatagtttaa cagagcctgt 780

cattaatgtg acctgtgaga agacttggct agaggtggtg aaatatcttt cctctatccc 840
tcccaaagac aagaaaaacc tatggatgag gatgaaaatt tggcacaaga gcaatcattg 900
gcggaagttg aatctgaaac tgttgacacc aattcaagtt aatgctgcta gaggctgatc 960
ctcaggaagc tttcttgact ccagaggtta ttatcataag tgatgatgaa gacaattagg 1020
aggctgtggg actggaaaca aatacagcaa taagaaacag gagcaaaatt tttagaacaa 1080
gattaaaacc tccctaagaa ggtaattaaa attggcatct ttacatgtgt cagatattac 1140
ctgttcaaag tttgagtgac ttagagttct ataaagaggt gctatgatgc catcaaacat 1200
aatcatattg gacagaaaca atcttcaata gaacttaaat catgtgccat ttattactgt 1260
tgctggacag ctgataaaac taccttctga caaagtttga tttaattaga ctctaataaa 1320
aggtcctatg agactttcta aaagactata ttgggaagaa agaaacctca gaaaagtcta 1380
aattatcaag tagtaccatt taaatactct tactggacag ctaataagct accttcagac 1440
aaagattgaa tgattaaatt gaactccata cagaactgct aaggtgtctt caaaaaggac 1500
ttgagaagat gaaagcatct ttagaagggc cacttaaatt cacttgcttg atagaaataa 1560
agcctcaagc aagttgttat aacttcagga ttcgacttca ctgactctaa gagtatagac 1620
atccataatt tgaactaatg aatagtccac ttctgttcat tgcttctctg tcacccccat 1680
ttgccactac cataatgagt gatagataca tcttcatcac ctctggaaat catctcagga 1740
tctaaatgga aactgtataa agcctatcat ttttactgat ttaaactatg taaactcatt 1800
attcttttta tgtaatgtgc tgttgttatt gtttacctgc ataaaaatat ttatgagggt 1860
tttcaacagt ttacttgaga cctcattttt gcccattttt ttccttcccg atatcatgat 1920
cccctcagct gaactttctt accttggggg ttgttcagga actgactctc atggggaaag 1980
agggattact atttctgtgt tcctatctct tggcaactgc ttaaccacag tcagtcttga 2040
actaatggaa ggagcactgg acttgggttc ttgagacctg ggttcatgtt cagttctgcc 2100
actgattatt gtgacattgg gccagtcact tgatttctct gagcctcagt ttcatcacct 2160
gttaagtgag gatagtaata cctggcacaa atatcacaat attagtgata attgaatata 2220
attataagta cccaatggct attaaaagta aaactaggaa gtgctgaata accataatat 2280
cattatattt gtagcatttt ggaccttatc aatgaacaac tgagaaaact aggtttttga 2340
attcttttac tttttaaagt aacttcctcc catttttatg tcaattatag aaaattttaa 2400
aaagaaaatt aaatgtgcct ataattttat aagccggagg taactaagtt ggtatttttc 2460
ttcttagtac ctctttgtct catcataaat tgttcatcaa tgtcaaaaac ttggaaaata 2520
aagataagca tatagaaaaa aataaaaacc acccataatc acaaatccca gaagcaatgt 2580
taatattttg gtggatttat ttccagtctt tttctatggc tatatgtgca catatataat 2640
ttttacatag aaaaagtcat aatgcataca gctttgttgc ttttagcatt tttatcatga 2700
atattttcct acatttatgc aaagtatttg taaatatcat tttcaatggt gtataatatt 2760
tcatcatagg atgacatcat ggtttagtta accattttct tttgttggat atttgagggt 2820
ctttccaaat ttggccattg taatttcaca atgtcttttt cattacctaa ctgaaaatat 2880
ttgctttggt gaaagcagag gatttttttgt tgtttgtttg tttgtttttg aagaagtcct 2940
tttaatagct acatttcatt gactaagtgg aacttcaaga gacaggtaga agaaaaaaaa 3000
aaagaaacag tagatgtaat ttcaagattg aggatttatt ttgttagtga ctgttccaga 3060
agctgaattt tggtgttaga gcaattcagg agggacagtt tgccaccatt ttatgatact 3120
ttactgtaga aaagttttca ggatttagac caggaaagag acatcctaac catatgggtt 3180
gattttattt tatggaccct gtgaagtctg ggactgatca ggtttctctt ttgttggcta 3240
ctagaaagct tggagtcaaa tgtgtggtca atgcatagca cttgtaatgg gactctacgg 3300

tatgtatgca ctttgtatta gctttctgcc aggctccatt tcgtgttcct atctttattg 3360
tttttgtttt ttccttttac tttcttatct actttgaatt tatgctatca tgttgtattt 3420
tgtgtattct tgtaagccac ctgacatcca tcttggaaca tggtggggaa taaacacact 3480
aataaataaa tacattaata aaaaaaaaaa aaaaaaggcc acatgtgctc gagctgcagg 3540
tcgcggccgc tag 3553

<210> 160
<211> 1971
<212> DNA
<213> Homo sapiens

<220>
<221> misc_feature
<222> (1)...(1971)
<223> n = a, t, c or g

<220>
<223> nbla-04261

<400> 160
gaattcctcg agcactgttg gcctactgga tctgtcacta acgtcactgc gaccgacgct 60
gatgacccag tttatggaaa cagtgcaaag ttggtttata gtatattgga agggcagcct 120
tatttttcca ttgagcctga aacagctatt ataaaaactg cccttcccaa catggacaga 180
gaagccaagg aggagtacct ggttgttatc caagccaaag atatgggtgg acactctggt 240
ggcctgtctg ggaccacgac acttacagtg actcttactg atgttaatga caatcctcca 300
aaatttgcac agagcctgta tcacttctca gtaccggaag atgtggttct tggcactgca 360
ataggaaggg tgaaggccaa tgatcaggat attggtgaaa atgcacagtc atcatatgat 420
atcatcgatg gagatggaac agcacttttt gaaatcactt ctgatgccca ggcccaggat 480
ggcattataa ggctaagaaa acctctggac tttgagacca aaaaatccta tacgctaaag 540
gtagaggcag ccaatgtcca tattgaccca cgcttcagtg gcagggggcc ctttaaagac 600
acggcgacag tcaaaatcgt ggttgaagat gctgatgagc ctccggtctt ctcttcaccg 660
acttacctac ttgaagttca tgaaaatgct gctctaaact ccgtgattgg gcaagtgact 720
gctcgtgacc ctgatatcac ttccagtcct ataaggtttt ccatcgaccg gcacactgac 780
ctggagaggc agttcaacat taatgcagac gatgggaaga taacgctggc aacaccactt 840
gacagagaat taagtgtatg gcacaacata acaatcattg ctactgaaat taggaaccac 900
agtcagatat cacgagtacc tgttgctatt aaagtgctgg atgtcaatga caacgcccct 960
gaattcgcat ccgaatatga ggcattttta tgtgaaaatg gaaaacccgg ccaagtaaat 1020
atctccatgt tgttaatgct gaatatgttt gtatacaact gtctcatatt tgactaacct 1080
gcattatatt gtgcatttgc atcatttatg atctgcaaag tctcaggcaa gaaacacatc 1140
caaatgggaa cagagaggga attttttttc tccatcagat gttatttctc ttcctactta 1200
tgaccaaatt ggctcctaaa gtggaacaac ctgctgtgcc acaaacccag agccaaatca 1260

ctgtcttaaa atatcttggg atatggatga tagcaggaga tgtcttagag tgacgggggc 1320
tgaattttta ctttcaaatg acaagaagca aggtaaaacc tcgtggagag gagacatttg 1380
tttcacagca aacatatgaa taccaaataa tagtgaaacc caagaaggcc agtggtttat 1440
agaaagttgt aggaagtgaa tattgaaatc tgtaaatatg taaatatatt tagataaaca 1500
taagatgaac aacacgtttt acatgtgtgt attagtagaa catttttgt gcctcatatt 1560
gcatagatgg gtcaatgaaa acagaaaaaa aagaaacata aagaaagtga gagagaggga 1620
gaaggaaagg gagagggaga gggaatggga atgggagtgg gaggaaggga gggagggaga 1680
gagaaaaaga gggaggaaga aagggaggaa aatagttaaa caagcaagat gaacagaaac 1740
aaatatctgc tatactctga atgtcaaaat ggaaatagcc tgtgtaaagt tctgcaaaac 1800
cataaatgta atagatttat tttaacaagg taccaaagta ctgtatgttt aagaaattta 1860
tcatttttca acttcctaat ttatttctgg atggtgacat tttaatttaa ataaacagca 1920
gctgacagta aaaaaaaaaa aaaaaaaagg ccacatgtgc tcgagctgca g 1971

<210> 161
<211> 785
<212> DNA
<213> Homo sapiens

<220>
<221> misc_feature
<222> (1)...(785)
<223> n = a, t, c or g

<220>
<223> nbla-04264-f

<400> 161
gnnnnnnttn nnnnnnttnn nnttggattc ctcgagcact gttggcctac tgggatagaa 60
atttatgctc tctccatttg tctgatgttc aatgaaagac acaaacaact aaacaggtag 120
ttacaaatac tgtgtaataa gttgttgtgg aaagtacatt gtgctatgag aactcaggaa 180
taagacatag tccagatttg gaggatcagg aaagacctcc atgggaagag atgtataagc 240
taagactaga aatgggacct ggaaataaaa cttgggctca ttacggtagc aacaagacct 300
ggagacttag atgtaagata gaagaagaaa ggctccttga gaagcttaag gagacttaga 360
tgtaagatag agggaaaaga aagcctcctg gagaagctta aggaggggca tgggatgcat 420
aatgggcaag gtggggctgt ccatgtatta actaagagga gagctggaaa agacgatgaa 480
gggccttgac atctaaatta cacacaaaca tgtaaaatta catgcaatgn attttctttg 540
caaagaagac agagtgatgt ctaatatatt tcagatttct gncataaaag cattttaaaa 600
ggtcataaaa cttaatattt aaaatggtan ttttttttag aaaaatacct tgngtgactt 660
aagnatttcc tgaataaatg agctttnctg ggaatcctca tantatcaag cctttggtct 720
taanaaangg accaaggtnn tgtnggaacn nggaaaaaaa tggnttnaan aatttaataa 780
tcntt 785

<210> 162
<211> 784
<212> DNA
<213> Homo sapiens

<220>
<221> misc_feature
<222> (1)...(784)
<223> n = a, t, c or g

<220>
<223> nbla-04267-r1

<400> 162
nngnagnnnn nnttntgtgg cctttttttt tttnnttttt aagtaatggg taaagtttat 60
ttcattttta ataacaatta gaggacaaaa tgtttaaaat ttgcagttta aaacatgcac 120
attcacaaaa ggccaatgac acataacact gcatagaaat aatattactc aattttaata 180
actataaaac acagtgcatc aaacatcaag ataaacaaac ctgagaaaac tactataacc 240
acagattcaa tactctccac tcatgcagct tcacaatttc tacagcagtt tcaggaggaa 300
tggtttttca gggagctgaa aatactactt tatctttaac gcaaaactgc agttttctgt 360
agtagctgcc ttccaaggct gccctgtttt tcttaaccta ataaacttga aaatgtaaaa 420
aatgacgatt aaagtagtta aacaacagat agtatttact gcatttatgg cttccattta 480
gaaccatgaa acataaaaat attattttta actattctgc tcacatcttt gcaagaacag 540
atttacctgt ggaaaggtgc tggtaattca aagtaagcaa atatgaaatc taagtttcta 600
cttaagggag attattgctg agaagtttgg gagccttant aagaaaagtc ttaaangcag 660
cttaacngga agatcaantn aattggtcaa aattctttgg aaataatcta cttattaaaa 720
aagtgggccg anggnccant aattggctct ggaattttaa attcaggtag gccgggaaaa 780
actt 784

<210> 163
<211> 793
<212> DNA
<213> Homo sapiens

<220>
<221> misc_feature
<222> (1)...(793)
<223> n = a, t, c or g

<220>
<223> nbla-04271-f

<400> 163
gnnggnngnn nttgnnnccn nnnntttgan tcctcnncac tgttggccta ctgggttcat 60
gatgtccaag aanattgaga ggagctactg aattgtgttt ccctaagttc atttacactt 120
tagcagcaaa tcttgatgat cagagcctaa ggatgcctaa gtttacatgg agtggtaatg 180
gttaagtaaa ttgatttggg taatttttca acctcatcta caccacacta agaccaaagt 240
tctctaaaaa tttctgaaat aatgattttt atatagnttc attaaaagtt ttaaaagtat 300
ctatcagcat tttctgtaaa agtgtcaggc tgaaaagata ccactaaaca gtcagctcaa 360
aaaagatagt agataaaata tgatgaattg ccacatctgc atttcctatg tttgcaagcc 420
accagtaaga ctgcatgatt tccaagttgg aggacagagt ctaggaatgt gaaggatgaa 480
gggtttcttg tgcaccgttc tcttgagcac gtacgccttc agaaggtaga taggctaacc 540
tgcatcactg gataaaaact ggatttctct tactttttca tgtgtatgtg gctcatgtag 600
attttggtat ttttcttgga aaatatgttc ccgctgncta ctgaagtaac tgngtaataa 660
tttncaattt tctgntgggc attacctcat ggacaccatg ccacccttta aacttcattt 720
acccaaaacc atactcaant attcccccat aanccttttc acctatggtt cnttatcaaa 780
atcacctttc tta 793

<210> 164
<211> 795
<212> DNA
<213> Homo sapiens

<220>
<221> misc_feature
<222> (1)...(795)
<223> n = a, t, c or g

<220>
<223> nbla-04271-r1

<400> 164
gaggggtnnn nttggtggcc tttttttttt tnntttgcca gtttttatgt tgaagaagaa 60
aaaatatgaa taaacaaata agtaacctat ttttggtatc aagtaaatgt gcagcctaga 120
gaagggggat aattctagaa attggaaaca aagttggccc tttcaagtta aaccactaga 180
aatatacaag ggctgacatt tctgcagata ctctaagaga cggtgagttc caagggaaaa 240
ggggacctcc caaatcaaat cagggcatta caaaatctaa tcaaggtgac aaggatatat 300
ccccagcata agactgtaac taaagtggaa gggggcagga tcattcactc attcattctt 360
gcatatattc atttcaccat ttattattaa tagcagcaat ctgctttgtg taaagtgcta 420
cacagagata aacgagaaat accttgcatt tagacagcaa cttaaaattt caaaagcagn 480

tttatcattt agagtccgtg gcagggaaca gatggcaaac ctgactttgg taatttaagg 540
agcctttaat aaagggactg gttattaang ngtgggtaga gtacagggag accacanggg 600
ccagtactct ggggncttta ctacctctan ggcctaagga gccncatgaa ggangtnatt 660
gnttcaacag aacctggaga tatgaaaact gtnnaaangg nccacttgca caactgggnc 720
cttaatcaan ggactctntn attctaaggn gacatgttgg aangggatgg ggaatccttt 780
tnaccnccnc tntnt 795

<210> 165
<211> 3028
<212> DNA
<213> Homo sapiens

<220>
<221> misc_feature
<222> (1)...(3028)
<223> n = a, t, c or g

<220>
<223> nbla-04300

<400> 165
cactgttggc ctactgggat gcatgggaaa agcactgctg tgctagaaat ggactattca 60
attgttgatt ttctaacgct gtgtgaaaca ccatacccaa atgattttca agaaccatat 120
gctgtggttg ttcttctaga aaaggattta gtacttatag accttgcaca aaatggatat 180
cctatatttg aaaatcccta ccctttgagt atacatgagt cccctgttac atgttgcgaa 240
tattttgcgg attgtcctgt ggaccttatt cctgcacttt attctgttgg agctagacag 300
aaacgtcaag gttacagcaa aaaggaatgg cccatcaacg gaggtaattg gggcttgggt 360
gctcaaagtt acccagaaat aattattaca gggcatgctg atgggtcagt taagttctgg 420
gatgcttctg caataactct acaagtatta tataagctaa agacatctaa agtatttgaa 480
aagtcaagaa ataaagatga caggccaaac acagacattg tagatgaaga tccatatgcc 540
attcagatca tctcctggtg tccagaaagt agaatgctgt gcatcgctgg agtttcagct 600
catgtcatta tttatagatt cagcaagcag gaagtaatca cagaagtcat tccgatgctt 660
gaagttcgat tattatatga gataaatgat gtggaaactc cggagggtga gcagccacca 720
cctttgccaa cacccgtggg agggtccaac cctcagccca tccctcctca gtctcatcca 780
tctaccagta gcagttcatc tgatgggctt cgtgataatg taccttgttt aaaagttaaa 840
aactcaccac ttaaacagtc tccaggttat caaacagaac tagttattca gttggtttgg 900
gtgggtggag aaccaccaca acaaataacc agcctggcag tcaattcttc ctatggactg 960
gtggtttttg gtaattgcaa tggcattgct atggttgact acctccagaa agcagtgctg 1020
ctcaacctgg gcactattga attatatggc tctaatgatc cttatcggag agaaccccga 1080
tctcctcgta aatctcgaca gccttcagga gccggtctgt gtgatattag tgaaggaact 1140
gttgttccag aggatcgctg caaatctcca acctctgcaa agatgtcaag gaagttaagc 1200

ttacctactg acctaaagcc tgatttagat gtaaaggata actcctttag ccgatcacgg 1260
agttcaagtg taacaagcat tgacaaagaa tcccgagaag cgatctccgc tcttcatttc 1320
tgtgaaacgt ttactcgaaa gacggactcg tccccttccc cttgtctatg ggttggaaca 1380
acgctaggaa cagtgcttgt cattgcactg aaccttcccc caggggggaga gcaaagactt 1440
cttcagccag taattgtgtc tccaagtggt actatattga ggttaaaagg tgcaatcttg 1500
agaatggcat ttctggatac cacaggctgc ttaataccac ctgcgtatga accctggaga 1560
gagcacaatg ttcctgaaga aaaagacgaa aaggagaaat tgaaaaaacg gcggcctgtc 1620
tcagtatccc cctcctcttc tcaggaaatt agtgaaaacc agtatgcagt gatatgttct 1680
gaaaagcaag caaaagtaat ctcactgcca acccagaact gtgcttataa gcaaaatatt 1740
acagagacct cgtttgtgct tcgtggagat attgtagcat tgagtaacag tatctgcctt 1800
gcctgtttct gtgccaatgg acatataatg acttttagtt tgccaagttt aagacctctg 1860
ttggatgtgt attacttgcc ccttaccaat atgcggatag ccagaacgtt ctgctttacc 1920
aacaatggac aagcattata ccttgtttca cctacagaaa tccagagact tacttatagt 1980
caagagacct gtgaaaatct tcaggaaatg ttgggtgaac tcttcactcc tgtagaaaca 2040
cctgaagcac caaacagggg attctttaaa ggcttatttg gaggtggtgc acaatctctt 2100
gacagagaag aactatttgg agaatcgtcc tcaggaaagg cttcaaggag ccttgcacag 2160
catattcctg gccctggtgg cattgaaggc gtaaaagggg cagcatctgg agttgttggt 2220
gaattagcac gagccaggct ggcactagat gaaagagggc agaaacttgg cgatctggaa 2280
gaaagaactg cggccatgtt atcaagtgca gagtcatttt ctaaacatgc tcatgagatt 2340
atgttgaaat acaaagataa gaagtggtac cagttctgac aaccagaatc caataagtcc 2400
aacttcagcc agaaggaaaa aagttttcca tttttattac attctttagg aaagttaacg 2460
ttaaagggat gttcgtcact gaatactgtt ctttcctagc acagtcatgc actgttttac 2520
ctcagtcatg tggctttaac tgaggagtgt tcacacgcac tcgaaatgga gtatatggtg 2580
tgtgccagtt atgagttgac catttgggaa ttaaacaggt cacacgtgac agatgaagaa 2640
accaaggggg ctgctgagga gacctggtgc agggactaat cctggatcat tcctgtatta 2700
aactttcata tgccaaaagg gtttgtgccg ttttatctgc catcagtgtt tgacctgttt 2760
agggcagagg caataagtca gaagtcttga agttgaaata gttatatgtg tgtcattgga 2820
ctggattata aacagctgtc ttggactttc cctctcttaa cactgactgg tcatcagtat 2880
cattagtgaa aaagaaacaa attgtttgtt atcatctctt tagacagata agctgaatgg 2940
tgggctttaa ataataaaaa catacacata gttgaaaaaa aaaaaaaaaa aggccacatg 3000
tgctcgagct gcaggtcgcg gccgctag 3028

<210> 166
<211> 784
<212> DNA
<213> Homo sapiens

<220>
<221> misc_feature
<222> (1)...(784)
<223> n = a, t, c or g

<220>
<223> nbla-10010-f

<400> 166
gggnnnggtt gngnnnnnnn tttggaattc ctttagcact gttngcctac tgtnaaaaaa 60
anattggaaa atatttaaag gagagtaaaa ggaataaaca cagaattata atagattgat 120
aagatatacc ctccaaagta caagatttct ataattttaa tccatgggta agagaaatgt 180
ctaactaaat tttgtgtgtg tgtgtgtgtg tgtgtgtgtg tgtgtgtgtg tgtacatgcg 240
cgaatgtttc caatcagatc ctcgggaata aacaaggaga agaattaaat atcagtactg 300
gacccttgat ctttcctttc accttaggct gcctttagat tccctttttgg agctatgaat 360
atacccctta gttacaggtg tacattgcca atgtatgtat cttagcctgg gatagctgaa 420
gtgatttgtc tagttatttg aattttagca tttgagagat tttttaagtg aacacctagc 480
ctttaacacc ataagaaaag ccttgtgaaa tgatagaagt atttttaatt tggacagttg 540
aggggaagat atcctaacag caattttttt ttaagtgttt ctgcttttat tcttctagaa 600
atttagaaac cgtttcaagt gaaatccact tttttgtgg ggcagaagac tatatataga 660
ttaatttggg catttttttc taacgtggag tcttcttatn cagaaaaatg gatgtcttcc 720
caattttttaa aaatanttca acttttattt taaatgcagg ggggtcatgt gcanatttct 780
taca 784

<210> 167
<211> 781
<212> DNA
<213> Homo sapiens

<220>
<221> misc_feature
<222> (1)...(781)
<223> n = a, t, c or g

<220>
<223> nbla-10010-r1

<400> 167
gnagngtnnn ttggtggcct tttttaannc cctttttttt tctgtgacgg agtctttctc 60
tgtcacccag gctggagtgc agtggtgcga tcttggctca ctgcacctct gcctcctggg 120
ttcaagcgat tctcttgcct cagcctcctg agtagctggt gcatgcgcca ccgcacgtgg 180
ctactttttt ttgtattttt agtagagatg gggttttgct atgttagcca ggctggtctc 240
aaactcctga cctcaagtga tccaccgaac ccagcctcag atgatattct taacagaaat 300
attaaaggaa ggtgtaagag aacatgtggt gacttgagtt gacaaaagaa catactggtg 360
aaaacgtggc accagagttc tgaagagatt ggcactccga gctgtgcatt aggaaatcat 420

gttgccatag aggtggtaac tgaagtcttg ggatttttaa gaacacgctt actgcctata 480
tagaaactga aaaggtaggt ggtggataaa tgtgtgtctg gagttgttta agaatgagtt 540
aacaaaaaga caacatgctt ctgttcaaga gtcaatattt cacaagtttt cagctcatgg 600
aaaagactga aataaataga aaaaggaatc tagaaagaag tactccttat tctcttgaat 660
actctttgaa gatgcagaaa atattctatc taagaacact cttaccctta atggatcctt 720
aaanctttaa tctatgctga ttagaaacac attacactgg aatcattgat ttgccttttt 780
t                                                                  781

<210> 168
<211> 1475
<212> DNA
<213> Homo sapiens

<220>
<221> misc_feature
<222> (1)...(1475)
<223> n = a, t, c or g

<220>
<223> nbla-10070

<400> 168
ttctaggcct gtacggaagt gttacttctg ctctaaaagc tgcggaattc ctcgagcact 60
gttggcctac tggtttcgct aaatgctata gctttcttca aataaaaatt agccagaaaa 120
ataccttaga aaagattttt tttttcattg tggattccag gagactttta agacatggaa 180
ttctcggtga ttatattcaa atggcagagc cacatttgct aacagtattt tctaatttta 240
tctttcttat aagaaaaaaa gggtaaaaat cggatctgaa atactgacag ggttccccaa 300
agttagatat taacacagct tttcatttct cttatgagaa ctttactgta aatatacctt 360
tatcatagaa atgagatatc caatacacat tacaaaatgt catatttaaa gatgacaaat 420
ctattcttaa cccagattct tcatccacag aagtttaatg actaaaataa cacaacagaa 480
tgaacaaatg ttttattggc atgttcattg ttgtaaacag catctggcat gaaaaagttt 540
taccaaaacc ttttttattg atttttataa attagatggt atgtcaaaat ttatgtagaa 600
ttgtgttcat tgagtacttt tatttagcaa agcaaaaact tgctgatact aataaactct 660
gcaatgaaat catgttgact gcatcagtct gtaccatgac ctgacctcat tagcaagttt 720
attcagaaca atgttaagac gtttcccaaa agttaaacta catgtaacta caaattgact 780
ctaaaaatta aaactggact tacaagcatt ctagaagatt caaacttgaa gtaaatcttc 840
aaaactagat gtacaaagag aaaaaaattg aagccgctga acagagaaac ctcattaaca 900
agggcaacga caatcctatg ggacctagca ctgaggaacc tcactgagga ttacatttac 960
atagattgct atacttctca atgtacacaa atgtacagaa tgaatgatac gtgaaaagaa 1020
taatcatggc agatagttga actgcaatta tgataactca acaggatcaa ttttatttac 1080
atgctgaata aatgaatgaa acatattctc tacatccata acttaagtac aatcagttat 1140

aattacaaca ctgcagcaat ctgagctgcc acctgctggg atcggtctaa gggaaacgtt 1200
ctgatgttcc agaataatgt tcttaatact ctgcaaaaaa tgaaacaaaa taaaaacaca 1260
acttcacaat tccttactgg gttacggctg gttccaagaa tttcttttct tcagctcctg 1320
tttttgctgc tttccaagag tcaacacacg ttggatttac ttctgaattg tcaatgaggt 1380
aatgtgtgca tcagtgaaat aaacagaaaa ttcattcaaa aaaaaaaaaa aaaaaaggcc 1440
acatgtgctc gagctgcagg tcgcggccgc tagac 1475

<210> 169
<211> 1557
<212> DNA
<213> Homo sapiens

<220>
<221> misc_feature
<222> (1)...(1557)
<223> n = a, t, c or g

<220>
<223> nbla-10071

<400> 169
gcctgtacgg aagtgttact tctgctctaa aagctgcgga attcctcgag cactgttggc 60
ctactggctt tcatgcagcg aggacaatag aattttactc tgggataccc gctgtcccaa 120
gccagcatca cagattggct gcagtgcgcc tggctacctt cctacctcgc tggcttggca 180
tcctcagcaa agtgaagtct ttgtctttgg tgatgagaat gggacagtct cccttgtgga 240
caccaagagt acaagctgtg tcctgagctc agctgtacac tcccagtgtg tcactgggct 300
ggtgttctcc ccacacagtg ttcccttcct ggcctctctc agtgaagact gctcacttgc 360
tgtgctggac tcaagccttt ctgagttgtt tagaagccaa gcccacagag actttgtgag 420
agatgcgact tggtccccgc tcaatcactc cctgcttacc acagtgggct gggaccatca 480
ggtcgtccac cacgttgtgc ccacagaacc tctcccagcc cctggacctg caagtgttac 540
tgagtagatt ggatttaaga caaaaagcaa gtcccccatg agtgtccact tctttgccct 600
gccctctcag cttgtgagac aacacaggag ccttctatag tatgttgata tgctagatct 660
gtgccgttaa taggcatcgt ctctcagcct gagggaggct ggattctggg ttcctgtagt 720
cacagggagg aaaagctttc ttaaaaatgg acatgtatgt gcgtgtgagt gtgtgtgtag 780
atttatagtt tttggtagtg gcaggaataa aaaaaatcca tcctacatct tccctaagca 840
ctgcctctct ctcacccccc aaaacaagtt gacgaaaggg ttttatgtag ctgtctatga 900
ggaattggcc gtgtctgggt gggttatggg atgtgggcat ccctgggttc ttggaagcag 960
ctcttatgct actcatagag atgggattga ctttattttt ttatagtgct taattcacca 1020
ttatgagaaa tgcttccagt cacaaaaatg cagcccagct cactctgagg aagaagcagg 1080
acttggtacg gttttacaca actccttacc attaaactga atcagaaatc catttctgg 1140
ctgaataaaa agtttggctt gcctgtgtaa tgcccactcc cttcccctg gctccctagt 1200

gatgggacat atatgagaga gaagtgtttt tctatcatag acaccatagg ggaaagtttg 1260
gggatgaagg agagcttaaa ggtgtttcaa ttaagttaga aaactgacac aggctgttga 1320
gaattctttg ccacttttcc caccccaaaa cagcatgggg cctgacatct tctgccctgg 1380
tcccctttct cttgatgtgg aaagtctgaa tgcagtattt atagacttct aaggttttaa 1440
aatccagtat caagaagaaa atcagaaata ccggttggtg aaataaagag tttaggcatt 1500
gttaaaaaa aaaaaaaaa aaaaaaaaa aaaaggccac atgtgctcga gctgcag 1557

<210> 170
<211> 1783
<212> DNA
<213> Homo sapiens

<220>
<221> misc_feature
<222> (1)...(1793)
<223> n = a, t, c or g

<220>
<223> nbla-10111

<400> 170
gcctgtacgg aagtgttact tctgctctaa aagctgcgga attcctcgag cactgttggc 60
ctactggtta ctatggatca gtcggcactc gggaacagca gcaaggaaaa aaagcaaatt 120
tcattcacat gttctgtgtt catacctctt ctctacctaa ttgttcattt aaatttcagc 180
cttattcctt gataagggat tttaccacat gaagtcatcc agtgacccta gctcttattg 240
tgaagttagt ggagtatact tagaaatgtt acaactttaa aatgttacaa aacattcatt 300
aaagctcata tttaaagtag agcatctagt ttgagaaata gaaatcaatt attaaagatg 360
tctttttct acccatttaa ctagttaaaa ccatgacatg taaatgtaga agtagaataa 420
tcatagaatt ccctaaaata tttctgttta ctaacatata ttgaccaagt acatcaagca 480
ggagagatct tccttcattc tgttatagtc cacatcattc taattttgct cagttgttat 540
taagagcata ttcctaaacc atacactttt gtttcaataa agtttatttt tgttgagatg 600
aataaaataa caaagttata agctgcataa gacaaaagtt caattgttca aaaaaaattt 660
actgggatag ctttctatta caggtattgt tagattatat tgtgctgata agattacttt 720
ctaaaaaatt tgtacttttc tgtaaattaa aagaatatgg agtcataaaa tggcaagtgt 780
tttaggatta gcctaaaatt ggacattgtc attgatttca aagaaggtat gaactagcag 840
tcttacagcc taattcttct ttggactggt ccttggcagc agttcctttt cagactcgat 900
aaacagaatt cagatgatgt aagtcaaaac aaaactttac aaagccaagc gtattatctt 960
ttgcattaac ctattttttt ccatcataca tgctactagt atgtgcatta gcatgatatt 1020
ctcatataca ttgcattaaa aattaaaagg tggcagctca gggtgagctc ttctgttgct 1080
catttgttcc taaattttta agggcttttt ctcagtcaat agtttgtaca aactggttag 1140
tttaacttca ttacccattt cattaaagtt gatgggtcgt gtgatgagat gcatttaagg 1200

ccgatagtga tagatgtttt ttttatttct tgaacacagg ctttgtctga atgatgttct 1260
tttatctctt gaacacaagc tttgaatgat aactacaggt tttaagtgct gttacattaa 1320
taccataatg tgatgtgtta gaaacaaagg gatatttcaa aggtagatat ttgaaaattc 1380
tctagtctca atatgtatgt gtattgaata tactctaaaa ataaatgtgc aatttgctag 1440
taggacaatg cagtgactga ctagcattag gtatgtttct tttatatcct ggctatgtcc 1500
cactttcttc taagtgcaat cctttcatgt tcacttgctg ttttacccca tctactctaa 1560
cttcatttgg aaggcttgtc tagagtatag catgtatttt tacctttgca gtgaattgca 1620
tgtgctaatt gtaaccacag ctatttttat gttgacataa ctccaaatgt tatattaaat 1680
gttctattat atattagctc taatccctta agtaaatttt aagaaataaa tacttgttca 1740
aaaaaaaaaa aaaaaaaaaa ggccacatgt gctcgagctg cag 1783

<210> 171
<211> 2121
<212> DNA
<213> Homo sapiens

<220>
<221> misc_feature
<222> (1)...(2121)
<223> n = a, t, c or g

<220>
<223> nbla-10120

<400> 171
   ttctaggcct gtacggaagt gttacttctg ctctaaaagc tgcggaattc ctcgagcact 60
   gttggcctac tgggttactc agcgtagatg tgtgttcaca caaattgctc tgcattcagt 120
   gttcattgtg aattgggagt gtgagtcttt ctgtagggta gaaagaagcc tcctacccag 180
   caaaccagta gacccaaaag ttgaaaaaaa ctgatgacag acaacaagca tgaagatggc 240
   atatttgatg tcactttggt tctttttccc agaaggctta tacagtgact cagtcgggaa 300
   gctttccagc ttccagccct tgaatgtgaa gtgtcattgg catgtctggc agtagtctct 360
   cattcactcc tcaataaaca acattgaata caaaagaggc ttgtgtaaaa actcagtact 420
   gtctggcttg gattcatttc atgtttttaa tataagaatg atctaatatt tttttaaagt 480
   aatagctatc agtaatagct gagtgttttt tcccctaata ttttccttgt gcaattcaga 540
   cttaagcatc gagtttttac catcttccac tttaagctaa gttatgatac ctattccatt 600
   cacaattggt gttcttttta aggtttgcaa atttcagcca attttgtagc taagattgtt 660
   ctgatcagct caaaaagatt tggcttagtg ttttcattgc aaattataat tgctgtagag 720
   ccacacacaa cttttgaact tttaattata agtgttatgg ctaaagttat ttactgaaaa 780
   tttcagtaaa atgtgtgaat gtttctttat gtattaacct catagcagta aatgacttgc 840
   tgttgtttaa tttttctaag gcatcttaat agacttctgt tgaaaacttc agtgttaaca 900
   tttttatagt ttgtactaaa tttaaccgtg atataaaaat gaattttatg catagatcag 960
   aattttaaat taaaggtttt ttctttaaat gatttgtatt actttattaa aactaaatct 1020
   gaaatggaat agaaaataga atggattaca tacagatggt ttccttgtta gcagatgccc 1080
   ttcaaatata ttttacgttt gcagcacaac accattgccc ggaggcttcc gaatctagca 1140
   aagcagcata ttaaatgact ccacctgcgc agtccttgtg ttcagtggat gaatacctgt 1200
   gggctcttgt aatgtgtggt aatatttgga gctttatagc ctgtattcct tgggagaaaa 1260
   aaatgggaat tggagtaaaa agtgtgtcaa gacaaccact ttctgctttc ttatgtagca 1320
   tctgcaaagc cgattcatgt actgatgcca atcagctaga gcaatgtagg ctttttttaa 1380
   tttaaattat tactacactt tattactaca cttgcagaaa agaaacatgt taaaatcatg 1440
   gcacacctgc agaatttcag atgacagtgt ggtcagaaga tgttttttgg gagaacctag 1500
   tgtttgctgg gcactatgct aagcactctg ggagcaagaa cctcctacct tccagtttaa 1560
   tgataaaagc agaaaagcaa agaggtaatt ggagttgaat caagttaata gggaaggttg 1620
   gtagtaaatg ctgttagtgt gacatgattc tgaaacccag agctcagaac taactgtgag 1680
   atgtaccatg aactaacaat tctttgaaag gacctgcaga agcattttta atacctcatt 1740
   ctgtctatgc aagatgaaaa tccatggagt tttttcttgg cttttgtacc aaatttaggg 1800
   gtcttgcatg tcagcaagtg atatatattg tttataaatg caagctattt ctggagggga 1860
   aaaatgtgtg tggctcttac gttttctggg aattttgtaa caagttacac gcacaagtgt 1920
   tagaaacttt ggcataactc aatttggagt attttttaaa tgctgtatct ttaaagaaaa 1980
   gtaaaattgt cttaaatcta attgacactt ctgtaatgag aggcaacctt ataactctgg 2040
   agttgtagtt tgacagaacc atcagtaaag acataagcaa ataaaaaaaa aaaaaaaagg 2100
   ccacatgtgc tcgagctgca g 2121

<210> 172
<211> 801
<212> DNA
<213> Homo sapiens

<220>
<221> misc_feature
<222> (1)...(801)
<223> n = a, t, c or g

<220>
<223> nbla-10130-f

<400> 172
nnngnngnnn nagnnngtgg gggggggttn nnatntgant cctttgcact gttggcctac 60
tggcgtcttc agcaacctgg acatggaggc cgcactgcag ccactgggcc aggccaagag 120
cttagaggac cttcgtgccc ccaaagacct gagggagcag ccagggacct ttgactatca 180
gaggctggat ctgggcggga gtgagaggag ccgcggggtg acagtggcct tgaagcttac 240
ccacccgtac aacaagctct ggagcctggg ccaggacgac atggccatcc ccagcaagcc 300
cccagctgcc tcccctgaga agccctcggc cctgctcggg aactccctgg ccctgcctcg 360

aaggccccag aaccgggaca gcatcctgaa ccccagtgac aaggaggagg tgcccacccc 420
tactctgggc agcatcacca tcccccggcc ccaaggcagg aagaccccag agctgggcat 480
cgtgcctnca ccgcccattc cccgccggcc aagctccagg ctgccggcgc cgacttggtg 540
acgtctcana gcggctgcag acggatcggg acaggcgaac ttgcctgagt ncaaggcttc 600
tgcctggtgt tgtccccaag gcccactgaa ctgttcaagc cgntcaaccc ttgggcccgg 660
ggcttgangc acgaacagtg acgccttgtt ggcctctgga ccggtnagac aanctggtca 720
ggcagaacct tccgtaaggc ccgcaacccg atgtngcanc ccattaaccc ccaattaant 780
tccccttgnn gggaaccacc c 801

<210> 173
<211> 800
<212> DNA
<213> Homo sapiens

<220>
<221> misc_feature
<222> (1)...(800)
<223> n = a, t, c or g

<220>
<223> nbla-10130-rl

<400> 173
gagggngntt nnnttgtggc tttnnnnntt gntttctttt tttttttctt ccccaagatg 60
attttattga atgcacacaa agttcatcct tgggtttgca aaaagtccca caagtgaaga 120
ggcagcagtg ctcatgtgaa catggagcgc tcaccccagc ccctcagcac agccaggggg 180
ccttggggta cacaccctcc ttccctgggg ccgccagcac ctcctctgcc ctatcccgga 240
tggggcctgg gggtctgccc agggtgcgaa actggaatct atgctgaaac acctaagtgc 300
ccaggaggtg cccccatggc ccaggagtga cacggntccc ccagcagcca gagcccattc 360
ctgagccaga caggtcacgg ttgacccagg aagagccatg tgccaggatg gccgccaagc 420
ctactgagca tgtgcagcag tggcagcctn tagacataga gggggctccc tgggtgacat 480
nttcagagac ccccttgtcc ccagacaccc ctgggtagac tgngtctgac ctttacaaat 540
nggaaatgag aacttgggtc gaaaagntta caatttnctg ggngncttaa aagggtggtt 600
tcttaaaagg gcnggccata ctttaactgg gttatgggaa tgaatcnntt naagaattat 660
caaantaatt agggaaaaag ggaaaagaaa gatnttaacc nttaatctaa ctttggngac 720
cttggcctaa gaaattctgg gctggcctgg aactttggca agttggggag gacttgggtt 780
ttaaccttct tctngnttcc 800

<210> 174
<211> 1698

<212> DNA
<213> Homo sapiens

<220>
<221> misc_feature
<222> (1)...(1698)
<223> n = a, t, c or g

<220>
<223> nbla-10143

<400> 174
gcctgtacgg aagtgttact tctgctctaa aagctgcgga attcctcgag cactgttggc 60
ctactggagt cgtttatgct ccactttcaa agaagcagga gatcttttat acagccattg 120
tgaaccgtac aattgcaaac atgtttggat ccagtgagaa agaaacaatt gagttaagtc 180
ctactggtcg accaaaacga cgaactagaa aatcaataaa ttacagcaaa atagatgatt 240
tccctaatga attggaaaaa ctgatcagtc aaatacagcc agaggtggac cgagaaagag 300
ctgttgtgga agtgaatatc cctgtagaat ctgaagttaa tctgaagctg cagaatataa 360
tgatgctact tcgtaaatgt tgtaatcatc catatttgat tgaatatcct atagaccctg 420
ttacacaaga atttaagatc gatgaagaat tggtaacaaa ttctgggaag ttcttgattt 480
tggatcgaat gctgccagaa ctaaaaaaaa gaggtcacaa ggtgctgctt ttttcacaaa 540
tgacaagcat gttggacatt ttgatggatt actgccatct cagagatttc aacttcagca 600
ggcttgatgg gtccatgtct tactcagaga gagaaaaaaa catgcacagc ttcaacacgg 660
atccagaggt gtttatcttc ttagtgagta cacgagctgg tggcctgggc attaatctga 720
ctgcagcaga tacagttatc atttatgata gtgattggaa cccccagtcg gatcctcagg 780
cccaggatag atgtcataga attggtcaga caaagccagt tgttgtttat cgccttgtta 840
cagcaaatac tatcgatcag aaaattgtgg aaagagcagc tgctaaaagg aaactggaaa 900
agttgatcat ccataaaaat catttcaaag gtggtcagtc tggattaaat ctgtctaaga 960
atttcttaga tcctaaggaa ttaatggaat tattaaaatc tagagattat gaaagggaaa 1020
taaaaggatc aagagagaag gtcattagtg ataaagatct agagttgttg ttagatcgaa 1080
gtgatcttat tgatcaaatg aatgcttcag gaccaattaa agagaagatg ggatattcaa 1140
agatattaga aaattctgaa gattccagtc ctgaatgttt gttttaaagt ggagctcaag 1200
aatagctttt aaaagttctt atttacatct agtgatttcc ctgtattggg tttgaaatac 1260
tgattgtcca cttcaccttt tttattatat cagttgacat gtaactagta ccatgcgtac 1320
ttaaatagat ggtaattttc tgagccttac caagaacaaa gaagtatcca tattaagttt 1380
agattttcag ttaatttttg agactgagta gtattcttgg atacaggctg atgtgtactt 1440
aaccacttcc agatttatac agtcttcctg tggaagttta gtaaatgtct ttttccctcc 1500
tttcttctag taatgcagtt catgggcttt aggtacttca gttatgaagt aggcttttca 1560
tggggagaga ttgggattat gctttctgtt gtttaagaaa ctgtttgatt ttagagtcta 1620
tttctatgag atagtttacc aaataaatgt tccttataaa aaaaaaaaaa aaaaaaggcc 1680
acatgtgctc gagctgca 1698

<210> 175
<211> 793
<212> DNA
<213> Homo sapiens

<220>
<221> misc_feature
<222> (1)...(793)
<223> n = a, t, c or g

<220>
<223> nbla-10198-f

<400> 175
gnnnnngnnn nnnnntnggg ggcnttnnna tttgattcct ttgcactgtt ggcctactgg 60
tcattttgtt ttggacaccg agcaggagct ggcggccgct gcagacgaaa ggcaggaaag 120
ggcaggccgg gtgagcagac ggatcggccg actagacagc caaccagcaa caacgaactg 180
agctcgcata ctaccgctta cgcatctaac caaccgccca tctagctaac ccgagcccct 240
ccaccgtcaa ctcaggttcg gccggtcccc ggcccgcctg ccggagccgt ggtggcagcc 300
ccgggaggag cactggcgtc tgtttccttc gattctcggg attcgaagat ggctgcacag 360
tcagcgccga aagttgtgct aaaaagcacc accaagatgt ctctaatgga gcgctttact 420
aatatgctga agaacaaaca gccgacgcca gtgaatattc gggcttcgat gcagcaacaa 480
cagcagctag ccagtgccag aaacagaaga ctggccagca gatggagaat agaccctctg 540
tccaggcagc attaaaactt aagcagagct taaagcagcg cctgggtaag agtaacatcc 600
aggcacggtt aggccgaccc ataggggccc tggccagggg agcaatcgga ggacgangcc 660
taccataatn cagaaangct tgccananga ngactacgtg ggggacgtgc cancagaacc 720
ctacttaagg gccgggatgt catttcgagg tcaaaanctg ttccgaggtg gacnagccgt 780
acttcccgaa tgg 793

<210> 176
<211> 784
<212> DNA
<213> Homo sapiens

<220>
<221> misc_feature
<222> (1)...(784)
<223> n = a, t, c or g

<220>

<223> nbla-10198-r1

<400> 176
   gagnggggnt gnnttggnng ccttnnnttg acnncctnag cgaacagctt gttataatga 60
   accagaaata caagattcca ctgaaactga acagttgaca gaatatggtt gaacttaaac 120
   cttcaaggga aacaagggca aaacaaagct aatgagtgga aaagtccaag attagtttgg 180
   gataaacatg aggatataat tgcattttag catggctatc ttctgacctc ttccagcagt 240
   tcgtctgcca tcattctccc ttctgacaca cctaccaaat caaatggctt ctgatcctct 300
   atattgcagt ataaaccaac cctatagtac ccctctggtc atgatacaaa cccagaagga 360
   aagtctgtaa atctcataca aaggtagaaa gtgcaattca acccaacacg ctggttatgg 420
   tatatgtgct gtcaatgtac atggtagcaa cttgaatcaa accagagctg ctaccacatg 480
   taagcaacct cttatgctgc cctgtagtga tgtcaatgtt ctcagtttac taaattatca 540
   atgtggaaga acgcattttt gagctccaga ccttctaaac attttaaaca ggctatcgaa 600
   gaggatgtaa cagaacccta tttcagaaac ctaaggatca nggaggaang aacactgacc 660
   gggttttctg ggatggttct gtgggggcac agggatgaac aaaagaaatg gtancatgtc 720
   anaacatttg caagtgaagg ggagccgaag tnggaaaagg gngtctannc ccaanggcca 780
   tgct 784

<210> 177
<211> 1880
<212> DNA
<213> Homo sapiens

<220>
<221> misc_feature
<222> (1)...(1880)
<223> n = a, t, c or g

<220>
<223> nbla-10236

<400> 177
   gtacggaagt gttacttctg ctctaaaagc tgcggaattc ctcgagcact gttggcctac 60
   tggaagatgc acatgttgca aaggggaaga gtgttttcct tgagcaaatg aaaaagtttg 120
   tagaatggct caaaaatgct gaagaagaat ctgaatctga agctgaagaa ggtgactgaa 180
   ttttgaaact acaccctcag taaagcaaac aggagttgta gataaaatgt catgtctcat 240
   gtgtcctggt tcttacatct tcctacctcc ctgtatcaag catgatataa gggctttcat 300
   ggcaaatttt attttaactg tttctatggt tgctggaaat gttgggttta gtttctaaaa 360
   ccatgtttta agtagctaca ggagctatag atttgaatct aatgttgcat tagtcttttc 420
   agttatcttc tacctcctgt attttctact gtaataatgt aatttaaggc cttccacaat 480
   gaacagttca ctttattccc tgggttttct ataaacagtt ttaaggatat gatttggtta 540
   aaaaataatt tgttataaaa attctgtttg caaattaaac tggaaaagta tccagagtct 600
   caaaaggcaa tgatttgtga gataatatgg catgcccgga gccctgctca tcaatgaaaa 660
   acccatatgt aataatcgaa ttcatttaac atgaatcttg agtacgtgga ccattgcttg 720
   catgttaact ttttgttttg ttttgttttg ttttgttttg catttttaac tccagatatc 780
   ctaaagctca attgtttggt ctctggtttt catccttaga gaagccatgg agaacagact 840
   tgaaaagttt aggaaatcat aatgtggcag aggtggtggg aagaagaaag ttgagctttt 900
   tccccttgag aaacttctgc atttagtttc tatctttcca ggcaaaacaa atgggtattc 960
   ttttcataca accattttca aatgaacctt agaaaagtct taacatttaa ggtattttat 1020
   gcacagaata cacttagatt gataggaaag aactcgtaat ggagtttgag taaagaaaat 1080
   gactgatgta ctaaacccag taaaaattgt tgaaaatgtt aaaggtcagc atgttctaat 1140
   tgggaatcta gatatagctt agatttccta ttggcttaga gtatttgcta taacaaatga 1200
   agtgcaatga caattatata ttcctactcg gtcatactgg actggcttcg ttctcttaat 1260
   atactcagta atgactcaag cctctggcta ttaacatacc ctagttgccg ttttttaatt 1320
   gccatgagcc aaatacttct tggtatacaa ttgatccatt tattttaatg gctgcctttt 1380
   cattttcatc ttttcttgct gctacccatc tatgtatgta gtcattgggg ggaaaatgta 1440
   gccacatttt ttatgggaag actttgtgtt aaaagtgaac attttgaagg tttttaactg 1500
   gtgaaactag cctggaataa tgccaccaga gactgagtgg aaatcgcccc ttttgaaggt 1560
   gccattctta tgagccaaaa gtttgtcatt taaaagttca ttttgaggga ataacatgta 1620
   atataatttg aaataaaggt atagtaacct taaaaagaac attataactg attgttgtga 1680
   atggggtgaa tttgttaaaa tgagtaactt tgataaagtt tttcatgcac aggcaaaatg 1740
   tattcactag atttctacgt agtgatctgc ttttactttg taatttgtag ttctcaaaag 1800
   actttttttt aaaaaaataa agtccatact tacacttaaa aaaaaaaaaa aaaaaaaggc 1860
   cacatgtgct cgagctgcag 1880

<210> 178
<211> 2075
<212> DNA
<213> Homo sapiens

<220>
<221> misc_feature
<222> (1)...(2075)
<223> n = a, t, c or g

<220>
<223> nbla-10241

<400> 178
gtacggaagt gttacttctg ctctaaaagc tgcggaattc ctcgagcact gttggcctac 60
tgggatagtg ttaaatgaga tcactggatc ctgctaaaca tgttctagtg ctcttgcctt 120
ttgctgtatg tttggtgtca ttcatcagca gggaatggct gaaatgtgag gtgtaaattg 180

acagagtaga gagaacctat acagttttaa caccatagcc acagacatcc aggcatccta 240
acattttgtc ctagatcttc ctgaacctgg acggcaggat gagaacacta gtgattttat 300
tggaatgaag ccaagacaaa taaccttttt ttgtgctctt tataatatag ctctcaagaa 360
tgaacaaata aaaagaatgg ggtcaccata tatatcattg tttagcaagt gacaagtgac 420
acagtgagca atccttagtc agtggcatga caaggaaata ctcaagacag acactgacct 480
gtattgtgaa tgaggtgcct tggtgaagca aaggtttggg cccaatagtg agtcaaggag 540
ccatttcaaa gatgggctac agtcactcat taaaagggat agaacacaac tgtgctgatc 600
gtagaattaa ctgtgacttg tccataaaat agaggaggga tgcaatctgt tttatagaag 660
gcatatcttg cagggccttg tagatttgtt aaggatagat gatagtcagc aggctgaaat 720
tttctctcta gtggaccact ttgacacatt ctcaaggttt tatttgtata cattaccaca 780
agacatggac ttcagttaca atggcagcaa atataataca tgctactaac tatgaatttt 840
taggtaagat gaaatgcttc ttaaaagatt gttggtcaga aatctggaga ttggtacttt 900
ttctcagaaa gaaaatcagc tcaacctaat ggctgtatac tgcattgtaa catttatttt 960
tatttaaaag ttggattttt atgatttaaa aaagtaatgt gagtcttcat ttaaaagttt 1020
aaatggcata aagacagcaa gttttcatat agtagtaaaa ttgtatgaat tttagacaat 1080
gattgatggc ctctcttgac tgtatcttaa acaaaaggac ttactgcaat tttttcatta 1140
tcagtgttat gcaaatatac cagtaaacga ctgctttctg agatcttagg gaagtaatga 1200
ttaatgaagt ataaaaatgt aattgtaatt atatcaccta aattatgcca aagagataca 1260
ttggtgacat acttatgcta aaaagagaga gagaaaaaaa gactccctct ctccccaaat 1320
cttacaacac attaaatctg tcttctgttg catgttgaat ataacagtat caggaactag 1380
atgaaagttg gttattcatt tatgatgatg atgaggctgc tgaagcagtt ggtatagttt 1440
tgtgcattac agagaatgtt gtatgccagt taacaaatag acagccacta tgtgcccagc 1500
acagagagaa atgagtcatg gtttgtgacc tccatggatc ctaacatttc ttctcattaa 1560
ttccttcact tggtatttac tgaatgccag tatgtaccaa atattgagca tgacactggg 1620
aataagggtg tccagaagac aaaatctttt tcttgatgga gttgacggtt taatggagtc 1680
atggcataag cttccgaaac agttgaagga tgagtaatac acagaaaatt gtcaggagaa 1740
aattatgtgg ggtataacca ttgcactaga ttgttttgta gaggagacag attggcactg 1800
aactttgtgg ctaatatttt agaccatgag ataggaaatg tattcaaaag gctgaaaatt 1860
acatgggaga gagtaaatat gatcagagat ttaacccata ggctgttacc tggagaatgc 1920
ttgggtatac attgacttat aactaagaaa gatgaatttc aattttctgc ttaggtatgc 1980
tgaagaaaaa ttaaaagtgt aactccagag aagtaagttc agaaagttag caaaaaaaaa 2040
aaaaaaaaaa aaggccacat gtgctcgagc tgcag 2075

<210> 179
<211> 1754
<212> DNA
<213> Homo sapiens

<220>
<221> misc_feature
<222> (1)...(1754)

<223> n = a, t, c or g

<220>
<223> nbla-10242

<400> 179
gcctgtacgg aagtgttact tctgctctaa aagctgcgga attcctcgag cactgtggcc 60
tttttttttt tttttttttt ttagacaagg tctcacttta tcacccagct ggagtgcagt 120
ggtgcaatca tgacttgcta cagcctcgac ccccaagggc tcaggtggat cctcccatct 180
cagcctcccc agtagctggg accataggcg tgccccacca cacctggcta atttttgtat 240
tttttgtaga gacagggttt caccatgttg cccagaccgg tcttgaactc ctagactcaa 300
gcaatcctcc tggctcagcc tcccaaagtg ctgggattac aggcgtgagt caccacaccc 360
acccaagaat tttaatttat gtggactacg cttacacctt gctgcccttt gatttcttc 420
caaacagcaa tgtttaatca ttttcttgct ctcataggac taggttaact taaactgact 480
cattcactga tacagggcct acagcaggaa gcccaggacc tcccacccct caaagccttt 540
ttctggcaag actgtccaaa gcccctttgt cacgaactca ccttccaggc atggtatgtg 600
cccgaggggt cagtctgata gagcctagga gtgccatcaa agtcgaaacc cacgatgagg 660
gcagagatgc caaacggcct gcgcccattg ctctgcgtat aacgctagca agaaaagaaa 720
caggtcagtg ccatccaatt aagaaactgc atgtgaggaa agaaccgtac ttagtgaaga 780
tacgaacatt ttaaaccatg cctgagcccc ttctcaggtt atcagcagtt caaatgatac 840
attagccaat agctttcaac atccttttct gcattttcac tcctagaaaa actcacctag 900
gaccagaatt gtgaactcag gttactggaa attactttga caggtagaca cacattctgc 960
ttacatacta atggcatgtc ctcatagggt ggcttccagc cccttcaac tgctttgtga 1020
ctggtcactc aggatggcaa gacaaaagct atcatttccc tccagacaca ggaatttgga 1080
atatcccatg ccaggaactg agaacagaac aatgttactg aaattggtga gctaggtcaa 1140
gttacacttg tgcaagtcaa gattaggatc acgccccaga ataaatcagt tttctgattc 1200
acctgaagtg acatggcagg accctccatg gcggagccgt gactgccctg ctgccagagg 1260
tgagcatgca agcgggcagg cacccacctg cttcagactg gcgatgtagc gggtgatgta 1320
ctccacagtg accgggtcct ccacagtcag ccggtggctc tggcactcca cccgggccct 1380
gttgatgact atccttgcat cggcggtgag gcctgcaagg aaagcagaga ggcttctgct 1440
agagagacag cacaaactac agggtgggga cagacacgat gacccagcat ttaaccttcc 1500
acagaccccc caaaccggca gcaaggctat tctaactgac tggcagcgga acctatcagc 1560
ccctgggaac ttccaggaaa ttgggtacag atgcttcttg atgtgcagtg gggtggtatc 1620
ccgctaaact catcgtaagt caaaaatacc tggctgaaaa tgcatttact accccataaa 1680
cccattgtaa ataaaaaatt gtaggctgcc gtgcaaaaaa aaaaaaaaaa aggccacatg 1740
tgctcgagct gcag 1754

<210> 180
<211> 1668
<212> DNA
<213> Homo sapiens

<220>
<221> misc_feature
<222> (1)...(1668)
<223> n = a, t, c or g

<220>
<223> nbla-10283

<400> 180
gtacggaagt gttacttctg ctctaaaagc tgcggaattc ctcgagcact gttggcctac 60
tggctgtgtg aatcctgtac accctgggtt ggctgcagtt ttcctttcaa tctcgtggat 120
gctgtgtcct tcttacttgg tttttcccag gcagcccttt cctgtagagc cagtatgagg 180
tagccaggtt ctcagctgtt tcgagtgctt tgccttctct ggttatctca agcatcagga 240
aaattatatg ttgcatacat tccacagagg tcccatggtg atggggaaag gtcagctcag 300
gcattccctc tgcttagcca cacttcatca tactgcttgg atacatgttg ctgcctagga 360
gactttctta acccaaacag gccccactgc ctttagcagt ttcttcatct tctctgacat 420
acaattcagc tttggcccag agtggagatt aagatatggg tctgactctg cctctctttt 480
ccctgtgtga ctgctccagt tgctcctaac tgaatgccct gtagctccca agcttgcagg 540
ctctgccatg ggatgcctgg gaaagtaagg cacttgtact cttccatttt tgtctggatg 600
ataccagtgt ctgcctcttc tcccattatt catgtaaatg caagagaaaa aaagcacttt 660
aataattttc aaaaatttct catattacat tctctattgt gtctggctct gaagttaggt 720
ttttgtgttt ttttcccaca attattccta ctgtatgtcc ttgctgcaat ccaagtgctg 780
accttatttt tttagagctc gttctttcta aagttaaact ttactatgca tagaagtaat 840
tttgaatctt tcaaaatgca gattctcatt gagtaggtct ggggtggggc ctgagattct 900
gcatttctca taggctcccg gggtgcttgt attgctgagc cacagtccag tccatctcag 960
agtcctagag gaggtaatct tctctggttc ttttccattt atccatttca taagcatttc 1020
tcacatctta tctggccttt gccacattca tgaaacctat cactttatca gtaaggtatt 1080
taaaacactg ccttgcacag agtaaacatt catcatgttc tagatattca catgtacatg 1140
aacacacaca tatctatcac acttaatatt attgctgttt tatagttaaa cttcttctaa 1200
tgtcaatcta gaaacttgtt ttagtagtgc attttgagag aaaccatttg gtatcatctt 1260
gcatggaaac atccgcttcc ttaggaatga ccaaaaacta taattagtga taatgaatct 1320
tttatgattt ggcaagagtg ttttaagtgg agttcttttg atgatacgat tgttcaggtc 1380
aggctgaagg agccccaata ataatgtttt tctgactgtt ttcctctggt aatgtttgtg 1440
ataaaattcc tttgtcataa ataaatattg ttgctggtgc agactgaata tttgtattcc 1500
cccaaaattc acatgtcgaa accctaaccc ccaatgtgat agggtttgga gatggggctt 1560
ttgggaagta attaaggtta gattaggtaa tgagggctgg tccttcatga tgagattggt 1620
atccttatta aaaaaaaaaa aaaaaggcca catgtgctcg agctgcag 1668

<210> 181
<211> 2161

<212> DNA
<213> Homo sapiens

<220>
<221> misc_feature
<222> (1)...(181)
<223> n = a, t, c or g

<220>
<223> nbla-10300

<400> 181
gtacggaagt gttacttctg ctctaaaagc tgcggaattc ctcgagcact gttggcctac 60
tggccatatt gctgctctca ttgcagagta tctgaaaaga aagggttact ggaaagtgga 120
aaagatttgc acagcatccc tgctctcgga ggatacccac ccctgtgata gcaactcatt 180
actaacaact cccagtggag gaagcatgtt ctctatggga tggccagctt ttttgagcat 240
tacaccaaac attaaggaag aaggagcgat gaaagaggat tctggaatgc aagatacacc 300
atacaatgag aatatcctgg tggagcagct atacatgtgt gtggagtttc tctggaagtc 360
tgagcgatat gaactcattg ctgatgtcaa caagcccatc attgctgtct ttgagaaaca 420
acgagacttc aaaaaattgt cagatctcta ctacgacatt catcggtcat atctgaaagt 480
ggcagaggtg gtgaattcgg agaagcggct gtttggtcgc tactatcgtg tggcatttta 540
tgggcagggc tttttgaag aagaagaagg taaagagtat atttataaag agcctaagct 600
gacaggtctg tccgagattt cccaaagatt actcaagctc tatgcagata aatttggagc 660
agacaatgtg aagataatcc aggattccaa caaggtaaac cccaaggatt tggaccccaa 720
atatgcctac atccaggtga cctatgtgac gccgttcttt gaggaaaagg aaatcgaaga 780
ccggaagaca gatttcgaaa tgcaccacaa catcaaccgc tttgtcttcg agacaccctt 840
cacgctgtcg ggcaagaagc acggtggggt ggcggagcag tgcaagcggc ggacgatcct 900
gacaacgagt cacctgttcc cctacgtgaa gaagagaata caagtaatta gccaatcgag 960
cacagaactg aatccaattg aagtggcaat tgacgagatg tccaagaagg tttctgagct 1020
taatcagctt tgcacaatgg aagaagtgga catgatcaga ctgcagctca aactgcaagg 1080
aagtgtcagc gtgaaggtta atgctgggcc aatggcctat gcacgagctt ttcttgaaga 1140
aaccaatgca aagaagtacc ctgacaacca agtaaagctt ttgaaggaga tcttcaggca 1200
atttgcagat gcatgtgggc aggcccttga cgtgaatgag cgcctcatca aagaggacca 1260
gctggagtac caggaagaac tgaggtccca ctacaaggac atgctcagcg aactctccac 1320
agtcatgaat gagcagatta cgggcaggga cgacctgtca aagcgcggag tggaccaaac 1380
ctgcactcga gtaattagca aagcaactcc ggccctaccc acggtctcca tctcatctag 1440
tgctgaagtc tgaggactct gcagcatcag acccacctct aagagaactt tctgaatttg 1500
cagctaatct cggggaagag aaagataggt ttaatttatt tgaagttttc atggtgttaa 1560
tatttttgtt tacctcgcta gcttcagaat tttgccaacc tctgaatttg cacattttgt 1620
ataatttttt ttttctttga gcagtgttga tcaagccaag ttgaatattt gccatgaaat 1680
tccagtgaat gtgtagctca aatgcaaacc ctaagtttgc tgtcagttat tgtatggtca 1740
gtaccccagt cctagtacac atattttaaa ggttaaagtg aatgtttttg taacatttaa 1800

gcatatttca gatgtaaata aaagattgta aaatatacgg tttttaccaa atttaaaaga 1860
tcctttttag ttaatactat gacagtacta aaaatatatg aataacattt cagataccat 1920
tatattaaaa tatttgtgta tgtgtacaaa agcgttgata aatactaatc tttaaagttt 1980
gtggagttcc tttatttgta atatatgtgc tcttaaaagc aatgggatgt gaaattatga 2040
aagtatttta ttgttcatag aaataaaaaa cacagttact ttgcaaaaaa aaaaaaaaaa 2100
aaaaaaaaaa aaaaaaaaaa aaaaaaaaaa aaaaaaaagg ccacatgtgc tcgagctgca 2160
g 2161

<210> 182
<211> 777
<212> DNA
<213> Homo sapiens

<220>
<221> misc_feature
<222> (1)...(777)
<223> n = a, t, c or g

<220>
<223> nbla-10313-f

<400> 182
gngnnngtgg ggnnnnnnnt ttggantcct cnagcctgtt ggcctactgg agatgtttgg 60
ataaatacta aaaaagcaaa aaacaaaaac aaagattaac agtgcatgtc agagatagct 120
gaggagagaa cagctgaaga atttagttag atagaaagcc aagttatggg cctataaagc 180
tggaattcaa gatatggaaa ccagaagggc agaacaatga tgcagaactg tataacaaaa 240
taaggcattg gagtctttct tgtattctct ttgtatctta gatgcctcag cctccaatgc 300
tgcttgctgt tacaacatag aaaaacattg gagtgatttt agagatgatt tttttgtgaa 360
tatgtcagta aagtttgtgt tgactgttgg taacattgtt catctactga agcactctaa 420
taataagaaa agtgaattgg agcttgaaaa aattgctgtg acattggaag aagagagaat 480
gtaaatgaag gggttaattc ttgctgccac tttcttctgg tgttgctgat gcctnaatca 540
ttcactcaag aaaatggtta ctaaagtacc tcttatggtt tggacattaa actgggtaca 600
aaaggatgta gaagttgnac ttgcanancc gcgccngaag aaaccacgca ctgactttgg 660
cttctcggac gactgtacag aagtcttcaa agccttctnt tttctgngca aaaatggcaa 720
ctcttaangg aaaactcatt ggaccanttg cgggaagaag aagcaacaag ttccaac 777

<210> 183
<211> 788
<212> DNA
<213> Homo sapiens

<220>
<221> misc_feature
<222> (1)...(788)
<223> n = a, t, c or g

<220>
<223> nbla-10313-r1

<400> 183
gntgngggtt nnatttgang gccctttgtt ngnnnccntt ttacactaca atagttaatt 60
ttatttgttc aagagctcag attgcaagca ttaaaccaag cataggcttt gattctgtga 120
gcccaaattc acatattgaa gaagatcaaa gcaaactgtg atccatgtac atggatgaaa 180
actaaaggct cgagttaatc acattgtagt ttttaaattt ctacagccta gagctcacta 240
gtcacaggtc ttttaggtcc ttctggatgt cccacagggt atctgcactt ttcttgagct 300
gagcaacctc atcatccttt agcttctggt tgataacgct ggttaatccc cgggcattga 360
ggatacatgg aaggctcagg aagacttcat tctcaatgcc atacatcccc tttaccattg 420
ttgacacggg atgaatcctg gatagatttt tcaacatgga ttcaataaga tcagccacac 480
ttaatccaat agcccagttg gtatatcctt ttaacttgat gacttcatag gcactttcaa 540
ccaccatctt atgcacttnc tttcaatttt cactatcatt ggcaggtccc atttctggat 600
tcaattcctg gagagaaaca cctggcacat tcacaccant tcaaacaggc acactttgag 660
tcggcatgtt ccccaaaatc catncatggn aagcttnttg ggatgaatgg ccaagttttt 720
naagccntaa ggaacgnaaa tntagcngaa tccanattac attcactttc aatacgccgg 780
gggttggg 788

<210> 184
<211> 2324
<212> DNA
<213> Homo sapiens

<220>
<221> misc_feature
<222> (1)...(2324)
<223> n = a, t, c or g

<220>
<223> nbla-10314

<400> 184
   ctgtacggaa gtgttacttc tgctctaaaa gctgcggaat tcctcgagca ctgttggcct 60
   actgggatat tcaaaaatcc atggctgcct tcaaaaacct ttcagcccaa tgtcaagaag 120
   tccggaagag gaaagagctt gagctgcaga tcctgacgga agccatccgg aactgggagg 180
   gcgatgacat taaaactctg ggcaacgtca cttacatgtc ccaggtcctg attcagtgtg 240
   ccggaagtga ggaaaagaat gaaagatatc ttctactctt cccaaatgtt ttgctaatgt 300
   tgtctgccag tcctaggatg agtggcttta tctatcaggg aaagcttcca acgacaggaa 360
   tgacaatcac aaagcttgag gacagtgaaa atcatagaaa tgcatttgaa atatcaggga 420
   gcatgattga gcggatatta gtgtcgtgca acaaccagca ggatctgcag gaatgggtgg 480
   agcacctaca gaagcaaacg aaggtcacgt ctgtgggaaa ccccaccata aagcctcatt 540
   cagtgccatc tcataccctc ccctcccacc cggtcactcc gtccagcaag cacgcagaca 600
   gcaagcccgc gccgctgacg cccgcctacc acacgctgcc ccacccctcc caccacggca 660
   ccccgcacac caccatcaac tggggacccc tggagcctcc gaaaacaccc aagccctgga 720
   gcctgagctg cctgcggccc gcgcctcccc tccggccctc agctgctctc tgctacaagg 780
   aggatcttag taagagccct aagaccatga aaaagctgct gcccaagcgc aaacctgaac 840
   ggaagccttc agatgaggag ttcgcgtccc ggaaaagcac agctgctttg gaagaagatg 900
   ctcagattct gaaagtcatt gaagcttact gcaccagcgc caaaacaagg caaacactca 960
   attcaacatg gcaaggcact gacctgatgc ataatcacgt cttggctgat gatgaccaac 1020
   caagcctaga ctccctgggg cgtcgcagta gcctttctcg tttggagcct tcagacctct 1080
   cggaagactc tgactatgac agtatatgga cagcccatag ttacagaatg ggttctacat 1140
   ctcgtaagag ctgttgctca tatatctctc accagaacta atgcacttct gagcttttct 1200
   taacaaatgc ttgttgtatc gcagcctgct tttcttagat gttttcttgc tgttccttgt 1260
   ctcttttatg tgatatttta acaacttttg agagtgttcc tgatatttcc cattgtactt 1320
   tgtggaggct taactgccga tgcgagtata cattcgaatg actgactgtt ggagggaggt 1380
   ggtgtgttca cgtgagccta catctgaggg tgctcttcgt aaggggtagt tgttgtttta 1440
   tagctccaaa tggttttttt attctcagtg tagcaactgt ttgatttctt tctggataca 1500
   gctgtatgaa cacttttcta ttttatactg aaatcacaca ggtttgtgat ctaagtgcca 1560
   gaaccatgga ggagactctg gccttatctg tagggaccta aattttttaa gtgctatgaa 1620
   cactgatcaa gtagttgtgg agagatgaga agaatatatg cagcaaaggt gctgtgagcc 1680
   cagttccaac ggctgtgttc tggtaatcgt gcagaacagc atgtgggctc tgggccttgg 1740
   caggtgcaca gacgggcggg cgtcacgtgt gttcagagct tgtctccacc tcacggctct 1800
   gtgctcttct gtgtgctgcg ttgtggccgt gaagtgggag ctctcctttc caaaggcaga 1860
   gcttactgtc atggctcaga tgttcactcg catcttcacc ccaaacttcc ctgaattgca 1920
   gtgatttctg ttataaggca catagcaagc ctgtaccact ctacagtaaa atctctacac 1980
   taacagttta aagcccgaaa gctgaagcat tcagcctctg caagtgtaga ctactggact 2040
   gtagtagagg catttggaat acttacaaag tcaagtgctg tggcttcagt tcccccggct 2100
   ccccacccct gacatccata cttatgttgt ctggaagagc catggacaca tcattatttt 2160
   cagcaaccag ctgcagtgaa actccattta cagcaacccc atagtataaa accacaatgc 2220
   ctcattgcaa tgggatattg aagttcattg aagaattact aggttgccat gtggctcact 2280
   tatttaaaaa aaaaaaaaaa aaggccacat gtgctcgagc tgca 2324

<210> 185
<211> 783
<212> DNA

<213> Homo sapiens

<220>
<221> misc_feature
<222> (1)...(783)
<223> n = a, t, c or g

<220>
<223> nbla-10327-f

<400> 185
gnggnnnnng ttgggntcnn nttganncct ttgcactgtt ggcctactgg ttacgatgca 60
gctgatgaag agggagaaag gggtgttggt ggctttgccc aaatccaaat ggatgaatgt 120
agaccatccc attcatctag gagatgaaca gcacagccag tactccaagt tgctgctggc 180
ctctaaggag caggtgctgc accgggtagt tctggaggag aaagtagcac tagagcagca 240
gctggcagag gagaagcaca ctcccgagtc ctgctttatt gaggcagcta tccaggagct 300
caagactcgg gaagaggctc tgtcgggatt ggccggaagc agaagggagg tcactggtgt 360
tgtggctgct ctggaacaac tggtgctgat ggctcccttg gcgaaggagt ctgtttttca 420
acccaggaag agtttgctgc aacagggtgt gctggagtat ctgtctgcct tcgatgaaga 480
aaccacggaa gtttgntctc tggacactcc ttctagacct cttgctctcc tctggtagaa 540
gaggangaag cagtgtctga accaaacctg angggttgcc agagcctgtg atgacttgga 600
gntagcagat gacaatctta aagaagggga ccantttgca ctgagtccag ccagcaggaa 660
cccatnaacc aagtcaagct ttacacgcct taacaanttt tccttggtac tacttttacc 720
aaaccggaan atggacagnn tatggtcctg nanccctggg aatgnnccct tgctcnnncc 780
gga 783

<210> 186
<211> 775
<212> DNA
<213> Homo sapiens

<220>
<221> misc_feature
<222> (1)...(775)
<223> n = a, t, c or g

<220>
<223> nbla-10327-r1

<400> 186
   gnnngnngnt tnnnttgntg ccctttttnn nnnccntttc ctcctttctc ttcagagagg 60
   ggatctgaag tagctggtgt gtccagtttc atgaaggctg cttcaatagc ttggctgaag 120
   gaattttgaa aactgggcac aggaacacgg tctgaattat cactctcccc gtcgctgtcc 180
   acaggggcag gaggaactaa gctgttctca tctttctttg gagcagtttt gggccacaca 240
   tctgcttttg cttttccaac cctcagcatc tgggcaaagg aagggaaggg agagtcttct 300
   tccagactcc caacgcagaa tgagggactg ccctgactgg cagtgggtga cagaggggtc 360
   agcagaaagt ctgcatggga acctggactt ctgctgagag gagaaatgga gagggcccca 420
   tggccctcgg tgctggtggg acccaaagca gaatcagagg agcaggtata agaattgaag 480
   gcaggaaact gctgtagatt ctcgagggga atgtggactt ctgggtactt gccctgtttc 540
   ttgtctcctc tatctcaatc ctgcgctctc ggcggccgtt cctccgagcc ttcttttggc 600
   gctgaccgtt tcccttntca atgncatctg agaacatctc tangggttcc ttaaagacca 660
   caggaggttg caagccngnt cacagatctg aactcacagg tgaatgcaag tgaaagagat 720
   atctgngacn ctgncaacat ncttaaacat ggggtaccnn caatttcacc ccagt 775

<210> 187
<211> 2671
<212> DNA
<213> Homo sapiens

<220>
<221> misc_feature
<222> (1)...(2671)
<223> n = a, t, c or g

<220>
<223> nbla-10329

<400> 187
gcctgtacgg aagtgttact tctgctctaa aagctgcgga attcctcgag cactgttggc 60
ctactggctt ttgatgagtc cttttccctg cacggagctt ctggtgaagg tggtgggagt 120
tgttcttgca gagcacatgg gagtccaacg gcctggcagt gggccaggat cttgttgcca 180
tggtgactga cactgttttc gctgaaattt tgccagatcc tgaagatctt cgtggacaat 240
ctctttgcta tcgtcctgct gaagcaggcc acagctgtgc gctgcttgga catgagtgcc 300
tcccgtaaga agctggccgt ggtagatgaa aatgacactt gcctggtgta tgacatcgac 360
accaaggagc tgctttttca ggaaccaaac gccaacagtg tggcctggaa cacccagtgt 420
gaggacatgc tctgcttctc gggaggaggc aacctcaaca tcaaagccag catcttccct 480
gtgcactggc agaagctgca gggctttgtg gtcggctaca atggctccaa gatcttctgc 540
ctccacgtct tcatttctgc cgtggaggtg ccacaggtaa ctgggagcac ctgtccactc 600
tcagcactgg caaggccagt aagaccaggg aagccgggcc cctggctggt gctttgagga 660
gagagtagca aggagagaag aatggcagct gtgatgtcgt gggaggcagt ctgggcttgg 720
aacctgcagc cctatctcca ctccttgcaa cctgtgtgac tgggggcaag gagctcaacc 780
tccctgtgcc tcaatttctt cctggtcccc cttctggtaa ggattcagag gggtatacag 840

tgttgtcagc agagtgtatt ttacagagag cccagtgtgg gccaagtgga gagagcagca 900
aagccgggag tccagcaggg tgtgtgtgtg tgagcacatg tgtgatggtg gtgatgtgtg 960
tgtgagagag ggggaagaga gagagaatga gtgggtgggg ggggtgaggc ctgaggtctg 1020
tgagccaata agggagcatg ggactctgca caagccccct gaactaggag gtggccacag 1080
cagaaatggg gcactgaagt gtggagccac agaatgcggg agggcagaac cacagacagg 1140
aggctgagat tgacctcctg agtgcaagct ggtctcccct tcacctcctg caccccacgc 1200
agatggtgct taccatagga ttgccgtaaa acagagacac gcaccagcga gaaactttag 1260
cccttagtat cccatcctca ggacagaatc actcttaaac atgttgaaat acatctgctt 1320
agagcttttc tatgtgtcta tataatgtat gcataatata caattagaag catgtgattt 1380
tataacattt ttaaacaaaa gtggaatcac actgcaaata ctgtttcatg acttttatgt 1440
gtgtaagtat tgctacctat agatggaacc tcagatttac tgtattcccc aagcctggac 1500
tgtaaacaag acttctcatc ccttcctcta tggccatctt ctcatttcac ctcacatttg 1560
gttatgacag ccccatcact gtctatgaag tgctggctgt aaactcgtca tctggccttt 1620
tcactcagca atctcctgaa gcatggttct gtgtcagtaa atgttcttat ccagcatggc 1680
aaaatgttaa ggtaattgta gactcacata agttgcagga aataatacag agatccctta 1740
taccctttgc ccagtttccc gcaatggtaa cagtgtgcaa agctccagga cgattttgcc 1800
acccatgttg gcatgtgtaa ccgcccccca cagtcacgac actgaacatg gccaccacca 1860
caagtgccta gtgttgtgct acccttttac agccagcgcc cctccctcct gcccggcccc 1920
cctgcgttgt atttatggct gcattttgtc ctgtcttgtg gatgttccat ggcggacaga 1980
caggctgaac aaacaaaggg tgtgcaaaga agcccagagc aggagagaag gctccccagc 2040
ctggctcttg gtcagcacag tcacaggctg gctctgcctc tcttggctgc actgtcctga 2100
agcaggaaat atgagaaaaa caagtaaagg gaaaacaagt ccagttggag ctatgataac 2160
attatctgca aggccaggca ggggccccaa agaaatggga tccaagagca gggacgagaa 2220
aagcaagttc ttagtctccc cctggaattc tttccccatg ccatcattct ttgttctgct 2280
cttataacta tttttgtaac tatttctgca agtttgcaag gattttataa gttcatgttt 2340
tcccatctat gcaggatggc aaaggtcaca agacatgcct gaattgcaaa acctgtcaca 2400
gtttgattaa ctgcctttgt tctgcttctg taagctcgct ttccccaccc cgcaagtttt 2460
gcaccactgg ctagccactc cccttcagtc gcctgaataa aagtcaagcc tgatctttgt 2520
tcgttgctca gcctctggat attaatccgc tgggccagtg gccacctgaa taaaatcctc 2580
ctatttcaaa aaaaaaaaaa aaaaaaaaaa aaaaaaaaaa aaaaaaaaaa aaaaaaaaaa 2640
aaaaaaaagg ccacatgtgc tcgagctgca g 2671

<210> 188
<211> 3129
<212> DNA
<213> Homo sapiens

<220>
<221> misc_feature
<222> (1)...(3129)
<223> n = a, t, c or g

<220>
<223> nbla-10363

<400> 188
gtacggaagt gttacttctg ctctaaaagc tgcggaattc ctcgagcact gttggcctac 60
tggatttgaa aacacgctcc gggagctaga gcctgaggtc ggcggcgcac gctgttgccc 120
cgtgggcttc tgctccctcg cttgtcttct cgggcttctc gccccggccg cggccgggtc 180
ctcagtagtc gagaatgact aaaaaaagaa aacgccaaca tgattttcaa aaagtaaaat 240
tgaaagttgg taaaaagaag cccaagttac aaaatgctac tcctacaaac tttaaaacaa 300
agactataca tctgcctgag caactcaaag aggatggaac acttccaaca aacaatagaa 360
aacttaacat aaaggatttg ctgtcacaga tgcatcacta caatgctggg gttaaacaaa 420
gtgctcttct tggacttaaa gacctttgt ctcaataccc atttataatt gatgcacacc 480
tttcaaacat attaagtgaa gtgactgctg tgtttacaga taaagatgct aatgtacgat 540
tagcagcagt tcaacttctt caattcctgg cccccaaaat acgagctgaa caaatttctc 600
cattttttcc tttggtaagt gcccatctct ctagtgccat gactcacatt actgaaggaa 660
ttcaggagga ctctttaaaa gttttggaca ttctgctgga acagtaccca gctctaatta 720
ctggccgtag cagcatattg cttaagaatt ttgtagaact tatttctcat cagcagctgt 780
ccaaaggact gataaataga gacagatccc agtcctggat actttctgta aatcctaatc 840
ggagactcac ttctcagcaa tggaggctga aagtcttagt gagactcagt aaattccttc 900
aggccttggc agatggatcc agtaggttga gagaaagtga aggacttcag gaacagaaag 960
aaaatcccca tgccactagc aactccattt ttatcaactg gaaggaacat gccaacgacc 1020
agcaacacat ccaggtttat gaaaatgggg gttcacagcc aaatgtcagt tcacagttca 1080
ggctacggta tctggttgga ggactgagtg gtgtggatga aggcctgtca tctactgaaa 1140
acctgaaagg atttattgag ataataattc cattgctaat tgaatgctgg gttgaagctg 1200
tacctccaca actagctact cctgttggga atggtataga acgagaacct ctacaggtta 1260
tgcagcaagt tcttaatatt atttcccttc tgtggaaact ctctaaacaa caggatgaaa 1320
cccataaatt ggagtcatgg cttcgaaaga actaccttat tgattttaaa caccatttta 1380
tgagtcgttt tccatatgtc ttaaaagaaa taaccaagca caaaaggaaa gagccaaata 1440
aaagcatcaa gcattgcaca gttctctcca ataacataga tcatctctta ctgaatttaa 1500
cactgtctga tatcatggtc tccctggcaa atgcgtcaac tttgcagaag gattgcagtt 1560
ggatagaaat gataaggaaa tttgtaacag agacccttga agatggctct aggctaaata 1620
gtaagcaact gaacagattg ctgggagtat cctggaggtt aatgcaaata cagccaaaca 1680
gagaggacac agagactctt attaaggcag tttatacatt atatcagcag aggggcctta 1740
tccttccagt tcggactttg ttattgaagt ttttcagtaa aatctatcag acagaagaac 1800
tgagatcttg tagattcaga tatcgtagta aagtgttatc ccgttggctg gctggcttac 1860
cattgcaact tgctcatctt ggctcccgaa atcctgagct ctctacacag cttatcgata 1920
tcattcatac cgctgcagca cgagcaaata aagaattact aaaaagttta caagcaactg 1980
ccctccgaat ttatgatcca caagaaggtg ctgtggtggt tctccctgca gactctcagc 2040
agcgtttggt tcagcttgta tatttcctac ccagtctgcc ggctgatttg ctttctcggt 2100
taagtcgttg ctgtattatg ggaagactca gttcaagttt ggctgccatg cttatcggga 2160
tactgcacat gagatcatca ttttctgggt ggaagtattc agctaaagac tggttgatga 2220

gtgatgtaga ctatttcagc ttcttatttt ccacacttac agggttttcg aaagaggagt 2280
tgacttggct tcagagcctt cgaggagttc ctcatgtcat ccagacacag ctttcccctg 2340
tgcttctcta ccttacagat ttggatcaat ttttacacca ctgggaagta acagaggcag 2400
tttttcacag tttattggtt attcctgccc gaagtcagaa ctttgacatc ttgcaaagtg 2460
ccatcagtaa gcatttggtt gggttgactg taattcctga cagcacggct ggctgtgttt 2520
ttggtgttat ctgtaagctc ctggatcata cttgtgtagt tagtgagact ctactgccat 2580
ttctggcttc ttgttgctac agtcttcttt attttctgct cactatagag aaaggggaag 2640
cagaacatct aagaaagagg gacaagctgt gggggtctg tgtctccatc ctggctctct 2700
tgcctcgagt cctcaggttg atgctgcaga gcctgcgggt gaacagagtt gggcctgagg 2760
agctgcctgt tgtgggccag ctgcttcgac tgctgcttca gcatgcaccc ctcaggactc 2820
atatgttgac caatgcgatc ttggtgcagc agatcatcaa gaatatcacg acattgaaga 2880
gtggaagtgt tcaggaacag tggctcacag acttacatta ctgctttaac gtgtatatca 2940
ctgggcatcc ccaagggccc agtgcactgg ctacagtgta ttgaagaggc catagtacct 3000
cctgtttgaa gttgtttatt cacatctatc ttatttgaag aaaaagactg atgtaataga 3060
tctttgtcat taaagctgaa cttttaaaga aaaaaaaaaa aaaaaaggcc acatgtgctc 3120
gagctgcag 3129

<210> 189
<211> 589
<212> DNA
<213> Homo sapiens

<220>
<221> misc_feature
<222> (1)...(589)
<223> n = a, t, c or g

<220>
<223> nbla-10367

<400> 189
gcctgtacgg aagtgttact tctgctctaa aagctgcgga attcctcgag cactgttggc 60
ctactgggaa gatgcaaata aatactttga tctatgtttg attttgctaa taatatttga 120
aggagattgc ctaccaagga caaaacaata aatttaaaaa tcaaacgatt tctccatacg 180
ctcatagtca catatggaat tttgagaaaa taaagcatgc tgtctttagg aatttttata 240
cttctttgtc tttcttcctt aatatttgct tctagctgct cttggcaatg atgaattgtt 300
atgtatgcat taatgttttg cagcccaaaa gttgttcaca tttttcctat ataagatctg 360
tggagtgtgt gtttcaaaga gagaactaca gaaatgttaa agcgggaaaa cctgaatgtg 420
atgtgcacat tttcatccca catggacaat gtatgtgttt taataaatgg aattttcaga 480
ttcaaaaaaa aaaaaaaaaa aaaaaaaaaa aaaaaaaaaa aaaaaaaaaa aaaaaaaaaa 540
aaaaaaaaaa aaaaaaaaaa aaaaaaggcc acatgtgctc gagctgcag 589

<210> 190
<211> 788
<212> DNA
<213> Homo sapiens

<220>
<221> misc_feature
<222> (1)...(788)
<223> n = a, t, c or g

<220>
<223> nbla-10371-rl

<400> 190
gnagngggtt nnnttgatgg ccctttnttn ntncntttta ttgctgatat gttggtttta 60
attcaaaaga aacgatgatg ccaatggttt ggattgacag cagcttaagg aggaaaaaaa 120
aaaaaagaaa gaaaaaaaga aaaggctggt gacacccccc tggcgtggtt gacctggtcc 180
cagtcgcagt ccctatggga cagcagagct gtcaaaacag aacccaatga tgacggctga 240
aaatcaagcc agactgctgt gcggtcagct tgccgccgtg gaatgccttg tgtcttggcc 300
atgtccagcc aagggtacaa cgtgcttgtt cccgatcacc caggtttgcc attggaagtc 360
aaagacagaa tcgcttcatg gcactacaga tgtggaaaaa taaaaatctc agctagaaag 420
aacgtccgat ttggagatag cgggaggaca cgaaggagtg ggggccattt tggtgctgag 480
aggagggtgc cccaacttca ggaggaccat gtgacggctg tggttgtttt cggggtcact 540
tgcagcacac acagcgtccc cttgatgctc gatggggacc gcaaacggcc tgcagaccta 600
acccctggct gtggacagga aaagcttgct tcaagcttct ttccttctca atgctttaca 660
cangatttcc ttcatttggg ctttcctttg ggttaacaag ttaaaaaaga anatgagggg 720
gcnaaatggg ttggcacttt gtccaaactg gaaanaanag gtggaantgg gccccnaaat 780
ttctgggg 788

<210> 191
<211> 2935
<212> DNA
<213> Homo sapiens

<220>
<221> misc_feature
<222> (1)...(2935)
<223> n = a, t, c or g

<220>

<223> nbla-10383

<400> 191
   gtacggaagt gttacttctg ctctaaaagc tgcggaattc ctcgagcact gttggcctac 60
   tggctcttac agtggtccct gctggccctt ggtgacgggt cgcgtcagtt ccgacccgga 120
   cccgtacgct gctgcgctga cgtggctcct ggaagcaggg ctggcgtagg gccgccatgt 180
   tgcagcagga tagtaatgat gacactgaag atgtttcact gtttgatgcg gaagaggaga 240
   cgactaatag accaagaaaa gccaaaatca gacatccagt agcatcgttt ttccacttat 300
   tctttcgagt cagtgcaatc atcgtctgtc ttctctgtga gttgctcagc agcagcttta 360
   ttacctgtat ggttacaatt atcttgttgt tgtcgtgtga cttttgggca gtgaagaatg 420
   tcacaggtag actaatggtt ggcctacgtt ggtggaatca cattgatgaa gatggaaagg 480
   gccattgggt gtttgaatct agaaaggagt cctctcaaga gaataaaact gtgtcagagg 540
   ctgaatcaag aatcttttgg ttgggactta ttgcctgttc agtactgtgg gtgatatttg 600
   cctttagtgc actcttctcc ttcacagtaa agtggctgga atctgaacct gtgatgttaa 660
   gaaatcagta aatattaaaa agaagatgga tgcaagaagg atgaagaaag aagaaggact 720
   cacagaaaac actggacttc cccggaagct acttgaaaaa catgacccct ggccggccta 780
   tgtcacctat acctctcaga cagtgaaaag actcattgag aaaagcaaaa ctagagaact 840
   ggaatgcatg cgtgccctcg aggaaagacc ctgggcatca aggcagaata aaccttccag 900
   cgtcattcag ccgaaaagga ggaagtcttc caagtcttct ggcaaagctg tgttcaggga 960
   cacgctgtca gaatccacgt tatcaatgtg gggcgcttat tcggtattgg ccatggctcc 1020
   taccatgatc ccagaaccca cacacttaca tgcggattcc agagactgtc caactgaaaa 1080
   ctataacaag atcatctttg cccgcaagcc tatgatgagg atgctcccta cagttcgcta 1140
   ctgaccaaca aggagaaaca tgcaaatgtt ttagccaggg gtccctgcag ccattcccca 1200
   agaacgtaca aaagttaagc tccatttacc actcacttga cagtgtcctt taaatctgaa 1260
   cttgctcttg ctcatgcgga gcttggctaa gagaaaccag atcagaatgc aagctgtagg 1320
   tgggtgggtg agtgtgtccc ctcccacact gctctctggt gggtttgggc agtccctggc 1380
   tctagtctct gctgcagatt ctcaggacca aaatgtgggt gatttgtctg ggtccaggca 1440
   ggagacagaa atcacacaag ctcttttatt agagataata taaagaatga ttaaccaggt 1500
   gacagtaaag ggcaggaagg gaatacaaag gtatccatgg aggtagcacc acaggaagca 1560
   ggcatcacac ttgaggcttg ggaacaaggg gaagatgctg gactcattga aaggtggttg 1620
   cttggagaag ggacatgggg ctgaatctca gacttctgag gtgggggagt ggggatgcca 1680
   ctcaatgcag ctggtatctc tgagctctga ggagagtcct gtggggatgg tgccccctcc 1740
   tctgaatagg ggagatgttt tgttggtgtc tctgagggga cccccatgag gctgcttttg 1800
   tgagtgttgg aaaaaccaaa aactgcattg aaatgtttct gggaggagtt gccactgcct 1860
   gggtgaagaa aaagcactgc tagggttagc aacctgtctg gcaagggaca ggaagctgat 1920
   agaaagtgaa caggaagcaa acaggaagtg agcaaggggc cacttcctac ttcctccttt 1980
   agcctagcag attccttcta gctccccctg ggggctgagc ctatcagggc acaaagccag 2040
   aatgtggttt gtagaatccc agccccagca tctccaagca taggatggat tttgaagcca 2100
   agagggaatt agctctcggc cgggttctga gtgatcccat ccagctgctc ctggacctgg 2160
   tattgagagg tttttccctt ggagggcaaa caaagtggca gtggggacca gggtgtcccc 2220
   tgctgagaaa tggagagctc ttttcccctg tacttctctg gggtttgcct tgtggaacca 2280
   agtgcttggg ggaggagctc ctggcaggac tccagctgtt atttgtcaga ggacagctag 2340
   gattggttca cccttgctct gagttggccc caaagcgagc tatgctgaac tcttctccat 2400
   ctccaagcag acaacctttg tctttacatg caagagggat ccaattatga caggctgacc 2460
   cagctggttt catgtttggg gtttatctac tgagtaaggc tgaccttacc tgagtttctg 2520
   tatgtgtatt tgcaagacag ttaatactaa tccatcatcc ctcacagaga tgtagaggat 2580
   gagatgtagt aacttatagc agtgtcattt ggaaaagttg aatttgccct acaaggcatg 2640
   ttctccattc aagggtcttg tgtaaacgtt tgcagacaca ggtgcagctt tggagaactc 2700
   acctctggct cttgtgcttt tatgttggga cagctgccta aattaggagc tctagacctg 2760
   ttattggttc ccaactgttg gccagaatga attcttcgaa aaatataggt ggtttccagg 2820
   gaatttgaat gattgtattt tgtgtccatg gaacgtttat agacatggag gcaggcaata 2880
   aattcctcca ataattaaaa aaaaaaaaaa aaaggccaca tgtgctcgag ctgca 2935

<210> 192
<211> 2139
<212> DNA
<213> Homo sapiens

<220>
<221> misc_feature
<222> (1)...(2139)
<223> n = a, t, c or g

<220>
<223> nbla-10388

<400> 192
   ttctaggcct gtacggaagt gttacttctg ctctaaaagc tgcggaattc ctcgagcact 60
   gttggcctac tggaaatgta gatagcctta caagctttaa gtccattaaa aatggtaatg 120
   ataaggacat cactgctgaa ctttctgctc ctgtgaaagg acagtctgat gttttacttt 180
   ctaccaccag caaagaaata atttcatctg aaatgttagc tacttccatt gatggcatgg 240
   aaagaccagg aaatttaaat aaagtagtgg agcagagaca gaatgttgga acacttgaaa 300
   gtttctctcc aggagatttt agaactaata tgaatgttgt tcatcaaaac aagcagttag 360
   agagctgcaa tggtgaaaag cctccttgtc tggagattct aacaaatggg tttgcagtgt 420
   tggaaactgt aaatcctcag ggaacagatg atctggacaa tgtagctgat tcaaagggac 480
   ggaagcctct tagcactcat agcactgagt ataatttaga ctctgtacct agtcctgctg 540
   aggaatttgc agattttgcc acattttcca aaaaggaaag gatacaatta gaagaaatag 600
   aatgtgcagt tttaaatgat agagaagcac taaccattcg ggaaaacaat aaaattaata 660
   gagtcaatga actgaattct gtaaaagaag tggctttggg tagaagcttg gataacaaag 720
   gagacactga tggagaggat caggtttgtg tttcaaaaat aagcatagtg actaacagag 780
   gtttcagtgt tgaaaaacaa ggccttccaa cactgcaaca ggatgaattt ttacagtcag 840
   gtgttcagtc aaaggcttgg agtttggtag actcagctga taattcagaa gccattagga 900
   gagaacaatg taaaactgaa gaaaaacttg acttacttac ttctaaatgt gctcacctat 960
   gcatggattc tgttaaaact tctgatgatg aagttggttc tcccaaagaa gaaagtagaa 1020
   agtttactaa tttccaaagc ccaaacattg accccacaga agaaaatgat ttggatgatt 1080
   ctttaagtgt aaaaaatggt gatagtagta atgactttgt gacttgcaat gatatcaatg 1140
   aagatgattt tggtgatttt ggtgactttg gctctgccag tggctcaact ccaccttttg 1200
   ttactggtac tcaagattca atgagtgatg ccacttttga agagtcttca gagcactttc 1260
   cacattttag tgaaccaggt gatgactttg gagaatttgg ggatataaat gctgtttctt 1320
   gccaagagga gacaatatta acaaagtcag acctaaaaca gacttctgat aatttatcag 1380
   aagaatgtca attggcaaga aaatctagtg gaacaggcac tgaacctgtt gcaaaactta 1440
   aaaatgggca agaaggtgag attggacatt ttgattctgt gccaaatatt caggatgact 1500
   gcaatggttt tcaagactct gatgattttg cagacttcag ttcagctggt cctagccaag 1560
   ttgtagattg gaatgctttt gaggatgaac aaaaagatag ttgttcttgg gctgcttttg 1620
   gagaccagca ggctactgaa tctcatcatc gaaaggaagc ctggcagtca cataggacag 1680
   atgaaaatat tgatactcca ggaaccccca aaacgcacag tgtaccttca gcaacttcca 1740
   aaggagcagt tgctagtggc catttacagg aatcagccac ttcagttcag gtatttacta 1800
   attttctcta ttttgtatga tgtattaatt gttgtggagg cttctaattt cagcttttca 1860
   aaaaatattt tttcaactga gtacttgtta aaggagtctc gttatgatgt ataatgcctg 1920
   tctgatttgt gggttggtta catttttcac aaccctttgg gaaactacaa atagtcctat 1980
   agtaaagcgc agtaagattt ttctttatct caatgagttt cgttaatatt tacagttact 2040
   catcaacttg gatttcttgg tgactataaa taccatatat gttaaaaaaa aaaaaaaaaa 2100
   ggccacatgt gctcgagctg caggtcgcgg ccgctagac 2139

<210> 193
<211> 821
<212> DNA
<213> Homo sapiens

<220>
<221> misc_feature
<222> (1)...(821)
<223> n = a, t, c or g

<220>
<223> nbla-10390-f

<400> 193
gngnnggggt gggntncnnt tttgaanncc cttgcactgt tggcctactg ggctctcgag 60
tgtctccaag caagatggcg gaggagccgc agtctgtgtt gcagcttcct acttcaattg 120
ctgctggagg ggaaggactt acggatgtct ccccagaaac aaccaccccg gagcccccgt 180
cttccgctgc agtttccccg ggaacagagg aacctgctgg cgacaccaag aaaaaaattg 240
gttgtgaatt cactccttaa accaaaaacc aaattcttat gcagaatgaa gactgctaag 300

ggaaagctaa aggcatagat gttacctttt taagaaacac attttggaac ctttttgaca 360
ttttgctaaa ggctgtggga gacactccta ttatgaaaac aaagaagtgg gcagtagagc 420
gaacacgaac catccaagga ctcattgact tcatcaaaaa gtttcttaaa cttgtggcct 480
cagaacagtt gacccaagag gtataggtcc tattattttc catatcttac aaataaagag 540
gatgagagat tatttgtaaa gccaggattc aaacccagtt tatttatgtg aatcaagtcc 600
tttgntnctt cccagaccaa gaagttggaa ctctctatga gtggtttggc agtgatggna 660
aactgggttt acantactgn aagtctnaag cgtggggatg aaccccaagg aaaatcaact 720
tgttctacat gaaaaggatt tcacgggaag aaacagttnt gaaagtttga tcttgtggaa 780
gaacttacag atgngaccat taagatgggg tacttatggt n 821

<210> 194
<211> 821
<212> DNA
<213> Homo sapiens

<220>
<221> misc_feature
<222> (1)...(821)
<223> n = a, t, c or g

<220>
<223> nbla-10390-r1

<400> 194
gnaggggnnn nntttgtggc cttttttttt ttntttgaga tggggtctca ctctgttgct 60
cagcaggaat gcagtggtac aatcacgact cactataccc ttgaacttct gggctcaggg 120
gatcctcttg cctctgcagc ctaataattt tttttaatt tttatttttt tgtagagttg 180
gggatcttgc cttgctaacc aggctggtct caaacttgtg gcttcacgca atccccacac 240
ctcagccttc caaagtgctg ggattacagg tgtaagccac catgcctgat ctggaatgct 300
ttctaaaaat atggcattat aaacataata ctaaagagaa aaaaaggtta aatttcactt 360
caagaaatta tcagtctaat agcatcagta aaaatggcac ttttagtcat ggcataaaat 420
atacaataag gctatttttc aattattgnt ttcaagttgg aaaatgcatt atgaaacact 480
caaaaagatg ctactgggtt tactcatcaa gtgcttttga caccatataa atgacagagt 540
aacaccacag ttatgtgatt gggactcaag aatccttaaa tggtaggact gaaaacaggt 600
atatgactta ttgnccagaa attatatttt tctggatcca ataaaataat taagccctgt 660
gtattgggna aaaatacagg ctttcaatct ggcctatgng cttgntnttc tggctagata 720
ttagcttaaa antagcaaaa cagccatttt ccctatattt cccancaaaa tgggaaacca 780
aacgcctaac ctttttgcct gggggctgcc ttactggctn t 821

<210> 195

<211> 2131
<212> DNA
<213> Homo sapiens

<220>
<221> misc_feature
<222> (1)...(2131)
<223> n = a, t, c or g

<220>
<223> nbla-10396

<400> 195
   gtacggaagt gttacttctg ctctaaaagc tgcggaattc ctcgagcact gttggcctac 60
   tgggacaaag ctagcgatgc tttatgctag gaaacttgtt gacagtaacc tgtgcgactt 120
   tatgcagaag acaaatgcta gtaattatta tgcacagagg aaaaatcatt ttaagtatgt 180
   ggtaaagcag cttcatcttt caaaattgat ttgctctggt ttttctttag tccattagat 240
   tccagaatgt ccttttactg ggaatttagt tatgtattaa gataacctgt tttcagttct 300
   ttttgaaaag aagacattat ttatattgaa ccaccttatt ttaaaatttt taacttttat 360
   ataccacttg tgtgattcca gtgtcatgtc ttgggtttga tgtcgttgga cagaaaagtg 420
   tatcaattat tttaaatgaa tttttcccca tgtttgaggc ttagtctgta aatgtgttgc 480
   tgtaacagaa aatacttggg tatgcattac ttgaatactt gaaagctgaa attaataaga 540
   tgtattacat aatgaattag atttctctga acagttttta cactgaaaat cttcatttct 600
   ggattgcagt ttgaaatgga atgaagacct gaattatttg ggtagaaaaa attatgatag 660
   tgcttataag aactgtaaac tgttttaaac tattttgtgt ttgacgcatc aaacttcaag 720
   ttttttgtaa gtttctctcc tgaaattttc tttctcttct atactttatg cacttactat 780
   actactgatg taataaaaga gcagggttaa aaatattgta tctgtattca ttgtgaatcc 840
   tgtagctttt ctagttaaca aaaaatcgct ttctaaaata ctcttaatcc cattgttttg 900
   gttaacatct tacccatttg ttgtatttca aatgccatta atcattttag tacaacacct 960
   atgtttataa aaatttgaaa acattacata ttgtatttaa aactaattag tgaagagtaa 1020
   gaaaaaaact agccaacaga attgtaggtg atgcattagt taaatttcaa aactcataat 1080
   aaaggaactt tcagagattg gttgaaaccc agtggtatcc ctgtaaatta gctcctgtga 1140
   ctggaaaaga ccccaaaaag gcagtagagg agattagtgt ttacttgctg tggttgtggt 1200
   gtgctgctac ttaattatag gtagtgacac actgaaattc ttatttgtcc aataatctga 1260
   agtagtttcc tatatttatc tgtactaaat tgactataaa ttgagtctgc aaagaggaaa 1320
   ctttttgact gtactgtatt taggagcctt tgtacagctt ggtcaaattt ccatgatatg 1380
   aagtatttga gttttaaaat atactgttat taaaaggaaa aagacatggc cattattcca 1440
   tgtgcttaaa tgataatttc cttattcagt ttcagaagaa aaagaatgaa attgggtaac 1500
   tgtcattgcg ttagctttat gttgaattgg gaaattgtgg cataaagctt aaattcgtgt 1560
   ttatcaaatg tgaaccatag tagtataatg ctgctttgta tataatgtaa gtgctacaaa 1620
   tagtctcagc actgaaaatg tattgatacc tcttaaatga atgcaacttt tgatgtaggt 1680
   gttttgctat gcctcagaaa atatctgtct gagaatttgt taatctgttt gataatgaag 1740
   atacttcctg ttttcttgtt tcatattttc atgttcaaaa tttaagtttt acatttttac 1800
   tactgttaat ttaaataaaa tttgttctgt ggataaaatg aggttggcag tgaagaaaat 1860
   taaaaacagc ctcattcatg taactggtta agtaaaaata cattttcact atgtgttcat 1920
   aaacttttaa tgaagctgtt tgtctttcag ttcaaatata agtgatgttt aggctttatt 1980
   tctgttaata aggcttttta ccattgatta aatgaaggaa tgtatctttt tgaagagatt 2040
   tatattctgt aaataaaaat tcgttgtaac aataaagttg agttctaact acaaaaaaaa 2100
   aaaaaaaagg ccacatgtgc tcgagctgca g 2131

<210> 196
<211> 775
<212> DNA
<213> Homo sapiens

<220>
<221> misc_feature
<222> (1)...(775)
<223> n = a, t, c or g

<220>
<223> nbla-10398-f

<400> 196
ncctnggcct gttggcctac tggcattagc tcagagaact atctaattat aggtggacga 60
gatcagcaac agaatgaaat acttgtgaaa agatacttga caccaggaga acccatcccc 120
ccacggacct tgactgaagc tggcacaatg gcactttgct acagtgctgc ttgggatgca 180
cgagttatca ctagtgcttg gtgggtgtac catcatcagg tatctaaaac agcgccaact 240
ggagaatatt tgacaacagg aagcttcatg ataagaggaa aaaagaattt tcttcctccc 300
tcatatctaa tgatggggtt tagcttcctt tttaaggtag atgagtcttg tgtttggaga 360
catcagggtg aacgaaaagt cagagtacag gatgaagaca tggagacact ggcaagttgt 420
acaagtgaac tcatatcaga agaaatggaa caattagatg gaggtgacac gagcagtgat 480
gaggataaag aagaacatga aactcctgtg gaagtagaac tcatgactca ngttgccaag 540
angatatcac tcttcagagt ggcagagatg aactaaatga ggactcattc aggaagaaag 600
ctctgaagac gaaggagaat atgaagangg tagaaaagat caggattctg gtgggngaaa 660
tgaaggntga angggaaaga gaccttaaat attctgntct nccatggcct ggnttacctt 720
aancccnaag gtcattcnna aattggnttn aaaanaggaa cntttaattn nnggn 775

<210> 197
<211> 791
<212> DNA
<213> Homo sapiens

<220>
<221> misc_feature
<222> (1)...(197)
<223> n = a, t, c or g

<220>
<223> nbla-10398-r1

<400> 197
   gngnnnnntt tgtggccttt ttttttttnn ttttggtgaa tatagcaagg caatgtttag 60
   ttcattttta taatgcggaa atcctgatac atggtgcttt catctttgaa cttccaaaag 120
   gctataaaat tggctcttct caaatatttt agaatttcat ttcagctatt tcctttttac 180
   gttcagaaga ttgggtgcag acactttcac tttgccagga atgtttcttg ataaatctgt 240
   gtcctttacg ctgcggaata agtctttttc tcttgctgtt gcttctttgg aatgcatgaa 300
   actattcaag gctgtttttg cagcttttcc ctttttctgc actccaggag taagtttcac 360
   tttatatttg tagtttgtca tggtggtgta aggggcacat attggaatgg caaacagtag 420
   tgcatcttca ggatgtggct ggcctgtcaa agaatcaaat aggttttcct catttccctg 480
   ntgatccana tcttgctctt ccttgcatca tgnggatcat ctacagcaaa gtcttgnaac 540
   tcatgagtta tacctcaagg aacggagntt ctttcttaat ggtgcagaaa ccctttgtcc 600
   acctntangt tctggggctg gtctttacan ggtcngccct tggttttcct tcttccctct 660
   tcctttttct tcttggttga accctgnaga acccnanaac ttnatganaa gtcacgggct 720
   tnttaatccn ggccttggaa ttnncnttna atttttcaat ttaanccttt ggccncgntn 780
   natngnnggc n 791

<210> 198
<211> 802
<212> DNA
<213> Homo sapiens

<220>
<221> misc_feature
<222> (1)...(802)
<223> n = a, t, c or g

<220>
<223> nbla-10399-f

<400> 198
gnngnnnnnt tnnnnnnnnn ntttganttc ctcnagcact gttggcctac tggaaatctg 60
taaaagacct ccagcgctgc acagtgtctc ttgcacggta tcgagttgta gttaaagaag 120

agatggatgc ctccattaag aaaatgaaac aagcctttgc tgaattggag agctgtttaa 180
tggatcgaga agtggcgttg cttgctgaaa tggacaaagt gaaagctgaa gcaatggaaa 240
ttttgctcag ccgacaaaag aaggctgaac ttctaaagaa gatgactcat gtggctgttc 300
aaatgtcaga gcagcaattg gttgagctca gagctgatat caagcacttt gttagtgaac 360
gtaaatatga tgaggatctg ggacgagtag cccggttcac ctgtgatgta gagaccctaa 420
agaagagcat tgattcattt ggacaagtgt ctcatccaaa gaacagctat tcgaccanat 480
cccgatgtag ctcagttaca tctgtgtcct tgagtagccc aagtgatgcc tctgctgctt 540
cctctttcac ctgtgcctnt nctccacctt acaagtgcta acaaggaaaa actttgcacc 600
gggagagact tctgcagcca tagcaaactt caatgggcaa gcctaccagn cctttnggan 660
gnantggcag ggaacagacn angaggacag gctataggnc acaaggccaa agtccaatga 720
cccattaacc aagggcggat tacantttng gncntacnna acannttngg ggattnaant 780
ggttcaagnt taaaangctc cn 802

<210> 199
<211> 811
<212> DNA
<213> Homo sapiens

<220>
<221> misc_feature
<222> (1)...(889)
<223> n = a, t, c or g

<220>
<223> nbla-10399-r1

<400> 199
gnngngtnna ttgtggccct ttttaatttc ctttgggcat acttatgcag ttgtttttat 60
ttattcaact gtaaagactg cagcaatttt tttgatgagt aatgccttag attaacacta 120
attatattgc tataaatttg gttcaggatc agaatagggt tcaggaaagg gagagaacaa 180
aaaaaaagtc tccaaacatt aaaacagaga aatgcttttt tttttttttt gagactgagt 240
cttgctctgt ctcccaggct ggagtgcagt ggcacaatct cagctcactg cagcccccgc 300
ctcctgggtt caagcgattc tcctgcctca gcctcctgag tagctgagat tacaggcaca 360
cgccaccatg ctcagctaat ttttgtattt ttagtacaga cagggtttca ccatgctggc 420
caggctggtc ttgaactcct gacctagtga tccacccgcc tcagcctccc aaagtgctgg 480
gattacaggc atgagccaca acgcccaacc tttttttttt taaactttaa ttaaatgcta 540
ctttctcata gacttcccta nactgggttg caatctaagg gaatanggaa acaaggcatt 600
gggcattttg aaaaaactgn agggntaagc tatggggccc agccnaanac ttaaccctgg 660
tttgagggca aatnttcagn ncaatttggg gannaaggca gggtcttaaa aaggttccta 720
agggncnttc ctttnactaa ataaaanttc cctttttggg ggaanccngg ccnnnttnca 780
accccctgga ttggcctttt tgggnnaaaa n 811

<210> 200
<211> 1941
<212> DNA
<213> Homo sapiens

<220>
<221> misc_feature
<222> (1)...(1941)
<223> n = a, t, c or g

<220>
<223> nbla-10454

<400> 200
ctgtacggaa gtgttacttc tgctctaaaa gctgcggaat tcctcgagca ctgtggcctt 60
tttttttttt tttttcaaa cacttcttgt tctgatacgt tcaaacatct tggtgtttag 120
gttggggaag ctgagttgtt gtttatgagt tatctccagt gctcagatat ccggcagctt 180
tttcctagga agtcagcttt gcacatgaga gaaaggaaaa aaaaaaaaaa aaaaaaaaaa 240
aaaccaaact gctggaggag aggtggggga aggttgagaa agttggagaa gggggcaaag 300
atcagaaaga ataaagcctc gatatccagg attgtcaaag attaaagtca tgtggacatt 360
tccatcccta gaaagttttc cttctttggc cgggagcggt ggctcatgcc tgtaatccca 420
gcactctgcc cgactgcctt tatgaaatat ggtaatttaa tgcttaaaca actgtttcat 480
ttgtgtactt tttgggagaa agtactacgt aaaaccagtg cttaatgatt ttgacaatta 540
aattatatgt gattattatc ttcctatagt gttttttttt ttagaagagc tactcagata 600
tagcagatta atatcactca ggcacctggg tttcctagcc cagcaattct gaatgtacag 660
agtacaagca tataatgcca actggtaaag tcagagctcc agatgacaag gtacagcatg 720
taatcttttg cagtctattg acagcaatct aaataaaagc aaaggaagaa gcttccagta 780
ctacaaactt tatgggaact tagggtctct caaagccaca ctgtttgggt cactagcagg 840
aggtacagta tattaaagca agcagctcag cctctcacta gtggtgagat gtatgaaaat 900
cctggcaatg ccagggggaa aagaatctat gagtcttccc ttcggtcatc tctcctattt 960
cttgcattag ttttttcagg gttaaatacg catctcacag aagagctagc tatatatggg 1020
ctgggtggtc ttcagtgaat ctgcaaatgt atcaaacaaa aatagcaagc tggaactgga 1080
ataaagggtc agggaataca ggaaagaaga aaacaaaggg ttctaggtgt tcagaagtca 1140
aatacatctc agggctaaaa aaaaaaagaa agaaagaaag aaagaaagaa aaagaaatct 1200
gagtgtgtcc tgtgttctac ctccctgtag cttatgaatt ctatagaact caaaacatca 1260
aaccaccaaa gacagatgat taagaaagaa aagacagatg aaaatgtgac agtcattgaa 1320
ctctaaaatt caaagcaatt attactcaga gttctagaga tttcctatga aaggtgagtg 1380
gagataaaat tgcataaaat ggaattacga tcctcctctg caatctgacc agacagagga 1440
agaagaccca agaaaatgtc aaataaactt ttgctcacca atcagcccat gccttgagac 1500
ttttaatccc tctttttcct tctttacttt tattagaaaa aaaacagaaa aaacaaaaaa 1560

aacaactata atggtaccaa gacacatttt aacaagaagg tttttgtttg ttgttttttt 1620
ttcctctctc tctctctctc tttttttttt tttttttacta aggccaggtt tttgagtgat 1680
tcaaaacatt cttttgtggt gtgatcatat tcattctgca ctaacacctc tagccttttt 1740
tttttttttt tttcaaacac ttcttgttct gatacgttca aacatcttgg tgtttaggtt 1800
ggggaagctg agttgttgtt tatgagttat ctccagtgct cagatatccg gcagcttttt 1860
cctaggaagt cagctttgca catgagagaa aggaaaaaaa aaaaaaaaaa aaaaaaaggc 1920
cacatgtgct cgagctgcga g 1941

<210> 201
<211> 794
<212> DNA
<213> Homo sapiens

<220>
<221> misc_feature
<222> (1)...(889)
<223> n = a, t, c or g

<220>
<223> nbla-10456-f

<400> 201
ggnnnnntgg ggnncnnnnn ttngaattcc tcgagcactg ttggcctact gggtcatgta 60
gaacattatc cacgtggctg tggctgtggc tgtggctggg ccctggcagg tggaaaacca 120
tctcccagaa acctgaaagc acctgccaat gacgcagata accctggccc tacagcctgc 180
ttgctccgcc tataccacag agcacagcct ggacattatg gagggtgtgg cgggacggcc 240
cacacctggg tcctccatcg ggaacttttc atgcttcttt ctccacctga ggtcttggtc 300
tgaagaagac ctcaggactc acatcttcac tcctgggcct ttgcacttcc agacgacagg 360
tcatcgttca agcagaatgc agacaggcca ttcacgagcc caagttgaag agaagagacg 420
cccatccgtg aaggagcaga ccatccatcc gatcctcccc ttccctgtcc ttcttcgtgg 480
attgtctcca ttgtccagac agtgccccca cctnccaccg ncttgcctac tggcaatctg 540
gactcgatgg agaacatncc cccaccttca tttggcacta cccaagtgga gtgtaccctt 600
gcctttcacc tgtaccaccc actncaacct tccccaactt gccaatgctt ctggggaatt 660
taatagctac catgcaggcc acaaggaatt tgtgangctt ctttggcatc tttggatctn 720
caagttggct tctttnttca tanccctgct ttactttcct tccttggaat naggggtccn 780
ttanccattt gcnt 794

<210> 202
<211> 807
<212> DNA

<213> Homo sapiens

<220>
<221> misc_feature
<222> (1)...(807)
<223> n = a, t, c or g

<220>
<223> nbla-10456-r1

<400> 202
gaggngtnnn ttggtggcct tttttttttt tttttgccaa gataaatcac ttttatctct 60
ataggaaagg gaggatctaa aaaaaatata aattacatta gtaacacaac ataagaaaaa 120
gacagggaca aaaacaacag agaagtctga atgatgctac cctaacctat ttataaaaag 180
gccctgcatc agaaattcac aatcctaccc acttctaaaa atatatttag acatgtacag 240
aagcggtggg cttgttttta aattgtttgc tttttttgta aaaatatatt aaaggtgaat 300
agaaatcctc tctcccttcc ccctgtccag cccccagcta gggactggag atcaggggta 360
actatctcat ggtgttctaa accttgatta ctaacactcc caacccctcc ccaactcact 420
tcacttagaa cctgaaacat taaaaaaaac aaaaacaaag acatcagcct ctttggaaaa 480
gcataactct gagggtaaac ttgcttttca ttggaaacaa cacaaaacaa caaacagggg 540
gctctaagaa cccaactaag ctaaatgaaa cccagtcaaa cagaggctgg gtttcaagct 600
ccacagaaaa cttctaacaa agaaatagag gggaaaaggc cggaatcggg gggatttgct 660
agcaattagc tgnctttgaa agatcaagaa ggggtgttaa tggacaaaaa ggctggggac 720
ctgacttggt tcagcagtca ccgcatagtc aggcagttgg ctatacagga canagccttt 780
tctggtntnc cttantaccc ttggtgg 807

<210> 203
<211> 2180
<212> DNA
<213> Homo sapiens

<220>
<221> misc_feature
<222> (1)...(2180)
<223> n = a, t, c or g

<220>
<223> nbla-10457

<400> 203
gtacggaagt gttacttctg ctctaaaagc tgcggaattc ctcgagcact gttggcctac 60

tggctgagca tcagactaat tctgaagcat atttgctaga ggagctactt tgcttttcac 120
aatggggtgg agaggattct ttcacttgtc ccattaaccc tcttctagtc tagatgagat 180
gaaatctgtt aatgtgtgtg tagaagaaaa cgtatgttct tctactcagc attgcccttt 240
tccacctcct cacttcacct ccgagtagct tgtttatcaa gaatgaatga atgtctttgt 300
cttaaatttt gcccatgtgt taaaagatgt aattctcaga atgggagaga aatgactacc 360
tttgttccta ctcttttata taattatcct tttagggaaa gacttggtca actctaatat 420
atctagaagg aagactatat ctggtgtaga ctaatatgag atgttttaga agagttaacc 480
tgaacacttt gagggagaga ttattcttgc cagcaaaaag ctagccagga atgagcctac 540
cacattattt gagaatatca aacctcaggc ctggggggat gaggggaaga agattaccag 600
aagtgcagga aagagaagtt tgaggaacac ccttggctta gcaacatgtg ataatgcaaa 660
gctgttataa cctgttaatc ctacgtacta tgtgttctgt acctttacat gtttttaaat 720
ttaagatagt ttgtaagaac tgtacaaaaa aatgcttctg gagatttctt tggcagaaat 780
gcctttcatc tataatttca tggagaactg ctttaattag cctaggtgaa aagtagtcct 840
agcagtgtaa atatgtataa ttagagtttt ctaatttcac tgtgagatct ctaacttttg 900
agtggcaaac agatcaagtc ttttgctcat agacttttct gtggggttat taaatgcaa 960
aagctttatt tttttaata atgccatact ccattagtgt cagatgatgg tatggaattt 1020
gttcccttgc tttcccccac tgttactgct tcagtttata gattgccagc agagttcaga 1080
aatagagcag ggatttaccc gttctttgct tggacatccc atttctttt gtccagaccc 1140
atgttggcaa tcatgtatga actgtgttat acttctcagt gctttctttt ttcttttga 1200
taagatggat atcaaaaata gttgctgtgc aaaagttagt agtcttcttc aagaagaaaa 1260
tcaattcttt ttctaataat atcctgtgaa attgcttcat tcattcattt atttttaagc 1320
caaatgtcag cagagtgctg ctgcttttat ctagtaattt tgatatgtaa gtattaatgc 1380
atttttaaaa gatgtctaca ttgaaacatg ttcttcccag tgtcctgctt atgatgcttt 1440
gttcagattt tttgtaagag accagttagt acactggggg tgtatattgt gtacatgtgt 1500
cattttagtt aggcattgta ggccaaatgt gattataaat gaagttgatg aacattaatt 1560
ttgttattag tgagtttttt gaattgtaaa tggatttcca gtttaccttc tgttgtctac 1620
agctttttta attttaaggt ttgactaatt gtatccatct cattgtacag tgttttagtt 1680
gcaagcagaa agtagaattt ggtataaagc aggttatttc tatattgaaa ggagtacagt 1740
tgaaattgta gatttaagat tgttaaaatc atgacaattc taacttgtct attctaacct 1800
attgtgtaca atctgatttt ttaaaattgt aaacatgtat gatcttggtt tcatgtgttt 1860
ttgaaagtgt tattgtttaa aaaatgaaaa aagcatatct gctaaagagc tgtcagtttt 1920
cattactgac tctgtaaaat acactgttct ttgtgtactg tgtgttattt tgccagctgc 1980
tgcattagcc ttcaaaagta tttggaaact taagatgaac tacatttctt gcaaagtaca 2040
ttcctttctg tggtattttg tcctgtaact gaagtatagt aattatttta tggaaatgtt 2100
agcaattctg taccaacttt gaataaaatg aaaaatttat aaaaaaaaaa aaaaaaaggc 2160
cacatgtgct cgagctgcag 2180

<210> 204
<211> 805
<212> DNA
<213> Homo sapiens

<220>
<221> misc_feature
<222> (1)...(805)
<223> n = a, t, c or g

<220>
<223> nbla-10473-f

<400> 204
nnngnngggn nngttgggnn nnnnntttga nnncctttgc ctgttggcct actggttttt 60
attaatctac tgtatcaata aaattctgta atttgaatga gtttttaata gtctagaatg 120
ttattgtgta tagatatttc ctcttgaacg ttatgttcag aaaatgcaaa ttacactata 180
atataaaacc tgatatatac acattagaaa tactccagtt ctccgtaaca gtgtaaagtt 240
aatcagaaag aaaaaatttt attgatgcag tgtgtcttta taaagctgtt cttgaaagcc 300
aagtgttttg tattttatag tgagttgcat gtttttgaaa aaaagtgctg aaaatgggat 360
gtgctgtttt ctgtaaattc atatttagcc atagtatatg atagattgcc ccataacata 420
gtttttcatc aagagtttat tattgnactt tattttggag ccaaaaaatt gattctgggg 480
ggtgggggca gcgtagaagt ggtatatcca agtatatcag ctactgnagt tgcacagctt 540
gtgaactttg aatgaaattc cactttgcca gattgggaga agtggaaatt atttggattt 600
agagcangnc tttttttttc ttcattgnaa tctgcaaaat gtangaaata aattacttaa 660
ngcagtatcc tttatacagg tgnataaact ggattttgga cccaaacata tagggtacta 720
cttaatgctt acctaatttc attttaagnn tttaagngct ggtaaggttt ttcatgggta 780
caccngangg ggaaaaatca ttttt 805

<210> 205
<211> 808
<212> DNA
<213> Homo sapiens

<220>
<221> misc_feature
<222> (1)...(808)
<223> n = a, t, c or g

<220>
<223> nbla-10473-r1

<400> 205
gnagggngtt nnnttggtgg ccttttttnn nnnccttttt tttttttttt ttagaaagcc 60
atttaacctt taatacctgg aataagtttt acagccacat ttgtaattta catatacagt 120

gaagatcact gaaaaagcct cctgttgcac tgcagcaaga aattttatta taatgtagat 180
taaacaaatt tgctacagag tgcagcaaga taacctcact gaggaataag attttcataa 240
tggaacttgt tacagggagt taaatatttt cttaatgtta tacagtacat attttcaaaa 300
tgaacattat cactgaaata gtttgctaac aattgttagc aattaatgnt ttatacacac 360
ttaattttac tgtaaagtat gtggctctcc aaatcttact acatgaaatg ttaagcttaa 420
acgtaagata atcccctaag atggagactc aagacatcaa aactttgatc attattgcac 480
ctatttttct acacaaaaaa aaaaaaaaga aagaaagaaa agaaaaattt acatcgtaat 540
ccacccaaaa aaagaacaga caggcgtaaa attcagactc attggcattg aacataacaa 600
aatctacaat gaacattttt ttgngaactg ctgtcagcat tacaaaaatg tgaaatgaaa 660
tttgcttaac tataatcatg ccaataaaaa tccttnacaa tcactttttc gnatggttta 720
taaaangtct taatatttcc ataaacantt ntggggcaat ttaatcaatg gccaaaatcc 780
aaggttttnc ttaatcntta tggtatag 808

<210> 206
<211> 802
<212> DNA
<213> Homo sapiens

<220>
<221> misc_feature
<222> (1)...(802)
<223> n = a, t, c or g

<220>
<223> nbla-10474-f

<400> 206
gnnnnnnnng gnnnnnnnnt tggnttcctc ngcctgttgg cctactggcg cattatttta 60
ttcactgaag atccaagggt atttaaagta acatatgaaa gtgagaaagc agagttagca 120
gagcaagaaa ttgcagtggc attacaagat gttggaattt ctcttgtcaa caattacacg 180
aagcaagaag tagcctatat aggcattaca agttctgatg tggtttggga aacaaagccc 240
aagaagaagg caagatggaa gccaatgagt gtaaagcacg ctgagaagtt agagagagaa 300
tttaaggaat atactgaatc ttctccttca gaagataagg ttattcagtt ggacactaat 360
gttccggttc gcctaacccc tactggtcat aacatgaaaa ttctgcagcc gcatgtaata 420
gctctacgaa gaaattatct tccagcatta aaagtggaat ataacacatc tgcacatcaa 480
tcatcattta gaattcagat ttacagaata cagatccaaa atcagataca tggtgctgta 540
tttccctttg tgttttatcc tgttaaacct tcaaagtcgg tcaccatgga ttcagcacca 600
aagcccttta cagatgtcag tattggcatg agatctgcag gacattccag atatcacgta 660
ttaagtattt caaagtattg attcaagaaa tggatctcan gttacatctt ggggttatct 720
atgctttaac agaccttatg acagaactga ggtgactgaa atacngaggt gggccttttc 780
ataaaggatn tngaancttt cn 802

<210> 207
<211> 810
<212> DNA
<213> Homo sapiens

<220>
<221> misc_feature
<222> (1)...(810)
<223> n = a, t, c or g

<220>
<223> nbla-10475-f

<400> 207
nnggngnnnn nttgnnnncc ntntttgnnn nccnttgcac tgttggccta ctggcttttc 60
agtcgggcgc tgagtggttt ttcggatcat gtctggtggc tccgcggatt ataacagcag 120
agaacatggc ggcccagagg gaatggaccc cgatggtgtc atcgagagca actggaatga 180
gattgttgat aactttgatg atatgaattt aaaggagtct ctccttcgtg gcatctatgc 240
ttacggtttt gagaagcctt ccgctattca gcagagagct attattcccg gtattaaagg 300
gtgtgatgtg attgctcaag ctcagtcagg tactggcaag acagccacat ttgctatttc 360
catcctgcaa cagttggaga ttgagttcaa ggagacccaa gcactagtat tggcccccac 420
cagagaactg gctcaacaga tccaaaaggt aattctggca cttggagact atatgggagc 480
cacttgtcat gcctgcattg gtggaacaaa tgttcgaaat gaaatgcaaa aactgcaggc 540
tgaagcacca catattggtg ttggtacacc cgggagagtg tttgatatgt taaacagaag 600
atacctttct ccaaaatgga tcaaaatgtt tggtttggat gaagcanatg aaatgttgag 660
ccgtggnttt aaggatcaaa tctatgagat tttncaaaaa ctaaacacaa gtattcaagg 720
ttgggntgct ttctggcaca atgcccgact gatgtgtttg gaagtgccca aaaatcatga 780
ganatccaat tcgaatctgg ngaaaaaagg 810

<210> 208
<211> 756
<212> DNA
<213> Homo sapiens

<220>
<221> misc_feature
<222> (1)...(756)
<223> n = a, t, c or g

<220>
<223> nbla-10484-f

<400> 208
gnngggnnnn nngntgggnt cnnnttgaan nccttggcac tgttggccag gctggtcttg 60
aactgctgat ctcaggtgaa ccacccacct cggcctccct aagtactggg tttacagtca 120
tttttttaaa aaataaatct tgntctttgg aagaccatta agaatagtca taaaatatcc 180
ttacaaatag aactgctcaa aaaagaaact caaaaactaa atttgagttc agtggcttct 240
gagggtatac cacgagtgaa gtctcttatg ttctcaacaa gggagcgaac ggcaaagccg 300
cacacctgag ggctcagtgc tctcaggatt catggcctgg cttcctgcta agtggtagca 360
ggtccctctc tttcatttgg actgagtgtc caaagtgcaa gtggattagg agccagggag 420
atcctccatt tttggtgttt tggtttaacc aatagtccta ggagcttagc acacctgggg 480
tgtcaccttt tgcttgtgtt tgtgaacctc tcttatcagg gacaagaacc atgtgtccat 540
ctagctgcag gcctctaggg tggggacagt atccatcaag acagggattc ccatggtgca 600
aatgatggct cttctncttc gacacatctg tgctgnactc aagccatttg tcaaggntag 660
cagagcctgg gggaagcant gtggaaagag ctgcttcgga gatagcttgg gcccaagtct 720
gaatcctggc tntgntgcac tttccanncc aatgac 756

<210> 209
<211> 751
<212> DNA
<213> Homo sapiens

<220>
<221> misc_feature
<222> (1)...(751)
<223> n = a, t, c or g

<220>
<223> nbla-10484-r1

<400> 209
   gnagnggggt nngtttgggn ctttttgann ccntttttag acaacagaaa tttatttctc 60
   acagttctga aggctggaaa gtgccagatc aggtggccag cagatctggt gtttgatgag 120
   ggctctctgc ttcctaaata atgttttctt gctgagtctt cacatgggag agggaggcca 180
   ggcaactccc tccaacctct cctataaggc tctaatctca ttcatgaggg ttccaccctc 240
   attccctaag caatttaatg ccttcccaaa ggccccacct cttactacga tcgctttggg 300
   tattgggttc tgacacatac attacctcat ttactttcaa aatagtagtg tgggcatttt 360
   taaaaactat aaaatatttc atgcataaaa aacacacact atatatatta acagttaaag 420
   aataaaatgt ctgcttccac cacttagaaa gagatggtta caaacacctn tgcaggctcg 480
   cgtctgttcc caaacacatc ctttccacac tcatcccaga caaagttact gccttaaatt 540
   tgagattatc aggactcact gntaggagct ggagacaagc agcgggcttc ccactcatgg 600
   aagaggctga aacaaaacaa aaacaggtat ggatccctga caactacnga gtggaggctn 660
   acagcaaact gnaggcccan ggtgggggca ccgtaccatg ggcctntgaa aagccagtca 720
   ccctggcttn tggacctgaa tctgcaagng g 751

<210> 210
<211> 2038
<212> DNA
<213> Homo sapiens

<220>
<221> misc_feature
<222> (1)...(2038)
<223> n = a, t, c or g

<220>
<223> nbla-10485

<400> 210
   gtacggaagt gttacttctg ctctaaaagc tgcggaattc ctcgagcact gttggcctac 60
   tggaaagatg cttgaaagat aaatacgttt aattaggggc agaggatgat cattaaagga 120
   agtctgactg ctgcagaagt cattaacaat cttaaatgaa atttttattt ttatgatagc 180
   catttacatg tataataaga gccaatgatt cttgggagca gtgtgacaga aacagagtgt 240
   ggcgagaact catgtaagac atactattta aatgagctgg tgttagctat tcatatttgt 300
   taatgaacta gatatgcagg gctataagaa tgaatgttct gatgctttaa attttaatat 360
   ctagaaatca agggtaagga ccaattacta gaatttccac ccttagaaga ataattaata 420
   acaatgtaac aaatgtacag ccccaaacct cttttaaaaa agtttaattt aaaaaattta 480
   atatcaatag ttgaaaaaca gccaaacagc aatgtcaagc tgtagtagtt tgtgattgtg 540
   gcagcttttt gtgttggtca tggatcataa ttctaaacgt tttaatatac agtaatccat 600
   atgaatgcat tgcagaccct gttatcacct gtagttaatg gtgcctgtga tacaagtgaa 660
   caggctgttc tttgctctta cctttatggt aaataaacca gcattctgga gtcacaataa 720
   ttaaataaaa tgtaaatccc taatctcatt tggctatttg gaccaagaaa cttacccggc 780
   tattacctgt ctagttctaa tgattactaa tgatttattg ttcttttttt ttattgttct 840
   aaaagtgctc tgatgtggtg ccttgatgtc taatgaaaag aaattggtct taaaaagcta 900
   ctcattgttt atcctgactc tgagataaca cattttcttt ctgataaaat attttcttta 960
   tttattgttc aaaacacaca atatagacaa taactctaat tctggtgcag ttttatttag 1020
   tagtgtttta tccccggtgc ctggctgtct acagtcatac ctcagcacat agctttactc 1080
   tgagataagc cctctctcct ttgctctttc tcccatgctt tctggagttt gtctgcagga 1140
   tgcaggtttt gaactgaaga gcgcagtgga gttcttactg tgcctttccg gttccagcac 1200
   aatcaaagtt ttgtgtttgg aattgtcctt gacattcttt cgtcccacaa tggggtggac 1260
   acagtgtgct ggaaaccatg acatcattat cattgacaca gacttaaata aatcacctgc 1320
   atcttggcct ctttctgctt attcaaggat tcttcatctt aaggaagaaa gtgctcttta 1380
   tttttacaaa tcttcatccc aggggtgact ggaagacgca aacttttaca agtgttttaa 1440
   aaataaatat actatgtggt tttagacaca gtatgctgag tcagaggtgt cttagagaat 1500
   gtttaaggtt agacataagg cagaaagaca gccagattgc ctttaattcg gaggcaatat 1560
   tcctaaactg tgtatcatta tgtccttctg aatatggatt cacttgaaac ttaattaaat 1620
   aggcatatac ataacatttc aaatacatac ctgaaaaggc ttagtagtgt ttcattttca 1680
   cacattgagc ttgatattca aaaaagaaaa agattgatgc tcagtgaatc aaagcaaagt 1740
   actttctagg taaaagtgac accatctcat ccaatgtctc tgaaagatct cagttgcaca 1800
   gtctttggat aaaaagggtt ataactaaat aatactacaa aatgcaattt acacaatgtg 1860
   ctgactacac atttgtatac ccagcattta ttaatttggg aagcaaccaa ctactgtaac 1920
   ttctctttac tttttcataa gaaaatgaca atgaggaagg aagtgcagca gaaatcttga 1980
   acattaccac tattatgcaa aaaaaaaaaa aaaaaggcca catgtgctcg agctgcag 2038

<210> 211
<211> 1690
<212> DNA
<213> Homo sapiens

<220>
<221> misc_feature
<222> (1)...(1690)
<223> n = a, t, c or g

<220>
<223> nbla-10500

<400> 211
   gtacggaagt gttacttctg ctctaaaagc tgcggaattc ctcgagcact gttggcctac 60
   tggagtgttt tttttttgtt gtttttttaa acatttcatg ggtttgtaga tttttggaaa 120
   taatttcagt taatctcagg gcttcccaaa ccttgaaatg gttaaacacc aaccactgct 180
   taacttggtt gttgtaaaca aagcccgaaa tgaaacccct gtgtatttgc tgcagattcc 240
   caggtgcctc ttaagtgtga ggataagctc agaaaaggct taataggttt attaagttgt 300
   catccattaa aaggttcaca tgctccttgt gtgttgcata aggacaggtg tctgccacct 360
   cattgcattt ctcactgagc attgcctagg agagggtaag ccctaggatt tagatccctc 420
   tcagatgttc aggcagaata attttgtcag gacaaaagta aggtcacgga actatatgaa 480
   aatggattat gaatcctaag acatcagcct attctcccct atgtctaaga agaatattcc 540
   tcagcttatg actggaatgt cttgctaggg gtgctgggta agttttgtgg aggtccccac 600
   cagcagaaaa aaatcattga agaggaacac ttaattcaca atggctgctg attaaaaaga 660
   aagcctcaat attcacctat aaccctaaca tccttaacag cttagcagtg tggtaactgg 720
   cacaaactat ggcgtcttgg tcaaatccaa cctcagtgaa ggtagttcac atcctagaga 780
   tgagctgaat gctgatagct ttgaggactt gtttatttgg gggaagggaa agagatggtg 840
   agggtagaga taggaatctg tctgtcgtaa taggatttgg tctgtgtatt aatggaatga 900
   actggccttt tttgctctgc ttcttttgtc cttgctgaga agggaggctg aagtttccaa 960
   gagttatgga tgaagttgga gagtattaga ggaactgttc tctgaccttc ttgatggtaa 1020
   tgcaagctgt tgggcgagtt tggagaagag cgcagggtgg tgtgaggacg agggccgtat 1080
   taacattttg ccttaaaaca ggaatacaca aattaaagca ataactgcta ctctgctttc 1140
   ttgtttctgc aatcttctgt catttgacat tgttttttcc aaagcaacat gcgaccattt 1200
   tcttcaggat tttcttttca gttatcatat gggtttgttg ttggattgag aaaaaaaaat 1260
   ctgcctcatt tgagcaggcc tctctagatc ttttcttatg actgtgtaga gggtacttcc 1320
   tcatttttct tcctctcaaa gacatcaaat tcccgagcat ttaactaaaa ttattatcaa 1380
   ctaaaaatgt tttttttaaa acagttctta gaggtaactg tttagactta attattgaaa 1440
   tgcatgaagg ccttctgcct tgcccccatc ccactctttt gcctgcctct ctcaccaaca 1500
   aaaacatggc agaggaacag tttattaaca atagcctgtt tgaaagggaa gcttcagttt 1560
   ttagctaatt atttgctaat aaaagtttta aaaaaatttt cttttaaaag ttgtaaaacc 1620
   tctgtctata tttcaaaacc cctcaatacc aaaaaaaaaa aaaaaaaggc cacatgtgct 1680
   cgagctgcag 1690

<210> 212
<211> 1965
<212> DNA
<213> Homo sapiens

<220>
<221> misc_feature
<222> (1)...(1965)
<223> n = a, t, c or g

<220>
<223> nbla-10512

<400> 212
gtacggaagt gttacttctg ctctaaaagc tgcggaattc ctcgagcact gttggcctac 60
tgggagagaa ttcatacagg taagaaaccc tatgaatgta aggagtgtgg gaaagctttt 120
agtgtatgcg gacaacttac ccgtcatcag aaaattcata ctggtgtaaa accctacgaa 180
tgtaaggaat gtggaaagac ctttagactt agtttttacc ttactgaaca cagaagaact 240
catgcaggta agaaacctta tgaatgtaag gagtgtggga aatcatttaa tgtgcgtgga 300
cagcttaatc ggcataaaac aatccatact ggtataaaac cttttgcatg taaggtgtgt 360
gagaaggctt ttagttatag tggtgacctc agagtacatt ctagaattca tactggagag 420
aaaccatatg aatgtaagga atgcgggaaa gcctttatgc ttcgttcagt ccttactgaa 480
catcagagac ttcatactgg tgtgaagccc tacgaatgta aggaatgtgg gaagaccttt 540
cgagttcgtt ctcaaattag tctacataag aaaattcata ctgatgtgaa gccctacaaa 600
tgtgtacgat gtgggaagac ctttagattt ggtttctacc ttactgaaca ccagagaatt 660

cacactggtg aaaagcccta taaatgtaaa gaatgtggaa aggcctttat tcgtagaggg 720
aatcttaaag aacatctgaa aattcattct ggtttaaaac cctatgactg taaagaatgt 780
gggaagtcct ttagtcggcg tgggcagttc actgaacatc agaaaattca tacgggtgta 840
aaaccataca aatgtaaaga atgtgggaag gcctttagtc gtagtgtaga ccttagaata 900
catcaaagaa ttcatactgg tgagaaaccc tatgagtgta aacaatgtgg gaaggccttt 960
agacttaatt cacaccttac tgaacatcag agaattcaca ctggtgagaa accctatgag 1020
tgtaaggtat gtagaaaggc ctttagacaa tattcacatc tttatcaaca tcagaaaact 1080
cataatgtaa tttaatataa gaaaaggttt ccatgtcatg ctctatttat agaatatcaa 1140
aatatttatg gccagaagtt ctgtcaatgt gttgatgttt ttttacacat attaacttaa 1200
taaatgtatg agtcttaaat acctcttagt tctcattaaa tttaggaaaa ttcacactag 1260
aaaataaagc tgttaatgta acagttgtgg agaagtgttc tagcaacagc atatacttat 1320
catcattgcc tttccactac tctactatct gtgtgatatt agacaaaata tttgcttctt 1380
ggtacctcag ctgtaaaatg aaacacacct aaaagtgtgg ttgtttccaa catgtataat 1440
acagcaacaa ctatctggcc caaactgctt tggattaata ttggatatta ctgtttttat 1500
tatcatcaac attattatta gtggatttct taataggaag atgcaatgga gatgacaaat 1560
ttggaaaaac cactcatcac ttacatttca tgaagtactt ctttgataaa atctgttatg 1620
ggctgaatgt ttgtgttccc gtaacaattc ctatgttgaa acacgaatcc caaggtgatg 1680
gtatttgaag gtagggcctt taggaggaaa ttaggtcatg agggtggagc cttcatgagt 1740
ggaattactg cctttataag aagaagccaa agagccagct agctctttca accacatgag 1800
gttacagcaa gaagtcagca gtctacagtg caaaagaggg ccttcaccag aacccaagca 1860
tgctggcacc ttgaccttgg actttcagcc tccaaaactg tgagaaataa acctcagttg 1920
tttataaaaa aaaaaaaaaa aaggccacat gtgctcgagc tgcag 1965

<210> 213
<211> 1980
<212> DNA
<213> Homo sapiens

<220>
<221> misc_feature
<222> (1)...(1980)
<223> n = a, t, c or g

<220>
<223> nbla-10520

<400> 213
gtacggaagt gttacttctg ctctaaaagc tgcggaattc ctcgagcact gttggcctac 60
tggcaaagca ggttattcag tgattcctcc ccaccattct taagaacgtt aaataatgct 120
gttgtgttag ctctgagtag aaaggaaaaa gtaaaacctc tgtttggagg taatattggg 180
ttgaattctg actgcccctt tctagctgga cctttaacaa atcacccaat ctttttgtg 240

tttctctaaa gtcatttata cattaaatgt aattatagca actgtggggt tctgttgaga 300
attaagagct aacactatat atgtaaagtt tccagtacta gtcccagaat ttagaatatg 360
ctcaacacaa agtaaacagc attatataag tttatatttt tgtgagttat aaagtacttt 420
gatatattct cattaaatct gtaaatcacc tctataagta agtggtaata ataaagcaga 480
tatttttgtc cccatttaaa aaatgaagaa attaatgctt aatagggtgg taccctggaa 540
aggatctggg aagtggtaga atttctggtc tgtactttta caaatggagc ccttgggagg 600
tgggttaggt aaaagaagct ttttacttaa cgttgtctta tttccagtct aattttacgc 660
tgtagcagaa ccagatggct gagaaaattc tggaactatg gatcttgacc ccaaggatat 720
attattttat tccaagaaag atcaggtagg cgaaaagatg acaggataca gagtcaatcc 780
ataaactaaa tatttataac tgttctgaat tatacagagt ctaaaaatat gtgtcagcta 840
cttcattcct gtaaatactc ttgctgtgtt ataaatatgg caagaaataa acatgaccaa 900
tatcaataga cttcttgagg ctactataag ttttgagaaa taaggttcaa aaaataagaa 960
tgctaacact taagcacaga ctagagcttg cttgggtttc ttcctgcatt acaaggtaaa 1020
aatttgttaa tgtttgtttt tattcagctt gggaaagctt tgtgccatga atacgtcgca 1080
tttaataaca agcaacacac ggcatataga aataacttta attaaaaaac ttacatagaa 1140
gattataata tcagacgtga caaagatttg agtttatttg cctggacaac ttgggtttgt 1200
ctggcttttg ttttcttttt ctttaaaaat aaatgtacag taaaactaca agcaaaagtt 1260
tgtcagtatt gaattgaatt ttttacccct taaaaggact agtataattt ccaatctcta 1320
acaaaaactt agtgtcaaat ctcacagata aggccaaatg gccaatattt tcagttatgt 1380
gggtagtaca acttgagtaa ccttttttac atgacaaaaa gtgagttata taaattgtcc 1440
tcaactttca cataggaaaa aaatggttta atagcttcaa aaggaatttt ctttcatgta 1500
tactcttcag tatccaatat tgaagctttg ttctttgaaa aattttaatt tccaatctag 1560
gatgcaagca agaatatatg tttatttgaa tagagtaagc tatggcaaag aatgaccaaa 1620
ttagctagaa atagaaatca gccagaatta actaatttct tgctaatcta gaaatacaat 1680
catctttttt tttttcaaa ttttatactg atagggcttt actgtttgtg gctcatttta 1740
aaactggtgt cttctcttca tgagacacat taattggtaa aactcaaatt gagttttcaa 1800
agatgtgata gtattaaagt gcaccaatat ttgactcaaa tttgcttgct ttattttgtt 1860
aggagtaaac agaaagtagc ctgtgtttag tcccaaagat agcagtgatt ttgaataaag 1920
gagttttgtg ttgcctggaa aaaaaaaaaa aaaaaaaggc cacatgtgct cgagctgcag 1980

<210> 214
<211> 772
<212> DNA
<213> Homo sapiens

<220>
<221> misc_feature
<222> (1)...(772)
<223> n = a, t, c or g

<220>

<223> nbla-10522-f

<400> 214
gnnngnngtg ggntcnnnnt tgaanccctt ggaatgnccc ctactggact gaaatatcag 60
ctcatagaaa gtaacaaaat ttgctgtcac cttaaataag acattttaat tttgttataa 120
tgtacaattt agaagtttga ttaattatat tatctattta ggcattaata taaaagaggt 180
aggagtctgt tatttaaaaa aagcattaaa tttaaaaaaa aactgtcttg tctactttta 240
gcttcattct cccatatttt gaagggtgtg taacttcagc tctgcaggat tgcatggggt 300
aaaacttgtt accaacacat gtgaaccatt gctacattgt aggttgtgat cattttgccc 360
cactgaagcc catgtatctg accttacgtg ccttttgaac taggagaatc gggctaattt 420
attaatgatg ataattataa tgtatctgta cagcactttt tacatttgcg aagtgctttc 480
caatccatgt tagttactaa gttattacag ctgtaaggat aaaacacgtc atgtggattc 540
attttgaatt ggtgctattg gtatttcctc tgntattgct aataaatgaa aatggtggta 600
tgaaaaaaaa aaannnnnaa aaaaaaggcc acatgtgctc gagctgcang tcgcggccgc 660
tagactagtc tagagaaaaa acctccacac cttcccctga acctgaaaca taaaatgaat 720
gcaatggtgg tggtaactng gttattgnag cttataatgg gtacaaataa ag 772

<210> 215
<211> 769
<212> DNA
<213> Homo sapiens

<220>
<221> misc_feature
<222> (1)...(769)
<223> n = a, t, c or g

<220>
<223> nbla-10522-r1

<400> 215
gagngggntn natttgancc cttgtnannc cttttttttt ataccccatt ttcatttatt 60
agcaataaca gaggaaatac caatagcacc aattcaaaat gaatccacat gacgtgtttt 120
atccttacag ctgtaataac tagtaactaa catggattgg aaagcacttc gcaaatgtaa 180
aaagtgctgt acagatacat tataattatc atcattaata aattagcccg attctcctag 240
ttcaaaaggc acgtaaggtc agatacatgg gcttcagtgg ggcaaaatga tcacaaccta 300
caatgtagca atggttcaca tgtgttggta acaagtttta ccccatgcaa tcctgcagag 360
ctgaagttac acacccttca aaatatggga gaatgaagct aaaagtagac aagacagttt 420
ttttttaaat ttaatgcttt ttttaaataa cagactccta cctcttttat attaatgcct 480
aaatagataa tataattaat caaacttcta aattgnacat tataacaaaa ttaaaatgnc 540
ttatttaagg tgacagcaaa ttttgntact ttctatgagc tgatatttca gtccagtagg 600

ccaacagtgc tcgaggaatt ccgcagcttt taaagcagaa gtaacacttt ccgtacaggc 660
ctanaagtaa aggcacatca ctgnggagca gtctttgatt tgaccaccac cggaatccgg 720
gcctggaata aaagaccaaa ngactaaact tccagttaac ttttgggtt 769

<210> 216
<211> 2755
<212> DNA
<213> Homo sapiens

<220>
<221> misc_feature
<222> (1)...(2755)
<223> n = a, t, c or g

<220>
<223> nbla-10535

<400> 216
   ttctaggcct gtacggaagt gttacttctg ctctaaaagc tgcggaattc ctcgagcact 60
   gttggcctac tggcgtacgg tgttttcttg tgcattgact gttccggggt gcaccgctcc 120
   ctgggcgtcc atctgagctt catcaggtcc acagagttgg attccaactg gaactggttc 180
   cagctgaggt gtatgcaggt cggcgggaat gccaatgcga cggctttttt tcgccaacat 240
   ggatgcacag ccaatgatgc caacaccaaa tataatagcc gagctgccca gatgtaccgg 300
   gagaagatcc ggcagctggg gagtgcggcc ctggctaggc atggcactga tctttggata 360
   gacaacatga gtagtgccgt tcctaatcac tccccagaga agaaggactc tgatttcttc 420
   acagaacaca ctcaaccccc tgcctgggat gcgccagcca ctgagccttc agggacccag 480
   cagccagccc cgtctacaga gagcagtggc ctggcacagc cggagcatgg ccccaacaca 540
   gacctgcttg gcacctcacc caaagcctca ctggaactga aaagctccat cattggcaag 600
   aagaagccag cagcagctaa gaaagggctg ggtgccaaga aaggcctagg ggcccagaag 660
   gtgagcagcc agagcttcag tgagattgag cggcaggctc aggtggcaga gaagctccgt 720
   gagcagcagg cagccgatgc caagaagcag gcggaggagt ccatggtcgc ctccatgcgt 780
   ctggcctacc aggagctcca gattgatcgt aagaaagagg aaaagaagct acagaatctg 840
   gaagggaaga agcgagagca ggcagaaagg ttgggcatgg gcttggtatc ccgaagctct 900
   gtctcccact ccgtgctgtc tgagatgcag gtgattgagc aggaaacccc agtgagtgca 960
   aaatcctctc gctcgcagct ggacttgttt gacgatgttg gtactttcgc ctctggaccc 1020
   ccaaagtaca aggacaatcc cttttcctta ggggaaagct ttggctcccg ctgggataca 1080
   gatgctgcct ggggtatgga cagggtagag gagaaggagc cagaagtgac catctcaagc 1140
   atccggccta tttcagaaag agccacaaac cggagggaag tggagaaccg gagctcaggc 1200
   ctcgagtcta gtgaggcgcg tcagaaattc gcaggagcca aagccatctc atctgacatg 1260
   ttctttgggc gggaggtgga tgcggagtat gaggccaggt ctcggctgca gcagctctca 1320
   ggcagcagtg ccatcagctc ttcagacctc tttggggaca tggatggagc tcacggagca 1380
   ggaagtgtat ctctggggaa cgtgctgcct acagcggaca ttgcccagtt taagcagggt 1440
   gtcaagtctg tggctgggaa aatggctgtg ctggccaatg gtgtgatgaa ttccttgcag 1500
   gatcgctacg gttcctactg atccgagctc tgtgactcag gcttacgatg gtgacggcaa 1560
   caagaactcc acagttccca ggctggggat gctttgcctt gtggaagctg gggaggattt 1620
   gttacttcgt atgtgtggtg tgtgtgtggg gtggcctttg aggcgctcac tcctgtgagg 1680
   ggaatggtca gtaccagccc ttgtcctctg cctgtggact gagcccttta ttccctctca 1740
   caccaccctc cgtgtgttag actcttgtcc ttctgtcctg cccccacagc tgctgctcac 1800
   ttatcctgcc atactgggaa agggggttcc cccacgatgg cttattctgg gtccagactt 1860
   tccccaggta gggaaagcgg aaggtagaag gctttttttg ctggctctag ggttcttcta 1920
   gttcgaggcc ttgggtcccc atcctctgga accaggggga ggcctggaag gagttcactg 1980
   tagacccgtc ccatggggaa agaggctgcg gacttgctgc tgctgctgct gccagtggcc 2040
   tcttctgggt gccaggagag gggaaggacc tttgtctggg cgttaccaag ggctggaaac 2100
   tttacctggt acctaaaggt ttcatttggt atcagaccgg agacccttgg gttctcccgt 2160
   ctcaccaccc ctttctacag taagcacttg gaagattgtt tcagggtgtc tcagggtccc 2220
   tctgtaccat ctgctgtgga atgcaggacc ctctgtgaca ttctttatcc cttcttcccc 2280
   gggttggtgg ccatggaggg tcttgtctgc tgtgattcga ctctggatgc tgtgagcttg 2340
   atgctggcca gggaagcaga ggatgtgaga ggcagaggca ggctcctggg gctgagctcc 2400
   ttcctctgca tcattctggg cttggcctgg acagcacccg ccagtgagag ctgtgggcct 2460
   caccctctgg cagctgagcc aagcactgtc attcttggtg ccatcttccc ctgccgcacc 2520
   ggcagtctca gcccagcccc cacctttggg ttgtaggttg ggctcccaag caacacagac 2580
   cactcttccc cttgcccctc ccccagaggg acttgacttt ctttctggac tgtttgtatt 2640
   gaaacaaagt ggtgtcaaaa taaagcccct gcagggcctg gctccctgtt ggtctgagtg 2700
   aaaaaaaaaa aaaaaaaaaa aaaaaaaaaa aaggccacat gtgctcgagc tgcag 2755

<210> 217
<211> 2100
<212> DNA
<213> Homo sapiens

<220>
<221> misc_feature
<222> (1)...(2100)
<223> n = a, t, c or g

<220>
<223> nbla-10545

<400> 217
ttctaggcct gtacggaagt gttacttctg ctctaaaagc tgcggaattc ctcgagcact 60
gttggcctac tggcagcttt ttgtttttgg cctgaataga acaagacaat ttacctatgt 120
gaaaacctga gtgaaagctt ggggtgtgat atgttaagta gaacagtatt ttaaagccac 180

tttgattcat aagtttcttt aacataaaca ggttgtattg cctcttgaaa aatgataggt 240
tctatggata acatttaatt agctttataa tacatatgat gatttaacct gaattaatgg 300
tactactttt tctgacttcc ttttgatctt ggccctttct ctgtaaagtt ttttgttaac 360
ttattacaaa ctgaaccctg gcttgctcct cttatcttta gtctcctttc aatctgtctt 420
tatttctgct gaggttatat tctatttgtt tttcagatga gacccttttt gctcttcctg 480
ctgcatctga gcctgtgata cgctcctctg caggcaagtt ctttctgcca gccttttatc 540
ctctctgtat tctaacttta actctttagt gtgttaacac tatgcagagt agtttgggtt 600
tactaaaaat tagcatggtt ccgagaatgt tttgtctact ggaaaagttg aaatgtgcat 660
tttatatggt atgcccaagg gaaaaatgag caaaatctga tgaatacttg gaagtacata 720
aggaattctt ggacccttga attacattta catatttacc attccctttc tccaaataca 780
gtttgagtat agccagaaag gctttcctgt aaataaatac cagtctatgg catttaccca 840
gtgccccttc tttcctatgc tgtcaccgaa actcttttca cagatattct tctcttctct 900
aaaactagct attagtatct tagttgataa catttgaggt ttactcagac ttgatttgtc 960
tttgcacagc atgtaattgc ctctgtagcc atgcatatgt tatatttaca atagcttaat 1020
attttcagta atgattgaag tctactctat tgttacttat taattgctat taacatttca 1080
ttttagtaaa cagtaagtct ttactaacac tattttggat ctgtcataaa acttgcatta 1140
actctgagaa cactggcatt ctctaacttt cctttctgtc catggtcaca gaaaatatgg 1200
acttgaagga gcagccaggt aacactattt cggctggtca agaggatttc ccatctgtcc 1260
tgcttgaaac tgctgcttct cttccttctc tgtctcctct ctcagccgct tctttcaaag 1320
aacatgaata ccttggtaat ttgtcaacag tattacccac tgaaggaaca cttcaagaaa 1380
atgtcagtga agcttctaaa gaggtctcag agaaggcaaa aactctactc atagatagag 1440
atttaacaga gttttcagaa ttagaatact cagaaatggg atcatcgttc agtgtctctc 1500
caaaagcaga atctgccgta atagtagcaa atcctaggga agaaataatc gtgaaaaata 1560
aagatgaaga agagaagtta gttagtaata acatccttca taatcaacaa gagttaccta 1620
cagctcttac taaattggtt aaagaggatg aagttgtgtc ttcagaaaaa gcaaaagaca 1680
gttttaatga aaagagagtt gcagtggaag ctcctatgag ggaggaatat gcagacttca 1740
aaccatttga gcgagtatgg gaagtgaaag atagtaagga agatagtgat atgttggctg 1800
ctggaggtaa aatcgagagc aacttggaaa gtaaagtgga taaaaaatgt tttgcagata 1860
gccttgagca aactaatcac gaaaaagata gtgagagtag taatgatgat acttctttcc 1920
ccagtacgcc agaaggtata aaggatcgtt caggagcata tatcacatgt gctcccttta 1980
acccagcagc aactgagagc attgcaacaa acatttttcc tttgttagga gatcctactt 2040
cagaaaataa gaccgatgaa aaaaaaaaaa aaaaaaaggc cacatgtgct cgagctgcag 2100

<210> 218
<211> 1815
<212> DNA
<213> Homo sapiens

<220>
<221> misc_feature
<222> (1)...(1815)

<223> n = a, t, c or g

<220>
<223> nbla-10570

<400> 218
gtacggaagt gttacttctg ctctaaaagc tgcggaattc ctcgagcact gttggcctac 60
tggatgcatt tcgaaagcaa acagaagaaa aaagtatatt ctcacagttt ttccccagga 120
agtctggaga aaaagtcctc atgtcaccaa tttctctgtt gcatgaattt tagtgttttg 180
ctataagaca gcatgagggc tatcaggcca aaattcatta ttgtatttcc aatacagttt 240
gtcttgaatg tctgtctctt aaaccagttt ttacttgtca gttttctctc agcaaacagt 300
cttactatta tgtggaattt aacttttcag agaaaatttc caaagtcctc taccttattt 360
ggctttatc tgtgtctttt ggaaaggag ctgcatggcc tcagtgcctc cataaaaagc 420
ttaagttgag ctcatgggag aacattaaga tcgttttttt tccctttag ctcggtaatc 480
tcatgtacca caggtaatga ccaagcatct ctgctagagg atgtacctct cataactcct 540
gtgtaaaatg aggatcctgt tggtgatcct cattccagtt tccaaagcga gaggagcctt 600
ctgagcagaa gtatgcaaga ctgattctgt ttggaacagt ttcctatttg gctgcattct 660
aggaggcctc ctgacttatt catagtattg tcgcctggtg gcattggtgt ggcgtagctt 720
gtcctgctac ttgctaaagt tctctttctt tgagaaaact gaggaaccag aatgcttaga 780
gttcataaaa taatctctaa accatgttag gttaaaacaa aatgaccatc tcacaaaaac 840
gagtttagtt gaatgctcaa ttccttacaa aatcttcatt taaggctgag tgtggtggct 900
catgcctgta attccagcac tttgggaggc cgaggcagga agactgcttg agcccaggag 960
ttcaagacca gcctgggcaa cataggaaga ctctgtctct acatagaaat tttattataa 1020
aagtagctgg ggatggtggc acacacctgt agtcccagct actcaggagg ttgaggtggg 1080
aagattgctt gagcccagga gtttgaggtt gcagtgagct gatcatgcca ctgcactcta 1140
gcctaggcta cagagcagga ccccatctcc aaaaaaaaaa aaaaaaaagt catttaaaaa 1200
ctgaagaatg ttctcttctt gctcagttcc ctaaattagc tggggggatt gggatacttt 1260
acaattttta catttttaaa tttgaagaat ttgttttaaa cttacttggt aatggcactt 1320
ctacttactg accaactttt cagctctttt tgaccaggat ggttgcaaat tagggaaaat 1380
atctttatac tatacatcca attcaagagg actttaatta cttaaaagca tttaggtcat 1440
aattaaaatt attttcactg tttccataaa ataagcatta tgattgcaca ctccttctac 1500
tgtctgaatt tatgtaagag gtatagcttg cttaaaatac atatatatgt aaagttatat 1560
tttcttagaa acatggatgt ttacagccat tgctttcaaa gagattatta ctgtgtattc 1620
tccctgtttt acaatatgtt ttcatgaaga ctatgttaca ccagaggctt ctgattatca 1680
aagtgataac cagttttaac tgccggtatg taccagattt gtgaactaat ttaaaaaacc 1740
acaaactatg aataaaatgg ggtttgcaaa ctaaaaaaaa aaaaaaaaaa aaggccacat 1800
gtgctcgagc tgcag 1815

<210> 219
<211> 730
<212> DNA

<213> Homo sapiens

<220>
<221> misc_feature
<222> (1)...(730)
<223> n = a, t, c or g

<220>
<223> nbla-10594-f

<400> 219
cnnnngcctg ttggcctact ggttgacttt ctatttgaca agtagttata attggcaagc 60
agtttgagtt tatactcatg acatactgaa agcatctctt tggggtcaag aaagaatccc 120
tagtggattt gggattctag aggaggtgtt ataattaatt attcagaaaa atgcatgtat 180
ttacctcttc aaccaaaagg gcagtaaaga atagggataa tatattttaa tgtaaatttg 240
gaagaaggat aaaatcaccc acagttactc tccctattat tttcactggg tcaagtttta 300
atcttttggc ttcatgagtt ccactccctt tgatgatata tgttgtatat ttttagttgt 360
aaagcagtaa tctttaactg tagcattcct ctaattataa acagtgaaaa atcaaaatca 420
gtccagagtt cttttgttga cttaattcct gatcagtata gacaggacta tatatattta 480
atcagagaat tacattatat ctgaagatgg agctcaggag aatgcaccaa gactttgaat 540
tgnatttaca ttcactgtca gtaaagacct ggctacgctt tgagaaatca ggatctncta 600
ggccattgct tcgcaactgt gattactgaa atcctnctca taggagataa agtccgangg 660
gtagagtcca agatgtcacc ttggaatttc tttggaagtc acatgtttat ccttaaaata 720
ttcttattta 730

<210> 220
<211> 763
<212> DNA
<213> Homo sapiens

<220>
<221> misc_feature
<222> (1)...(889)
<223> n = a, t, c or g

<220>
<223> nbla-10594-r1

<400> 220
   gnngnngnnn nnnnttntgg gcctttttnn nnnctttttt tttttagcac tttctacaaa 60
   aaatgcttta tttaaaaata tgatcttttt aaaaaggaga cttttataca cacttacttt 120
   agaaacagtc aaaaacacaa cagaatcaat ccagatttag aaatgatgat ttgtaacata 180
   caatattggt atgctgaaca agacagttta aatgtatgat tctttttgtc ccttcacatt 240
   tcaataattc acatttgtta aaactagtgg ttgggactaa agaaaaggtc atatctgaat 300
   cctttcccct tcagaaatct gtcacccttc tgggcagtta cagaaatggc agcagctagg 360
   caatgtttgt atttctttgg gtgcctctgt gtcaaaaaat atatctccgc ttttattctt 420
   tttctatgaa atttatattt atgtggctat agggcctggt cgtatgtccc acgcctttta 480
   aaatctaaat ttctaaaaac agacactccg taagggcaag gagcatacac tgctatgacc 540
   tcactggact cagagcttaa tgctcttagg ttatcagcaa gagacatgat tttgatcttc 600
   cagttagcac tccaagctgc tatcaaccaa cctttttcag cacggtagtg aaatatttca 660
   aaccatattc aaacatctaa tatagattta taatnaaaca ggaaaaatgg cttgncaaat 720
   taaagngcta tgagctttta cattcaanan ggtatcttga ntt 763

<210> 221
<211> 797
<212> DNA
<213> Homo sapiens

<220>
<221> misc_feature
<222> (1)...(797)
<223> n = a, t, c or g

<220>
<223> nbla-10623-f

<400> 221
gcactgttgg cctactggcg atgggcgagt agtcagcaac ttcatcctgc aggcgctcca 60
gggggagcca ctcacggtat acggatccgg gtctcagaca agggcgttcc agtacgtcag 120
cgatctagtg aatggcctcg tggctctcat gaacagcaac gtcagcagcc cggtcaacct 180
ggggaaccca gaagaacaca caatcctaga atttgctcag ttaattaaaa accttgttgg 240
tagcggaagt gaaattcagt ttctctctga agcccaggat gacccacaga aaagaaaacc 300
agacatcaaa aaagcaaagc tgatgctggg gtgggagccc gtggtcccgc tggaggaagg 360
tttaaacaaa gcaattcact actttcgtaa agaactcgag taccaggcan ataatcagta 420
cattcccaaa ccaaagcctg gcagaataaa gaaaggacgg actcgncaca gntgaactcc 480
tacttttagg acacaagact accattgtnc acttgatggg ganngtattt tttggctntt 540
ttttgntgtc gtttaatgaa angactttac aggcgtcatg aagaacnaac tnggantttc 600
attctgaagt tggctttaat gaaatggatg tgccctaana ncntcccttg aanaactnna 660
gaattttgcc ntgnancntt tgnaattttc ctnttttgta aaatagcntn antccatntt 720
ttgcgnnttt gtaannnttt ttattttgct ngnnanagca ttttnngnnc tnccgttnac 780
agtcttattt aantgnn 797

<210> 222
<211> 811
<212> DNA
<213> Homo sapiens

<220>
<221> misc_feature
<222> (1)...(811)
<223> n = a, t, c or g

<220>
<223> nbla-10623-r1

<400> 222
gnaggggnnn nnttgggcct tttttttttt ttcntgactg gtgagtttca atattttatt 60
aaaatagcat atttaaccat ggttaaccaa gaatatgtta actttgtgcc ccccaacctg 120
cccccgcccc agatacgcag caaaacacag tagtgatttc aaatatcctt gatcatgacc 180
aacaggaata tttcaaagtc aagtttgcaa ctcaaaagta gaccttggaa aaactctgaa 240
ttaaaagaga aacataaagc tctcacacag aattctttta tctgccctaa aatcaatgcc 300
ggcgcaccac cacctgacaa tactgctagg atttttcttt atgcatagta taacgactta 360
tttattaagt acccactttt ataaataaaa tcggcatttt ttgtcccgct taatgttcct 420
tttcagcttc acaaagaaac cagtaaataa aactgtcaac gacagtcaca acatatgctc 480
tcacagcaag ataaaaaaac ttgaaaatac gcagagatgc atctacgcta ttttacataa 540
aaagagagat tcaaaaggtg caaggcaaaa tctgcagctt tttgagggga gcttttangc 600
acatccattt cattaaagca agcttcagaa tgaaattcca gtttggtctt catgacacct 660
ggtnaaggct ttctttaacg accaccaaaa aaagnccaaa atncttccat aagtgtncat 720
ggnagncttg ggnctaaaag tgaggagtca actgtggcga gtccgccctt cnttattctg 780
gaggctttgg nttgggatgt ccgatatttg c 811

<210> 223
<211> 825
<212> DNA
<213> Homo sapiens

<220>
<221> misc_feature
<222> (1)...(825)
<223> n = a, t, c or g

<220>

<223> nbla-10624-f

<400> 223
nnnngnagnn gnnngtgggg nnnnnnnttt gattcctgtg cctgttggcc tactgggaag 60
aaaggacaca agataaacaa taatttggat aattcgtaaa ttggtatttc attcttgact 120
tcttgagaat tcattgtata cgctactcct caaaaataag gaaaaccata aacaaaaatt 180
ctggtctgaa atatcttggc agtaatgtgt ttgctctccc ctgtcctgaa tattttaggc 240
taactttcaa ttaaaatgga ttgcaattaa attattttgg gcagaaatgt gtcttaacct 300
ttgacaaagt tgatcatttc agcattttca aatgcttaaa ctatttctta aaatgacaca 360
aattgtggcg ttgccattta ccgaggttta catgctcttg atgcttaagg attcggcttt 420
ctttttcccc ccgcatttat tcactgaaaa catgattgat tcagttttgt acctatgcag 480
aatgccactg acgtgatgcc agatatgccc tatttagaaa aaataaaaat taaaaggata 540
tttttctgca cagtcaaaat tatgcagatg tggatttaaa agactacatg tcaaaaaata 600
cacangtaaa ggttacttac tncatggttc cttttacact cacagacagc agatttacag 660
gcaccaaaca gccttcatca aaacaatgaa atgcccaatg tggctctttg atgacttcat 720
tctggctttn acgtatgccc aaaccctnat aattgcaggt gctttttctg accaattatt 780
tgggggctct ggggtatttc caggcatgcc tangncantc tggtt 825

<210> 224
<211> 829
<212> DNA
<213> Homo sapiens

<220>
<221> misc_feature
<222> (1)...(829)
<223> n = a, t, c or g

<220>
<223> nbla-10624-r1

<400> 224
gngtgggggn nnnnntttgg ggccnttttn tnnntttttc ttgctaggaa tgagtctaca 60
aaatgcagga gagcaaacca aatagacaaa tgtaagggaa tattgtctaa ggggactacc 120
agatctccaa aatgccacta ctttttgtat cctactattc tcaacattct gtgtcagtta 180
cctgtgggct ctaagattca cgtacagaga agaaaaaatg gatgagcatt aaagacaaaa 240
aatgaagatg tcatagtaga aggagaaata gtaagttata aggaatagat tagaaataga 300
tttaccatga ttaatctatt atgctagcaa acttatatag agccatgcat tagtagattg 360
attagataaa tcttatgaat ctctttattt gaaaaccaaa agtattacat tgccactcag 420
ttttggacct ttactcacga gtatctgatt cttgctcctg gttctgaatg atacttctac 480
ttagaactgt gcctcagatg tcacacagct cccagataaa tgagggtaat caaagttctt 540

gaacatcagt gtttctgatg ttcaagatag taggtgtagg acagtcagga agggaacaca 600
gaccttcaca aggcttataa gagtttctac atatgaactg cagagattga ggtgctttcc 660
ccaaccacac gtgggttcct taaagacaaa gacctaaaaa atgaggcact ggcaaatca 720
taaacaggct ctttcggaag atcagtttat cagngnacaa atacacttac tggttaacta 780
tatctttcan ggnattcttt ctctatttna aatgggactt gantttaac 829

<210> 225
<211> 2547
<212> DNA
<213> Homo sapiens

<220>
<221> misc_feature
<222> (1)...(2547)
<223> n = a, t, c or g

<220>
<223> nbla-10635

<400> 225
ttctaggcct gtacggaagt gttacttctg ctctaaaagc tgcggaattc ctcgagcact 60
gttggcctac tgggaaaatg cattgacttt gtagtcttta ttctcctagc cgcacttcgg 120
gtatatccag aaggaagagg aaatgcaaag aggaagaaga tgtaatagtt cttctctcca 180
gctaggtgca cttgaggttg ttcataaatg taaaattatg tcaggtttct aacatgggac 240
actgcacaca gttgtctgac ctgatgaacc atcccatttg aaagtataga ttattattat 300
ttcttgtagt atttggttgt tttccatctc attcatgaac aactcaacct gatagtagta 360
tccaataaat gcctttcagg gctcaggaat gaattgacat cctagttaag aaatgagact 420
taataatgga gactgaggcg gtttgtatta aattatatgc catgaagtgt tcattttagc 480
tttaacctaa ttatgactgt accaccatga agtacagaat gaaaaattat atatatgggg 540
gggaaacaga atgaatatct gattcttttg aatgcttgtg gaaatctttg agatcgtgca 600
gggcatacca caaaatagcc tttagaacag atacccaatt ttacagttca taggacaaca 660
tcaaacatta gtaagtctaa ataagatgaa tagaattttt gttatgtaaa ttttgctaga 720
acagtctatt ttcttgcacc cctcaagtta acctcttaaa aaaatgaatg tataatttct 780
accgaaagaa tatcagagag aatctctctg gcctatagtg ttaaaatatt gttcacaaat 840
cctgattagt taagtgcata cattatgaaa cttacagaat aaaacttatt atacatctct 900
ttcttaaatt aatatcttta cacattttca actggctccc caagtctgat aaggaaggat 960
taaaagaaaa aagaaatgta ttagttgggt ggccaaggag tttcctttgt aatgttgaga 1020
gacttccgct ttctgaattt cgctggttct ctaaggtaaa agagttaaat agtacccttg 1080
ttcaccaagg aaagtgatcc aaactatata tctagtgcag atatttcctt tgcattattt 1140
agtcttctct ggagagaaaa tacagtttcc ccttcctctt tctcttcaca tttactcttt 1200
tcaacccaaa ataagagaca tagaaagcaa accacagcca gtttggcatc ttctcagtgc 1260

tactagtata ggcacataca catacacagt ctcagcaagg ttataaagaa ccctgtcagg 1320
tccacttgca acatggcctt gctacttgga ttagctcctt taagcctgaa aataactttc 1380
ctggtcatgg aagaactgga cgcatctttt aacttatgaa atagaagttg aacttgaaaa 1440
ctctttttaa aaaatcctgg ttttgcagga cagctacata atgaatgtat atattaagac 1500
tgtagctgaa ttgcacatga aatcagattg ccaacttctt gactttcaat gttagacatt 1560
tatccttaag ttgtgagcga tatatgtagc atgctgtgaa atgtctgtta tagctcttta 1620
attcatcagt attaatacag aattatcatt tgcgtttctt ggtacttttt attcaatgta 1680
atcagaagct gtgatgtttt gcctttgtag tcctgtgctt tgttactgta attttttttt 1740
ttttttttta cgaagcacgt gactggacta atgtaaggca gatgacgtga tctttaagac 1800
tgctatatat atcagtctct tactctataa ggttttaaat tagaataagc ttttatcaaa 1860
tagataattg atgcaattta ggattcacgc aagtttcagt gtcaaatggc ggtcttatag 1920
tttcaattct gaaaatagca aacttaataa acagccactt taaacttgtt ctggcaaacc 1980
agaccctgct gtagatatag tctaaggtag ttaaccatat aagccttttc aactcttaat 2040
gccctccaca tgaatcagca gttaagaagg ttctagaacc catgaaagct tttgtatgta 2100
ttactaggtt ttgtttttct tatgtttgct gattttacag ttctgactaa agctgaccta 2160
aatggatcag tttatgtgta atattctagt gctttaatga ctcttttttt ctttggaggg 2220
agggtaacat tatttggaca gatgcagaag gaactgttag tgagtcaaga caaacacatc 2280
tgaaataaag gaactgtgta ttaacatgtt aacaattcat aactgcactt tttatgacat 2340
tttgaaaatc tatttatagg tacagaacaa tgggttttgt taaactgtat cacatttata 2400
cttgcagaaa tttatttcat tgttattagt aggaatttta ttggttcaat aaaattggca 2460
aaactgaaca ccaaaaaaaa aaaaaaaaaa aaaaaaaaaa aaaaaaaaaa aaaaaaaaaa 2520
aaaaggccac atgtgctcga gctgcag 2547

<210> 226
<211> 823
<212> DNA
<213> Homo sapiens

<220>
<221> misc_feature
<222> (1)...(823)
<223> n = a, t, c or g

<220>
<223> nbla-10667-r1

<400> 226
ggggggntnn nttganccct ttnganncc ttnnnttttt tttttttag ttacatagca 60
tctaagtttt ctgatcacta ccaggtaatt ttcaaccaat aagaaaaagg aaccaacact 120
cagctctgta gaaatctacc ttcttttaga aacctcaggc ctctgcaccc ctttagacaa 180
ctcatttaca accacaacct tccatggctt ctcacatgcc agccagggca gggtaaaaac 240

ggcaatttct tacaccgaaa ggtccttttt atgtaacaaa cagacctncc caaaccacaa 300
cttttttgnc ctattgatga ctcgcatgag atcaaatctc tagtgtcccc tgactgcccc 360
gacagggtct aggacaacac aggttaaaaa ggacagcagg gtccgttcaa ctcccacact 420
gnctcaggtg ttttctcaaa gcacgacaat tctgcaatgg agacttacac gtggaaaaga 480
atcaaactgt gcaggtaccc tcagtcctnt gncactacca gctcaatagc ttgagctcag 540
aggatcaagt caagnctctg ctcactttta atctagcaca caagangcca actcagcagt 600
acacggnatt gaagcatgca caggcattgg tacaaaactc agttagatta agagtccnta 660
aataaacctt gnagntgagt agaaatccca aacagntggt gangacaggc tgtangctaa 720
ctttacattc aaggttttat cncccagnag gcagattcca aatgntagat attggnccgg 780
aaaatttcn gggnaaagaa atcntnttnc nctttatggc cng 823

<210> 227
<211> 838
<212> DNA
<213> Homo sapiens

<220>
<221> misc_feature
<222> (1)...(838)
<223> n = a, t, c or g

<220>
<223> nbla-10672-f

<400> 227
gngngggtng nnttnnnatt tgaantccnt ttnctgttgn cctactggtt caaaaccttc 60
aagtgatatg tggaaaaaag tgaatgagac ctctgatagg gggttttcag aaccttgttc 120
acaccaaaat gtgacagttc tttcatgttt tcctaaacca agttaaaatt acatgtatat 180
tttggtgtta aggttgattt ttaagatact tctgatttgt acaaaaggaa tgtttccttt 240
ataaatcaca gaagaaaatg acaatatctg ttggatattt gatataattt aatggtgtta 300
taaaaccttt aagaggattc atggtgaata tatgtgataa catctttata ctttgaaaaa 360
tgttccactt acccttcaga tatttgttgt aagttaattc aattcttaat actttaattt 420
tgctccaaca agggctttat gttgctggta agagaattta tttactaaat gcactatgta 480
taaagtgaaa gatagtttac ttatctgact ttgatattag atggctgaca ttagtgcaca 540
taatgcagag tttaaccttg attcttcaac agagtccaga tttaaatgtc tacttagtta 600
attagttagc tgatattctt ncacaattaa tatattcaat ttcccatcag tatatcactt 660
taaattttat ggttttctaa ngnaactttt cacagaattt taaacaactg atgcatncat 720
actcanggtg tagggaagaa tactttgcan ttnaaaaccc tgtcaacctg caaccagcnc 780
angagaattn naacttcant gagaatttag aaaaatttcc taaagngaga tgcntttt 838

<210> 228
<211> 882
<212> DNA
<213> Homo sapiens

<220>
<221> misc_feature
<222> (1)...(882)
<223> n = a, t, c or g

<220>
<223> nbla-10672-rl

<400> 228
ggccgnntgn atcaattgnn anccctttga ancacccaan cnnatttcgc caaatcnccg 60
nnttgnnacn nnatcacnnt cccnnccact atncactana ttttcaannn nancanctnn 120
ttntatnntc gggggcatt tntaaacacc tacannccc cccnctnant tcatngcnna 180
tactacncnn tnantncnnc anncacatta ccntctcatc anngannnnc ntnannntna 240
nnccttnan atcncantaa cntncccnat tacancnnac tactgncnna nntncaancc 300
ntnagnaant ctntntccnn nnnnattacc cnntaatnga acnnntcnna ancaatctna 360
atncttactn aacgncttac nctntananc ctannctctc atanncanna nnncnnnnna 420
ctntcttnnt nntnttnntn acatcatcct atactantcc natacnncta anctcnant 480
tntttnttct gcannncnaa nanntnanct attcancaaa nnctnccnnt nntcacttan 540
ncacnnntnn ntnnaancct tnnttanana nacnnttnan tatnncnant ancnanccnt 600
ctancncntc natncnanta nnttnctcnt anantnctnn ctananntnn aaantannnt 660
cactcacctn cctcacttnn aatnnganna tntnannnnn ntntaaacan tacnntntat 720
aatnnntnta ntnnntcctc cncntnanat aatnctatnt nnntatatac acccatttat 780
ctnncnnnct nnncccctcna nntntnacnc nntctantnc tactataatc cannaatctt 840
canaactcat ntncatctnn tnacatancc cntnccatnn cg 882

<210> 229
<211> 2570
<212> DNA
<213> Homo sapiens

<220>
<221> misc_feature
<222> (1)...(2570)
<223> n = a, t, c or g

<220>

<223> nbla-10677

<400> 229
gtacggaagt gttacttctg ctctaaaagc tgcggaattc ctcgagcact gttggcctac 60
tggtacttct aaataaatga attactcaat ctcctatgac catctataca tactccacct 120
tcaaaaagta catcaatatt atatcattaa ggaaatagta accttctctt ctccaatatg 180
catgacattt ttagacaatg caattgtggc actggcactt atttcagtga agaaaaactt 240
tgtggttcta tggcattcat catttgacaa atgcaagcat cttccttatc aatcagctcc 300
tattgaactt actagcactg actgtggaat ccttaagggc ccattacatt tctgaagaag 360
aaagctaaga tgaaggacat gccactccga attcatgtgc tacttggcct agctatcact 420
acactagtac aagccgtaga taaaaaagtg gattgtccac ggttatgtac gtgtgaaatc 480
aggccttggt ttacacccag atccatttat atggaagcat ctacagtgga ttgtaatgat 540
ttaggtcttt taactttccc agccagattg ccagctaaca cacagattct tctcctacag 600
actaacaata ttgcaaaaat tgaatactcc acagactttc cagtaaacct tactggcctg 660
gatttatctc aaaacaattt atcttcagtc accaatatta atgtaaagaa gatgcctcag 720
ctcctttctg tgtacctaga ggaaaacaaa cttactgaac tgcctgaaaa atgtctgtcc 780
gaactgagca acttacaaga actctatatt aatcacaact tgctttctac aatttcacct 840
ggagccttta ttggcctaca taatcttctt cgacttcatc tcaattcaaa tagattgcag 900
atgatcaaca gtaagtggtt tgatgctctt ccaaatctag agattctgat gattggggaa 960
aatccaatta tcagaatcaa agacatgaac tttaagcctc ttatcaatct tcgcagcctg 1020
gttatagctg gtataaacct cacagaaata ccagataacg ccttggttgg actggaaaac 1080
ttagaaagca tctcttttta cgataacagg cttattaaag taccccatgt tgctcttcaa 1140
aaagttgtaa atctcaaatt tttggatcta aataaaaatc ctattaatag aatacgaagg 1200
ggtgatttta gcaatatgct acacttaaaa gagttgggga taaataatat gcctgagctg 1260
atttccatcg atagtcttgc tgtggataac ctgccagatt taagaaaaat agaagctact 1320
aacaacccta gattgtctta cattcacccc aatgcatttt tcagactccc caagctggaa 1380
tcactcatgc tgaacagcaa tgctctcagt gccctgtacc atggtaccat tgagtctctg 1440
ccaaacctca aggaaatcag catacacagt aaccccatca ggtgtgactg tgtcatccgt 1500
tggatgaaca tgaacaaaac caacattcga ttcatggagc cagattcact gttttgcgtg 1560
gacccacctg aattccaagg tcagaatgtt cggcaagtgc atttcaggga catgatggaa 1620
atttgtctcc ctcttatagc tcctgagagc tttccttcta atctaaatgt agaagctggg 1680
agctatgttt cctttcactg tagagctact gcagaaccac agcctgaaat ctactggata 1740
acaccttctg gtcaaaaact cttgcctaat accctgacag acaagttcta tgtccattct 1800
gagggaacac tagatataaa tggcgtaact cccaaagaag gggtttatac tacttgtata 1860
gcaactaacc tagttggcgc tgacttgaag tctgttatga tcaaagtgga tggatctttt 1920
ccacaagata acaatggctc tttgaatatt aaaataagag atattcaggc caattcagtt 1980
ttggtgtcct ggaaagcaag ttctaaaatt ctcaaatcta gtgttaaatg gacagccttt 2040
gtcaagactg aaaattctca tgctgcgcaa agtgctcgaa taccatctga tgtcaaggta 2100
tataatctta ctcatctgaa tccatcaact gagtataaaa tttgtattga tattcccacc 2160
atctatcaga aaaacagaaa aaaatgtgta aatgtcacca ccaaaggttt gcaccctgat 2220
caaaaagagt atgaaaagaa taataccaca acacttatgg cctgtcttgg aggccttctg 2280
gggattattg gtgtgatatg tcttatcagc tgcctctctc cagaaatgaa ctgtgatggt 2340

ggacacagct atgtgaggaa ttacttacag aaaccaacct ttgcattagg tgagctttat 2400
cctcctctga taaatctctg ggaagcagga aaagaaaaaa gtacatcact gaaagtaaaa 2460
gcaactgtta taggtttacc aacaaatatg tcctaaaaac caccaaggaa acctactcca 2520
aaaatgaaca aaaaaaaaaa aaaaaaaggc cacatgtgct cgagctgcag 2570

<210> 230
<211> 1991
<212> DNA
<213> Homo sapiens

<220>
<221> misc_feature
<222> (1)...(1991)
<223> n = a, t, c or g

<220>
<223> nbla-10679

<400> 230
   acggaagtgt tacttctgct ctaaaagctg cggaattcct cgagcactgt tggcctactg 60
   gtaggaaaag tcatgttttt cttctcagaa aggttgatca catgacatgt ctactaagaa 120
   ttttcacctc tgtacttgta tgtatatttt attgttactc aatcttgtat tttatttaca 180
   aattcaacac tgtcaaccct gggaattcta aaataccaat gtatttttag gttgtagcta 240
   atgttgtatt cactttcaat tctcagttgt ccacaccggt gatataagag gaacaaatca 300
   gaatcattaa atactttgta atgccatcat aaactcatat attcatcctc aaactccctt 360
   gtttaatgct aattggtggc ctggaacttc actgagatgc aaaatcaaga actgaagcct 420
   agttgctaga taacgaaaag ctataaatgt ttatgtatgt gaattttaaa ttagaataac 480
   cgtcttaaac tcctacttgc catttctaag gcaaagcatt cattttaata ttgtactttg 540
   ccttttcatt cagttagtgg agtaagtcat gaaaccctta ggaagaaaaa caagttatga 600
   cttattcact aaaattgatg caagacagtt ggttctagat gaccatggcc atgtgttcat 660
   catataaaac cttcagttct ctctatggtg cttggctgga gattgacatg tgaggatgtg 720
   ccaatcatat taaatggact tggtctatgt gggtgatatg tggcctgaat gtaactgtga 780
   tagactgaaa tttgttctta gctctcaaaa tccactgaag aagtcaagtg aaggtgggta 840
   aaatagggag attagtgaca actttgtgcc aaatttttta aaaaatggaa gcaggtagcc 900
   aatattagaa tgataattta agggtgtggt tgaattttag ttagttgtca catagttatt 960
   gaacctcata tgctcagtgc tgtgggaatc aaacatggaa gaggtatggc tcctgcccct 1020
   aatgagaaca aggggaaaaa atccagatat aatctaaatg ctaggttatg tcagggtata 1080
   ggaacacaga gaatggggga cctgtaagaa ctggaagagt cagagagggc tccattgaag 1140
   aggtcaaaca taattccgga aagaattagg tagtgaggag attgtgccag gaaaataagt 1200
   gggaaaggcc acagttatgt ttcctttgaa tggaagagag acaaagctat cagctataga 1260
   tcattgtttt cttaagacag ccaaactggc cctttgaaac cattcaaatt accccagttt 1320
   agctccctac cttttagtct ccgtgaggaa gacaagctgt tgcattatca tattcctctg 1380
   tgctgagcag ctcaagactc agccacaata tgcaaattgc tttaatgcca tattacggca 1440
   gttgatttag acatttgcca gtgcaccaaa ccatgagaga ttgtccgacc taatgccacc 1500
   tggcagatgt gtacccagag atttttctgt agctccatgt ttcccataaa gggcattgga 1560
   aatgcacaga tgaagatctt cctttggaac caggcacatt tggccccttc tcagtgactg 1620
   cactgtggaa ctcttcttaa gaaaatattg aaaacagctt aatgctttca tatagtgacc 1680
   gacatttagt tgaaaactac tgctgcatag caaatattgt gactcttcat gtgtccacag 1740
   gagctcttgt gtgggtttaa agctatgaag tgtattcaca ttgtgaagtt ttaattatct 1800
   ttattgaaat taattgtgta aaaatggtat gtgctctatt aggtattcag tttgtatgtg 1860
   aattctataa agaaagtggt ttttgttctt tgagtttgtt ttatttcttg aagattagca 1920
   ataaatatct aagagactat attcctgaaa aaaaaaaaaa aaaaaaaagg ccacatgtgc 1980
   tcgagctgca g 1991

<210> 231
<211> 2473
<212> DNA
<213> Homo sapiens

<220>
<221> misc_feature
<222> (1)...(2473)
<223> n = a, t, c or g

<220>
<223> nbla-10687

<400> 231
gtacggaagt gttacttctg ctctaaaagc tgcggaattc ctcgagcact gttggcctac 60
tggagagcga gggcgggcgg gtgtgctccg ttcggcctgg gaatccgatt ggttatcgat 120
tcccgcggaa atcaccccgg tgcctctcgc gccccctggc cggcctatgg cgataacttc 180
acggagacgg gcgtggctgg agttttgctt tattgaagtt gaacggttgg ggccaggctg 240
cgaaggagac tgagagcgcg acaaggccct ggacagatgc ccgcgcgggg aaggaaggct 300
ggcttaggag ggggcgtcag ctatggttta gcagcttcca gcgaccgaaa cctacatcac 360
agccgcagca cacaattcca ggaaacctgg tgctcaatat tgggatctca ggaatcagga 420
ccggcggctg gggtgaaagc ctagcggtca gagcttagga gtgaggcaga agctgttaga 480
gactcttcgc aagtgacccc ggggctacat atggatgatt caagagggag tctcctcgcc 540
agaaagttca catgtttcac tccttctggc cttcatacca acccattgtt aaaactggaa 600
gtccctccac ctggggtcac tgactccact gttttcagat aggatgggga ccctgcactg 660
gagactgctc tctagcccct gggcaggaga ggcacagaca ctgctacgca cgcggagcag 720
ggatcaccag cgctttgact gactttgctc gtccaggagg ctggggacat gcgtgcccat 780
gctcagccca ccccctctgt agacacaatt ggtcgcccat tctgtttata ggaagtcttc 840

catcctgcac cagcagctgt ccctagacag ggattgggag acatgcaagt tgcttggctt 900
cctattacct tgtgttgaaa acacaatagg ctttgggatc aggagttcat tccagttatt 960
ttaatgttat ttactagcag tgtgccctcg tgcacattat caaactcctc tgactcttgg 1020
aatcctcatt tttcaataat aactcacaaa gctgtcaagc ctaagggttc taacagctcg 1080
aacagcacct ggaacagaag caatagccgc cactcagagg ttacttcttc cttctccgcc 1140
ttctctggat cacctcccta cctcatcctc aatctaacgc tggaaatatt ggatatgggg 1200
taggtcacca aatgttgcca gaagactcaa attatgccac ccatatctca gctgggaagg 1260
gcttaccgtg gcctgactct cctcagggtc caagtcccca tttatgatgt ccaggcagaa 1320
acacttgttt ctgacacata gtaggcgctg gaaaatgtta gtcaaatgaa cgaggtctgg 1380
tgtggagaga agagaaatct tgggagagtc atttaagggg gtgcccagtt gtcctcataa 1440
aaaacactat catcatcagg gtgaccaaag cagacatcat aggcaggggg tggagtcaga 1500
ggctctaatg tggggactgc cgccaagctc tgcagatcag gagcagtgga cacagcccgt 1560
ggtccccgaa gccgaaggtt gattggagct cttccagggc ttccttcctg ggccatggac 1620
ccctctgagg ccattcgcct ctgttccagt ttggctctcc tggacccctc tggatggcta 1680
ggttgttcct cctcaggtgc tgggggtatc acaacagtgc tgtagggggg tggttggtcc 1740
aactcttggg ggcggcactg ggattctagt cccaccacgg cctcttcata gactggcact 1800
tcaaaggctt cattgtccct gagggaagag aaataaggcc attggtggct tccatgaaag 1860
aagccctttt tccctttttt ttgtctgctg tgggcaaaag atcctctgtt ggcctctgac 1920
ccctcctccc gctgatgcca atgacaggaa accagagact ctctgcctca gagactacag 1980
ggagagaggg aggatgggcc caaggtgtat tcctcttacg atgcccaccc ttaactcacc 2040
gtgcattgcc tgagggccct gggctctcag tctgcattga ttggagctgg gaccgaagcc 2100
cccattccag aaaccggacc aggagatagg caaacaagaa gaagccaccc aatgtgagaa 2160
agaaataagc aacgggggtg atgtccgtct ttatgcccag caaagccagc cccagtagga 2220
aggaggcgca gcacaggagg agaagcggct tctccagtct ccccagtaag tgcattggca 2280
ccatggcccc tccccaggct cctgcaaaaa gacacacagc tattgtgtct acactagcaa 2340
ttggtgtcat ggctgtgtca gataccaaag taaccatatg atgtttcttt gtttggtttc 2400
taggttcttc aatctcaaat aaatcatctg taaaaaaaaa aaaaaaaaaa ggccacatgt 2460
gctcgagctg cag 2473

<210> 232
<211> 1945
<212> DNA
<213> Homo sapiens

<220>
<221> misc_feature
<222> (1)...(1945)
<223> n = a, t, c or g

<220>
<223> nbla-10696

<400> 232
gcctgtacgg aagtgttact tctgctctaa aagctgcgga attcctcgag cactgttggc 60
ctactgggac aatccccaga gccccgcggc ccggcctcgg ggcagccatg gactcgcagg 120
aattgaagac tttgattaat tactattgtc aagagagata tttccatcat gtattactgg 180
ttgccagtga aggaattaag aggtatggaa gtgatccagt cttcaggttt tatcatgcct 240
atggcacatt aatggaaggt aaaactcaag aagctcttcg agaatttgag gctattaaaa 300
ataaacaaga tgtatcactt tgttctctac ttgcactgat atatgcccat aaaatgagtc 360
ctaatccaga tagagaagct attctggaat cagatgccag agtgaaggaa caacgtaaag 420
gagctggaga gaaagcctta taccatgcag gcttattttt atggcacatt ggtcgccatg 480
ataaagcaag ggaatatatt gacagaatga tcaaaatatc agatggtagt aaacagggac 540
acgttttgaa agcatggctt gatattacaa gaggaaaaga gccttacact aaaaaagcac 600
tgaagtattt tgaagaggga ctccaagatg ggaatgatac ttttgctctg ctgggtaagg 660
cacaatgcct tgagatgcgc cagaattatt caggtgccct ggagactatg aaccagataa 720
tcgtgaattt tccgagcttc cttcctgctt ttgttaagaa aatgaaatta caactagcct 780
tgcagaattg ggaccagaca gttgagacag cacaaaggtt gctgctccaa gatagccaaa 840
atgtggaagc actgagaatg caggcactct actatgtgtg tagagagggg gatatagaga 900
aggcttccac caagctggaa aacttgggaa atgcattgga tgccatggaa ccacagaatg 960
ctcaactttt ctataacatt acactcgcct tcagcagaac ttgtggacgt agtcaactta 1020
ttcttcaaaa aattcaaacg ttacttgaga gagcttttag tttaaaccct cagcaatcag 1080
aatttgctac agaacttgga taccaaatga ttttacaagg aagagttaaa gaggcactga 1140
agtggtataa gaccgccatg acacttgatg agactagtgt gtctgcccta gttggattta 1200
tccaatgtca gttgatagaa gggcaattac aggatgcaga tcagcagcta gaatttttaa 1260
atgaaatcca gcaatccatt ggaaaatctg cggaattaat ctatttacat gcagttcttg 1320
ccatgaagaa aaataaacga caagaagaag ttattaattt gttaaatgat gtcctggaca 1380
ctcacttttc acaattagaa ggtttgcctc ttggcataca gtattttgaa aagctaaatc 1440
ctgatttctt gttagaaatt gttatggagt atctgagctt ctgtccaatg caggtaagta 1500
attatgggac ttactttcaa ggatgcgttt acttgatgtt ttatgaaaga acttaaaagt 1560
tttctttgat gcattagact atatcggcat cttaaaatgg tagtatttgt acctatgtta 1620
gttgaaatcg cttttaatca gactgttttt agtaatgact gctgtcaact actgaaagta 1680
ttggtttgca aatattcatt tgtatttcat aaaatcttaa aatcttcagt ttaaagaaat 1740
attaaaaggc atctaattca gacacctacc ttatatttgc taccagtctg taacatcact 1800
gtcaatgtct atcccatgtc tggacacact aaaatgaagt tcttgtcata taactatgat 1860
tgttagcctg tgtttcctgt gttaagtcaa actctgcttt tggttaaaaa aaaaaaaaaa 1920
aaggccacat gtgctcgagc tgcag 1945

<210> 233
<211> 2489
<212> DNA
<213> Homo sapiens

<220>
<221> misc_feature
<222> (1)...(2489)
<223> n = a, t, c or g

<220>
<223> nbla-10712

<400> 233
gcctgtacgg aagtgttact tctgctctaa aagctgcgga attcctcgag cactgttggc 60
ctactggcag ttagaatagg taaagcatta ataatggtag gctatatatc ataatcacta 120
tataaagaaa catcagaatt tggaaacccc ttaagcaaaa atatatctta gaaatcttaa 180
ggacacttaa tggcaagcaa aaaagtttaa atttagtttt ttataaatta catatagcac 240
aatttatgtg tacatgtaga atcttttagt ttttagtgtt tatgtaatat attagacaat 300
aataaacatt tacatttatg tttacattta tatttaattt ttcaaattta atttaagttt 360
ctaaatgctt ctgtggattt aaattttgga ataatctttt cttccccatt gatactggaa 420
tcttgggaag tttcttactc atatcacaat atgttttatt taaatggatg cagcttacat 480
tgcagagttt atataaataa tcacataaca agccaattcc ttagtcattt tctgtattaa 540
aaaactcaga gatctagaga gcttttggat tgaaatattt ccttggtgat tttggatgca 600
aacctgattt ctgtgttcac accatggcca gagatgggac tgttagcacc acctgcttct 660
ttaatgtcgt ccatatgatc agcatcagca ccagaaactc caggtgtccc aaacttaaag 720
tgaaagccat agagtccttt ggtgactccc atgttctgtg ctggttctag aacatgctgg 780
taaatatcag agtttctatg cttatggctg ataagtagac atttttaaaa atcataaaaa 840
ctgtattttc tatatagttc agctaagttt atgaaaaaga tgcagactca gtatttggag 900
cattgctatc gctccatgat gtgtttaaaa attattttta aattgacaaa attttatatc 960
tacatatata tatatcttgt aacacatgat gtttgaaatg cttaattcta gccaattaaa 1020
aatgctttac ctcaatagtt atttttattg tgagaaaggt caacatggtc ttggcacttt 1080
aaaaaaaaaa acaatatatt ctcaataact gtattcactc tgcactacaa taggtctctt 1140
gaactaattc ctcctagcta acaggcattg tttatcattt aacagataat tctctaagcc 1200
cccttctcct aaaacttcag catctcttaa ccttctttct agtctctact tttatgagat 1260
caacttgttt agattccacg tgagtgagac catgaggtat tcatctttct gtgttctgct 1320
ccattggtct atttatctgt ttttaatgcc agtattggac tgtttcagtt actgtagctt 1380
tgcagtatat ttgactgtca agtagtgcag tgcttccagc cttcagagtc ttttactgta 1440
ctatatgaat ttttgttttt tttttgtgaa aaattttatt gatattttga tagacattat 1500
gttaaatata tacatcattt tgtgtaacat agacttttta gcaatattaa tttggccaat 1560
ttatgaataa gggataactt ttcatttatt tatgtttaat tttatacctc agtttctttt 1620
agcttttaat gtagagatct ttaacatttt tgaataattt tatttggaag taaactttct 1680
gtaactattg caggtgtcaa ttgttttctt tatttctttt tcagatagtt tgttgttagt 1740
gtatagaaat gctactgacc cttatatctt gattttgtat cctgcaactt tagtgaattt 1800
attctaagta tttttaaagt tttctattta taagactgtg atatctgcaa acagggacaa 1860
ttattttctt tctttccaat ttggttgtct ttggttttgt tttctaattt ctctggcctg 1920
gaaattcagt agcatgttaa ataagagtga tgaagttaga tatatttgcc ttactataac 1980

tcttagaaaa aaggcttaca acttttgaag gtttagcatg atgtcagatg tccatttgtc 2040
atatatggcc tttattggct tgaggtatgt atgttctata tgtaatttgt tttgagattt 2100
tgtcataaag taatgttgaa ttttctaaaa ttctttttct gcattcactt aaaagttata 2160
attggttctg agatccttca tctttctcag aatctaggag gatgcatctg tacctagagg 2220
aggacacaaa ggcatcatat gttacattct cgtgacatat aatgtctact gcttataatg 2280
tctactgtga gtttctgact ccccaaatga aattgaggcc cagaaacttt caactgcttt 2340
gttcgttgaa taatgccaag tgtctggaaa tgtgactatt cagattaaat gcttaatgtg 2400
tgtgtgttga ctaaataaat ctcaaccaga accttctcag aaaaaaaaaa aaaaaaaaaa 2460
aaaaaaggcc acatgtgctc gagctgcag 2489

<210> 234
<211> 2385
<212> DNA
<213> Homo sapiens

<220>
<221> misc_feature
<222> (1)...(2385)
<223> n = a, t, c or g

<220>
<223> nbla-10751

<400> 234
gcctgtacgg aagtgttact tctgctctaa aagctgcgga attcctcgag cactgttggc 60
ctactggatt tttggcctgt caggtccatc cggcgatgct gggtctggac gagctcggga 120
ggagtggttg tggccattgc acacaggcgg atctgaggtt cggcgacgcc gctggtcgcg 180
acccgggaca ggacaacgac aggaacaccg ccgagcccgc gttccccccc cgcccagagt 240
catggcggca gcagccgctc tgagggcgcc tgctcagagc agtgtgacct ttgaagatgt 300
ggctgtaaac ttttccctgg aggaatggag tcttcttaat gaggctcaga gatgcctgta 360
ccgtgacgtg atgctggaga ccttgacact tatatcctcc ctgggttgtt ggcatggagg 420
ggaagatgag gcagcacctt ctaagcagag cacgtgtata catatataca aagaccaggg 480
aggtcatagt ggagaaaggc cttatgagtg tggggaatat aggaaattat ttaagaacaa 540
gtcctgcctc actgaaccca gaagagatca caaacacagg aatgttcgca ctggagaaag 600
gccttatgag tgcagcaaat atgggaaatt atttcaccaa aagcctacac tccatattca 660
tgagagattt catactgggc aaaagaccta tgagtgcagt gagtgtggaa aatcatttca 720
ccaaagctct tcactcttgc agcgtcagac acttcacact agagaaaggc cttatgagtg 780
tattgaatgt gggaaagcct ttgctgaaaa gtccagtctc attaaccaca ggaaagttca 840
ctctggagca aagcgttatg aatgcaatga atgtgggaag tcctttgctt atacatctag 900
tctcattaaa cacaggagga ttcacactgg agagaggcct tatgagtgca gtgaatgtgg 960
gagatccttt gctgaaaact ccagtcttat taaacacttg agagttcaca caggagaaag 1020

gccttatgaa tgcgttgagt gtggaaaatc atttcgccga agctcttcac tcttgcagca 1080
tcagagagtt cacactagag aaaggcctta tgaatgtagt gaatgtggga aatcctttag 1140
cttaaggtcc aacctcattc accatcagcg agttcatact ggagaaaggc atgagtgcgg 1200
gcagtgtggg aaatccttta gccgaaaatc tagccttatt atacatctga gagttcacac 1260
tggagaaagg ccttatgagt gcagtgactg tgggaaatcc tttgctgaaa actccagcct 1320
tattaaacac ttgagagttc acactggaga aaggccatat gaatgcattg attgtggaaa 1380
atcatttcgc cacagttctt cgttccgtcg ccatcagaga gttcatactg gaatgaggcc 1440
ttataagtga agcaaatttt ggaaattctc ttgcccaggc tttttgctcc ttcaaggcca 1500
gagagttcac accggatcaa ggtgttatga gtgtgacaaa tggggaatat tctttagcta 1560
gaatgctagc ttctttacat aaaagagtgc tcccactgaa gaagtgcctt ttgagtgcaa 1620
tgaatgtgag aaagccttca gccctctctc attggttacc acaatattta catgaggaaa 1680
ataccacaga tgtggaggta atattatttt ttcttcaata taacactgga ggaaacccct 1740
tatgaggatg ccatctgcct aaattgaatg tcatacatca aatagctgca tacattccag 1800
gtatgtggga actgcatagt atttttcgac ctgcccaggt ctcttgctag acttctgtga 1860
cttacagttc tcttctaaaa agcatttcac ctttaccact tggcaggtgc ccacagtaca 1920
tcattcgcct aactcatcat gatccagaag taactttgat tttgtttcat tcaacagagg 1980
gatttttag tatcctcagc actcattcta ttctcatttc tttctctcca atgagttagg 2040
ccatgaacct gactcatttt tggcacagag gactcttctg ataacttgaa aaaatggact 2100
acctttttca gggatctttt ggatctcaca gagttcttaa gatggaattt tccagcctgc 2160
cagtcatgaa tctcagacag cctgccacct attgccctga tttgtgttat aataaaggcc 2220
ttgttgctta aaaaaaaaaa aaaaaaaaaa aaaaaaaaaa aaaaaaaaaa aaaaaaaaaa 2280
aaaaaaaaaa aaaaaaaaaa aaaaaaaaaa aaaaaaaaaa aaaaaaaaaa aaaaaaaaaa 2340
aaaaaaaaaa ggccacatgt gctcgagctg cagtcgcggc cgtag 2385

<210> 235
<211> 758
<212> DNA
<213> Homo sapiens

<220>
<221> misc_feature
<222> (1)...(758)
<223> n = a, t, c or g

<220>
<223> nbla-10759-f

<400> 235
gnnnnnnnnn tnnnnnnnnn nntttggant ccttgagcac tgttggccta ctggtttatg 60
aaataagttt gcattgcaca tccgtgcaga aatctttctg actttgaaat ttttagggcg 120
tcagctgtca gatacgaaag gtagatatca ggtaagaatc tggacttagg aaatagtcac 180

aaaactgtca taggttgtaa ttttatcaac attcgcttct agtaaaatta aagtcaatta 240
agaaatagaa cttgggtcaa agttctgtta caaagcttca taatttgtcc cgaagcatat 300
ggtggagcat tctgagaaat ttgctttttg tgtgtttgac attcctaatt tgggagtcct 360
tcagctgaat tactattctt ttagaagttg agacagcagg taagcaaagg acctagttca 420
tgtaaacatg gacatcatga tggctattta aaaaatattt gttctacacc ttctcccctg 480
aggcttgggg agtgtgttca gccgctgcag tttctctgct catggaggtc ttgtttggat 540
ctgtgctggc ggctgagcat ttagtgtgag ccagtgaccc atgaacttgc cgctctgtga 600
gggccagagt cagggccagt catggtaatg ggcctgaagg cacttncaga accttttatg 660
tctctcgtga gccatctgtt aagaacgtct tcttggtgtg gnttgtagcc tacctggcng 720
ctattctggg aaaccttctt gagtgctatg caaaagtg 758

<210> 236
<211> 767
<212> DNA
<213> Homo sapiens

<220>
<221> misc_feature
<222> (1)...(767)
<223> n = a, t, c or g

<220>
<221> misc_feature
<222> (1)...(767)
<223> n = a, t, c or g

<220>
<223> nbla-10759-r1

<400> 236
gnngnnnnng gngnnnnnnn ttgtgtggcc tttttttttt tncttttttt tttttgcaa 60
cacaagtcaa tctttattga aaactgcagt attaatacat aacaattctt gttacaataa 120
acgtgctttt gagattttta aatctgagct catctcatca gattgcataa aaaattaaaa 180
tagtatcaat tgacacctaa ctgaactggc tcaggatgga aattccattc cttggcatgg 240
atacgtaagt tcaatgcaga ggtgagggat gcctttaaca ctggaagaca atgctgactt 300
agcttaaaaa aagtaccgag agaacggtgt aaaaaacggt atttaaaaat cattttaaa 360
aaaacaaaaa ggaaccgttt cttctttagt tacaatccat gaggctctct agggcctctc 420
cgtgtggcca gcacagcaac cctgctagga gcacaaacgg ctggcctgag atctggccca 480
gctgccttgc ccactggtct gcacagggac tcatgggcac agcctgtggt gaggaggaga 540
cacctgtcat gccagtcctg ggagcacacc acccttctgc aggcccgggg ggggtncct 600
acacagacgc cggtcatctc tgcttccctt tccaagtgac ttcacttcag tcctgtggac 660

caagataang ctattcccac aggctnttct tcttcaaata atgagtngga aaaagagaca 720
aactttntga aacatgcaan tttcgancca aattnggcca catcccc 767

<210> 237
<211> 785
<212> DNA
<213> Homo sapiens

<220>
<221> misc_feature
<222> (1)...(785)
<223> n = a, t, c or g

<220>
<223> nbla-10773-f

<400> 237
gnnnnnnttg nnnncnnnnn nttgnantcc tcgagcactg ttggcctact ggcagaatag 60
acatttgata atggtcttta aattaccctg tacttttagg ggggttatat gtcataggca 120
cagacatctg actctctaat gtcattatga tacacttttt ttatatatat atttctttc 180
taaatcataa aatgagttta cagcagcaga ccccagcttt tctgagaagt acctacactg 240
ttatattgtg taatcgctgt accaactaat atttttcgtc agaatatgta caatacagag 300
ttgaagaatc tgtatcaaaa tgatcagctg tgatgagtct gagctagagg gctttttcac 360
aaagcatgtt ttgttctctg gtgaacttat attagaccat ggctgccaac ttaaaattcc 420
tttcttttaa ggatgcaatg ttgcataatt agtctgctat tgcagtcagg atgcttgcac 480
cctttatttt actcctggta tagaatgcaa tagactatca gagttgaaag gaacctctga 540
gaccatctaa ttcaacttgc tcgtaatata gatgagaaaa cagcctctgt cagttttaat 600
gtcgcacagc aaggtagctg ccattaagaa tctcagaaat aagtttcatt ttagaaaatc 660
tcaagtattt tctggataac aaatagaaat agatnaaaaa aagtagctta aaacaatact 720
attttttcta atgatcnttt cantgacaga acangccttt gnttcacgtg ggaccaattg 780
ggggt 785

<210> 238
<211> 797
<212> DNA
<213> Homo sapiens

<220>
<221> misc_feature
<222> (1)...(797)

<223> n = a, t, c or g

<220>
<223> nbla-10773-r1

<400> 238
gnngnnggnn nnnnttngtg gccttttttt tttttcnttt tctaagaaga aagggatagg 60
aatcatgaag tcatttccag caaatgtcct taataggcaa gcttgccaaa atatcaccgt 120
gcccctgtct ttcccaaaac cttccaacag ctatcagcaa cagctgacaa gcactagcta 180
atttgaaatt gtatgttctc tcctgtcaac accagaccac acacacagct ttgaaaaaca 240
cccagtgaat accataattt ttaaacagag ggctcatcat gttccatttt cttgtgccta 300
aaaagtaata tgtactttct attcagaatt taaaaacctt caccaccctt tgaagtttta 360
gatttaaaat tttattcttt ggcatctgaa ggaagaggac atcaaatatt agaggcaata 420
aagcaagtag aagtgacaac ttcccctaag gttggtggta ccagacactg gcgcaaattc 480
atccatctgc atgtctcaag gcatattccc acatgaggat ctaggaggga aagtgtcacg 540
gaacgaacag atgggcttcc ttgcctagac tagcctaatg gagcttttag gcaatttgca 600
tactagctgc aagtgacaca ggtgtgtgtc aagacttgag agaagctaaa agaaacctta 660
tatagggnat actttcatgc tgattcatgc ttgncttcat tgngaatgct aatangngat 720
attttggtta naanggcaac anttcaatgg ngggcccttt ggtttttctt ttntaagtac 780
aggcttgagg canttnn 797

<210> 239
<211> 2438
<212> DNA
<213> Homo sapiens

<220>
<221> misc_feature
<222> (1)...(2438)
<223> n = a, t, c or g

<220>
<223> nbla-10797

<400> 239
ttctaggcct gtacggaagt gttacttctg ctctaaaagc tgcggaattc ctcgagcact 60
gttggcctac tggatgtatc tttatgctct ttcactgctc ttctgtgcgt tggagtttag 120
caattcagtg agactgggga gtgtctcaag aatggaacct ttgccttcaa ggttgggtca 180
tcaaaaatcc ctctaaagga tctaactgta atgaactgtt tttatttttg ccattagtgc 240
aagtatcaac tggcagcctc aaacagcttt agactatgtc tcaggtctct catagttata 300
cttagaactg ctctttcttg aagctaaaag aggtttttgg agtaaagatc tgcttcatgt 360

ctgctagcat ggtatctgct gttcaaccta gtatagagca tgtgaaatgc aactgtcctt 420
taatggaatt cagccaaact ccaaaaatac tgagttgctt ctagttatct aaagtagtac 480
agaatcacta tcccgatgaa gaagtggggt ttttcagaag attgtaaact catttccatg 540
gcacagatac aaatcatcgt caggacttgt tagccagtcc atgtctcatt gataggatgt 600
agcctgccat ctgcatagat ggtttaacat ggtgctgttt ttgaaaactt gctgtggaaa 660
taaatttgac agacgagtag ttatttgtaa agctgtccta gaaagaggtt aattcttttg 720
gtatatgaat atttctcctc caatcctttt aattaggtgg ttcagcttat cttgtatttt 780
ctgagtcata actatactct agggccaaga cctactataa aatctggggt cctaaccaga 840
gagttgttta gatcaatatg atatgctttg ccaacatgat ctaagaaggt attcattctt 900
tctgggtttg agaagagttg acgaaagtgt aagattatac tctcatgcta aaataaggta 960
taggctaagt gaaatactgt aaatgggttg gttagaatta aagtttagaa ctctaaatct 1020
tatgcttatt tttaaagtaa aaagttgcct cactgtcatg aaatcctgtt actttcatta 1080
aaaaaaaaaa tcctgtaaaa tggtcaaatt ggataatgta agagataatg atggctttga 1140
ggcatttcat actttaccag tttacatttg gctagcagac tggtaaggtt tagggttctc 1200
tccactaatg gaaaattaaa tgctaaacgt cctaagaatt atgttgtttt caaggctttg 1260
actcagattt gtactttgat gtatgaaagt gaatatcaaa cttgtttata gaaaagacta 1320
tatagaaaaa taagtctgtg ttgtgtatag tgatacatgt tttatgttta cccaatgcct 1380
gtactaaatc ttgtgcttgg ctttgaatta tatatgtgtt tacctatgac ttttttttga 1440
aataaacaga acagtcatct aaaaacataa ggttttggaa aggagatgct ttttcaattc 1500
ttaccatctg attaggtgga atgttttata gacttttggt gttgaaaaaa aaaagtttga 1560
taacaatgca ttttgcttgg aatacccccc aatccagaaa agttggtgac ttctctccat 1620
tgtatatatg tacatagttt tcattactag attgtaagct cctcaagggc aggaattgtt 1680
ttatctttgt tgttcctgca gcacctaatg taaagtgcct tgcacagggt aggtgctcat 1740
gacgtgtaga atgagtgagt aacttcctgc ctcatcagga tgaaatgtgg ttcacttccc 1800
ctgtgttcaa cactacaaag ctactcctgc cagaagaggg cagagttctt tcttgggttt 1860
gaactggctt ttttgggttg aaccagatca tcccacatcc ttagcactac ttaaccttct 1920
tttcctgaag attacatatc agtgtcctgc gtttttatct aatattagag ctgaggttcc 1980
taggaaaatt ttgaagtggg gaagattatg gagaaaatga aatggaaatt tggggagggt 2040
ggtttgtcag tataaggccc tgtggggtaa tcatatttta gttattactt gttaaaaggg 2100
tagcagaacc tctgtttctt taccaagtcg ctggttggcc atttctccaa ttgatgaact 2160
accattggtt ttagctcttt ctcatttggg attcccctca attgcttaca tgattctggt 2220
acttagagtc taatagagtt gctgtgaata tgtagtgctg tacataatgg cagttttgta 2280
aatgtgagat aaccttgaat atgttgtggt ttgtatacag agccattagt attgttattt 2340
tggccacttt tttattcaaa ataaataaca taattatttg taaaaaaaaa aaaaaaaaag 2400
gccacatgtg ctcgagctgc aggtcgcggc cgctagac 2438

<210> 240
<211> 1739
<212> DNA
<213> Homo sapiens

<220>
<221> misc_feature
<222> (1)...(1739)
<223> n = a, t, c or g

<220>
<223> nbla-10798

<400> 240
gcctgtacgg aagtgttact tctgctctaa aagctgcgga attcctcgag cactgttggc 60
ctactggtta gatgtttctc aggatcccct cctgcgcagg ggttctctga ttttcgtgtt 120
ctctgcccag atgggctggg ggagttgaga gtgtgcttat tttcactgcg atcatgagac 180
cacagttctg ggttatctcc tctcatacat caagccccag aggaggcggc aagaggaaca 240
gccacaaaca agtactttac cccacagctt agtggccagt aaacaccctg gggactagga 300
aaaggaacca actgtaggca cctctccagg gcctagggag acaagtgtcc tctcttctgc 360
atacatttgg gctcccctta cagagccctt tgccctggct ctctggtcct tgttgctcta 420
acagtccaga tgtacaccca gcctcagggg gaaggcagct ctctccagac agagtctcag 480
ggcccagcaa ggtcaggtta tctgctttca ttcagggcaa caaatgatac aaatggtgcc 540
agggagtggc aaggccatgg gggtaggtgg gggtgtcttt ttcttttcat aaagtaacaa 600
cagacgagac tgaggttaaa catcagaaaa aaacctctgg aatgaccttc ctcattccag 660
gaggccctgg aataaggaag aggcttcttt ctgagggagc tttgaggaat tttgacagct 720
gttgacatgg gatttgggaa aggtgaagct gtgactggag gggcaggaga tggtccaagt 780
gtccatccag agatgagact cttagaatca aagtgttcag cccaggaagt cttggagatc 840
ccaccttctg tggccctgca ccttatggga agccattaag ggggctcatc taggaattct 900
ggttacagcc cagtgctcat cccagcgtat gctgcctctt tagggcagcc ccaagggcca 960
gccagcctgt actctgggca agagcccaaa atggctagga atgtttgact cccttaatct 1020
cttccccagc tacagaggaa tcttttctct gcctggtctc agaatgggac tgccaactgg 1080
ctcattggtg ggagacacag tatcctcaaa cctgtggcca ctggcatgac agtggtgctc 1140
tgtctccctg ggtgacaccc accctaggct tcctcctgga tgtgatgggg attgccagag 1200
aggctcttag cataaaaggc attaggtggg cattttctg tgtgcccccca aaaagctcca 1260
tggaaacagg cacctggtag ctgcggaaca cccgtggact tgtgtatatg gtcataggct 1320
ttgggaagac aggacgtaaa ggaaaatgag agaaacaaaa tgggtcagat agctttggcc 1380
acagccccag gcagcctttg gggcctatga cacttagtgc ccttagatgg gatacatctc 1440
gcctcggccc caagactcct ccaacttacc cgtcccatcc aaggcctgca cagcttagag 1500
aggctcacag cttggcaaat gctagggctt catcagacca ctgacttgac tcagtgtttg 1560
ttaaaatgga accactcccg ttagcctact gtttctctcc tgtacttctt gtaatgatag 1620
ttatttattg actctggtag caggcagttc ttaaataaag atggtttctc aacctgttgg 1680
ggaaaaaaaa aaaaaaaaaa aaaaaaaaaa aaaaaaggcc acatgtgctc gagctgcag 1739

<210> 241
<211> 794

<212> DNA
<213> Homo sapiens

<220>
<221> misc_feature
<222> (1)...(794)
<223> n = a, t, c or g

<220>
<223> nbla-10805-f

<400> 241
nnngnnnnnn nnnnnnnnnn nnnnnttgg aattcctcna gcactgttgg cctactggtt 60
aaatttggct ctaaagttgg tgtagcagca gttgatcaga ctgaaaaacg gtttttagtc 120
tctggaaaaa gactgatttt gcttttttta taaatattat tagatttatt aattnttctg 180
tgctcaatgt gtaaattgta ttataattca ttgtgattta tttcactttt aatttgctgg 240
tgttttaata aatgggggtg ttactgaatc tttcttccca cttccatttc ttttgaccac 300
cccttaaccc tcaactgtga cggtagtagt attatcattt gtaccaaagt tttgcatagt 360
ccctgttgac tttgtaatgt taacggagtc ataaaagcac taggcaagag aaagatagaa 420
atttgctttt aatctttttg ccttttattt tgcacattat gcaaaaggaa aaacattaaa 480
gaacactttn ttttaagtga gtgaaaacat ggtaaaagac atacaaggct tttatgcaca 540
ttggtaagct aaatcaaggn cattataatc atttcctttt tattaagatt taagtaacca 600
aatttanaat ttcagcattt caaaatgatt tatttttcag gcttaaatca tatttacacc 660
taggttgagn taaagatntg aaatatntaa ggatgatnca gggtttcaaa tatctaatac 720
ttttggaaat aactctggtt tngntttttn ggggnggntt taccaaattt aaggttccaa 780
actgnaaagt tatc 794

<210> 242
<211> 803
<212> DNA
<213> Homo sapiens

<220>
<221> misc_feature
<222> (1)...(803)
<223> n = a, t, c or g

<220>
<223> nbla-10805-r1

<400> 242
   nnnngnnggn nnnnntttgt ggcctttttt tttttntttt aatttaaaga tagaatttat 60
   tctctgatgg tttactcatg caaactgcac ataaaagaaa atagtacagt ttgtgtaaca 120
   ttgcaaatat aaggactgaa aatgctaggt acagaagtag tatgacatcc tgaaagtcaa 180
   aatggaattt gtgtttatac aactaataat gatttttatt tgctcagtac agactgattt 240
   acaatgaaag ttttgctaac cttggtaagc ttgttaaccg tttacatgac ttcttacatc 300
   tatgtagttt tattttacag ttgcaagaat tctgttacca aagaacctta acttgcataa 360
   gtaataagat gaaagaaaaa tgtactgaag gacttaagag tgaaaaattg aggcttttcc 420
   taacccattc aaactacaat aaaaaatagc cttcttgcag aattcatatt ttgngccttt 480
   gagataactn cttagaataa tgatatcaaa atcatgattt gaaaaactga ttactttaaa 540
   ataatgaaat taggtaaaaa ttaaaaattt aaaaaaatta tcaaataaaa atgctctcat 600
   tttaaaaagc caaaccatta aattnccant atcttcatac tnaagtctgg aatggcattt 660
   atataattgn atggttccca ntttggacnt agcaggagga tanttccntg gattatcaag 720
   gttttccata cgacctttaa aangcagctc attngaaatg gccctggnac tttnccaaaa 780
   gggganntng atccggacaa ctn 803

<210> 243
<211> 789
<212> DNA
<213> Homo sapiens

<220>
<221> misc_feature
<222> (1)...(789)
<223> n = a, t, c or g

<220>
<223> nbla-10808-f

<400> 243
ntttgaattc ctcgagcact gttggcctac tggaaaaaac aataggacgg aaacgccgag 60
gaacccggct gaggcggcag cagagcatcc tggccagaac aagccaagga gccaagacga 120
gagggacaca cggacaaaca acagacagaa gacgtactgg ccgctggact ccgctgcctc 180
ccccatctcc ccgccatctg cgcccggagg atgagcccag ccttcagggc catggatgtg 240
gagccccgcg ccaaaggcgt ccttctggag ccctttgtcc accaggtcgg ggggcactca 300
tgcgtgctcc gcttcaatga gacaaccctg tgcaagcccc tggtcccaag ggaacatcag 360
ttctacgaga ccctccctgc tgagatgcgc aaattcactc cccagtacaa aggtgtggta 420
tctgtgcgct ttgaagaaga tgaagacagg aacttgtgtc taatagcata tccattgaaa 480
ggggaccatg gaattgtgga cattgnagat aattcaagac tgtgaaccaa aaagtaagct 540
cctaaggtgg acaacaaaca aaaaacatca tgtcttagaa cagaaaagac cctaaggact 600
gggtgcgtaa canccgtnaa agaggagaaa atgaagaacc ntaagttaga agaagaattt 660
gagtggctaa agaaatntga agnctggcta cacttgnnan aaaaagggaa tttaagttcc 720

aantttaacc tttaaccctt gganc ntgaa tgtanccnna acngtnccng nnaatgaagg 780
nnaatgcan 789

<210> 244
<211> 804
<212> DNA
<213> Homo sapiens

<220>
<221> misc_feature
<222> (1)...(804)
<223> n = a, t, c or g

<220>
<223> nbla-10808-r1

<400> 244
gnnggnnnnn ntttgtggcc tttttttttt ttcntttttt acaataaaca aaaagatttg 60
tattagaaca tatacactca gggaagaaag aggtatcatc atcaaatgtg gaatgttgaa 120
gaaatagtta aaataaataa agactccaag cacagctggg actggctcag gctggggctc 180
acagaggcca ccgcacatca gctccaggct gcaggagcca ccacctggcc atactggctt 240
cctccctgac acagcacagc tgtgcctggg acacagagtc gctctcaagt actggagcag 300
ctagcaagct cactccccac tctcctcact tatctctgtg acaatgtcta tcaggctctg 360
gagcccgaag atatagccag catcctggcc ctcatgcacc acggtgtcct cgccatacag 420
cctgcaggtg gtgtgtgcaa agtcgatcat gcgcacatct acagagctgg cgccgatggg 480
tttgtaggca taggcaccag cagactcatc aagctgattc ctctgacagg tcttcaaatc 540
tcacatctga gtncaggacc acttngggcc cggtccttgc catcataaat gaccagcang 600
gaacttgagt agaaacggta ggactnctgg cgttcaacac tggcttgact cagtcagctt 660
cttgacacaa gggccaagaa ttcacggggg caagtcccgc cattggggga aaactgggaa 720
aagngcctcc ttgaancctt naccgatagc tttcgnccgg ggacctggtc atgaacatga 780
cttgccaatg ctgcttggnc acct 804

<210> 245
<211> 1916
<212> DNA
<213> Homo sapiens

<220>
<221> misc_feature
<222> (1)...(1916)

<223> n = a, t, c or g

<220>
<223> nbla-10830

<400> 245
ttctaggcct gtacggaagt gttacttctg ctctaaaagc tgcggaattc ctcgagcact 60
gttggcctac tggggtacaa agtcatgatg attccctgag taacaaaggg aaatgaaaac 120
tgtttgtgat cttccgtatt cactgaaaaa taaatgcttg tttagtatca aatttatttt 180
cacgaaagtt tttttaaaga acagaaactt ttccaattaa aaaaaaaaag cagacttctg 240
gttcaagata gctcactgga atacatgttt acctctttct ttcctaaatt gcattgaatt 300
gataggaagg atggcggaat cttaaagtga tacatgctaa ctgtagaaaa aaatagaaaa 360
tgcacataag caaaaggaaa catttaaatg ctatctttca aagataacta ctcttaaaac 420
cttgagtatc ttttcagacc tttttcttgg caaatgaatc catattgaca tatttgattt 480
ttttaaaaac atggaaacgt actgttttgt aaattctttt taactgcaca tctactgttc 540
ataaatatac ctctgtaaca taactttttg tggttctaat gtactctgtt gtatagctaa 600
tacaagttag gatgcttttg gccagaggta acagtgtcca aatataattg gcctaagtaa 660
cctaggaaat tgtttgacat aacacagggt tcaggggtgt cattaaagac acactttttt 720
tgccttgacc tcagttggtt tgttttgcct taggctattc cacctctcga tcacaagagc 780
tgctgctata acttccagtt ttacatcttt gtaaaattgt atctcaaagg cagaaaaggc 840
catttcgtct ctcatttgtt ttatccatga ggaagatttt taacaaaagc ctccagaaga 900
tttcccctca gtttccattg acttagatca ggttacagag aaaggcaatg tctgacattt 960
ttggtctctg ttagaagtag actctgttga aaagaaagaa gctaagctag gtgtgaagaa 1020
tggaattgga agcccactgc cttcccataa gaaaggttta ccataattta ctcacttttt 1080
tctgtgttag acattttgat tatctgcagt ttattactac aagcagtggc agagtgaatg 1140
tccttgtaca ttttgagtta catgcttaat tatgtccttg agaaagtttc taaaagtgga 1200
atgattggtt tgactggttc atagggcttt aattatacaa tttacccctc taattagtac 1260
tatatgtatg tgacttccct ccccctgcca gaatactcct tggtcaattg taggtattct 1320
ttttggttta atttttgcca atgtaattaa aaaatggtat gtcattttta aaatttgtat 1380
ttctttcatt acaaataaga ttgttatgtc agtattgtta ttggcttttc gtattcctct 1440
taacgtgaac cgtctgttca ttgtttttac ctgttttcgt tttagcaagt agtacttaat 1500
ttaaagtgtg aacttaatat ataagatgcc aggaccatca tattgatgac aaaaatctat 1560
tatgtggttg tagtgcatgt cttggagtta aactcttaag tatcaagaag taaaataatt 1620
tcattgtttc tgttactaac attaaaagtg tgcaaattgt ataaaattag gtacttcgat 1680
tagtaaaaga aaaaaatttg aatgtttttt tttttatttt attaagcatg cccagttatg 1740
ccaagcatag taaataaagg tcaagtagca tttataatag aggaagtatt gttatcccta 1800
gcatgagtgt aatggtgata tgaaaaactt tgtcttgtca ttataataat aaaaaaatga 1860
acatttatta tggaaaaaaa aaaaaaaaaa aaaggccaca tgtgctcgag ctgcag 1916

<210> 246
<211> 1858

<212> DNA
<213> Homo sapiens

<220>
<221> misc_feature
<222> (1)...(1858)
<223> n = a, t, c or g

<220>
<223> nbla-10833

<400> 246
gcctgtacgg aagtgttact tctgctctaa aagctgcgga attcctcgag cactgttggc 60
ctactgggtt caaaaaaaat gtatagaatt atccatgcaa gggattctgg tataggaggt 120
gtgggcggtg agcaaaaaga gatagtggac gtagactact tgtttaaaaa gcctgcagtg 180
tatagaagga aaaaaaaata agctgaccat cagttattgg ctgggagatg cccaggaaag 240
acatgccttg gctcacaccc tggggcagat tctgagggca ctgctgctgg aggctttcag 300
ctaactgtgc ccatcacagc tgagtgacaa aatgtcttgc agggagatct gagcggcaca 360
tgtccttggt ataatttatg ccactataaa caggacacta acactgaacc atttatgcca 420
ctataaatag gaagctaaca ctgaacctga tgaacgaatc catcaggaac aaaagacaga 480
cagacacgca cacacacaca tgcacacgca cacatcagca tggctgtatt tggccatcta 540
tcagtagtca acggttaatc agactgtata ttgggaacat acatggcttt gttttcttac 600
agacttaaat ttgaagaaca gtgatgatga actcattcta tatcactttg gtttaaaagt 660
tgcttagcaa tgggacaaaa acaaaagcaa ctaggtttat atttagtgct ctatctatat 720
gcatctggag cacatttcgg aaataattca aaaaggccaa ttcatttcat gccttctgct 780
actaaatgaa tgtgggagtc aagctgctat tttcacagtg caagacattt attgttgact 840
taaaaaaact ctcccacttg cacataatca ctatcctgac aaatgacact atagagtttc 900
agctccttaa gcataaggta gaacaatctt taagaaataa atattaggcc acaggagtta 960
aacataaagt atcatccaag cacagattca aagcatttaa acacatacat tattctcaag 1020
tggaccagtt cttcactgag tatgtgttct ttaggtttgc aagactaatc ttatgtctca 1080
cccctcctca attgaacagt attggcactt tgattattca gtcagtgtag gactttttaa 1140
ctccgtgcca ggctcatttg cagctatcag tgctgataca acttcatgcc atataatact 1200
tttaacagac tacttccaca cccctgcaaa aaaaaaaaaa gttaataagc aactgatcaa 1260
ttaaggttaa tggtagattt aggcctataa attgtaccca tcttagtcat ttctgcttcc 1320
taaaaattta tacaagaaca gtttttttct ggggtaagag ggtgtgattg acaccctaat 1380
tctgtttctt ctgtttagat tgtggaaata taatcggacc gtttcagggc tctatgagtg 1440
tgatccttgg acatgaaaca gtttccgagt acagagtatg aagtgctggt gagcttttgt 1500
caccaacagc acttaagaag tattttggcc aggtgcagtg gctcatgcct acaatcccag 1560
cactttagga gactgaagag gcaggatgat tgcttgagct caggagttca agaccagcct 1620
gggcaacata gtgaggcccc atctctacta aaaatttaaa aaaaaaaatc agccaggcgt 1680
gatgtcacat acctgtagtc cctgctactt gggaggctaa ggcaggagga tcacttgagc 1740
ctggatgatt gaggctgcag tgagctgtaa ttgtgctact gcactctgcc taggagacag 1800

aaggagaccc tgtctcaaaa aaaaaaaaaa aaaaaggcca catgtgctcg agctgcag 1858

<210> 247
<211> 1934
<212> DNA
<213> Homo sapiens

<220>
<221> misc_feature
<222> (1)...(1934)
<223> n = a, t, c or g

<220>
<223> nbla-10857

<400> 247
ttctaggcct gtacggaagt gttacttctg ctctaaaagc tgcggaattc ctcgagcact 60
gttggcctac tggagtgggt gtgagtgaat gggtgggtga aagagtatac atgcgtagat 120
ggataggtgg atgggtagat agatggatgg atgaaagaat gaatgggtag gtgggtagat 180
gggtgagtgg aagagtaaat ggatggatga cctcaaatgg ccccaaggga ccactgggct 240
ctgccttgcc ctgtgtattt ggtgtctatg gtttcaggcc agggctctaa gtggcacgag 300
atgctcccca aacctctcgc tcttccactt tccaccaccc tttgctgttt tgccaccttc 360
tgaaagatac caaagagaat gaaggttaaa tctgaccctt cctcctccct gccccaccct 420
gggcccaaca ttcagagaag agttaagtga cagagcctcg ttctcctgta tgaattaata 480
attgattcag taacaaatca ctaatattgc agtagtcgtg gtgaataaga taatgaatgt 540
gttctgtcca gggagtgtca tgttgtgagg attcagctga aattattcat aaatgtgcta 600
cttttcctca tctccaacta ctgctagttt agctcctctt tttaatcttc ccaacctgtc 660
acattcgatg gtagaaatcg tcaggctcca tatacagcag aatattaagg gggaactgcc 720
aaggcattat gtcaaatttc aataggtaat aaaaacaaag tgcttgaaga ccattttcca 780
cttctcttaa agttaaaaaa agaagacttc caaaaacctt tctagatggg cactttctcc 840
catcattggg gggaaaaaaa cctgagttca aggtattcag aaacttgggt atgatcccca 900
ctttaccaca aagttgctgc gtgactttga gccagtactc aaccatcctg ggctaagtct 960
ccaatctcta aacaaggggt gcctccctgc ttttctcctg ggtgtcatga tgtaattgtg 1020
aggtgatggg aaagaaagtg ttctaggcca ctaatagata gctaggctgg attttctcca 1080
ctatcacacc ctacaccaac caggtcagca ggttcactat tctctaaaca tgccttccat 1140
gtggccacca cagagagggc ccctgccctc ctgttcaccc tccatgtctc ttcccttctc 1200
catctgaccc aaccagcact ctgggctcag ttcagccacc acttgcttca tgaaaccttt 1260
cccgatcctg cctgacagac atattttctc ctactccaca tctccccaga gcaccctggc 1320
ccactcttac acatacctct tacactgctt tccacatacc atcttcccta gtagactgca 1380
agcatctcaa gaatggaagt tgtcttctac atctttgagt acaataagaa aggagaaata 1440
tttgtagaat gagtccactg gtggatgagt ggggagatgg atagatagat gagtaggtgg 1500

gtggatcggt ggtggatgag tggatgggta gatggatgag tggatgggta gatggatgag 1560
tggatgaatg ctggatggat ggatgaatta gggccgggct ttgtggctca cacctgtaat 1620
cccagcactt tgggaggccg aggtgggtgg atcacgaggt caggagattg agaccatcct 1680
ggctaacacg gtgaaacccc atctctacta aaaatgcaaa aaattagccg ggcacggtgg 1740
tggctgcctg tagtcccagc tactcgggag gctgaggcag gagaatggca tgaacctggg 1800
aggcagagct tgcagccgag atcgcgccac tgcactccag cctgggtgac agagcaagac 1860
tccgtctcaa aaaaaaaaaa aaaaaagaaa agaaaaaaaa aaaaaaaaaa aggccacatg 1920
tgctcgagct gcag 1934

<210> 248
<211> 779
<212> DNA
<213> Homo sapiens

<220>
<221> misc_feature
<222> (1)...(779)
<223> n = a, t, c or g

<220>
<223> nbla-10860-f

<400> 248
gngnnnnnnn ttgnnnncnn tttggantcc tcgagcactg ttggcctact gggtgctttt 60
gtgcacttta tctggggtaa agttgtaaat gttcagtttc ttggtaaagg atattatgtt 120
ttaagttttg gtggccattg tccaattttc tttaaagagc tgccccaagt tacagtcaga 180
ttaacagtgt agagcaccag ctttctcatg tccccagcag cactgagagg cggaagatag 240
tgcctcactg atggtttatt tcacacttcg ttacttatgg ttgagagaga atatttttca 300
gattttattg gacattgata tttgtaaatt gttcattcct tttgcccagt tttctattga 360
gtggttcata gtttctcatg ggtatccaag agttctggat atgtagaggt ggagggtcaa 420
tctcatcact tccttgtttt aaaaatcttc catggttttg tcatcactat gggctcaaac 480
gccgtggtgt ggcatacaaa gccctcactt ctgaaccacc ctgcttccag cctcatctcc 540
catgatccct gccacgcggt gccgtttgcg ctttcatgtc caagtgcttc cttcatgttg 600
gtttcttcct tacaatgccc tttccttatt gccaacagag tgcttctaca ccttgaggat 660
cataagaacc tgngtttcca ttttattntt tatcataatt ttggggccta tcttcccaaa 720
gactttgatt cantggncan tgtgangnct gatctcattt tctttatctg aacgatgac 779

<210> 249
<211> 788
<212> DNA

<213> Homo sapiens

<220>
<221> misc_feature
<222> (1)...(788)
<223> n = a, t, c or g

<220>
<223> nbla-10860-r1

<400> 249
gnngnngnnn nnnnttngtg gccttttttt ttttccnttt tttttttaag acagggtctt 60
gctctgtggc ccaggctgga ctgcagtggt gaaatcacag ctcactgcag cctctacctc 120
ccgggctcgg gcaattctcc cacctcagcc ttccaagtag ctgggactac aggcacacac 180
caccatgccg agctaatctg tgcatttttt tgtaaagaca aagtgtcact atgttgctca 240
agctggtctt gaactcctgg gctcaaacaa tctgcctgcc tctgcctccc aaagtgctgg 300
gataataggc atgaacaatc acacctggcc aaaatctcca tttctccctt atgaaacata 360
ttctcataga atcttaggaa taataggcct taaagtctat aaataaggga aaaaatccat 420
tgacattagg aacctgcgaa aaaaatacat aacaaaacaa tcagtgtttc aggcacctaa 480
attgtgaaca aatcacacta tctacacgta gctatttcca tagacccgta gaaatatctc 540
tgcctaaaga cacagacaag tatgttctct ttctcacata tcaaggagaa aaacagtaga 600
acgttgttgt acactgncta agaaacaatt ggtctttaaa acagactcaa taaaagcaaa 660
atgnctttnc aaaagggtct aatacttcct gccttctaaa ggggtctaat ggcatttttt 720
tttaattaag caaaaagtga atagaatgga aaaatttggg gtcttcatat taactactna 780
catntacn 788

<210> 250
<211> 789
<212> DNA
<213> Homo sapiens

<220>
<221> misc_feature
<222> (1)...(789)
<223> n = a, t, c or g

<220>
<223> nbla-10875-f

<400> 250
nnnnnnnntn nnnnnnnnnt ttgnnnncct ngagcactgt tggcctactg gactttgcac 60

atgatgtatc acgaagctac agcttgccat gtgactggag atttagtaga acttctgtca 120
atatttcttt cggttttgaa gtctacacgc ccttatcttc agagaaaaga tgtgaaacaa 180
gcattaatcc agtggcagga gcgaattgaa tttgcccata aactgttaac tcttcttaat 240
tcctatagtc ctccagaact tagaaatgcc tgtatagatg tcctcaagga acttgtactt 300
ttgagtctcc atgattttct tcatactctg gttccctttc tacaacacaa ccattgtact 360
taccatcaca gtaatatacc aatgtctctt ggaccttatt tcccttgtcg agaaaatatc 420
aagctaatag gagggaaaag caatattcgg cctccgcgcc ctgaactcaa tatgtgcctc 480
ttgcccacaa tggtggaaac cagtaagggc aaagatgacg tttatgatcg tatgctgcta 540
gactacttct tttcttatca tcagttcatc catctattat gccgagttgc aatcaactgt 600
gaaaaattta ctgaaacatt agttaagctg agtgtcctaa gtgcctatga anggtagcca 660
cttcatcttg cactggtncc caaactttgg actgactatg ccagactcaa gctgctatgt 720
caaaaaactg gatnaagctt ttgggggaaa ancctggttt cgnanaatnt attaaatgga 780
tcctaangg 789

<210> 251
<211> 791
<212> DNA
<213> Homo sapiens

<220>
<221> misc_feature
<222> (1)...(791)
<223> n = a, t, c or g

<220>
<223> nbla-10875-rl

<400> 251
   nnnngnngnn nnnntttgtg gccttttttt ttttntttta tttttacatc aagtgtgctt 60
   tatttcctcc acaggtattc tgttaaataa agcaccattt atatactgcc aggccacagc 120
   taaagaggat tctttacaga atcaaatttc ttgtggttgt tccgtataca agtaaactta 180
   attttgataa taagaaccac agcgatcgga ggcaatctgc ctctataagg tacaaaactg 240
   gcacagagga caccatatca tacacagtaa aaatgctgta agtttaaatt acattgtaca 300
   gggctaggca accctgttct tcccagacag ccatattaaa tgaaagccac taaagtgaac 360
   tcttaattac ataaaacata tccattatct gattgccctt taggaagtat actgaagatg 420
   caagtttttt tcatctggag ttctgcctga ccaagaatta agcctataaa tctatcttgc 480
   cattcaagca gagagcactg gacaaactga agcacaaaaa caaataagca aaacttatac 540
   aaacagcatg gggttggggg gtgagggact taaaagtaga catgctacac ctaatgtcaa 600
   gaagcanctt ggtttccttt gccagatatc cttgtgacga catggattgn agattcaatg 660
   gtcctacaca nggtatctaa aatgncatgg tgcttcatat gactaaagag tctttgggaa 720
   tgggcccact gagagataaa gcaccttaag gtcctgggan ggcaaaactt gggcattaca 780
   acttttnaan n                                                791

<210> 252
<211> 789
<212> DNA
<213> Homo sapiens

<220>
<221> misc_feature
<222> (1)...(789)
<223> n = a, t, c or g

<220>
<223> nbla-10881-f

<400> 252
nnnnnnnnnn ttgnnnncnn tttngantcc tcnngcactg tggccttttt tttttttttt 60
ttcaaattcc aaccagaagc taaatacaat tggaaactgg taagcactag ttttactcca 120
aaggagtagg atcattcaga tttactccaa taaaagtatg caacccttaa gcaaagcttt 180
tcttcattta aaaggagaaa aaaaaaaaac ctatacagta gtctttcctt atgttcattg 240
cacaaaatga gttctgcttt tagaactttg acactcaatg gttaatttta caatttaaga 300
ttccaacttt ataacctttt ttctactcca aaacaccctt gtaaagtttt tctttaggat 360
ggtgtaaaaa ccagcatttc tgcacaattc actggaattt ttttctttgt aataaaaatc 420
tcttctctgt aaaaccaaaa acaaaacaaa acaaaacaaa acaaaaccaa aagaaaagtc 480
ctctacctat catggtttct gcagctatgc atgtatttct gttttatagc tgctttatag 540
ctacttcaga ctccagatct gctttaatgn gtataactgc atccacacgc agcagaatac 600
tcttacaata gcaacttggg gaaagagatc tggaaaaaaa aaatcatgag taccaggaaa 660
caaacatggc ccagtaaaat atgaggcaaa aatgcctaca atgagatgcg tntttcattt 720
aagatttctt tccatgggtg gaatttcctt tttttaaacc agtntgggca ttanaaagna 780
ttgacagng                                                    789

<210> 253
<211> 784
<212> DNA
<213> Homo sapiens

<220>
<221> misc_feature
<222> (1)...(784)
<223> n = a, t, c or g

<220>
<223> nbla-10881-r1

<400> 253
gntgnnnnnn ttngtggcct tttttttttt cnttttggaa agcaaggatc acacttcccc 60
ctccctgttc cttaatccct tttctaaaaa gggggaaaa tccggatgga ttttagggat 120
tggtctggtg tcagctgtgt tttattgcac acctaaatcc tgattatagg cttttcattt 180
ctccgcaaag cctttatttt ggcagttaag ccaaatgtgt tttccagaaa gttagttatt 240
ttctcctctt tctttccttt ctttcccccc tttttcccgt ctgaccccaa acgttattgt 300
ccaaacatga ctggacagca gcttttgttt cttgaccctg taatatgaca gtctgctaat 360
attgacagaa ggtgcagntt ttgggttata gtcgtgattt tcgctaatca atcatattag 420
caggaaaaaa aatgacttgn ttctgttgta cttgagtctt aagaaaaagt gcccatagtt 480
taagtgacaa tttccaaagg ctttagtacc acctgtattt caaaatgggg gacccaaact 540
cccggaagaa acaagctctg aacagactac gtgctcagct tagaaagaaa aaagaatctc 600
tagctgacca gnttgacttc aagatgtata ttgcctttgn attcaaggag aagaagaaaa 660
agtcaagcac tttttgaagg nctganggta taccagcatg acaaataatt atgaagaaaa 720
tatcctgaaa ggtgtgcgaa aatncagctt ttccttggna agtncctaaa cctttacaga 780
agga 784

<210> 254
<211> 2690
<212> DNA
<213> Homo sapiens

<220>
<221> misc_feature
<222> (1)...(2690)
<223> n = a, t, c or g

<220>
<223> nbla-10895

<400> 254
gcctgtacgg aagtgttact tctgctctaa aagctgcgga attcctcgag cactgttggc 60
ctactggaaa ctccgagtga gccagtattc ataaaaaatg ttaaaaagca gaagaaagga 120
cacaagataa acaataattt ggataattcg taaattggta tttcattctt gacttcttga 180
gaattcattg tatacgctac tcctcaaaaa taaggaaaac cataaacaaa aattctggtc 240
tgaaatatct tggcagtaat gtgtttgctc tcccctgtcc tgaatatttt aggctaactt 300
tcaattaaaa tggattgcaa ttaaattatt ttgggcagaa atgtgtctta acctttgaca 360
aagttgatca tttcagcatt ttcaaatgct taaactattt cttaaaatga cacaaattgt 420

ggcgttgcca tttaccgagg tttacatgct cttgatgctt aaggattcgg ctttcttttt 480
tcccccgcat ttattcactg aaaacatgat tgattcagtt ttgtacctat gcagaatgcc 540
actgacgtga tgccaaatat gccctattta gaaaaaataa aaattaaaag gatatttttc 600
tgcacagtca aaattatgca gatgtggatt taaaagacta catgtcaaaa aatacgcaag 660
taaagtttac ttactccatg tttcctttta cactcacaga cagcagattt acaggcacca 720
aacagccttc atcaagacaa tgaaatgccc agtgtggctc tttgatgact tcattctgtc 780
tttcacgtat gcccaaaccc tcataattgc aggtgctttt tctgtaccca attatttggg 840
gtctctgtgt tattcccagg catgcttagg tcattctggt ttcccagggc tccctttagg 900
gcctctagat atccaagttg tcacagtctc atttctcagc ttagtctttc cagactctcc 960
aacataccca tgttcaattc tacatcacat ggcatcaaga cagggactca acattgccta 1020
aagcagtttg ggtcactaac ttatttgata aactaatttc tgttaaggct ctttgttctt 1080
tcattctcag ctgaaatgtg tgtatggcaa caggggcctc ccatggacag tccacttttg 1140
gaagctcttg gatggtgtca ccatgaaaga gtagagaaag atgtttagga atgtgcccac 1200
ttgatgcgag aattgtgaga acagtccttc atagatggac aatggtgggt tcattaaata 1260
gcactctggg aataaagtct cttggtaaag taagcaagag aatattcata agctggccag 1320
tcgttcattc ttccatgcac acatttgttc agtacatttt attgtgctag gtcctggaga 1380
tacagaagag aggagttaga cactccatct ccatgtgtgg tttacagtct agcagggatg 1440
catccatgtg agagagctgt gcacctgttc agttttgctc acctctagcc cagttgaaaa 1500
caaaattatg gacaacaatt aatctaataa aaaaataata agttagtcaa gtgttgattc 1560
agatcacatg cctctcattg ttcgtctctg gacaaagttg tgaagaatgc tgcatctcag 1620
actgcccatg ttattgactt tttttaaaat cacctggtaa tccctttctc ctcatatcaa 1680
atgcaaacac agcactctct cagactcact tataagagtg cgtttttatc cattcttact 1740
cttgcttttc cttttattct ccctctgccc agctataaca aattggattg tgattttgtc 1800
cacaattatt attcctaaaa tgacatttat atttggaatt gtattttcat tacagagaaa 1860
caggttaaaa atcaagtcac aattgaaata gggaaagaat accctgaaag atatagttta 1920
gcagtaagtg tatttgtaca ctgataaact gatcttccga agagactgtt tatgattttg 1980
gcagtgcctc atttttttagg tctttgtctt taaggaaccc acgtgtggtt ggggaaagca 2040
cctcaatctc tgcagttcat atgtagaaac tcttataagc cttgtgagag gtctgtgttc 2100
ccttcctgac tgtcctacac ctactatctt gaacatcaga aacactgatg ttcaagaact 2160
ttgattaccc tcatttatct gggagctgtg tgacatctga ggcacagttc taagtagaag 2220
tatcattcag aaccaggagc aagaatcaga tactcgtgag taaaggtcca aaactgagtg 2280
gcaatgtaat acttttggtt ttcaaataaa gagattcata agatttatct aatcaatcta 2340
ctaatgcatg gctctatata agtttgctag cataatagat taatcatggt aaatctattt 2400
ctaatctatt ccttataact tactatttct ccttctacta tgacatcttc atttttgtc 2460
tttaatgctc atccattttt tcttctctgt acgtgaatct tagagcccac aggtaactga 2520
cacagaatgt tgagaatagt aggatacaaa aagtagtggc attttggaga tctggtagtc 2580
ccttagacaa tattccttac atttgtctat ttggtttgct ctcctgcatt ttgtagactc 2640
attcctagca aaaaaaaaaa caaaaaaggc cacatgtgct cgagctgcag 2690

<210> 255
<211> 2457

<212> DNA
<213> Homo sapiens

<220>
<221> misc_feature
<222> (1)...(2457)
<223> n = a, t, c or g

<220>
<223> nbla-10909

<400> 255
gcctgtacgg aagtgttact tctgctctaa aagctgcgga attcctcgag cactgttggc 60
ctactggctt gcaataaaat aatgcaaatg aaaacacatg agggtaaaat aataaaataa 120
gataaaaatt atttaaagca gaagccgcta gtcagggtta gtaaataagc ttagtggagt 180
tcatgaaccc actggaattc catgtatatt tttgcatatc tgttttatgg aggtggtcct 240
tcatacggcc aatcaattca ttgattctga gtgatttgat tatgatttgc ttgcctaaaa 300
gaataatgtt tagatgattt tgagcatcta agaaaacctg atagttataa ttttgaactg 360
gtttgcctta aagttcttga atataattta ggaaggtatg ttaagacaca catatgtgtg 420
ggtgtgtaca gggggagtac aaaaaaaacc acatttttaa gttcagaaaa aaaatcattg 480
caatttgttg taaacagcat ggactaatga tacaggatga tgttggttga attttcagga 540
ctagcaatgt aactttgcaa tggatacgta gatgccattc aaataagtga ttctgttatt 600
tatcctgttt ttttaaagta aaaatattaa acataactta gtttgtataa gaaaaaataa 660
ttgcaggagg taaatgtaac ctgtctgaga taacacacaa aactctgatg attgtatttt 720
ggagttaaga ctatgaagct aaaaaatgtg tgtgcacata atttcaaata ttaggcccaa 780
gtaattttat tttcggaact gctcattaat tatgggagca ctcagtgttt caggaagtgt 840
taagacttca gggtttcagc aatgaaattg ataaggctct tccctagatc taagaagaga 900
cagacaataa acattcaaaa gcaagaacat aagatactga taaattctaa gaagaaaacc 960
cagtaggatg atatacaggg gtgtgactag gaggtcagag gaggttgctc tgaggaggtg 1020
atgtttatgc aaatctgaat gataggaagc ccagcaagag atctgggagc agagccttcc 1080
agggaaaggg aaggacctgt gcaaaacccc agaggcgaag tccatctagg cttgctcaaa 1140
gacaagaaag aggacaagag cattaagtgt ggggagagtg gcaagaggca agatcatcag 1200
gcaagggcgc ctcagacaag accacgccaa ggggagagca cagggcagag caggactgtg 1260
tggaaattcc aacgtgaatg acttccaaaa tcaggacaca ggctctctcc ccagcctgac 1320
ctcttctggt gcttaactaa cttgttagca aaactccttg gggcacagca ctgagtcctc 1380
cagccaggct gcccctttgt attgacatgg cagggataca ggaggcacga gagactgtaa 1440
ctttctagag ttagaatgtc tctagtaact ctagagacat tttagtgcta acttacaatt 1500
gatctggcaa aggaagatag gcagaactat taaagtgttc aatttccttc cagagagatt 1560
cttccatttt ctctcattac aaaaccagaa gatcagctgt gtggggccat cagctcccag 1620
cctaaggtcc tataacctga agcttgaagg caatcagtac ctctgcttta taattgatca 1680
ctttgaggag ccaaaggaaa gagtgaaaga ttgggactgc tttgagtgga gatggcactg 1740
aactcgttgt aataactaca aatgcaattt aaagtaaaag catgagtata taaattgaaa 1800

gggcaggtgg acagaaagaa gagactgact cctagacagg tgctgagaaa gcagtgtaat 1860
taaaaagata aggaagggaa aggagctaca acatatacca cacacacaca cacacacaca 1920
cacacacaca cacgttatca gacattcaaa aaattagatc ttagactcca caatacaaat 1980
cccagaggac aatggattac agtgttgaca ggggagaaat attgtgataa aatcattgca 2040
tacttagtta tgttttcatt gttaaagaaa taaacagacc attttgaggt agttaaacct 2100
cagagaagaa tagcatgtat ttactcttct tgaaatctat gttggcttta tgccccagct 2160
gagataggaa tcaaaggtga ggttgaaaat aaatagggat aatataaacc gtccaccaga 2220
ttgtgttaaa tctaaagaat cgttcagtat tttattgtat ctcactgtat gtgaaaagaa 2280
acaagtttca ccaaacaata cttagcctta ttttgtatat gcagtgcatt ataatatttt 2340
ctattttgtt ctgtctcttt ttttgttcat gcttgacaca aaacattaaa ttggttttgc 2400
aacctacaat ttgaaataaa aaaaaaaaaa aaaaggccac atgtgctcga gctgcag 2457

<210> 256
<211> 821
<212> DNA
<213> Homo sapiens

<220>
<221> misc_feature
<222> (1)...(821)
<223> n = a, t, c or g

<220>
<223> nbla-10919-f

<400> 256
ttnnnnncnn ntttgaattc ctctgcactg ttggcctact gggagatatg actgtcagtt 60
gcagtgagtt ctgcatccca agtggaggga attgggtttg tggcaaagag cttgacccag 120
aaatagatgg tgccccccaa attgtctcca catgaagatg tactgatgac gccccagaaa 180
tgctgcttcc atatcagctg ctgctagcgc cagcgcagac tctcagggag tcaccacagc 240
ttgtcttgtg cttggtgagt gagggtctct ctactcagtg tcagacatct acaggaaaga 300
aacaactggt ggaaaagagc aataaattgc ccggtgctct gcagggctgg aatttcaaac 360
agaaagaggg aataagatcc tgtgattttt ctcacctgct tttccacgca ctgtggtcat 420
cactgtgcaa tctacatcta gtatgaaatc cacacatagg agagctgggg cacaagggga 480
ctggaggcag ttgctttgca agatggctga ngagaaagca cactgggaac acaatccaga 540
atggtctaac aataagtttt caagtgaata aaccactggc aagacaattn catgtgcacc 600
tttanggtac ctatatagnc tnctaagaag atcaggatga aagacctaga tgatcccctg 660
aggataaaac cttcatccct aaaatgattt ttttaaatcc actgcnttac tgtccaggag 720
gcaaaatgtt tttctggctt tgggccaant cctgctgana cnggatttta ctcttggtac 780
caaatctatn tncctaangc anggnttcaa gcctganttc t 821

<210> 257
<211> 837
<212> DNA
<213> Homo sapiens

<220>
<221> misc_feature
<222> (1)...(837)
<223> n = a, t, c or g

<220>
<223> nbla-10919-r1

<400> 257
   gnngnagnnn nctttgnggc cccttttntt tttttntggt ttcagtagtt tattcacatt 60
   tttttttctc tgnttatttt aatatgcacc acgtgtaagt tagatgaggg ctacggnaaa 120
   aacatttttg atgactgaaa tcttactttt ccttctaacn atctggagca aatcacatat 180
   tcattgntca tgacgaataa aatgatttct gntacaatgn tgagaaaaaa tgngntcatt 240
   aatatgaaca catgtctaca gagatgattt acacnatgca agtaatgaaa gagttcttcc 300
   ttaactagnc tactatcaaa ctgaaaagat gaatcttaaa attcccttga aaataccata 360
   tgattggtca tgngtcagta aaagaaagac ggtccccatn accaggccac caccattaga 420
   gagctatntt aaatacttgg gaaaaaatat ggaaacaccg gtggtgatga ttaaattgat 480
   aattacntag atncactgnc ataatgatac atatttggtc tatgataagt gaaatcaaaa 540
   ataagatcaa nttggntata gaaattcctt aaagaacacc accacttaat tatttcaaac 600
   ttggactatt ccaagaatgn cnagaaaaaa atttgaaagc nctggcttaa agccaagcaa 660
   tctggatccc caatgnagtc agcatgnctt gggcttggct ggtaaccctn tggggacatt 720
   tttgacattc aggacttggg ancctaatgg tnnaaagaat ggcnttcatt tngntactna 780
   aganttttac ttgctnggna cattcactgg tattnncntn nacnctcaat tttcnng 837

<210> 258
<211> 1788
<212> DNA
<213> Homo sapiens

<220>
<221> misc_feature
<222> (1)...(1788)
<223> n = a, t, c or g

<220>

<223> nbla-10927

<400> 258
ctgtacggaa gtgttacttc tgctctaaaa gctgcggaat tcctcgagca ctgttggcct 60
actggttgct aaaagctgga agttgggcta aaaagattgc tgtgaaaaga gctcttaaat 120
ctgaagatct tagcataaat ctaataagtt ctgaatttgt tgggcttgat attcagcaga 180
aacttacagt cttttactta gaacccaaga ggtttggaaa tcaaatcact atgcaaagaa 240
aatctgaaac acagatttcc cattctaaac attcagacat atctacaata gcaggaccta 300
ataaaggaac gactgccagc aaaaaacctg ggaaccgaga atgcaatcat ctttgtaaaa 360
gtaaacatac atgtggacat gactgctgta aaattggagt tgcacagaag tcagaaatta 420
aagagtcaac aatttcctca tatttatctg atttaagaaa caggaatgct gtttcatctg 480
ttcctccagt taaacgtctg aagatacaga tgaataaatc tcaaagtgtg gaccttaaag 540
agtttggttt tactccaaaa ccttcccttc ctagtatatc aaggtcagaa tatttaaaca 600
tatctgaatt gcctataatg gagcagtggg atcagcctga aatctatgga aaagttagac 660
aagaaccatc tgaatatcaa gacaaagaag ttttggatgt gaactttgaa ttgggaaatg 720
aagtttggga tgattttgat gatgaaaact tagaagttac tagcttttca actgatactg 780
agaagacaaa aatatcagga tttggaaaca ctttgagttc aagtaccagg ggaagtaagc 840
taccccttca agagtcaaag agcaaattcc aaagagaaat gtcaaacagt tttgtttcat 900
cacatgagat gtcggatatt tctttatcaa attctgctat gcccaagttc agtgcatcct 960
ccatgacaaa attacctcaa caagctggaa atgcagttat tgtccatttt caagaaagaa 1020
aaccacaaaa tctgtcacca gagattgaga agcaatgctt tactttctct gaaaaaaacc 1080
caaattcttc aaattataaa aaagtggatt tttttattag aaacagtgaa tgtaaaaagg 1140
aagttgattt cagtatgtat catcctgatg atgaagctga tgaaatgaag tctttattgg 1200
gaatatttga tggtattttc taaaacaaac aaatactttt tatattgata agagaaagaa 1260
taaagacacc taatcacaaa gcatgctttt tatttttaaa aagtagttca taattgttcg 1320
gatttttttc tcctgtattt gattagcttt gttcactttt tgcaacaaaa tgtatgggcc 1380
gatttatttt cccattatat tgtaacttct tgaaaagctc ttttacagtg gctttgtttc 1440
aataagccct taagttaagg gctgattgca cttagaaata agttgcactt agaaataatt 1500
atttggctat taattggatt ttgacaagaa ccaatgtaaa atttcatttt tatattttct 1560
taacaagaag aaaaatattc tatgaaagca aagtaatgct aacttaatga atctgcttat 1620
tgtatggatt aatcactaat attattccca tgtattttgt tttactaaag atttttaaga 1680
atccagtggc tatttgaacc tactgttatc taactttgag caacaataaa tgaaacatta 1740
atcagtataa aaaaaaaaaa aaaaaggcca catgtgctcg agctgcag 1788

<210> 259
<211> 815
<212> DNA
<213> Homo sapiens

<220>
<221> misc_feature

<222> (1)...(815)
<223> n = a, t, c or g

<220>
<223> nbla-10934-r1

<400> 259
gagnngnnnn nttgtggctt tttttttttt ttactttaca aatagtttat ttccccttca 60
caaattcatg cgcctctgac cattatgcaa ggcaacatga attaaatact tatgtcagaa 120
catatttgtc ttacattatt caagataaag tggattttaa aagcaagtgg gtaccaatga 180
tggggagatg aggagaatag ggcaggcagc aacagggcaa catgcatttt tcaagagtgt 240
ttattaaaat aggcagtaat cagtacatgt acatcatatg agcagttttt caaaattagc 300
acttccagga gaggggctac atctcagttt tttctgtctg tacagtaaaa tgccaaaagt 360
acttccctaa agtacaaagg catttcccta gtagtcttgg taccagtaac aatatgatta 420
ctaaacatct ccaatgtggt tttcattaca aagaaacatg tttcacataa aagcttcata 480
atataatcca gagacagaat ttgtgcatgc ttacaatttg aagccaggct gaatatattt 540
gatatatatt ttttcatttc tccagtgttt tttattttta taaatatgca cctagtttcc 600
taccagttta aaacacacac gaaacactgn ttttgagcac catgattgcc ttacagtagc 660
ctctttggga ctncaatcct agttcaagga ggggagtcct aatttctatt ctgacttgnt 720
tcnttcccca aatnatgtta gnggttgccc aaatccatca attttnagat gccaacaaag 780
ggttttgggc naangggcca atnataattt tgant 815

<210> 260
<211> 835
<212> DNA
<213> Homo sapiens

<220>
<221> misc_feature
<222> (1)...(835)
<223> n = a, t, c or g

<220>
<223> nbla-10939-f

<400> 260
gnngnagngg nngtngggnt tnnnntttga ttcccttgca ctgttggcct actgggcaag 60
attgcactac ggaaggacct ggataacgct gaggaaaagg cagatgctct gaataaggag 120
ctgctgatga ccaaacagaa gttgattgat gcagaagaag agaaaagacg gctggaagaa 180
gagtctgctc agttaaaaga aatgtgccgt cggaaactcg acaaggcaga atctgagatt 240
aaaaaaaaca gttctatcat tggtgactat aagcagattt gttctcagtt gagtgaaaga 300

ttggagaagc agcagacagc caataaggtg gaaattgaga aaattcggca aaaagtggat 360
gactgtgagc ggtgccggga atttttcaac aaagaagggc cgtgtaaaag gcataagctc 420
aaccaaggag gttttagatg aggacacgga tgaagagaaa gagacgctca agaaccagct 480
gagagaaatg gagctagaac tggcacagac caaactccag ctggtggagg ccgagtgtaa 540
gatacaggac ttggaacacc atttagggct tgccctcaat gaggtgcagg cagccaagaa 600
gacgtggttt aacccgaaca cttgagcttc ataaagacag caacccgggg ttcaagggaa 660
agagacttgc tgagaacagc ttgncggctt ccgcaccttn agaaaacacg acacctttg 720
gtgccttctt tggcaagatg tgtgaatctg tgacttgtcc angancagaa tgtacctaag 780
tcaagatcca taacgcatgt tggtaaggtc acttggacca naactttgtg aacan 835

<210> 261
<211> 824
<212> DNA
<213> Homo sapiens

<220>
<221> misc_feature
<222> (1)...(824)
<223> n = a, t, c or g

<220>
<223> nbla-10939-rl

<400> 261
gnagtggggt tnnnttngng gccctttgtn nnnttcntta aaggtgctaa aatccattcc 60
atagctttga agtgagaact tacagcccat ctgaacagtg aggtttgtac ccaggcatac 120
tctgaggagg gcccaaaggc ccagcccatc tgggagggac aggcccggag gatggagacc 180
tcgcagcatg tgcaccctgc gcagacattt acagacacgc aactcagtct ttctcacttg 240
gacaacctgt tgtgcaaaaa cactaggaat cttgaagtga gggagctctg tccacagctc 300
ccgtaatggc agggaggaga gcagagccca atgaaccttg agtgaaatta agtgctcgaa 360
taaccacata attaaattgt gcagatcagc agtcctagtc tggaatttaa acactgtcaa 420
cggggcatat tgggaaagat atttatatat atatatatac atacacacac acacacacac 480
acacacacac acacacacaa acacattaag caaaataaga tttgtccatg ttataatcca 540
gtctcacagt caagtcaccc accccagcct tttgaagtca tttggcagct aatgctaaaa 600
agtaacagga actatgagag ttgcatagaa ttgatcagaa acatgaataa tgccaaaccc 660
aaaacctgna ccatcagtta acagaaccag gtggcttcag atgcactatg aaacccttct 720
atttaaaaca aagngcttnt tcatgtctac ctgggggacc ttttgntggg cattgaacag 780
aaaagaanta tttgaattct ggatccttgg ccaaccccac tttt 824

<210> 262

<211> 816
<212> DNA
<213> Homo sapiens

<220>
<221> misc_feature
<222> (1)...(816)
<223> n = a, t, c or g

<220>
<223> nbla-10946-f

<400> 262
gnggnngtgg ggnntnnnnt ttgaannccc ntgcctgttg gcctactggt tgtcttgctg 60
ccttctttgc cagagatgaa ctaaaaggtg acaatatgta cagttgtgaa aaatgcaaaa 120
agctgagaaa tggagtgaag ttttgtaaag tacaaaactt tcctgagatt ttgtgcatcc 180
accttaaaag attcagacat gaactaatgt tttccaccaa aatcagtacc catgtttcat 240
ttccgctaga aggcttggat cttcagccat ttcttgctaa ggatagtcca gctcaaattg 300
tgacatatga tcttctgtca gtcatttgcc atcatggaac tgcaagtagt ggacactata 360
tagcctactg ccgaaacaat ctaaataatc tctggtatga atttgatgat cagagtgtca 420
ctgaagtttc agaatctact gtacaaaatg cagaagctta cgttcttttc tataggaaga 480
gcagcgaaga ggcacaaaaa gagaggagaa ggatatcaaa tttattgaac ataatggaac 540
caagccttcc tcagttttat atttctcgac agtggcttaa taaatttaag acctttgccg 600
aacctggccc tatttcaaat aatgactttc ttttgnattc atggaggtgt tccttcaaga 660
aaagctgggt atattgaaga cctgggtttg atgctgcctc anaacatttg ggataaccta 720
tatagcaggt atggtggagg cccactgcaa ncatctgnac atttggcatc ctggccaatt 780
gaggcggnga aaattggaaa angaagaaaa ctggat 816

<210> 263
<211> 826
<212> DNA
<213> Homo sapiens

<220>
<221> misc_feature
<222> (1)...(826)
<223> n = a, t, c or g

<220>
<223> nbla-10946-r1

<400> 263
gnnagngggn nnnnntttgn ggccntttnt ttgnnncccn ttttnttttt ttttacattt 60
taaagacatt tttattgagc taattttaac aacattgctt tagctggtga cagctgcccc 120
aaaccaaaac aaagccatca tgaatgctat tcaacatcct caatgtaatc cagtatgttt 180
ttgtacttgg aatatagtta aacttttgac attacataat caagcaaata gcagtgcata 240
ctatattatt caaaaagact ttatctattt catttaaaaa atcaagttgc aagtggcctc 300
agctttatca acaatcgtag tgacacattc cacacttcat gctctcaaaa taaaaagtgc 360
cctaaaacta actctaagtt ttttagtcac tgacattaat actaaccagg ttacaggaat 420
tgaagtttaa cattgtacaa tataagcggc aataagttac tgatatctgc tgacaaattc 480
cactcaaact aaatatatcc ttgatcattc aaaaagacat tttgtaaatt ttaataagct 540
agttagtgna tatgctgcag tgcaaaaaaa gtgacaccct agtttcctga gctttaggaa 600
attaacaatt ttctgctact acttttaact taaaataggc tctttattat atgaagcctg 660
gcaaaactnt ncaattaaat tgggcttttg ggagaactga aagactggtc agaagtcang 720
ggggatttan gacaatggaa aaagnttcaa gncaaagttt ttccacactn ccaaaggttc 780
tntgggnnct catgtnnngg gaagattaaa gataaccncc tggttt 826

<210> 264
<211> 1735
<212> DNA
<213> Homo sapiens

<220>
<221> misc_feature
<222> (1)...(1735)
<223> n = a, t, c or g

<220>
<223> nbla-10949

<400> 264
gcctgtacgg aagtgttact tctgctctaa aagctgcgga attcctcgag cactgttggc 60
ctactggtgt tttaacactt gtttgtgtga gattgtgtga gaaattcagg acctgtacac 120
tgcagtacat ttgaggattc ctggccctca cctcctaaat cccaccagct acccttcccc 180
cattattttg agaattaaaa cacctacttg tttccagatg ccacctgcct ctgtgagtgt 240
gggcatgagt atgtgtgtgt tgcggaggtg ggtgagggag ttggaggagt tttgttacac 300
atctgagtta tttcatcatg gtaagatgtt attgttcttt catttcatta aaaaaatctg 360
tctaattctt taaaaataga gttaaagaaa gaagatattt atgcagtgga gatagttggt 420
ggtgctacac gaatccctgc ggtaaaagag aagatcagca aatttttcgg taaagaactt 480
agtacaacat taaatgctga tgaagctatc actcgaggct gtgcattgca ggtgaatatt 540
ctttctttta taggtaacca tttataaatc attgtaacaa gatggcagtg gtttatgtat 600
ctaataacct gggattagct cagggtgact tcagggtgtt gagattccaa aacctttgtt 660

ttaaaataga ggtgatgttc ctaaaaataa ttgtactcta tagcacataa gatgaattaa 720
ttgactagaa acataggtaa ctctgagcct gaagtatgac tggaagtttg gcctgttatg 780
tatgtctgta tgaaatagtg tacattaatt aaataagggt tggggttaat agaaaaataa 840
ggaatagcta gattatatag gtaattgaca tgatagaagt ttgtaaaact gttcagacac 900
cattaaatta ctaatataaa tattttaaaa agaccattga ttttctgaag taatttgtat 960
gtttaagtga tattatcagt ttatattgtc tatacttgtt aaacggaatt tttgaaaatt 1020
attattctag tgtgccatct tatcgcctgc tttcaaagtc agagaatttt ctatcactga 1080
tgtagtacca tatccaatat ctctgagatg gaattctcca gctgaagaag ggtcaaggta 1140
tcatgtttat taatcattta catatctaaa gttaattgac tattaggtct gagtaatatg 1200
ttgcttttca tttcagttta cataaatctg taacatttta tcatagtgca tgaaggaagt 1260
ggtcatttat tttagtcaga ataaatttgt gtccttaatt agtttctcac acttgattct 1320
gctgaaatca tcggtgcctt ttatgtgcag attgacgtat attccttttt tactgacttt 1380
atatataaag attcttggga actcttataa tctggaaata ataccaattg aaaagagaga 1440
gaaactaaca ctgtacaata attacaaacc tagaacacat gggtaaaagt gttaggaata 1500
tcctgaattt gtgtttttgt ctttttcctc cattcagtga ctgtgaagtc ttttccaaaa 1560
atcatgctgc tcctttctct aaagttctta cattttatag aaaggaacct ttcactcttg 1620
aggcctacta cagctctcct caggatttgc cctatccaga tcctgctata ggtaagtaaa 1680
gagttggaaa ttaaaaaaaa aaaaaaaaaa aaggccacat gtgctcgagc tgcag 1735

<210> 265
<211> 725
<212> DNA
<213> Homo sapiens

<220>
<221> misc_feature
<222> (1)...(725)
<223> n = a, t, c or g

<220>
<223> nbla-10967-r1

<400> 265
   gnngnggnnn nnnttgannc cnttnnttnn cntttttttg tgattcaaag atttactaag 60
   gtactctatg cattctatct atacagaaac acctatttat tattacagtt gatttatgca 120
   ggattaacat ttttggtgtt aaaattgtta aacatcatgc ttactgcaca tcaactctcc 180
   ataatgaatc tgaacttcac aatgatttac caaacacttt acttaaatta ctaattaaat 240
   aaatgaaaaa tgcaccgagc caaacaaaac ttaactgggc tataaagaaa aaaacccttc 300
   aaaacagtcc atatacacat ctatttcaaa actacctttg ctagctagcc cctttcccaa 360
   agttttagcc tgaaaaaaca gtaaaatcta cacaaaattt tattgcaatc atacaagggt 420
   tacattaggt caacaaatac tatgatgcaa ttttacattt attaaactac agttcaaagc 480
   acaaatttac acattctaaa tacactaaac gttatctaat gaagtcacac tggcttctaa 540
   catttgatat atctgggtga aagacatgaa ctttacaaga ctttaaacac aaatccttag 600
   tataaaaact gngttcttgn atgtaaacga tttaatgggg aacccaatta atgggcttnc 660
   atcttcacta agcatncatt tggtgnatat ttattttaaa cgcttaangg gtaggacaaa 720
   ctggt 725

<210> 266
<211> 710
<212> DNA
<213> Homo sapiens

<220>
<221> misc_feature
<222> (1)...(710)
<223> n = a, t, c or g

<220>
<223> nbla-10977-f

<400> 266
ggnnnccntt ttgaganccc tcgngcactg ttggcctact ggaagtagtt ccttgaatgt 60
ctgttgatga aatagtcact gtttaaggaa aaaagtaatt atgaggtgta gcagattgca 120
gaaaaacagg attagaaaca cacttaaaaa gaacacacat ttagagtctc tcttcctcct 180
cagcgaacca ctaggccccc tttttaaaaa cacctttaga gcctaattac tccaataaaa 240
gtaactagag gtttggagtc tggttaaata aattctgagt aaaattctta agccaaatgg 300
aaattcttaa tgcaatcatg aggacttcta ttgtctctta ctgttgtatt anatcctata 360
aattgaactg atttttccat aaggaaaatg cttcttttga gattaattct aataacgtat 420
ttgctattgc agtgcagagc ccactgcaac tgctaggact gaaagcagag gctgggtgcc 480
agagcacgtg attcttaaca tcatttccac agacccctct gccctgaccc tctgcattgg 540
atgcaggaag ctgggaaaga ctgatgttga nttggaaaca tgggctgaaa atgaaggccc 600
atagtgcata ggaacaagta aagccagggt gcttgacgtg tgtgtgtgtg tgtgtgtgtg 660
tgtgtgtgtg tgtgtggngn tgngngtgnt tgnncctgca cccctacann 710

<210> 267
<211> 717
<212> DNA
<213> Homo sapiens

<220>
<221> misc_feature

<222> (1)...(717)
<223> n = a, t, c or g

<220>
<223> nbla-10977-rl

<400> 267
   gnngngngtt nnntttgtgt gncctttttt tttctttttt gaattaagtc tcatttattc 60
   tagggcaaac tcaaaagggt tttttttttt tcttttttag caaatcctag cacattatta 120
   acaaaaaaat ctgtacattt gccatgatac acttgaaagt gaaacaaata agatataaat 180
   acagatatgg atgtaacatg aaaaagcccc ccccccaaaa aaacccaaaa aacaaaaaaa 240
   cccacccaac ctgggggtgt ggggaccatg cgtgtgaaca cagtgccaac ataactattt 300
   tggtggctga tattttttca gagcacttct ttatttccaa acattattgt tagcaaagtc 360
   ctggtgggat tctacaaagt tcttatttgc tgtcctttaa agtgtaacat tacgcttgat 420
   aaataaaggg tggcagggaa agaaaaagac tgatgagaca aagtcagttg cattacctgc 480
   acagggtggt aagctagtga ggcgcataga aaaggcaggc ccgtggcatc gccgcaggtc 540
   cgggaagcac acacagtgta cttgaaggaa gctgcgtgcc ccgaggaccg gntgnaccct 600
   gcccanaccc tggctgcaag ggaggcaaca acttgganga acagggagtc aancccgcca 660
   gcccgnatgg ancaacgggg gtcttgggcc tggcagtccg gtanacaggg angcaaa 717

<210> 268
<211> 1979
<212> DNA
<213> Homo sapiens

<220>
<221> misc_feature
<222> (1)...(1979)
<223> n = a, t, c or g

<220>
<223> nbla-10993

<400> 268
ctgtacggaa gtgttacttc tgctctaaaa gctgcggaat tcctcgagca ctgttggcct 60
actggaggat gcgctccccg gcgcccagca gcagaggcca ccgctcccag aaatgcatgc 120
gaccgatccc cttctcccgg accccaggag ccggcgcccc cgccctgtag ggttacgact 180
cactgattaa aaagagggac tttttcaaat actttgcact tttgattgtg tattatggat 240
accaaggaag agaagaagga acggaaacaa agttattttg ctcgactgaa aaagaaaaaa 300
caagccaaac aaaatgcaga gacagccaca gctgtagcta caaggactca tactgggaag 360
gaagataata atacagtagt tttagagcca gacaagtgca acattgctgt ggaagaggaa 420

tatatgactg atgagaaaaa aaagagaaaa agtaatcagt taaaggagat caggcgtaca 480
gaactaaaga gatattatag tattgatgac aatcaaaaca aaacacatga taaaaaagag 540
aagaagatgg tggttcagaa gccccatggg actatggaat acactgctgg aaaccaggac 600
accctaaact ccatagcact gaaatttaac atcactccca ataaattggt ggaactgaat 660
aaacttttca cacatactat tgttccaggc caggtccttt ttgtgccaga tgccaactct 720
ccttccagta ccttaaggct atcatcatcc agtcctggtg ctactgtctc tccttcatca 780
tcagatgcag aatatgataa attgcctgat gctgacttag cacgaaaggc cttgaaaccc 840
attgaaagag tcttatcgtc tacttctgaa gaagatgagc caggtgtggt gaaattttta 900
aaaatgaatt gtcgatactt caccgatgga aagggtgtgg ttggcggtgt tatgatagtg 960
actcctaaca acatcatgtt tgaccctcat aaatctgatc ctctggttat tgaaaatggg 1020
tgtgaggagt atggtctcat ctgccccatg gaagaggttg tttccattgc gctctacaat 1080
gacatttctc acatgaagat caaagatgcc ttgccatcgt aagacattta tttgtttacc 1140
aggaaaaaag gggtgttgag agagctaaat gtagcttaaa aatgagggca tttgcatgat 1200
tgagggattg tgtagaggtg attttgaaga tggaagactt gtgcactgaa gaaaatgaga 1260
aaaatgagaa gaaatgaaaa gaataaaatc aatgatggga aaagttgaac atataacgat 1320
taaaggagaa aaacaaagaa gccgtcatgt aaaaatagta tttgttgggc ttatttttct 1380
aaaaagcagt gcacgttctt aatgaaatta tgaaggaaga aaggcagttc tctgaaagaa 1440
gtttatccaa ttatcaataa gagaataatg ttttcttctg ggtttaatta aggagagtta 1500
tgtttgtctt catttaactt ctaggaaaag cagtctccct gattcatgtc ctccctcagt 1560
cctgcatgga gagaggtttg ggtctacagt gtagtggtag ccaccttctc atgctgtgaa 1620
gagggagtaa taccagtttg ctttttccct gaaatacaga tgaatatgac ttcagtcctg 1680
attacttttg ccttataatg ctggatttat tgtaaaaaag agagggaagc tccccaggaa 1740
aaaagagaaa gcattaagaa agctcaggaa attgattaac tgatacagat aatctgattt 1800
ttactgtcct ttcgctctac tgtgtctgtt tctctataaa agccagcagt aaaaaacttt 1860
aaaaaccttc agtgatggga agaggcaaag cagtaggtcc taacagtaaa gagggaaact 1920
agcccttggg gcttatatga aaaaaaaaaa aaaaaaggcc acatgtgctc gagctgcag 1979

<210> 269
<211> 727
<212> DNA
<213> Homo sapiens

<220>
<221> misc_feature
<222> (1)...(727)
<223> n = a, t, c or g

<220>
<223> nbla-10999-f

<400> 269

gnnnnnnggg ggggnttnnn ntttgannnc ctttgngcac tgctggccta ctggctgttc 60
cagaaaaact gttactcccc ctcacccact ccctccttcc ccagttgttc ccttcagact 120
caggggctcc accaatgcca tcccaaaaca gggtcagaca ctgcccagct tccctccagg 180
aggttcttgt ctctgtgtaa gggcttgtct ccctcccagt ttttcttttg ctccacgtca 240
ttttgtcagg ctggttataa gccggaggca gctttaacca gcccccaggg atgattgtga 300
aggaggcccc tccccttgtg aggaggggc actcctctcc agcccctggt accacagtcc 360
tcacgatggt gcagtgattt ctagccaggc gtcaagatgc gctgctttcc ctctcctgcc 420
tcatcccttg ttggcagctc cagttcaggc cgtggaggga cgtgatgctg ggctgtgttt 480
actaaaccca cgggttttca gcctcttaag cccagctccg atctccaatt aagttgagag 540
cgctgggttg actaacctct ggtatctgag cacagacaga gggtgctgtg ggtctgctgg 600
gtggcagaaa tggttccttc cggcttggcg ttctctcctg gcactcttnc tgctgcctct 660
gactactcag ccttggtttc ggngtgaagc cccaactgcc actgggaact ggcggctnat 720
gcttgct 727

<210> 270
<211> 731
<212> DNA
<213> Homo sapiens

<220>
<221> misc_feature
<222> (1)...(731)
<223> n = a, t, c or g

<220>
<223> nbla-10999-r1

<400> 270
gnngggnnnn nnttgtgngg ccttttnttt ntcttttttt ttgcagggag tgcaacattt 60
atttcataac agaacccctt ttccacagag cagctgacag ggggctgcat gaaacatact 120
ttggaaatta aagtgaactc tccacttggg cataatgtta tgtgggcaca tggattggct 180
taaaagggaa acaagaatac ttcaacattt gatcaacagt aggcagttgc tggacatttt 240
agaaaaagga gaaatccatt ttttgaccat ggctaaacat ggggaaacag catcacattt 300
tcctgaacca ccctaatccc agcccctcaa gatccaccag gtatgcaacc ccaaacccca 360
gtcacataca ttaaatctac acttttattt ttttgttgta aaatgtgctt tttcctcaat 420
gaactttaat cagtccagga cctacacaca cacacacaca cacacacaca cacacacaca 480
cacacacaca cacacaccac ccccaccctn caaccaggtg aaaaatcccc ggcgattagt 540
ctataacaaa tattgacacg aggggggaca aaagcatgtt tgaaatgtat tagaatcaag 600
aacctgtaca aaaaaaaaaa attaaaatat ncctgaaact gtcaagttca aaagtctagt 660
taagtcnttt ctcttgccan gagaatgcca tcatcagaag gctctgggtc atnaacccaa 720
ggngggtgna a 731

<210> 271
<211> 1834
<212> DNA
<213> Homo sapiens

<220>
<223> nbla-11030

<400> 271
   ctgtacggaa gtgttacttc tgctctaaaa gctgcggaat tcctcgagca ctgttggcct 60
   actggtggag tcaagctaat agaatatttt ctgaggaaca ttattatttg tggaatattt 120
   tttctaccct catccatttc cttctctgca tttttctttc ttactggtct ttgtacagat 180
   tttattcaat tttctgttaa agttgtaata gtatgcataa gacctgtggc attgagtact 240
   tttttaaaat cccacataga gtcaaaaatc ccaattactt gcatttgtta gttgctgaga 300
   agacagacct tataaagagc tgaattggtt aaagtccaga gcaccaattc atgatgttgt 360
   gaccaattta caaagacatt cagacacagc tggataccat agacaaagat gaaattatga 420
   taatgctgtt caaaattaac ggtccataac tatcaaaggc aggtgcaagg caaattgaat 480
   ttggatattg atgaggctga aatggatagt ttcatacagg gaccttggat ttatactgtt 540
   gcttttttat ggccatgtta aaagcatcta ctttccccat gggaggaagg tgcatgctga 600
   gtgatcctgt tgagctgtca ctgctctgtc aggagattct tgttgatgga catatgtctg 660
   accacttgag aattgtgttg gagtgaaata cacttgcata agtcaattat taatgacagt 720
   tcctttagca actcccagag aaggtggggc atgacttctt ccctggagct gacttcagac 780
   aaattcacag atgctaaacc ctggcttttt tttttttaac attttaattt cctctcatag 840
   aatcatcaca aaataagaaa acacttcttt atatcgtaat cataattcca gtgttttcag 900
   ttttgtttcc tttttccact aaaatcattc ctgtgtttca atcagtaaag tgggcttctt 960
   gatttcattt gggatttgta tttgtgtttt tgttttccat tcgtttatgt ttctttgaaa 1020
   ggcgctgtga aacgcctgca ggtatggtgc tagccaagtg atctctagag acctagattc 1080
   caaaaatcca agccattgtc catctgaatg ctataaactt catggacatg ccctcacctc 1140
   atgagtgtcc agtgcctctc agatgcaccc tgtatattta ctgttcatcg tggaactcgt 1200
   gccactgaaa atttttaagt gactatattc aaaaacagca ggttgcatga cagtttctca 1260
   gtgaagaggt tcaaaaaagg tgagatgcta ttgctttgtg aatttacaaa ggaaagaata 1320
   atttaactgc tcagaattac atgtccggtc actgcttttt aatttaaaaa ataatagagc 1380
   atcattagta atcttgtttt ctctttgata cataggtaaa gggtgttttg tgtctggatg 1440
   cctaaggtga ttccagggga ggggatggaa gatatgtgac atcttccctg aaatttatat 1500
   tgatatgcaa tgctttgtca tttaaaacct aagctaatgt tttctacaat ccataactct 1560
   gagtttatct ttttggaaac atagaagggg atgacattga agatgaaatg gatacagcaa 1620
   ttgctgaatg acagtttgcc caaattagtg cagttaaaat atgctgacgc ccctgcatgg 1680
   ccaggaagac ttctgctcca tgcacacaag caccaagtat caagcgacca ccaacacgtt 1740
   cccattcctt taggcctcca tagctttgct tttgctttct gtttcctgaa ctaaaaaaaa 1800
   aaaaaaaaaa aggccacatg tgctcgagct gcag 1834

<210> 272
<211> 1871
<212> DNA
<213> Homo sapiens

<220>
<221> misc_feature
<222> (1)...(1871)
<223> n = a, t, c or g

<220>
<223> nbla-11042

<400> 272
gcctgtacgg aagtgttact tctgctctaa aagctgcgga attcctcgag cactgttggc 60
ctactggttc aaaagaactg ctgcccagtg actccgtgct gcacggaaga atatcagctc 120
cagaaacaga aaagatagtc ctagaggcag gaaatggatt accatcctgg aaattcaatg 180
accagctttt tccctgtgac gtgtgtggga aagtgtttgg ccgacagcag acattgtccc 240
gacacctctc gctgcacaca gaggaaagaa aatacaaatg ccacttgtgc ccctatgctg 300
ctaagtgccg tgcaaatctg aaccagcact tgaccgtcca ttccgtgaag ctggtgagta 360
cagacaccga ggacattgtc agcgccgtca cctctgaagg cagtgatggg aagaaacatc 420
cttattatta cagttgtcac gtgtgtggat ttgagaccga gctcaatgtc cagtttgtca 480
gccacatgtc actccacgtg gacaaggagc agtggatgtt ttcgatctgc tgcactgcct 540
gcgacttcgt caccatggag gaagcagaga taaagactca cattggcacc aagcacacag 600
gggaagacag gaagaccccc agcgaatcaa atagcccctc ttcatcctcc ctctcagctc 660
tgagtgattc agccaacagc aaagatgatt cagatggctc ccagaaaaac aagggcggga 720
acaatctgct ggtcatctct gtcatgcctg ggagccagcc ctcactgaac agtgaggaaa 780
agccagaaaa agggttcaaa tgtgtttttt gcaactttgt ctgcaagacg aagaacatgt 840
ttgagcgtca tctgcagata cacctcatca cccggatgtt tgagtgtgat gtgtgccaca 900
agttcatgaa gacccccgaa cagctgctgg agcataagaa atgccacact gtccccaccg 960
gtgggctcaa gtgcccattc tgcatttatt ccaccaaccg ccccgctgcc atggagtgcc 1020
acctcaagac ccactacaag atggagtaca agtgccggat ctgccagacg gtgaaggcca 1080
accagctgga gctggagacg cacacccggg agcaccgcct gggcaaccac tacaagtgcg 1140
accagtgcgg ctacctgtcc aagaccgcca acaagctcat cgagcacgtg cgcgtccaca 1200
ccggggagcg gcccttccac tgtgaccagt gcagctacag ctgcaagcgc aaggacaatc 1260
tcaacctgca caagaagctg aagcacgccc cacgccagac cttcagctgc gaagagtgcc 1320
tgttcaagac cacacaccct ttcgtcttca gccgccacgt caagaagcac cagagtgggg 1380
actgccccga ggaggacaag aagggcctgt gtccagcccc caaggaaccg gccggcccgg 1440
gggccccgct cctggtggtc gggagctccc ggaatctcct gtctcccctg tcagttatgt 1500
ctgcctccca ggctctgcag accgtggccc tgtcggcagc ccacggcagc agctcagagc 1560

ccaacctggc actcaaggct ttggccttca acggctcccc tttgcgcttt gacaagtacc 1620
ggaactcaga ttttgcccat ctcattccct tgacaatgtt ataccccaag aaccacttgg 1680
atctcacatt ccaccctccc cgacctcaga ctgcgcctcc cagcatcccc tcacccaaac 1740
actccttcct ggcctatctc ggactgagag aaagagcaga gactgtctga gggcagccat 1800
gttctgtacc aaaaacagag agacaaaaga caaaaaaaaa aaaaaaaagg ccacatgtgc 1860
tcgagctgca g 1871

<210> 273
<211> 2169
<212> DNA
<213> Homo sapiens

<220>
<221> misc_feature
<222> (1)...(2169)
<223> n = a, t, c or g

<220>
<223> nbla-11051

<400> 273
ctgtacggaa gtgttacttc tgctctaaaa gctgcggaat tcctcgagca ctgttggcct 60
actgggtctt aaatttgtta agctaaatat atgttggttc tttttatttt ggaatccttt 120
aagcatctta aacatttttt ttttgaagag aagttacaaa taacatttct atcaggtagt 180
acttgtatga aaccaccttt cttattctat aattttgatt tttcaatttt atatacttaa 240
tatactcact gtcttactat cagaaagtta ttttgaccaa gatttttatt atcttcatag 300
attcagaaag agatgctaat tctgtaccaa tgtcttcctg gttactattc tcttccctct 360
aatatatact ggccatttgt aaaaccattg tgttgttggg atcacttagt tatactatac 420
gcagatagag catctcaact ctgtcatagt gttcgctgaa cagttttcag tgtcatgcac 480
ctttggctgc taattgttcc tgacgtgcac tcttccgagt tggtaaaggc acagtgtgtt 540
catgccagac ttctaagaga aacaccagcc tcttaaatca gaagcctaca cacaaccccc 600
ttaacaatcc aaagaagctt gatggtgtgc aaagaagcat cctgccagcc ttgtcattgt 660
tctgttctat gctaatcctg ctgtgttgtc taaaagatgg agggaagagg acatcagtgt 720
ctgatagtga aatcatcagc aggaaagtga agctctttcc ttggttacag ataagacttg 780
gtttacacta ttggccagta tctgctaaac atatgaagac ttaactattc agtgttgcct 840
aggcattcgc ctgcacaaca ttttgaggtt agaacataga atattttcag aaatactgtt 900
gtagtttgtg agtgttgttc attagttaca cattagctat agagtggatg catgaagccc 960
catgacacca gtaaacttct cttaccagta ggtaaaccaa acaccattct gtcattagca 1020
gccctcttaa atgttgcctc tccgtatcct gttgcatttt tgtgtgcatt gtgtttctac 1080
tgatctctct taggttttta cggaatcaaa ggaaactaat ttttccttaa tagcaagaaa 1140
gatgaagagg taaagggcat tgaagcagaa atgtatagtt tggggtacga ttagaaaact 1200

cgtaaggaaa acagaagtcc taatttcaaa ctgactgctc ttcgttaagt gctcttaagg 1260
agagtctagt aacagtaaca ctttctggcc atttctagtt tagattctct tcgttactga 1320
aacttttgag aaatattacc tgtggattaa ttttgcacaa tgttctattc tcataatgac 1380
ttacaaatta aactaggttt ttactgaact acctcacact aattttctat gctttcccaa 1440
gtaagctgtt gccctgttgg atctttactg agtgaattat aaatgtgtgt taaatacttt 1500
ctagccaatg ttgacacaat accagtaagt atgtaaagta tataccttac atcagtaaga 1560
gacacgtgta aaatctttga ctgtatgtct tgcaaaattg tgctcgttga cattattact 1620
gtttttgtaa gtagaaacct gctcgtgata tcggtccatt tacattttac aaaaggagta 1680
aatcttagta aaaattttac gaagaaataa attacttttg taggcccaat atttggtata 1740
tttttgagaa gctgttaatc ttttagctga ataatgaagt tagactgaat tacgtgtctc 1800
cctggactgt gacatctatt ttctcattac agtttatcct ggtcagcagg gtgtcacacc 1860
tggaaacctg agtatgatag ctgacatttg cttttctccc tctgcgatgt cattcctcct 1920
ccattcctct ccttccctgt gttccgttcc ctctcctttc ctctagacaa aacaaaatgg 1980
ggcacttttt agggaatgct gagaacatta ttgtggtttt tcatcattca tgccctagtc 2040
attaaacatg caccactgga atgtaaacaa tgttatctag tatgtcaatt ggttataata 2100
ttttaaataa aaaagaaaaa agtggtatga aaaaaaaaaa aaaaaaggcc acatgtgctc 2160
gagctgcag 2169

<210> 274
<211> 733
<212> DNA
<213> Homo sapiens

<220>
<221> misc_feature
<222> (1)...(733)
<223> n = a, t, c or g

<220>
<223> nbla-11067-f

<400> 274
gnnnnnnggg tggnttntnt ttganncctt tggcactgcc ggcctacttg aaacacttgt 60
acttctggaa atggactgga gactttttaa atttgagtcc actattgaca tggaaacccc 120
agtggaatca gatnntccct caaagaccat gatggtatcg gactagtttt cagacactgc 180
ctgttgctgt ccatcagcac ttggtctctt atcttcagtg agaaggtgac ccgccttctt 240
cccatggtgg ctgcctaaag tgcttctttt ctaacccaaa acagttctac tcacttcctt 300
ttacagaatt caccggccat tttcctgtta cctgatcctt ctacaggatt tttaaaaagt 360
aagagagttt cagagaagcc gatcccataa tccccagtgc agccagggtt tgtgtgtcat 420
cgtactggag tagagggccg actcttccca tgaaggtgag cacagctgtg agtgagtgag 480
ctcatatctc catttgtcag tgctggactg gtaccagatc gtaaccttcc cgttggtcgg 540

aaacttttcc atctgtcgcc tagaaaaaga gaaagcttac catcgagggt gtggntgatc 600
cttgaagctg cttggtaaag tatcatttct caagcttttc ttttggactc ctatcatgna 660
tcattaatat ctaccagtcc cttttcattc taagacaaaa catttnttct aaatctctga 720
ataaaatcag ggg 733

<210> 275
<211> 736
<212> DNA
<213> Homo sapiens

<220>
<221> misc_feature
<222> (1)...(736)
<223> n = a, t, c or g

<220>
<223> nbla-11067-rl

<400> 275
gnnggnnnnn nntttgagtg ccctttgaan ncctttgtta ctggcgaaac tcatctttat 60
taaacttaaa cagagaagct acttctgcac atgttaaaca cacatggaac caaacagctc 120
gatggagaca ccctgcactg cttatggtga tgctgcagga atggcggcag cgatgcccgc 180
tacactccat ggcctgctgc acatgtgaca ggacagttat ttcatctatg cttacacctg 240
caggtccctt accctagaaa cactctatat cttcttaggt acttcccttg aaacaggatg 300
agttctagag gaaataaaac tgctgcactt acaaacctag aaatattcag ggaccaagat 360
tagttgttca gtgacctagg aaaccttaga atcctgtagg ttcctcagga gaggtgcttc 420
caagtacttg agaacccaca ggcagagatg cttccctttc catggtggat gtgtatgtca 480
ctccaggaca aactgcggag tcacggagtg tggaacacat ctggttgtgt acagtacact 540
atcccagagg aaaatcacaa agcaacacaa ctctgcagca aaaactagtc ccaaagtcag 600
aagataaaac tagcaaaaac tttgccaata tttagagaaa agctctangg tgtggaaaag 660
tccgggcttc ctttttctnc atgcccaagc agagccagta tcattcctta agctntaaca 720
aaaacccttt gnataa 736

<210> 276
<211> 719
<212> DNA
<213> Homo sapiens

<220>
<221> misc_feature

<222> (1)...(719)
<223> n = a, t, c or g

<220>
<223> nbla-11090-f

<400> 276
ggnnnnnnnn tgnnnnccnn tttgagancc ctcgagcact gttggcctac tgggtaaaaa 60
gactggctct ccaggaggcc ctggtgtgag tggtggtagc cctgcaggtg gggctggagg 120
tggaagcagt ggtttaccac catccaccaa aaagtcaggc cagaagcctg tcttagatgt 180
tcatgcagaa ctagacagaa ttacacgaaa accagttaca gtttctccca caacacctac 240
atctcctacg gaaggagaag cttcttgaag ataccaaata aagccattta ttctgttttc 300
tgggataatg taaacatgcc tctgcctttt ccttcaaaag tggaattaga aagctggagt 360
gcttcttcag atggactaaa tttatgtcgt gtgtgtgtgt gtgtgtgtgg ttacccattt 420
tttagaagga gccgtacaga agaaaaatta ttctacatta tgtaggattg ctgtttgcat 480
tgccattttg cataagaaag taatttttga ttttgaaaat ctcaaaactt ttanatctga 540
aatacagcca tgtgatcgat catattctaa aggctattta aaacatgtaa aaggatttgg 600
ggaacggcag aaaacatgca gtttgggcat ttgactgact tggaaagtct aagcttattt 660
taagttataa ctattaaaat catttttaaa aatttggtaa gtttgctgac agagaaaaa 719

<210> 277
<211> 715
<212> DNA
<213> Homo sapiens

<220>
<221> misc_feature
<222> (1)...(715)
<223> n = a, t, c or g

<220>
<223> nbla-11090-r1

<400> 277
gaggnnnnnt ttntgtgccc tttntttttc ctttttttgaa cctgaatgca nagaataaat 60
actttattaa ctgattacag ttgaaagtca caaacaaaaa aggggtata ttaaggcaaa 120
gccatatata tatatatata tgtatagaca aatccaaaga ttgttacttc tttacatttt 180
aataacctct gcatttttct tttacacatt taggcagtgt gctttctttt ctcctcagtt 240
aaagcaacac cttggccaat atttctaaat catattttga tgacaactta caggatcagt 300
gattgagaca atgactagtg tatcaaacgc tatctgatag ttttcttagc atattccgtg 360
tgtggtggta catttattgt ggtgtgcaaa atctgacaac tgacgatttt tctctgtcag 420

caaaactaac aaatttttaa aaatgatttt aatagttata actaaaataa gcttagactt 480
ccaagtcagt caaatgccca aactgcatgt tttctgccgt tccccaaatc cttttacatg 540
ttttaaatag cctttagaat atgatccgat cacatggctg tatttcacat ctaaaagttt 600
tgagattttc aaaatcaaaa attactttct tatgcaaaat ggcaatgcaa acagcaatcc 660
tacataatgt agaataattt ttcttctgta cnggtccttt taaaaaatgg gtacc 715

<210> 278
<211> 2961
<212> DNA
<213> Homo sapiens

<220>
<221> misc_feature
<222> (1)...(2961)
<223> n = a, t, c or g

<220>
<223> nbla-11110

<400> 278
ctgtacggaa gtgttacttc tgctctaaaa gctgcggaat tcctcgagca ctgttggcct 60
actggagttg cagggtcctt tttggaagct cttgccgtca tcgctgactt gggcaatggg 120
gccggtgggg tttgggggcg gaagagaccc tcggggttga gaagtatgtg gtggcctttc 180
gtcccctgta aaacattgtc acacggtgtg gggcggcagc gctggatctt tgcaagttaa 240
gtaggcgctg gcgttgttta cttgtcacct tctctgtatg ttttttccct cattttccct 300
acccttgggg ctagtgagtc tccaacctct gtgcccattg tgtgattctg ccttgatgac 360
gtgagttgct cgttctgact tccaggtgtc acattctctt ctaatctttt tttgattggt 420
gtagatacct gtggagggcg gagctgtagc cctctttatt accagtatcg gagtatatac 480
tactacctgt ctgacctcag gtgctttctt ttccaacgat cttatttctg catattcagt 540
gagcaaagtg tagatagaaa tgtctcagag tggcctggct attacttccc tgttcatatt 600
aaaacccttt ggtttatttg ggagttcaat ttatttcctt tcataacttc tctaccttct 660
caaatcttct cttttctcat cgtccgaggc tgtagttact ttctgttctc acgtattttg 720
acctttttgc attgcaaaac aaaaacctcc agttctggaa atcaggcttg tttggtttta 780
ctttaattgt cagatccagt tggtgtctgg tacctatgta gtatgttcta gtagattttt 840
gcagtagaga ctaaaaaaag aaaataaaag aaaagaatgg tgtgaaaatt taagaaaagg 900
atcctcttgt tactgccatc actgtcacag ataaaaacac tgcctttacc ctggaacatg 960
ttagtcctct cttggaggaa atgaaaccct aaaggttgac tgtggtctct gcagccaccc 1020
agtcctgtat ttggagagcc ccctggcttt tccagtccat cagatttagt ttgtttagaa 1080
aaaaataaat aagcatgtaa tgtatgaaag ttacgtgaga ctatcttggc agttataaat 1140
taataaacat ttacggtaca gaagtggtgt gaattgtaag aggatgtaaa ccaaacaaaa 1200
tacggcttta aagagggcct gaagataagt gagaaaactg aaattcttga aacatgtatt 1260

ttatggaccg aaagccaaag tgtgactata aataaagcag gacttaagag aatagacaac 1320
aatattggaa gaaaatgaga attgtggtag agtttggata gatgaagaag agaggccatt 1380
taagttggca atattacagc aaggacaaat aaaatcagaa ttgaatgttg tcgtggataa 1440
aagcattagg aagaaggact tttgtttaaa tttctgcaaa tctctttaaa tcctggttta 1500
ttagaagaaa ggctggtttc ttttgttgtc ttctacattc agtcttttgg ggtatgttat 1560
tttagtcgaa gtgtatggag aaaatacagc tgcaatgata ggtagtcaga aacagaggag 1620
cgtttttcag ataattgtgg atgtttttg acactacacc aaagctcaac aagtggaaga 1680
ttcatgtcag ctgcaaggtg gaatctgaaa ccctatcaat gagcatttca ttcattgtta 1740
cattaaaatc cattagccca tcttgcactt tgaaaggctc ttttacccat gcatggtttt 1800
ataacatgat gccatggcca cttagaaaat gttggtttac tgagttatgc acatctttta 1860
aatatggata cgtttcatta cacaatatca aaatatcaca tttcattaaa ttacaactga 1920
tcttgtcaga aatgtctctg aaaattctaa gtattgaaga gttgtcaagc tcatacttgt 1980
ggatgtaagt ctttcaaaat ccaattttca tttgaaagtt tgactcataa ttatcattgg 2040
tatcaaatac tgtcagtttt tctttaagtg atagattccc tttgttcatt tgacaaaatc 2100
tgccaaaaag cgaagtctga ataaccagtt tgacaattgt tctttcaagt aaaaatggtg 2160
ttccttgaag agtggttggt ttagctttta actctaaact tttcctggag atagccatca 2220
catcttggta tgcagcagga ctttatgtat gcatgcttca tattttaccc cacaaagtat 2280
taaaaagagt caaggttgaa atttcaataa aatgaattta tgctgctttg tgaagcacag 2340
tcttaattta aattgtccct ttttgaactt gtggtgaaga atacaatgat tactggtgca 2400
gtttggtgcc actgtcttga ttcatgctaa tgcaccagta gttttaccca ctgtggctat 2460
gaaccatgag tgcaaatgtc agcacagtga aaaaggcaaa tagcatctta gtatttatta 2520
tgagaatagt tttcaacttc tgaatctcca gaaaggattc tggggactac cagggcctag 2580
ggatttcaca cttggagaac ttctgccaat catctactaa atattcagga catgggccag 2640
gtgtggtggc tcacacctgt aatcccagca ctttgggagg ctgaggcagg tggatcagtt 2700
gagctcagga gttgagacca gcctgggcag catggcaaag ccccattcct actaaaaata 2760
caaaaaatta gccgggcgag gtggcaaatg cctgaggttc cagctactca gaaggctgag 2820
gtgggaggat tgcttgagcc caggaggtgg tttgtggagg cagttgcagc aagctctgat 2880
agtgcctggg caacagaggg aaacttcgtc tcaaaaaaaa aaaaaaaaaa aaaaaaaagg 2940
ccacatgtgc tcgagctgca g 2961

<210> 279
<211> 701
<212> DNA
<213> Homo sapiens

<220>
<221> misc_feature
<222> (1)...(701)
<223> n = a, t, c or g

<220>

<223> nbla-11176-f

<400> 279
tngnnngntn nngtggggnt cnnnttgaan nccttgggca ctgtnggcct actggggaaa 60
ggagtgttcg ttctacccag ggtaccacag ttccccacag tcaaaaccca aaagcaagga 120
gatgagttga aagacagttt ttctttaagt catcagtatg ggatgtcagc agaacaaaaa 180
ttaaaaagat taattttcct tttgatctaa aacttcctta gtttgagcag taggtgctac 240
aaaattattt acatatctta gtatcatagt taaatgtaat gtgtttagga gaggaaaaca 300
aaagatacat ttgctttaaa ttcattaaga aattttcaaa ttcactttgt agcccatgct 360
gatagaattg ggctgtgttg gtacatttga aacactgttt atgttgcttg aaacacttat 420
ttatttaatc gccgatgtga tgatgcctat ggccgagatc aaatatagct agattggcta 480
gactacttat ttgtttactt aaactatggg aagaagcata ttattgtgtc attctgttgt 540
gtgtgtatgt gtatatacaa tataaatata tatatataaa gttatttttt ctttgggtta 600
atttattata agttgtaaca cttggctagt tttgnttgna tatgtcttaa aatgttttct 660
tatgatattt aagtgacagt taaagaggta tcaaggtaac t 701

<210> 280
<211> 704
<212> DNA
<213> Homo sapiens

<220>
<221> misc_feature
<222> (1)...(704)
<223> n = a, t, c or g

<220>
<223> nbla-11176-r1

<400> 280
gnagttggtg gggttnnntt gatggccttg ttgnnccctt tctacacact ttcatttatt 60
gtctggacag ataaagtaca aataattcaa acacaacaag attatgaaaa aaaaacattt 120
acaatacttt cccttggaaa tcatctaaac tagcaagatt aacatgaagt cattcattaa 180
taactaaagt atacaactgc ctggtcctaa gatttttttt cctttttttt tcccccaaga 240
ttgaacacat tatagagaag attttggcat gcctttccca ctaatgctta gcaaatgcat 300
caaaactttg ccagcaaact acgttcattc atagcaggtg acgtaaaaag ctttccctga 360
atgttgaacg agtgttctct gaactgaaat ctggtgttat atccaaaacc gaaaagagga 420
aaaaaaaaca gaaaaaaaat ttaaaggagg agtctatgat gcagccttgg tattcgttat 480
ctgtctacca ctacaacgac tactacaaag tttgcctttt cagtgtgaaa acaacagtcc 540
tgtctacatg cacttagtgg gttcagcatg ggaaaaaatg cacaaaataa gcacangtta 600
taaataacca taacgtttta ttaccattaa gactaaactt gnattgaaaa gattttatat 660

aacangacan gaaaagcaat agcaaattta actatcaact agat 704

<210> 281
<211> 2035
<212> DNA
<213> Homo sapiens

<220>
<221> misc_feature
<222> (1)...(2035)
<223> n = a, t, c or g

<220>
<223> nbla-11189

<400> 281
ttctaggcct gtacggaagt gttacttctg ctctaaaagc tgcggaattc ctcgagcact 60
gttggccggg tggtgcttgg gtggtcgcca ccaagaagac tttggtgggg tagtctcggg 120
gcagctcagc ggcccgctgt gcccgtttct ggcctcgctc gcagcttgca cgtcgagact 180
cgtaggccgc accgtagggc gagcgtgcgg gtcgccgccg cggccgcctc ggggtctggg 240
cccagccgca gcctcttcta ccgcggccgg ttgggagtcg ccgcgagatg cagcctccgg 300
gcccgccccc ggcctatgcc cccactaacg gggacttcac ctttgtctcc tcagcagacg 360
cggaagatct cagtggttca atagcatccc cagatgtcaa attaaatctt ggtggagatt 420
ttatcaaaga atctacagct actacatttc tgagacaaag aggttatggc tggcttctgg 480
aagttgaaga tgatgatcct gaagataaca agccactctt agaagaattg gacattgatc 540
taaaggatat ttactacaaa atccgatgtg ttttgatgcc aatgccatca cttggtttta 600
atagacaagt ggtgagagac aatcctgact tttggggtcc tctggctgtt gttcttttct 660
tttccatgat atcattatat ggacagttta gggtggtctc atggattata accatttgga 720
tatttggttc actaacaatt ttcttactgg ccagagttct tggtggagaa gttgcatatg 780
gccaagtcct tggagttata ggatattcat tacttcctct cattgtaata gcccctgtac 840
ttttggtggt tggatcattt gaagtggtgt ctacacttat aaaactgttt ggtgtgtttt 900
gggctgccta cagtgctgct tcattgttag tgggtgaaga attcaagacc aaaaagcctc 960
ttctgattta tccaatcttt ttattataca tttatttttt gtcgttatat actggtgtgt 1020
gatccaagtt atacatgaat agaaaaagat ggtgttaaat ttgtgtgtag gctgggaatt 1080
cttgctgaag gaattggaga aaacctgttg ctgcaaaatt ttacatgttc cagatggaaa 1140
gggaagtcta agcgcttttt aaaacaattt tttttgtat ttaattaagc aattgcagtt 1200
atctgggatt tttgggtcag aattttaaat tctgtttgat tctccatatt ccagtgaata 1260
aaatacaaaa gcattgtgtt tttaagattg tgtcgatatt cacctaaaaa cttgtgccaa 1320
aagcacctgg attggtaatt atatttcact taaagggtaa atttgacaat atcttgataa 1380
tcaaaagtgc aatttttttc ttcaaaatgt tttctccagc atcacagatc ctgcagatat 1440
atatttatat ttatacatat atatttatga aataattctt actcacaaaa tatatttctg 1500

ataaacatta agatattaaa tctgatgcac aaactttaat ttggccatta atctttttta 1560
tttaaaaatt taaatttgtt tttaaaattg tatatagttt ttaaaatctc acacatgctt 1620
cgatacttcc ttgttaagaa ttcttaataa ctactaaaac tgatttttaa tagttgctga 1680
tatatatttg gtttgtttgg gtatactttt caaaaccatt tttgaatgtc caaacatctg 1740
atttaaagtt tctgtttatc tttctgacca aaggagcaag aggtataatg gatatggcat 1800
tcattaaaat ctttactatg tacaaaaaca gtaatattta cagcatcagt aaatattttt 1860
aagtggtact tctaaatcat aaaagttggg gaaagagacc tttaaaatct tgtggtgttg 1920
aacaatgtta tatgaagtag aaaaaataaa atacttccca gttgtgtgtt ttgttttaca 1980
aaaaaaaaaa aaaaaaggcc acatgtgctc gagctgcagg tcgcggccgc tagac 2035

<210> 282
<211> 726
<212> DNA
<213> Homo sapiens

<220>
<221> misc_feature
<222> (1)...(726)
<223> n = a, t, c or g

<220>
<223> nbla-11201-f

<400> 282
   gngggnnnnn gtgggntcnn nttgannccn ttggcctgnt ggcctactgg aggttggaca 60
   tccaacaaga tagactctaa ttcagcctcc ccagtctctg gccacccagc tcactcaggg 120
   attcagctac ccttggctac ccaagtagtc taaacatttt atctttatca ggcacatata 180
   cagtctggtt ctctgtagcc aaatttagat acccatgaca tttatctaat gatttcaaac 240
   caaacaggtt cctaaaaaaa cattcagcct acttctagaa cttccccatt ttgcttggct 300
   gtgaaaatgt atttgtccca tgcaggccct tgctccagcc cagtggaagc attagtacct 360
   cttcctagcg ggaactcagt ccagctacac accagagcca ggccagtggt tgtctgggaa 420
   atcttcaaag gccgtgacca ctcagcaagg gtcagtggca cctctcccca ctgtctcctc 480
   accaggtgca ttcatcccat ccatccatgc attcactcac ccttcgtgtt gtccagcact 540
   atgtatagtg ctgcagaaac aagaaggata tagtgcttgc cttcaaagac ctccaaagtg 600
   gggagatagt ctggatgaca ggtgctgtaa tagaagtgtg cccccggatg ctataggagc 660
   acagaaagan ggcaactgat gaacactggg gtgaaaagtg gaagangaga cctgggaaga 720
   ctcctn 726

<210> 283
<211> 727

<212> DNA
<213> Homo sapiens

<220>
<221> misc_feature
<222> (1)...(727)
<223> n = a, t, c or g

<220>
<223> nbla-11201-r1

<400> 283
gnggggngng tggggggcct nttggannnc nttttcaaga caggagtgca gtggcacgat 60
ctcagctcac cacaacctct gcctcccagg agaatcaagt gattctcctc tctcagcctc 120
ccgagtagct gggattacag gcacatgcca ctaccgccca gctaatttat gtatttttag 180
tagagacggg gtttcaccac gttggccagc tggtctcaaa ctcctgggct caagcaatcc 240
gcccgcctca gcctcccaaa gtgctgggat tacaggcgtg agccaccacg cccagccaag 300
gttcttcctt aaatgccgat gctctctgaa gtttcagctt cagcccttgc atctttctgg 360
tgggattgct actcagggct tcaagggtcg cccctaggag tgactccaag gtggatctct 420
ccatctctgc cttgcctatt tccagccagc tgtactcctt ctcctgggga acgacaggct 480
ctccatgctc aacacagtaa ctgctctcct ctgaccacac cgagatcaac ttctattcca 540
tcttccatgt ccagcagact ggagagtctg ggagtcaatc tcgaatcttc tcttactcct 600
tactttatta aggcactggg acctatagct ttcacttctt taatatctca tattggggtt 660
ccttcctttt caacttatcc tacagacact ttacttnang cttngccatt ctgncctgaa 720
tcaaata 727

<210> 284
<211> 704
<212> DNA
<213> Homo sapiens

<220>
<221> misc_feature
<222> (1)...(704)
<223> n = a, t, c or g

<220>
<223> nbla-11212-f

<400> 284
gatccctcgg cactgttggc ctactggttc ttccttctcc tctacccctc cttcccccct 60

ccccattaga gtagtgtgga gataaggctg gactggtcta tcagattgaa ctccaagaat 120
gatcacacaa aatgtttagg gagatgttcc ccgtggtgta tcctcatggt aacaacgaca 180
aaaaatgccg gttgtctttg ttctcttttc actattccta acatgtgtac atgatagctt 240
tgattctgca agtaaaagta aatcctgtgt tgtgactggt gctttcatat atttgtgaca 300
atttttgagt aatattgcat gaaaatgtcc ctatgttaca tccattcaga agttttgttg 360
ttttactcta aagctgggaa aggaaatgag agggaaaaga ccccggagag ggaagaaaat 420
ctcagtattt tgaaaattga gattacttca gagccttagc cacacctaaa atacctccta 480
gttaatagtg gtataaatgc ctcttcaata cgttttccag aatccaaagc attttggttt 540
atccaggacc canggcagca cagctgtcac caagcaggag agttaaggat tcaccatgag 600
ctgggaaatg ctttggcatg agtatgagca aattccctct ttcctgaatc atggacattc 660
tagattaaaa gaacattttt gggctcttaa caagaaaacc ntgg 704

<210> 285
<211> 720
<212> DNA
<213> Homo sapiens

<220>
<221> misc_feature
<222> (1)...(720)
<223> n = a, t, c or g

<220>
<223> nbla-11234-f

<400> 285
tgttggccta ctggtgtggt cctaagacct ggagaggaca ggctaagtga agtgtggtcc 60
ctggagccta caagtggtct gggttagagg cgagcctggn aggcagcaca gactgaactc 120
agaggtagac aggtcacctt actacctcct ccctcgtggc agggctcaaa ctgaaagagt 180
gtgggttcta agtacaggca ttcaaggctg ggggaaggaa agctacgcca tccttcctta 240
gccagagagg gagaaccagc cagatgatag tagttaaact gctaagcttg ggcccaggag 300
gctttgagaa agccttctct gtgtactctg gagatagatg gagaagtgtt ttcagattcc 360
tgggaacaga caccagtgct ccagctcctc caaagttctg gcttagcagc tgcaggcaag 420
cgttatgctg ctattgaaga agcattaggg gtatgcctgg caggtgtgag catcctggct 480
cgctggattt gtgggtgttt tcaggccttc cattccccat agaggcaagg cccaatggcc 540
agtggtgctt atcgcttcan ggtangttgg cacaggcttg gactagagag gagaaagatt 600
ggtgtaatct gctttcctgt ctgtagtgcc tgctgtttgg aaagggtgag ttagaatatg 660
ttccaagggt ggtgaagggc taaaatgnac gcntttaagg ttgnancccc tgtgcaaggg 720

<210> 286

<211> 758
<212> DNA
<213> Homo sapiens

<220>
<221> misc_feature
<222> (1)...(758)
<223> n = a, t, c or g

<220>
<223> nbla-11234-r1

<400> 286
gngngggnnn nnttgnnncc ntnnttnnnc cnntttggat tctaacactt tattaagagg 60
tcacaagcca caggacttta aagtgcatga aatttattgg caatgaagcc gcatgtatac 120
caggctcccc tagtccccac caccttgccc catccatcat gacgtggtgg ggagggttg 180
agacccatct ttaaaagtgg gggacagcag ggacaaagga tgggaaactg gaggaacagg 240
agtattcatg aactgaagct gggacaggag actgaccaca gtgattctgc tcactgaagg 300
aagggtgggg tcagtctagt ctctcaaagg ctggtgaaaa gtttttttc acctattcct 360
cacagcgagc ccctggcatt tcaaatagac ctaactcctc ctcttcatct ttctctggtt 420
ttctcctcta tgagtaattt ggaacccaca gtccagcttg agggtatccc tgatatgcac 480
aatgaataca aaccacggcg gatgcagatt tcaggatcat gctggggcct ggcccatttg 540
ggtgggagga gctttctgtc aggagttgct gggtgcgggg taccgtgtgt gcccttcant 600
gtggtgaagt gagaacatgt gaatgtgtgt gcatgtncca tccaaaccgt aagaggcaac 660
ttgggtcang aggaccccac ttnttccttt atgggacaac agtggantgg gaanaaacng 720
gggcttttga ntccanaaag aaagganagg nctncttc 758

<210> 287
<211> 714
<212> DNA
<213> Homo sapiens

<220>
<221> misc_feature
<222> (1)...(714)
<223> n = a, t, c or g

<220>
<223> nbla-11269-f

<400> 287

gtggcctact ggagcatcct ccttgctaga catggacatg acatttttaa aggaattatc 60
tggttacagc gttttaagca aactgttcta aggagtctgt acattgcatt atttttttcta 120
ttcttttttt cttttgcata tatgtaattc tggaggtaaa gaaaaagcct gccagggatt 180
cagaaggcat cccactagcg atcagctgac attcctaact gaaggctgca atgtgttgct 240
tattcatttt gtaccgtggg agctgcgggg actagcagag agctaaacta tgcatttcaa 300
acagcagtgc ttgtgcagaa agaggggtga gagagaggca gccggcgagg aaagagcaca 360
gctggacttt ctccttgntt ttatccattt ctgcaggatc atgtattcat aagggatgag 420
gcgggccacg gcgatcccag gcctgagccg cggctaccca gtcagttcag agccaggccc 480
tncactaccg gaacagagag cgctttgcac gatcaaatca gcatctttgg ttacacgaca 540
gatccatgag catgagcagg agaacgaagt tgcgggaaca agatgtcang gtataagccg 600
gatgccgcgg ccagcaccag aagcagctga tcgcctggag aacaagctga angctgagat 660
ggacgaacac cggcttcaag ctacagaagg aggtggagac gcatgccaac aact 714

<210> 288
<211> 715
<212> DNA
<213> Homo sapiens

<220>
<221> misc_feature
<222> (1)..(715)
<223> n = a, t, c or g

<220>
<223> nbla-11269-r1

<400> 288
gaaatggcaa aaaatttaat ctcatctgta gtcctcctta ggaaaatcta atgtaaccaa 60
attcccaaat cccattctga ggctctccat gncaaaagtt tnaatctctc gctcttgcct 120
ttccaatagg ttctttattc tctcgctgcg ttccttccga agggcagcca gctcctcttc 180
aatcttctgc tcaaggtgtg ctctgcgcag agacactctc tgctctagct tctggagctc 240
acgttcatgt tgtgcctctg tttgcatctt gattttgctc tggtaggccg ttgagcagct 300
ccatttcctg ctggagctgt agcctcaagg cctggcattc tgcttcttga gcctcatcta 360
gccgtaacgc ttgagaggcc atcatttcat ttatactctg tcatactgct ctgcaaaatg 420
gcaagttttc ttgtctgctc atctttcagt gtctttaaga ttgntttgtg ctcattcttt 480
ggagtaactt ccaactggtg attcttgagt gctttatact ggttggnctg gactttgcaa 540
gtgcctgaaa ctgnttttta atttgcattt ncatggcctt aagctttttg gctgttgccg 600
aaagtncatg acatgccttt ctgtgcagnt ctctttttnn gcctcttatt gnacttcaan 660
ctggttttcc nnttccgctg ggtgctgnna aaccngatca caatccatgc tancc 715

<210> 289
<211> 746
<212> DNA
<213> Homo sapiens

<220>
<221> misc_feature
<222> (1)...(746)
<223> n = a, t, c or g

<220>
<223> nbla-11270-f

<400> 289
gnngnnnggg gggnttnnnn ttgaanncct ttggcactgc cggcctactg gagatctgct 60
tctccccggc tcggggcgcc gaggcggcgt ccgggaggtg tcttctgcaa aggttgcctg 120
gcgctgtcca acatggngga ggcaccggca gcgggcgtta cctgcgagcg ggactaaccc 180
ctgcctggcg ttccgggcgg cagtgcctgg tccagagtcc agggactcgg ctgcctgcgc 240
gccccgaggc ggaccccggc tctgactgct cccctggccg cgggcttctc gactgggacg 300
cggcgcgagg agggagcgcg gcggccccga gtctccctga gccatgggca acgaggcgag 360
cttggaaggg gaagggctcc ccgaaggct ggcggcggcc gcagcggctg gaggaggagc 420
tagcggggcg gggagcccct ctacaccgcg atcccggccg gcatggaggc ggatttgagc 480
cagctgagcg aagaggagag gagacagatc gccgctgtca tgtcaagggc gcaggggctg 540
cccaagggaa gcgtcccccg gccgctgcgg agtcgccttn catgcacagg catgcacagc 600
atgatgtata tgtccgggca tatatgtaca gcttccgtgc acatatgtgc caacctgtat 660
gtgccaacac ggcggtacat atatgtcgnc tccgtcattt attcttcccc antggggtgg 720
ncataagaga tcctaagctt gtgatg 746

<210> 290
<211> 761
<212> DNA
<213> Homo sapiens

<220>
<221> misc_feature
<222> (1)...(761)
<223> n = a, t, c or g

<220>
<223> nbla-11270-rl

<400> 290
gnngnngngn nnnntttnnn ngccttnttn nnnnncnttt tataaagtgg aggataagga 60
tctacaccta tatatccagg tctttcgtgc ttacagtatc tcaaatgaaa aattgtaggt 120
ttgccttcag ttgtcataaa agaggaattg tttcagaacc acggacagaa aactgttctc 180
tactagaggg tgcaaagtta aagggtttgt ttacagtaag aaaggcaagg caggcacaat 240
gattataccc ctctcagtac tctaattaga aaacaactaa tctattggga tgaaggaaaa 300
tggagagtta caggcggaaa tgaaaacatt catattactg attagctctt atacttgcct 360
atgcaagttg ccatttattt gctttgtttt taaaagtatg tattgagaat tattttctaa 420
ggtaataaaa ttcacatgtt cttccctaaa ctgaaggcca cttttgcata aaacagagca 480
gtggcttaaa aatagcatat gctaatttag aaatatatgt acagcacctg ctctgctgtg 540
accaaaccat ctgtttaatg cccacatatt ttagggtcct caatgaatat aaaaggnata 600
ttcattctat tccttctacc taaacagctt ctgctctcct ctgggggtgg ggtggcaaag 660
aaanggcaaa tcacccacaa nggggattaa agttacagaa atatttagac atcattcaca 720
gagaaaaaag acccccaatg ggccaaaang tttaaacaag a 761

<210> 291
<211> 731
<212> DNA
<213> Homo sapiens

<220>
<221> misc_feature
<222> (1)...(731)
<223> n = a, t, c or g

<220>
<223> nbla-11279-f

<400> 291
gngnngngnt gnttncattt gaannccttg gaacactcct ggcctactgg catgtgaaag 60
cactttgaaa attgtaaagc gctatgtaaa tgtaaggtat tatngaaaca tctttaacat 120
atagtttcat accattcatt ttttaacaaa gaaagggaaa agtctgcttg taagctggtt 180
gaaaaagtta atcttgatat aaatttgtgt ttgataaata tcctctcagt gttttatctt 240
ccatgtttca acaactattg aaatatgaaa tgcctgtgaa ctcttaaagc ttcatgagca 300
gctgcttgag ttcgggaagt tcactgttag aaataggctt tgttagctga ctagggtcag 360
ggaaactttt ctcttcaaat ttgaaagctg nttctgtttt cattttacat tattattcag 420
aaatggtagc tattctatac ctatggttaa gtaaatattt ctgaataagg cttcaccata 480
ctgtaagcat tttaggtaga ttgccttaaa ggttatggga gggcatgagg gaacacttct 540
tatgagaaaa catttataaa caaaagaaac atttataaac taaagaaaaa ctaaaagaat 600
gacagaacaa tcatcttaac accctttctn acaataatat aaaaatatta aaagaacata 660
ggcaggcttt ttttaaattt ggcttttttc tttccttttt tcaaattgac ttttataggn 720

atttnctgga a                                                          731

<210> 292
<211> 734
<212> DNA
<213> Homo sapiens

<220>
<221> misc_feature
<222> (1)...(734)
<223> n = a, t, c or g

<220>
<223> nbla-11279-r1

<400> 292
   gnagnggnnn nnttgannnc cnttganttc cttttttttt ttgggtaaaa tcaaaatata 60
   ttttacttcc cattagattt taaaacaatc acaataattt atcaacagtt gccatttcac 120
   agcaattaga aaagaggagt tgatacaagt tgaacagaaa ggtatctttt cttcttttag 180
   gaaatatata tatatatgta tatatatttc acatacacac ataacattat ctttaaaatt 240
   ccaagtggtt acttttgtat tataaaaaaa ttatctccct atggtcaaaa tactgaaaaa 300
   gcgcaaaggc tgaaaaaaga aaaccacatt tgccacttta aaaaaataca ttattgtctc 360
   agtggttcta tttatttaat tgattataaa gtattatgac tcctagtact gtactggacc 420
   tgatggaatt aaatacaccc ctttctcagt ctccagaaag gatactccta acccacccta 480
   aaatatgata aaataaatgc ccctattctg aactgttaaa ctgggaaata aaccatgccc 540
   acctggaaac aatataagtc ataaaagttt atgaatctgt gggagagact acacgaatca 600
   gaaagcaatt ctttggatat aagttaggag cttttaagaa gtatataaac tcttttangc 660
   atctagagta gagacnccaa gaggatttct tcataaataa tgatttancc agngcaatgg 720
   ntggttgcca tggc                                                      734

<210> 293
<211> 761
<212> DNA
<213> Homo sapiens

<220>
<221> misc_feature
<222> (1)...(761)
<223> n = a, t, c or g

<220>
<223> nbla-11307-f

<400> 293
gaggnnnnnt tgnnnccntt nttgancccn ttgnncnncn ncnntgnnng atnnggataa 60
gctgctctca ttgagtagta cctcagnttc ttcatcttta aatgtaagta tctgccttac 120
ttacagagta tatgtgaatg taaagtgggg taattacatg aaagtattct gccaatgcag 180
tgtgatttta ttaatactag tttccttgtc tcacattatg ctcatttcca tttgtgtatt 240
tctgctactg ctacctacaa ttgtcttctt aaattttaca taatcaaagt cagtctctga 300
gcttctagct acccacaact gtgcaactcc tgtacaactc ctcctgcgtg cccacaggtc 360
tgttacaagt actcagggtt acactggtaa ctcttctctc agtccctatt ctgcatgtag 420
tcaaccggnt tcctatttag tgtctaatgt cttggttttt tttttttat tttcttcatt 480
agatttgaaa tgacttgatg gcaaggaata tatactttat atatggttta tacatttcat 540
acattttctg gnatttttat aggcctggaa tggaccttgg agctgaaaaa aatagacntt 600
acttggnatt actataagct agggaaatag aaagggttaa tcccaatata ttnctttata 660
acctggnggg taaccattcc tttctggatt ctgaanagnc cctacctttg gatacctata 720
ctaaccgaan ttttcagggc ttntgaacct taannaanat c 761

<210> 294
<211> 751
<212> DNA
<213> Homo sapiens

<220>
<221> misc_feature
<222> (1)...(751)
<223> n = a, t, c or g

<220>
<223> nbla-11307-r1

<400> 294
gnagngggtn nnttgngtgc cttnntnnnc ctttttgntt tttttgagac ggagttttgc 60
tcttgttgcc caggctgaag ggcaatggca cgatctcagc tcaccgcaac ctctgcctcc 120
cgggttcaag caattctcct gcctcagcct cctgagtagc tgggattaca gacatgcacc 180
accacacctg gctaattttg tatttttagt agagacgggg tttctccatg ttggtcaggc 240
tggtctcgaa ctcctgacct caggtggtcc acccgcctcg gcctcccaaa gtgctgggat 300
tacaggtgtg agccaccaca agactgagtt tatctatctg tttcatctct ttgatttgta 360
tttgtgtggn tttacgtagg aggcagtgat atggcgagta taaggtagaa gaaggcataa 420
ctgtgagagt ggatatggta tgcaatgcat acagaagagt catgaatggc tacactagaa 480
taaaacacct accaagatat gccatgctaa aaagctggga agtctatcag gaangcaaca 540

gaaaacagtg gacagacatt caaatggntg catataagca tttnctntca gttaaaaagc 600
nccactttaa angcnggaaa tccngaccta aatnccactt ggtactactt tttggttaaa 660
gcacaagctt nttaaaactc ctaaaattat tggataactg naaaanaggg taattgnncc 720
cttttaaaac ttggttnaac ttaaaaaaaa a 751

<210> 295
<211> 735
<212> DNA
<213> Homo sapiens

<220>
<221> misc_feature
<222> (1)...(735)
<223> n = a, t, c or g

<220>
<223> nbla-11378-f

<400> 295
gnnnnnnnng ngnnnntgnn gnttgtnttn ganaccttgn gcctgctggc ctactggtat 60
aaagtggtgg caagataggg aagggataat ctgagaattt ttaaaagatg atttaatgag 120
aagaagcaca attttgattt tgatgagtca ctttctgtaa acaatcttgg tctatcttta 180
cccttatacc ttatctgtaa tttaccattt attgtatttg caaagctagt atggttttta 240
atcacagtaa atcctttgta ttccagactt tagggcagag ccctgaggga gtattatttt 300
acataacccg tcctagagta acattttagg caacattctt cattgcaagt aaaagatcca 360
taagtggcat tttacacggc tgcgagtatt gttatatcta atcctatttt aaaagatttt 420
tggtaatatg aagcttgaat actggtaaca gtgatgcaat atacgcaagc tgcacaacct 480
gtatattgta tgcattgctg cgtggaggct gnttatttca accttttttaa aaattgngtt 540
ttttagtaaa atggcttatt ttttcccaaa ggtggaattt agcattttgn aatgatgaat 600
ataaaaatac ctgtcatncc cagatcattt aaaagntaac taaagtgaga atgaaaaaac 660
aaaattncaa gaccttttta aaagaatggc tggcctacac acttttatgg attggttttc 720
ttacatccca tcttt 735

<210> 296
<211> 737
<212> DNA
<213> Homo sapiens

<220>
<221> misc_feature

<222> (1)...(737)
<223> n = a, t, c or g

<220>
<223> nbla-11378-r1

<400> 296
ggnggnnggt tnctntgagg ccnttgtnna nccttttact aagaaagcca gtaactttac 60
tgaattgtct gcaaaataca aattatacct tatttccagc aggagaagct tttgaaaatt 120
tcccactttt attcactaca gacagctgaa ttaagtccct tgagatacac agatgtggtt 180
taacttagtt ataacatctt gtcatctagt ggcgacaaag tccagcttat gccgctgtga 240
gcctctattt gaatatttgt tgcaaatgct tgtatgccct catttacaag cgattttaat 300
tactctcatt taatggaaac aagtccccag gaaaagaaaa agaagaaaaa tgatacagca 360
aaccatctga agcaatccaa gtcctatgcc tacagtgacc caggtctact ttgctcgaag 420
agaacactgg tactgcattc aaaccacaac agaatccgtc acttaactga cctctctgaa 480
ttaaactgag gtatgaatca atttgaggta tgaacaggga aggcaaaatg caataccaga 540
ttatgaaaat aaccaaaatg gaaaaatttg atgtagaaga tatgtggctt ggattgctcc 600
acatctttgg gttaaaacat attaagaaaa gcctttaatt acatctgnag ctgaagcatt 660
ctaacagaca ccagtatttc agcaatcttc aaaagatctg tantggcagg gaaattaacg 720
ccataaaagt gacgaca 737

<210> 297
<211> 723
<212> DNA
<213> Homo sapiens

<220>
<221> misc_feature
<222> (1)...(723)
<223> n = a, t, c or g

<220>
<223> nbla-11379-f

<400> 297
gggnnnnnnt ngggntcnna tttgaatcct ttgagcctgc tggcctactg gcagatgctt 60
tttctttttc tgttggaaac tgaacacact acagacagtg aaaaaaggta catattccat 120
tttctcattg cctgaagatc tctgctgatg ctcctggaga atgactttgg gggctttaga 180
aagaatattg ccagtccgtc tcggcaagga gatgatggga gcgctttata tggaggcttt 240
acatgacttg taaattaaat gtgaatgagg gcagttgatt aaaattggta ttacagaagg 300
gccctgctga ggtttgaaaa cagctgagct gctgatgtct caggcctttc cctgaattag 360

cactgcggtt ctccaggata tcagcaaaga gggcaagtaa tagaagcccc tgataaggag 420
cgtcagccga caggcaagct tgggaggctg tgggaatggg tctgccccca gcttcacaga 480
cctcttcctt cagcctctga atcccattag ccacagccta gaacattagc tgagctgcac 540
aagctnaccc acccctgtgc caaggggccc tgacctcctt catgccatgt ttttggctgg 600
atctacggna cttaacaata nggggccttt tattttcata cagagatatt ttgaaaaatt 660
taaaaagaca tgaactnaca ttaacngnta tggatgatag gtnaaaagag aaaccggtgg 720
agg 723

<210> 298
<211> 718
<212> DNA
<213> Homo sapiens

<220>
<221> misc_feature
<222> (1)...(718)
<223> n = a, t, c or g

<220>
<223> nbla-11379-rl

<400> 298
gaggggtnng ttgtgtncct ttgatccttt ttaatatatt cagagcacag aatttattga 60
acataatatg gagacagcta gtacaaaagt ttatgccttg gcagggcagg tttcatcagg 120
ctgataatgg aacatgggag aacatgctgt aaatggctct tttggtctgg accctgcaga 180
aatacatctc cttctctctg ggaaacatca tgtcagctgc caagccatga atttattaca 240
tatcagaaaa ccaaaatgtg caacagtaaa gagtcctttc ctgcaccaac tttttaaatt 300
acttccatgt caaaacagca aatacaatga aaaatacata aaggacaaat tatgaaaact 360
aaagcattct aagacatctc ttaagatact cagttcctaa catgttaaca tacattgaag 420
actataaaca cacacatcag aaactgtggg aatctttttt caacataggg agaaagacgt 480
agaataggga gagacagcat gggttactga aaccatttat tcagaaagca gaggaaaggc 540
gaaaggacac tgaaatanac atttttaggt tcttatagga agttccaaat ggcagcagtg 600
ataaatcgnc tttaaaaang gaacgacagc accngaaatg agaacccaat ntattttgca 660
tttttctctc ggngnatcat cttgaggcac ccttacttaa gcttcacaac gaatggaa 718

<210> 299
<211> 726
<212> DNA
<213> Homo sapiens

<220>
<221> misc_feature
<222> (1)...(726)
<223> n = a, t, c or g

<220>
<223> nbla-11469-f

<400> 299
ngngnnnggn gggnttnnnt tgacnccttn gncctgctgg cctactggaa atatggggct 60
tcttggtctt tatatgcaca tctgagcatg ctactgaagg cagaggaagg gagatctctt 120
agggagtcag cacatgcaaa atgctgtgcc ttcagactgc ctctgctacc tcttacaagg 180
agcctcacac cgcaggctgc cctttagctg ttcccttgga atgcagcctt gttaagtcac 240
tgccagcgtt tcccctcttg gcgtcccttt ctcttaccta agttgttaac tccagacttc 300
gctgctttct taaaggctaa agtaatgggc agagtaaaac cctgaaatgt ttagaagcta 360
tgcagtatta tattgtaaac ctgtaaaaat tcttaacaaa gttagtggct tggaaagatt 420
ttcatgagaa cttcagctaa aaggcagtaa agtaaatgcc ttaaaataac taaactttgg 480
cttataacaa ggccattgtt tggcaagtgt ccacttttta aagacttttc agaacttgga 540
ggaggaaatg atcctgagac aaagtagtga tttaaaaaat gaagacagta aattaacatt 600
gacattattc gncaacaatt gattcttggc tttcaacttt ttaccctgca ttctgaataa 660
atggaccatg tgaaggacta gaagattact ttaagggttt ggtttgattg gaaatgggnt 720
tggaaa 726

<210> 300
<211> 3121
<212> DNA
<213> Homo sapiens

<220>
<221> misc_feature
<222> (1)...(3121)
<223> n = a, t, c or g

<220>
<223> nbla-11473

<400> 300
ctgtacggaa gtgttacttc tgctctaaaa gctgcggaat tcctcgagca ctgtggcctt 60
tttttttttt tttttttttt tttttttaat gtgcagagga ttgacctgtg catgcttttg 120
atctctcatt caaaggatca atattaaata aaattgtcat gagctgtgtt gaagacaggg 180
tgctttcaaa tagaggtaat ttgctcttgt gttgtaagag gaacatgtca acaaagatag 240

gaaatgaggg tgatcgtgca gatggcttgt atcttatata tgcaaaggag ccaatctcag 300
aagcacaaag aaaaaagtgt gcatacctta ttttgtacag ataaagatga tgtctttttg 360
ttattgtctg tctgttttgt atgtgtctga gataagggat agagaggaaa catccgtcag 420
gctaatttaa ctacatttta ttttaaaaat agagaaacat aacctctaga tgggacagca 480
gaggacagtt agtagaggcc acaaactgtt atgggctgct gtgttttgtt ctaaaatcaa 540
tatggttgga gcatgtatat cttaggtgat catttcacat cttaggaatg cctactcatt 600
ttattttatt ctagtgatgc tcaattcact atttaattta ttatattttc tcttctgtgg 660
cacttataca aaatatctct tcacctactt agttctacag ggttttaact ttggagcaac 720
atgaataaaa tcatcgagaa ggccaatatt gtttagcaac atgaatacaa tacagtttaa 780
agttgtacac atcctgctca actttattca tatacatttc ctttctgtgg ttttcttttg 840
cttcttagaa attctgttag tggttagtaa agaatttgaa agtactttct ccttgctgtt 900
tttttttttt tttaagacat tcctcccaga atactccagg gggcagtgtt ttataacaca 960
ttttccccac tgggtgattg aaggatggag gatttttgaa aatttgacag ctacatgaaa 1020
catgagaaaa cattttcctc acttctgaag tcggtttgca gctggtaact tgttcatcca 1080
gaaaacattc taaagcaatg agactttgtg agctgtgctt acagtttggg agaatcatga 1140
agattctttc tatattttgc atttacttcc cagtgcttca tagctgcatt ttgtttgtaa 1200
ctaagacaga agaatttcgt aatccttgaa attgaaaaaa aaaaaattgt gtttttaaag 1260
agtgaaaaca gttagaaaac aagtagaact gtaatcagaa cgctgcttca attgatatta 1320
aaaataacct caataataat gtaaaggttc ctttctcttg tgtcagttat attcttaggg 1380
atagcctaga aggaatatat ggttagaact aagtgtgact aatcatctga gccttgaaga 1440
gaaacttcag tgcctctaaa cagatcatct acaaaacaac aggtaaacat ttatgccagt 1500
taagtgggtc atgtttttgt ttcttgggtt tttcctaaat ttaagtgagg ttgggcttac 1560
cttgtagata aaattatgtt ttctttttgg taaatacttg aacgtggata acgtcaaatc 1620
agaatatttt gtgaggaggt gatgatttga aattaagcta gatttctagg gaggtgttgg 1680
ttccaatgaa ggatgggaag aaattaaaat agtcttcaaa cttcttcctt attatatttg 1740
gttgctttgg aaaagattgg tcctatcctc aatctaattt attcactatt aatattttaa 1800
aaaacattcc tgagatactt aaaaagaccc acttagcgat tatagttgct caatgaaaca 1860
agaatttatt tatgcataga tttttctctg tatcttacca aaatccactt tacttagata 1920
acactaaatt gttcttaaag actactcatt tcccaataat cctttatgat ttcaaaattt 1980
ctagtggctc agaagtgaat tttattttat ttgtctttca cttgaataaa tgagaaccca 2040
gaaattaata atgttgttta ttgcttactg tcaggactat ttcaaagact aagaagagtt 2100
tcttctaacc cctccctctc aaaggaatcc taaattatta gttgttagat aagttttgta 2160
tgctaagata ttcgggttta tagtttatgt atgtgtgtat atatataaat atatatgtat 2220
atataaatat tatgttcagt ttggagtctg gcacaactcc attatgtgga ttagagagta 2280
agatattatg ggtgataaag tactaaatga aacataatat ttatttataa aagtgtgtag 2340
attgttaaat cacaaaaaga gtgctatgac cattatgtat gaggaaacag gcctttgacc 2400
tcctggaaag cactgctcaa aagtcattag tgcccatttt tgaattcccc aaacagaaag 2460
cttcttagaa aacatgctga gattttattt acagggaatt ctttgacaca tttcaattgg 2520
tgtgtagtca agtatagcaa gtacttaata atgactgaat ttcatgttcc tacagtcata 2580
catattcatt agaagtttta tgttgttggt ctgatctgat tcttctttgt ttgtgggtgg 2640
aacggcactg agagaagtat agttttttaa acttgaacat gttcagtagt tacattgcct 2700
tagaaaaccc agacacatag cagtggaaat gaaagaaatg gcatcagaag tgacttaatt 2760

tagcaattgt gattcctctt gtaaaacaaa acaaaaaaac aatgccatat tttttggaga 2820
aaagttggca atataggggt ttcgttgtct gtttcacaag aagactcatt tgttcttttg 2880
ggggaaccag tgccttacag attttgtata tactgtaatt attcaggact agggaacaaa 2940
caattgtatt gtatttgtta cagattgtat atggctttgt tttaacattc ccctaaataa 3000
aatggcttca ttctcccctt ggaaaaaaaa aaaaaaaaaa aaaaaaaaaa aaaaaaaaaa 3060
aaaaaaaaaa aaaaaaaaaa aaaaaaaaaa aaaaaaaagg ccacatgtgc tcgagctgca 3120
g 3121

<210> 301
<211> 718
<212> DNA
<213> Homo sapiens

<220>
<221> misc_feature
<222> (1)...(718)
<223> n = a, t, c or g

<220>
<223> nbla-11483-f

<400> 301
   gnnnnngnng ngnttnnntt gaganccttg agcactgntg gcctactggt ggaccaacaa 60
   aggggccaga tgagtgaatt gctgaaagca tcttgctgta agacagggtc acaggacaca 120
   acattccaag tcatgtctct tcgaaacatg caataactta gaatcagcat ggtggtcata 180
   tgctcagaca gttcagggag caccatgact cttgggcgga gcacatttta aacttggtga 240
   atttaaattt cttttctttc ccctagcctt aaatggcctg aaatcctgaa gtgaataccg 300
   aagacatctt aagagcgtgc ttttagtgat aaatgcctat gtttggggtg ttttaaataa 360
   tgaggttgtt tgaaaagtag tgagaagtct ttccataaga atggtttgtg actggatgtt 420
   atatattttg gtttacaaaa ttgtctgaaa acctttctgg aataagtcag ggtctatgta 480
   aataaatctg cataaggaaa gtactgtgct ctttctaccc attcacccta gagactgcag 540
   gtttttaaat tttatggact tcacttcana gcagtttang gtcacagnac anttgaagac 600
   tgcancgggt ttaacacang tgncttcctg ggctcttntc ataactgttg caacccaang 660
   gaatggcttt ctaaggagac ntaaaatctg naaggctgnc ctgtgcttaa nccaatgg 718

<210> 302
<211> 703
<212> DNA
<213> Homo sapiens

<220>
<221> misc_feature
<222> (1)...(703)
<223> n = a, t, c or g

<220>
<223> nbla-11483-rl

<400> 302
   gngnnggggn nnnnttgagn cccttttgan tnctttttta aacaattgaa tcctaagggt 60
   atttttcttt tggcgctgaa acgatattaa aatttactgc aactttattt aaaaaaatag 120
   aaaactaatg tttctctggg tcttccattt ttgaataaaa ctgactctta ccacccaaca 180
   cgtggatttg gaaggatagc agtacaccac cttcttagct cacttggctg gaacatgagc 240
   tgatgaagtc ctagtctcaa gtttggcctt caagaaaact agataggctt gccctatgcc 300
   ggagccaatc tagacctgcc atcagttctg tttcttcttt gtgtgtgtgt gtgtgtgtgt 360
   gtgtgtgaga gagagagaga gagagagaga gagacagaca gacagacagg gtctcactct 420
   gtttcccagg ctggagtgca gtggcatgat catggctcac tgtacctnaa ccacccaggc 480
   ccaaagtaat ccttccacct caccttcaga gtagctggaa ctcaagggca tgctccacac 540
   ccagctaatc ttggattttt tatagatcca ggatcttgca tggtgctaag ctggctnaaa 600
   tgcccaactg gccttgactc cnggctcaag aatcatccnc ttggcttcac annggtggaa 660
   tcaagggngg caacccacct ggccatnant ntgtttttga cca 703

<210> 303
<211> 2650
<212> DNA
<213> Homo sapiens

<220>
<221> misc_feature
<222> (1)...(2650)
<223> n = a, t, c or g

<220>
<223> nbla-11485

<400> 303
   ttctaggcct gtacggaagt gttacttctg ctctaaaagc tgcggaattc ctcgagcact 60
   gttggcctac tggcctgctt ggtggtggaa ggttatatag taaacttcac tgtcattaag 120
   aaagcgcttg tccaaaatgt ttaaagtact agaaaaggga taattcagca ctttttctgt 180
   tacaacacta aagtcttaat ttatcttcag agtcttaggt actgtaagtt tttggatgat 240
   tatttaaaaa tataaaacat actacataat atggggtcag atcacattta tataaataat 300
   tcttatttta aaaaagacaa taacattttg tacctttcta gcaattccaa gtgattgcct 360
   cattttctct tgttaagcat ttcaagctta agaatttgga atttgagaga aggtgagggg 420
   gaaaattccc taaaataaaa atttgtttca aaccatttta gaaaaataaa tcactaggaa 480
   aataggtagg gattctctcc ccctttaagt cttcagggaa tgagcagtat ttagaatctt 540
   tggctgaaat tcaagccttt ttactttgta aatttttaat attaattcac tgacatgctt 600
   tttacaccag aggactgaaa atggatctta atgtttttaa gttgtggaag ggtatatttc 660
   taaaatgagg ggggatgatt aactaggtta gtattaccag cattagtaag tttaaggaat 720
   tttagtatgt aatagcaaag tgtttgatta aaagtctaat cctatattac tagtcaaaat 780
   tagttacatt ctgatctagg aaagataata gtcattggat attgatatgc tgtgctgtca 840
   gaaaaccaga ctaagataga aacctttatc tgactcccac atgttgcttg ttgtgatggg 900
   atgtatactt tacagcagct tattgctgct ttgaaaaaaa atgtataaac tatacatttg 960
   gagtgtttgc atataattct ttataacctc cacttaaagc tgtcagacat tggtatttta 1020
   tcagtccaca ttgttgaata aaactaatgt tcttaggaat ccagcttgta cacactgttt 1080
   aaaaaccctc agggacagtt tacacactct tctcactcaa ttcaggtact ttgatgctat 1140
   tcttaaacct aacagtgact tgtatttttc tgttttgctt ttatttcaac gtgctggtag 1200
   cacatctgat gaatgtcaac ttaaaaaact cagttcaggt atccaggtat aactcagcca 1260
   aacagatttt aaagctgcat ataactctcc agcacatggt gcctcacact cttatagtgg 1320
   cattctatat attcagttat tactactgag cagataatat gggggttcct gttaacagtg 1380
   tattttttaa aaaaagcgca taaatgtata gccagcacac tacacacaca cacacaaaca 1440
   tgcacagtta aactatatat ttttaaatgc cactaatagc cagcacaact aaaacaaaag 1500
   acattcctaa ctgctctgta agctgttaac agatgcagtt ccttcttgat gtggctcttg 1560
   cttcttcacg ataactacta ctaaattcaa gcactggtcc ttgggtgtct gacctctaca 1620
   ttctagttta tgcaatgtct ttagagaatt ttgtgcactg gccactgtga tggaaccatt 1680
   gggccaggag tgctttgagt ttatcagtag tgattctgcc aaagttggtg ttgtaacatg 1740
   agtatgtaaa atgtcaaaaa aattagcaga ggtctaggtc tgcatatcag cagacagttt 1800
   tgtccgtgta ttttgtagcc ttgaagttct cagtgacaag ttttttctga tgcgaagttc 1860
   taattccagt gttttagtcc tttgcatctt taatgttaag acttgcctct ttaaaattgc 1920
   ttttgttttc tgcagtacta tctgtggtta acaaaataga ttatttctct gctttaatat 1980
   ttgatatctt acatctaaaa taaattctct ccacatataa aacccatagc ctttggagat 2040
   atggaaaatg gtatctttca gatttctaga agttcaagtg tcatacaaca aaacaggaac 2100
   cccctttact cttatggacc tcatttcaat atactgttta cagtttgatg gaattgtata 2160
   atttaatatt tctcttgtac tgtagtttat atttatttac agattttttt gtactgtgtg 2220
   atttgaactt tttgttcctt gctatgatca atgtttatgt agtagagcac ttatgatcac 2280
   aaattaagtt ttttggtttg attgcactac attaaatttt ttaatgcagt tctgattttt 2340
   gactggacta aaactgtgtc ttaatgtatg tgatgagtac ttaaaatttt aatccatgtg 2400
   gtcccccccc tttttttttt ttttgcattg tatgtcaaaa gcgcttgttc tttcgtgcat 2460
   gtgtaagatt taatggttcc attgtattat ttgaccatga cattttggag aaacattccc 2520
   agctgtaatg ttgtgtatgg tagttctcac tggatgctag agttttcaaa accactattc 2580
   ttctaataaa ttttgttgtg aaaaactgtt aaaaaaaaaa aaaaaaaggc cacatgtgct 2640
   cgagctgcag 2650

<210> 304
<211> 743
<212> DNA
<213> Homo sapiens

<220>
<221> misc_feature
<222> (1)...743)
<223> n = a, t, c or g

<220>
<223> nbla-11519-f

<400> 304
ggnnnngngt ngnnncnnnt tggnttcctc ggcactgttg gcctactgga atcgctgcag 60
taacttcagg gctgtttgct ctgctgctga cctcactgta cgcaaattag cccttcttct 120
gaggctcaac tatgctatgt ccattaccct agttactgct cacaatctac ataatgtgac 180
agatcatgaa acatgaacta gtaaaatgtg tatttataaa gtaggcaata ccgttattgt 240
tttcagagga aatgacagta tgtctagaga gctcatgaaa aatagaagag atagaaaatg 300
ggtattccaa agcctagacc ttttaaaagc aattatcaat cataagctat aatgaaaaga 360
atgtgattca caacagtggt tcttctgtga gccagatggg tgtggattcc attcagaagt 420
agtgcatagt ccccaagctt gcacctcact tgagccctgn atccccaaat cattctgttt 480
ttgtccagag tttcacaagg ttgatttgga aaatgttccc accagctctg gctatgggaa 540
tcacagtggg aaataaacgc catagcatct tccccatcat ccccgtctcc tcctgagaca 600
aaacacattt tcccctaaat gctaactggg ctgctgcctt cagcatagac aatcttttac 660
atttttacca caaagtagtt ttaactgcct ggccacatac caattgaata gtatgatgtc 720
tgggtaaggg cagaatantg gnt 743

<210> 305
<211> 755
<212> DNA
<213> Homo sapiens

<220>
<221> misc_feature
<222> (1)...(755)
<223> n = a, t, c or g

<220>
<223> nbla-11519-rl

<400> 305
gnngnngggn nnnnttgtgt ggcctttttt ttttnttttt ttgaaagtaa caatatcatt 60
ttattgcagg gtggcatagg tagttgtact tattaaatac atgacaaata ggtaccagca 120
gatgagttag atgtagggat acagatggaa ataaacttcc atggtcccca ttcatcaaat 180
ttattgtcct ctgagaaaga catgagttca tctgatcatt acagaggaga tgagtgtttt 240
gaaggagaag cagagggttt tgtggatgtg attagcaggt ggacctaacc agcacagtgg 300
gtcagggagg gatttctca ggaaataaca ttcaggctgg agtctgaatc ttgagctctg 360
aagcaggtgt caggacattc agagcaaagt ggattatatt tgcaaaactt cacatgctaa 420
aagatgagag aagtccagtc agtagcaagt tgttctttgg aggtcctcag aatatgtcac 480
agcttcttga ttcctcctta catgcctgta agagatattc actatccaat caattgttta 540
tcacagaccc tactatgaat gagattccat agaggataca aaggatgtgt aatacacttc 600
aaagtaattt aaggctcang agcaagctac gaatgaatta agaaaataag gcaaagacaa 660
gtaaatattc aatgncatta aagagcatgc cggaatataa gaaatggtgg tcaaactagg 720
tccactcaat gggtcaagtt gangggcata tgtca 755

<210> 306
<211> 731
<212> DNA
<213> Homo sapiens

<220>
<221> misc_feature
<222> (1)...(731)
<223> n = a, t, c or g

<220>
<223> nbla-11530-f

<400> 306
gnngnnnngn nnnnnnnnnt tnnnnttcct cgngcactgt tggcctactg gcttttcttg 60
aaacatcttg attgcttttc ctcggcagct ttcaaaaaac caaataataa tagttatccg 120
tcttctactt catggaagat tgttttggtg ccctgaccct ctgaagtgcc cagttcctgc 180
catctgaaac ctcggcctga tctgatctca tgttggaatc tgcctgtctt tcacacaggg 240
ctggtcttgg tcctttacat gccagttttg cttgtgaatt cttgcttttt tcctctcatc 300
agccttaagt ttaggcgttt gttgttctcc agtgatgtag acagttccct tcacaagtca 360
cagttcttcc cataaatgag gcccgctgac ctctgcggga ctttaaaaat ctattcagat 420
atttccgagt aagtggcttg tttaaattct tcctgtgtct ttctttattc cttaattggt 480
tggtggaaag aagagatgct tgggaacctt gggttcttag gtttggattc tttaataata 540
tctaaaaagc taaattttaa ataccagctt tacataaatg attggtgact ctggtctgnt 600
tctgacacct ttccagaaaa aagcaattgg tcaagtacac caaagaggaa gaagaactgt 660
ggangccacc ctntacaaag ctttatagaa cttctggatc taacttacaa acaagctttc 720

agaagagact n                                                    731

<210> 307
<211> 766
<212> DNA
<213> Homo sapiens

<220>
<221> misc_feature
<222> (1)...(766)
<223> n = a, t, c or g

<220>
<223> nbla-11567-f

<400> 307
gnnnnnnngn gggnnnnnnn nnnttngnat tcctcgngca ctgttggcct actggatcca 60
gttcaggccg atcagggctg gtggaaacaa ctaccaagtg tctctctgtc tgctttgctt 120
tgggagaaga aagtggaggg agccccagca tgttgtagag gaactggcct gcggaaacga 180
gaggaggagg gtgcaggggg cagcggaaca gactgaacct cagagcatca gttggaatta 240
cnatctgagg ctgtcagaga tgactctggt tctgtccatg aatagattct gcgagcccat 300
tgtctcggaa ggagctgctg aaattgctgg gtaccaaaca ctatgggagg ctgacagcta 360
cggaggccca agccccccag ggccagcaca agctcctttg cagggagacc ggggagctgg 420
tcccccactg gcaggatcac attacagggg aatttcaaat cctataacaa catccaagat 480
cacatacttt aagaggaagt atgtggaaga agaggacttt cacccaccac tcagcagctg 540
tagccataaa accatctcaa tttttgagga acgagcccac atnctttata tgtccttaga 600
aaagctaaag gttatcgatg atcctgaagt gtaccttcga agatctgncc ntataaacaa 660
tttgatgaaa aggatccatg gagaaattat catgcnngaa tactggngct ttccttgctg 720
gtctttaatg gnaccttttg ccaaantggt tatgntcaag actgnc                766

<210> 308
<211> 772
<212> DNA
<213> Homo sapiens

<220>
<221> misc_feature
<222> (1)...(772)
<223> n = a, t, c or g

<220>
<223> nbla-11568-f

<400> 308
gnnnnnnngt ggngnncnnn nnttggnatt cctcgagcac tgttggccta ctggagctct 60
gtcttcatct tcaaaagatt tagaaacaat agttactctt aagaagacta tcagaatctc 120
accacacagg gagagtgacc attctagtct aaataacaaa tatttgaatg gatgtggaga 180
aatatcagtt tcagaaatga atgaaaagtt gacaactctg tgttatagga agtataatga 240
tgtctctgat ctttgcaaat tagaaaataa acaatattgt aggtggtcca agaaccaaga 300
tgacagtttt acaatttgca ggaaacttac atatccttta gaaactcttt gcaatagtcc 360
aaatagaagt acaaatacat tgaaagaaat gccttgggga catatcaata acaacgtaac 420
acagagctat tctattggtt atgaaggtag ctatgatgcc tctgctgatc tctttgatga 480
tattgctaaa gaaatggaca ttgcaactga gattaccaaa aaatcacagg atattttggt 540
aaaatgggga acatctttgg cagaaagtca cccttcagag tctgattttt cactgagatc 600
actttctgaa gacttcattc agccttnaca aaaattatcc ttgcaaagcc tatctgactc 660
tangcattca agacatgctc ttcaacacct nattttcaat cagattcang aatatnattt 720
tgnaaatagt caagactttg gtccatgttc acagncaact tccaatttca ng 772

<210> 309
<211> 771
<212> DNA
<213> Homo sapiens

<220>
<221> misc_feature
<222> (1)...(771)
<223> n = a, t, c or g

<220>
<223> nbla-11568-r1

<400> 309
gagnggngtt nnnttgtgtg gccttttttt tttcnttttg gtttgcaaag tctttatttc 60
tggatgtata tgtgatttta acaaacatta atactttcaa aaacaatctt aacaatatta 120
ttaaagtaca ataaacagta taagataaag tgctcaacta cctaatcata gtattctaga 180
taaaaccctg gggaaaacaa tggctttata ttttaaaagg cacagcagct cccagcagag 240
tcctgttttc aatttgcttc tctccaagtg ttcaaaatgt aaagaatgct ttccgtaccc 300
ctgaaggcaa acatttccaa acagatttaa aagttcagga attgggttca ggtgactttt 360
tatgaaaaca attcaggtga ccaagcactt cgagtttcac acactgaagg tgggcctttt 420
tctgaaaatg gaaggcaaca tttgacttct gaacaagaat caggtccaag aatagggtgc 480
agctgaggtg ctgctaagac ttgaatgtct ggacatttgg aaatccagcc tgaactttta 540

caggtatatt tatgagtttt ctttttcgta acaactgntg caagcatatt ctttaatttc 600
ttcttaatta aacctttatt gggtccttgg ctaatatggt tcaanttctt tcttggnaac 660
tcttggtgga tcaagggaaa tgattggcag caaggcattt ncctaganct atgncttggn 720
atccagcntt tntgaaggga atatttttg ggggtagga nggancentt n 771

<210> 310
<211> 753
<212> DNA
<213> Homo sapiens

<220>
<221> misc_feature
<222> (1)...(753)
<223> n = a, t, c or g

<220>
<223> nbla-11600-f

<400> 310
   gnngnggnng nnnnnnnttt gaannccctt ntttgagatc cctcgagcac tgttggccta 60
   ctggaattag ctttagatac agcagtagcc ctttccttat gtccttgtga tgcctgagta 120
   aagaacatag tttttacttg ctaagctatg agattttgcc gcctgtagta gtttttaaaa 180
   tattgagtgg cctatctcca gatctcccaa acagattcat gaacaccatc ttgcttttga 240
   acatgtcgat tttaaagtac gaggccaata tgatattgcc ccaagctcat cattgttctt 300
   gatgagaaat gtagtccagg attcttagaa actttcttaa tgtgatcttt catttaatgt 360
   tctgtgtcat tttaaggact agaatatgct attttcttaa gtgttttttt ctcccttgac 420
   agttttaaac atattgtctt ttgcttccaa gcccttccat tgctgaacag tttttgtagg 480
   acagtcttca gctttatcat taagctgaat ccctatcaac atctagctgt ccaagtgagt 540
   tatagatggc cagtggctct gctttttact agcctgcacc ctcttagcta accttctttt 600
   tttagggttt gnatttttag ctataactta tttaacacta tcaggtgtta gatgccatgc 660
   caactggttt atgtacttga gaagcangtg ttaacttcct cagttacaaa tgagaaaacc 720
   cagcttaang ggattgactc atttgncaat agg 753

<210> 311
<211> 751
<212> DNA
<213> Homo sapiens

<220>
<221> misc_feature

<222> (1)...(751)
<223> n = a, t, c or g

<220>
<223> nbla-11600-rl

<400> 311
gngnnggngg ttnnattgag tgccctttgt taatnccctt tttttttttg agacagagtc 60
tcgcactgtc acccaggctg gagtgcaatg gcaggatctc agctcgctgc aacctccacc 120
tcccaggttc acacgattct cctgcctcag cctcctgagt agctgggatt acaggcacat 180
accactacac ccggctagtt ttttgtattt ttagtagaga tggggattca ctatgttgac 240
cagactcgtc ttgaactcct gacctcgtga tccaccccac tcggattccc agagtgctgg 300
gattacaggc atgagccacc gcgcccagcc agcattgtat actcttccaa aggtacttct 360
gaagtttgaa ccacatgaat aaattttcta acataaaaag taaatttatt gttcaaaagt 420
tcattatcaa gtggcccaaa gctataaaac ttatggntaa catatttagc ctcaggaaac 480
ttagctttag gaaagtcaat ctggagagac atgaatgaaa aatgattgcc tgctgaaaat 540
taagcantta taatggttaa cgataataac agaaaaactt ctatggcatt tactatgtgc 600
catttgaaac tttaccaaaa ctaactcatt taatccactc aagaatccgc ttaagtaggt 660
actggtggta ttctttncat tttncanaag angagaaact gaggggtaag acattcagna 720
ntttggncaa aggtacataa taagnnangg g 751

<210> 312
<211> 736
<212> DNA
<213> Homo sapiens

<220>
<221> misc_feature
<222> (1)...(736)
<223> n = a, t, c or g

<220>
<223> nbla-11618-f

<400> 312
   gngnnnnnnt ttnnnncttt gananccntt gggcactgtt ggcctactgg tctcatctat 60
   gatgtctttt gggtattttt ctcagcctac atcttcaata gcaacgtcat ggtgaaggtg 120
   gccactcagc cggctgacaa tccccttgac gttctatccc ggaagctcca cctgggcccc 180
   aatgttgggc gtgatgttcc tcgcctgtct ctgcctggaa aactggtctt cccaagctcc 240
   actggcagcc acttctccat gttgggcatc ggagacatcg ttatgcctgg tctcctacta 300
   tgctttgtcc ttcgctatga caactacaaa aagcaagcca gtggggactc ctgtggggcc 360
   cctggacctg ccaacatctc cgggcgcatg cagaaggtct cctactttca ctgcaccctc 420
   atcggatact ttgtaggcct gctcactgct actgtggcgt ctcgcattca ccgggccgcc 480
   agcccgcctt ctctatttgg tgccatttac tttattgcca ctcctcacga tggcctattt 540
   aaagggcgac ctccggcgga tgtggtctga gcctttccac tccaaagtcc agcaagcttc 600
   cgattcctgg aagtatgatg gatcacgtgg aaagtgacca gatggccctc atggnccttt 660
   tctctnaact catgggttgg ttcctcttaa agctggnctg gnacttaaaa atgtacctgn 720
   ggttaaagga actgcc 736

<210> 313
<211> 733
<212> DNA
<213> Homo sapiens

<220>
<221> misc_feature
<222> (1)...(733)
<223> n = a, t, c or g

<220>
<223> nbla-11618-rl

<400> 313
   gnnnnnnttt gagggccttn nnnntttttt tttttttttt ttgctttttt cctttttttt 60
   ttttttctta aaggagtgag ggcatggaaa atatacagca caccaatgaa acaaaatgtc 120
   aaaaaaaaaa tacaaaaaaa tagaaaatag aaaaatgtat ttacaagtag tacgttacta 180
   tgatgagaaa gcacaaagac acctgctgct ataatacatg cattggctcg agaagaaact 240
   acggaacacc ctgcgaggga naagcctaga aaagaaagaa agggccaaaa ggtttgaact 300
   cttcatcccc aatttgctac actgatcaaa accaagtaag ggctcctgaa gtccatgagt 360
   ctatcatcaa tcagcacaaa tgctatacta gtttgtaact gcggggtcag ttgtgaaggg 420
   gaaggacagc agcttatcca tatacaagga agccacagta aactgctcga catgctcaaa 480
   acgacagcaa gcccctgtca gctacacagc gtgatcttag caggatttca tttttgntta 540
   ttcatatata tctatgtgtg ngtttatata tatgtccgta tgtataaaaa ccgtgccctt 600
   gttcactggc aacacaacat ntgggtggac atgaactcat tcaacaaatt cttatgtgga 660
   tgttgtaaga agcactcaat gnctggggat ttaataagca ctttacaccn cagaaaatnt 720
   ttaataaatc cca 733

<210> 314
<211> 2036
<212> DNA
<213> Homo sapiens

<220>
<221> misc_feature
<222> (1)...(2036)
<223> n = a, t, c or g

<220>
<223> nbla-11652

<400> 314
taggcctgta cggaagtgtt acttctgctc taaaagctgc ggaattcctc gagcactgtt 60
ggcctactgg ctagtttgca tttgcggaag atcccatgca gaaacagatg tcacactgca 120
gctgcaagga cagtgaggag ggagatggcc atggtgcctt ggtccccaga gtgacggcat 180
gcggaggcgt caatgcatct acctccagca caccaggcat gatgtcaggt gcagcaggag 240
gtacctggcc ctttgctaca cagaccacat ggtcttgctg ggacaagacc ctagggaaca 300
gctcattttg gtacagtgtg gttggttcct ggagagggag agggaatagc ccacgggcta 360
agcagcccac tgcaggtacc taatgcaacc aggaaggtca gggaaggaga tggccagcca 420
cgcggtggag tttgaacatc atgtagcagt tagccaggtg aagaggagat gctggggaga 480
cagggagagg ccactcctgg ctgagggacc tgtacctgca aagactctca ggggaggagg 540
acggctttct gtcactgttt ctgtgtgtga gggaaatcag agggtaggcc cggctgtccc 600
ctgcctttcc tgtggggcct gactgcatgt accccctctc cccaaaccct ccaggagttc 660
tgagtctcta cctggatctt gattccactg gcatgaaatc tgtgaatctc acctcgagaa 720
tcctccctct gctgccgcag gatggaccca acagttccct gcagtggcaa gggagctgtt 780
cacacactga gcgagcagtc acagctgccc tgagctccac attctcctcc tcgggggact 840
cccctggaat tacctttgtc attttacttt ctcaacccct gtgtttcctc tgatatctta 900
cttatctctg gtctttttgt ccagacagct cttaagcctt gggaggaacc ttttcctctg 960
atcccttttt aggccattat caagttcagg gtgggagggt agagtcactg tctttccagg 1020
ccttgctgca tcccttagag accttcggag ggggcacttt gtgaccagtg gggatggggg 1080
aaggatggct tggtgtgtct gtcctcaccc actaccctct gcccacttct gcctgccctt 1140
cctctgtgtg gcatatacag tactgcaccc ctgagcagga cattccccag tgagagcccc 1200
aggcccgaca cttccaggct gtgtggcctt gatcaagtta gtttcccttt cgtgattcag 1260
attcctctca ggctgcttgt gcaaactcaa cataagaaag tctgcgaagc tcttaaccca 1320
gggcctgggc catgctaggg ggatcagtaa gcagtttctg ccacctctgt tactgccgct 1380
ttcacctttc cttgtggaaa agagaagcag ccagaaaagc tgggtttag ccccagctct 1440
gcctcttaac tagctaagta aatgtgggca gaatgcttga cttctctgaa tctcattttt 1500
tttttttttt tttgtcaaac aagatgaatg aacaggatcg gtcttttttg atggggactt 1560
gaatggtgtg tgggggggaca gttctttgtg tgggacggtc cacactaaat gccagttatc 1620
cccacccatt gttacaaccc aaatgtcacc ccccaccaag tatttctcca tgtccctatg 1680
ggttggtggt ggaactgtcc ctgactgagg accactgaac tagcttaccc taaacttcac 1740
tcctgctccg gtgtcctgtg tgggtggaaa agtgaggacc cagtggagca ggtagacaaa 1800
gatcagttca acagggccct ctgctccttt gatattggga cagcttcact tcctctgaat 1860
tagaagcttc ttgacctcca tatattgagg aaggaagggg tttctaacaa atgaagaaac 1920

aggctgggca cagtggctca tgcctgtaat tcccctttgg gaagcccagt tccatctcta 1980
aaaaaaaaaa aaaaaaaggc cacatgtgct cgagctgcag gtcgcggccg ctagac 2036

<210> 315
<211> 728
<212> DNA
<213> Homo sapiens

<220>
<221> misc_feature
<222> (1)...(728)
<223> n = a, t, c or g

<220>
<223> nbla-11674-f

<400> 315
nnnnnngnnn nnttgnnnnc cnttgggnnc cctcgagcct gttggcctac tggaaaaagg 60
aagcaaaaga agtttttcca aagatgttat gaagggctct aatccaaaag taccacagca 120
aaacaacttc agtgactgtg gtgtatatgt attgcagtat gtagagagct tttttgagaa 180
tccaattctc agttttgaac tacctatgaa tttggcaaac tggtttcctc caccaagaat 240
gagaacaaaa agagaagaaa tccgaaacat aattctgaag ctacaggaag atcagagcaa 300
agagaaaaga aagcataagg acacttactc aacagaagca cctttaggcg aaggaacaga 360
acaatatgtc aatagtatct cagattgacc atttctgtta cttgtcattt ctactttcag 420
aaactaaatg actttcaaat ttgggtatag acaataaaga actgaagtgc tcactactca 480
gtgatttgga aattttgatg cttgtataaa tgtcagataa ttaatttcca aaggcgtatg 540
tattaagtaa aagtctgtaa atatgttaat gaggccaatt tttccagcat ttataattat 600
ttttttcact tggtaggaag cttttggtat gnatttctgg taatagtacc taaaattgca 660
acttctaacc caaatnaaaa ggaaaatatt tataggagga aatgattaat tggtatctta 720
gggaactg 728

<210> 316
<211> 729
<212> DNA
<213> Homo sapiens

<220>
<221> misc_feature
<222> (1)...(729)
<223> n = a, t, c or g

<220>
<223> nbla-11674-rl

<400> 316
   gnnngnnnnn nnnttgtgtg ncctttgttt ttcntttttt ttttttttat cccagtttta 60
   ttatagattg aaaagcattt tgccatgttc agtaaataat aaaattaaac ttgnttttca 120
   tatgagctaa ttaagatatc tgagctaaca gctctaagtt attactataa agagttaatc 180
   agaaatatag ttcaaagtaa caaactacag ttgatatcac taatggtgac ctttcttaca 240
   ttttaatatt tctcttactg agactctact gcgtctcaat agatattttt tggttgttgt 300
   taatactccc attcaccctg aacccaccca tgggacttta ttaaattcaa gaaaataccc 360
   taagatattg actgcaatgt cagagatggc caaattaaaa ataaaataat tgctgatgat 420
   taggctttct ttttattctc aactattttt gatatatgtg tatttaaaaa ttcaatattt 480
   aacaaaaaat gactaaaata tgtttcaagt ctcaaagttc atttgaaaga agtatacacc 540
   atttcatttg aaaaataatg aagtttctgc agatatatgc aagatcccta tatgtgctat 600
   aaatctgnca tggaaaccat caaattttag ttattttaaa ggggaattca aagatttttg 660
   ctggattatg caactggtgg aatacgtact ttacatgtaa aaaataatta ccctggagna 720
   actcaagcc 729

<210> 317
<211> 776
<212> DNA
<213> Homo sapiens

<220>
<221> misc_feature
<222> (1)...(776)
<223> n = a, t, c or g

<220>
<223> nbla-11679-f

<400> 317
gnagnttggn nccccttttg ngantcctcg agcactgttg gcctactgga agttagttat 60
tttctcctct ttctttcctt tctttcctcc ctttttcccg tctgacccca aacgttattg 120
tccaaacatg actggacagc agcttttgtt tcttgaccct gtaatatgac agtctgctaa 180
tattgacaga aggtgcagtt tttgggttat agtcgtgatt ttcactaatc aatcatatta 240
gcaggaaaaa aaatgacttg tttctgttgt acttgagtct taagaaaaag tgcccatagt 300
ttagtgacaa tttccaaagg ctttagtacc acctgtattt caaaatgggg gacccaaact 360
cccggaagaa acaagctctg aacagactac gtgctcagct tagaaagaaa aaagaatctc 420
tagctgacca gtttgacttc aagatgtata ttgcctttgt attcaaggag aagaagaaaa 480

agtcagcact ttttgaaagt gtctgaggtt ataccagtca tgacaaataa tttatgaaga 540
aaatatnctg aaaggtgtgc gagattccag ctattccctt ggaaagttcc ctannagctt 600
ttaccagaan gatgtgggtc cagntncatg ctnctngata tcaagtccta tgnnaanggg 660
anngaaattn gctgtactca ggnagatgga tttcantcnt ttggnccttc ngnantgann 720
tttgaaaaaa atncgntggn nntcctggtt tctaaggggn taanaattct natgng 776

<210> 318
<211> 753
<212> DNA
<213> Homo sapiens

<220>
<221> misc_feature
<222> (1)...(753)
<223> n = a, t, c or g

<220>
<223> nbla-11679-rl

<400> 318
gnngnngntt ggtggccttt tttttttttt tttccctggt tgtattttct tttttaaaac 60
cagttctggt cattagaatg tatctgacag tgctctggaa caactgtgat tatagcaaag 120
ttattgtttt taaaaatgtt atttatacct gttacattca cttctcaccc tctaaatact 180
gatgacagat gtcaaagagg caaaaaccag cacaaatggg aagaccataa aaatgagtat 240
caccttgtgc tttcagatac atttaagcat ttcagtgtac aatagtcctt tcatttcaca 300
tttttagtaa gatactgatg cagtgcagca aatatgcaaa gcatcttctt tcacacagtc 360
cgtgcacgac atctaatttt tgtttaagtt ctgagataaa aatgatttaa aaaaatccag 420
gatgaacaag tttcaaaatg catagtgttc tgtgcatgag tccattttct ttaaactctg 480
aaagaataaa gtggtaagaa aacaagaaaa aaaactgctg ctgcattctc tgatcttctc 540
cattttgctg gcagtactct actctggcat ataaggacng aggtggatcc tctatggngc 600
caactggcca ntgggtgaac tggtcctgtg gcangtagaa gcaanatgct gataacttgg 660
aagattggaa ctttggtttt tggagtctgc aagtgctgct gtcantgaan aagaaacact 720
gactgggtag gattggtggn cacgattcca ggc 753

<210> 319
<211> 799
<212> DNA
<213> Homo sapiens

<220>

<221> misc_feature
<222> (1)...(799)
<223> n = a, t, c or g

<220>
<223> nbla-11753-r1

<400> 319
gntnagntgt gccttttttt tttttttaa gtgtaaaagt tatattttat tgccatgcta 60
caaaatgtat gaagttggca ctgataggga gaaatagaga acaaagggtg ggaagggata 120
gagggaaaat tatgttgtta catatacaac aaggttttat tttaattaac agtggttacg 180
ttttgccaat attaaaaatg caaaccaaaa tttaaaatgc tgatctgaaa cagcattaag 240
atacaatgta tgcatagtac agtatcactt atgtcttttt attagagaaa tatggaatgt 300
ttataaaaga aattaaccat gggggtaaaa ttcatatttc atatacaatt tggcaatggt 360
agtcccactg ttggacaatt ttttataaaa gaaaaaatta aaaatctaat aagctacctt 420
tatacaaagt tgctatattt atgcctttac gtaggaaaaa aacatttata atgcaaatta 480
ggacatacaa tagtcttaca atattataca atgtaatgaa aataaaacat aacacaaagt 540
ttgcctttat aaaatgnata ttttgcatta ctaatgcaaa tgtggcacac tggtgactac 600
tgnactattt atccagtctg tttccatgct ataactgtcc aattgccata gaactgcaat 660
tataatgatg cttcaaaaga actgngttat accaaaaatg aggaagtagg gatggggaac 720
agggatgtaa agaacaggaa agatagatag ggtgcctggt ttgggcanag atactggggt 780
ccgtataaaa aatggtnat 799

<210> 320
<211> 759
<212> DNA
<213> Homo sapiens

<220>
<221> misc_feature
<222> (1)...(759)
<223> n = a, t, c or g

<220>
<223> nbla-11763-f

<400> 320
ttggnttcct tggcctgttg gcctactggt gtgaatattg gaaattaatg ggtttcattt 60
tcatttattt cccttgcctc ttcttgagga ggattcgaca ggcgatgaaa tgggtaaaaa 120
ggcagtcaga ctaacaggaa gaaggaaaga taggctagct gtcaactgat gagcctgcct 180
gaataatctc aaatgggctt ttgaaaagac cttaacaaac actgttgcac tcttgtcaaa 240

tatctttatc tacaccctac ttataagtac caaaaagaca gcatttgatg ccaagtttga 300
agcagttagg tcctatgcag gctgtgtatt aacatacagt gaattgcact ttaaaattca 360
gaaagctttc atttggtcac atcaaaaata aacactacaa aatatataaa aagttaaatg 420
ataggactct ttttttggta ttaagagtaa aatagaagaa gatctaagaa atacaaatac 480
ttacattatt tctccttatg acattaactt tgggactaga gccacaccac ccccttcctt 540
cttcacttac tgattggatt gcatataatt ttcatgccca cataaatcct gattcatata 600
tttgnaactg natatgacaa attgagangc angaataaga aaanggaaga ttatngangg 660
aagatgttaa gaaagancag ttcttctaan tactctggca gatcgggtgg ctgaaagagg 720
tnattagcat ttgaaatgga tgggctgatg nggggctca 759

<210> 321
<211> 759
<212> DNA
<213> Homo sapiens

<220>
<221> misc_feature
<222> (1)...(759)
<223> n = a, t, c or g

<220>
<223> nbla-11763-r

<400> 321
ttggtggcct tttttttttt tttttaaaa tttttttcc cattcttgag ttttacaatg 60
tctcctatcc aaacttcaaa tttcattaga gccggctaca tttgttttag tgccagatac 120
aggtatcaag tgatgacaga cacttacgtg aggtatacaa tcaattaaag actggaaatt 180
ggggtattat catgtaagac atttgagagg ccaaaagtca tataatttta agatacttca 240
tgatgttaca gtaataattc atctatataa caattatttt gacataagtg gcatatcaga 300
atttaactta tttacttaga tcttaaggaa tattagatgt attctcacat gaacactcaa 360
atcatgctat tattatcagt cactgggaga acagccaaag taatcaggca gtctaaggca 420
catatagacc caataattta gaaattttca ccattatgag gtccctgttt ttaataaata 480
tgacaataac aggcgcttga aatgggggca aggtgaataa gtatccagtc aattgnatac 540
ttttataaag tagtgnattt gagacataaa ctgagatcaa aangaatttn cttactttna 600
aaaaaggngc ttctatctta gnagattctt ttaaatactt ancntatatt naaaagtagt 660
cnttacatac ttaagaaatc accgtaatct aaaaaantaa angcagtttc ttggnaatga 720
acctgngatc caacaggcca caatgnggga attactcag 759

<210> 322
<211> 778

<212> DNA
<213> Homo sapiens

<220>
<221> misc_feature
<222> (1)...(778)
<223> n = a, t, c or g

<220>
<223> nbla-11767-f

<400> 322
gttnnnnttn nnnnnnnttt gaattccntt agcactgttg gcctactggg agagaccaca 60
cgtagagctc ggtcaccaag gaatgcccaa catcccttta cctctcacaa tgagcagcca 120
cgctcaccgg gaagcagtgt ggggacttca gtggggtgtc ccttagtgga gaacaccttt 180
catcattttt atagagacaa ttcccttgag aatgtaggct tttcatctta ggaacaatta 240
ggaagaatct ccagctggga gtgttcttat gtatacagaa acaatgggtc gcattggttc 300
atacttaggt gatattaggt ttggctatat tgatgtttgg tgccacttac cttctagatt 360
ttcccaaaag ttgggtcatt gctatgggaa attggggcct gcagatgggg gaggaatgat 420
cattattggt gcagggaagg ggttcaggca gagctggagt ctgaggctca gtgtgagaaa 480
gcactggcag ttttctagta gagtcaggtc ctgggctcct ctcctnaccc actgtgtccg 540
aactgaggcc agctgtgatc ggcaaattgc ttatctctag gnaggttaaa gaaaacgtaa 600
gaatttgggg ggtggtggga gaataactgt agaaagacct aaactcttnt ttccccanca 660
aattatcttg gataatggtt aaaaacccca aggccnnact tcccttttac cgngaatcan 720
aaggaggatg gcantgggcc canggcnnaa tggcccactt tgaacncatt ttaaaang 778

<210> 323
<211> 800
<212> DNA
<213> Homo sapiens

<220>
<221> misc_feature
<222> (1)...(800)
<223> n = a, t, c or g

<220>
<223> nbla-11767-r1

<400> 323
gnaggngann nttggtggcc tttgaaaccn tttgnttttt tnttttttttt tttttttttt 60

tttttttttt tttttttttn ntnnnnnnnn tnnnntnnnn nnnnnngggn nnnnnnnnnn 120
ggnnnggggn nnngggggg gggggggna ccnnnggnng gnaanggcaa nagaaanaan 180
cccncaaaac ccccnggggn gggggggaa aacccngggg ggnnnnnaaa gnaaancnan 240
anggnagggg nggcccnnan naanaaaaan ggaaanccaa nncnccncaa accccccccn 300
caanccccnnn naanccannc aaaaaaaan nnaaaannnc nccnaaanan aaccccaaaa 360
ancnnaccnc ngaacccnan nnncnccccc cccaaanggg anccaaagnn aannncnaac 420
ncnanngnnn gggacaccca acccnngcgg gggggcaan nccnccccnn gnggnngnn 480
tanaaaaaac cncaaaccgg aacccnnnng cccccccnna ngnnnccncn nanacaccng 540
annggcacc nccnaaaacc tnngnnccnn aggnaccaaa gnanngncc cctncnngna 600
nncgngccnc ccccnaggtt nanaannaaa ccncannnaa annaaanaaa nanancccct 660
ntnnctcaan cngngnccgg nnnnanccna nccncnaacc caangnctca cccccngngc 720
ccgnnanccn gnacaccana nccaccntag ggnggnncna cnccaacnan cngnncngtc 780
aaannncgcg ncnnnagccg 800

<210> 324
<211> 783
<212> DNA
<213> Homo sapiens

<220>
<221> misc_feature
<222> (1)...(783)
<223> n = a, t, c or g

<220>
<223> nbla-11775-rl

<400> 324
   gnntnttgnn nnntgggtgg cctttngnnn nnncttttta tcctgtcatt tagcatttaa 60
   taacaaaact atacagaatt atgtgcatac caatgaatgt tttgtaccat ctagttaaat 120
   tttttaaata aagttttatg ggttaagcag aagacaactg tcatactgaa ttttattaaa 180
   agtatatata cttcaaattc aaagcatccc ttaggaccca cagaatatat taaaactacc 240
   acccttaaat tttatatttt tgctttaaga cagacaatgc aaaggtaact ggcaagaggt 300
   gagcaaatgt tttagaacat ttatattatt gcttaaaatg agatttgaaa ttgtaataaa 360
   attcttggtt atgaagtctg atgtcttctt tgagcaccag tttaaaagga aacatttcaa 420
   acagtaaaat aaatcagtgt gtactttatt ttgagccaat gttttacata agaccaacaa 480
   actaagtgtg ggcaaactgt tcaatcactt ttgagaaatt acatggttcc tatttaggtt 540
   tgaaaaagta gtttgttgcc ataacatcag accttaaggt gttttaaagt gtatcctaan 600
   ggtctgacag aagaaaaata cagggtatgg aagtatccca actcatgcca cacagtatag 660
   atcattcatg gngaataatg gtcatctggt ctccaaaggt agtggaaaat attaagtcat 720
   caaataatgc tcagaantac caanggacag gttaaaaagg accaggatcc aaacaggttn 780
   ttt 783

<210> 325
<211> 2676
<212> DNA
<213> Homo sapiens

<220>
<221> misc_feature
<222> (1)...(2676)
<223> n = a, t, c or g

<220>
<223> nbla-11778

<400> 325
taggcctgta cggaagtgtt acttctgctc taaaagctgc ggaattcctc gagcactgtt 60
ggcctactgg cactgtctgg aaaacttgaa ttattttacg ccgtgaaaga aaaaggaaaa 120
aaaaaaaatc ttttctgttc ctagaaaatc tgaagtactg tgtttctccg ctagagggca 180
gactgctaat gaaatttcag gaccctcact gactgcagta gcaggattgt tcagtactga 240
gtggatgggt tagggtgtct gtgaacaaga tctagcccac acagaaacag gggatttatt 300
ccacagttag caccacagat tgcgacttgg gaagaaacca tcagctagag gggtctagtt 360
cgtatccaat cattattgac tgactgactg atactcaact agcaaaggca agaatttgga 420
agcacgctct acccagatgg gactttggag ttccttccct tccagagtcc ttattgaggt 480
gttgaagtgt tggcatgccg aagaatctta gtgacattta gccatgggtg tttctttaaa 540
aaaggaaaag aaaatgtctc aacgaagctt tgaaatggga gagtgttgat ttctagttac 600
attgctgggt tatgtagttg tatctgtggc taatttttct agtcctcaac aaatacacag 660
acatgctatt atggggttta attcaattta caaataggtt ttccttcctc acagtggagt 720
aatagaaaaa attgattttc tgctcctttg cagctgtgtc cagagacgga caatgaatcc 780
acaatttatt aggcagaggc acaacttctc caccattcgt tttatccttc tcttttctct 840
gtttccctcc acctctcttc ttcttctttc acactgagca gaaaacatat cttcaaaatg 900
aattcgcctt tgccatgtgc atattctctt ccttaaaagg acatcaacag tgttggagat 960
gaggagatga acattagatt ttggaatttc tgggagggag agggttaggg gtgactcagg 1020
gcctcccttc gcccaaaggt gaatcagaaa aggcacccct ttctctggga tacaacctga 1080
gcaggggtct cagctggcaa gtggagcagg gtgggggctc aacctcctca ccgcttgatc 1140
agctgccgtt gtacagagca tgacctgtat aaccttgagg gcagccctgg gtagatctgc 1200
agagtgaccc tctatatagt tagaatccag atgaaggcca taagaaatag tccatgccat 1260
gccgttaggc ccactttcat gtgcagtact tgagaaagca catcgtagcc tccttttcag 1320
acacacaaat ggcggctgga ctgagagcat ggcagagaag gggaggagga gaatgtacta 1380
aacttgctca ctgaattgag cttgagttat tagattgtag aagagcttga tgtctggtga 1440
ttttgttaca ggaagggggt cccgatccag accccaagag agggctcttg gatctcatgc 1500

aagaaagaat tcagggtgag tccataaagt aaagtgaaag caagtttatt aagaaagtaa 1560
aggaaggaaa gaatggctac cccatagtca aagcagctct gagggctgct ggttgcccat 1620
ttttatgttt atttcttgat atgctaaaca aggggtggat tattcatgcc tccccctttt 1680
agatcatata gggtaacttc ctgatgttgc catggcattt gtaaaccgtc atggcactgg 1740
tggtagtata gcagcgagga caaccagagg tcactctcgt tgccatcttg gttttggtgg 1800
gttttagcca acttctttac tgcaacctgt tttatcagaa ggtctttatg acctatatct 1860
tgtgctgacc tcctgtctca tcctgtgact cagaatgctt tagccatctg ggaatgcagc 1920
ccagtaggtc tcagcctcat tttacccagc tcccattcaa gatggagttg ctctggttca 1980
aatgcctctg acaggttcag cacctgagaa aagtaccttg aatcaggcct tggggcagag 2040
tcttcaagat tttaaggtga tagggcctgt ctcttaagaa tttattaatc gtaagtgctc 2100
actgctggtt tagagttaga tacatttccc tcaaaacggc catgggcagg ttatgtactc 2160
agaactgcca gggtgaaaac aatgagatcc agaaaaatcc cacaacaatc caggtttttc 2220
actcctcttt tttttctcaa tcttccgata cttgtggtac ctcttccatc aacagatacc 2280
tcatgtagtg acttccagta tcaagttttc aaaacttcct acccaccctc atcagtgcct 2340
ccttcactac agagtcacaa acattggtgc tctcaaataa aaatcagccc caaacaatgt 2400
caaagagatc ccttgagtag ttttcaggcg agagatatgt tctcatttca agtcttggag 2460
aaaactggat ccatgttcac gtggaacttc tctcgcggag gtgctatctc attttgcatt 2520
aaactttaag caggacagat tgctgaagcc atgatattta aggtttgact ttttaaaaat 2580
ctcattactc ttaaaataaa tgctgccaca tcagaaaaaa aaaaaaaaaa aaaaaaaaaa 2640
aaaaaaaaaa aaaggccaca tgtgctcgag ctgcag 2676

<210> 326
<211> 792
<212> DNA
<213> Homo sapiens

<220>
<221> misc_feature
<222> (1)...(792)
<223> n = a, t, c or g

<220>
<223> nbla-11822-f

<400> 326
ctcggcactg ttggcctact ggctgactac ctagatgatt ttgcattaat ataataacca 60
ttacctgcct tagtgctttg tacagtattg tggcaaaata gctaagccta aaggagttat 120
acaaaaagca gaattccata atgaaacaga attttacttt ccacataaat agcatgcttt 180
tttttttat ttttttaag accgaaatat tatatcagaa gtgtgttttc tttcctgtat 240
gatagttact tgcatggnac ctggttggtt cattttgttt tgttttttta ataccatgga 300
gaaatagaag ctttctaact tttctgtttg ccaatacatc tgttgtatcc attgacttgt 360

aattatgaaa acttgttttc cacaggggc ctgaggtgat gangcntaat tttcttctat 420
ncaggctagn ttgnatangg gcatnanaaa ctcggnctag ttnntatttt tttnctcctc 480
tntctcttna atggntgctn atntnctctg nctgnnntng ncgnnacttt ntgggancnn 540
nntanttcaa atttgnagnt gntccggtta tntgcnttan ccagtggann nnngccntcc 600
tngtcangct tgtacnnaag tncaggncnt gnatgctngn cngntctcta attnnntggn 660
cccnttncat gnngtttttc ggnnngcann nnntaagnnn ttttnntntn gcantncant 720
anntgnntcc gcannatnnt nnngcngttn ttcnnnatnc ntttntnttt cnnnctcnca 780
gtanttcgng ac 792

<210> 327
<211> 807
<212> DNA
<213> Homo sapiens

<220>
<221> misc_feature
<222> (1)...(807)
<223> n = a, t, c or g

<220>
<223> nbla-11822-r1

<400> 327
nnagnnnntt tgtggccttt tttttnnncn tttttttttt tttttttttt tttcanattt 60
catgccttct accttaacct ccttgagata acaacattca tttctgatgt tttctgtgct 120
acagggttca tgccttaact gggagttcaa gctaattctt gatttctttc tcaaaagacg 180
atttaaatnt aaggcaaaaa aaaaaaaaaa aatttccgat actgtaacta ccttaactgt 240
ttgagtntnt anaaatgagt atgttttatg agttaacaat gaatcatana aggcaattta 300
tgaanaacta gaatgattct gtctagctgc cttcagggat canaaattca aattaaactt 360
ntggcanacn tttaaagaat gctagcatac aaaactgaat ttaaaaaaac gcacacagtt 420
aacactcctn agnggagttt caagaggaaa tcatgaatta aaaaaacaca cggcntgata 480
cactgttgac tgatnccaac tgggtgtggt cagatcccag gtgcacccaa acctgcggga 540
atggcagaaa tacagctgng cttggccatc acagntntgc tagtatggcc tgctgtacct 600
taataccagc aactgggaaa acggtgttag cactaaaact ggtggtaaaa gcccataccc 660
actgntgggn aaatctgcct ganacgggtt tttntttaaa aangactctn tatcatntga 720
anctgaaaga cttttaaaat atatgcccta ttatttcctt taacccccaa tggggaaacg 780
tanatgatgg gaagggaaaa atttnaa 807

<210> 328
<211> 835

<212> DNA
<213> Homo sapiens

<220>
<221> misc_feature
<222> (1)...(835)
<223> n = a, t, c or g

<220>
<223> nbla-11860-f

<400> 328
gnngnnttgn nnnccnttnn tttgantcct cgagcactgt tggcctactg ggctatatcg 60
aaggttttc ccccagcagt atgggggccg tacaggtgat cgatcattaa tgtcattgca 120
attgttattt tttaaaataa atttataaaa ataaccgaaa agtaatgtga cttttgagga 180
atctgcaact cctgggttct agtttgtgtt tccggtgttg aaattttgat ctcccccgac 240
aagacttgtc tgcatggacg gtgtctctgt aaacagtatc aaacttgtaa tgtctgagcc 300
tcttagcttt atctcccctt aatttcttcc cttttggccc ttccaccctg ttgaggagat 360
tttttctgtc acctgcaggc tgatcataca tttgatgcta ctactgaaag tgtttatcaa 420
aatgtgatga agtaattcca tgaggaaaat tagccgttat cacaaaaaca gccttaagta 480
ttgtagaatt tcaggtagtg aagatgtact atttacgttt tgttagattt tgttttggca 540
gaagaccaaa acaaaagtac ttttctgatt gttaaaaatc cacagcatgc tcaccttggt 600
actggtttac cttacggtcg ctgggaatct aagcaacaaa gactagaagc ctccgatatg 660
ccattatcaa cacacaagag acaaacagcc cgaggaacac tcaatgatga gagcagtaga 720
cctgncctgc agatgaaaga ngagaactct ccacaatgga aggaaaacac tgncggattt 780
gatagcttta agcaaactgn gcttgagttg gcttaaatta tcccgtggac ctgnc 835

<210> 329
<211> 849
<212> DNA
<213> Homo sapiens

<220>
<221> misc_feature
<222> (1)...(849)
<223> n = a, t, c or g

<220>
<223> nbla-11860-r1

<400> 329

gnntgnnagt nnatttgngg cctttgaann nccctttgtt agaagagaat actttatttt 60
gaaataaaat acaaatgtgc aaaggaaatt agctcctcct gcccccctt tgaacaatga 120
gtcaatagaa tgtgagactg ggtagattag cagataatag gcaaaggtct agcttttcag 180
tggcaacttg aagaaaccaa agatgaatga tgctaaagga atgacccttt ggtacctgtt 240
ttaaagtact tctggccccc ttcttttata aacccccagg agcccagcac cacaccttgt 300
taccctacaa tgatcactca cgctccacga tgtcactaat gtaataactg aagatatggg 360
ccagtttgtc catgtcacgt tccgacttgt aggtcagggt gaacctgtcc attaatgctg 420
taaaccctga ccaacaaagc agaaattacg atgttaactc caaatcctaa acttttggc 480
cccttctcc ccacacatca ggccagtgta aaggaaacag atcccttcaa gctgagtaat 540
tcctttatcc attaacatgg tgtaacttag aaactactaa gggccaggtt ggttgctgct 600
gccactggga atccaaaggc caccctntct gntttcaaga gctggagatg gtgccctttt 660
catctggagc cagctntttn ccctactctc ttaagctgcc taactatngg aaacaatagg 720
atccctccta atgtcttggc tagagaacca ctnacacagt gagggcaggc acctggnatt 780
acccatcttn atatncctgg cacagantaa gnttttggta aatnaacgac ttncggagtg 840
gnttgtnat 849

<210> 330
<211> 885
<212> DNA
<213> Homo sapiens

<220>
<221> misc_feature
<222> (1)...(885)
<223> n = a, t, c or g

<220>
<223> nbla-11874-f

<400> 330
   gnnnntttgn nnncccnttn nnttgaattc cccgtgcctg ttggcctact ggttgatgca 60
   ataccaaagt gcttcccaga gaggctgtgc caatgtgtgc tccctcccct cccacgggtg 120
   agagtgccca tccactgtat ccatactggt gctgagtgct atcattactt gacatctttg 180
   ccatttattt aaagagcata tggctggttg ctaaaggtaa ggtggcaaac aaaaaaaact 240
   gtgaagagaa gcagaagttt aaattccaga caataaatga aaattggtaa aggcatctgg 300
   ttcttctaga aacatccttc cattaatttg tagatgagta tctcctattt ctaggaggat 360
   gtgtcaacat ttcacagcct cctagagtat tagctgcccc tatgcctctc tctatatatt 420
   ctcatctggc attaatgcac attagatagg gatttggaat gtttaccacc ttgttagaac 480
   ttctgagcca tttctaattt ctttcctatg ctgaaccctc caataaaaag caagtgactg 540
   nttatataat attagagcat tttgaggtac ctctgcagct aactagttta aaaatgacaa 600
   attaagtcaa agtttgattt gctccatgat ctgatggttg catttcctga gatgtttcan 660
   ggaactgact aaaaatttga tggagacagt ctttgagaat ggtctaaaaa actccacgan 720
   ggcatctaat tcaacccctt gctctaggac aaaccagcca aacatgaatc catgaggaag 780
   gaaacaattt tcaattttca ntcacagnag ttaanngntt tgccaaatcc ccgacaccgn 840
   tttttcctga cnggggnttg ggancangna ataannngga gttgt 885

<210> 331
<211> 888
<212> DNA
<213> Homo sapiens

<220>
<221> misc_feature
<222> (1)...(888)
<223> n = a, t, c or g

<220>
<223> nbla-11874-r1

<400> 331
gnnntttgag gcccttttnn tttttctgnt tgtttttttt tggttgttaa gtctcccatg 60
tttagtaatt aacagacccc gccagagagg aacagaacaa aaatgagcat gcaaatgact 120
aatcaaggaa agctggaatc aacaagatat gggtaaaaag atgctcctct gagcctggcc 180
atcatgcggc aggttctgcg cctctgatca cactgagctg cccaccttcc catgccctgg 240
aggtaaagct atgtagtcct accctggtag cataggctgt gccactacac aattcagcat 300
ttgacttgaa caagaatggg atactttgga tacttttttc tttaaaatgg ttccagtctc 360
ccaaacacaa gtatggttgt atgatgcatt ttaggaattt atttatcaca ctgtgaattt 420
ccacactgcc ttcttcataa gaattcaaaa tgcttgttca catttctaag gcatctaatg 480
gggggcaagt attaaaatgc ctccatttta cagataaggt aaactgagca aagtaattta 540
tcccaggtca gaggcaaagc aagtataagc cctgttccaa tctcaccaag tcataccacc 600
tctgcaattc aaaatggctc ctttaatttt ctcaaggtac ccaaaaaaaa aacacatttt 660
catatcttgn acaaactaaa aagctctaca gtggttctat ggcanctgtt ctttgcnaaa 720
gggggtncag tggtagacat ctacattttn atctttcatt tttataagca atgctttggt 780
gaacatctct ggatttttta cnaaggaaat gatgatcttt gcncagcaaa ctnttattaa 840
agcncagggn cangcccata tttaacctgn tttagccaca gnaatttt 888

<210> 332
<211> 885
<212> DNA
<213> Homo sapiens

<220>
<221> misc_feature
<222> (1)...(885)
<223> n = a, t, c or g

<220>
<223> nbla-11880-f

<400> 332
gnnnnnttnn nnnncnnnnn nnttgnattc cncgntcact gttggcctac tggagtgtga 60
caactgatcg ggtgaacgat gcaccactaa ccaccatgga aacaaggaaa aataaagcca 120
gctcacagga tctctcttca ctggattgag agcctcagcc tgccgactga gaaaaagagt 180
tccaggaaaa agaaggaatc ccggctgcag cctcctgcct tcctttatat tttaaaatag 240
agagataaga ttgcgtgcat gtgtgcatat ctatagtata tattttgtac actttgttac 300
acagacacac aaatgcacct atttataccg ggcaagaaca caaccatgtg attatctcaa 360
ccaaggaact gaggaatcca gcacgcaagg acatcggagg tgggctagca ctgaaactgc 420
ttttcaagac gaggaagagg aggagaaaga gaaagaagag gaagatgttg ggcaacattt 480
atttaacatg ctccacagcc cggaccctgc atcatgctgc tattcctgca aatactgaag 540
aagcatggga tttaaatatt ttacttctaa ataaatgaat tactcaatct cctatgacca 600
tctatacata ctccaccttc aaaaagtcat caatattata tcattaagga aatagtaacc 660
ttctcttctt caatatgcat gacattttgg acaatgcaat tgtggcactg cacttatttc 720
agtgaagaaa aacttgnggt ctatggcatt catcatttga caaatgcaag catnttnctt 780
atcaatcagc ttctatggac ttactacact tgctgnggaa tcctaagggc catacatttt 840
gaagaanaag cttagataag gcatgccctc cnaattatgg cttct 885

<210> 333
<211> 859
<212> DNA
<213> Homo sapiens

<220>
<221> misc_feature
<222> (1)...(859)
<223> n = a, t, c or g

<220>
<223> nbla-11880-r1

<400> 333
gtggcccttt tttttgtttt tgttcttttt ggagtaggtt tccttggtgg tttttaggac 60
atatttgttg gtaaacctat aacagttgct tttactttca gtgatgtact tttttctttt 120

cctgcttccc agagatttat cagaggagga taaagctcac ctaatgcaaa ggttggtttc 180
tgtaagtaat tcctcacata gctgtgtcca ccatcacagt tcatttctgg agagaggcag 240
ctgataagac atatcacacc aataatcccc agaaggcctc caagacaggc cataagtgtt 300
gtggtattat tcttttcata ctctttttga tcagggtgca aacctttggt ggtgacattt 360
acacattttt ttctgttttt ctgatagatg gtgggaatat caatacaaat tttatactca 420
gttgatggat tcagatgagt aagattatat accttgacat cagatggtat tcgagcactt 480
tgcgcagcat gagaattttc agtcttgaca aaggctgcca tttaacacta gatttgagaa 540
ttttagaact tgctttcagg acaccaaaac tgaattgcct gaatatctct tattttaata 600
ttcaaagagc cattggtatc ttgnggaaaa gaccatccct ttgacataac agacttcaag 660
cagcgncaac tagggtaggt gctatncagt atntaaaccc ctttttgggg agtacgcctt 720
tatatctagg gtccctcaaa tggacataga cttgctggca ggtattaggc aaaagttttt 780
gacncgaagg ngtatccata gatttcagct ggggtctnaa gtactntacn ngaaaggaac 840
atantccact tttcanttt 859

<210> 334
<211> 2013
<212> DNA
<213> Homo sapiens

<220>
<221> misc_feature
<222> (1)...(2013)
<223> n = a, t, c or g

<220>
<223> nbla-11882

<400> 334
taggcctgta cggaagtgtt acttctgctc taaaagctgc ggaattcctc gagcactgtt 60
ggcctactgg tcagcaagac acatcagtac cacaagatcc aggtgtggcc ctccagaatc 120
cagccggctg tctttgcttt cccatgtgct acacttatca ctgtgatgtt ggccttcatc 180
atgtacatga ccctgcggaa tgccactcag caaaaggaca tggtggagaa cccggagcca 240
ccctctgggg tcaggtgctg ctgccagatg tgctgtgggc ctttcttgct ggagactcca 300
tctgagtacc tggaaattgt tcgtgagaac cacgggctgc tcccgcccct ctataagtct 360
gtcaaaactt acaccgtgtg agcactcccc ctccccaccc catctcagtg ttaactgact 420
gctgacttgg agtttccagc agggtggtgt gcaccactga ccaggagggg ttcatttgcg 480
tggggctgtt ggcctggatc atccatccat ctgtacagtt cagccactgc cacaagcccc 540
tccctctctg tcacccctga ccccagccat tcacccatct gtacagtcca gccactgaca 600
taagccccac tcggttacca ccccctcgac cccctacctt tgaagaggct tcgtgcagga 660
ctttgatgct tggggtgttc cgtgttgact cccaggtggg cctggctgcc cactgcccat 720
tcctctcata ttggcacatc tgctgtccat tgggggttct cagtttcctc ccccagacag 780

ccctacctgt gccagagagc tagaaagaag gtcataaagg gttaaaaatc cataactaaa 840
ggttgtacac atagatgggc acactcacag agagaagtgt gcatgtacac acaccacaca 900
cacacacaca cacacacaca cagagaaata taaacacatg cgtcacatgg gcatttcaga 960
tgatcagctc tgtatctggt taagtcggtt gctgggatgc accctgcact agagctgaaa 1020
ggaaatttga cctccaagca gccctgacag gttctgggcc cgggccctcc ctttgtgctt 1080
tgtctctgca gttcttgcgc cctttataag gccatcctag tccctgctgg ctggcagggg 1140
gctggatggg gggcaggact aatactgagt gattgcagag tgctttataa atatcacctt 1200
attttatcga aacccatctg tgaaactttc actgaggaaa aggccttgca gcggtagaag 1260
aggttgagtc aaggccgggc gcggtggctc acgcctgtaa tcccagcact ttgggaggcc 1320
gaggcgggtg gatcacgaga tcaggagatc gagaccaccc tggctaacac ggtgaaaccc 1380
cgtctctact aaaaaaatac aaaaagttag ccgggcgtgg tggtgggtgc ctgtagtccc 1440
agctactcgg gaggctgagg caggagaatg gtgcgaaccc gggaggcgga gcttgcagtg 1500
agcccagatg gcgccactgc actccagcct gagtgacaga gcgagactct gtctccaaaa 1560
aaaaaaaaaa gaagaggttg agtcagcagg gacttgggtt ccctgtgtgt gagggggcca 1620
ttcttgcctg ccagctgctc ccgaggtggc cttgagaagg aagaagcagg atgacagagc 1680
ctgagcagcg gaaccagcct gcaccctccc ttctggccca gcgacctggg ctgtggctga 1740
gacaataatg aggccagaag tagccggagc ctgtcaggaa gggcagggga ggactgtggg 1800
gtctgggctc tgtcgctgta accatctgct cccaggctgt gtgcagaaaa tggcatttac 1860
actattgtgc agctcattct catgaaatac tgccattgtt gctaaataaa gcttgtgtgc 1920
tctgaatata gccaagcaaa aaaaaaaaaa aaaaaaaaaa aaaaaaaaaa aaaaaaaaaa 1980
aaaaaaaaaa ggccacatgt gctcgagctg cag 2013

<210> 335
<211> 1907
<212> DNA
<213> Homo sapiens

<220>
<221> misc_feature
<222> (1)...(1907)
<223> n = a, t, c or g

<220>
<223> nbla-11895

<400> 335
taggcctgta cggaagtgtt acttctgctc taaaagctgc ggaattcctc gagcactgtt 60
ggcctactgg gaactaagct acagccacca gggatttgt ggggaagatt acctccatac 120
tgggaggggc ccatgcaatt tgcatcacca tagctgccct ctccaggagt tcttgggaga 180
gcaattctgt ttaggtacat agcagaagag caagctccta gagtggaagt taaaggggct 240
gcttaatgtt tggtgtgtac tttgtgaggt tagggaggta agatgttaaa gtcagatgct 300

gctctactta gtatgggatg tcaataaggt acttacttca aagtaggtac tggaacatac 360
ctttcttcta agagactaag atttgttgt tgttcttcag agaagcattt gtggaatatt 420
tctgtagtgc ttgaatggcc aaagatcaat aaacaaaata aattacagtc ctggtggatt 480
ttaatattta aatttagata attatgatgt ccaaagagaa gagaaagaga tgctttaagt 540
cattaccaaa aatatcttag tccttgagag ggatttgctg aaccaaagtg agaatgaagt 600
ctgtgtgtcc ccttttctgc aaagaagctg acgccacatg atgcaaaggc ctgcccttaa 660
cccagttaat atgaacatgg catggcacca aagaggagac agaatcgaca gggagagtcc 720
cagttttaag ctcccaagca agatctttca gtggggatgg gaggggtcct tttacccttt 780
cctaatgact aaattaaacc aaacctaccc aagtggcccc tcctctcctc ctgggagatg 840
gtggtgccat tgtcaagata ggtgggacac atttgggaga gattggggga acacagtttc 900
accttcacat ttaagaatcc aatttactcc tgtggccttg tttgaactct ccttccaaca 960
ctcatataat aataattata tgtccttgcc ttctgtaagc tcatttcagt tggtgtagca 1020
aatatatttc taccacacac acaaaaattc ttctcttagg ggttcatgtg gaaatgaaaa 1080
cacaatgtgt tccactccac gtgaaaagca agtgctgtct ctgcagcaaa gtgcttcctg 1140
acaaggactg agctgaagtg tcatgaagct actcacctgg agggggcatc agcatctcct 1200
gcctcttaag tcgaccaggg aaagcttgga caagagcagc catgtaggtg ttacaggaaa 1260
ggtcctgatc cagaccccta gagaacgttc ttggatgtcg cgcaagaaag aattcagggt 1320
gactccacgg taaaagcaag tttattaaga aagaaaagga ataaaagaat agctactcca 1380
tggaccagcc ccgagggctg ctggttgccc atttttatgg ttatttcttg gtgatatgct 1440
aaacaaggga tggattactc atgcctcccc tttctagacc atatagggaa acttcctgac 1500
attgccatgg cctttgtaaa ctgtcagggt gctggtggga gtgtagcagt gaggatgacc 1560
tgaggtcact ctcgtcacca tcttggtttt ggtggctttt ggccagcttc tttactgcaa 1620
cctgttttat cagcaaggtc tttatgacct gtattttgta caaacctcct atcttatcct 1680
gttacttaga atgccttaac cttctgggaa tgcagcccag taggtctcag cctcatttta 1740
cccagctcct attcaagacc gagttgctct ggttcaaatg tctctgacat gggggctaat 1800
gacatcagaa ctggaaagaa agatgataaa ggaatgagtt tttttttaaa aaaaaaaaaa 1860
aaaaaaaagg ccacatgtgc tcgagctgca ggtcgcggcc gctagac 1907

<210> 336
<211> 777
<212> DNA
<213> Homo sapiens

<220>
<221> misc_feature
<222> (1)...(777)
<223> n = a, t, c or g

<220>
<223> nbla-11988-f

<400> 336
gangnnnttg attcctcnng cactgttggc ctactggtaa ttgctattga tgtggagatt 60
ttgcagaaca tggcagagtt tttgcgtctt tcacaagaaa tatataattg aatgatgagt 120
gcctggtgtt agcttttcaa ccttatttct tttggtttaa caatgcagaa ttgtgaacag 180
ctgcagcaaa taggcagcta caatatctag ctattatcaa ataaatgcag atctggcata 240
tgagctgagc taccactttt ggatctgcac ctcttgaata aacattctga agtgtggaag 300
catgaaaagt gaatgatttg ccctgaggtg tttaatcctg ccttgcattc ttgactgaaa 360
ttcttcacaa ttagggtgac taacattaat atgtggccag atgcatttgc acagattgat 420
ggattccccc ccacccttga tgttcctcga acaagtggtt aaaagtgtga gaggctttaa 480
aggcaaagcc acgtgccttc aggtggctga tattttccca aagaccatca tcaaacttcc 540
agccaggtgg ataatatcat ggcacccaac caggtgtcaa cactgaattc acttggcttc 600
aacgaggacc tggccataag agagagcaaa caggcctggt ttcaagtatc cangacctct 660
gctcagatcc ctcactgnaa ttcttaaggg aagcagcatt gtgaaagact ggctaggaga 720
aaagagaagg taatggcaaa tatttcctta attctgngac tttgggcaag cttactt 777

<210> 337
<211> 829
<212> DNA
<213> Homo sapiens

<220>
<221> misc_feature
<222> (1)...(829)
<223> n = a, t, c or g

<220>
<223> nbla-11988-r1

<400> 337
gnnagnnnnn ntttgaannc cntttgttgt ggcctttttt tttttttttt ttccccgaga 60
cgaagtcttg ctctatcttc caggctgcag tgcagtggca caatctcagc tcatggcaac 120
ctccccctcc caggttcaag caattctctt gcctcagcct cccaaagctg ggactacagg 180
catgtgccgc cacacccggc tcgttttttg tattttagta gagacagggt ttcaccatgt 240
tgcccaggct ggtctcaaac tcctgagctc aagcaatccg cccgcctcgg cctcccaaag 300
tgctaggatt acaggcgtga gtcaccgcac ccagccaaat gtccattttc ttctggctgt 360
aatcgtaaag gtaactatcc actctaaata catatggctt tctgggcagc actgagcaac 420
tatatagcta tcaactagct gaaagaagga aacaaaaaat atggccctag gatagaatca 480
gcccttctgt gtcatattca taaggactgt gccggaagca catgggaacc tggtccggaa 540
acccttcttt ctaaaatgtt agcaagactt ttttctttgg tagttcatca ttgcattgta 600
ccagtcacct ttctaagctc tttagaatca ttaactctgn gaattctcaa taataactct 660
aatgggtggg aagtattgca gcccattttt caagtaggga aattgaggat tgagaagtta 720

acntggccaa agtcccagaa ttaagggaaa actttggcat taccttntnt tttttctaan 780
ccagcttttn aaatgntgnt tnccttaaaa attgcantgn ggggactga 829

<210> 338
<211> 812
<212> DNA
<213> Homo sapiens

<220>
<221> misc_feature
<222> (1)...(812)
<223> n = a, t, c or g

<220>
<223> nbla-12017-f

<400> 338
gnngnnttnn tntgaatccc tttgtaagat ccctcgngca ctgttggcct actggtaggg 60
gttggatcct agagtagatg cctgaggcca catctgaaca gacctgtcag ccaggcctgc 120
caggtcttca cgttgaggat tcaactggcc aatcacagga caggtgtcct ggcctttctt 180
cctgaggtct ctaggggagg ggcatgggta agggtgtttc ctcagcaccc tcctggggtg 240
gggattatgt ctgctgtcat gtctgggtct ttagggtagg acaggctgtg gtatgagagg 300
caggagtctc cacaaggctt catgtggccc cttatagggc aggccctgcc ctctgggaag 360
gtcccttcat gctggaggca cacagcttta aggaagtagg ttgaagtagg actccttcgt 420
cctctcactg gctttggctc cctcaataaa ctgtgtggga acctggctca gtgtctgtct 480
ctctctctca ctctctggtt ttcctatctg aggtctttca tctctcactt cangaaaaca 540
caagttcaac aaagtcttca natgccatcc tgtgtaggaa gaaaataccc ttctggtgcc 600
ccatgaaaaa gggaaatacc aaaaccattt ggtcactgac ctttgcaata aggtgcttcc 660
ttaccaattt caaaaagctt cccttgcaag tagatatcga gagagcacac tttncattan 720
agcctggtaa tacccatatn acctttgntn tgaagctggg cctgcaactt gngaancntt 780
tggaaaagtn ancccttgac cccnaancta aa 812

<210> 339
<211> 851
<212> DNA
<213> Homo sapiens

<220>
<221> misc_feature
<222> (1)...(851)

<223> n = a, t, c or g

<220>
<223> nbla-12017-r1

<400> 339
gnngnnnttg nnnnccnttn atttgtggcc cttttgattt ttccnttttt tttttttttt 60
tttttttttt tttttttttt tttttttttt tttttttttt tttttttttt tttttnnnaa 120
nntttnttnn ttnattnttt nttttggnaa nngggnnnna nttnnnnncc nnggnngggg 180
nncntnggcn naannnnggt tnnccnnacc nttnnccncc gggcctnngg ggaccccccc 240
nccnaanccn ccngngnngn tnggnctncn ggnnngngtn nccnnnttgg gnnaattttt 300
ttttngaaan aaantttngn tttngtnncc naanntnggg ggnaagggcn nnanntnggn 360
taacnggccn aagntancct tngtncccaa gttaaagngn tttnncggcc naaaccntta 420
aaggggnnga aattnnnggc ccccnccncn aantcnggnt ngnttttttng nnttttnaaa 480
aaaaatgggg tnccccccntn tnggccangn nggtntaaaa nnccngtntt nnggggaccc 540
ccctntttgg gcccccnaaa nngncgggnt tncngggggn aaccattggg cccggccnnn 600
ttttggnntt tttnggaaaa aaggggtttt gccattttgc cnagggtngg ttaaaacncc 660
nggggntaaa aggganccan nanntttngg ttnnccaaaa gggcttggna ttnnnggnnn 720
gggccccccn ccctagggtg ggtanttttt ttaaaaccgc cnttttgccc anaaaaaggc 780
cccttnggtg gaaaaccctt ggaaccccng gggggggggn ccnctttttaa aaacccgcct 840
tanggggnng a 851

<210> 340
<211> 768
<212> DNA
<213> Homo sapiens

<220>
<221> misc_feature
<222> (1)...(768)
<223> n = a, t, c or g

<220>
<223> nbla-12022-f

<400> 340
   gntncctcgg cctgttggcc tactggctac ttaagtgtaa acaaggcaag cctcagacca 60
   gcaataaatt actcagtttg gataacatta ttttgtgcag ttaatcaaat ttgccaaagt 120
   ctttatctgc ccctttaaca agttgagtaa aaataaaagg tattttttag tcaatgtgtt 180
   ccatgatttt gcttaaatta atacttttaa gtaatggaac ttttttcaaa ggcaaattta 240
   aactatttaa gaaatagctc ctaatacttg ggatcttgtt tagagaatcc actttctgga 300
   agttctcagc ataattagtg ttgagagtgg ttcagttgtc tttaatgttt gtcatgtgga 360
   aatggaagta gcctcttttt gttctgaaat tgagtttatt caaagtgtaa aagcacatac 420
   tgcattttct gctgaaagat cattatgttt aacaggcact taatctcagt aaagtcagtt 480
   gccagttaaa gttcccccag tagtcagtcc cctttgtagt tagtgggatt atttgataat 540
   tgggtagatc atacttgaaa atttaatgct ttgggtaatt ggttggaaaa cagtggaatt 600
   gggaaacgca aacttttggn cctattcnag taagggnaat ggagtcctgn ggaacccaat 660
   tgnatacgna aatttgnagg agtctaggta cattangcca tccntcntaa ncctggtgag 720
   tngaatgcnn tttagaanga ntacctacat cctttggnaa nncctaaa 768

<210> 341
<211> 847
<212> DNA
<213> Homo sapiens

<220>
<221> misc_feature
<222> (1)...(847)
<223> n = a, t, c or g

<220>
<223> nbla-12022-rl

<400> 341
gnnagnnnnn ttgtggccnt tttttttttt nttttccctc agtaagtatt ttattgagat 60
ttaaagcaca aaataggtga acaaatcggg aggaaggtgg taacacagga atgggaacgt 120
aaaagtcaaa gtaggcagga ctcaaactgg ccataaaggc actacggcat gttcataaac 180
accatcaagt aaaagaaagg ggaatcaaga tgttctcact tcttttctgc tccaccttaa 240
tactccatct agccactggt atggagcatg tcatcaactc attcttctca caagctagca 300
agggaattaa acattttatc agtcaaccct atgctcacca agtgagccaa tgttacacag 360
tgattttgtg catccagtga ctgagaacta caggcaagga taggccctga ctgttaataa 420
attggtcgtg gctattacta gaaaattatg ggtgcaggca tttacctata caatgacctg 480
cttactgtgg cagctaacaa tatagtcaaa ccagtctatt accatatgat gtcctattaa 540
caagagagcc cagttgaagg atgtttcata cttttagaaa gcctaatttc ttactgnaat 600
atcctccatc cctcttttaa aaaggctgna gttcaaaaag cttttatgnt ctttcctgaa 660
acaaagagaa ctttggtcaa aagccaattt ttaagaagca tcacttctgc tgagtaggca 720
aaacaatgga ctntnaccac cagacatnat tttctttcct taaaatctga nggttatgct 780
ggnaaaataa cccatttttt tttaaacatc taattaataa aggntcaang aaatnagata 840
tggagga 847

<210> 342

<211> 914
<212> DNA
<213> Homo sapiens

<220>
<221> misc_feature
<222> (1)...(914)
<223> n = a, t, c or g

<220>
<223> nbla-12048-f

<400> 342
ggnnagttgt tcctntgcct gttggcctac tgggttacta gcttgtgtga agacattctt 60
ttctccttcc attcttgttt acagttagct gagcagatga cacagtcaga tgcacaggat 120
aatgctgact acagattatt tcagaaaaca ctcaaattgt gtcgtttttt tgccaactcc 180
cttttgcact acgctaagga atttcttcct ttcctctctg attcttgctg tactttgcac 240
caactgtatc ttcagataca cagcaagttt cctccaagcc tttatgctac caggatttct 300
aaagcacacc aagaggaaat agcaggtgct ttcctagtga cactggatcc acttatcagt 360
cagctgctca catttcagcc tttcatgcag gtggttttgg acagtaaatt agacctgcca 420
tgtgaactgc agtttccaca atgtcttctt ctggttgttg tcatggataa gctgccatct 480
cagcctaagg aagtgcaaac cctgtggtgc acagacagcc aggtctcaga aacgacaacc 540
aggatatctc tactcaaagc cgttttctac agttctgagc agtgttctgg tgaactctct 600
ctacctgttc atttacaggg attaaagagt aaggggaaag ctgaggtggc tgcaccttgt 660
atcaagcatg tttgtgttca tctgnggtac atttattact tcctttcatc cctcactggt 720
tggtgaactg gatgctgctc tgctgaatgc tgtacttaag tgctaatatg aacacctctt 780
tgttagctnt gggatgcatg gngcttcctt gctcgatatn gggactgctt gaactggggt 840
gcaccaccat gttaaccata agtgggntcn atcttgataa aagncnatnc ccctgggnga 900
aatgttttn aacn 914

<210> 343
<211> 995
<212> DNA
<213> Homo sapiens

<220>
<221> misc_feature
<222> (1)...(995)
<223> n = a, t, c or g

<220>

<223> nbla-12048-r1

<400> 343
ggnnagttgg gccttggnnt ttcctttttt tttttttttt tttttnttc aaactttaca 60
aaattngttt tatatctgtt anaaaangtn cagacataag tnttttcagt ngacaaagcn 120
tcaaacccag ttntgccagt tctgcctagn gataagtttc accctagagt atgtatgtaa 180
cgttttagct tatccatcct ttntnggagc gcctccnttt ccattgaaag ccaggctgga 240
ncaggaccct ttnggagtag ggactnagtt gnttccaaag cccctgntnt tgtatgcagc 300
gctgacctgt actnttnttc ccaggggaac tcctgacgag ctntttttgc ntaaggctgt 360
aaanatgacc tttttgnttt ttntacagtc acattagcaa agggttccca naaaatacct 420
ttttcctgtt tnacacgttc cantttggca gnttcagttt natttacaaa cccagtnttt 480
tccanaangg atacaacttt gttnttagtt tnttcanaac tganacactg gggaactatc 540
tnttcatgat tngttccctc agcgaactga gtaaacgcct ccaaggtntg ttgttttagc 600
agccaactcc tgtcagntag cagtaaggca aacatacagg gacaggttgg gcagaattnt 660
tggcctggga tangcttcag gtataaaaag tttttccnta anggaaggat ccccaaattc 720
cccaanattt ggccaaaccn naacattagg ttcaggggaa gngcttttta nttttaaaag 780
ntaaggccgg gggggtcttt ngacaaaaag gtttnaacct tgcncctngg aanggttttt 840
ccantttttt tanggggatc ttttaaagaa gtttttggga antgggaaaa aaaaattccc 900
ccaaancagg ggngggggaa nggtaaaaaa aangggcctg ggaaaatggg tcttgggtng 960
ggaaaaaaat ataaaggggg ggtggngatc ccncg 995

<210> 344
<211> 961
<212> DNA
<213> Homo sapiens

<220>
<221> misc_feature
<222> (1)...(961)
<223> n = a, t, c or g

<220>
<223> nbla-12055-f

<400> 344
   nngttgttcc tctgcactgt tggcctactg gctgaaatat taataggtgg cctataagga 60
   tctttcatgt tttttgaatt tcttaattaa gtatcatttt gtgttaagct tagaagactg 120
   agagctgagg agagtggaca aataaatgtt ggaattagga aagttacatg aaggaaagtg 180
   ggtctgagct gggttttgaa agtaggcaga atttagatca gtagagcaaa gaggatagga 240
   tagtctaaat gtggagaatg ctgtatgaat ggaggcagaa tatgtattga acatgaggaa 300
   ccatcaagag gccggctcag tgagggaaga agggggcttc tggaggcacc ataggtagac 360
   ataagacatt aacagattat tgaccaacag gcaggggagg ttaatatgct ccaatgaata 420
   aagagccatc atatatttta taatggagac taacaggttg atgttgctga gaaaataata 480
   tggactttgc attcaatgat gtatcttttc acaaccatat ctttgataaa cacaatttgg 540
   gttatactgg cagggattaa gtagtggaaa atctcttaga agaaatgtgc ttctactgtc 600
   ccccttacca aaatagaaca gatgagccct aatcagagct ggtaactgta atggtgattg 660
   ctctgaggac ccaatgtata taaacatcac tatttgaata cctagatttg ggccaagtaa 720
   taagggaggg aaagtttttg acccctgaga ttacccccca gaattaaaag taggggaccc 780
   tttttggaga tggggcttaa aacangnttt ttcaaggtnc agttagncac cccttggctt 840
   tttcaagaaa ggaaggcngg accnggaaaa ttccccctnc ctttggaant ccanaatttg 900
   gtatngtttt cccgggaatt ttttaacccc gaccttaaga tctcagggcn ggggttcanc 960
   a 961

<210> 345
<211> 902
<212> DNA
<213> Homo sapiens

<220>
<221> misc_feature
<222> (1)...(902)
<223> n = a, t, c or g

<220>
<223> nbla-12055-r1

<400> 345
   gattgggcct tttttttttt tttttactaa ttcagttcat ttaactttgc ctccagcagc 60
   tcaccataaa atttgggtcc aaaaaggcca aggaatgtat tatcagaaat gttagctatc 120
   tctaggaaaa tctgtcattc atttaagtga taatctttaa aattagcata actatattct 180
   ggatcatttt attacgagac tatcatgtga tttcaatgtc tgagtttatt accattgtac 240
   gagcaacatg ctaagttaca actgtaactc ccagggttgg tttagcagga aagtgatgga 300
   ggcagactta acagtgatgc gtgtagttca gcaaaggcca aatgacagca ctgggagcca 360
   taatggtggt ttcctagtaa ttcaaacaga ggaaagtagc aagaatacac tgatctcaga 420
   ataccaggct tgtcagacta aaaaatcctg tgctttaaca gtcagtggtt gacagcaact 480
   tatcctaagc aaaaaaaaaa aaatcatcta aaacagatgt catggtaatt tcaattttat 540
   agctttctga agtgttgatt ctatttaatt tgcatgtttg ttttgttttc atggctgtat 600
   aaagacctat tacacaagag ttcaaagcct gttttttaac atatacttta atctcatgca 660
   atattataaa gccaaaaaat taaatatgat tttagaggta ttntattcaa gatgattttt 720
   ccccttttag gaagggccct tttcatttct caaaatttaa cctngcagtt tacccattgn 780
   ctttnttttg gttattcnag gccncctatt aaccttttca aaattattgg gtttancttt 840
   ggnaaaaang gctttttaaa tcttttcctg cccaggctaa aaattctnga gaacaagcng 900
   cn                                                    902

<210> 346
<211> 1532
<212> DNA
<213> Homo sapiens

<220>
<221> misc_feature
<222> (1)...(1532)
<223> n = a, t, c or g

<220>
<223> nbla-12074-f

<400> 346
   ggcccgannt acnctnngga aagncccccc accgtttngg gaaaaaggac ccgccggggg 60
   tggctgcgtg ccctngtggc ggatntccag cctnttgtat gtcggcccan gngcgctgaa 120
   ggctgagggn gctttntgcn taggaancgn cncctgcggt ggangaggcn gcggttgccc 180
   catgnagtgt tactacattt gnaattagct tnacgcattt tannatcgag acacttnngn 240
   aacatnaagg gagnanntcc ggggaccttt tagcaagaac ngggagcana antnnggact 300
   ntgctganaa aggaanatac caatngaana agcttctgga agatctgaan gnnaatttnt 360
   antgttgaac ttcatgnnac aatgnantac tataagcacc acgagttcnn caattacant 420
   taatttnata ngancatgct nacagncatg aggcttnant ggaaatnnaa acannangng 480
   aatntgctcn aaaatntagc atctacnntt tnnncgcnga cgtnnngann nagaaaanna 540
   gngtannaag ggctaccttt naaananacc cggncannan annntgnnna ttggaanctt 600
   ataantaaaa angggaaaca anatngnnga ntggnagggc acnacctntn nggtaanggn 660
   agngnnacan ccncannnga tnttnacgaa antctnnaan cnatancncn gtngtatatc 720
   cttggtcntg atancaaagc nataaaaaaa cnnctgnggt atatatntgt ggaaaaaaaa 780
   tatnttgtct cncatnaaaa ntagangatn tntcnagnct ccttgntang cgaaanctnt 840
   ntntgtatnn atgtntntan atagtatccn cccnnnaatc tntacacngc aaaatttcnt 900
   ttacaacaan aangannnat ggancntcnn ttcattttna tatgttnttn anaaaagtat 960
   tgatntacgt nncaaaaaag cttaaangna aatnctnnnc tcntctattg acncgcnncn 1020
   atctggtana ntgtaaangg ctcaagnaca ctntacagat ttanagnctt tttcagnant 1080
   nanngtaaaa aaannctctc nggtaataaa nantaaangt ggttttccnt ncagcnannt 1140
   attnagtntt ntngtttant nccccacnan gtnccacact atatntttaa tttnttaacn 1200
   cnncatnann ntgntntntn nntggggntn gtnttataan aaaaaatcct tctcccgaga 1260
   nnnggncgat atatttatnn caccncngta nannnncccn cgggtannta nanttnccag 1320
   tgtgntcgna gacagcgtcg cntataaagn gnaaaaaant nttnccttcn naannanagn 1380
   atanagngnc cncnanntca tanaancgtg aaatncncnt aantganaan atcnttatgt 1440
   atanaacnta cgcgtnnact ctcnttnntn ntctcntgtn tcttatctaa tnggcnaaag 1500
   nattcgaatn cnntctccnc annacaanag cg 1532

<210> 347
<211> 1007
<212> DNA
<213> Homo sapiens

<220>
<221> misc_feature
<222> (1)...(1007)
<223> n = a, t, c or g

<220>
<223> nbla-12074-r1

<400> 347
gttggggcct ttgtttttt cntttttctt ttacttnaat ggaaccagta ttcatgggtc 60
tctttcaacc ttattttgtt gtattttna tctagagtgt cctgttttat ttgacttggn 120
agtanaatta tacttttctg ctnctctgat cttacaggaa atgtttttat acctctgacc 180
tgtgttctca ttttttccag aatcacaact actggagaga atatcatcag ccaaaagagg 240
agtgacttgg ctgcatttat ttttgctact tccaatttca caatcagtga gtccttctgt 300
agatacatcc ttgtaatctg aaagtggtcc catgcaaatg tcttcctgct tctgctgaga 360
acaaagatct tttaagtctg gcttattttt atctagatta ttatttactt tagtagactt 420
gaagtnaaaa catagaggat tacagctata ggaaattgat ggtttcgtag ntgtataaac 480
cagcatttct gatggccact gaagaactgt agatctgtgn ttgttaagga caggtacaaa 540
tggcntcaca ttgncttttg cangaaattt gctnttctta tgtantagnn atgttnaccc 600
tcttcagtat ttttgaaggg ggcccaattg tctcggggac cattttttta ttttcaacct 660
caanttgngg gannccncg gtttgcttta acattttcc ttctntnggg nannctctgn 720
ccncannatt tngccantna attttnacnt tgtncaagaa acntggcggg attttntttt 780
tccnntaacc aaaccttcaa aaancnatgt ttnntcccca nnttaaacaa ggggngcnag 840
nntcctttcc aattttnatn ttanccaacc cncccttttn ttctnnngga gnggggnnaa 900
tttttnngtn cccagcgncc tataacnngg gggggacccn cngaaanann ttttntgtga 960
ccncaaantn ntntttctng tcgcttttn cagagaaaan ntaanca 1007

<210> 348
<211> 963
<212> DNA
<213> Homo sapiens

<220>

<221> misc_feature
<222> (1)...(963)
<223> n = a, t, c or g

<220>
<223> nbla-12075-f

<400> 348
ggnagnnntt ccttggantg ttcccctact ggttcgactt atctatnagc atcttcaaca 60
atattcctac agttgtatgg gtggcatgtt ggccatttgt gatgtagccg aatataggaa 120
gngtgccaaa gacttcaaga ttccaatggt attacatctt tttgatactc tgcatgctct 180
ttgcaatctt ctggtagttg ccccagataa tttaaagcaa gtctgctcag gagaacaact 240
tgctaatctg gacaagaata tacttcactc cttcgtacaa cttcgtgctg attatagatc 300
tgcccgcctt gctcgacact tcagctgaga ttgaatttac aaaggaattc agtgtcagtt 360
cctttacaga ggaatgtctt atacttcagc agccctcggt tgatagaaag cacaggagat 420
accttatgac acagccaaca ttttgtgaaa caatgactgg aacaaaacag cagccatact 480
tacctttgag gttttattta aagtttggat accactagct atattttgct tttttcccct 540
cacattgaat tttaattcca ttcttgaatg tagaaatttc agattctcta aaactacatg 600
tcactgtttt tatcctagaa aatgttgctg tcagaaggca aaggaaatgt taccagtgtt 660
ttccggttct tgtacttttt aacatattcc atttagaaat ttttgccatt ctgntttcca 720
ttaataatag gnngaaatac agggaaaaac tacatttttgg tnattccctc aagtttttta 780
antgaccctt tttcagncat caaatttggt taantcaang aatnattttt taagggtttt 840
tccnnaanaa taaatttttt tttggncttc nttttccaaa aagggttnaa acnaatttan 900
ancattactt ttccttgncc annttngggt ttganttggg aantttttttt tttttggng 960
ggg 963

<210> 349
<211> 977
<212> DNA
<213> Homo sapiens

<220>
<221> misc_feature
<222> (1)...(977)
<223> n = a, t, c or g

<220>
<223> nbla-12075-r1

<400> 349
gttggggcct ttgtttttc nttttggat ngcttaaaat gtnctctcat gttttatttg 60

agatttttt tcgtttttc ttttttncaa attgacactg tacaaagagc ttaaanggcc 120
agtnaaacta ctgccttaaa attgttggct ttaagtaaca gaggtaaaat ttgaattcac 180
atcagagctt tctaaaatgg caatagtaca agcaaatgac tttctctagt atccaaaacc 240
tgcgctggct ctcccttggc tagaaacaag acagccttct aaagctgcca taatgtccta 300
aaatgctctg aaagaagttg gtatacttca ttccccagct taccttggta acactggact 360
aaacagaaaa tgaagaaaat tcagtcaata gtaacttaaa cagtactaag ttagctctgg 420
acttagaagg tttggtaaag ttgatgacat tcagaataag gttctcagaa gaaaagcagt 480
tttccaaaaa taggctaaaa agcctatatt aaagaaaaaa tgccaagagt gaattgaata 540
cttaagttta tctgttccta gaagactatg aacaccacaa tgggagctga aacactatat 600
ataatttaga atgcattctc ccagagcaag tttataaggt ttatagaata gtaatggcct 660
ttctaaattt tcacttggat ttttaaaaat ttttattaag gtgattttat ttantgtcca 720
ttccaaaatt taaagcccna tttcccattt aaacttatta nttcccaagg gttngggaaa 780
ttaaaccttt tcangaaaaa tggccntttt tgnnaaaaaa aggggaacca aattggaccn 840
ngganccctt tttnangaac ncntgggaaa aaaggccnaa accnanggcc cnttttggca 900
aaaagggcnt ncattctttc ccaaaaattt ttattcaaat nttccttcaa aattnctttc 960
ctttanaaaa naaaaaa 977

<210> 350
<211> 2698
<212> DNA
<213> Homo sapiens

<220>
<221> misc_feature
<222> (1)...(2698)
<223> n = a, t, c or g

<220>
<223> nbla-12092

<400> 350
   taggcctgta cggaagtgtt acttctgctc taaaagctgc ggaattcctc gagcactgtt 60
   ggcctactgg ggtgctttct gtttaacctg gaaagtgtga ttatattcgt taccttcttt 120
   ggtagacgga atagttggga ccacctttgg tacataagaa attggtataa cgatgctctg 180
   attagcacag tatatgcata cttctccaaa gtgatatatg aagactcttt tctttgcata 240
   aaaagcatta ggcatataaa tgtataaata tattttatca tgtacagtac aaaaatggaa 300
   ccttatgcat gggccttagg aatacaggct agtatttcag cacagacttc cctgcttgag 360
   ttcttgctga tgcttgcacc gtgacagtgg gcaccaacac agacgtgcca cccaaccccc 420
   tgcacacacc accggccacc aggggccccc ttgtgcgcct tggctttata actcctctgg 480
   gggtgatatt ggtggtaatc acagctccta gcataatgag agttccattt ggtattgtca 540
   cacgtctcct gcctcgcttg ggttgccatg tttgagcgat ggccctgttg atttcaccct 600
   gccttttact gaatctgtaa attgttgtgc aattgtggtt atagtagact gtagcacatt 660
   gccttttcta aactgctaca tgtttataat cttcattttt aaagtatgtg taattttttt 720
   aagtatgtat tctattcata tggtctgctt gtcagtgagc cagacttgct tactatattc 780
   ctttataata atgctagcca cttcctggat tctttagtaa tgtgctgtat gcaagaactt 840
   tccagtagca gtgaaggagg gttgcctctc caagcttcct aagggatgct gccctgtgtg 900
   gggatgcatt gcagaggcac tagtagcatg ggggctagag tggggagcga gatgtaaaag 960
   ggtgggggga taggagaatt ccagagtgct tccagcatta gggtcctgag aacttctgag 1020
   ttcagagaaa catgcaaagt gactaacaaa atagctactt acctttgcag ttctacagac 1080
   cctgggagct gctttgggag tgagaaaggc aaccctccaa tgtgtttcaa ctttaaaatg 1140
   ttgaattctt ttcagacatg gtatctcatt tattctcctt ttctagcgtt tgttgaattt 1200
   caggcagaat gtcttacaga atgtcctaga accagattat catttaatct gaaactgctg 1260
   aggaagggac agagaaggta caagggcaag gcagcacaaa gcagatcagg agaatgaaga 1320
   gggaatgctt tggttttttg ttttgttttg ttttttcttt ttcaagtaac taaaacagca 1380
   tctacatgta gagtgttgtg gagagctgag accagggtaa agtcaagtgc agcatcagta 1440
   ctgcgagacc caccagcccc tggagagggt cagccgagaa tctggtagtg aagcctgtct 1500
   agggtcccgg caccctcacc ctcagccacc tgcagagagg ccagggcccc agagactagc 1560
   ctggttctga agtgggcagg ggtgctgcca gagccctctg ccccttatgt tgagaccctg 1620
   ctttcaggac aggccagccg ttggccacca tgtcacattc tgagtgagtg tcacaggtcc 1680
   ctaacaataa ttttctgatc tggagcatat cagcagaatg cttagcctca aggggcctgg 1740
   aagctgtaat gtttgattta tgatgagaac tatccgaggc cacccttggc ctctaaataa 1800
   gctgctctag ggagccgcct actttttgat gagaaattag aagagtacct aatgttgaaa 1860
   acatgacatg cgctcttggg atctgctgtt ctctccaggg ctccagaacc tgatacctgt 1920
   taccaaagct aggaaagagc tttatcacaa gccttcactg tcctggcatg agaactggct 1980
   gccaggctca gtgtacccca ttaactgtga atgaatctga gcttggtttc ctttattgct 2040
   tcctctgcaa tatgattgct gaaacacatt ttaaaaattc agaagcttgt cactcctgtt 2100
   aatgggagga tcagtcacac atgtgtagta caaggcggac tttgtgtttg tttttggtgt 2160
   taatttttag cattgtgtgt gttgcttccc caccctgagg agaggacacc atggcttact 2220
   actcaggaca agtatgcccc gctcagggtg tgatttcagg tggcttccaa acttgtacgc 2280
   agtttaaaga tggtggggac agactttgcc tctacctagt gaaccccact taaagaataa 2340
   ggagcatttg aatctcttgg aaaaggccat gaagaataaa gcagtcaaaa agaagtcctc 2400
   catgttggtg ccaaggactt gcgaggggaa ataaaaatgt tatccagcct gaccaacatg 2460
   gagaaacccc gtctccatta aaaatacaaa attagcctgg catggtggcg catgcctgta 2520
   atcccagcta ctctggaggc tgaggcagga gaatcgcttg aacccaggag gcggaggtcg 2580
   cagtgagccg agatcatgcc agtgcactcc agcctgggta acaagagtga aactccgtgt 2640
   caaaaaaaaa aaaaaaaaag gccacatgtg ctcgagctgc aggtcgcggc cgctagac 2698

<210> 351
<211> 968
<212> DNA
<213> Homo sapiens

<220>
<221> misc_feature
<222> (1)...(968)
<223> n = a, t, c or g

<220>
<223> nbla-12112-f

<400> 351
gttgttcctt ggcactgttg gcctactgga agatatattg attgcctgcc gattgaacca 60
aaaaaaaggg gcatatgata ttttcctgaa tgcaaaagag caaaacattg tgtttaatgc 120
tgaaacctac agcaatctca ttaaattact gatgtcagaa gattatttta cacaagcaat 180
ggaagtgaaa gcattcgcgg agacccacat caagggcttc acacttaacg atgctgccaa 240
cagccgcctc atcataacgc aagttaggcg ggattatttg aaagaggctg tgacaacact 300
gaaaacagta ttggatcagc agcagacccc ttctaggtta gcagtgaccc gtgtcatcca 360
ggcattggcc atgaagggtg atgttgaaaa catagaagta gttcagaaga tgttaaatgg 420
actcgaagac tccattggac tttcaaaaat ggttttcatc aataacattg ctttggctca 480
aataaagaat aataacatag atgccgcaat agaaaacatt gaaaatatgc ttactttcag 540
agaataaagt cattgaaccc caatacttcg gcttggcata cttattnaga aaagtaatag 600
agggagcagt tggaaccagc agttgaaaag ataagcattc atggcggaga gattggccaa 660
ttcaagtttt gcaatttatt aaacctgtcn ctgatttttt cctttcncct tgtnggattg 720
caaggcaaag gtggattgat gccaagaagc tcttcctaca gganaatgtg gntgccaatt 780
gcttgaacca aaaccccgga tttttgttgg ntggtccctc cttaaggaan ttcttaggga 840
aaccnangga aagggccttn taancttgtg gaaantntng gngttnaaaa attgntttcc 900
ctggntttaa attgaaaang gngngnangc cttccnnatt tcccnnatgn naaaagnntt 960
tttnttct 968

<210> 352
<211> 913
<212> DNA
<213> Homo sapiens

<220>
<221> misc_feature
<222> (1)...(913)
<223> n = a, t, c or g

<220>
<223> nbla-12112-r1

<400> 352

gtggcctttt tttttttttt tttataggga tcacttttat ttcaaacaat taaatacaaa 60
ccaatatttt accccttcat agatgaaatc acatcttttc aggatatgag tataaagtaa 120
caagcctagg gcagagcttg tactgacaaa gtcctgaaac tacaatgaga ggaaacacat 180
tgctctactt cgggataagt catgaccgag actcaatttc agagacgctc tatgaacaga 240
ggtgcttgaa gccacagtgg cagaagggaa agatggggaa gtgtgccgaa gagcctccag 300
gcatgacaga cagtcccctg accaagcaca agtaacaggc cctttgggtc tctgcttctc 360
actggaaaat gatgaagcct agatctgatg actcctagtg ccaacattta acaaagttcg 420
aaagttatgc aggacttcac acatgtacgg aatggctgta tcacagaata ttatgcgtta 480
gaaagttcac ggtcactata cctagctcta aaattttca gagaaacagc agactaataa 540
gtgaatctta aataaaggaa taacatttta atgaaataaa tgaaacagag cagggaaacc 600
aaagagccaa ttaggggaag antcctgaaa aagtatggct tnacacagca gcagcattga 660
agaaaacact atcgattttt nccagagaaa aaaactccaa aaatgtncaa taacccaagt 720
gcccagagtt ntgggtcaaa taaagacttt tgaaccatgc catcaccaca taccntntgg 780
tcccttaaag naaaattttt tcantttttt tttttcccnt caagntataa ctttaaaaaa 840
taacccntgg tgggaccacc nngaatcncc aaaattattt tcccnaaaac caaaagttnt 900
cgccntggnt tnn 913

<210> 353
<211> 1133
<212> DNA
<213> Homo sapiens

<220>
<221> misc_feature
<222> (1)...(1133)
<223> n = a, t, c or g

<220>
<223> nbla-12119-f

<400> 353
gttgattcct cngcactgtt ggcctactgg aagtttaagg aattttagta tgtaatagca 60
aagtgtttga ttaaaagtct aatcctatat tactagtcaa aattagttac attctgatct 120
aggaaagata atagtcattg gatattgata tgctgtgctg tcagaaaacc agactaagat 180
agaaacctct atctgactcc cacatgttgc ttgttgtgat gggatgtata ctttacagca 240
gcttattgct gctttgaaaa aaaatgtata aactatacat ttggagtgtt tgcatataat 300
tctttataac ctccacttaa agctgtcaga cattggtatt ttatcagtcc acattgttga 360
ataaaactaa tgttcttagg aatccagctt gtacacactg tttaaaaacc ctcaggacaa 420
gtttacacac tcttntcact caattcaggt actttgatgc tatncttaaa cctaacagtg 480
acttgtattt ttctgttttg ctttnatttc aacgtgctgg tagcacatct gatgaatgtc 540
aacttaaaaa actcagntcn agggtatcca ggnntnactc anccnaacag atttaaagc 600

tggcctntta actctncagc acatgggtgc ctcacacnnc ttataagtgg gcnttcctat 660
ntaatncagg tcntttactt cnctgnnncc agnatnaatc attnggggnn nncccnngtn 720
tacanaaggg nctatctttt ttcaaannaa anaaggccgc cttttantnt tgtttttngg 780
tccaggncta tcaacttttc ancacnacnc tntctcttaa gaacccttgn gcncnntngt 840
tttaaacaac tcttnnttnt ttttctanaa nantggggcc cncnntaatn tnangnccct 900
angctnacan aactcatttg ttaaagcnaa tctgtnnntc tcttancctc tnacaatctn 960
ngnnnctcct cgggnnaagc gncccgggng ngtaaaacca angaaaattn ngccacgngc 1020
tntccctttt ttntatactc ggactntcat gtgggcctnn catctntgag ngtttattnc 1080
ttcttcncta gcgtnngtaa nnacntccta ccccttnctt natanaaacc tct 1133

<210> 354
<211> 952
<212> DNA
<213> Homo sapiens

<220>
<221> misc_feature
<222> (1)...(952)
<223> n = a, t, c or g

<220>
<223> nbla-12119-r1

<400> 354
   ggnnagttgg ggcctttttt tttttttttt tttttttcca acaaaattta ttagaagaat 60
   agtggttttg aaaactctag catccagtga gaactaccat acacaacatt acagctggga 120
   atgtttntcc aaaatgtcat ggtcaaataa tacaatggaa ccattaaatc ttacacatgc 180
   acgaaagaac aagcgctttt gacatacaat gcaaaaaaaa aaaaaagggg ggggaccaca 240
   tggattaaaa ttttaagtac tcatcacata cattaagaca cagttttagt ccagtcaaaa 300
   atcagaactg cattaaaaaa tttaatgtag tgcaatcaaa ccaaaaaact taatttgnga 360
   tcataagtgc tctactacat aaacattgat catagcangg aacaaaaagt tcaaatcaca 420
   cagtacaaaa aaatntgtaa ataaatataa actacagtcc aagagaaata ttaaattnta 480
   caattccatc aaactgtaaa cagtntattg aaatgaggtc cataagagta aagggggttc 540
   ctgttttgtt gtatgacact tgaacttnta gaaatntgaa agataccatt ttccatatnt 600
   ccaaaggcta tgggttttat atgtggagag aatttatttt agatgtaaga tttcaaatat 660
   taaagcagag aaataatcta tttttgttaa ccacagaata gtactgcaga aaaacaaaaa 720
   agcaantttt aaaggaggca aggtntttaa ccattttaaa ngatgccaaa agggacttaa 780
   aaacaactgg gaaatttagg aaacttttgc cattcannaa aaaaaaactt ttgttaacct 840
   tggaanaacc tttcaaangg ggttaccaaa aaataacccc cngggccaaa aaaacttggt 900
   cttggcttgg attatttgcc aanaccccta naaacccttt ttggttttaa tt 952

<210> 355
<211> 957
<212> DNA
<213> Homo sapiens

<220>
<221> misc_feature
<222> (1)...(957)
<223> n = a, t, c or g

<220>
<223> nbla-12121-f

<400> 355
gtgttcctcn gcactgttgg cctactgggt catgagactt attcactatc acaagaacag 60
ttcaggaaag acctgccctc atgattcaat taccttccac caggtccctc ccacaacatg 120
tggaaattca agatgagatt tgggtgggaa catagccaaa ccatatcaac ctctttaata 180
aatatttgtt gaataaataa atgaatacat ggacgaacgt ttcttcagcc tttactgcct 240
ggtgcaagtt gagtgggaat tctgcctcta gtgcatcctt gttgccaaga gagacgcagt 300
tctgctgctg ccaccagatg catttctctg ccaatgtgtt ggtgcaggct aatggaaatg 360
cactgctgct cctacatctt ccttgtggat cttcatcctc cttccccatg cttttcaaaa 420
aatgagatga ctacttctct cttctatatt atttattctc tgcttggctg ggggcagtag 480
aatgggttga attctcacag caagattact tggttcctaa attttttttt agcaattatt 540
gtgacagctc tttcaatgaa tatctatggt tctattactc tccagggagc acacacacct 600
ccatagtgtc ttcaaaagga tttgggtttg gatnagaaca agcagccatg ctcaagccnt 660
ggtctacctc ttccttctga cctttatgca ttgtttataa agtcangntc actgtgacaa 720
cattcatttc ntttccttnt tgatgaattt tctttcaang ggctttatta acccttccct 780
tnacaaactg ggctttctgg ccaatnccan ccatnnttan gtcttgttgg gncacaattc 840
tttgggcnaa gttttggttt tattnnaggg atttcaagnc ntttnttntt ntnaaaagnn 900
ttctttcaat ttttttattg gtaaaccaac ctttnngccc aatgggggga acacnac 957

<210> 356
<211> 891
<212> DNA
<213> Homo sapiens

<220>
<221> misc_feature
<222> (1)...(891)
<223> n = a, t, c or g

<220>
<223> nbla-12121-r1

<400> 356
ggnnagttgg ggcctttttt tttttttttt ttgtagagat agggtctcac tatgttgcct 60
aagctcgtct caaactcatg ggctcaagta atcctccttc ctcagcctcc caaactgcta 120
ggatcacagg cgtgagctgc tgcacctagc cagaagatca attttgatac acactatggt 180
atcaattgaa tttttatat atttgactgt ggagaaaata atggactgaa aaccctagcg 240
ttcttacttt gaaatccatc actgcatgtg caatgaggtc ataatcaagg gtggtgagga 300
taccgagtca ggtctattct ggaaaatgca gtcctcctct gaaggagaag gtttgtctca 360
agaagtcagc atcaaccttt taatctttct ataattgcat gctgtctaag tttttttccat 420
ccaaccttcc ctctcttctt tgcaagtgta agactgtcat ccaactctga tggctctcct 480
ggcctcctct aacttcccat ttttcctcac aggtatttcc cctcatacat aacttgcatg 540
tctaatcctg tcttggagtc tgattctcat agacctcagc ctaacaaaaa tggcaccaga 600
agtggtccaa gaaaacaaac agtataatag agatatagga acggctcata taccaccaag 660
tgggcaaaaa gaacagtatc ctgactggng gatngtgaca cattgtccct tgaatcaana 720
atggtaaaga actctagctc aatatttagg tagattcagc agtggaatta tctaaccaag 780
nggncctata ctgggccttt acaaccaaaa tttaacagct tcatggtttg gcttttcttt 840
aatactattn ccctcttttn tttgngaaan ccaattattt tttttttntt g 891

<210> 357
<211> 978
<212> DNA
<213> Homo sapiens

<220>
<221> misc_feature
<222> (1)...(978)
<223> n = a, t, c or g

<220>
<223> nbla-12124-f

<400> 357
   gttgttcctc ggcactgttg gcctactggt gctgttgcta tggtgaaaac aaatcaatat 60
   cacatggcta ccagccagtc ccgcgctcag ggagtgccat agaaattcaa gatcaggtac 120
   tcaagattcc aagcaagaat tgggaaaaga ggatttagat gctgagctta agagaattcc 180
   ttatagtaga ataagtaggt tcaatctctc atgcattttc tcttcctttt ttcctgtact 240
   ttcctaccga taacaagaaa aagatgaaaa ggaaggtata ctgatttgtt tacatttcca 300
   aaatgacaaa acagattatc aacatagaaa taggttggca acattgcaag gggtgaagga 360
   ttctgattcg ggaaaaggtg gataactaca agacatgttg gttgttgaaa acatagtttt 420
   gggggccttt accatcttca ggtacagcta catgtcagag ctggctgggt tgtgccaagc 480
   agagaaacct ggcaactcta gggggcaggt gggcagtgga gataaagggg gttataaaaa 540
   ggtagggagg tgtgggcata aaaatcaaca agcaagaaag cccagagaga gctgcagggt 600
   caagagtcag gccaggcaag cacaggatcc ctgnggcatg gcagaacagg gagaggacag 660
   tttcgccctg ttcacgaaat ctgttnaaga cccaagctna agggctagcc anagaaaggc 720
   angaaaatag cttgcnnaga ggaagtnaag gtgtcatggg gcccaggcct tcctttcccc 780
   ggaanggggt ncnncntnca accttggttg aaccattngg ttnccccccc tnggttgggg 840
   gangangcca aaanancttn ggnnattttt taaangcntt tggggtttcc nttttccaaa 900
   aaaaaattna anggccnggg aannnacttt ttanccgggc tttttgntna natttantnt 960
   taaccttccn ntttttnt 978

<210> 358
<211> 916
<212> DNA
<213> Homo sapiens

<220>
<221> misc_feature
<222> (1)...(916)
<223> n = a, t, c or g

<220>
<223> nbla-12124-r1

<400> 358
ggnnagttgg ggcctttttt tttttttttt ttgatttttt ttttgntttt tttttgtaa 60
ctctgctttt aaggaaaaag ggggaaagcg aaattctgca gagtcaggaa acaggtttcc 120
ttgctagaga tagagacagt gagataaagg catggggctt cccccacacc ctgcagctca 180
naagtgtggg acaggagtct aagacttcct gttgttggct ggtatttaca gctttttaga 240
tgatatgtcc attttcccca ggcactttgg ggccaaaact cagtatgctg agactcagaa 300
attgctctca gtagcttcat attttgattt ttttttgcac cattcttatc tatctattgc 360
tcatctgaca tctttatatg ttatttaaac agtacttatc tggagaaata taacagaggt 420
caagaaagta catacataca gatacctaaa actcaaaagt ccatctcttg ttctccccca 480
atcactaaaa ctgccccaag cctaaaatta atctgatggt taacattgct aaattaatat 540
tagccaataa agttattcaa gtctttgaaa cttacaattt tgcatcattt attttgtca 600
cacacacaaa ggcaggtatt aggttactga gttgggtttc tcaccaaata gcaaagccag 660
ggaagcccan aggtgtttaa taaaccttgt tgactgattg atgggaatat actacctagt 720
ttaattnaan caatgcattc ttttggnaaa aacactaagg gtaatttatt ttgggtcccn 780
cttttttttt accccaagcc ggttttcttg cttgtnnttg ganggaaggg ccagggccna 840
nnccccaaaa aaattnggtc ccntttaaag ccttnaagna gntttaaggg ttttacnaaa 900

aaagaaaaaa ccnttn 916

<210> 359
<211> 970
<212> DNA
<213> Homo sapiens

<220>
<221> misc_feature
<222> (1)...(970)
<223> n = a, t, c or g

<220>
<223> nbla-12137-f

<400> 359
gttgntcctn ggcctgttgg cctactggat aacctgtttc ggaatagtct caagatgctg 60
cttactggtg ggaaatcaag tcgtaaaaac aggtcaagtg gtggcatctc cacagaaagc 120
gactgtgctt ttgagccaga ctacgctgtc ccgccacttc cagtgagtga aggtatgcag 180
cacattcgga ttatggaggg catgtctcgc tctcttccat cctccccctt actcacacat 240
cagtctatca gtgttcggct ccagcctgtg aagaagttaa ctggtgatgc tgaacaggag 300
cttggccccc ctccatctgt agatgaggca gcaaacacac tcatgactcg tctgggtttc 360
ctccttggag agaaagtcac agaagttcag ccaggtgacc aatacagcat ggaagtacag 420
gatgaaaatc agacctcagc aatcacccag cggataagtc cttgttccac tctgactagc 480
agcactgcct ctccaccagc cagtagcccc tgctctacac tcccacccat cagtacaaat 540
gcaactgcca aggactgcag ctatggggct gttactagtc caacctctac ccttgaaagc 600
agagatagtg gcatcattgc tacattaaca agctattctg aaaatgtgga acgcacaaaa 660
tatgctgggg gaaagcagta aagaattang atctggagga aaacataaaa ccttgggcaa 720
ttttcaaaaa atccancatg ggacctcctg ntttgntatt cggagtagat tgaaaaacat 780
taacttgctt nccaccctat tnagtncttg gaataaagaa tccccacang aagaaanttt 840
tgggaaaaca gtggnttaat tcttaaatca agtacanggc taatttcctt ttggnaatct 900
taaatggccc ccggggccaa annttnagtt nngcaggcca ccnagttttt gccccacttt 960
gggaaanant 970

<210> 360
<211> 950
<212> DNA
<213> Homo sapiens

<220>

<221> misc_feature
<222> (1)...(950)
<223> n = a, t, c or g

<220>
<223> nbla-12137-r1

<400> 360
tgtggccttt tttttttttt tttggacttt ggaatatttg catatatata atgaaagatc 60
ttggtgatag gacttgggtc taaacaagaa attcatatgt ttcatacaca ccttatacac 120
atagcttgaa ggtaatttta ctcaacattt tgtgaaacaa agttttcact gcaacccatc 180
acatgaagct aggtgtggaa ttttccactt gtggtgtcat gttggcactc agaaagtttt 240
tgatttcgaa gcattttgca ttttggattt tcagattagg gatgctcaac ctgtacatta 300
tgatcagcac agttcacctg caatatggct agggctgaat ctggtccgca gtcacttctg 360
aaatccaaga tcttagaaag gttatttccc ccattaccac agccccttta catctgtttt 420
ctttcagaat ttcgaggaac taatgcttcc aaatgttgat aattacacaa tgcctgtcta 480
tgaggagtac aatgtatttg tgtttatctt tctctagact cagcagaaat gaactggtca 540
catgctaaaa tggatcattt cacttcttag ggaggtttaa gtaagtatgg gaataggaga 600
caaagcaact ttttggcttt ttgcccattt ccatctgttc ccgggcaccc tttanagccc 660
ttctgtggtt ctaacctctt aaaatccctg ctgtgtgcga gcnccccccc atgctctgtc 720
ttggcattcn ntagannaag ttcctcaaag ggtgttattg ttcttntggc tcattcccag 780
ggttaagcan aggggncntt tncaaangtg gggccacgaa ccctcnggtg ggcctggcct 840
tanctggaaa nttttgggcc ccgcgtgggc cnttgganga nttncnanng gggnanttnc 900
gcccctggtc ccttggantt gggnaaaaaa aacnnccttg gtttttccca 950

<210> 361
<211> 980
<212> DNA
<213> Homo sapiens

<220>
<221> misc_feature
<222> (1)...(980)
<223> n = a, t, c or g

<220>
<223> nbla-12156-f

<400> 361
gntgattcct cngcctgttg gcctactggc attttgagtt gttctagtgg tatatgaagg 60
aggctgggat aactagcttg aaagaaattc agtctagtta tagacatctt tggcattaat 120

ctgatgttta ctagtgatat ctcatgctag gcagttatgc tttgcttcta ggggcttctc 180
tttttaaaac aaaagaaagc tcttttcgtt ttctgtgtgc tgcatgctcc agtgtgtgtg 240
tttacaccat cggttcttct ccctctagag attagcataa ctccctttgc tgttggattg 300
ttattttgag caatatgttt tggaaaggtt ggttttcatc atgagtgcac gcaaatcatc 360
agatgcatct gcttgctcct cttcagaaat atctgattcc tttgtgaaag agtttctagc 420
caaagccaaa gaagactttt tgaaaaaatg ggagaatcca actcagaata atgccggact 480
tgaagatttt gaaaggaaaa aaacccttgg aacaggttca tttggaagag tcatgttggt 540
aaaacacaaa gccactgaac agtattatgc catgaagatc ttagataagc agaaggttgt 600
taaactgaag caaatagagc atactttgaa tgagaaaaga atattacagg cagtgaattt 660
ttcctttcct tgttcgactg gagtatgctt ttaaggata attctaaatt tataccatgg 720
gttatggaat atgtcccctg gggggtgaa aatgttttca catnctaaaa angaaattng 780
gaaagggttc nngtngagcc ccccannccc cgggnttctt atgcnagcct ccanaataag 840
gnggcntnnc natttcggn gtacccttcc ctttcnactt aggacccctc atttttccg 900
ggaggantct taaaaanccc tgngaaaaan tcttctttta aatttggacc cccttcaaag 960
gggcttttt ttccccgccg 980

<210> 362
<211> 942
<212> DNA
<213> Homo sapiens

<220>
<221> misc_feature
<222> (1)...(942)
<223> n = a, t, c or g

<220>
<223> nbla-12156-r1

<400> 362
ngtggccttt tttttttttt tttgggttt ngaaccnnag nnttantngg naangggtna 60
ntccatnnta cnttncnnta ntcnannncn tttnangggc aaaattnccn ntttnnngnn 120
tttncntttn nnnnantaag gggggtanna nnnggggnta ntttnttttc cnnnnngggg 180
nnnnnnantn gggnccttta nnttaaaaac caaaggtccc ccaantttcn ngnnttnttn 240
ttaannaang gangggnaaa ccnaannntn ggnnnttant anggttttnn ggggnnnttt 300
ttttangggn cccagnnttn aancngtttt natttnaaan nttnnncngn tnttnaantc 360
caaantttna aggttggttt taaanngatt tttttttctt aaccggnnga ttttaaantt 420
nacccantnn nnngggntng gcaanncggg gccatnnctt aaaatngann nccctttatt 480
tttanntanc ccngtttttt tnannaangg taantttant tttnncngnt ncctngttna 540
aaaatggnnt tnnttttttg attttaatta aaggtatttt tggtaannnt ncangacctt 600
tttaatccng ccnnttaann gnttggaacc atttttttta ccatttccat ncttngtttt 660

ggntttagna aaaaatnttt cttccnttgg ggattttaaa ggggattttg ggtnntcncc 720
atcattntta aatntggacc nttntanccc tntttggnnn gtnttcccct tttttnaaa 780
aatttttnn aaaaaanaa aanacnacct gncctttgng ctgggggtaa aantttttc 840
nnnanaaaaa gnttngggnt ttgggttccn ttttancaaa aagaggtttt ntttttccca 900
naaaaccnaa accnttttt cntntaaaaa ntcngnggac cg 942

<210> 363
<211> 727
<212> DNA
<213> Homo sapiens

<220>
<221> misc_feature
<222> (1)...(727)
<223> n = a, t, c or g

<220>
<223> nbla-12204-f

<400> 363
   gnttgattcc tcggcactgt tggcctactg gcaatcaagt aattttatta gcttcttttg 60
   gaccctctaa atattgactt ctctcatgaa aaaataaatt gatgaaacta atgattacaa 120
   agatataatc attttttaaa aagtgattgc ccaatgtatt tctctaacaa ttgtcacaag 180
   agaaagcata acaataaaaa tacaaaaaca tacagattta gatgtaaaat ctatataagc 240
   tatattttta gggaggctaa gcagatagta ttactgtgga agaattatca agttttattc 300
   acctcaaatc ccactgggtt cttaaaactt gaaaattcaa attgtagaga attatgagac 360
   acaatgtgat gtttagttaa agtcatgcta tacctttctg ggccacatat tgctaactct 420
   gtggctaatt atgcaattaa ttctcaacgt atcaaagctt ttcactggca gtaaattctt 480
   tgccctcagg tgaagtggat tgaaaagaca tcaaggatca aggataatca ctttgaatct 540
   gttagttttt ccccctacat tccagacact ttaaatttgg atgctttcat tttttttaaa 600
   tcaaaccaca caaatatgca gatactttcc cagaatttcg cagttaaatg gctgatcctc 660
   ttgaaaacta accttaatgg aattctaaac atttcagttt agaatgactt tgaaaaattc 720
   cttagat 727

<210> 364
<211> 709
<212> DNA
<213> Homo sapiens

<220>

<221> misc_feature
<222> (1)...(709)
<223> n = a, t, c or g

<220>
<223> nbla-12204-r1

<400> 364
ggnnngnttn gggcctttt tttttttttt tcctttttt ttttttttt ttttttttt 60
tttttttttt tttttttttt tttttnggca gggggggtng gaananccaa tgtttttntt 120
taanaaagtc ctgnttaaca gcntnttnta ncanaaaang nnggnttttt aaaacgggct 180
tgaacttttn cncaatntgt tttgaacnct ggcntgcccn cctgngtnca tntntnctgn 240
tnggngctnt ggtttaatt tttaatttca aaaaatttg ataaaactaa aangttaaat 300
gncccctnta atgaaatcnc tgntttaact aacnccnncc ananggnttg ntttcnttgg 360
aaaattttt aaagcntttt gaattaaagt aanaactttg tttttaaan aacnccnttt 420
aanggncgat taanggnngc nntttgcccc aangnngggn ttccctaatt tgcctaangg 480
taaaaancnc tggggngntt gttaanagtn ctgttttnca gggcctnccc cagngttttt 540
gaatntaaat ttttannggg gaaaggggga atttgcctga acccaancaa ttnctgtant 600
cagggaagtt tccaagctcc cctnntttac ggcaaanggg ttttgaaact ngcntttaaa 660
aanaccatag gnggggngaa ttagtnnttt gggcngtttt attttttacn 709

<210> 365
<211> 724
<212> DNA
<213> Homo sapiens

<220>
<221> misc_feature
<222> (1)...(724)
<223> n = a, t, c or g

<220>
<223> nbla-12217-f

<400> 365
gnttgattcc tttgcactgt tggcctactg gattgtggga tgatcatggg aacgattctt 60
ccttaagccg aagtcaaagg gaaatccacc ctcacgctgc atgtagtttt cggtcattta 120
cacaaaccaa cagttttatg gagcgccttc tacacgctca gagataggga tacgtaagat 180
aaatggccca gggtctggag aactagacga ataagcagtt tcagcacaat atgataaacc 240
ccacctaaga ggcagggaaa cccctcaaaa ttagtttcct tattccccct atcttattcc 300
tcctatctat atctttctta gggttgaaaa tcctaattaa aaagtagtaa ctcgcgggag 360

atttcattgc taaacatttg tcaaccactt tcttgtttga ataagtgact tccccaaatt 420
agcaaagacg actctgaaaa tagaatgctg cataggcaag attggatgag agagagaaga 480
tcttggagac aggacaggta actggagatc gttcctgggc aagtgtgtat ggagaagtgt 540
ccgtaccctc aatcagtaaa tcttagtgta atgtcatcag aatgagcatc aaagagagcc 600
ttaagggacc cgagaataaa ctaggattct caaaccaaac atggcccaca gacagcttgg 660
tggacaggca tgtttttctt ttgctgcact ctagcctggg cgacagtgcg aggctcttgt 720
ctca 724

<210> 366
<211> 869
<212> DNA
<213> Homo sapiens

<220>
<221> misc_feature
<222> (1)...(869)
<223> n = a, t, c or g

<220>
<223> nbla-12217-r1

<400> 366
gnattngtgg cctttttttt ttttcttttt tttttttttt tttttttttt tttntttttt 60
nttttttttt tttttttnnt tntttntntt ncnnanggnn canttnnttt nntnanaagg 120
ggggnttngn naaggagcnc tnactncnaa nncnnnaana cnngnggggn ggggnttttn 180
cntnttnaan anggnngncc cncncnctnn nacctaaccc naaatggcct ngggggnccc 240
nccccccttt nnngagntgn anggggnggn tgnnaacnnn nngggggaaa gacnggnacn 300
gggcaggggg gntnnnttna ctgnanacan nagcannnga ctaagaatga cnnaagacnn 360
aacnacnnnc caaggctnna aaggggtnng taananngaa aacnaggggc aagggggcat 420
angggggtgag gaantnaanc tttaggancg gggggacnaa aaacanngga ngnntgaacn 480
nncttgacat ngtgntacnc ttangaacga ngganacccc tgnaactcac tngncacttt 540
atatncntct cccccngggg ggggaatcct naaaaaancg cnttttnttn accccgngaa 600
atanaaacac ccccccntnt tngaangngg annnttcaca gggnggngnt ttnnncntat 660
nggccccaan agtttccttt ncttannana anacaaaann cntttttcaa ggccccnnnn 720
ttnnnncncc gnggggnctt tttaangncc canaaggcna agggncatnt gccntnctat 780
cangggnggg gggggcntt tnccataata ancgccaccc naggggggggg ggncanaanc 840
caccncnagg ngngggggggg gcttcnaca 869

## Description

### Technical Field

This invention relates to nucleic acids whose expression is enhanced in human neuroblastoma with favorable prognosis based on comparison between human neuroblastoma with favorable prognosis and human neuroblastoma with unfavorable prognosis.

### Background Art

### (Tumorgenesis and Genes)

Individual tumors exhibit distinct characteristic natures, and their biological properties are not necessarily identical even though the basic principle of oncogenesis is the same. Rapid advances in the understanding of cancer from a molecular biological and molecular genetic perspective in recent years have opened the way to an explanation of oncogenesis and tumor cell biology on the genetic level.

### (Neuroblastomas)

Neuroblastoma is a pediatric cancer occurring in sympathetic gangliocytes and adrenal medullary cells which originate from cells of the peripheral sympathetic nervous system. Of these sympathetic nervous system cells, neural crest cells in the initial stage of development migrate to the abdomen, differentiating and maturing at sites where sympathetic ganglia are formed. Some of these cells migrate further to the adrenal bodies, penetrating through the adrenal cortex which is already in the process of formation, and reaching the medulla and forming medullary substance there. The neural crest cells also serve as a source of other peripheral nerve cells, differentiating into dorsal root ganglia (sensory nerves), skin pigment cells, thyroid C cells, some pulmonary cells, intestinal gangliocytes, and the like.

### (Prognosis for neuroblastoma)

Neuroblastoma is characterized by a varied clinical profile (Nakagawara, Shinkeigashu no Hassei to Sono Bunshi Kiko [Neuroblastoma Development and Molecular Mechanism], Shoni Naika 30, 143, 1998). For example, neuroblastomas occurring at less than one year of age have very favorable prognosis, with the majority undergoing differentiation and cell death, and spontaneous regression. Currently, most neuroblastomas discovered by a positive result in the commonly performed mass screening of 6-month-old infant urine are of the type which tend to undergo this spontaneous regression. On the other hand, neuroblastomas occurring at age 1 or higher are highly malignant and lead to death of the infant in the majority of cases. It is also hypothesized that a somatic mutation occurs in highly malignant neuroblastomas in infants older than one year of age, which are of monoclonal nature, whereas in naturally regressing neuroblastomas, the genetic mutation remains at only a germline mutation. See Knudson AG, et al.: Regression of neuroblastoma IV-S: A genetic hypothesis, N. Engl. J. Med. 302, 1254 (1980)).

### (Genes which allow the diagnosis of prognosis for neuroblastoma)

With recent advances in molecular biology research, it has become clear that expression of the high affinity nerve growth factor (NGF) receptor TrkA is closely connected with control of differentiation and cell death. See Nakagawara A., The NGF story and neuroblastoma, Med. Pediatr. Oncol., 31, 113 (1998). Trk is a membrane-spanning receptor, existing as the three main types, Trk-A, -B and -C. These Trk family receptors play an important role in specific nerve cell differentiation and survival in the central nervous and peripheral nervous systems. See Nakagawara, et al., Shinkeigasaiboushu ni Okeru Neurotrophin Juyoutai no Hatsugen to Yogo [Expression of Neurotrophin Receptors and Prognosis in Neuroblastoma], Shoni Geka (Pediatric Surgery), 29:425-432, 1997. The survival and differentiation of tumor cells is controlled by signals from Trk tyrosine kinase and Ret tyrosine kinase. In particular, the role of TrkA receptor is most significant, with TrkA expression being notably high in neuroblastomas with favorable prognosis, and its signals exerting a powerful control over survival and differentiation of tumor cells, and cell death (apoptosis). In neuroblastomas with unfavorable prognosis, on the other hand, TrkA expression is significantly suppressed, while tumor development is aided by a mechanism in which survival is promoted by signals from TrkB and Ret.

It has become clear that amplification of the neural oncogene N-myc has become clearly associated with the prognosis of neuroblastoma. See Nakagawara, Nou-shinkeishuyo no Tadankai Hatsugan [Multistage Oncogenesis of Cerebral and Neural Tumors], Molecular Medicine, 364, 366(1999). This gene, first cloned in neuroblastoma, is ordinarily only present in a single copy per haploid set in normal cells and neuroblastomas with favorable prognosis, whereas it has been found to be amplified several dozen times in neuroblastomas with unfavorable prognosis.

Up till the present time, however, no oncogene other than N-myc is known to be expressed in neuroblastomas, and absolutely no genetic information other than that of N-myc has been known in relation to favorable or unfavorable prognosis.

### Disclosure of the Invention

This invention has been accomplished in light of the circumstances described above, and its object is to identify the gene sequences which are related to favorable or unfavorable prognosis of neuroblastoma, and to allow the provision of their genetic information as well as the diagnosis for favorable or unfavorable prognosis.

As a result of conducting diligent research, the present inventors have examined the prognoses of neuroblastomas and have succeeded in constructing cDNA libraries from both clinical tissues with favorable prognosis and with unfavorable prognosis. Approximately 2400 clones were respectively obtained from these two types of cDNA libraries and were classified according to the prognosis of neuroblastoma (whether favorable or unfavorable).

Moreover, the present inventors found that the expression of a considerable number of the genes is enhanced only in clinical tissues of neuroblastoma with favorable prognosis among the classified genes.

Based on such knowledge, the present inventors have succeeded in providing base sequence information for the detection and cloning of the genes only expressed in clinical tissues with favorable prognosis.

Furthermore, based on the aforementioned base sequence information on the regions it has been made possible to carry out the method for detection of prognosis and to design tumor markers which can be used therefor, and this invention has thereupon been completed.

Specifically, this invention aims at providing a nucleic acid whose expression is enhanced in human neuroblastoma with favorable prognosis based on comparison between human neuroblastoma with favorable prognosis and human neuroblastoma with unfavorable prognosis, the nucleic acid comprising any one of base sequences set forth in SEQ ID NO:1 to NO:366 in the Sequence Listing as well as providing a nucleic acid comprising a portion of the base sequence. This invention also provides an isolated nucleic acid characterized in that it hybridizes to the nucleic acid described above or its complementary nucleic acid under stringent conditions.

The nucleic acids of this invention are those whose expression is enhanced in human neuroblastoma with favorable prognosis based on comparison between human neuroblastoma with favorable prognosis and human neuroblastoma with unfavorable prognosis and those nucleic acids are characterized by their capabilities of being used in the diagnosis for prognosis of human neuroblastoma. The preferred nucleic acid for this purpose is a nucleic acid comprising any one of base sequences set forth in SEQ ID NO:121, SEQ ID NO:125, SEQ ID NO:132, SEQ ID NO:133, SEQ ID NO:165, SEQ ID NO:171, SEQ ID NO:187, SEQ ID NO:188, SEQ ID NO:210, SEQ ID 229, SEQ ID NO:232, SEQ ID NO:273 and SEQ ID NO:281 in the Sequence Listing or a nucleic acid related thereto (e.g., a nucleic acid comprising a portion of the base sequence). The particularly preferred nucleic acid is a nucleic acid comprising any one of base sequences set forth in SEQ ID NO:121, SEQ ID NO:125, SEQ ID NO:132, ID NO:165, SEQ ID NO:171, SEQ ID NO:187, SEQ ID NO:188, SEQ ID 229, SEQ ID NO:232, SEQ ID NO:273 and SEQ ID NO:281 in the Sequence Listing or a nucleic acid related thereto (e.g., a nucleic acid comprising a portion of the base sequence).

This invention also provides a diagnostic agent for the detection of a neurological disease characterized by containing at least one nucleic acid comprising a portion or the whole of the base sequence set forth in SEQ ID NO:1 to SEQ ID NO:366 in the Sequence Listing. Specifically, such tumor detection diagnostic agents include DNA chips and microarrays both of which are produced using the above-mentioned nucleic acids, for example. Accordingly, this invention also provides a microarray composition characterized by containing plural numbers of nucleic acids comprising a portion or the whole of the base sequence set forth in SEQ ID NO:1 to SEQ ID NO:366 in the Sequence Listing. Preferably, such composition comprises all the nucleic acids, i.e., a total of 366 nucleic acids each of which comprises a portion or the whole of a base sequence set forth in SEQ ID NO:1 to SEQ ID NO:366 in the Sequence Listing. More preferably, the composition is that which contains a total of 13 nucleic acids each of which comprises a portion or the whole of a base sequence set forth in SEQ ID NO:121, SEQ ID NO:125, SEQ ID NO:132, SEQ ID NO:133, SEQ ID NO:165, SEQ ID NO:171, SEQ ID NO:187, SEQ ID NO:188, SEQ ID NO:210, SEQ ID NO:229, SEQ ID NO:232, SEQ ID NO:273 and SEQ ID NO:281 in the Sequence Listing.

Further according to this invention there is provided an isolated nucleic acid which is DNA characterized by hybridizing to the nucleic acid mentioned above or to its complementary nucleic acid under stringent conditions. There is also provided a diagnostic kit for the prognosis of human neuroblastoma containing as the effective ingredient, a primer set comprising a pair of those nucleic acids (DNA).

In addition, this invention provides a method of diagnosing the prognosis of human neuroblastoma, the method comprising detecting the presence or absence of a nucleic acid comprising any one of base sequences set forth in SEQ ID NO:1 to NO:366 in the Sequence Listing from a clinical tissue sample of the neuroblastoma.

### Brief Description of the Drawings

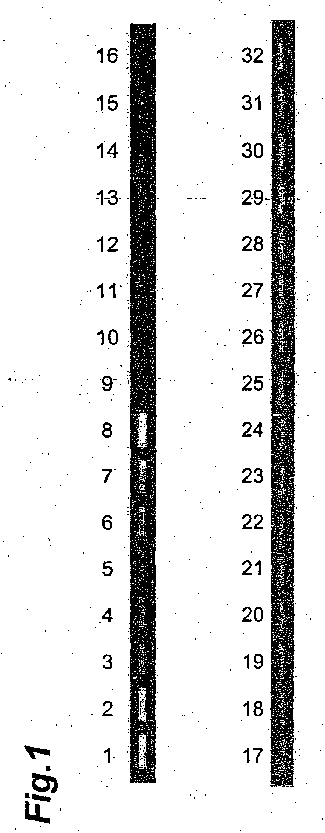

Fig. 1 is a figure corresponding to an electrophoregram showing an example of the results of determination of the gene expression levels in human neuroblastomas with favorable prognosis and with unfavorable prognosis by semi-quantitative PCR.
Fig. 2 is a figure corresponding to electron micrographs showing a primary culture containing SCG neurons of a new born mouse.

### Best Mode for Carrying Out the Invention

The term "nucleic acid(s)" as used in this invention refers to, for example, DNA or RNA, or polynucleotides derived therefrom which are active as DNA or RNA, and preferably they are DNA or RNA.

The term "hybridize under stringent conditions" means that two nucleic acid fragments hybridize to each other under the hybridization conditions described by Sambrook, J. et al. in "Expression of cloned genes in *E*. *coli*", Molecular Cloning: A Laboratory Manual (1989), Cold Spring Harbor Laboratory Press, New York, USA, 9.47-9.62 and 11.45-11.61.

More specifically, the "stringent conditions" refers to hybridization at approximately 45 °C, 6.0 x SSC, followed by washing at 50 °C, 2.0 x SSC. The stringency may be selected by choosing a salt concentration in the washing step from approximately 2.0 x SSC, 50 °C as low stringency to approximately 0.2 x SSC, 50 °C as high stringency. Also, the temperature in the washing step may be increased from room temperature, or approximately 22 °C as low stringency conditions, to approximately 65 °C as high stringency conditions.

The term "isolated nucleic acid(s)" as used in the present specification refers to a nucleic acid or a polynucleotide containing substantially no cellular substances or culture medium, if prepared by recombinant DNA techniques, or containing substantially no precursor chemical substances or other chemical substances, if prepared by chemical synthesis.

The term "favorable prognosis" as used in the present specification refers to a condition of human neuroblastoma in which the tumor is localized or has become a regressing or benign sympathetic ganglion neoplasm, and is judged to have low malignancy based on N-myc or other tumor markers. According to a preferred embodiment of the invention, a favorable prognosis is a case of stage 1 or 2, with an onset age of less than one year and survival without recurrence for 5 or more years after surgery, and with no noted amplification of N-myc in the clinical tissue; however, there is no limitation to such specific cases. The term "unfavorable prognosis" as used in the present specification refers to a condition of human neuroblastoma in which progression of the tumor has been observed, and it is judged to have high malignancy based on N-myc or other tumor markers. According to a preferred embodiment of the invention, an unfavorable prognosis is a case of stage 4, with an onset age of greater than one year, death within 3 years after surgery and noted amplification of N-myc in the clinical tissue; however, there is no limitation to such specific cases.

The nucleic acids of this invention have been found in the clinical tissues of human neuroblastoma and such nucleic acids have the characteristics described below.

Neuroblastoma is a tumor consisting of actual nerve cells, of which only two types of tumor are known in humans, and analysis of the genes expressed therein is expected to provide very useful knowledge for understanding the biology of nerve cells. Specifically, it is extremely difficult, and practically impossible, to obtain site-specific homogeneous tissue from the brain or peripheral nerves. On the other hand, a neuroblastoma consists of an almost homogeneous nerve cell population (though tumorized) derived from peripheral sympathetic nerve cells, and thus offers a high possibility of obtaining homogeneous expression of neuro-related genes. Furthermore, since neuroblastoma is a type of cancer, it will characteristically have many important genes expressed in the immature stage of neurogenesis.

Clinically and biologically, neuroblastoma can be neatly classified into favorable prognosis and unfavorable prognosis types. Cancer cells from neuroblastoma with favorable prognosis are characterized by having a very slow rate of proliferation, with spontaneous regression beginning at some point. Findings to date have confirmed that nerve cell differentiation and apoptosis (nerve cell death) occur in the spontaneous regression, and that the differentiation which occurs in the maturation stages of normal nerve cells and programmed cell death are phenomena very closely resembling each other. Consequently, it is highly probable that the analysis of genes expressed in such tumors will lead to obtaining important information relating to nerve cell differentiation and apoptosis.

Neuroblastomas with unfavorable prognosis are tumors consisting of cancer cells which continue to exhibit definitely malignant proliferation. The probability is very high, therefore, that they have a large number of important genes connected with nerve cell proliferation or genes expressed in undifferentiated nerve cells. In other words, it is highly probable that these will allow the obtainment of genetic information completely different from the profile of genes expressed in neuroblastomas with favorable prognosis.

It is commonly reported that nerve cells contain more expressed gene types than cells derived from other organs. Neuroblastoma cell lines are derived from clinical tissues with unfavorable prognosis, and it is believed that the gene expression profile in the case of tumor development and progression is substantially altered from that of normal nerve cells.

Neuroblastoma is characteristically a pediatric tumor, and because of the very low possibility of effects by acquired factors, it is expected that analysis of the mechanism of cancerization will also yield embryological information with high probability. More surprisingly, the genes or the gene fragments according to this invention include genes whose expression is enhanced only in specific cell cycle phases, and this further suggests the very strong possibility of obtaining genetic information highly useful for the analysis of cancerization mechanisms and related to development and differentiation.

The nucleic acids, having the characteristics mentioned above and from which the aforementioned information can be obtained, are available from human neuroblastoma clinical tissues and have any of the base sequences set forth in SEQ ID NO:1 to NO:366 in the Sequence Listing, or a portion thereof.

As a result of comparing gene expression levels in clinical tissues from human neuroblastomas with favorable prognosis and with unfavorable prognosis, a highly significant difference was found in each of the nucleic acids having base sequences set forth in SEQ ID NO:1 to NO:366 in the Sequence Listing. That is, the expression of these nucleic acids was enhanced in human neuroblastomas with favorable prognosis. Thus, in addition to providing the useful genetic information described above, the base sequences set forth in SEQ ID NO:1 to NO:366 can also be utilized as data for tumor markers to diagnose favorable or unfavorable prognosis of neuroblastoma, by detecting DNA and/or RNA having any of these base sequences.

Specifically, this invention will make it possible to obtain various forms of diagnosis for prognosis on or relating to human neuroblastoma through the following means.

### (1) Probes for hybridization

The nucleic acid comprising a portion or the whole of a base sequence disclosed in this invention (which may be referred to as "nucleic acid(s) of the invention") may be at least used as a probe for hybridization in order to detect genes expressed in human neuroblastoma. The nucleic acids of the invention may also be used as probes for hybridization in order to determine gene expression in various tumors and normal tissues, to identify the distribution of the gene expression.

When the nucleic acid comprising a portion or the whole of a base sequence disclosed in this invention is used as a probe for hybridization, there are no particular limitations on the actual method of hybridization. As preferred methods there may be mentioned, for example, Northern hybridization, Southern hybridization, colony hybridization, dot hybridization, fluorescence in situ hybridization (FISH), in situ hybridization (ISH), DNA chip methods, and microarray methods.

As one application example of the hybridization, the nucleic acid of this invention may be used as a probe for Northern hybridization to measure the length of mRNA or to quantitatively detect gene expression in an assayed sample.

When the nucleic acid of the invention is used as a probe for Southern hybridization, it enables, the detection of the presence or absence of the base sequence in genomic DNA of an assayed sample.

The nucleic acid comprising a portion or the whole of a base sequence disclosed in this invention can also be used as a probe for fluorescence in situ hybridization (FISH) to identify the location of the gene on a chromosome.

The nucleic acid of the invention can also be used as a probe for in situ hybridization (ISH) to identify the tissue distribution of gene expression.

When the nucleic acid of the invention is used as a probe for hybridization, a nucleic acid residue length of at least 40 is necessary; and among the gene sequences according to the invention, a nucleic acid having 40 or more contiguous residues is preferably used. More preferably, one having 60 or more nucleic acid residues is used.

Nucleic acid probe techniques are well known to one skilled in the art, and for example, conditions suitable for hybridization between a probe of specific length according to the invention and the target polynucleotide may be readily determined. In order to obtain hybridization conditions optimal to probes of various lengths, Sambrook et al. "Molecular Cloning: A Laboratory Manual, 2nd Edition, Cold Spring Harbor (1989) may be followed for such manipulations which are well known to one skilled in the art.

The probe of the invention is preferably labeled in an easily detectable fashion. The detectable label may be any type or portion which can be detected either visually or using devices. As commonly used detectable labels there may be mentioned radioactive isotopes such as ³²P, ¹⁴C, ¹²⁵I, ³H and ³⁵S. Biotin-labeled nucleotides may be incorporated into DNA or RNA by nick translation, chemical or enzymatic means. The biotin-labeled probes are detected after hybridization using labeling means such as avidin/streptavidin, fluorescent labels, enzymes, gold colloidal complexes or the like. The nucleic acid may also be labeled by binding with a protein. Nucleic acid cross-linked to a radioactive or fluorescent histone single-stranded DNA binding protein may also be used.

### (2) Primers for use in PCR

For other possible methods of detecting genes, DNA that is the nucleic acid comprising a portion or the whole of a base sequence disclosed in this invention can be used as a primer in a polymerase chain reaction (PCR). For example, RNA may be extracted from a sample to be assayed, and the gene expression can be semi-quantitatively measured by RT-PCR. This may be carried out by a method well known to one skilled in the art. For example, "Molecular Cloning: A Laboratory Manual," (T. Maniatis, Cold Spring Harbor Laboratory Press) or Idenshibyo Nyumon [Introduction to Genetic Diseases] (Takahisa, S.: Nankodo Publishing) may be followed.

When the DNA that is a nucleic acid of the invention is used as a PCR primer, a base length of 10 to 60 is necessary; and among the gene sequences according to the invention, the nucleic acid having 10 to 60 contiguous bases is preferably used. More preferably, one having 15 to 30 bases is used. Generally, a primer sequence with a GC content of 40-60% is preferred. Also, there is preferably no difference in the Tm values of the two primers used for amplification. Preferably there is no annealing at the 3' ends of the primers and no secondary structure is formed in the primers.

### (3) Nucleic acid screening

The nucleic acid comprising a portion or the whole of a base sequence disclosed in this invention can also be used to detect the expression distribution of a gene which is expressed in various tissues or cells. This can be accomplished, for example, by using the nucleic acid of the invention as a probe for hybridization or as a primer for PCR.

Expression distribution of a gene can also be detected using a DNA chip, microarray or the like. That is, the nucleic acid of the invention may be directly attached to the chip or array. RNA extracted from cells of a tissue may be labeled there with a fluorescent substance or the like, hybridized, and an analysis can be made of the type of tissue cells with high expression of the gene. The DNA attached to the chip or array may be the reaction product of PCR using the nucleic acid of the invention. United State Patent No. 5605662 to Heller et al. describes an example of the method for attaching nucleic acids to a chip or array.

By utilizing the aforementioned technology, it is possible to use as a diagnostic agent, at least one among the nucleic acids comprising portions or the whole of base sequences disclosed in this invention. In recent years, it has becoming clear that the genetic information possessed by individuals governs their susceptibility to a certain disease as well as governs the effectiveness of a particular drug. The DNA chips or microarrays produced using the aforementioned nucleic acids are used to clarify the cause-effect relationship between the disease of a subject and the nucleic acid, which will enable not only the diagnosis of that disease but also the selection of a drug to be administered. Specifically, if the result detected using the DNA chip or microarray is employed as an indicator of selection of a drug to be administered, the expression level of one nucleic acid among the nucleic acids of the invention can be examined but also the expression levels of two or more nucleic acids can be comparatively examined to select the drug to be administered, which will then enable more accurate judgment. As used herein, the disease is not particularly limited insofar as it is that which can be diagnosed by the nucleic acids of this invention. Preferably, the disease is a neurological disease and more preferably, neuroblastoma.

### (4) DNA cloning

The nucleic acid comprising a portion or the whole of a base sequence disclosed in this invention can be at least used for cloning a gene which is expressed in human neuroblastoma. For example, by using the nucleic acid of the invention as a probe for Northern hybridization or Southern hybridization, or as a primer for PCR, cloning of a gene containing the nucleic acid comprising a portion or the whole of a base sequence disclosed in this invention is possible. As the genes capable of being subjected to cloning there may be mentioned genes with differing levels of expression particularly between neuroblastoma with favorable prognosis and neuroblastoma with unfavorable prognosis, genes whose forms of expression differ in other tissues or cancer cells, genes whose expression is cell cycle phase-dependent, genes induced upon neurodifferentiation and genes whose expression is regulated by oncogenes or tumor suppressor genes.

### (5) Methods of diagnosing tumor prognosis and tumor markers to be used therefor

The nucleic acid comprising a portion or the whole of a base sequence disclosed in this invention can be used as a probe for hybridization, or as a primer for PCR to determine the presence or absence of enhancement in the gene expression in sample cells, which enables the identification of prognosis. To determine the presence or absence of enhancement in the gene expression, any method that utilizes nucleic acids (probes) capable of hybridizing to the nucleic acid comprising any given sequence of the base sequence disclosed in this invention is provided for use. Prognosis can be diagnosed as favorable if the amount of nucleic acid hybridizing to the probe is increased in the sample cell. When the nucleic acid is used as a primer for PCR, RNA is extracted from the sample to be assayed and the gene expression can be semi-quantitatively measured by the RT-PCR method.

### (6) Antisense oligonucleotides

According to another embodiment of this invention there are provided antisense oligonucleotides having the base sequences according to the invention. As will be considered in practicing this invention, the antisense oligonucleotides may readily be prepared such that they can bind to RNA corresponding to the base sequence of this invention and can thereby inhibit the synthesis of RNA.

### (7) Gene therapy

According to a further embodiment of this invention, there are provided therapeutic genes to be used in gene therapy. As will be considered in practicing this invention, the gene according to this invention may be transferred into a vector for use in gene transportation, whereby the transgene can be expressed by an arbitrary expression promoter and can be used for the gene therapy of cancers, for example.

### 1. Vectors

The transferable viral vectors may be prepared from DNA viruses or RNA viruses. They may be any viral vector of an MoMLV vector, a herpes virus vector, an Adenovirus vector, an AAV vector, a HIV vector, a Seidai virus vector and the like. One or more proteins among the constituent protein group of a viral vector are substituted by the constituent proteins of a different species of virus, or alternatively a part of the base sequence constituting genetic information is substituted by the base sequence of a different species of virus to form a viral vector of the pseudo-type which can also be used in this invention. For example, there is mentioned a pseudo-type viral vector wherein the Env protein (an envelop protein of HIV) is substituted by the VSV-G protein (an envelop protein of vesicular stomatitis virus or VSV) (Naldini L., et al., Science 272, 263-267, 1996). Further, virues having a host spectrum other than human is usable as the viral vector insofar as they are efficacious. As for the vectors other than those of viral origin, there may be used complexes of calcium phosphate and nucleic acid, ribosomes, cation-lipid complexes, Seidai virus liposomes, polymer carriers having polycation as the main chain and others. In addition, methods such as electroporation and gene guns may be used as a gene transfer system.

### 2. Expression promoters

As for the expression cassettes to be used for the therapeutic gene, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, and most preferably expression cassettes capable of gene expression in the cells derived from a human. The gene promoters that can be used as expression cassettes include: for example, virus-derived promoters from an Adenovirus, a cytomegalovirus, a human immunodeficiency virus, a simian virus 40, a Rous sarcoma virus, a herpes simplex virus, a murine leukemia virus, a sinbis virus, a hepatitis type A virus, a hepatitis type B virus, a hepatitis type C virus, a papilloma virus, a human T cell leukemia virus, an influenza virus, a Japanese encephalitis virus, a JC virus, parbovirus B19, a poliovirus, and the like; mammal-derived promoters such as albumin, SRα, a heat shock protein, and an elongation factor; chimera type promoters such as a CAG promoter; and the promoters whose expression can be induced by tetracyclines, steroids and the like.

### EXAMPLES

This invention will now be explained in greater detail by way of the examples and production examples; however, the invention will not be restricted to those example.

### (Production Example 1) Construction of cDNA library from human neuroblastoma

### 1. Obtaining samples

Human neuroblastoma clinical tissue specimens were quasi-aseptically frozen immediately after surgical extraction and then preserved at -80°C.

### 2. Selecting samples with favorable prognosis

Prognosis of the samples obtained in 1. above was carried out based on the following criteria.

Favorable prognosis:
· Stage 1 or 2
· Age of onset less than one year
· Survival for longer than 5 years after surgery without recurrence
· No amplification of N-myc

Unfavorable prognosis:
· Stage 4
· Age of onset older than 4 years
· Death within 3 years after surgery
· Amplification of N-myc

Amplification of N-myc in the aforementioned sample types was confirmed in the following manner.

The clinical tissue sample obtained in 1. above was thinly sliced with a scalpel and then thoroughly homogenized after addition of 5 ml of TEN buffer (50 mM Tris-HCl (pH = 8.0)/1 mM EDTA/100 mM NaCl). Upon adding 750 µl of SDS (10%) and 125 µl of proteinase K (20 mg/ml) to the mixture, it was gently stirred and allowed to stand at 50 °C for 8 hours. This was followed by phenol/chloroform treatment and finally ethanol precipitation to obtain purified genomic DNA. A 5 µg portion of the obtained genomic DNA was completely digested with the restriction endonuclease EcoRI (NEB Inc.), and an N-myc probe was used to determine amplification of N-myc by Southern hybridization.

### 3. Preparation of mRNA from clinical tissue of human neuroblastoma with favorable prognosis

A 2-3 g portion of the clinical tissue sample of human neuroblastoma judged to have favorable prognosis in 2. above was treated using a Total RNA Extraction Kit (QIAGEN Inc.) and the total RNA was extracted. The extracted total RNA was purified using an oligo dT cellulose column (Collaborative Research, Inc.) to obtain a pool of mRNA with a polyA structure.

### 4. Dephosphorylation of mRNA

A 100-200 µg portion of the mRNA pool prepared in 3. above was dissolved in 67.3 µl of distilled sterile water containing 0.1% diethyl pyrocarbonate (DEPC), and then 20 µl of 5XBAP buffer (Tris-HCl (500 mM, pH = 7.0)/mercaptoethanol (50 mM)), 2.7 µl of RNasin (40 unit/ µl: Promega Inc.) and 10 µl of BAP (0.25 unit/µl, bacteria-derived alkali phosphatase: Takara Shuzo Co. Ltd.) were added. The mixture was reacted at 37 °C for 1 hour to effect dephosphorylation of the 5' end of the mRNA. This was followed by phenol/chloroform treatment two times, and finally ethanol precipitation to obtain a purified dephosphorylated mRNA pool.

### 5. Decapping of dephosphorylated mRNA

The total amount of the dephosphorylated mRNA pool prepared in 4. above was dissolved in 75.3 µl of distilled sterile water containing 0.1% DEPC, and then 20 µl of 5XTAP buffer (sodium acetate (250 mM, pH = 5.5)/mercaptoethanol (50 mM), EDTA (5 mM, pH = 8.0)), 2.7 µl of RNasin (40 unit/µl) and 2 µl of TAP (tobacco acid pyrophosphatase: 20 unit/µl) were added. The mixture was reacted at 37 °C for 1 hour to effect decapping treatment of the 5' end of the dephosphorylated mRNA. The dephosphorylated mRNA of incomplete length with no capped structure remained without decapping, and with the 5' end dephosphorylated. This was followed by phenol/chloroform treatment and ethanol precipitation to obtain a purified decapped mRNA pool.

### 6. Preparation of oligo-capped mRNA

The total amount of the decapped mRNA pool prepared in 5. above was dissolved in 11 µl of distilled sterile water containing 0.1% DEPC, and then 4 µl of 5'-oligo RNA (5'-AGCAUCGAGUCGGCCUUGGCCUACUGG-3': 100 ng/µl), 10 µl of 10X ligation buffer (Tris-HCl (500 mM, pH = 7.0)/mercaptoethanol (100 mM)), 10 µl of magnesium chloride (50 mM), 2.5 µl of ATP (24 mM), 2.5 µl of RNasin (40 unit/µl), 10 µl of T4 RNA ligase (25 unit/µl: Takara Shuzo Co. Ltd.) and 50 µl of polyethylene glycol (50% w/v, PEG8000: Sigma Corporation) were added. The mixture was reacted at 20 °C for 3 hours for ligation of the 5'-oligo RNA to the 5' end of the decapped mRNA. The dephosphorylated mRNA of incomplete length with no capped structure resulted in no ligation to the 5'-oligo RNA. This was followed by phenol/chloroform treatment and ethanol precipitation to obtain a purified oligo-capped mRNA pool.

### 7. Removal of DNA from oligo-capped mRNA

The oligo-capped mRNA pool prepared in 6. above was dissolved in 70.3 µl of distilled sterile water containing 0.1% DEPC, and then 4 µl of Tris-HCl (1 M, pH = 7.0), 5.0 µl of DTT (0.1 M) , 16 µl of magnesium chloride (50 mM), 2.7 µl of RNasin (40 unit/µl) and 2 µl of DNaseI (5 unit/µl: Takara Shuzo Co. Ltd.) were added. The mixture was reacted at 37 °C for 10 minutes to dissolve the excess DNA. This was followed by phenol/chloroform treatment and ethanol precipitation and column purification (S-400HR: Pharmacia Biotech Inc.), to obtain a purified DNA(-) oligo-capped mRNA pool.

### 8. Preparation of 1st strand cDNA

The DNA(-) oligo-capped mRNA pool prepared in 7. above was reverse transcribed using SuperScript II (kit by Life Tech Oriental, Inc.) to obtain a pool of 1st strand cDNA. The pool of DNA(-) oligo-capped mRNA was dissolved in 21 µl of sterile distilled water, and then 10 µl of 10X First Strand buffer (kit accessory), 8 µl of dNTP mix (5 mM, kit accessory), 6 µl of DTT (0.1 M, kit accessory), 2.5 µl of oligo-dT adapter primer (5 pmol/µl, 5'-GCGGCTGAAGACGGCCTATGTGGCC TTTTTTTTTTTTTTTTT-3'), 2.0 µl of RNasin (40 unit/µl) and 2 µl of SuperScript II RTase (kit accessory) were added. The mixture was reacted at 42 °C for 3 hours to effect reverse transcription. This was followed by phenol/chloroform treatment, alkali treatment and neutralization treatment to dissolve all the RNA and purification was carried out by ethanol precipitation.

### 9. Preparation of 2nd strand cDNA

The 1st strand cDNA pool prepared in 8. above was subjected to PCR amplification using Gene Amp (kit by Perkin Elmer Inc.). The pool of 1st strand cDNA was dissolved in 52.4 µl of sterile distilled water, and then 30 µl of 3.3X Reaction buffer (kit accessory), 8 µl of dNTP mix (2.5 mM, kit accessory), 4.4 µl of magnesium acetate (25 mM, kit accessory), 1.6 µl of Primer F (10 pmol/µl, 5'-AGCATCGAGTCGGCCTTGTTG-3'), 1.6 µl of Primer R (10 pmol/µl, 5'-GCGCTGAAGACGGCCTATGT-3') and 2 µl of rTth (kit accessory) were added. A 100 µl portion of mineral oil was gently added to the mixture and overlayed thereon. After denaturing the reaction solution at 94 °C for 5 minutes, a cycle of 94 °C for 1 minute, 52 °C for 1 minute and 72 °C for 10 minutes was repeated 12 times, and then the solution was allowed to stand at 72 °C for 10 minutes to complete the PCR reaction. This was followed by phenol/chloroform treatment and ethanol precipitation to obtain a 2nd strand cDNA pool.

### 10. SfiI treatment of 2nd strand cDNA

The 2nd strand cDNA pool prepared in 9. above was dissolved in 87 µl of sterile distilled water, and then 10XNEB buffer (NEB Inc.), 100XBSA (bovine serum albumin available from NEB Inc.) and 2 µl of SfiI (restriction endonuclease, 20 unit/µl, NEB Inc.) were added. The mixture was reacted overnight at 50 °C to effect SfiI restriction endonuclease treatment. This was followed by phenol/chloroform treatment and ethanol. precipitation to obtain a pool of cDNA which had been SfiI-treated at both ends.

### 11. Size fractionation of SfiI-treated cDNA

The SfiI-treated cDNA pool prepared in 10. above was electrophoresed on 1% agarose gel and a fraction with >2 kb was purified using Geneclean II (Bio101 Inc.). The purified cDNA pool was dissolved in 100 µl of sterile distilled water and allowed to stand at 37 °C for 6 hours. This was followed by phenol/chloroform treatment and ethanol precipitation to obtain a long-chain cDNA pool.

### 12. Preparation of cDNA library

The long-chain cDNA pool prepared in 11. above was ligated into the cloning vector pME18S-FL3 (provided by Prof. Sumio Kanno of the Institute of Medical Science, Tokyo University) using a DNA Ligation Kit ver.1 (kit by Takara Shuzo Co. Ltd.). The long-chain cDNA pool was dissolved in 8 µl of sterile distilled water, and then 1 µl of pME18S-FL3 pretreated with restriction endonuclease DraIII, 80 µl of Solution A (kit accessory) and 10 µl of Solution B (kit accessory) were added and reaction was conducted at 16 °C for 3 hours. This was followed by phenol/chloroform treatment and ethanol precipitation for purification to obtain a cDNA library.

### (Example 1) Transformation into E. coli

The cDNA library prepared in Production Example 1 above was used for transformation into *E. coli* (TOP-10: Invitrogen Corporation). The cDNA library was dissolved in 10µl of sterile distilled water and mixed with TOP-10. The mixture was then incubated on ice for 30 minutes, at 40 °C for 1 minute and on ice for 5 minutes. After adding 500 µl of SOB medium, shake culturing was performed at 37 °C for 60 minutes. Appropriate amounts thereof were seeded onto ampicillin-containing agar media and culturing was continued at 37 °C for a day and a night to obtain *E*. *coli* clones.

The *E*. *coli* clones on agar media obtained were collected with toothpick and suspended in 120 µl of LB medium prepared in a 96-well plate. The 96-well plate was then allowed to stand overnight at 37 °C for culturing of the *E*. *coli*. A 72 µl portion of 60% glycerol solution was then added and preserved at -20 °C (glycerol stock).

### (Example 2) Base sequence determination

### 1. Preparation of plasmid

The 10 µl of glycerol stock prepared in Example 1 was transferred to a 15 ml centrifugation tube, and then 3 ml of LB medium and 50 µg/ml of ampicillin were added and shaking was carried out overnight at 37 °C for culturing of the *E*. *coli*. A QIAprep Spin Miniprep Kit (QIAGEN Inc.) was then used to extract and purify a plasmid DNA from the *E*. *coli*.

### 2. Analysis of both end sequences

Both end sequences of the plasmid DNA prepared in 1. above were determined using a DNA Sequencing Kit (kit by ABI). There were combined 600 ng of plasmid DNA, 8 µl of premix (kit accessory) and 3.2 pmol of primers, and sterile distilled water was added to a total of 20 µl. After denaturing the mixture at 96 °C for 2 minutes, a cycle of. 96 °C for 10 seconds, 50 °C for 5 seconds and 60 °C for 4 minutes was repeated 25 times for reaction. The product was then purified by ethanol precipitation. Sequencing was carried out by polyacrylamide gel electrophoresis under denaturing conditions, using ABI377 (ABI).

### (Example 3) Homology search using database

An Internet-mediated DNA sequence homology search was conducted for the samples on which the both end-sequences were analyzed in Example 2. The homology search was conducted using the BLAST of the NCBI (National Center of Biotechnology Information, USA).

### (Example 4) Determination of gene expression levels in human neuroblastomas with favorable prognosis and unfavorable prognosis by semi-quantitative PCR

PCR primers were synthesized from portions of the genes obtained in Example 3, and the expression levels in the clinical tissues of human neuroblastomas with favorable prognosis and unfavorable prognosis were comparatively quantitated. mRNA was extracted from the human neuroblastoma clinical tissues by the method described in Examples 3, and rTaq (Takara Shuzo Co. Ltd.). was used for PCR reaction. Specifically, 5 µl of sterile distilled water, 2 µl of mRNA, 1 µl of 10XrTaq buffer, 1 µl of 2 mM dNTPs, 0.5 µl each of the synthesized primer set and 0.5 µl of rTaq were combined. After denaturing the mixture at 95 °C for 2 minutes, a cycle of 95 °C for 15 seconds, 55 °C for 15 seconds and 72 °C for 20 seconds was repeated 35 times, and then the mixture was allowed to stand at 72 °C for 6 minutes for PCR reaction. The reaction solution was subjected to 1% agarose gel electrophoresis. One example of the results of determination of the gene expression levels in human neuroblastomas with favorable prognosis and unfavorable prognosis by semi-quantitative PCR is shown in Fig: 1. Here, the explanations of the respective lanes are as follows:
Lanes 1-8: expression of nbla-4183 (SEQ ID NO:154, SEQ ID NO:155 in the Sequence Listing) in a clinical sample of neuroblastoma with favorable prognosis;
Lanes 9-16: expression of nbla-4183 (SEQ ID NO:154, SEQ ID NO:155 in the Sequence Listing) in a clinical sample of neuroblastoma with unfavorable prognosis;
Lanes 17-24: expression of GAPDH in a clinical sample of neuroblastoma with favorable prognosis; and
Lanes 25-32: expression of GAPDH in a clinical sample of neuroblastoma with unfavorable prognosis.

Consequently, nucleic acids whose expression is enhanced only in human neuroblastoma with favorable prognosis have been identified in the base sequences set forth in SEQ ID NO:1 to SEQ ID NO:366.

### (Example 5) Gene group with varying gene expression levels by NGF regulated apoptosis in murine superior cervical gangliocytes.

It is known that NGF (nerve growth factor) exerts protective action against apoptosis in human neuroblastoma induced by an antimitotic agent (such as neocarzinostatin). (Cortazzo MH, J. Neurosci, 16, 3895-3899 (1996)). This action is believed to be due to the inhibition of apoptosis by the binding of NGF to p75. It has also been pointed out that the regression of neuroblastoma (causing favorable prognosis) at clinical stage is connected to the apoptosis of the tumor cells.

In order to identify the apoptosis-associated genes in neuroblastoma, the gene group with varying gene expression levels by NGF regulated apoptosis in murine superior cervical gangliocytes was examined.

Superior cervical gangliocytes (SCG neurons) were isolated from C57BL/6J mice (0 to 1 day of age) in 7 to 10 animals. Seven wells of cells were arranged to be grown on a 24-well cell culture plate at about 1 to about 2x10⁴ cells per well. RNA was further collected from about 1 to about 2x10⁴ cells (Day 1 as control).

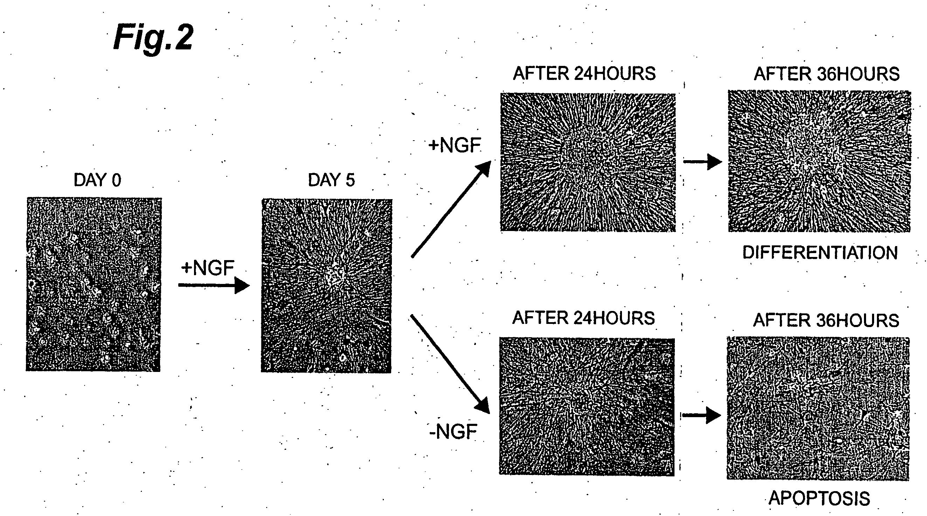

After the culture obtained by adding NGF (5 mg/ml) to the 7-well cells was incubated for 5 days, RNA was collected from one-well cells (+NGF; after 5 days). Cultures in three wells each of the 6 wells were exchanged with cultures with addition of NGF (5 mg/ml) or anti-NGF antibodies (1%), and they were respectively incubated for 12 hours, 24 hours and 48 hours. Using portions of the samples, cells were examined under an electron microscope to identify the morphological differences between the case where NGF had been added and the case where anti-NGF antibodies had been added. They results are shown in Fig. 2. NGF induced differentiation was observed in the cells with NGF addition, whereas apoptosis was observed in the cells with anti-NGF antibody addition. As a result of NGF depletion by the anti-NGF antibodies, it was apparent that apoptosis was induced. RNA was collected from the respective well cells after 12 hr, 24 hr, and 48 hr culturing. These samples were denoted +NGF 12 hrs, +NGF 24 hrs, +NGF 48 hrs, -NGF 12 hrs, -NGF 24 hrs, and -NGF 48 hrs.

As described above, RNAs of the respectively treated cells from approximately 160 mice were concentrated by ethanol precipitation and cDNAs were synthesized from 1 µg RNAs using reverse transcriptase. These cDNAs were used as templates and gene expression levels were compared by PCR. The primers used were constructed from the homologous genes in mice based on the genes found in human neuroblastoma cells. The base sequences of the primers used for the comparison of expression are summarized in Table 1 together with the gene identification numbers (ID) for the targeted genes for comparison.

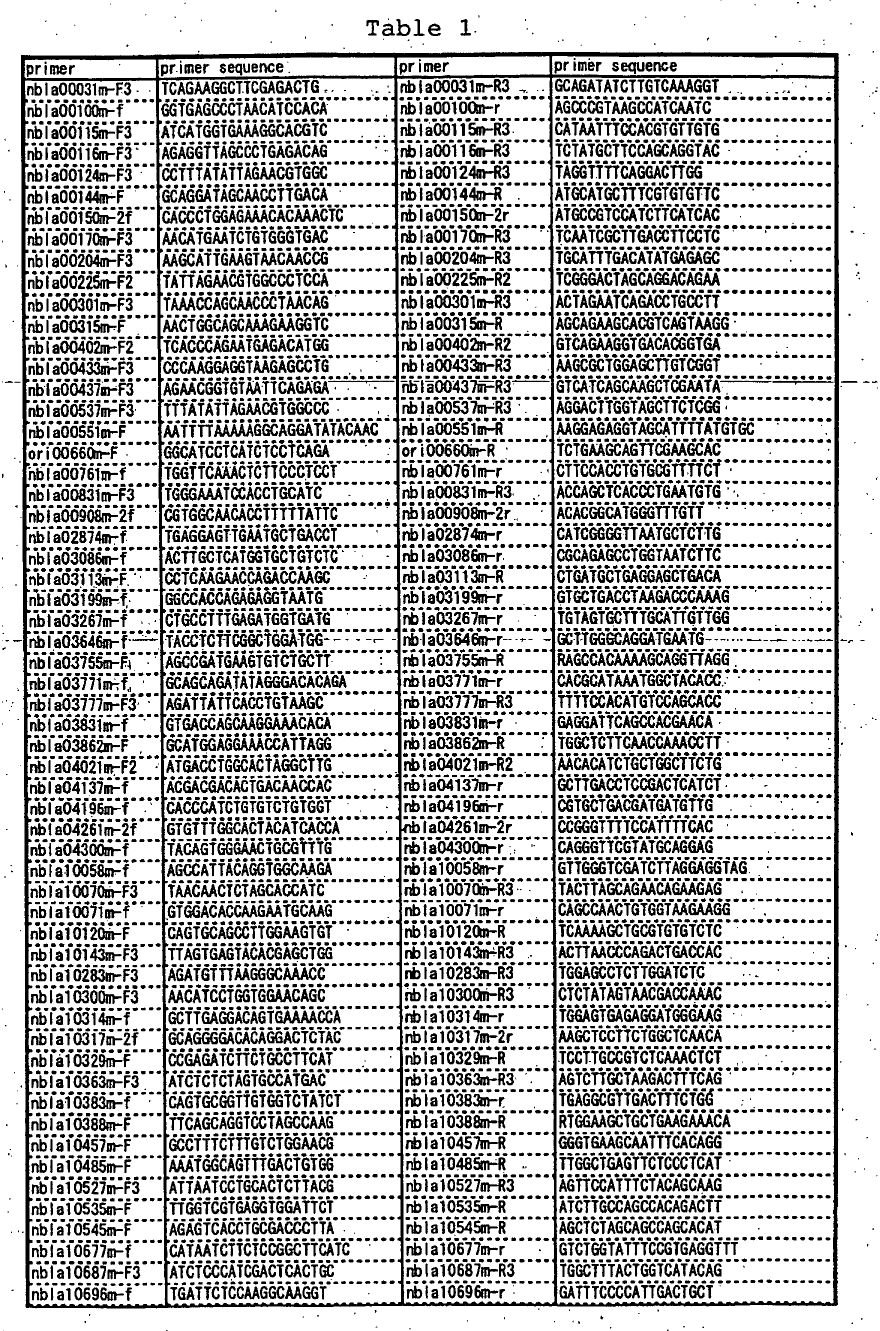

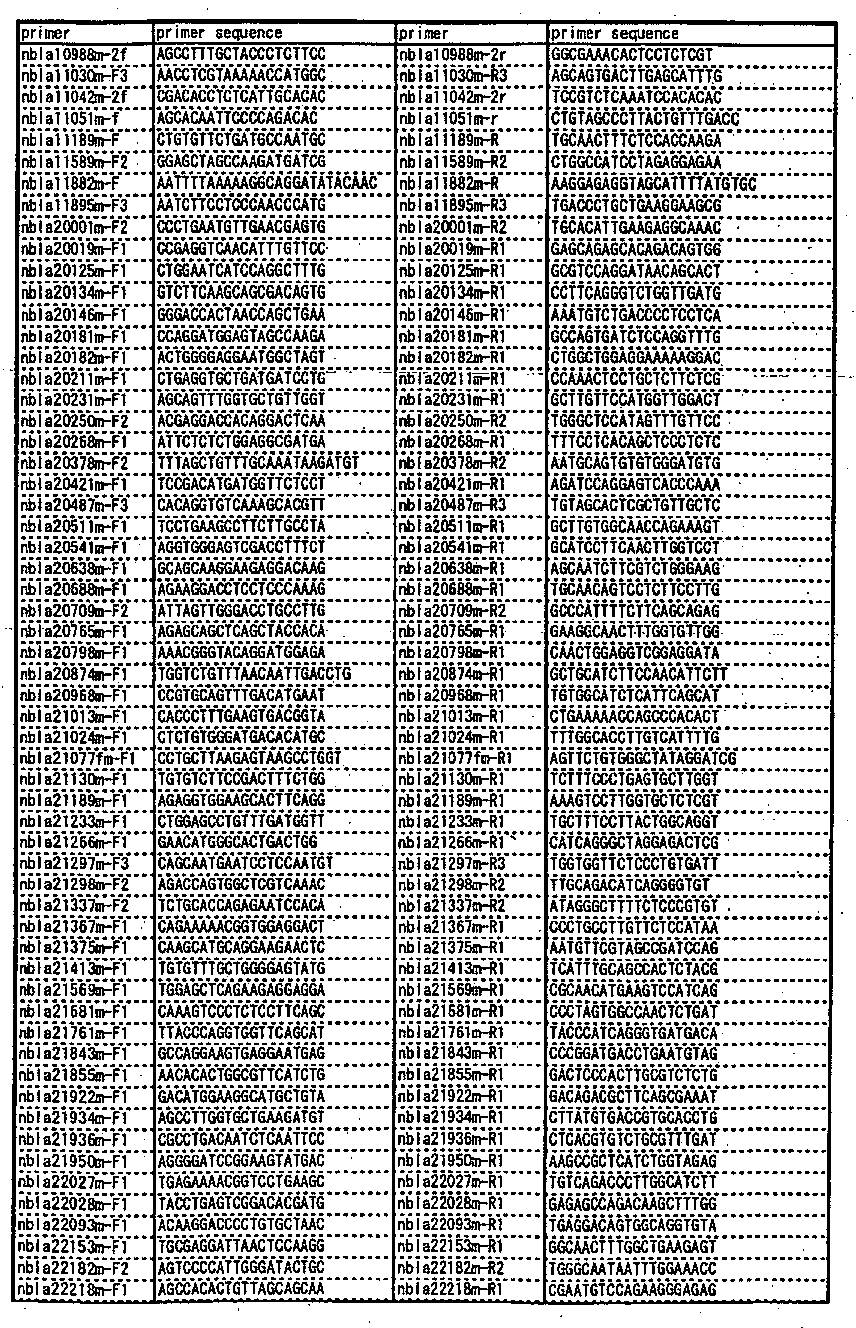

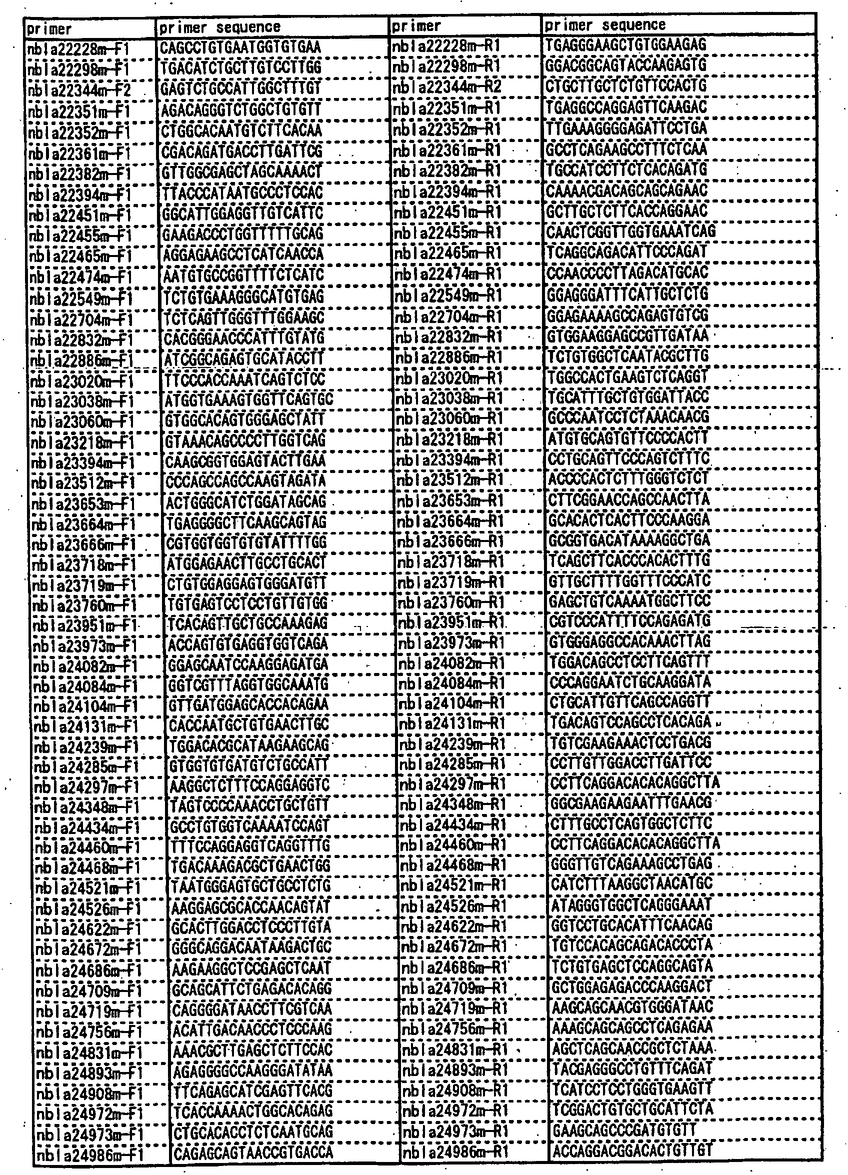

The results described below were obtained with the genes for which variations in the gene expression level were observed. The genes whose expression levels were decreased notably by NGF depletion (-NGF) were nbla-03646 (SEQ ID NO:121)(-NGF 12 hrs), nbla-03771 (SEQ ID NO:125)(-NGF 12 hrs) nbla-03831 (SEQ ID NO:132) (-NGF 24 hrs), nbla-04300 (SEQ ID NO:165) (-NGF 24 hrs), nbla-10120 (SEQ ID NO:171) (-NGF 48 hrs), nbla-10329 (SEQ ID NO:187) (-NGF 24 hrs), nbla-10363 (SEQ ID NO:188) (-NGF 12 hrs), nbla-10677 (SEQ ID NO:229)(-NGF 12 hrs), nbla-10696 (SEQ ID NO:232)(-NGF 12 hrs) , nbla-11051 (SEQ ID NO:273)(-NGF 24 hrs), and nbla-11189 (SEQ ID NO:281)(-NGF 24 hrs). The genes whose expression levels were increased notably by NGF depletion (-NGF) was noted were nbla-03862 (SEQ ID NO:133)(-NGF 24 hrs) and nbla-10485 (SEQ ID NO:210) (-NGF 24 hrs). The gene whose expression level was increased notably by NGF addition was noted was nbla-10143 (SEQ ID NO:174) (+NGF).

The primer sets used to amplify those particular genes are shown in Table 2.

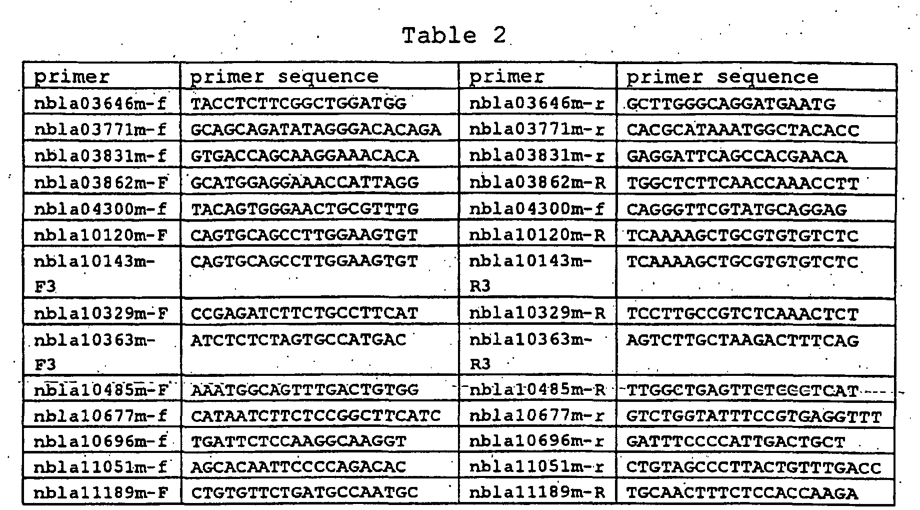

As described above, because the superior cervical gangliocytes are known to die (apoptosis) through NGF depletion, the genes that display variations in the expression level are closely connected to the mechanism of apoptosis.

### Industrial Applicability

As described above, this invention discloses gene sequences relating to favorable or unfavorable prognosis of neuroblastoma and will enable the provision of their genetic information and the diagnosis of favorable or unfavorable prognosis.

## Claims

1. A nucleic acid whose expression is enhanced in human neuroblastoma with favorable prognosis based on comparison between human neuroblastoma with favorable prognosis and human neuroblastoma with unfavorable prognosis, the nucleic acid comprising any one of base sequences set forth in SEQ ID NO:1 to NO:366 in the Sequence Listing.

2. The nucleic acid according to claim 1, wherein the base sequence is any one of base sequences set forth in SEQ ID NO:121, SEQ ID NO:125, SEQ ID NO:132, SEQ ID NO:133, SEQ ID NO:165, SEQ ID NO:171, SEQ ID NO:187, SEQ ID NO:188, SEQ ID NO:210, SEQ ID 229, SEQ ID NO:232, SEQ ID NO:273 and SEQ ID NO:281 in the Sequence Listing.

3. The nucleic acid according to claim 1, wherein the. base sequence is any one of base sequences set forth in SEQ ID NO:121, SEQ ID NO:125, SEQ ID NO:132, ID NO:165, SEQ ID NO:171, SEQ ID NO:187, SEQ ID NO:188, SEQ ID 229, SEQ ID NO:232, SEQ ID NO:273 and SEQ ID NO:281 in the Sequence Listing.

4. A nucleic acid comprising a portion of any one of base sequences set forth in SEQ ID NO:1 to NO:366 in the Sequence Listing.

5. The nucleic acid according to claim 4, wherein the portion of the base sequence is derivable from any one of the base sequences set forth in SEQ ID NO:121, SEQ ID NO:125, SEQ ID NO:132, SEQ ID NO:133, SEQ ID NO:165, SEQ ID NO:171, SEQ ID NO:187, SEQ ID NO:188, SEQ In NO:210, SEQ ID 229, SEQ ID NO:232, SEQ ID NO:273 and SEQ ID NO:281 in the Sequence Listing.

6. The nucleic acid according to claim 4, wherein the portion of the base sequence is derivable from any one of the base sequences set forth in SEQ ID NO:121, SEQ ID NO:125, SEQ ID NO:132, ID NO:165, SEQ ID NO:171, SEQ ID NO:187, SEQ ID NO:188, SEQ ID 229, SEQ ID NO:232, SEQ ID NO:273 and SEQ ID NO:281 in the Sequence Listing.

7. An isolated nucleic acid capable of hybridizing to the nucleic acid according to any of claims 1 to 4 or a complementary nucleic acid thereof.

8. A diagnostic agent for the detection of a neurological disease containing at least one, nucleic acid comprising a portion or the whole of a base sequence set forth in SEQ ID NO:1 to SEQ ID NO:366 in the Sequence Listing.

9. The diagnostic agent for the detection of a neurological disease according to claimi 8, wherein the base sequence is any one of the base sequences set forth in SEQ ID NO:121, SEQ ID NO:125, SEQ ID NO:132, SEQ ID NO:133, SEQ ID NO:165, SEQ ID NO:171, SEQ ID NO:187, SEQ ID NO:188, SEQ ID NO:210, SEQ ID 229, SEQ ID NO:232, SEQ ID NO:273 and SEQ ID NO:281 in the Sequence Listing.

10. The diagnostic agent for the detection of a neurological disease according to claim 8, wherein the base sequence is any one of the base sequences set forth in SEQ ID NO:121, SEQ ID NO:125, SEQ ID NO:132, ID NO:165, SEQ ID NO:171, SEQ ID NO:187, SEQ ID NO:188, SEQ ID 229, SEQ ID NO:232, SEQ ID NO:273 and SEQ ID NO:281 in the Sequence Listing.

11. The isolated nucleic acid according to claim 7, wherein the nucleic acid is DNA.

12. A diagnostic kit for the prognosis of human neuroblastoma containing as the effective ingredient, a primer set comprising a pair of the isolated nucleic acids according to claim 11.

13. A method of diagnosing the prognosis of human neuroblastoma, the method comprising detecting the presence or absence of a nucleic acid comprising any one of base sequences set forth in SEQ ID NO:1 to NO:366 in the Sequence Listing from a clinical tissue sample of the neuroblastoma.
